# EUROPEAN PATENT APPLICATION

(11) **EP 4 338 737 A1**
(43) Date of publication of application: **20.03.2024**
(21) Application number: 22934891.7
(22) Date of filing: 06.12.2022
(51) Int. Cl.: A61K 31/501, A61K 31/502, A61K 31/5025, C07D 403/14, C07D 401/14, A61P 35/00, A61P 37/00

(54) **PARP7 INHIBITOR AND USE THEREOF**

(30) Priority: 01.04.2022 CN 202210339089
(71) Applicant: Novostar Pharmaceuticals, Ltd., Shanghai 201210 (CN)
(72) Inventor: LI, Bing, Shanghai 201210 (CN); ZHANG, Ke, Shanghai 201210 (CN); NING, WenXing, Shanghai 201210 (CN); ZUO, XiaoFei, Shanghai 201210 (CN); HUANG, QiongLin, Shanghai 201210 (CN)
(74) Representative: Abel & Imray LLP
(86) International application number: PCT/CN2022/136804
(87) International publication number: WO 2023/185073

(57) **Abstract**

A poly(adenosine diphosphate-ribose)polymerase-7 (PARP7) inhibitor, and the use thereof in selectively inhibiting PARP7 activity or in treating or preventing diseases, disorders or conditions that are regulated by or affected by PARP7 activity or involve PARP7 activity or overexpression therein, wherein the inhibitor comprises a compound of formula (I) or a pharmaceutically acceptable salt, deuterated compound, solvate, ester, acid, metabolite or prodrug thereof.

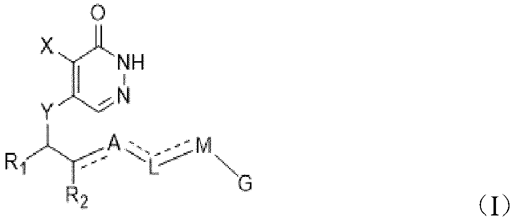

## Description

### FIELD OF THE INVENTION

The invention relates to the field of pharmaceuticals and, in particular, to pyridazinone derivatives and their preparation, as well as methods and uses thereof for the treatment and/or prevention of diseases.

### BACKGROUND OF THE INVENTION

PARP (poly ADP-ribose polymerase), a family of 17 enzymes that utilize nicotinamide adenine dinucleotide (NAD+) as a substrate to transfer ADP-ribose (ADPR) to target proteins, is a multifunctional protein post-translationally modifying enzyme present in most eukaryotic cells. It is activated by recognizing structurally damaged DNA segments and is considered a DNA damage receptor involved in a range of cellular processes such as DNA repair and genome stability.

The PARP family is divided into two subclasses: poly-PARPs and mono-PARPs. Poly-PARPs add multiple ADPR (ADP-ribose) molecules to proteins sequentially, resulting in ADPR chains that can reach hundreds of units. In contrast, mono-PARPs modify proteins by attaching only one ADPR molecule. Thus, poly-PARPs and mono-PARPs represent two different types of therapeutic targets because they have different ADP-ribosylated protein substrates and play different roles in cell signaling and regulation of protein function.

PARP7 (TIPARP), a mono-PARP, is a key factor in the regulation of innate immunity, transcription factor activity and cellular stress response. Several studies have shown that PARP7 plays a key role in innate immune signaling pathways, particularly as a negative regulator of the type I interferon antiviral response, and that knockdown of PARP7 enhances nucleic acid sensor agonist or viral-induced interferon-beta (IFN-β) expression in different cell types. PARP7 acts as a repressor of the activity of the aryl hydrocarbon receptor (AHR), which is activated in response to viral infection or upregulated expression in cellular stress after exposure to cigarette smoke. In addition, PARP7 is also regulated by liver X receptors (LXRs), hypoxia-inducible factor 1 (HIF-1).

PARP7 is not highly expressed in normal cells and is overactive in a range of tumors. When cancer cells or viruses are infected, intracellular free nucleic acid fragments trigger innate immune perception through the TBK1-mediated type I IFN pathway by phosphorylating the cytoplasmic interferon regulator IRF-3, which then translocates to the nucleus and binds to transcriptional co-activators to promote the transcription of IFN-α and IFN-β, which in turn recruits immune cells such as dendritic cells, macrophages, and lymphocytes to recognize and kill cancer cells. However, highly expressed PARP7 acts as a brake in cancer cells, preventing the phosphorylation of IRF-3, and subsequently preventing the production of type I IFNs to evade recognition and pursuit by immune cells. Inhibition of PARP7 effectively suppresses cancer cell growth and restores interferon signaling, effectively activating innate and adaptive immunity.

PARP7 negatively regulates aryl hydrocarbon receptor (AHR) and type I interferon (IFN-I) signaling, both of which are associated with intestinal homeostasis and immunity. Since the absence of PARP7 expression increases AHR and IFN-I signaling pathways, the absence of PARP7 expression decreased the expression of inflammatory genes including IL-6, CXCL1, and lipocalin-2 in a mouse model of dextrose sodium sulfate (DSS)-induced colitis. The experimental results suggest that PARP7 may be involved in the recruitment of immune cells to the site of inflammation. Thus, inhibition of PARP7 reduced the severity of DSS-induced colitis in mice and suppressed the production of pro-inflammatory cytokines in tissues from patients with inflammatory bowel disease.

Relatively few PARP7 inhibitors have been reported, and the most advanced ones are still in early clinical development. RBN-2397, a trifluoropyridazinone compound developed by Ribon Therapeutics in the United States, inhibits the enzymatic activity of PARP7 and inhibits the growth and proliferation of a wide range of cancer cells, leading to tumor regression in a mouse xenograft tumor model and a syngeneic tumor model (Gozgit J. M. et al. Cancer Cell 2021, 39, 1-13). However, RBN-2397 has poor in vivo pharmacokinetic properties, such as low in vivo plasma exposure, short plasma half-life, and low oral bioavailability. Therefore, there is a need to develop PARP7 inhibitors that can improve in vivo pharmacokinetic properties, have better antitumor activity and therapeutic efficacy, reduce clinical side effects, and can be applied to more tumor indications.

### SUMMARY OF THE INVENTION

The invention aims to provide a compound capable of selectively inhibiting the enzymatic activity of PARP7.

In one aspect, there is provided a poly(adenosine diphosphate-ribose) polymerase-7 (PARP7) inhibitor comprising a compound of formula (I) or a pharmaceutically acceptable salt, deuterated compound, solvate, ester, acid, metabolite or prodrug thereof, 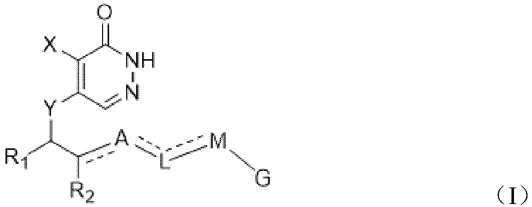 Wherein,
- - - represents a double or single bond, provided that at most one double bond is attached to either atom;
X is selected from the group consisting of halogen, C₁₋₆ haloalkyl, and C₂₋₆ alkanoyl, or forms a fused benzene ring or heterocycle together with Y and the carbon atoms to which they are attached;
Y is selected from the group consisting of NH and O, or forms a fused benzene ring or heterocycle together with X and the carbon atoms to which they are attached;
A is a nitrogen-oxo group selected from the group consisting of -N-O-, =N-O-, -O-N-, and -O-N=, and the N atom is optionally substituted with hydrogen, C₁₋₆ alkyl, C₂₋₆ alkanoyl, or C₁₋₆ sulfonyl when the valence thereof is not saturated;
L is selected from the group consisting of -(CH₂)ₙ-, 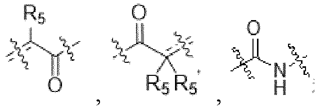 and 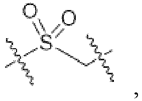 wherein n is 1, 2 or 3, R₅ and R₅' are each independently selected from the group consisting of hydrogen, C₁₋₃ alkyl, halogen, deuterium, and hydroxyl, or R₅ and R₅' together with the carbon atoms to which they are attached form C₃₋₆ cycloalkyl;
R₁ and R₂ are each independently selected from the group consisting of hydrogen, C₁₋₆ alkyl, C₁₋₆ hydroxyalkyl, C₁₋₆ haloalkyl, C₃₋₆ cycloalkyl, C₃₋₆ hydroxycycloalkyl, or C₃₋₆ halocycloalkyl, or R₁ and R₂ together with the carbon atoms to which they are attached form C₅₋₈ cycloalkyl;
M is a moiety selected from the group consisting of formulae (M-1), (M-2), (M-3), (M-4) and (M-5): 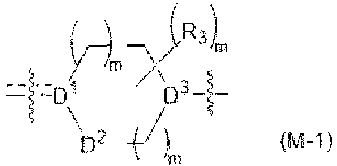 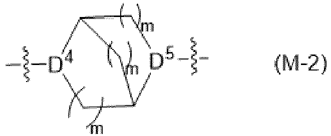 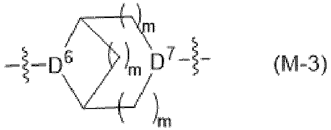 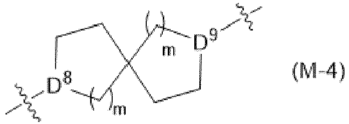 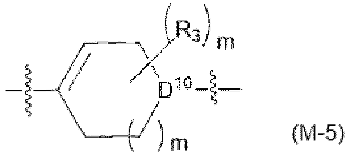
wherein D¹, D², D³, D⁴, D⁵, D⁶, D⁷, D⁸, D⁹, and D¹⁰ are each independently selected from the group consisting of N and CH, provided that at least one N is contained as the ring atom of the M moiety,
R₃ is each independently selected from the group consisting of hydrogen, halogen, hydroxyl, C₁₋₄ alkyl, C₁₋₆ haloalkyl, and C₁₋₆ hydroxyalkyl,
m is each independently selected from the group consisting of 0, 1 or 2;
G is a 5- to 6- membered aromatic heterocyclic group optionally substituted with one or two R₄ groups, wherein R₄ are each independently selected from the group consisting of hydroxyl, cyano, C₁₋₆ alkyl, C₁₋₆ hydroxyalkyl, C₁₋₆ haloalkyl, halogen, C₃₋₆ cycloalkyl, C₂₋₆ alkenyl, C₂₋₆ alkynyl, -NR₆R₆', -C(O)NR₆R₆', -NR₆C(O)R₇, -C(O)OR₆, -OR₆, -S(O)₂NR₆R₆', -NR₆S(O)₂R₇, -S(O)₂R₇, -C(O)R₆, and phenyl optionally substituted with halogen, or two R₄ groups together with the carbon atoms to which they are attached form C₅₋₈ cycloalkyl, phenyl, or heteroaryl optionally substituted with hydroxyl, methyl, halogen or oxo, wherein R₆, R₆' and R₇ are each independently selected from the group consisting of hydrogen, C₁₋₆ alkyl, C₁₋₆ haloalkyl, C₃₋₆ cycloalkyl, and C₁₋₆ hydroxyalkyl. In preferred embodiments, said heterocycle is a 5- to 8- membered monocyclic ring containing an N atom, preferably piperidine, pyrrolidine, piperazine, or tetrahydropyridine.

In some embodiments, wherein M is a moiety selected from the group consisting of formulae (M-1a), (M-2a), (M-3a), (M-4a) and (M-5a): 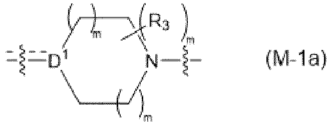 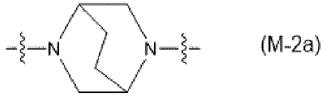 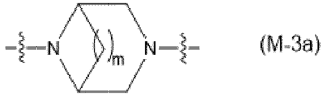 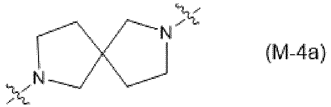 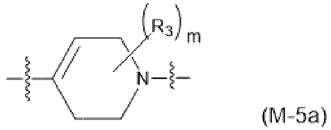 wherein D¹ is selected from the group consisting of N or CH, m is each independently selected from the group consisting of 0, 1 or 2, and R₃ is each independently selected from the group consisting of hydrogen, hydroxyl, halogen or C₁₋₄ alkyl.

In some embodiments, wherein G is a moiety selected from the group consisting of the formula (G-1), (G-2), (G-3), (G-4), (G-5), (G-6) and (G-7): 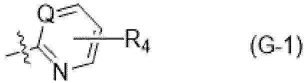 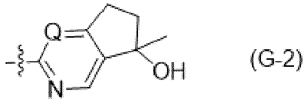 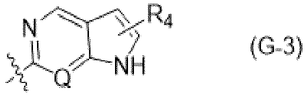 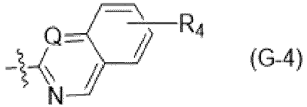 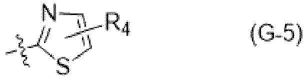 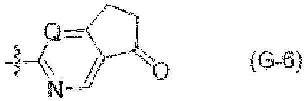 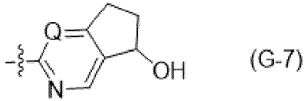 wherein Q is each independently selected from the group consisting of N or CH, and more preferably Q is N. R₄ is defined as previously.

Preferably, R₄ groups are optional substituents and are each independently selected from the group consisting of cyano, C₁₋₄ alkyl, C₁₋₄ hydroxyalkyl, C₁₋₄ haloalkyl, halogen, C₃₋₆ cycloalkyl, C₂₋₄ alkenyl, C₂₋₄ alkynyl, -NR₆R₆', -C(O)NR₆R₆', -C(O)OR₆, -OR₆, -C(O)R₆, and phenyl optionally substituted with halogen, wherein R₆ and R₆' are each independently selected from the group consisting of hydrogen and C₁₋₄ alkyl.

In preferred aspects, X is selected from the group consisting of bromine, trifluoromethyl, and acetyl, or X forms a fused benzene ring or a fused piperidine ring together with Y and the carbon atoms to which they are attached; Y is selected from NH or forms a fused benzene ring or a fused piperidine ring together with X and the carbon atoms to which they are attached.

In a further preferred embodiment, L is selected from the group consisting of -(CH₂)ₙ-, 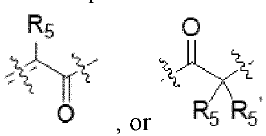 when D¹ in formula (M-1) or formula (M-1a) is N; and L is selected from the group consisting of -(CH₂)ₙ-, 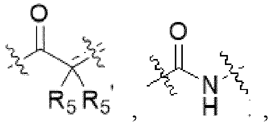 or 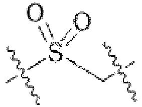 when D¹ is CH, and n, R₅, R₅' are defined as previously.

In some embodiments, L is 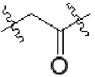 when A is -N-O-; L is 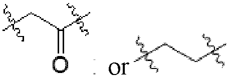 when A is =N-O-; L is 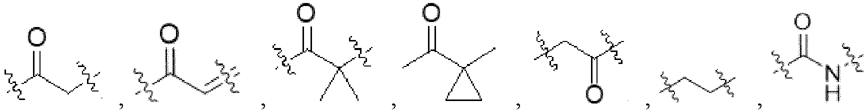 or 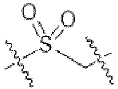 when A is -O-N-; and L is 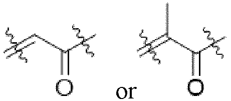 when A is -O-N=.

In a preferred aspect of the invention, there is provided a PARP7 inhibitor comprising a compound of formula (I) or a pharmaceutically acceptable salt, deuterated compound, solvate, ester, acid, metabolite or prodrug thereof, wherein, X is selected from the group consisting of C₁₋₆ haloalkyl and C₂₋₆ alkanoyl, and X is more preferably C₁₋₆ haloalkyl, in particular trifluoromethyl; Y is NH; A is a nitrogen-oxo group selected from the group consisting of =N-O-, -O-N- and -O-N=, more preferably -O-N-; L is selected from the group consisting of 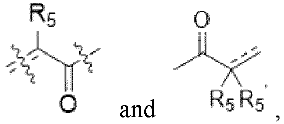 more preferably 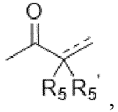 wherein R₅ and R₅' are each independently selected from the group consisting of hydrogen or halogen, more preferably hydrogen; M is a moiety selected from the group consisting of formulae (M-1a) and (M-5a), wherein D¹ is selected from the group consisting of N or CH, more preferably CH, and m is independently 1, and R₃ is each independently selected from the group consisting of hydrogen, halogen or C₁₋₃ alkyl, more preferably hydrogen; G is a moiety selected from the group consisting of the formulae (G-1), (G-3) and (G-4), more preferably formula (G-1), wherein Q is selected from the group consisting of N or CH, more preferably N, and R₄ is selected from the group consisting of C₁₋₆ haloalkyl, halogen, C₃₋₆ cycloalkyl, and phenyl optionally substituted with halogen, more preferably C₁₋₆ haloalkyl, in particular trifluoromethyl; R₁ and R₂ are each independently selected from the group consisting of hydrogen, C₁₋₆ alkyl, and C₃₋₆ cycloalkyl, or R₁ and R₂ together with the carbon atoms to which they are attached form C₅₋₈ cycloalkyl, and more preferably, R₁ and R₂ are each independently selected from the group consisting of hydrogen and C₁₋₆ alkyl.

In a further preferred aspect, the invention provides a PARP7 inhibitor comprising a compound of formula (I) or a pharmaceutically acceptable salt, deuterated compound, solvate, ester, acid, metabolite or prodrug thereof, wherein,
- - - represents a double or single bond, provided that at most one double bond is attached to either atom;
X is selected from the group consisting of halogen and C₁₋₆ haloalkyl, or forms a fused benzene ring or heterocycle together with Y and the carbon atoms to which they are attached;
Y is NH or forms a fused benzene ring or heterocycle together with X and the carbon atoms to which they are attached;
A is a nitrogen-oxo group selected from the group consisting of -N-O-, =N-O-, -O-N-, and -O-N=, and the N atom is optionally substituted with hydrogen, C₁₋₆ alkyl, C₂₋₆ alkanoyl, or C₁₋₆ sulfonyl when the valence thereof is not saturated;
L is selected from the group consisting of -(CH₂)ₙ-, 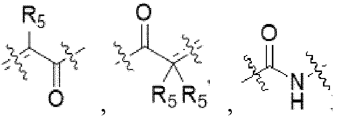 and 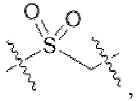 wherein n is 1, 2 or 3, and R₅ and R₅' are each independently selected from the group consisting of hydrogen or C₁₋₃ alkyl, or R₅ and R₅' together with the carbon atoms to which they are attached form C₃₋₆ cycloalkyl;
R₁ and R₂ are each independently selected from the group consisting of hydrogen, C₁₋₆ alkyl, C₁₋₆ hydroxyalkyl, C₁₋₆ haloalkyl, C₃₋₆ cycloalkyl, C₃₋₆ hydroxycycloalkyl, or C₃₋₆ halocycloalkyl, or R₁ and R₂ together with the carbon atoms to which they are attached form C₅₋₈ cycloalkyl:
M is a moiety selected from the group consisting of formulae (M-1), (M-2), (M-3) and (M-4): 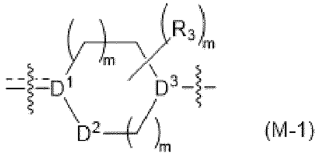 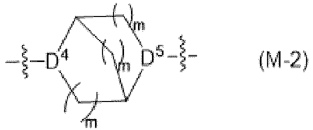 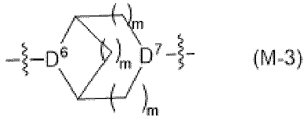 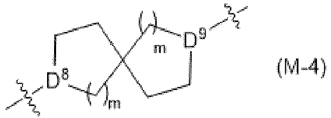
wherein D¹, D², D³, D⁴, D⁵, D⁶, D⁷, D⁸ and D⁹ are each independently selected from the group consisting of N and CH, provided that at least one N is contained as the ring atom of the M moiety.
R₃ is each independently selected from the group consisting of halogen, hydroxyl, C₁₋₄ alkyl and C₁₋₆ haloalkyl.
m is each independently selected from the group consisting of 0, 1 or 2;
G is a 5- to 6- membered aromatic heterocyclic group optionally substituted with one or two R₄ groups, wherein R₄ are each independently selected from the group consisting of hydroxyl, cyano, C₁₋₆ alkyl, C₁₋₆ hydroxyalkyl, and C₁₋₆ haloalkyl, or two R₄ groups together with the carbon atoms to which they are attached form C₅₋₈ cycloalkyl optionally substituted with hydroxyl, or methyl.

Further preferably, M is a moiety selected from the group consisting of formulae (M-1a), (M-2a), (M-3a) and (M-4a): 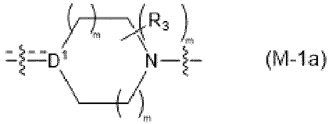 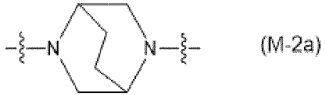 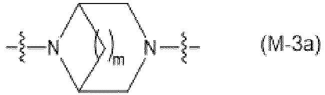 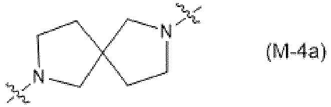 wherein D¹ is selected from the group consisting of N or CH, m is each independently selected from the group consisting of 0, 1 or 2, and R₃ is each independently selected from the group consisting of hydroxyl, halogen or C₁₋₄ alkyl.

Also further preferably, G is a moiety selected from the group consisting of the formulae (G-1) and (G-2): 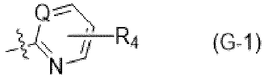 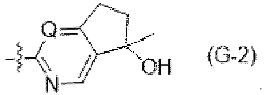 where Q is selected from the group consisting of N or CH.

Also particularly preferably, L is selected from the group consisting of -(CH₂)ₙ-, 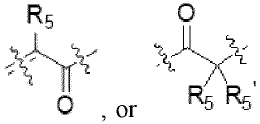 when D¹ in formula (M-1) or formula (M-1a) is N; and L is selected from the group consisting of -(CH₂)ₙ-, 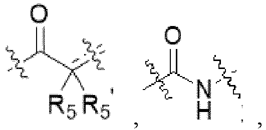 or 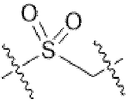 when D¹ is CH.

Also particularly preferably, L is 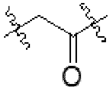 when A is -N-O-; L is 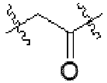 or 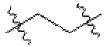 when A is =N-O-; L is 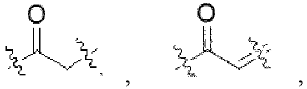 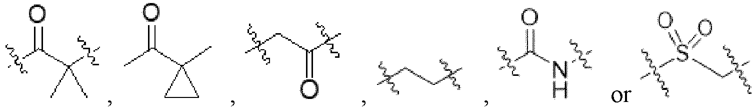 when A is -O-N-; and L is 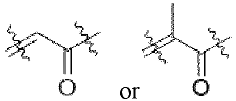 when A is -O-N=.

The invention also preferably provides a PARP7 inhibitor comprising a compound of formula (II) or a pharmaceutically acceptable salt, deuterated compound, solvate, ester, acid, metabolite or prodrug thereof. 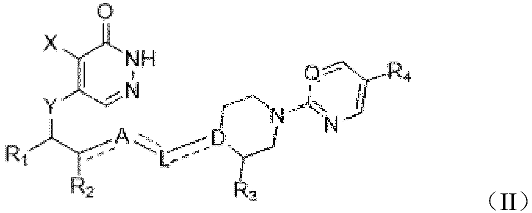
Wherein, represents a double or single bond, provided that at most one double bond is attached to either atom.
X is selected from the group consisting of halogen and C₁₋₆ haloalkyl, or forms a fused benzene ring or piperidine ring together with Y and the carbon atoms to which they are attached;
Y is selected from NH or forms a fused benzene ring or piperidine ring together with X and the carbon atoms to which they are attached;
A is a nitrogen-oxo group selected from the group consisting of -N-O-, =N-O-, -O-N-, and -O-N=, and the N atom is optionally substituted with hydrogen, C₁₋₆ alkyl, C₂₋₆ alkanoyl, or C₁₋₆ sulfonyl when the valence thereof is not saturated;
L is selected from the group consisting of -(CH₂)ₙ-, 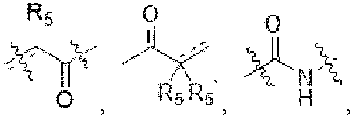 and 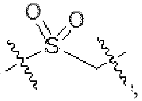 wherein n is 1, 2, or 3, and R₅ and R₅' are each independently selected from the group consisting of hydrogen or C₁₋₃ alkyl, or R₅ and R₅' together with the carbon atoms to which they are attached form C₃₋₆ cycloalkyl;
D is selected from the group consisting of N or CH;
Q is selected from the group consisting of N or CH;
R₁ and R₂ are each independently selected from the group consisting of hydrogen, C₁₋₆ alkyl, C₃₋₆ cycloalkyl, or R₁ and R₂ together with the carbon atoms to which they are attached form C₅₋₈ cycloalkyl;
R₃ is selected from the group consisting of hydrogen or C₁₋₃ alkyl;
R₄ is selected from the group consisting of hydroxyl, cyano, C₁₋₆ alkyl, C₁₋₆ hydroxyalkyl and C₁₋₆ haloalkyl.

In another aspect of the invention, there is provided a pharmaceutical composition comprising a compound of the invention, or a pharmaceutically acceptable salt, deuterated compound, solvate, ester, acid, metabolite or prodrug thereof and a pharmaceutically acceptable carrier or excipient, and optionally other therapeutic agents

Other aspects of the invention also relate to methods or uses of a compound of the invention, or a pharmaceutically acceptable salt, deuterated compound, solvate, ester, acid, metabolite, or prodrug thereof, to selectively inhibit the activity of PARP7, or relate to the use of a compound of the invention, or a pharmaceutically acceptable salt, deuterated compound, solvate, ester, acid, metabolite, or prodrug thereof, in the preparation of a drug for selectively inhibiting the activity of PARP7.

In further aspects of the invention there is also related to method or use of a compound of the invention, or a pharmaceutically acceptable salt, deuterated compound, solvent, ester, acid, metabolite, or prodrug thereof for treating or preventing a disease, disorder, or condition regulated by or affected by PARP7 activity or which involves PARP7 activity, or use of a compound of the invention, or a pharmaceutically acceptable salt, deuterated compound, solvent, ester, acid, metabolite, or prodrug thereof, in the preparation of a drug for treating or preventing a disease, disorder or condition regulated by or affected by PARP7 activity or which involves PARP7 activity or overexpression.

Preferably, a disease, disorder or condition regulated by or affected by PARP7 activity or which involves PARP7 activity or overexpression is an overproliferative disease, in particular a cancerous, autoimmune or inflammatory disease.

In more preferred aspects, said disease, disorder, or condition is selected from the group consisting of cancers including, but not limited to, squamous lung cancer, lung adenocarcinoma, non-small cell lung cancer, small cell lung cancer, head and neck squamous cell carcinoma, breast cancer, pancreatic cancer, pancreatic ductal adenocarcinoma, colorectal cancer, melanoma, ovarian cancer, esophageal squamous cell carcinoma, gastric cancer, liver cancer, oral cancer, urothelial cancer, prostate cancer, bladder cancer, renal cell carcinoma, gastrointestinal stromal tumor, cervical cancer, endometrial cancer, rhabdomyosarcoma, fibrosarcoma, neuroendocrine tumor, mesothelioma, brain cancer, or malignant glioma and the like.

In other preferred aspects, said disease, disorder or condition is selected from the group consisting of autoimmune or inflammatory disease including, but not limited to, ulcerative colitis, Crohn's disease, multiple sclerosis, autoimmune liver disease, type I diabetes mellitus, bronchial asthma, systemic lupus erythematosus, rheumatoid arthritis, ankylosing spondylitis, juvenile idiopathic arthritis, psoriasis, polymyositis, or dermatomyositis and the like.

### DETAILED DESCRIPTION OF THE PREFERRED EMBODIMENTS

### DEFINITION

Unless otherwise defined, all technical and scientific terms used herein have the meanings ordinarily understood by one of ordinary skill in the art to which the invention belongs. In the specification, the singular form also encompasses the plural form unless the context clearly represents otherwise. All publications, patent applications, patents or other references mentioned herein are incorporated herein by reference. In the event of conflict, this specification, including the definitions, prevails. Furthermore, the materials, methods and embodiments are illustrative only and are not intended to limit the scope of the invention.

Unless otherwise indicated, the invention employs conventional methods such as mass spectrometry, NMR, HPLC, protein chemistry, biochemistry, recombinant DNA technology, and pharmacology that are within the skill of the art. Unless specific definitions are provided, nomenclature and laboratory operations and techniques chemically related to the analytical chemistry, synthetic organic chemistry, and medical and pharmaceutical chemistry described herein are known to those skilled in the art. In general, the foregoing techniques and steps may be performed by conventional methods well known in the art and described in various general and more specific literature, which is cited and discussed in this specification.

The term "alkyl" refers to an aliphatic hydrocarbon group, which may be a branched or straight chain alkyl group. Depending on the structure, the alkyl group may be a monovalent group or a bivalent group (i.e. alkylene). For the purposes of the invention, the alkyl group is preferably an alkyl group having 1 to 8 carbon atoms, more preferably a "lower alkyl group" having 1 to 6 carbon atoms, and even more preferably an alkyl group having 1 to 4 carbon atoms. Exemplary alkyl groups include, but are not limited to, methyl, ethyl, propyl, butyl, pentyl, hexyl, and the like. It is to be understood that references herein to "alkyl" include all possible configurations and conformations of such alkyl groups, e.g., references herein to "propyl" include n-propyl and iso-propyl, "butyl" includes n-butyl, isobutyl, and tert-butyl, and "pentyl" includes n-pentyl, iso-pentyl, neo-pentyl, tert-pentyl, and pentan-3-yl, among others.

The term "alkoxy" refers to -O-alkyl, wherein alkyl is as defined herein. Exemplary alkoxy groups include, but are not limited to, methoxy, ethoxy, propoxy, butoxy, pentoxy, hexoxy, and the like

The term "alkoxyalkyl" means that an alkyl group as defined herein is substituted with an alkoxy group as defined herein.

The term "cycloalkyl" refers to a monocyclic or polycyclic group containing only carbon and hydrogen. Cycloalkyl groups include groups with 3 to 12 ring carbon atoms. Depending on the structure, the cycloalkyl group may be a monovalent group or a bivalent group (e.g., cycloalkylene). In the invention, the cycloalkyl group is preferably a cycloalkyl group having 3 to 8 carbon atoms, and more preferably a "lower cycloalkyl group" having 3 to 6 carbon atoms. Examples of cycloalkyl include, but are not limited to, cyclopropyl, cyclobutyl, cyclopentyl, cyclohexyl, cycloheptyl, cyclooctyl, cyclopentenyl, cyclohexenyl, cycloheptenyl, and adamantyl.

The term "alkyl (cycloalkyl)" or "cycloalkylalkyl" means that an alkyl group as defined herein is substituted with a cycloalkyl group as defined herein. Examples of cycloalkylalkyl groups include cyclopropylmethyl, cyclobutylmethyl, cyclopentylmethyl, cyclohexylmethyl, and the like.

The term "aromatic" refers to planar rings having an outlying π-electron system and containing 4n+2 π-electrons, where n is an integer. The aromatic ring may comprise five, six, seven, eight, nine or more than nine atoms. The aromatic group may be optionally substituted. The term "aromatic" includes carbocyclic aryl (e.g., phenyl) and heterocyclic aryl (or "heteroaryl" or "heteroaromatic") groups (e.g., pyridine). The term includes monocyclic or heterocyclic polycyclic (i.e., rings sharing adjacent pairs of carbon atoms) groups.

The term "aryl" as used herein refers to an aromatic ring in which each of the atoms comprising the ring is a carbon atom. The aryl ring may comprise five, six, seven, eight, nine or more than nine atoms. The aryl group may be optionally substituted. Examples of aryl groups include, but are not limited to, phenyl, naphthyl, phenanthryl, anthracenyl, fluorenyl, and indenyl. Depending on the structure, the aryl group may be a monovalent group or a bivalent group (i.e., arylidene).

The term "aryloxy" refers to -O-aryl, wherein aryl is as defined herein.

The term "heteroaryl" means that the aryl group includes one or more cyclic heteroatoms selected from the group consisting of nitrogen, oxygen and sulfur. The N-containing "heteroaryl" part means that at least one of the backbone atoms of the ring in the aromatic group is a nitrogen atom. Depending on the structure, the heteroaryl group may be a monovalent group or a bivalent group (i.e., a subheteroaryl group). Examples of heteroaryl groups include, but are not limited to, pyridinyl, imidazolyl, pyrimidinyl, pyrazolyl, triazolyl, pyrazinyl, tetrazolyl, furanyl, thienyl, isoxazolyl, thiazolyl, oxazolyl, isothiazolyl, pyrrolyl, quinolyl, isoquinolyl, indolyl, benzoimidazolyl, benzofuranyl, indazolyl, indazolyl, phthalazinyl, pyridazinyl, isoindolyl, pteridinyl, purinyl, oxadiazolyl, thiadiazolyl, furazanyl, benzofurazanyl, benzothienyl, benzothiazolyl, benzoxazolyl, quinazolinyl, naphthyridinyl, and furazopyridinyl, and the like.

The term "alkyl(aryl)" or "arylalkyl" means that an alkyl group as defined herein is substituted with an aryl group as defined herein. Examples of alkyl (aryl) includes benzyl, phenylethyl, and the like.

The term "alkyl (heteroaryl)" or "heteroarylalkyl" means that an alkyl group as defined herein is substituted with a heteroaryl group as defined herein_{∘}

The term "heteroalkyl" as used herein refers to an alkyl group as defined herein in which one or more backbone chain atoms are heteroatoms, such as oxygen, nitrogen, sulfur, silicon, phosphorus or combinations thereof. Said heteroatom (or atoms) may be located at any position within the heteroalkyl group or at a position where the heteroalkyl group is connected to the rest of the molecule.

The term "heterocycloalkyl" as used herein refers to a non-aromatic ring in which one or more of the atoms comprising the ring are heteroatoms selected from the group consisting of nitrogen, oxygen and sulfur. The heterocycloalkyl ring may be a monocyclic or polycyclic ring comprising three, four, five, six, seven, eight, nine or more atoms. The heterocycloalkyl ring may be optionally substituted. Examples of heterocycloalkyl include, but are not limited to, lactams, lactones, cyclic imines, cyclic thioimines, cyclic carbamates, tetrahydrothiopyrans, 4H-pyrans, tetrahydropyrans, piperidines, 1,3-dioxins, 1,3-dioxanes, 1,4-dioxans, 1,4-dioxanes, piperazines, 1,3-oxathiocyclohexanes, 1,4- oxathiocyclohexanes, 1,4-oxathiocyclohexanes, tetrahydro-1. 4-thiazine, 2H-1,2-oxazine, maleimide, succinimide, barbituric acid, thiobarbituric acid, dioxypiperazine, ethylenecarbazone, dihydrouracil, morpholine, trioxane, hexahydro-1,3,5-triazine, tetrahydrothiophene, tetrahydrofuran, pyrrolidine, pyrrolidine, imidazolidine, pyrrolidone, pyrazoline, pyrazolidine, imidazolidine, imidazolidine, 1,3-dioxahexene, 1 ,3-dioxolane, 1,3-dithiolane, 1,3-dithiolane, 1,3-dithiolane, isoxazoline, isoxazolidin, oxazoline, oxazolidin, oxazolidinone, thiazoline, thiazolidine, and 1,3-oxathiolane. Depending on the structure, the heterocycloalkyl group may be a monovalent group or a bivalent group (i.e., a subheterocycloalkyl group).

The term "alkyl (heterocycloalkyl)" or "heterocycloalkylalkyl" means that an alkyl group as defined herein is substituted with a heterocycloalkyl group as defined herein.

The term "alkoxy (heterocycloalkyl)" or "heterocycloalkylalkoxy" means that an alkoxy group as defined herein is substituted with a heterocycloalkyl group as defined herein.

The terms "halogen" or "halogens" mean fluorine, chlorine, bromine and iodine.

The terms "haloalkyl", "haloalkoxy" and "haloheteroalkyl" include alkyl, alkoxy or heteroalkyl structures in which at least one hydrogen is replaced by a halogen atom. In some embodiments, if two or more hydrogen atoms are replaced by halogen atoms, said halogen atoms are identical or different from each other.

The term "hydroxyl" refers to the -OH group.

The term "cyano" refers to the -CN group.

The term "ester group" means a chemical part having the formula -COOR, wherein R is selected from the group consisting of alkyl, cycloalkyl, aryl, heteroaryl (linked by a cyclic carbon) and heterocyclic (linked by a cyclic carbon).

The term "amino" refers to the -NH₂ group.

The term "carbamoyl" refers to the -CO-NH₂ group.

The term "alkylcarbamoyl" refers to a -CO-NH-R group, wherein R is an alkyl group as defined herein.

The term "amide" or "acylamino" refers to -NR-CO-R', wherein R and R' are each independently hydrogen or alkyl_{∘}

The term "alkylamino" refers to an amino substituent further substituted with one or two alkyl groups, specifically the group -NRR', wherein R and R' are each independently selected from the group consisting of hydrogen or a lower alkyl group, provided that -NRR' is not -NH. "Alkylamino" includes groups of compounds in which the nitrogen of -NH is attached to at least one alkyl group. Examples of alkylamino groups include, but are not limited to, methylamino, ethylamino, and the like. "Dialkylamino" includes a moiety in which the nitrogen of -NH₂ is attached to at least two other alkyl groups. Examples of dialkylamino groups include, but are not limited to, dimethylamino, diethylamino, and the like.

The terms "arylamino" and "diarylamino" refer to amino substituents further substituted with one or two aryl groups, specifically the group -NRR', wherein R and R' are each independently selected from the group consisting of hydrogen, lower alkyl, or aryl, wherein N is attached to at least one or two aryl groups, respectively.

The term "cycloalkylamino" means an amino substituent further substituted with one or two cycloalkyl groups as defined herein.

The term "heteroalkylamino" means an amino substituent further substituted with one or two heteroalkyl groups as defined herein.

The term "arylalkylamino" herein refers to a group-NRR' wherein R is a lower arylalkyl group and R' is hydrogen, lower alkyl, aryl or lower arylalkyl.

The term "heteroarylamino" means an amino substituent further substituted with one or two heteroaryl groups as defined herein.

The term "heterocycloalkylamino" means that an amino group as defined herein is substituted with a heterocycloalkyl group as defined herein.

The term "alkylaminoalkyl" means that an alkyl group as defined herein is substituted with an alkylamino group as defined herein.

The term "aminoalkyl" means an alkyl substituent further substituted with one or more amino groups.

The term "aminoalkoxy" means an alkoxy substituent further substituted with one or more amino groups.

The terms "hydroxyalkyl" or "hydroxyalkyl" refer to alkyl substituents further substituted with one or more hydroxyl groups.

The term "cyanoalkyl" means an alkyl substituent further substituted with one or more cyano groups.

The term "alkanoyl" refers to the monovalent group of atoms remaining after the hydroxyl group has been removed from an organic or inorganic oxygen-containing acid, and has the general formula R-M(O)-, wherein M is usually C.

The term "carbonyl" is an organic functional group consisting of two atoms, carbon and oxygen, linked by a double bond (C=O).

The term "alkanoyl" or "alkyl carbonyl" refers to a carbonyl group that is further substituted with an alkyl group. Exemplary alkanoyl groups include, but are not limited to, acetyl, propanoyl, butanoyl, pentanoyl, hexanoyl, and the like.

The term "aryl carbonyl" means that the carbonyl group as defined herein is substituted with an aryl group as defined herein.

The term "alkoxycarbonyl" means a carbonyl group further substituted with an alkoxy group.

The term "heterocycloalkyl carbonyl" means a carbonyl group further substituted with a heterocycloalkyl group.

The terms "alkylaminocarbonyl", "cycloalkylaminocarbonyl", "arylaminocarbonyl", "arylalkylaminocarbonyl", "cycloalkylaminocarbonyl", "arylaminocarbonyl", "arylalkylaminocarbonyl", "heteroarylaminocarbonyl" mean that the carbonyl group as defined herein is substituted with an alkylamino group, a cycloalkylamino group, an arylamino group, an arylalkylamino group, or a heteroarylamino group as defined herein.

The term "alkylcarbonylalkyl" or "alkanoylalkyl" refers to an alkyl group further substituted with an alkylcarbonyl group.

The term "alkylcarbonylalkoxy" or "alkanoylalkoxy" refers to an alkoxy group further substituted with an alkylcarbonyl group.

The term "heterocycloalkylcarbonylalkyl" means an alkyl group further substituted with a heterocycloalkylcarbonyl group.

The term "sulfhydryl" refers to the -SH group. The term "alkylthio" means that a mercapto group as defined herein is substituted with an alkyl group as defined herein.

The terms "sulfone" or "sulfonyl" refer to the functional group of the sulfonic acid after the loss of the hydroxyl group, specifically the -S(=O)₂ - group.

The terms "sulfinyl" or "sulfinyl" refer to -S(=O)-.

The term "aminosulfonyl" or "aminosulfonyl" refers to the -S(=O)₂ -NH₂ group.

The terms "alkylsulfinyl" or "alkylsulfinyl" refer to alkyl-S(=O)-.

The term "alkyl sulfone" or "alkyl sulfonyl" refers to -S(=O)₂ -R, wherein R is alkyl.

The term "alkylamino sulfone" means that a sulfone group as defined herein is substituted with an alkylamino group as defined herein.

The terms "alkylsulfonylamino" or "alkylsulfonylamino" and "cycloalkylsulfonylamino" or "cycloalkylsulfonylamino " means that the amino group as defined herein is substituted with an alkyl sulfonyl or cycloalkyl sulfonyl group as defined herein, i.e., -NH-S(=O)₂ -R, wherein R is alkyl and cycloalkyl, respectively.

The terms "cycloalkyl sulfone" and "cycloalkyl sulfonyl" refer to -S(=O)₂-R, wherein R is cycloalkyl.

The term "quaternary ammonium group" means -N⁺ RR'R", wherein R, R' and R" are each independently selected from the group consisting of an alkyl group having 1 to 8 carbon atoms.

The term "optionally" means that one or more of the events described later may or may not occur and includes both events that occur and events that do not occur. The term "optionally substituted" or "substituted" means that the group referred to may be substituted with one or more additional groups, each of which is individually and independently selected from the group consisting of an alkyl group, a cycloalkyl group, an aryl group, a heteroaryl group, a heterocycloalkyl group, hydroxyl, alkoxy, cyano, halogen, amide, nitro, haloalkyl, amino, methylsulfonyl, alkylcarbonyl, alkoxycarbonyl, heteroarylalkyl, heterocycloalkylalkyl, aminocarbonyl, aminoprotecting group, and the like. Among them, the amino protecting group is preferably selected from the group consisting of pivaloyl, tert-butoxycarbonyl, benzyloxycarbonyl, 9-fluorenylmethoxycarbonyl, benzyl, p-methoxybenzyl, allyloxycarbonyl, and trifluoroacetyl, and the like.

The term "pharmaceutically acceptable salt" herein refers to a salt that retains the desired biological activity of the subject compound and shows minimal undesired toxicological effects. These pharmaceutically acceptable salts may be prepared in situ during the final isolation and purification of the compounds, or by reacting the free acid or free base form of the purified compound alone with a suitable base or acid, respectively.

"Solvent" or "solvent compound" refers to solvent addition forms containing stoichiometric or non-stoichiometric solvents. Some compounds tend to trap a fixed molar proportion of solvent molecules in a crystalline solid state, resulting in the formation of solvent compounds. If the solvent is water, the solvent compounds formed are hydrates; if the solvent is an alcohol, the solvent compounds formed are alcoholates. The hydrate is formed by binding one or more water molecules to one molecule of said substance, wherein said water retains its molecular state as H₂O.

A "metabolite" of a compound disclosed herein is a derivative of the compound formed when the compound is metabolized. The term "active metabolite" refers to a biologically active derivative of the compound formed when the compound is metabolized. The term "metabolized", as used herein, refers to the sum of the processes by which a particular substance is altered by an organism (including, but not limited to, hydrolysis and enzyme-catalyzed reactions, such as oxidation). Thus, enzymes can produce specific structural transformations into compounds. For example, cytochrome P450 catalyzes a variety of oxidation and reduction reactions, while diphosphoglucuronosyltransferase catalyzes the conversion of activated glucuronic acid molecules to aromatic alcohols, aliphatic alcohols, carboxylic acids, amines and free sulfhydryl groups. Further information on metabolism may be obtained from The Pharmacological Basis of Therapeutics, Ninth Edition, McGraw-Hill (1996). Metabolites of the compounds disclosed herein can be identified by giving the compound to a host and analyzing tissue samples from that host, or by incubating the compound with hepatocytes in vitro and analyzing the resulting compound. Both methods are known in the art. In some embodiments, the metabolite of the compound is formed by an oxidative process and corresponds to a corresponding hydroxyl-containing compound. In some embodiments, the compound is metabolized to a pharmaceutically active metabolite.

The term "modulating" as used herein refers to interacting directly or indirectly with a target to alter the activity of the target, including, by way of example only, enhancing the activity of the target, inhibiting the activity of the target, limiting the activity of the target, or prolonging the activity of the target.

The term "prodrug" or "precursor drug" refers to derivatives which may not be pharmacologically active but which, in some cases, may be given orally or parenterally and thereafter metabolized in vivo to form a pharmacologically active compound of the invention. Non-limiting examples of prodrug include: esters, carbonates, sesquiterpenes, phosphate esters, nitro esters, sulfates, sulfoxides, amides, carbamates, nitrogen-containing compounds, phosphoramidites, glycosides, ethers, acetals and ketone acetals.

"Effective amount" means the amount of a drug or pharmacological agent that will elicit a biological or medical response in, for example, a tissue, system, animal or human being under study by an investigator or practitioner. In addition, the term "therapeutically effective amount" means any amount that causes the treatment, cure, prevention, or alleviation of a disease, disorder, or side effect modification, or a reduction in the rate of development of a disease or disorder, as compared to the corresponding subject who did not receive that amount. The term also includes an amount that is effective in improving normal physiological function.

The term "treatment" as used herein refers to the relief of at least one symptom of a disease, disorder or condition. The term includes administering to a subject and/or applying one or more of the compounds described herein to provide management or treatment of a condition. "Treatment" for the purposes of this disclosure may, but need not, provide a cure; rather, "treatment" may be in the form of management of a condition. When the compounds described herein are used to treat harmful proliferating cells, including cancer, "treatment" includes partial or complete destruction of said harmful proliferating cells with minimal impact on the destruction of normal cells. The desired mechanism of treatment of harmful rapidly proliferating cells, including cancer cells, is apoptosis at the cellular level.

The term "prevention" as used herein includes the co-prevention or mitigation of the onset of clinically significant disease progression or the onset of preclinically significant disease stages in individuals at risk. This includes the preventive treatment of individuals at risk of disease development.

The term "subject" or "patient" includes an organism capable of suffering from a condition or a condition associated with reduced or insufficient programmed cell death (apoptosis) or otherwise benefiting from the administration of a compound of the invention, such as humans and non-human animals. Preferred humans include human patients suffering from or predisposed to suffer from a disease or related condition as described herein. The term "non-human animal" includes vertebrates, such as mammals, e.g., non-human primates, sheep, cattle, dogs, cats, and rodents, such as mice, as well as non-mammals, such as chickens, amphibians, reptiles, and the like.

As used herein, GI₅₀ refers to the concentration of drug required to cause 50% cell growth inhibition, i.e., the concentration of drug when the drug causes the growth of 50% of the cells (e.g., cancer cells) to be inhibited or controlled.

As used herein, IC₅₀ refers to the amount, concentration, or dose of a particular test compound that achieves a 50% inhibition of the maximum effect in an analysis that measures the effect.

As used herein, EC₅₀ refers to a dose, concentration, or amount of an assay compound that elicits a dose-dependent response of 50% of the maximum expression of a particular response induced, stimulated, or enhanced by a particular assay compound.

### PARP7 inhibitor of the invention

The invention relates to a PARP7 inhibitor comprising a compound of formula (I) or a pharmaceutically acceptable salt, deuterated compound, solvate, ester, acid, metabolite or prodrug thereof, 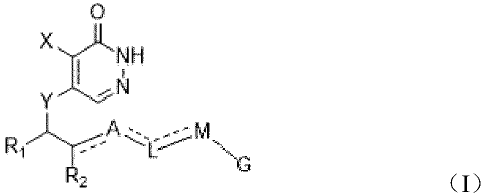 Wherein,
- - - represents a double or single bond, provided that at most one double bond is attached to either atom;
X is selected from the group consisting of halogen, C₁₋₆ haloalkyl, and C₂₋₆ alkanoyl, or forms a fused benzene ring or heterocycle together with Y and the carbon atoms to which they are attached;
Y is selected from the group consisting of NH and O, or forms a fused benzene ring or heterocycle together with X and the carbon atoms to which they are attached;
A is a nitrogen-oxo group selected from the group consisting of -N-O-, =N-O-, -O-N-, and -O-N=, and the N atom is optionally substituted with hydrogen, C₁₋₆ alkyl, C₂₋₆ alkanoyl, or C₁₋₆ sulfonyl when the valence thereof is not saturated;
L is selected from the group consisting of -(CH₂)ₙ-, 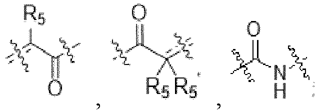 and 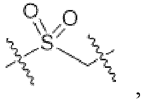 wherein n is 1, 2 or 3, R₅ and R₅' are each independently selected from the group consisting of hydrogen, C₁₋₃ alkyl, halogen, deuterium, and hydroxyl, or R₅ and R₅' together with the carbon atoms to which they are attached form C₃₋₆ cycloalkyl;
R₁ and R₂ are each independently selected from the group consisting of hydrogen, C₁₋₆ alkyl, C₁₋₆ hydroxyalkyl, C₁₋₆ haloalkyl, C₃₋₆ cycloalkyl, C₃₋₆ hydroxycycloalkyl, or C₃₋₆ halocycloalkyl, or R₁ and R₂ together with the carbon atoms to which they are attached form C₅₋₈ cycloalkyl;
M is a moiety selected from the group consisting of formulae (M-1), (M-2), (M-3), (M-4) and (M-5): 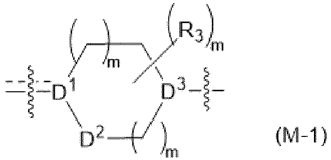 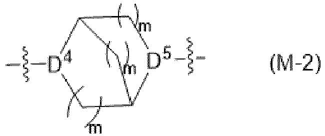 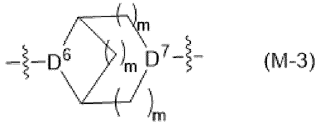 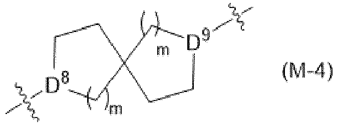 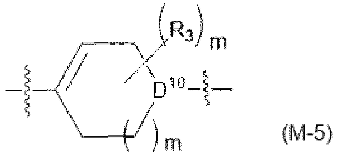
wherein D¹, D², D³, D⁴, D⁵, D⁶, D⁷, D⁸, D⁹, and D¹⁰ are each independently selected from the group consisting of N and CH, provided that at least one N is contained as the ring atom of the M moiety,
R₃ is each independently selected from the group consisting of hydrogen, halogen, hydroxyl, C₁₋₄ alkyl, C₁₋₆ haloalkyl, and C₁₋₆ hydroxyalkyl,
m is each independently selected from the group consisting of 0, 1 or 2;
G is a 5- to 6- membered aromatic heterocyclic group optionally substituted with one or two R₄ groups, wherein R₄ are each independently selected from the group consisting of hydroxyl, cyano, C₁₋₆ alkyl, C₁₋₆ hydroxyalkyl, C₁₋₆ haloalkyl, halogen, C₃₋₆ cycloalkyl, C₂₋₆ alkenyl, C₂₋₆ alkynyl, -NR₆R₆', -C(O)NR₆R₆', -NR₆C(O)R₇, -C(O)OR₆, -OR₆, -S(O)₂NR₆R₆', -NR₆S(O)₂R₇, -S(O)₂R₇, -C(O)R₆, and phenyl optionally substituted with halogen, or two R₄ groups together with the carbon atoms to which they are attached form C₅₋₈ cycloalkyl, phenyl, or heteroaryl optionally substituted with hydroxyl, methyl, halogen or oxo, wherein R₆, R₆' and R₇ are each independently selected from the group consisting of hydrogen, C₁₋₆ alkyl, C₁₋₆ haloalkyl, C₃₋₆ cycloalkyl, and C₁₋₆ hydroxyalkyl.
In preferred embodiments, said heterocycle is a 5- to 8- membered monocyclic ring containing an N atom, preferably piperidine, pyrrolidine, piperazine, or tetrahydropyridine.

In some embodiments, wherein M is a moiety selected from the group consisting of formulae (M-1a), (M-2a), (M-3a), (M-4a) and (M-5a): 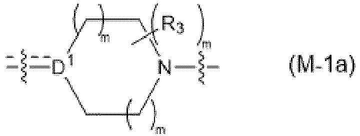 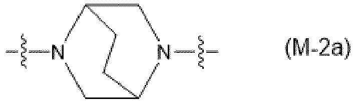 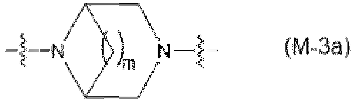 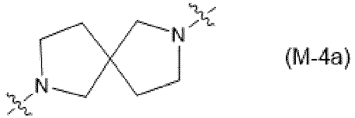 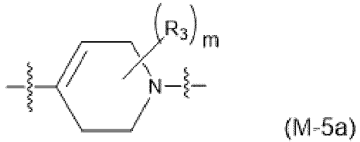 wherein D¹ is selected from the group consisting of N or CH, m is each independently selected from the group consisting of 0, 1 or 2, and R₃ is each independently selected from the group consisting of hydrogen, hydroxyl, halogen or C₁₋₄ alkyl.

In some embodiments, wherein G is a moiety selected from the group consisting of the formula (G-1), (G-2), (G-3), (G-4), (G-5), (G-6) and (G-7): 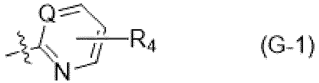 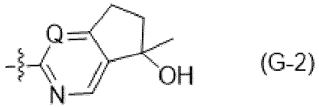 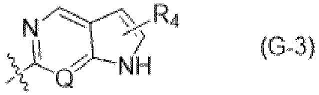 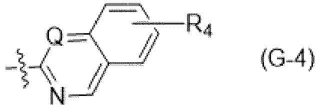 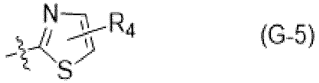 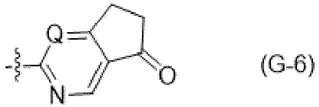 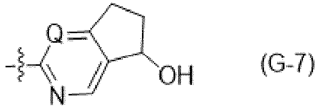 wherein Q is each independently selected from the group consisting of N or CH, and more preferably Q is N. R₄ is defined as previously.

In the description of the present specification, the substitution position of the optional substituent R₄ is not restricted and can be attached at any unsaturated carbon of the heteroaryl portion represented by G. For example, when the heteroaryl portion represented by G forms a fused bicyclic ring (e.g., Formulas G-3, G-4 above), the representations herein do not restrict the position of the R₄ substituent to the attached monocyclic ring, but rather the R₄ substitution can be made at any unsaturated carbon of either ring of the fused bicyclic ring.

Preferably, R₄ groups are optional substituents and are each independently selected from the group consisting of cyano, C₁₋₄ alkyl, C₁₋₄ hydroxyalkyl, C₁₋₄ haloalkyl, halogen, C₃₋₆ cycloalkyl, C₂₋₄ alkenyl, C₂₋₄ alkynyl, -NR₆R₆', -C(O)NR₆R₆', -C(O)OR₆, -OR₆, -C(O)R₆, and phenyl optionally substituted with halogen, wherein R₆ and R₆' are each independently selected from the group consisting of hydrogen and C₁₋₄ alkyl.

In preferred aspects, X is selected from the group consisting of bromine, trifluoromethyl, and acetyl, or X forms a fused benzene ring or a fused piperidine ring together with Y and the carbon atoms to which they are attached; Y is selected from NH or forms a fused benzene ring or a fused piperidine ring together with X and the carbon atoms to which they are attached.

In a further preferred embodiment, L is selected from the group consisting of -(CH₂)ₙ-, 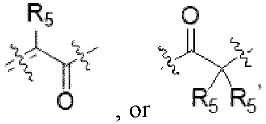 when D¹ in formula (M-1) or formula (M-1a) is N; and L is selected from the group consisting of -(CH₂)ₙ-, 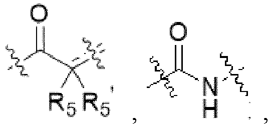 or 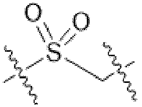 when D¹ is CH, and n, R₅, R₅' are defined as previously.

In some embodiments, L is 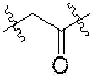 when A is -N-O-; L is 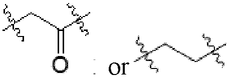 when A is =N-O-; L is 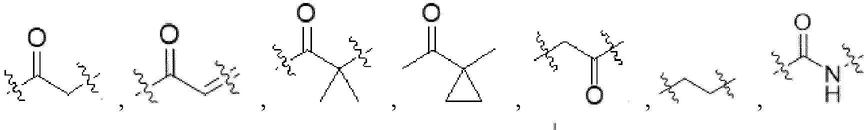 or 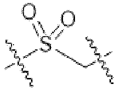 when A is -O-N-; and L is 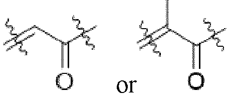 when A is -O-N=.

In a preferred aspect of the invention, there is provided a PARP7 inhibitor comprising a compound of formula (I) or a pharmaceutically acceptable salt, deuterated compound, solvate, ester, acid, metabolite or prodrug thereof, wherein, X is selected from the group consisting of C₁₋₆ haloalkyl and C₂₋₆ alkanoyl, and X is more preferably C₁₋₆ haloalkyl, in particular trifluoromethyl; Y is NH; A is a nitrogen-oxo group selected from the group consisting of =N-O-, -O-N- and -O-N=, more preferably -O-N-; L is selected from the group consisting of 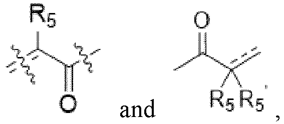 more preferably 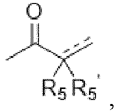 wherein R₅ and R₅' are each independently selected from the group consisting of hydrogen or halogen, more preferably hydrogen; M is a moiety selected from the group consisting of formulae (M-1a) and (M-5a), wherein D¹ is selected from the group consisting of N or CH, more preferably CH, and m is independently 1, and R₃ is each independently selected from the group consisting of hydrogen, halogen or C₁₋₃ alkyl, more preferably hydrogen; G is a moiety selected from the group consisting of the formulae (G-1), (G-3) and (G-4), more preferably formula (G-1), wherein Q is selected from the group consisting of N or CH, more preferably N, and R₄ is selected from the group consisting of C₁₋₆ haloalkyl, halogen, C₃₋₆ cycloalkyl, and phenyl optionally substituted with halogen, more preferably C₁₋₆ haloalkyl, in particular trifluoromethyl; R₁ and R₂ are each independently selected from the group consisting of hydrogen, C₁₋₆ alkyl, and C₃₋₆ cycloalkyl, or R₁ and R₂ together with the carbon atoms to which they are attached form C₅₋₈ cycloalkyl, and more preferably, R₁ and R₂ are each independently selected from the group consisting of hydrogen and C₁₋₆ alkyl.

In a further preferred aspect, the invention provides a PARP7 inhibitor comprising a compound of formula (I) or a pharmaceutically acceptable salt, deuterated compound, solvate, ester, acid, metabolite or prodrug thereof, wherein,
- - - represents a double or single bond, provided that at most one double bond is attached to either atom;
X is selected from the group consisting of halogen and C₁₋₆ haloalkyl, or forms a fused benzene ring or heterocycle together with Y and the carbon atoms to which they are attached;
Y is NH or forms a fused benzene ring or heterocycle together with X and the carbon atoms to which they are attached;
A is a nitrogen-oxo group selected from the group consisting of -N-O-, =N-O-, -O-N-, and -O-N=, and the N atom is optionally substituted with hydrogen, C₁₋₆ alkyl, C₂₋₆ alkanoyl, or C₁₋₆ sulfonyl when the valence thereof is not saturated;
L is selected from the group consisting of -(CH₂)ₙ-, 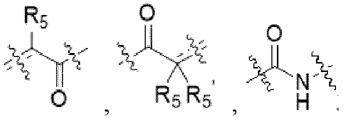 and 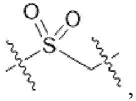 wherein n is 1, 2 or 3, and R₅ and R₅' are each independently selected from the group consisting of hydrogen or C₁₋₃ alkyl, or R₅ and R₅' together with the carbon atoms to which they are attached form C₃₋₆ cycloalkyl;
R₁ and R₂ are each independently selected from the group consisting of hydrogen, C₁₋₆ alkyl, C₁₋₆ hydroxyalkyl, C₁₋₆ haloalkyl, C₃₋₆ cycloalkyl, C₃₋₆ hydroxycycloalkyl, or C₃₋₆ halocycloalkyl, or R₁ and R₂ together with the carbon atoms to which they are attached form C₅₋₈ cycloalkyl:
M is a moiety selected from the group consisting of formulae (M-1), (M-2), (M-3) and (M-4): 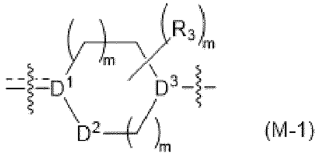 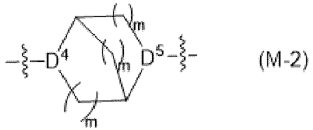 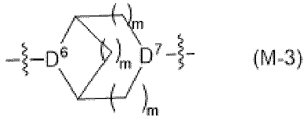 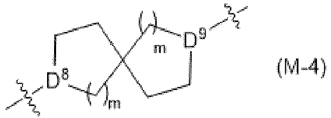
wherein D¹, D², D³, D⁴, D⁵, D⁶, D⁷, D⁸ and D⁹ are each independently selected from the group consisting of N and CH, provided that at least one N is contained as the ring atom of the M moiety,
R₃ is each independently selected from the group consisting of halogen, hydroxyl, C₁₋₄ alkyl and C₁₋₆ haloalkyl,
m is each independently selected from the group consisting of 0, 1 or 2;
G is a 5- to 6- membered aromatic heterocyclic group optionally substituted with one or two R₄ groups, wherein R₄ are each independently selected from the group consisting of hydroxyl, cyano, C₁₋₆ alkyl, C₁₋₆ hydroxyalkyl, and C₁₋₆ haloalkyl, or two R₄ groups together with the carbon atoms to which they are attached form C₅₋₈ cycloalkyl optionally substituted with hydroxyl, or methyl.

Further preferably, M is a moiety selected from the group consisting of formulae (M-1a), (M-2a), (M-3a) and (M-4a): 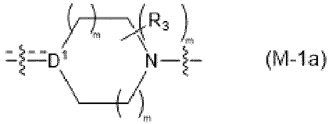 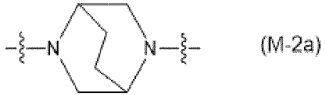 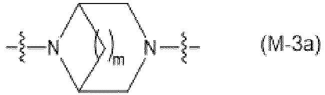 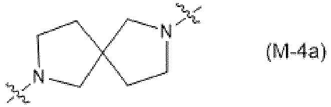 wherein D¹ is selected from the group consisting of N or CH, m is each independently selected from the group consisting of 0, 1 or 2, and R₃ is each independently selected from the group consisting of hydroxyl, halogen or C₁₋₄ alkyl.

Also further preferably, G is a moiety selected from the group consisting of the formulae (G-1) and (G-2): 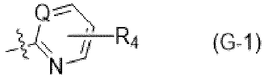 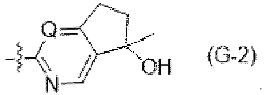 where Q is selected from the group consisting of N or CH.

Also particularly preferably, L is selected from the group consisting of -(CH₂)ₙ-, 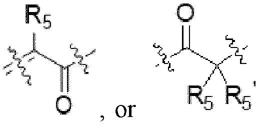 when D¹ in formula (M-1) or formula (M-1a) is N; and L is selected from the group consisting of -(CH₂)ₙ-, 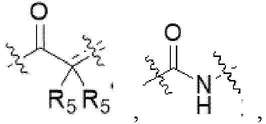 or 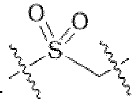 when D¹ is CH.

Also particularly preferably, L is 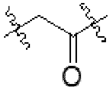 when A is -N-O-; L is 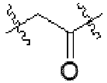 or 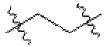 when A is =N-O-; L is 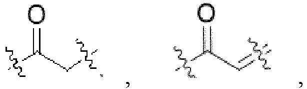 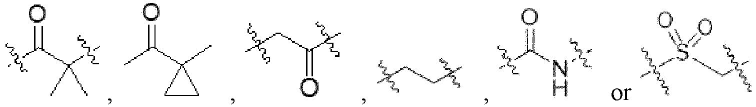 when A is -O-N-; and L is 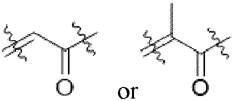 when A is -O-N=

The invention also preferably provides a PARP7 inhibitor comprising a compound of formula (II) or a pharmaceutically acceptable salt, deuterated compound, solvate, ester, acid, metabolite or prodrug thereof. 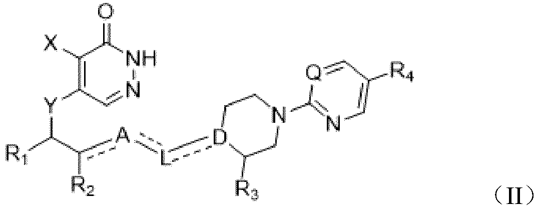
Wherein, represents a double or single bond, provided that at most one double bond is attached to either atom.
X is selected from the group consisting of halogen and C₁₋₆ haloalkyl, or forms a fused benzene ring or piperidine ring together with Y and the carbon atoms to which they are attached;
Y is selected from NH or forms a fused benzene ring or piperidine ring together with X and the carbon atoms to which they are attached;
A is a nitrogen-oxo group selected from the group consisting of -N-O-, =N-O-, -O-N-, and -O-N=, and the N atom is optionally substituted with hydrogen, C₁₋₆ alkyl, C₂₋₆ alkanoyl, or C₁₋₆ sulfonyl when the valence thereof is not saturated;
L is selected from the group consisting of -(CH₂)ₙ-, 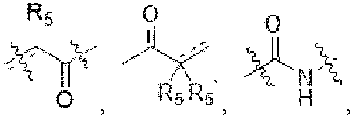 and 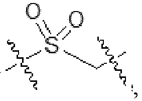 wherein n is 1, 2, or 3, and R₅ and R₅' are each independently selected from the group consisting of hydrogen or C₁₋₃ alkyl, or R₅ and R₅' together with the carbon atoms to which they are attached form C₃₋₆ cycloalkyl;
D is selected from the group consisting of N or CH;
Q is selected from the group consisting of N or CH;
R₁ and R₂ are each independently selected from the group consisting of hydrogen, C₁₋₆ alkyl, C₃₋₆ cycloalkyl, or R₁ and R₂ together with the carbon atoms to which they are attached form C₅₋₈ cycloalkyl;
R₃ is selected from the group consisting of hydrogen or C₁₋₃ alkyl;
R₄ is selected from the group consisting of hydroxyl, cyano, C₁₋₆ alkyl, C₁₋₆ hydroxyalkyl and C₁₋₆ haloalkyl.

Further preferably, the invention relates to the compounds, or a pharmaceutically acceptable salt, deuterated compound, solvate, ester, acid, metabolite or prodrug thereof, as shown in the following table.

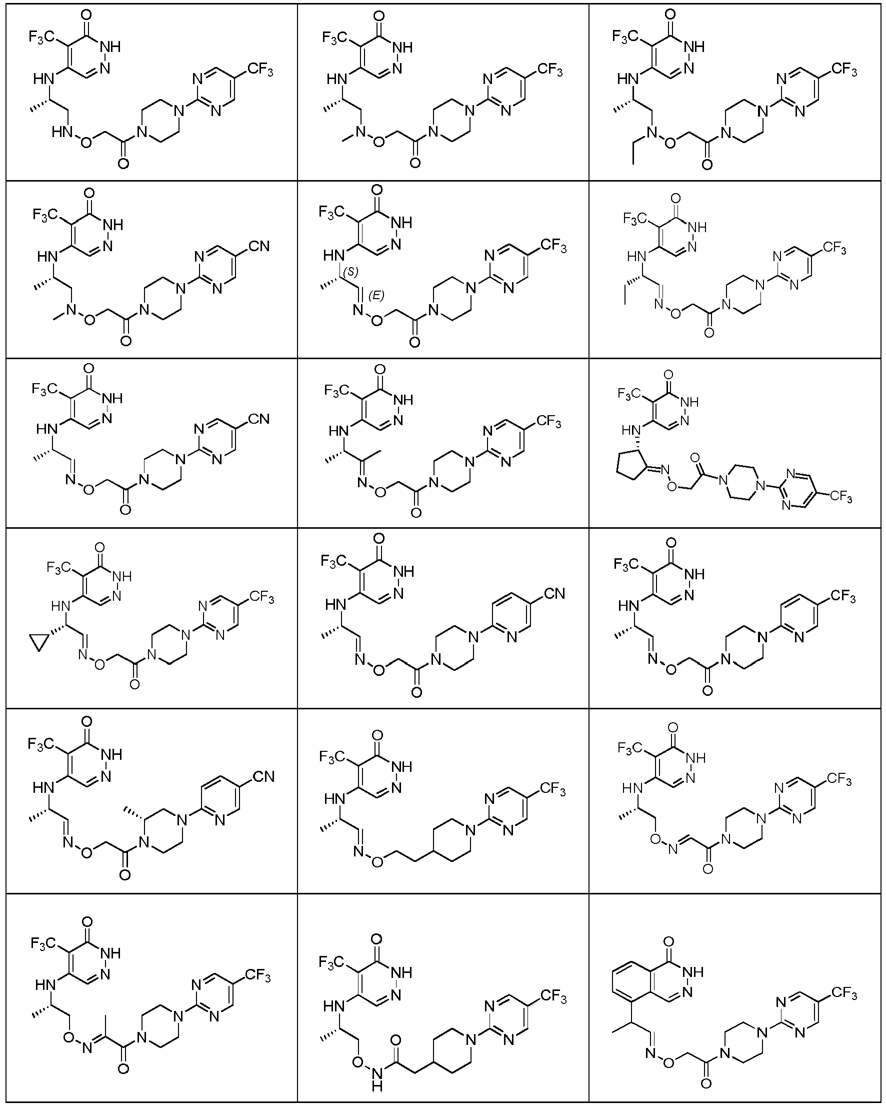

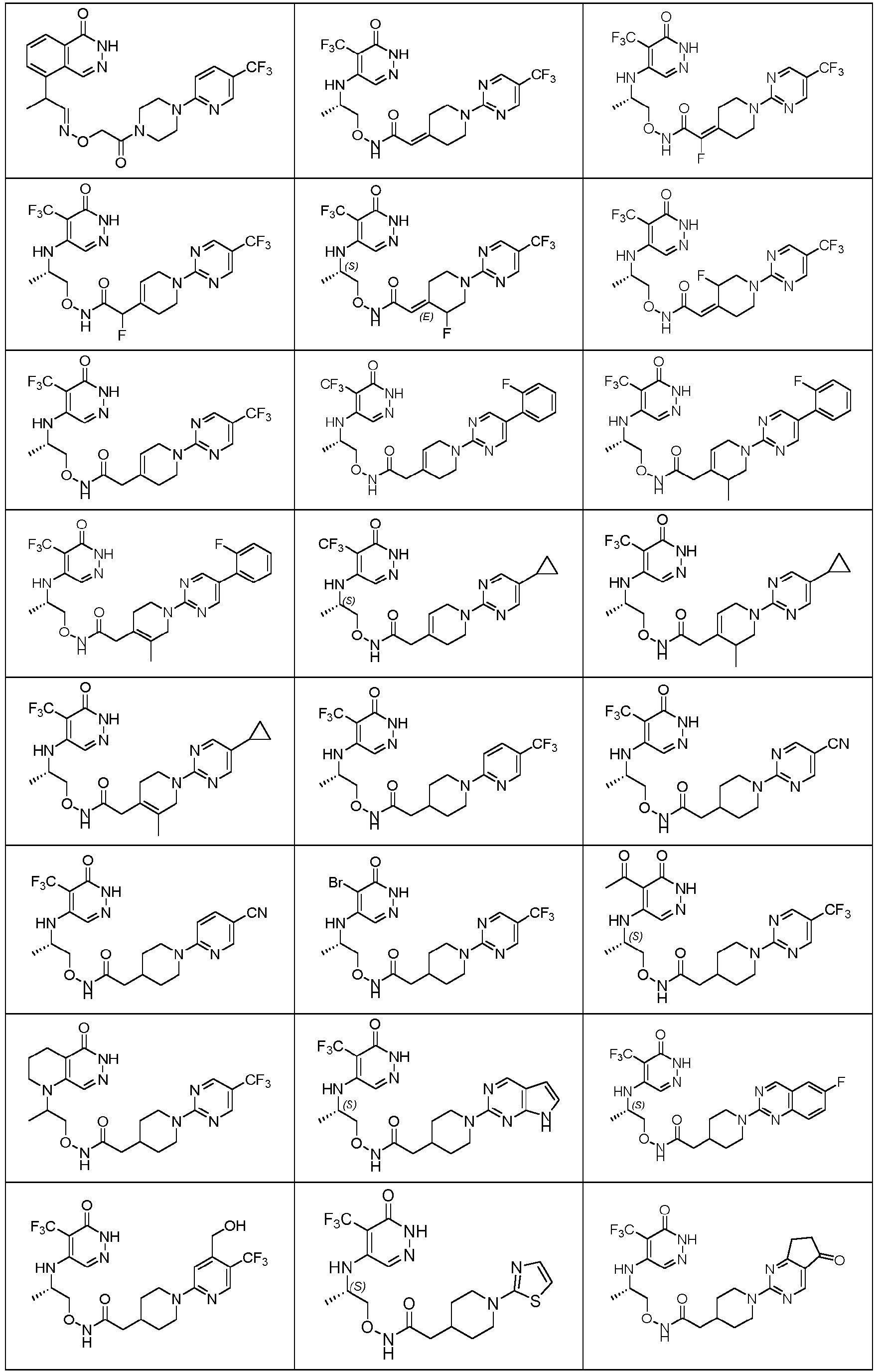

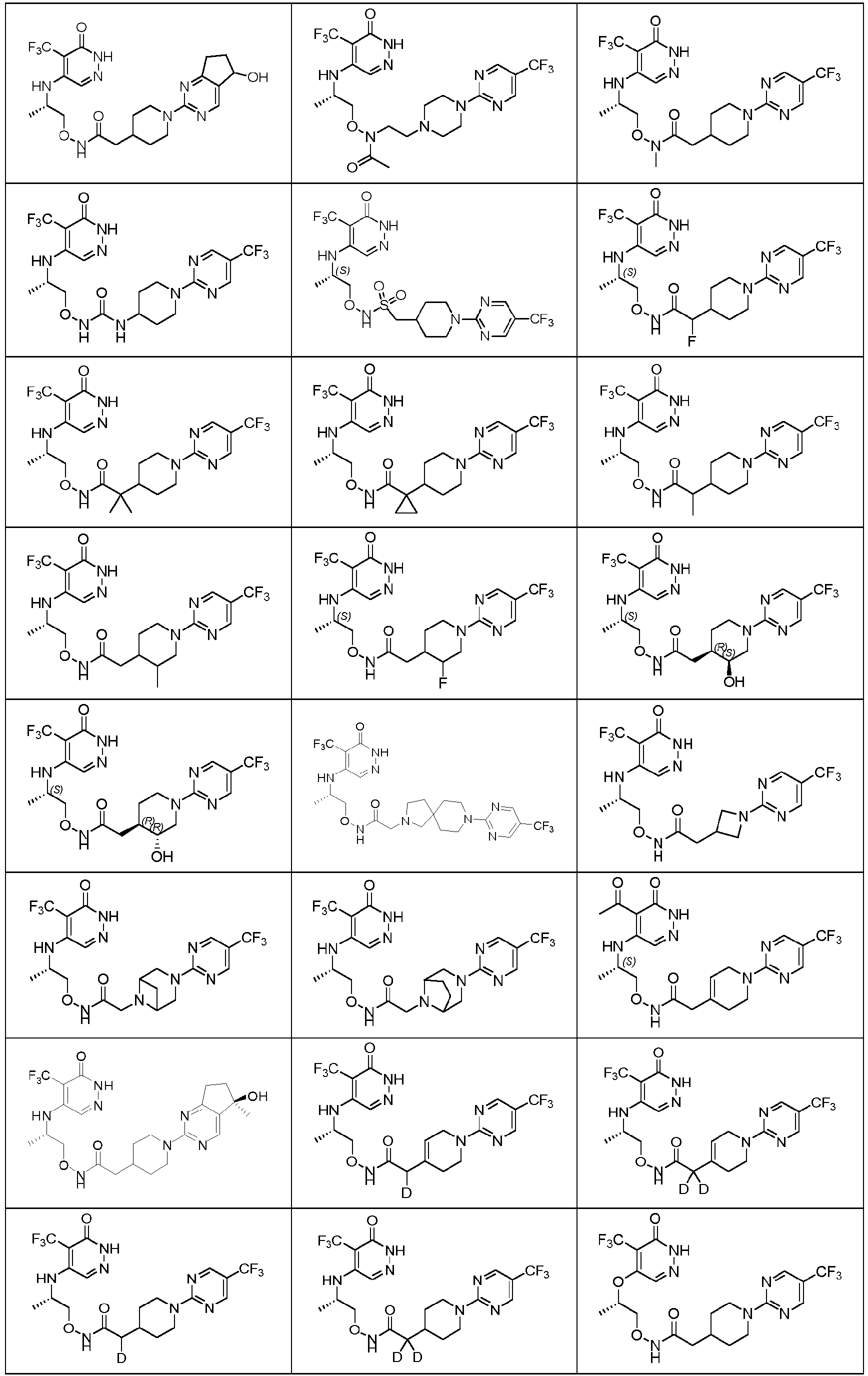

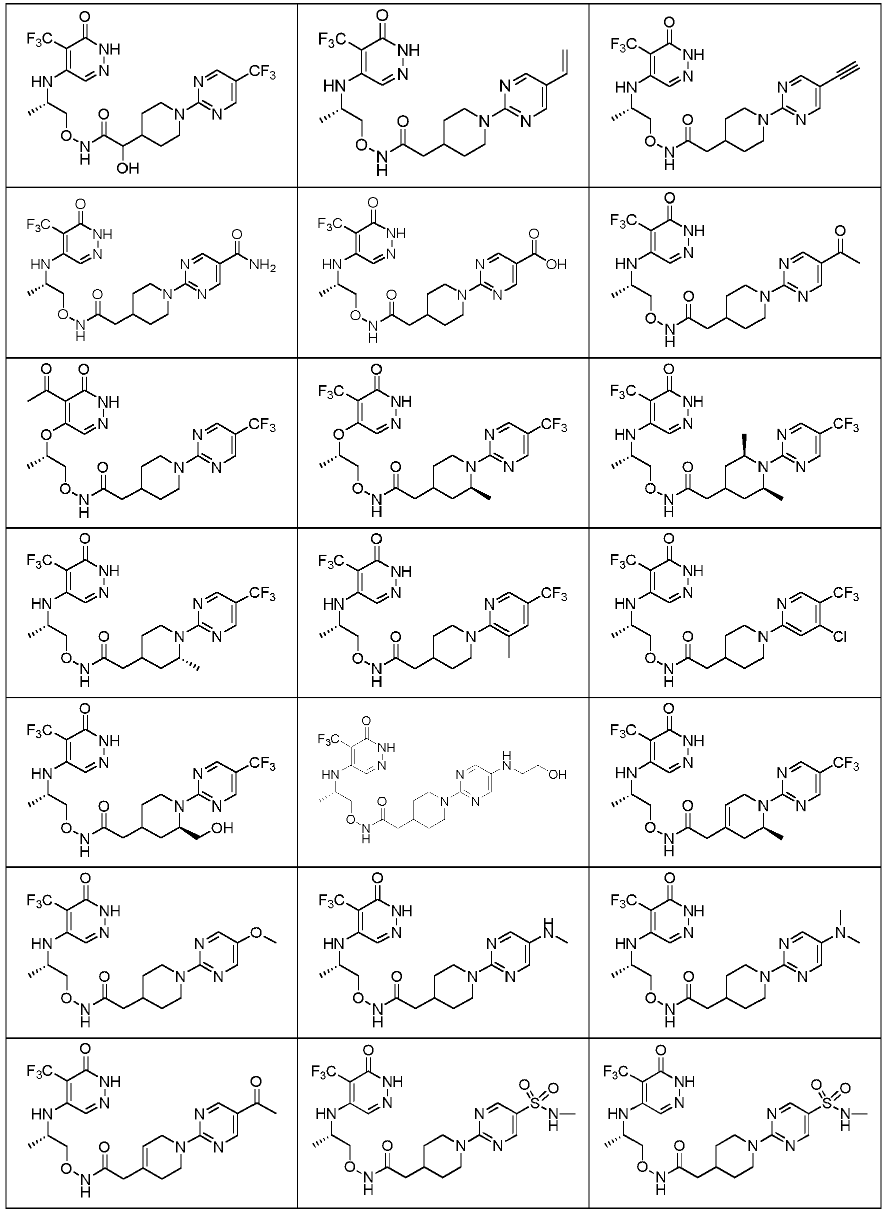

Described herein are novel PARP7 inhibitors. Also described herein a pharmaceutically acceptable salt, deuterated compound, solvate, ester, acid, metabolite or prodrug of this compound.

The compounds of the invention may be present in free form, for example in the form of free bases or free acids or amphoteric ions, or may be present in the form of salts. Said salt may be any salt, which may be an organic or inorganic salt, in particular any physiologically acceptable organic or inorganic salt commonly used in pharmacology.

Preferred salt for the purposes of the invention is a physiologically acceptable salt of the compound of the invention. However, salt itself is not suitable for pharmacological applications but which can be used, for example, for the isolation or purification of the compounds of the invention are also included.

The term "pharmaceutically acceptable salt" refers to a relatively non-toxic inorganic or organic acid addition salt of a compound of the invention, see for example, S. M. Berge et al. "Pharmaceutical Salts, J. Pharm. Sci. 1977, 66, 1-19".

Pharmaceutically acceptable salts of the compounds of the invention include acid addition salts of inorganic, carboxylic and sulfonic acids, such as the salts of the following acids: hydrochloric, hydrobromic, hydriodic, sulfuric, pyrosulfuric, phosphoric, and nitric acids; or salts of the compounds of the invention with organic acids, such as formic, acetic, acetoacetic, pyruvic, trifluoroacetic, propionic, butyric, caproic, heptanoic, undecylenic, lauric, benzoic, salicylic, 2-(4-hydroxy benzoyl)-benzoic acid, camphoric acid, cinnamic acid, cyclopentane propionic acid, digluconic acid, 3-hydroxy-2-naphthalenecarboxylic acid, nicotinic acid, paraffinic acid, pectinate acid, persulfuric acid, 3-phenylpropionic acid, picric acid, tertiary valeric acid, 2-hydroxyethanesulfonic acid, itaconic acid, sulphamate acid, trifluoromethanesulfonic acid, dodecyl sulfuric acid, ethylsulfonic acid, phenylsulfonic acid, p-toluene sulfonic acid, methylsulfonic acid, 2-naphthalene sulfonic acid, naphthalene disulfonic acid, Camphorsulfonic acid, citric acid, tartaric acid, stearic acid, lactic acid, oxalic acid, malonic acid, succinic acid, malic acid, adipic acid, alginic acid, maleic acid, fumaric acid, D-gluconic acid, mandelic acid, ascorbic acid, glucoheptanoic acid, glycerophosphoric acid, aspartic acid, sulphosalicylic acid, hemisulphuric acid, or salts of thiocyanic acid.

Pharmaceutically acceptable salts of the compounds of the invention also include salts of commonly used bases, such as and preferably alkali metal salts (e.g., sodium and potassium salts), alkaline earth metal salts (e.g., calcium and magnesium salts), and ammonium salts derived from ammonia or from organic amines having 1 to 16 carbon atoms, said organic amines being, for example and preferably, ethylamine, diethylamine, triethylamine, ethyl diisopropylamine, monoethanolamine, diethanolamine, triethanolamine, dicyclohexylamine , dimethylaminoethanol, procaine, dibenzylamine, N-methylmorpholine, arginine, lysine, ethylenediamine, N-methylpiperidine, N-methylglucosamine, dimethylglucosamine, ethylglucosamine, 1 ,6-hexanediamine, aminoglucose, sarcosine, serotonin, tris(hydroxymethyl)aminomethane, aminopropylene glycol, sovakine, and 1 -amino-2 ,3 ,4-butanetriol.

The invention includes all possible salts of the compounds of the invention, which may be a single salt or any mixture of said salts in any proportion.

The invention includes all possible deuterated compounds of the invention, where the deuterium atom may be arbitrarily substituted on a carbon or nitrogen atom.

Solvates are the term used for the purposes of the invention for those forms of the compounds of the invention which form complexes with solvent molecules by coordination in the solid or liquid state. Hydrates are those forms of particular solvates in which coordination with water occurs. Hydrates are preferred as solvates within the scope of the invention.

In addition, the invention includes prodrugs of the compounds of the invention. The term "prodrug" includes compounds which may be biologically active or inert in themselves, but which are converted (e.g., by metabolism or hydrolysis) to the compounds of the invention during their retention time in the body.

In addition, the invention includes all possible crystalline forms or polymorphs of the compounds of the invention as individual polymorphs, or as mixtures of more than one polymorph in any proportion.

In this specification, in some cases for convenience, the structural formula of a compound represents a specific isomer, but the invention includes all isomers, such as geometric isomers, optical isomers based on asymmetric carbon atoms, stereoisomers, reciprocal isomers, and the like.

The compounds covered by the invention bearing chirality can be in any configuration or mixed racemates. When the compounds used according to the invention contain more than one chiral center, they can exist in diastereomeric form. Said diastereomeric compounds can be separated by methods known to those skilled in the art (e.g., chromatography or crystallization), while the enantiomers alone can be separated as described above. The invention encompasses the application of diastereomeric compounds and mixtures thereof used according to the invention. The compounds used in the invention may be in different isomeric forms or in different geometrical isomers, and the invention includes applications of individual isomers and/or geometrical isomers of the compounds used according to the invention and mixtures thereof. The compounds used according to the invention can exist in amphoteric form. The invention includes applications of the individual amphoteric forms of the compounds used according to the invention and mixtures thereof.

Screening and characterization of pharmaceutically acceptable salts, polymorphs and/or solvates can be accomplished using a variety of techniques, said techniques including, but not limited to, thermal analysis, X-ray diffraction, spectroscopy, microscopic methods, elemental analysis. Various spectroscopic techniques used include, but are not limited to, Raman, FTIR, UVIS, and NMR (liquid and solid state). Various microscopy techniques include, but are not limited to, IR microscopy and Raman microscopy.

Accordingly, the invention includes all possible salts, polymorphs, metabolites, hydrates, deuterated compounds, solvates, or prodrugs (e.g., esters) of the compounds of the invention, or as individual salts, polymorphs, metabolites, hydrates, deuterated compounds, solvates, or prodrugs or as more than one salt, polymorph, metabolite, hydrate, deuterated compound, solvate, prodrug in any mixture in any proportions.

### PHARMACEUTICAL USES OF THE INVENTION

The compound of formula (I) or formula (II) of the invention, or pharmaceutically acceptable salt, deuterated compound, solvate, ester, acid, metabolite, or prodrug thereof, is capable of selectively inhibiting the enzymatic activity of PARP7, and is therefore capable of being used for treating or preventing a disease, disorder or condition regulated by or affected by PARP7 activity or which involves PARP7 activity or overexpression.

In preferred aspects, a disease, disorder or condition regulated by or affected by PARP7 activity or which involves PARP7 activity or overexpression is an overproliferative disease, in particular a cancerous, including, but not limited to, squamous lung cancer, lung adenocarcinoma, non-small cell lung cancer, small cell lung cancer, head and neck squamous cell carcinoma, breast cancer, pancreatic cancer, pancreatic ductal adenocarcinoma, colorectal cancer, melanoma, ovarian cancer, esophageal squamous cell carcinoma, gastric cancer, liver cancer, oral cancer, urothelial cancer, prostate cancer, bladder cancer, renal cell carcinoma, gastrointestinal stromal tumor, cervical cancer, endometrial cancer, rhabdomyosarcoma, fibrosarcoma, neuroendocrine tumor, mesothelioma, brain cancer, or malignant glioma and the like

In other preferred aspects, a disease, disorder or condition regulated by or affected by PARP7 activity or which involves PARP7 activity or overexpression is an autoimmune or inflammatory disease including, but not limited to, ulcerative colitis, Crohn's disease, multiple sclerosis, autoimmune liver disease, type I diabetes mellitus, bronchial asthma, systemic lupus erythematosus, rheumatoid arthritis, ankylosing spondylitis, juvenile idiopathic arthritis, psoriasis, polymyositis, or dermatomyositis and the like.

The compound of the invention may act systemically and/or locally. To this end, they may be administered in a suitable manner, for example by an oral route, a parenteral route, a pulmonary route, a nasal route, a sublingual route, a lingual route, an intranasal route, a rectal route, a dermal route, a transdermal route, a conjunctival route, or an auricular route, or in the form of an implant or scaffold.

Preferably, in embodiments of the invention, the drug comprising a compound of the invention may be administered to a patient by at least one of injection, oral, inhalation, rectal and transdermal administration.

Regardless of the chosen route of administration, the PARP7 inhibitors of the invention and/or the pharmaceutical compositions of the invention are formulated into pharmaceutically acceptable dosage forms by conventional methods known to those skilled in the art.

When treating a patient according to the invention, the amount of drug administered depends on numerous factors, such as the specific dosing regimen, the type of disease or condition and its severity, and the uniqueness of the treated person or host to be treated (e.g., body weight), but, depending on the particular surrounding circumstances, including, for example, the specific drug that has been employed, the route of administration, the condition to be treated, and the treated person or host to be treated, the dose to be administered may be routinely determined by methods known in the art. Typically, with respect to doses for therapeutic use in adults, the administered dose is typically in the range of 0.02-5000 mg/day, such as about 1-1500 mg/day. This desired dose may conveniently be expressed as a single dose, or as divided doses administered concurrently (or over a short period of time) or at appropriate intervals, such as two, three, four or more divided doses per day. It will be appreciated by one of skill in the art that, although the above range of doses is given, the specific effective amount may be appropriately adjusted according to the patient's condition and in conjunction with the physician's diagnosis.

The actual dosage level and time course of administration of the compounds of the invention can be varied so as to obtain an amount of the active ingredient that is effective in achieving the desired therapeutic response in a particular patient and is non-toxic to said patient.

### PHARMACEUTICAL COMPOSITIONS

Another aspect of the invention relates to pharmaceutical compositions comprising a compound of formula (I) or formula (II) of the invention, or pharmaceutically acceptable salt, deuterated compound, solvate, ester, acid, metabolite, or prodrug thereof, and a pharmaceutically acceptable diluent, carrier or excipient, and optionally one or more other therapeutic agents.

The compound of the invention may be administered in the form of a separate pharmaceutical agent or in combination with one or more other therapeutic agents, wherein said combination does not provoke unacceptable side effects. The pharmaceutical compositions include administration of a single pharmaceutical dosage formulation comprising a compound of the invention and one or more other therapeutic agents, as well as administration of a compound of the invention and various other therapeutic agents in the form of their own separate pharmaceutical dosage formulations. For example, a compound of formula (I) or formula (II) may be administered to a patient along with other therapeutic agents in the form of a single oral dosage composition, for example, in the form of a tablet or capsule, or each agent may be administered in its own separate dosage formulation.

When used in separate dosage formulations, the compound of the invention and one or more other therapeutic agents may be administered at substantially the same time (e.g., concurrently) or at separately staggered times (e.g., sequentially).

In particular, the compound of the invention may be used in fixed combinations or separate combinations with: other antitumor agent, such as alkylating agent, anti-metabolism agents, plant-derived antitumor agent, hormone therapeutic, topoisomerase inhibitor, camptothecin derivative, kinase inhibitor, targeted agent, antibody, interferon and/or biologic response modifier, anti-angiogenic compound, and other antitumor agent.

The compound of the invention may also be used in cancer treatment together with radiotherapy and/or surgical intervention.

### PREPARATION OF COMPOUND

The compounds of the invention can be synthesized using standard synthetic techniques known to those skilled in the art or using a combination of methods known in the art with the methods described herein. In addition, the solvents, temperatures and other reaction conditions given herein can be varied according to the art in the field. As a further guide, the following synthetic methods may also be utilized.

Said reactions may be used sequentially to provide the compounds described herein; or they may be used to synthesize fragments, said fragments being subsequently incorporated by the methods described herein and/or methods known in the art.

Starting materials for the synthesis of the compounds described herein can be synthesized or can be obtained from commercial sources. The compounds described herein and other related compounds having different substituents can be synthesized using techniques and raw materials known to those skilled in the art. General methods for preparing the compounds disclosed herein can be derived from reactions known in the art, and the reactions can be modified by reagents and conditions deemed appropriate by one of skill in the art to introduce various portions of the molecules provided herein.

If desired, the reaction products can be separated and purified using conventional techniques including, but not limited to, filtration, distillation, crystallization, chromatography, and other methods. These products can be characterized using conventional methods, including physical constants and graphical data.

Non-limiting embodiments of synthetic schemes for the preparation of compounds of formula (I) or formula (II) are described below.

### EXAMPLES

The following specific non-limiting embodiments will be construed as merely illustrative and do not limit the invention in any way. While no further detailed description is necessary, it is believed that the ordinarily skilled in the art can fully utilize the present disclosure based on the description herein.

The structure of the compound was determined by Nuclear Magnetic Resonance (NMR) or/and mass spectrometry (MS). The solvents for NMR determinations were deuterated dimethyl sulfoxide (DMSO-d⁶), deuterated chloroform (CDCl₃) or deuterated methanol (CD₃OD).

Where not otherwise specified in the embodiments, solutions are aqueous solutions.

The temperature of the reaction in the embodiments is room temperature, e.g., 20°C to 30°C, if not otherwise specified.

The monitoring of the reaction process in the embodiments was carried out by thin layer chromatography (TLC) and LCMS, and the systems of the unfolding agent used for the reaction, the eluent used for column chromatography of the purified compounds, and the unfolding agent for the thin layer chromatography included: A: dichloromethane/methanol system, B: n-Hexane/ethyl acetate system, and C: petroleum ether/ethyl acetate system. The volume ratio of the solvent is adjusted according to the polarity of the compound, and can also be adjusted by adding small amounts of basic or acidic reagents such as triethylamine and acetic acid.

### Example 1:

### (S)-5-((1-((2-oxo-2-(4-(5-(trifluoromethyl)pyrimidin-2-yl)piperazin-1-yl)ethoxy)amino) propan-2-yl)amino)-4-(trifluoromethyl)pyridazin-3(2H)-one

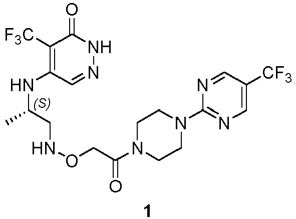

### 1.1) Synthesis of intermediates

### Step A: synthesis of 4,5-dibromo-2-((2-(trimethylsilyl)ethoxy)methyl)pyridazin-3(2H) -one

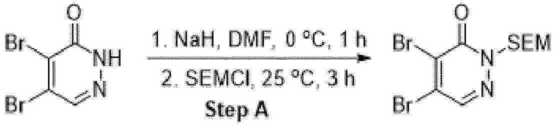

To a solution of 4,5-dibromopyridazin-3(2H)-one (10.00 g, 39.39 mmol, 1.0 eq.) in DMF (84 mL) was added NaH (60%, 1.89 g, 47.27 mmol, 1.2 eq.) in portions at 0 °C in ice-water bath under nitrogen. The resulting mixture was stirred for 1 h at 0 °C and followed by dropwise addition of 2-(Trimethylsilyl)ethoxymethyl chloride (7.22 g, 43.33 mmol, 1.1 eq.) at 0 °C. The reaction mixture was stirred for another 3 h at 25 °C. The mixture was quenched carefully by addition of water (400 mL) and the mixture was extracted with EtOAc (3 x 400 mL). The organic layers were combined and washed with brine (400 mL), dried over Na₂SO₄ and concentrated under vacuum. The crude was purified by silica gel chromatography (Petroleum ether/EtOAc= 20/1; V/V) to afford 4,5-dibromo-2-((2-(trimethylsilyl)ethoxy)methyl)pyridazin-3(2H)-one (7.45 g, 49.2% yield) as a yellow oil.
LCMS: *(ESI)* (M+H-28)⁺ = 356.6.
¹H NMR: (400 MHz, CDCl₃) *δ* 7.85 (s, 1H), 5.52 (s, 2H), 3.80 - 3.71 (m, 2H), 1.03 - 0.95 (m, 2H), 0.03 (s, 9H).

### Step B: synthesis of 4-bromo-5-chloro-2-((2-(trimethylsilyl)ethoxy)methyl)pyridazin-3 (2H)-one

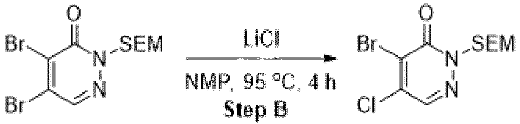

To a solution of 4,5-dibromo-2-((2-(trimethylsilyl)ethoxy)methyl)pyridazin-3(2H) -one (7.45 g, 19.39 mmol, 1.0 eq.) in NMP (15 mL) was added LiCl (814 mg, 19.39 mmol, 1.0 eq.). The mixture was stirred at 95 °C for 4 h. The mixture was cooled to room temperature and diluted with water (100 mL). The mixture was extracted with EtOAc (3 x 80 mL). The organic layers were combined and washed with brine (80 mL), dried over Na₂SO₄ and concentrated under vacuum. The residue was purified by column chromatography (Petroleum ether/EtOAc = 20/1; V/V) to afford 4-bromo-5-chloro -2-((2-(trimethylsilyl)ethoxy)methyl)pyridazin-3(2H)-one (5.45 g, 82.7% yield) as a yellow oil.
LCMS: *(ESI)* (M+H-28)⁺ = 312.8.
¹H NMR: (400 MHz, CDCl₃) *δ* 7.77 (s, 1H), 5.53 (s, 2H), 3.80 - 3.70 (m, 2H), 1.07 - 0.95 (m, 2H), 0.03 (s, 9H).

### Step C: synthesis of 5-chloro-4-(trifluoromethyl)-2-((2-(trimethylsilyl)ethoxy)methyl) pyridazin-3(2H)-one (Intermediate A)

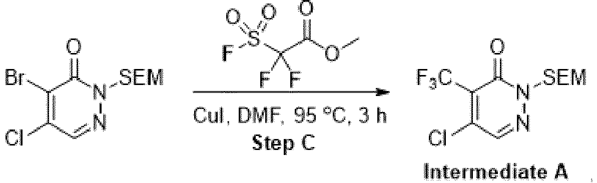

To a solution of 4-bromo-5-chloro-2-((2-(trimethylsilyl)ethoxy)methyl)pyridazin-3 (2H)-one (3.25 g, 9.57 mmol, 1.0 eq.) in DMF (10 mL) were added CuI (1.28 g, 6.70 mmol, 0.7 eq.) and followed by dropwise addition of methyl 2,2-difluoro-2-(fluorosulfonyl)acetate (5.52 g, 28.71 mmol, 3.0 eq.). The resulting mixture was stirred for 3 h at 95 °C. The mixture was cooled to room temperature and quenched by the addition of water (80 mL). The mixture was extracted with EtOAc (3 x 80 mL). The organic layers were combined and washed with brine (80 mL), dried over Na₂SO₄ and concentrated under reduced pressure. The residue was purified by column chromatography (Petroleum ether/EtOAc= 10/1; V/V) to afford 5-chloro-4 -(trifluoromethyl)-2-((2-(trimethylsilyl)ethoxy)methyl)pyridazin-3(2H)-one (2.80 g, 89.0%) as a colorless oil.
LCMS: *(ESI)* (M+H-28)⁺ = 301.8.
¹H NMR: (400 MHz, CDCl₃) *δ* 8.12 (s, 1H), 5.54 (s, 2H), 3.81 - 3.73 (m, 2H), 1.02 - 0.96 (m, 2H), 0.02 (s, 9H).

### Step D: synthesis of (S)-5-((1-hydroxypropan-2-yl)amino)-4-(trifluoromethyl)-2-((2-(trimethylsilyl)ethoxy)methyl)pyridazin-3(2H)-one (Intermediate B)

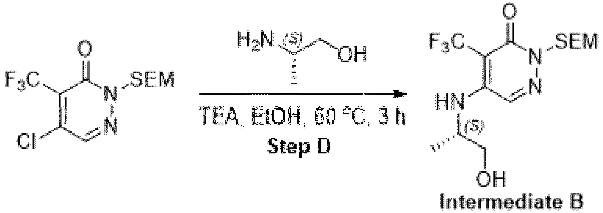

To a solution of 5-chloro-4-(trifluoromethyl)-2-((2-(trimethylsilyl)ethoxy)methyl) pyridazin-3(2H)-one (2.80 g, 8.51 mmol, 1.0 eq.) in EtOH (21 mL) were added TEA (860 mg, 8.51 mmol, 1.0 eq.) and L-Alaninol (2.56 g, 34.04 mmol, 4.0 eq.), the resulting mixture was stirred at 60 °C for 3 h. The mixture was cooled to room temperature and diluted with water (50 mL). The mixture was extracted with EtOAc (3 x 50 mL). The organic layers were combined and washed with brine (50 mL), dried over Na₂SO₄ and concentrated under reduced pressure. The residue was purified by column chromatography (Petroleum ether/EtOAc= 1/1; V/V) to afford (S)-5-((1-hydroxypropan -2-yl)amino)-4-(trifluoromethyl)-2-((2-(trimethylsilyl)ethoxy)methyl)pyridazin-3(2H)-o ne (450 mg, 14.4%) as a yellow oil.
LCMS: *(ESI)* (M+H)⁺ = 367.8.
¹H NMR: (400 MHz, CDCl₃) *δ* 7.74 (s, 1H), 5.90 - 5.79 (m, 1H), 5.45 - 5.35 (m, 2H), 4.00 - 3.89 (m, 1H), 3.87 - 3.80 (m, 1H), 3.76 - 3.65 (m, 3H), 1.35 (d, *J* = 6.4 Hz, 3H), 1.02 - 0.94 (m, 2H), 0.02 (s, 9H).

### Step E: Synthesis of tert-butyl 4-(5-(trifluoromethyl)pyrimidin-2-yl)piperazine-1-carboxylate

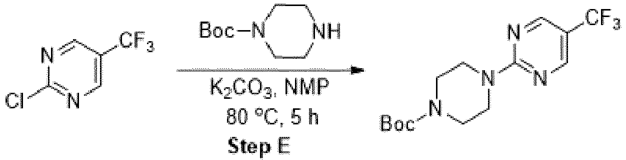

To a solution of 2-chloro-5-(trifluoromethyl)pyrimidine (4.00 g, 21.92 mmol, 1.05 eq.) and tert-butyl piperazine-1-carboxylate (3.89 g, 20.87 mmol, 1.0 eq.) in NMP (40 mL), was added K₂CO₃ (3.46 g, 25.04 mmol, 1.2 eq.). The mixture was heated to 80 °C and stirred for 5 h. The mixture was cooled to room temperature and diluted with water (150 mL). The solid precipitated was collected by filtration. The product was redissolved in EtOAc (150 mL) and dried over Na₂SO₄. The solvent was removed under vacuum to afford tert-butyl 4-(5-(trifluoromethyl)pyrimidin-2-yl)piperazine-1-carboxylate (6.4 g, 87.9% yield) as a white solid.
LCMS: (*ESI)* (M+H-56)⁺ = 276.8.
¹H NMR: (400 MHz, DMSO-*d₆*) *δ* 8.74 (s, 2H), 3.89 - 3.79 (m, 4H), 3.49 - 3.42 (m, 4H), 1.44 (s, 9H).

### Step F: synthesis of 2-(piperazin-1-yl)-5-(trifluoromethyl)pyrimidine (Intermediate C)

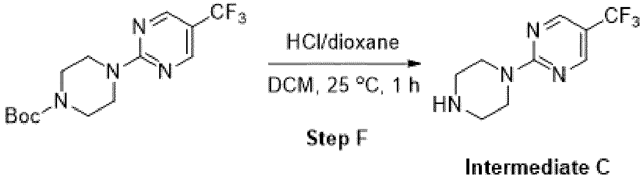

To a solution of tert-butyl 4-(5-(trifluoromethyl)pyrimidin-2-yl)piperazine-1-carboxylate (3.00 g, 9.03 mmol, 1.0 eq.) in DCM (10 mL) was added HCl/dioxane (4M, 10 mL). The resulting mixture was stirred at 25 °C for 1 h. To the mixture was added DIPEA dropwise to adjust pH 7~8. The mixture was concentrated under vacuum to afford 2-(piperazin-1-yl)-5-(trifluoromethyl)pyrimidine (2.90 g, 100% yield) as a white solid.
LCMS: *(ESI)* (M+H)⁺ = 233.0.

### Step G: synthesis of tert-butyl 2-oxo-2-(4-(5-(trifluoromethyl)pyrimidin-2-yl)piperazin -1-yl)ethoxycarbamate

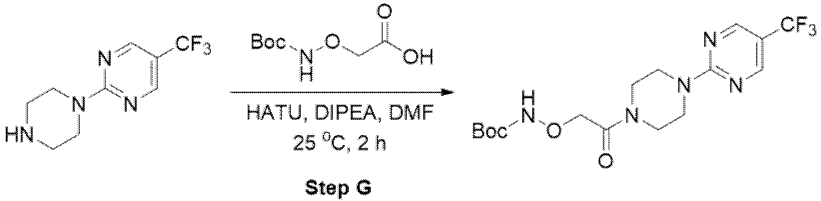

To a solution of 2-(piperazin-1-yl)-5-(trifluoromethyl)pyrimidine (2.90 g, 12.49 mmol, 1.0 eq.) in DMF (50 mL) were added DIPEA (8.07 g, 62.45 mmol, 5.0 eq.) and HATU (5.70 g, 14.99 mmol, 1.2 eq.), followed by dropwise addition of 2-(((tert-butoxycarbonyl)amino)oxy)acetic acid (2.62 g, 13.74 mmol, 1.1 eq.). The resulting mixture was stirred at 25 °C for 2 h. The reaction mixture was quenched with water (200 mL) and extracted with EtOAc (3 x 150 mL). The organic layers were combined and washed with brine (150 mL), dried over Na₂SO₄ and concentrated under vacuum. The residue was purified by silica gel chromatography (Petroleum ether/EtOAc= 1/1; V/V) to afford tert-butyl 2-oxo-2-(4-(5-(trifluoromethyl)pyrimidin -2-yl)piperazin-1-yl)ethoxycarbamate (4.50 g, 88.9% yield) as a yellow oil.
LCMS: *(ESI)* (M+H-56)⁺ = 349.8.

### Step H: synthesis of 2-(aminooxy)-1-(4-(5-(trifluoromethyl)pyrimidin-2-yl) hexahydropyrazin -1-yl)ethanone hydrochloride (Intermediate D)

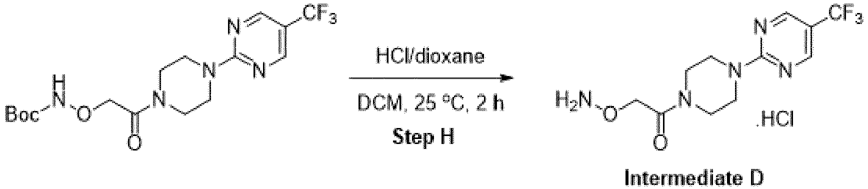

To a solution of tert-butyl 2-oxo-2-(4-(5-(trifluoromethyl)pyrimidin-2-yl) piperazin-1-yl)ethoxycarbamate (2.25 g, 5.55 mmol, 1.0 eq.) in DCM (60 mL) was added HCl/dioxane (4M HCl gas in dioxane; 12 mL). The resulting mixture was stirred at 25 °C for 2 h. The reaction mixture was concentrated under vacuum to afford 2-(aminooxy)-1-(4-(5-(trifluoromethyl)pyrimidin-2-yl)hexahydropyrazin-1-yl)ethanone hydrochloride (1.73 g, 91.2% yield) as a yellow solid.
LCMS: *(ESI)* (M+H)⁺ = 305.8.

### 1.2) Synthesis of compound 1

### Step A: synthesis of (S)-tert-butyl (1-((2-oxo-2-(4-(5-(trifluoromethyl)pyrimidin-2-yl) piperazin-1-yl)ethoxy)amino)propan-2-yl)carbamate (Intermediate E)

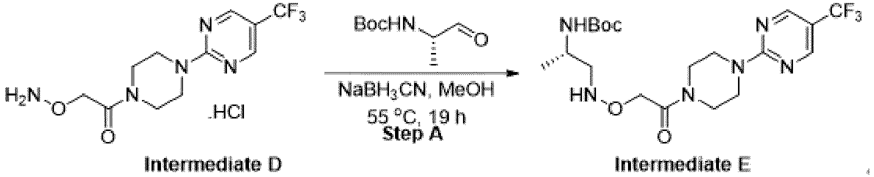

To a mixture of 2-(aminooxy)-1-(4-(5-(trifluoromethyl)pyrimidin-2-yl) hexahydropyrazin-1-yl)ethanone hydrochloride (342 mg, 1.00 mmol, 1.0 eq.) in MeOH (10 mL) were added DIPEA (388 mg, 3.00 mmol, 3.0 eq.) and Boc-L-Alaninal (208 mg, 1.20 mmol, 1.2 eq.). The mixture was stirred at 55 °C for 3 h. The mixture was cooled to room temperature and acetic acid (240 mg, 4.00 mmol, 4.0 eq.) and sodium cyanoborohydride (629 mg, 10.00 mmol, 10.0 eq.) were added to the mixture. The mixture was stirred at 55 °C for another 16 h. The mixture was cooled to room temperature and quenched with saturated NaHCOs aqueous solution (100 mL), extracted with EtOAc (2 x 80 mL). The organic layers were combined and dried over Na₂SO₄, concentrated under vacuum. The residue was purified by silica gel chromatography (EtOAc/Petorleum ether= 2/1; V/V) to afford (S)-tert-butyl (1-((2-oxo-2-(4-(5-(trifluoromethyl)pyrimidin-2-yl)piperazin-1-yl)ethoxy)amino)propan -2-yl)carbamate (330 mg, 71.3%) as a white solid.
LCMS: *(ESI)* (M+H)⁺ = 463.2.
¹H NMR: (400 MHz, CDCl₃) *δ* 8.51 (s, 2H), 6.58 (br s, 1H), 4.75 - 4.65 (m, 1H), 4.52 - 4.39 (m, 2H), 4.03 - 3.89 (m, 4H), 3.74 - 3.68 (m, 2H), 3.68 - 3.60 (m, 2H), 3.49 - 3.42 (m, 2H), 3.13 - 3.09 (m, 1H),1.45 (s, 9H), 1.19 - 1.14 (m, 3H).

### Step B: synthesis of (S)-2-(((2-aminopropyl)amino)oxy)-1-(4-(5-(trifluoromethyl) pyrimidin-2-yl)piperazin-1-yl)ethanone

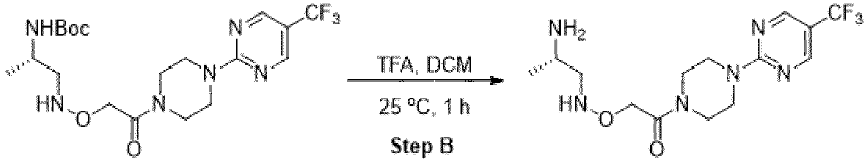

To a solution of (S)-tert-butyl (1-((2-oxo-2-(4-(5-(trifluoromethyl)pyrimidin-2-yl) piperazin-1-yl)ethoxy)amino)propan-2-yl)carbamate (148 mg, 0.32 mmol, 1.0 eq.) in DCM (3 mL) was added TFA (1.5 mL). The mixture was stirred at 25 °C for 1 h. The excess TFA was neutralized with saturated NaHCOs aqueous solution (40 mL) and the mixture was extracted with DCM (2 x 50 mL). The organic layers were combined and dried over Na₂SO₄, concentrated under vacuum to afford (S)-2-(((2-aminopropyl) amino)oxy)-1-(4-(5-(trifluoromethyl)pyrimidin-2-yl)piperazin-1-yl)ethanone (116 mg, 99.0%) as a light yellow solid.
LCMS: *(ESI)* (M+H)⁺ = 363.2.

### Step C: synthesis of (S)-5-((1-((2-oxo-2-(4-(5-(trifluoromethyl)pyrimidin-2-yl)piperazin -1-yl)ethoxy)amino)propan-2-y)amino)-4-(trifluoromethyl)-2-((2-(trimethylsilyl)ethoxy )methyl)pyridazin-3(2H)-one

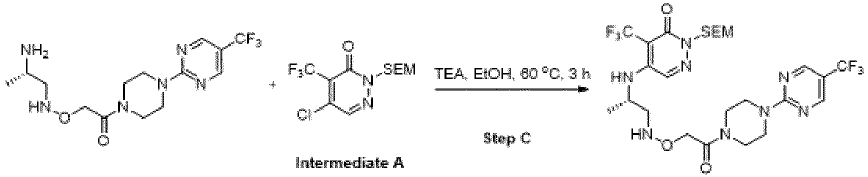

To a solution of (S)-2-(((2-aminopropyl)amino)oxy)-1-(4-(5-(trifluoromethyl) pyrimidin-2-yl)piperazin-1-yl)ethanone (116 mg, 0.32 mmol, 1.0 eq.) in EtOH (6 mL) were added TEA (162 mg, 1.60 mmol, 5.0 eq.) and 5-chloro-4-(trifluoromethyl)-2-((2-(trimethylsilyl)ethoxy)methyl)pyridazin-3(2H)-one (316 mg, 0.96 mmol, 3.0 eq.). The mixture was heated to 60 °C and stirred for 3 h. The mixture was cooled to room temperature and concentrated under vacuum. The residue was purified by silica gel chromatography (EtOAc/Petorleum ether= 2/1; V/V) to afford (S)-5-((1-((2-oxo-2-(4-(5-(trifluoromethyl)pyrimidin-2-yl)piperazin-1-yl)ethoxy)amino)propan-2-y)amino)-4-(t rifluoromethyl)-2-((2-(trimethylsilyl)ethoxy)methyl)pyridazin-3(2H)-one (37 mg, 17.7%) as a light yellow solid.
LCMS: *(ESI)* (M+H)⁺ = 655.2.

### Step D: Synthesis of (S)-5-((1-((2-oxo-2-(4-(5-(trifluoromethyl)pyrimidin-2-yl) piperazin-1-yl)ethoxy)amino)propan-2-yl)amino)-4-(trifluoromethyl)pyridazin-3(2H)-one

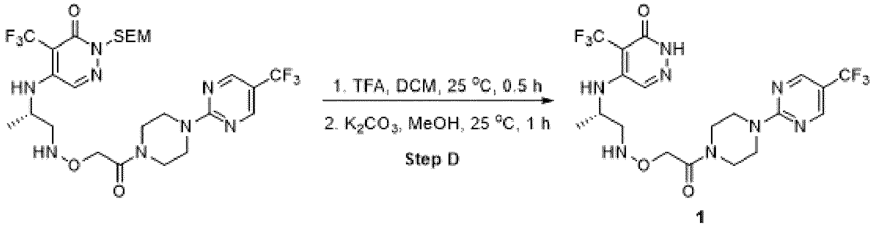

To a solution of (S)-5-((1-((2-oxo-2-(4-(5-(trifluoromethyl) pyrimidin-2-yl)piperazin-1-yl)ethoxy)amino)propan-2-y)amino)-4-(trifluoromethyl)-2-( (2-(trimethylsilyl)ethoxy)methyl)pyridazin-3(2H)-one (26 mg, 0.04 mmol, 1.0 eq.) in DCM (3 mL) was added TFA (0.4 mL). The mixture was stirred at 25 °C for 0.5 h. The mixture was neutralized with saturated NaHCOs aqueous solution (30 mL) and extracted with DCM (3 x 50 mL). The organic layers were combined and dried over Na₂SO₄, concentrated under vacuum. The residue was redissolved in MeOH (3 mL) and Potassium carbonate (20 mg, 0.15 mmol, 3.6 eq.) was added. The mixture was stirred at 25 °C for another 1 h. The mixture was filtered and the filtrate was purified by prep-HPLC to afford (S)-5-((1-((2-oxo-2-(4-(5-(trifluoromethyl)pyrimidin-2-yl) piperazin-1-yl)ethoxy)amino)propan-2-yl)amino)-4-(tlifluoromethyl)pylidazin-3(2H)-o ne (3.62 mg, 15.7%) as a light yellow solid.
LCMS: *(ESI)* (M+H)⁺ = 525.5.
¹H NMR: (400 MHz, CDCl₃) *δ* 10.50 (br s, 1H), 8.52 (s, 2H), 7.70 (s, 1H), 7.05 - 6.97 (m, 1H), 6.33 - 6.22 (m, 1H), 4.44 (s, 2H), 4.08 - 3.99 (m, 1H), 3.98 - 3.89 (m, 4H), 3.76 - 3.66 (m, 2H), 3.43 - 3.34 (m, 2H), 3.33 - 3.23 (m, 1H), 3.07 - 2.96 (m, 1H), 1.36 (d, *J* = 6.4 Hz, 3H).

### Example 2:

### (S)-5-((1-(methyl(2-oxo-2-(4-(5-(trifluoromethyl)pyrimidin-2-yl)piperazin-1-yl)ethoxy) amino)propan-2-yl)amino)-4-(trifluoromethyl)pyridazin-3(2H)-one

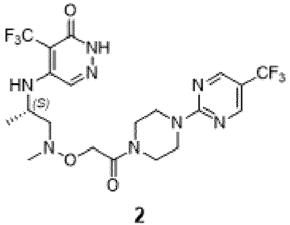

### Step A: synthesis of (S)-tert-butyl (1-(methyl(2-oxo-2-(4-(5-(trifluoromethyl)pyrimidin -2-yl)piperazin-1-yl)ethoxy)amino)propan-2-yl)carbamate

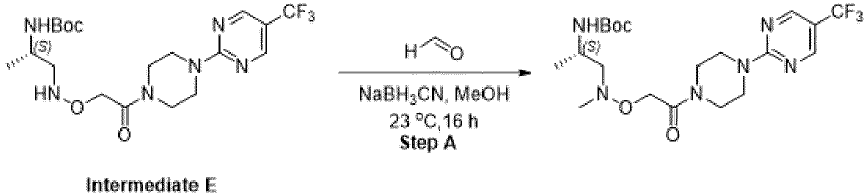

To a solution of (S)-tert-butyl (1-((2-oxo-2-(4-(5-(trifluoromethyl)pyrimidin-2-yl) piperazin-1-yl)ethoxy)amino)propan-2-yl)carbamate (240 mg, 0.52 mmol, 1.0 eq.) in MeOH (10 mL) was added formaldehyde (37% in water, 421 mg, 5.19 mmol, 10.0 eq.). The mixture was stirred at 23 °C for 3 h. Acetic acid (125 mg, 2.08 mmol, 4.0 eq.) and sodium cyanoborohydride (326 mg, 5.19 mmol, 10.0 eq.) were added to the reaction mixture. The mixture was stirred at 23 °C for another 16 h. The mixture was diluted with water (80 mL) and neutralized with saturated NaHCOs aqueous solution (10 mL). The mixture was extracted with EtOAc (3 x 80 mL). The organic layers were combined and washed with brine (80 mL), dried over Na₂SO₄ and concentrated under vacuum. The residue was purified by column chromatography (Petroleum ether/EtOAc= 2/1; V/V) to afford (S)-tert-butyl (1-(methyl(2-oxo-2-(4-(5-(trifluoromethyl)pyrimidin-2 -yl)piperazin-1-yl)ethoxy)amino)propan-2-yl)carbamate (200 mg, 76.0% yield) as a colorless oil.
LCMS: *(ESI)* (M+H)⁺ = 477.2.

### Step B: synthesis of (S)-2-(((2-aminopropyl)(methyl)amino)oxy)-1-(4-(5-(trifluoromethyl)pyrimidin-2-yl)piperazin-1-yl)ethanone

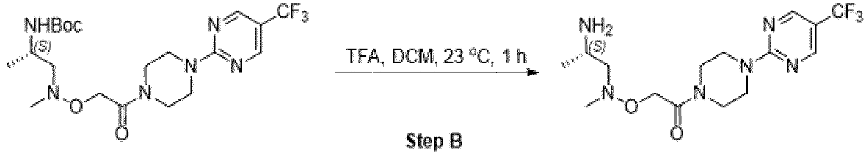

To a solution of (S)-tert-butyl (1-(methyl(2-oxo-2-(4-(5-(trifluoromethyl) pyrimidin-2-yl)piperazin-1-yl)ethoxy)amino)propan-2-yl)carbamate (170 mg, 0.36 mmol, 1.0 eq.) in DCM (4 mL) was added TFA (2 mL). The mixture was stirred at 23 °C for 1 h. The excess TFA was neutralized with saturated NaHCOs aqueous solution (50 mL) and the mixture was extracted with DCM (3 x 80 mL). The organic layers were combined and dried over Na₂SO₄, concentrated under vacuum to afford (S)-2-(((2-aminopropyl)(methyl)amino)oxy)-1-(4-(5-(trifluoromethyl)pyrimidin-2-yl)pi perazin-1-yl)ethanone (120 mg, 78.6%) as a white solid.
LCMS: *(ESI)* (M+H)⁺ = 377.2.

### Step C: synthesis of (S)-5-((1-(methyl(2-oxo-2-(4-(5-(trifluoromethyl)pyrimidin-2-yl) piperazin-1-yl)ethoxy)amino)propan-2-yl)amino)-4-(trifluoromethyl)-2-((2-(trimethylsil yl)ethoxy)methyl)pyridazin-3(2H)-one

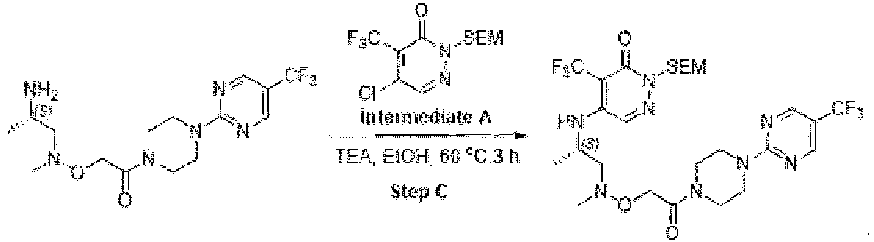

To a solution of (S)-2-(((2-aminopropyl)(methyl)amino)oxy)-1-(4-(5-(trifluoromethyl)pyrimidin-2-yl)piperazin-1-yl)ethanone (120 mg, 0.32 mmol, 1.0 eq.) in EtOH (10 mL) were added TEA (162 mg, 1.60 mmol, 5.0 eq.) and followed by 5-chloro-4-(trifluoromethyl)-2-((2-(trimethylsilyl)ethoxy)methyl)pyridazin-3 (2H)-one (210 mg, 0.64 mmol, 2.0 eq.). The mixture was heated to 60 °C and stirred for 3 h. The mixture was cooled to room temperature and concentrated under vacuum. The residue was purified by column chromatography (Petroleum ether/EtOAc= 2/1; V/V) to afford (S)-5-((1-(methyl(2-oxo-2-(4-(5-(trifluoromethyl)pyrimidin-2-yl)piperazin-1-yl)ethoxy) amino)propan-2-yl)amino)-4-(trifluoromethyl)-2-((2-(trimethylsilyl)ethoxy)methyl)pyri dazin-3(2H)-one (50 mg, 15.0%) as a yellow oil.
LCMS: (*ESI)* (M+H)⁺ = 669.3.

### Step D: Synthesis of (S)-5-((1-(methyl(2-oxo-2-(4-(5-(trifluoromethyl)pyrimidin-2-yl) piperazin-1-yl)ethoxy)amino)propan-2-yl)amino)-4-(trifluoromethyl)pyridazin-3(2H)-o ne

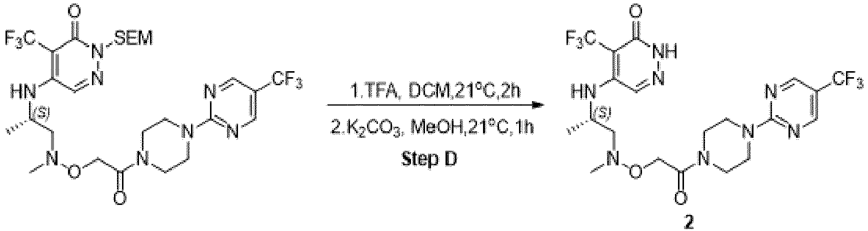

To a solution of (S)-5-((1-(methyl(2-oxo-2-(4-(5-(trifluoromethyl)pyrimidin -2-yl)piperazin-1-yl)ethoxy)amino)propan-2-yl)amino)-4-(trifluoromethyl)-2-((2-(trimet hylsilyl)ethoxy)methyl)pyridazin-3(2H)-one (25 mg, 0.037 mmol, 1.0 eq.) in DCM (2 mL) was added TFA (0.2 mL). The mixture was stirred at 21 °C for 2 h. The mixture was neutralized with saturated NaHCOs (30 mL) and extracted with DCM (3 x 50 mL). The organic layers were combined and dried over Na₂SO₄, concentrated under vacuum. The residue was redissolved in MeOH (2 mL) and Potassium carbonate (19 mg, 0.14 mmol, 3.6 eq.) was added. The mixture was stirred at 21 °C for another 1 h. The mixture was filtered and the filtrate was purified by prep-HPLC to afford (S)-5-((1-(methyl(2-oxo-2-(4-(5-(trifluoromethyl)pyrimidin-2-yl)piperazin-1-yl)ethoxy) amino)propan-2-yl)amino)-4-(trifluoromethyl)pyridazin-3(2H)-one (4.8 mg, 22.9% yield) as a white solid.
LCMS: (*ESI)* (M+H)⁺=539.2.
¹H NMR: (400 MHz, CDCl₃) *δ* 10.23 (br s, 1H), 8.51 (s, 2H), 7.75 (s, 1H), 6.04 - 5.95 (m, 1H), 4.46 - 4.36 (m, 2H), 4.19 - 4.09 (m, 1H), 3.96 - 3.86 (m, 4H), 3.73 - 3.59 (m, 2H), 3.48 - 3.39 (m, 2H), 3.06 - 2.94 (m, 1H), 2.92 - 2.83 (m, 1H), 2.71 (s, 3H), 1.34 (d, *J* = 6.4 Hz, 3H).

### Example 3:

### (S)-5-((1-(ethyl(2-oxo-2-(4-(5-(trifluoromethyl)pyrimidin-2-yl)piperazin-1-yl)ethoxy) amino)propan-2-yl)amino)-4-(trifluoromethyl)pyridazin-3(2H)-one

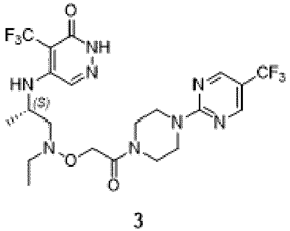

### Step A: synthesis of (S)-tert-butyl (1-(ethyl(2-oxo-2-(4-(5-(trifluoromethyl)pyrimidin -2-yl)piperazin-1-yl)ethoxy)amino)propan-2-yl)carbamate

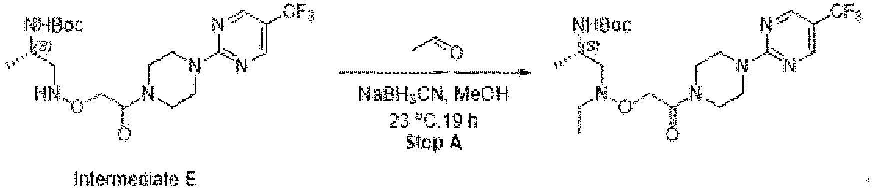

To a solution of (S)-tert-butyl(1-((2-oxo-2-(4-(5-(trifluoromethyl)pyrimidin-2-yl) piperazin-1-yl)ethoxy)amino)propan-2-yl)carbamate (250 mg, 0.54 mmol, 1.0 eq.) in MeOH (10 mL) was added acetaldehyde (238 mg, 5.41 mmol, 10.0 eq.). The mixture was stirred at 23 °C for 3 h. Then to the mixture were added acetic acid (32 mg, 0.54 mmol, 1.0 eq.) and sodium cyanoborohydride (340 mg, 5.41 mmol, 10.0 eq.). The mixture was stirred at 23 °C for another 16 h. The mixture was diluted with water (80 mL) and neutralized with saturated NaHCO₃ aqueous solution (5 mL). The mixture was extracted with EtOAc (3 x 80 mL). The organic layers were combined and washed with brine (80 mL), dried over Na₂SO₄ and concentrated under vacuum. The residue was purified by column chromatography (Petroleum ether/EtOAc= 2/1; V/V) to afford (S)-tert-butyl (1-(ethyl(2-oxo-2-(4-(5-(trifluoromethyl)pyrimidin-2-yl)piperazin-1-yl) ethoxy)amino)propan-2-yl)carbamate (180 mg, 63.8% yield) as a yellow oil.
LCMS: (*ESI)* (M+H)⁺ = 491.2.

### Step B: synthesis of (S)-2-(((2-aminopropyl)(ethyl)amino)oxy)-1-(4-(5-(trifluoromethyl) pyrimidin-2-yl)piperazin-1-yl)ethanone

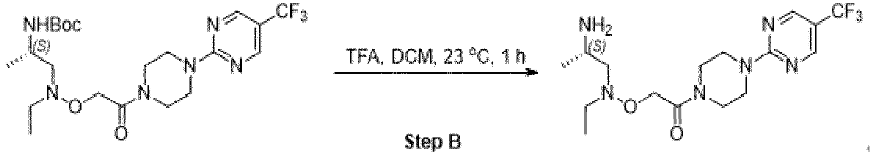

To a solution of (S)-tert-butyl (1-(ethyl(2-oxo-2-(4-(5-(trifluoromethyl)pyrimidin-2-yl)piperazin-1-yl)ethoxy)amino)propan-2-yl)carbamate (160 mg, 0.33 mmol, 1.0 eq.) in DCM (4 mL) was added TFA (2 mL). The mixture was stirred at 23 °C for 1 h. The excess TFA was neutralized with saturated NaHCOs aqueous solution (40 mL) and the mixture was extracted with DCM (3 x 50 mL). The organic layers were combined and dried over Na₂SO₄, concentrated under vacuum to afford (S)-2-(((2-aminopropyl)(ethyl) amino)oxy)-1-(4-(5-(trifluoromethyl)pyrimidin-2-yl)piperazin-1-yl)ethanone (160 mg, 95.5%) as a white solid.
LCMS: (*ESI)* (M+H)⁺ = 391.2.

### Step C: synthesis of (S)-5-((1-(ethyl(2-oxo-2-(4-(5-(trifluoromethyl)pyrimidin-2-yl) piperazin-1-yl)ethoxy)amino)propan-2-yl)amino)-4-(trifluoromethyl)-2-((2-(trimethylsil yl)ethoxy)methyl)pyridazin-3 (2H)-one

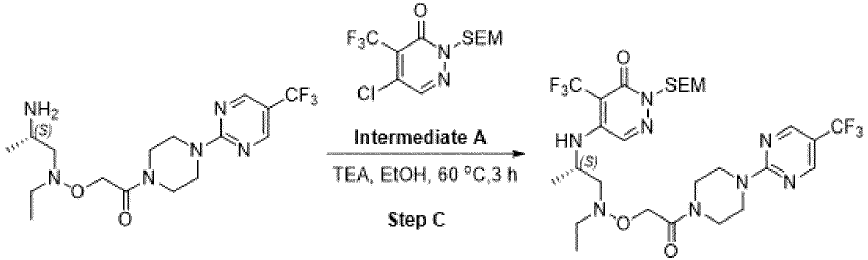

To a solution of (S)-2-(((2-aminopropyl)(ethyl)amino)oxy)-1-(4-(5-(trifluoromethyl)pyrimidin-2-yl)piperazin-1-yl)ethanone (160 mg, 0.41 mmol, 1.0 eq.) in EtOH (2 mL) were added TEA (207 mg, 2.05 mmol, 5.0 eq.) and 5-chloro-4-(trifluoromethyl)-2-((2-(trimethylsilyl)ethoxy)methyl)pyridazin-3 (2H)-one (270 mg, 0.82 mmol, 2.0 eq.). The mixture was heated to 60 °C and stirred for 3 h. The mixture was cooled to room temperature, concentrated under vacuum. The residue was purified by column chromatography (Petroleum ether/EtOAc= 1/1; V/V) to afford (S)-5-((1-(ethyl(2-oxo-2-(4-(5-(trifluoromethyl)pyrimidin-2-yl)piperazin-1-yl)ethoxy) amino)propan-2-yl)amino)-4-(trifluoromethyl)-2-((2-(trimethylsilyl)ethoxy)methyl)pyri dazin-3(2H)-one (110 mg, 33.4%) as a yellow oil.
LCMS: (*ESI)* (M+H)⁺ = 683.4.

### Step D: Synthesis of (S)-5-((1-(ethyl(2-oxo-2-(4-(5-(trifluoromethyl)pyrimidin-2-yl) piperazin-1-yl)ethoxy)amino)propan-2-yl)amino)-4-(trifluoromethyl)pyridazin-3(2H) -one

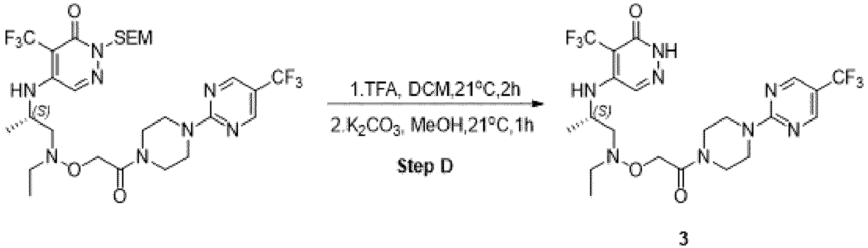

To a solution of (S)-5-((1-(ethyl(2-oxo-2-(4-(5-(trifluoromethyl)pyrimidin-2-yl) piperazin-1-yl)ethoxy)amino)propan-2-yl)amino)-4-(trifluoromethyl)-2-((2-(trimethylsil yl)ethoxy)methyl)pyridazin-3(2H)-one (105 mg, 0.15 mmol, 1.0 eq.) in DCM (2 mL) was added TFA (0.2 mL). The mixture was stirred at 21 °C for 2 h. The mixture was neutralized with saturated NaHCOs aqueous solution (50 mL) and extracted with DCM (2 x 80 mL). The organic layers were combined and dried over Na₂SO₄, concentrated under vacuum. The residue was redissolved in MeOH (2 mL) and potassium carbonate (77 mg, 0.55 mmol, 3.6 eq.) was added. The mixture was stirred at 21 °C for another 1 h. The mixture was filtered and the filtrate was purified by prep-HPLC to afford (S)-5-((1-(ethyl(2-oxo-2-(4-(5-(trifluoromethyl)pyrimidin-2-yl)piperazin-1-yl)ethoxy) amino)propan-2-yl)amino)-4-(trifluoromethyl)pyridazin-3(2H)-one (23.29 mg, 27.4% yield) as a white solid.
LCMS: (*ESI)* (M+H)⁺ = 553.2.
¹H NMR: (400 MHz, CDCl₃) *δ* 10.15 (br s, 1H), 8.51 (s, 2H), 7.76 (s, 1H), 6.04 - 5.92 (m, 1H), 4.49 - 4.40 (m, 2H), 4.24 - 4.15 (m, 1H), 3.95 - 3.85 (m, 4H), 3.73 - 3.54 (m, 2H), 3.46 - 3.35 (m, 2H), 3.01 - 2.83 (m, 4H), 1.33 (d, *J* = 6.4 Hz, 3H), 1.16 (t, *J* = 7.2 Hz, 3H).

### Example 4:

### (S)-2-(4-(2-((methyl(2-((6-oxo-5-(trifluoromethyl)-1,6-dihydropyridazin-4-yl)amino) propyl)amino)oxy)acetyl)piperazin-1-yl)pyrimidine-5-carbonitrile

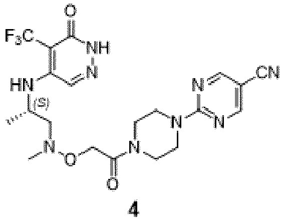

### 4.1) Synthesis of intermediate G

### Step A: synthesis of tert-butyl 4-(5-cyanopyrimidin-2-yl)piperazine-1-carboxylate

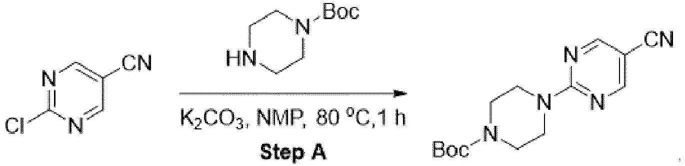

To a solution of 2-chloropyrimidine-5-carbonitrile (3.0 g, 21.50 mmol, 1.0 eq.) and tert-butyl piperazine-1-carboxylate (4.00 g, 21.50 mmol, 1.0 eq.) in NMP (30 mL) was added K₂CO₃ (5.94 g, 43.00 mmol, 2.0 eq.). The mixture was heated to 80 °C and stirred for 1.0 h. The mixture was cooled to room temperature and diluted with water (40 mL). The mixture was extracted with EtOAc (3 x 50 mL). The organic layers were combined and washed with brine (50 mL), dried over Na₂SO₄ and concentrated under vacuum. The residue was purified by silica gel chromatography (Petroleum ether/EtOAc= 5/1; V/V) to afford tert-butyl 4-(5-cyanopyrimidin-2-yl)piperazine -1-carboxylate (4.0 g, 64.3% yield) as a yellow solid.
LCMS: *(ESI)* (M+H-56)⁺ = 234.2.
¹H NMR: (400 MHz, CDCl₃) *δ* 8.50 (s, 2H), 3.95 - 3.84 (m, 4H), 3.58 - 3.45 (m, 4H), 1.49 (s, 9H).

### Step B: synthesis of 2-(piperazin-1-yl)pyrimidine-5-carbonitrile hydrochloride (Intermediate G)

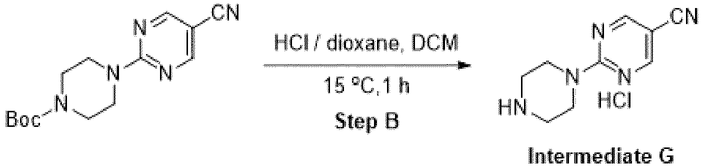

To a solution of tert-butyl 4-(5-cyanopyrimidin-2-yl)piperazine-1-carboxylate (1.0 g, 3.46 mmol, 1.0 eq.) in DCM (5 mL) was added HCl/dioxane (4M, 15 mL). The resulting mixture was stirred at 15 °C for 1 h. The mixture was concentrated under vacuum to afford 2-(piperazin-1-yl)pyrimidine-5-carbonitrile hydrochloride (720 mg, 92.3% yield) as a white solid.
LCMS: *(ESI)* (M+H)⁺ = 190.2.

### 4.2) Synthesis of compound 4

### Step A: synthesis of tert-butyl (2-(4-(5-cyanopyrimidin-2-yl)piperazin-1-yl) -2-oxoethoxy)carbamate

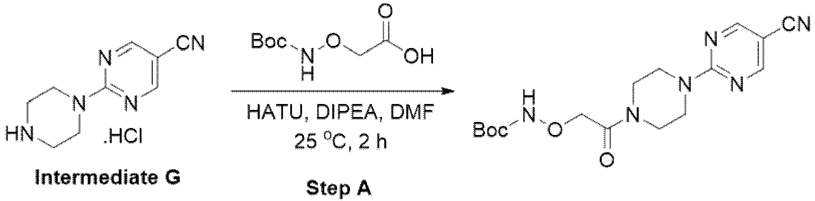

To a solution of 2-(piperazin-1-yl)pyrimidine-5-carbonitrile hydrochloride (7.1 g, 37.57 mmol, 1.0 eq.) in THF (100 mL) were added DIEA (7.2 g, 56.25 mmol, 1.5 eq.) and HATU (21 g, 56.25 mmol, 1.5 eq.) followed by dropwise addition of 2-(((tert-butoxycarbonyl)amino)oxy)acetic acid (7.1 g, 37.57 mmol, 1.1 eq.). The resulting mixture was stirred at 25 °C for 2 h. The reaction mixture was quenched with water (200 mL) and extracted with EtOAc (3 x 150 mL). The organic layers were combined and washed with brine (150 mL), dried over Na₂SO₄ and concentrated under vacuum. The residue was purified by silica gel chromatography (Petroleum ether/EtOAc= 1/1; V/V) to afford tert-butyl (2-(4-(5-cyanopyrimidin-2-yl)piperazin-1-yl) -2-oxoethoxy)carbamate (8.3 g, 61% yield) as a white solid.
LCMS: (*ESI)* (M+H-56)⁺ = 306.1.

### Step B: synthesis of 2-(4-(2-(aminooxy)acetyl)piperazin-1-yl)pyrimidine-5-carbonitrile hydrochloride

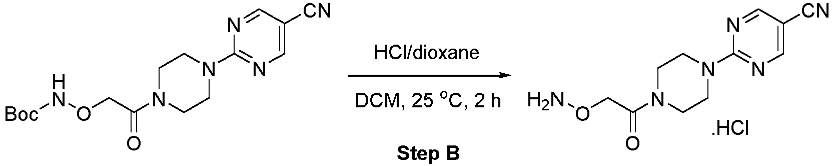

To a solution of tert-butyl (2-(4-(5-cyanopyrimidin-2-yl)piperazin-1-yl)-2 -oxoethoxy)carbamate (500 mg, 1.38 mmol, 1.0 eq.) in DCM (5 mL) was added HCl/dioxane (4M, 3 mL). The resulting mixture was stirred at 25 °C for 2 h. The reaction mixture was concentrated under vacuum. The residue was purified by silica gel chromatography (DCM/MeOH= 92/8, V/V) to afford 2-(4-(2-(aminooxy)acetyl) piperazin-1-yl)pyrimidine-5-carbonitrile hydrochloride (200 mg, 72% yield) as a white solid.
LCMS: (*ESI)* (M+H)⁺ = 263.1.

### Step C: synthesis of tert-butyl (S)-(1-((2-(4-(5-cyanopyrimidin-2-yl)piperazin-1-yl) -2-oxoethoxy)amino)propan-2-yl)carbamate

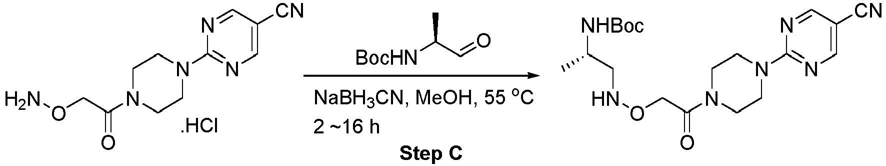

To a solution of 2-(4-(2-(aminooxy)acetyl)piperazin-1-yl)pyrimidine-5-carbonitrile hydrochloride (200 mg, 0.764 mmol, 1.0 eq) in MeOH (4 mL) was added tert-butyl (S)-(1-oxopropan-2-yl)carbamate (200 mg, 1.15 mmol, 1.5 eq). The mixture was stirred at 55 °C for 2 h. Acetic acid (182 mg, 3.1 mmol, 4.0 eq.) and sodium cyanoborohydride (380 mg, 7.6 mmol, 10.0 eq.) were added to the reaction mixture. The mixture was stirred at 55 °C for another 16 h. The mixture was quenched by saturated NH₄Cl aqueous and extracted with EtOAc (3 x 30 mL). The organic layers were combined and washed with brine (40 mL), dried over Na₂SO₄ and concentrated under vacuum. The residue was purified by column chromatography (Petroleum ether/EtOAc= 1/1, V/V) to afford tert-butyl (S)-(1-((2-(4-(5-cyanopyrimidin-2-yl)piperazin-1-yl)-2-oxoethoxy) amino)propan-2-yl)carbamate (200 mg, 62.0% yield) as a white solid.
LCMS: (*ESI)* (M+H)⁺ = 420.2.

### Step D: synthesis of tert-butyl (S)-(1-((2-(4-(5-cyanopyrimidin-2-yl)piperazin-1-yl) -2-oxoethoxy)(methyl)amino)propan-2-yl)carbamate

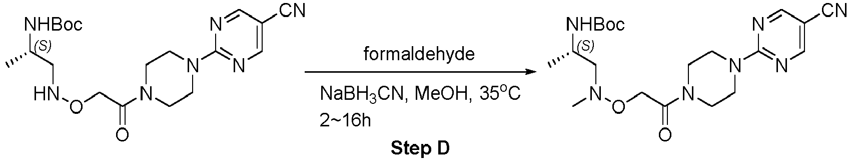

To a solution of tert-butyl (S)-(1-((2-(4-(5-cyanopyrimidin-2-yl)piperazin-1-yl)-2-oxoethoxy)amino)propan-2-yl)carbamate (850 mg, 2.03 mmol, 1.0 eq) in MeOH (12 mL) was added formaldehyde (290 mg, 2.44 mmol, 1.2 eq). The mixture was stirred at 35 °C for 2 h. Acetic acid (125 mg, 2.08 mmol, 4.0 eq.) and sodium cyanoborohydride (1.2 g, 20.3 mmol, 10.0 eq.) were added to the reaction mixture. The mixture was stirred at 35 °C for another 16 h. The mixture was diluted with water (35 mL) and extracted with EtOAc (3 x 30 mL). The organic layers were combined and washed with brine (40 mL), dried over Na₂SO₄ and concentrated under vacuum. The residue was purified by column chromatography (Petroleum ether/EtOAc= 1/2, V/V) to afford tert-butyl (S)-(1-((2-(4-(5-cyanopyrimidin-2-yl)piperazin-1-yl)-2-oxoethoxy)(methyl)amino) propan-2-yl)carbamate (650 mg, 74.0% yield) as a pale yellow solid.
LCMS: (*ESI)* (M+H)⁺ = 434.2.

### Step E: synthesis of (S)-2-(4-(2-(((2-aminopropyl)(methyl)amino)oxy)acetyl)

### piperazin-1-yl)pyrimidine-5-carbonitrile

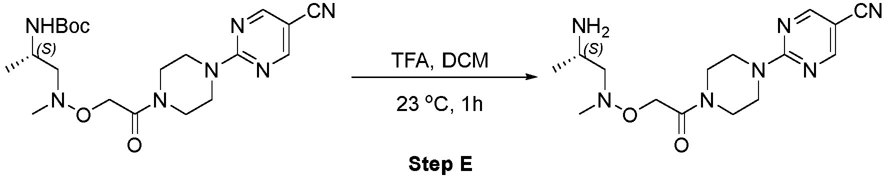

To a solution of tert-butyl (S)-(1-((2-(4-(5-cyanopyrimidin-2-yl)piperazin-1-yl) -2-oxoethoxy)(methyl)amino)propan-2-yl)carbamate (900 mg, 2.08 mmol, 1.0 eq.) in DCM (9 mL) was added TFA (4 mL). The mixture was stirred at 23 °C for 1 h. The excess TFA was neutralized with saturated NaHCO₃ (50 mL) and the mixture was extracted with DCM (3 x 80 mL). The organic layers were combined and dried over Na₂SO₄, concentrated under vacuum to afford (S)-2-(4-(2-(((2-aminopropyl)(methyl) amino)oxy)acetyl)piperazin-1-yl)pyrimidine-5-carbonitrile (500 mg, 72%) as a white solid.
LCMS: (*ESI)* (M+H)⁺ = 334.2.

### Step F: synthesis of (S)-2-(4-(2-((methyl(2-((6-oxo-5-(trifluoromethyl)-1-((2-(trimethylsilyl)ethoxy)methyl)-1,6-dihydropyridazin-4-yl)amino)propyl)amino)oxy)acet yl)piperazin-1-yl)pyrimidine-5-carbonitrile

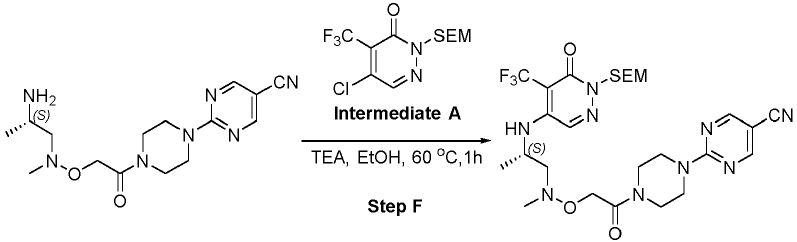

To a solution of (S)-2-(4-(2-(((2-aminopropyl)(methyl)amino)oxy) acetyl)piperazin-1-yl)pyrimidine-5-carbonitrile (340 mg, 1.02 mmol, 1.0 eq.) in EtOH (10 mL) were added TEA (247 mg, 2.44 mmol, 2.0 eq.) and 5-chloro-4-(trifluoromethyl)-2-((2-(trimethylsilyl)ethoxy)methyl)pyridazin-3 (2H)-one (400 mg, 1.22 mmol, 1.20 eq.). The mixture was heated at 60 °C and stirred for 1 h. The mixture was cooled to room temperature and concentrated under vacuum. The residue was purified by column chromatography (Petroleum ether/EtOAc= 1/1, V/V) to afford (S)-2-(4-(2-((methyl(2-((6-oxo-5-(trifluoromethyl)-1-((2-(trimethylsilyl)ethoxy)methyl) -1,6-dihydropyridazin-4-yl)amino)propyl)amino)oxy)acetyl)piperazin-1-yl)pyrimidine-5-carbonitrile (70 mg, 11.0%) as a colorless oil.
LCMS: (*ESI)* (M+H)⁺ = 626.4.

### Step G: Synthesis of (S)-2-(4-(2-((methyl(2-((6-oxo-5-(trifluoromethyl)-L6-dihydropyridazin-4-yl)amino)propyl)amino)oxy)acetyl)piperazin-1-yl)pyrimidine-5-car bonitrile

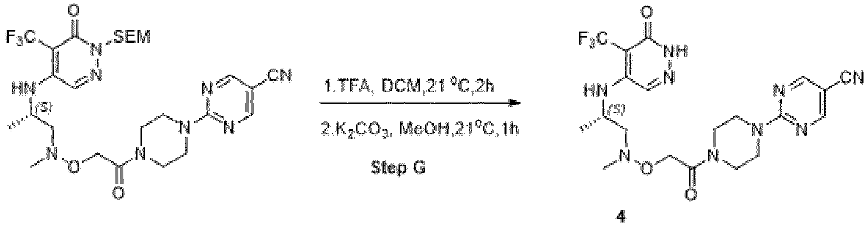

To a solution of (S)-2-(4-(2-((methyl(2-((6-oxo-5-(trifluoromethyl)-1-((2-(trimethylsilyl)ethoxy)methyl)-1,6-dihydropyridazin-4-yl)amino)propyl)amino)oxy)acet yl)piperazin-1-yl)pyrimidine-5-carbonitrile (70 mg, 0.112 mmol, 1.0 eq.) in DCM (3 mL) was added TFA (0.3 mL). The mixture was stirred at 21 °C for 2 h. The mixture was neutralized with saturated NaHCOs (30 mL) and extracted with DCM (3 x 50 mL) . The organic layers were combined and dried over Na₂SO₄, concentrated under vacuum. The residue was dissolved in MeOH (3 mL) and potassium carbonate (60 mg, 0.4 mmol, 3 eq.) was added to the reaction mixture. The mixture was stirred at 21 °C for another 1 h. The mixture was concentrated under vacuum and the residue was purified by column chromatography (Petroleum ether/EtOAc= 1/100, V/V) to afford (S)-2-(4-(2-((methyl(2-((6-oxo-5-(trifluoromethyl)-1,6-dihydropyridazin-4-yl)amino)pr opyl)amino)oxy)acetyl)piperazin-1-yl)pyrimidine-5-carbonitrile (20 mg, 36.3% yield) as a white solid.
LCMS: (*ESI)* (M+H)⁺ = 496.2.
¹H NMR (400 MHz, CDCl₃) *δ* 10.34 (s, 1H), 8.52 (s, 2H), 7.74 (s, 1H), 6.12 - 5.82 (m, 1H), 4.40 (d, J = 1.4 Hz, 2H), 4.19 - 4.07 (m, 1H), 3.99 - 3.84 (m, 4H), 3.74 - 3.58 (m, 2H), 3.49 - 3.37 (m, 2H), 3.08 - 2.93 (m, 1H), 2.93 - 2.81 (m, 1H), 2.71 (s, 3H), 1.34 (d, J = 6.5 Hz, 3H).

### Example 5:

### Synthesis of (S,E)-2-((6-oxo-5-(trifluoromethyl)-1,6-dihydropyridazin-4-yl)amino) propanal O-(2-oxo-2-(4-(5-(trifluoromethyl)pyrimidin-2-yl)piperazin-1-yl)ethyl) oxime

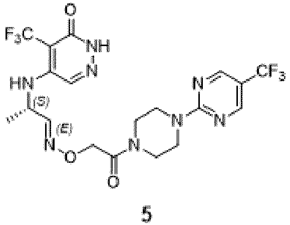

### Step A: synthesis of Boc-L-alaninal

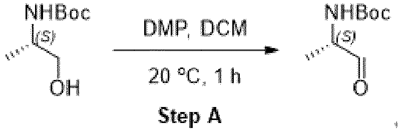

To a solution of Boc-L-Alaninol (5.26 g, 30.00 mmol, 1.0 eq.) in DCM (120 mL) was added Dess-Martin periodinane (17.81 g, 42.00 mmol, 1.4 eq.) at 0 °C in ice-water bath. The mixture was stirred at 20 °C for 1 h. The mixture was quenched slowly by adding water (756 mg, 42.00 mmol, 1.4 eq.) and followed by adding EtOAc/Petroleum ether (1/15; V/V, 150 mL). The mixture was filtered and the filtrate was concentrated. The residue was redissolved in EtOAc/Petroleum ether (1/15; V/V, 150 mL) and washed with a mixed solution of saturated NaHCOs aqueours solution and 10% sodium thiosulfate aqueous solution (1:1; V/V, 3 x 120 mL). The aqueous layers were back extracted with EtOAc/Petroleum ether (1/10; V/V, 5 x 150 mL). The organic layers were combined and dried over Na₂SO₄, concentrated under vacuum to afford Boc-L-alaninal (3.02 g, 17.44 mmol, 58.1% yield) as a light yellow solid.
¹H NMR(400 MHz, CDCl₃) *δ* 9.56 (s, 1H), 5.11 (br s, 1H), 4.30 - 4.17 (m, 1H), 1.46 (s, 9H), 1.34 (d, *J* = 7.2 Hz, 3H).

### Step B: synthesis of (S,E)-tert-butyl (1-((2-oxo-2-(4-(5-(trifluoromethyl)pyrimidin-2-yl) piperazin-1-yl)ethoxy)imino)propan-2-yl)carbamate

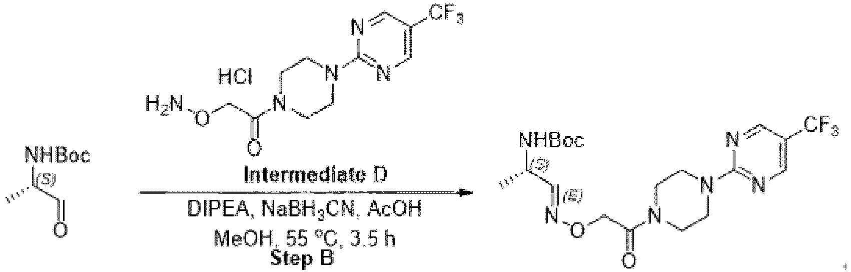

To a solution of 2-(aminooxy)-1-(4-(5-(trifluoromethyl)pyrimidin-2-yl) piperazin-1-yl)ethanone hydrochloride (400 mg, 1.17 mmol, 1.0 eq.) in MeOH (20 mL) were added DIPEA (454 mg, 3.51 mmol, 3.0 eq.) and Boc-L-alaninal (284 mg, 1.64 mmol, 1.4 eq.). The mixture was stirred at 55 °C for 3 h. Acetic acid (71 mg, 1.17 mmol, 1.0 eq.) and Sodium cyanoborohydride (111 mg, 1.76 mmol, 1.5 eq.) were added to the reaction mixture. The mixture was stirred at 55 °C for another 0.5 h. The mixture was diluted with saturated NaHCO₃ aqueous solution (50 mL) and extracted with EtOAc (3 x 100 mL). The organic layers were combined and washed with brine (80 mL), dried over Na₂SO₄ and concentrated under vacuum. The residue was purified by column chromatography (EtOAc/Petroleum ether= 3/4; V/V) to afford (S,E)-tert-butyl (1-((2-oxo-2-(4-(5-(trifluoromethyl)pyrimidin-2-yl)piperazin-1-yl)ethoxy)imino)propan -2-yl)carbamate (350 mg, 64.3% yield) as a white solid.
LCMS:(*ESI*)(M+H-100)⁺ = 361.2.
¹H NMR(400 MHz, DMSO-*d₆*) *δ* 8.73 - 8.71 (m, 2H), 7.39 (d, *J* = 5.6 Hz, 1H), 7.12 -7.10 (d, *J* = 8 Hz, 1H), 4.74 - 4.70 (m, 2H), 4.17 - 4.08 (m, 1H), 3.93 - 3.77 (m, 4H), 3.61 -3.50 (m, 4H), 1.35 (s, 9H), 1.15 (d, *J* = 7.2 Hz, 3H).

### Step C: synthesis of (S,E)-2-aminopropanal O-(2-oxo-2-(4-(5-(trifluoromethyl) pyrimidin-2-yl)piperazin-1-yl)ethyl) oxime

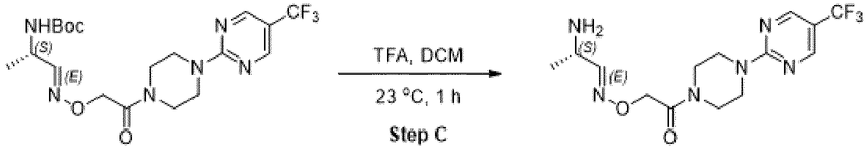

To a solution of (S,E)-tert-butyl (1-((2-oxo-2-(4-(5-(trifluoromethyl)pyrimidin -2-yl)piperazin-1-yl)ethoxy)imino)propan-2-yl)carbamate (300 mg, 0.65 mmol, 1.0 eq.) in DCM (6 mL) was added TFA (2 mL). The mixture was stirred at 23 °C for 1 h. The excess TFA was neutralized with saturated NaHCOs aqueous solution (50 mL) and the mixture was extracted with DCM (3 x 80 mL). The organic layers were combined and dried over Na₂SO₄, concentrated under vacuum to afford (S,E)-2-aminopropanal O-(2-oxo-2-(4-(5-(trifluoromethyl)pyrimidin-2-yl)piperazin-1-yl)ethyl) oxime (200 mg, 57.9% yield) as a white solid.
LCMS:(*ESI*)(M+H)⁺ = 361.2.

### Step D: synthesis of (S,E)-2-((6-oxo-5-(trifluoromethyl)-1-((2-(trimethylsilyl)ethoxy) methyl)-1,6-dihydropyridazin-4-yl)amino)propanal O-(2-oxo-2-(4-(5-(trifluoromethyl) rimidin-2-l)ierazin-1-l)ethl) oxime

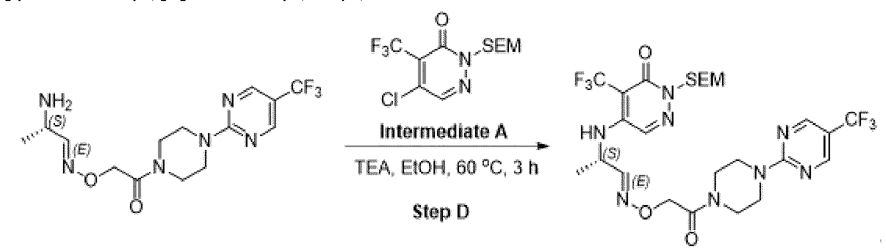

To a solution of (S,E)-2-aminopropanal O-(2-oxo-2-(4-(5-(trifluoromethyl) pyrimidin-2-yl)piperazin-1-yl)ethyl) oxime (200 mg, 0.56 mmol, 1.0 eq.) in EtOH (10 mL) were added TEA (281 mg, 2.76 mmol, 5.0 eq.) and followed by 5-chloro-4-(trifluoromethyl)-2-((2-(trimethylsilyl)ethoxy)methyl)pyridazin-3 (2H)-one (365 mg, 1.11 mmol, 2.0 eq.). The mixture was heated to 60 °C and stirred for 3 h. The mixture was cooled to room temperature concentrated under vacuum. The residue was purified by column chromatography (EtOAc/Petroleum ether= 3/5; V/V) to afford (S,E)-2-((6-oxo-5-(trifluoromethyl)-1-((2-(trimethylsilyl)ethoxy)methyl)-1,6-dihydropy ridazin-4-yl)amino)propanal O-(2-oxo-2-(4-(5-(trifluoromethyl)pyrimidin-2-yl) piperazin-1-yl)ethyl) oxime (160 mg, 40.0% yield) as a yellow oil. LCMS:(*ESI*)(M+H)⁺ = 653.2.

### Step E: Synthesis of (S,E)-2-((6-oxo-5-(trifluoromethyl)-1,6-dihydropyridazin-4-yl) amino)propanal O-(2-oxo-2-(4-(5-(trifluoromethyl)pyrimidin-2-yl)piperazin-1-yl)ethyl) oxime

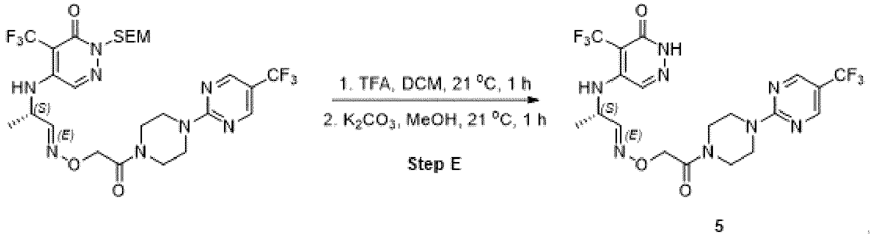

To a solution of (S,E)-2-((6-oxo-5-(trifluoromethyl)-1-((2-(trimethylsilyl)ethoxy) methyl)-1,6-dihydropyridazin-4-yl)amino)propanal O-(2-oxo-2-(4-(5-(trifluoromethyl) pyrimidin-2-yl)piperazin-1-yl)ethyl) oxime (150 mg, 0.23 mmol, 1.0 eq.) in DCM (10 mL) was added TFA (1 mL). The mixture was stirred at 21 °C for 1 h. The mixture was neutralized with saturated NaHCOs aqueous solution (50 mL) and extracted with DCM (2 x 80 mL). The organic layers were combined and dried over Na₂SO₄, concentrated under vacuum. The residue was redissolved in MeOH (5 mL) and potassium carbonate (114 mg, 0.83 mmol, 3.6 eq.) was added. The mixture was stirred at 21 °C for another 1 h. The mixture was filtered and the filtrate was purified by prep-HPLC to afford (S,E)-2-((6-oxo-5-(trifluoromethyl)-1,6-dihydropyridazin-4-yl)amino)propanal O-(2-oxo-2-(4-(5-(trifluoromethyl)pyrimidin-2-yl)piperazin-1-yl)ethyl) oxime (43.89 mg, 36.6% yield) as a white solid.
LCMS:(*ESI*)(M+H)⁺=523.2.
¹H NMR (400 MHz, CDCl₃) *δ* 11.06 (br s, 1H), 8.58 - 8.47 (m, 2H), 7.71 (s, 1H), 7.50 (d, *J* = 5.6 Hz, 1H), 5.91 - 5.81 (m, 1H), 4.84 - 4.74 (m, 2H), 4.48 - 4.39 (m, 1H), 3.99 - 3.88 (m, 4H), 3.77 - 3.68 (m, 2H), 3.56 - 3.48 (m, 2H), 1.52 (d, *J* = 6.8 Hz, 3H).

### Example 6:

### (S,E)-2-((6-oxo-5-(trifluoromethyl)-L6-dihydropyridazin-4-yl)amino)butanal O-(2-oxo -2-(4-(5-(trifluoromethyl)pyrimidin-2-yl)piperazin-1-yl)ethyl)oxime

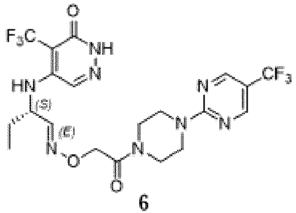

### Step A: synthesis of (S)-tert-butyl (1-oxobutan-2-yl)carbamate

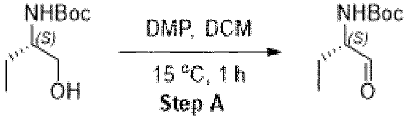

To a solution of (S)-tert-butyl (1-hydroxybutan-2-yl)carbamate (600 mg, 3.17 mmol, 1.0 eq.) in dichloromethane (20 mL) was added Dess-Martin Periodinane (1.88 g, 4.44 mmol, 1.4 eq.) at 0 °C. The mixture was stirred at 15 °C for 1 h. The mixture was diluted with water (12 mL) and EtOAc/Petroleum ether (1/15; V/V, 18 mL). The mixture was filtered and the filtrate was concentrated. The residue was redissolved in EtOAc/Petroleum ether (1/15; V/V, 18 mL) and washed with a mixed solution of saturated NaHCOs aqueous and 10% Sodium thiosulfate aqueous solution (1/1; V/V, 3 x 15 mL). The aqueous layers were back-extracted with EtOAc/Petroleum ether (1/10, 3 x 18 mL). The organic layers were combined and dried over Na₂SO₄, concentrated under vacuum to afford (S)-tert-butyl (1-oxobutan-2-yl)carbamate (355 mg, 53.8% yield) as a yellow solid.
LCMS: *(ESI)* (M+H-56)⁺ = 132.0.
¹H NMR: (400 MHz, CDCl₃) *δ* 9.58 (s, 1H), 5.07 (br s, 1H), 4.27 - 4.12 (m, 1H), 2.00 - 1.83 (m, 1H), 1.74 - 1.62 (m, 1H), 1.46 (s, 9H), 0.97 (t, *J=* 7.6 Hz, 3H).

### Step B: synthesis of (S,E)-tert-butyl (1-((2-oxo-2-(4-(5-(trifluoromethyl)pyrimidin -2-yl)piperazin-1-yl)ethoxy)imino)butan-2-yl)carbamate

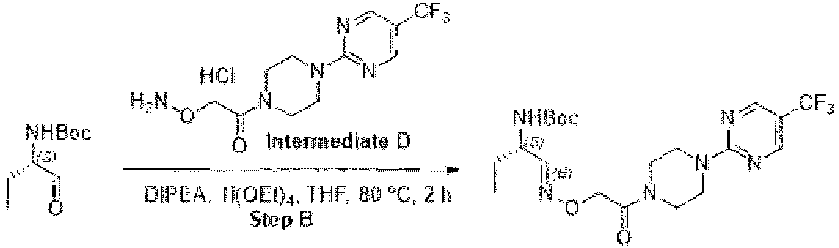

To a solution of (S)-tert-butyl (1-oxobutan-2-yl)carbamate (150 mg, 0.81 mmol, 1.0 eq.) in THF (10 mL) were added 2-(aminooxy)-1-(4-(5-(trifluoromethyl) pyrimidin-2-yl)piperazin-1-yl)ethanone hydrochloride (329 mg, 0.96 mmol, 1.2 eq.), tetraethoxy titanium (457 mg, 2.01 mmol, 2.5 eq.) and DIPEA (311 mg, 2.41 mmol, 3.0 eq.). The resulting mixture was stirred at 80 °C for 2 h. The mixture was cooled to room temperature and quenched with water (50 mL), extracted with EtOAc (3 x 80 mL). The organic layers were combined and washed with brine (100 mL), dried over anhydrous Na₂SO₄, and concentrated under vacuum. The residue was purified by column chromatography (EtOAc/Petroleum ether= 1/2; V/V) to afford (S,E)-tert-butyl (1-((2-oxo-2-(4-(5-(trifluoromethyl)pyrimidin-2-yl)piperazin-1-yl)ethoxy)imino)butan-2 -yl)carbamate (260 mg, 68.4% yield) as a white solid.
LCMS: *(ESI)* (M+H)⁺ = 475.2.

### Step C: synthesis of (S,E)-2-aminobutanal O-(2-oxo-2-(4-(5-(trifluoromethyl)pyrimidin -2-yl)piperazin-1-yl)ethyl) oxime

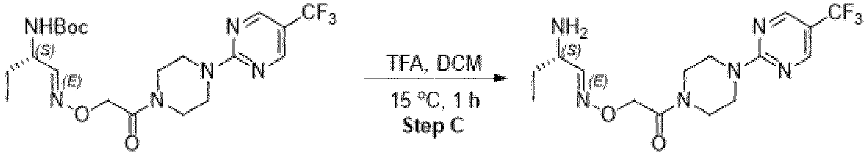

To a solution of (S,E)-tert-butyl (1-((2-oxo-2-(4-(5-(trifluoromethyl)pyrimidin-2-yl) piperazin-1-yl)ethoxy)imino)butan-2-yl)carbamate (220 mg, 0.46 mmol, 1.0 eq.) in DCM (6 mL) was added TFA (2 mL). The resulting mixture was stirred at 15 °C for 1 h. The reaction mixture was quenched with saturated NaHCOs aqueous solution (20 mL) and extracted with DCM (2 x 50 mL). The organic layers were combined and washed with brine (20 mL), dried over Na₂SO₄ and concentrated under vacuum to afford (S,E)-2-aminobutanal O-(2-oxo-2-(4-(5-(trifluoromethyl)pyrimidin-2-yl)piperazin-1-yl) ethyl) oxime (170 mg, 60.7% yield) as a yellow solid.
LCMS: *(ESI)* (M+H)⁺ = 375.2.
¹H NMR (400 MHz, CDCl₃) *δ* 8.55 (s, 2H), 7.48 (d, *J* = 6.0 Hz, 1H), 4.89 - 4.71 (m, 2H), 4.04 - 3.93 (m, 4H), 3.80 - 3.72 (m, 2H), 3.66 - 3.55 (m, 2H), 3.49 (q, *J* = 6.8 Hz, 1H), 1.83 (br s, 2H), 1.70 - 1.56 (m, 2H), 1.05 - 0.96 (m, 3H).

### Step D: synthesis of (S,E)-2-((6-oxo-5-(trifluoromethyl)-1-((2-(trimethylsilyl)ethoxy) methyl)-1,6-dihydropyridazin-4-yl)amino)butanal O-(2-oxo-2-(4-(5-(trifluoromethyl) pyrimidin-2-yl)piperazin-1-yl)ethyl) oxime

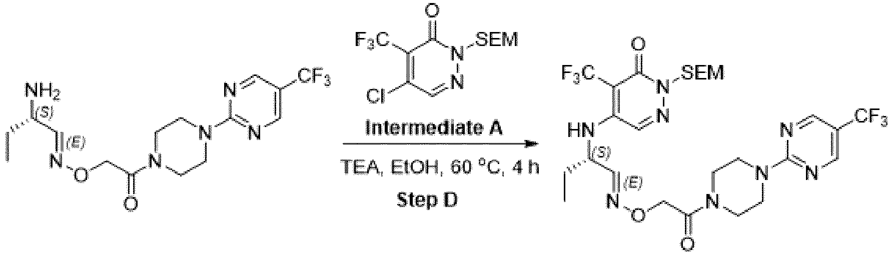

To a solution of (S,E)-2-aminobutanal O-(2-oxo-2-(4-(5-(trifluoromethyl) pyrimidin-2-yl)piperazin-1-yl)ethyl) oxime (170 mg, 0.45 mmol, 1.0 eq.) in EtOH (10 mL) were added 5-chloro-4-(trifluoromethyl)-2-((2-(trimethylsilyl)ethoxy)methyl) pyridazin-3(2H)-one (224 mg, 0.68 mmol, 1.5 eq.) and TEA (230 mg, 2.27 mmol, 5.0 eq.). The mixture was stirred at 60 °C for 4 h. The mixture was cooled to room temperature, diluted with water (100 mL) and extracted with ethyl acetate (2 x 100 mL.). The organic layers were washed with brine (100 mL), dried over Na₂SO₄, concentrated under vacuum. The residue was purified by column chromatography (EtOAc/Petroleum ether= 1/2; V/V) to afford (S,E)-2-((6-oxo-5-(trifluoromethyl)-1-((2-(trimethylsilyl) ethoxy)methyl)-1,6-dihydropyridazin-4-yl)amino)butanal O-(2-oxo-2-(4-(5-(trifluoro methyl)pyrimidin-2-yl)piperazin-1-yl)ethyl) oxime (110 mg, 34.1%) as a yellow oil.
LCMS: *(ESI)* (M+H-28)⁺ = 638.6.

### Step E: (S,E)-2-((6-oxo-5-(trifluoromethyl)-1,6-dihydropyridazin-4-yl)amino)butanal O-(2-oxo -2-(4-(5-(trifluoromethyl)pyrimidin-2-yl)piperazin-1-yl)ethyl) oxime

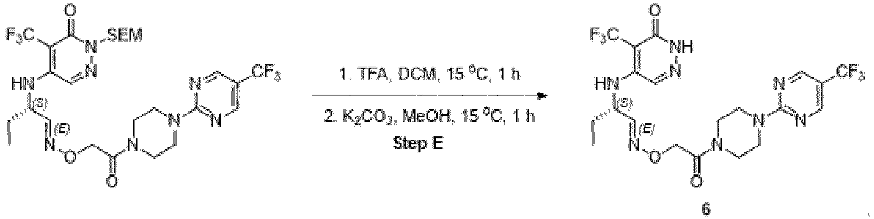

To a solution of (S,E)-2-((6-oxo-5-(trifluoromethyl)-1-((2-(trimethylsilyl)ethoxy) methyl)-1,6-dihydropyridazin-4-yl)amino)butanal O-(2-oxo-2-(4-(5-(trifluoromethyl) pyrimidin-2-yl)piperazin-1-yl)ethyl) oxime (100 mg, 0.15 mmol, 1.0 eq.) in DCM (3 mL) was added TFA (1 mL). The mixture was stirred at 15 °C for 1 h. The mixture was neutralized with saturated NaHCOs aqueous solution (50 mL) and extracted with DCM (3 x 80 mL). The organic layers were combined and dried over Na₂SO₄, concentrated under vacuum. The residue was redissolved in MeOH (4 mL) and potassium carbonate (77 mg, 0.55 mmol, 3.6 eq.) was added. The mixture was stirred at 15 °C for another 1 h. The mixture was filtered and the filtrate was purified by prep-HPLC to afford (S,E)-2-((6-oxo-5-(trifluoromethyl)-1,6-dihydropyridazin-4-yl)amino)butanal O-(2-oxo -2-(4-(5-(trifluoromethyl)pyrimidin-2-yl)piperazin-1-yl)ethyl) oxime (14.66 mg, 18.1% yield) as a white solid.
LCMS: *(ESI)* (M+H)⁺=536.7.
¹H NMR (400 MHz, CDCl₃) *δ* 11.02 (s, 1H), 8.52 (s, 2H), 7.72 (s, 1H), 7.46 (d, *J* = 6.0 Hz, 1H), 5.82 - 5.73 (m, 1H), 4.84 - 4.76 (m, 2H), 4.29 - 4.20 (m, 1H), 4.01 - 3.88 (m, 4H), 3.76 - 3.68 (m, 2H), 3.56 - 3.47 (m, 2H), 1.93 - 1.80 (m, 2H), 1.04 (t, *J* = 7.2 Hz, 3H).

### Example 7:

### (S,E)-2-(4-(2-(((2-((6-oxo-5-(trifluoromethyl)-1,6-dihydropyridazin-4-yl)amino)propyli dene)amino)oxy)acetyl)piperazin-1-yl)pyrimidine-5-carbonitrile

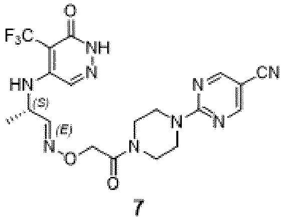

### Step A: synthesis of methyl 2-(((tert-butoxycarbonyl)amino)oxy)acetate

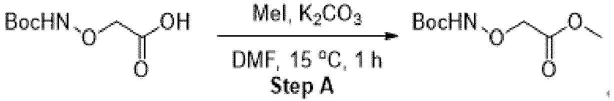

To a solution of 2-(((tert-butoxycarbonyl)amino)oxy)acetic acid (6.00 g, 31.38 mmol, 1.0 eq.) and K₂CO₃ (5.21 g, 37.66 mmol, 1.2 eq.) in DMF (50 mL) was added iodomethane (4.45 g, 31.38 mmol, 1.0 eq.). The mixture was stirred at 15 °C for 1h. The reaction was quenched with water (100 mL) and extracted with EtOAc (3 x 60 mL). The organic layers were combined and washed with brine (40 mL), dried over anhydrous Na₂SO₄ and concentrated under reduced pressure. The residue was purified by column chromatography (Petroleum ether/EtOAc= 2/1; V/V) to afford methyl 2-(((tert-butoxycarbonyl)amino)oxy)acetate (5.08 g, 78.9%) as a colorless oil.
LCMS: *(ESI)* (M+H-56)⁺ = 150.2.
¹H NMR: (400 MHz, CDCl₃) *δ* 7.82 (br s, 1H), 4.48 (s, 2H), 3.82 (s, 3H), 1.51 (s, 9H).

### Step B: synthesis of methyl 2-(aminooxy)acetate hydrochloride

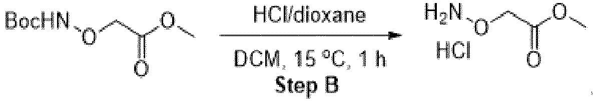

To a solution of methyl 2-(((tert-butoxycarbonyl)amino)oxy)acetate (2.85 g, 13.89 mmol, 1.0 eq.) in DCM (10 mL) was added HCl/dioxane (4M, 50 mL). The mixture was stirred at 15 °C for 1h. The mixture was concentrated under reduced pressure to afford methyl 2-(aminooxy)acetate hydrochloride (1.96 g, 99.9%) as a white solid.
LCMS: *(ESI)* (M+H)⁺ = 106.2.

### Step C: synthesis of methyl (S,E)-6,10,10-trimethyl-8-oxo-3,9-dioxa-4,7-diazaundec -4-enoate

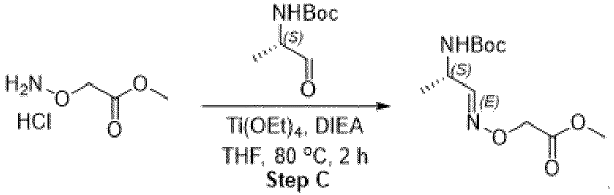

To a mixture of methyl 2-(aminooxy)acetate hydrochloride (1.96 g, 13.85 mmol, 1.2 eq.) in THF (120 mL) were added DIPEA (5.7 mL, 34.62 mmol, 3.0 eq.), (S)-tert-butyl (1-oxopropan-2-yl)carbamate (2.00 g, 11.54 mmol, 1.0 eq.) and tetraethoxy titanium (2.63 g, 11.54 mmol, 1.0 eq.). The mixture was stirred at 80 °C for 2 h. The reaction was then quenched with water (100 mL) and extracted with EtOAc (3 x 100 mL). The organic layers were combined and washed with brine (100 mL), dried over anhydrous Na₂SO₄ and concentrated under reduced pressure. The residue was purified by column chromatography (Petroleum ether/EtOAc= 2/1) to afford methyl (S,E)-6,10,10-trimethyl-8-oxo-3,9-dioxa-4,7-diazaundec-4-enoate (1.69 g, 56.3%) as a white solid.
LCMS: *(ESI)* (M+H-100)⁺ = 161.2.
¹H NMR: (400 MHz, CDCl₃) *δ* 7.59 - 7.49 (m, 1H), 5.01 - 4.80 (br, 1H), 4.63 (s, 2H), 4.33 - 4.26 (m, 1H), 3.81 (s, 3H), 1.48 (s, 9H), 1.34 (d, *J* = 6.8 Hz, 3H).

### Step D: synthesis of ((((6S,7E)-2,2,6-trimethyl-4-oxo-5-aza-3-oxaheptan-7-ylidene) amino)oxy)acetic acid (Intermediate F)

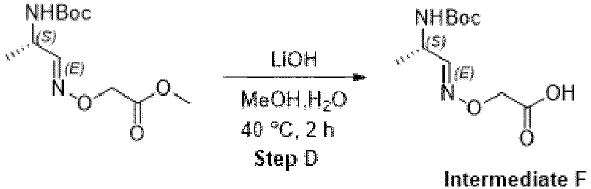

To a solution of methyl (S,E)-6,10,10-trimethyl-8-oxo-3,9-dioxa-4,7-diazaundec -4-enoate (900 mg, 3.46 mmol, 1.0 eq.) in MeOH (30 mL) and H₂O (5 mL) was added LiOH (124 mg, 5.19 mmol, 1.5 eq.). The mixture was stirred at 40 °C for 2 h. The mixture was cooled to room temperature and diluted with water (40 mL). The mixture was acidified pH 3 with 1M HCl aqueous and extracted with DCM (3 x 50 mL). The organic layers were combined and washed with brine (80 mL), dried over Na₂SO₄ and concentrated under reduced pressure to afford ((((6S,7E)-2,2,6-trimethyl-4-oxo-5-aza -3-oxaheptan-7-ylidene)amino)oxy)acetic acid (851 mg, 99.9%) as a yellow oil.
LCMS: *(ESI)* (M+Na)⁺ = 269.2.

### Step E: synthesis of (S,E)-tert-butyl (1-((2-(4-(5-cyanopyrimidin-2-yl)piperazin-1-yl)-2 -oxoethoxy)imino)propan-2-yl)carbamate

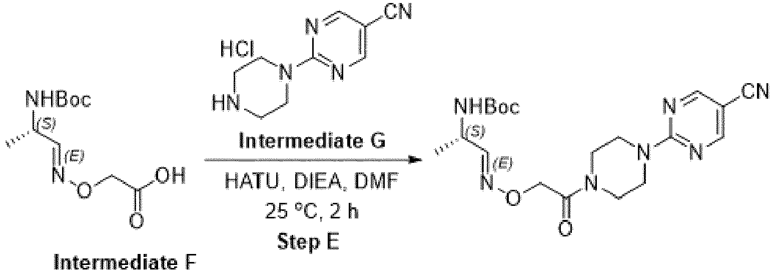

To a solution of ((((6S,7E)-2,2,6-trimethyl-4-oxo-5-aza-3-oxaheptan-7-ylidene)amino)oxy)acetic acid (135 mg, 0.55 mmol, 1.0 eq.) and 2-(piperazin-1-yl)pyrimidine-5-carbonitrile hydrochloride (136 mg, 0.60 mmol, 1.1 eq.) in DMF (8 mL) were added DIPEA (0.54 mL, 3.30 mmol, 6.0 eq.), HATU (250 mg, 0.66 mmol, 1.2 eq.) at room temperature. The mixture was stirred at 25 °C for 2 h. The reaction was quenched with water (40 mL) and extracted with EtOAc (3 x 40 mL). The organic layers were combined and washed with brine (50 mL), dried over Na₂SO₄ and concentrated under reduced pressure. The residue was purified by column chromatography (Petroleum ether/EtOAc= 1/1) to afford (S,E)-tert-butyl (1-((2-(4-(5-cyanopyrimidin-2-yl)piperazin-1-yl)-2-oxoethoxy)imino)propan-2-yl)carba mate (180 mg, 78.7%) as a yellow oil.
LCMS: *(ESI)* (M+H-100)⁺ = 318.2.
¹H NMR: (400 MHz, CDCl₃) *δ* 8.52 (d, *J* = 2.8 Hz, 2H), 7.50 (d, *J* = 4.4 Hz, 1H), 4.82 - 4.77 (m, 1H), 4.75 (s, 2H), 4.35 (br s, 1H), 3.98 - 3.93 (m, 4H), 3.76 - 3.69 (m, 2H), 3.62 - 3.55 (m, 2H), 1.43 (s, 9H), 1.31 (d, *J* = 6.8 Hz, 3H).

### Step F: synthesis of (S,E)-2-(4-(2-(((2-aminopropylidene)amino)oxy)acetyl)piperazin -1-yl)pyrimidine-5-carbonitrile

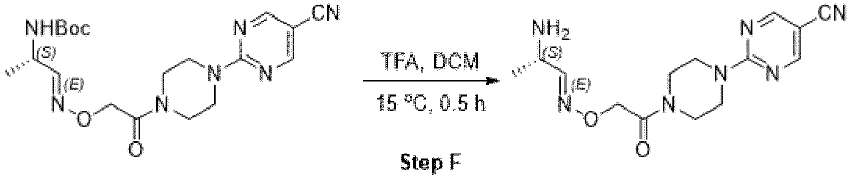

To a solution of (S,E)-tert-butyl (1-((2-(4-(5-cyanopyrimidin-2-yl)piperazin-1-yl) -2-oxoethoxy)imino)propan-2-yl)carbamate (210 mg, 0.52 mmol, 1.0 eq.) in DCM (8 mL) was added TFA (2 mL). The resulting solution was stirred at 15 °C for 0.5 h. The mixture was quenched with saturated NaHCOs (40 mL) and extracted with DCM (3 x 50 mL). The organic layers were combined and dried over Na₂SO₄, concentrated under vacuum to afford (S,E)-2-(4-(2-(((2-aminopropylidene)amino)oxy)acetyl)piperazin-1-yl) pyrimidine-5-carbonitrile (150 mg, 95.0%) as a yellow oil.
LCMS: *(ESI)* (M+H)⁺ = 318.2.

### Step G: synthesis of (S,E)-2-(4-(2-(((2-((6-oxo-5-(trifluoromethyl)-1-((2-(trimethylsilyl) ethoxy)methyl)-1,6-dihydropyridazin-4-yl)amino)propylidene)amino)oxy)acetyl)pipera zin-1-yl)pyrimidine-5-carbonitrile

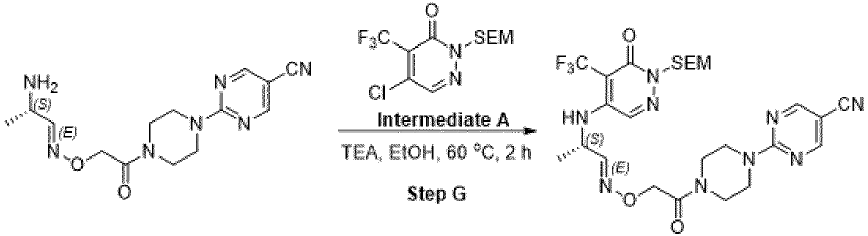

To a solution of (S,E)-2-(4-(2-(((2-aminopropylidene)amino)oxy)acetyl) piperazin-1-yl)pyrimidine-5-carbonitrile (64 mg, 0.20 mmol, 1.0 eq.) and 5-chloro-4-(trifluoromethyl)-2-((2-(trimethylsilyl)ethoxy)methyl)pyridazin-3 (2H)-one (73 mg, 0.22 mmol, 1.1 eq.) in EtOH (3 mL) was added TEA (41 mg, 0.40 mmol, 2.0 eq.). The mixture was heated to 60 °C and stirred for 2 h. The reaction was then quenched with water (20 mL) and extracted with EtOAc (3 x 30 mL). The organic layers were combined and washed with brine (30 mL), dried over Na₂SO₄ and concentrated under reduced pressure. The residue was purified by column chromatography (Petroleum ether/EtOAc= 1/3) to afford (S,E)-2-(4-(2-(((2-((6-oxo-5-(trifluoromethyl)-1-((2-(trimethylsilyl)ethoxy)methyl)-1,6-dihydropyridazin-4-yl)amino)propylidene)amino)oxy)acetyl)piperazin-1-yl)pyrimidine-5-carbonitrile (56 mg, 45.5%) as a yellow oil.
LCMS: *(ESI)* (M+H-28)⁺ = 582.2.

### Step H: synthesis of (S,E)-2-(4-(2-(((2-((6-oxo-5-(trifluoromethyl)-1,6-dihydropyridazin-4-yl)amino)propylidene)amino)oxy)acetyl)piperazin-1-yl)pyrimidine-5-carbonitrile

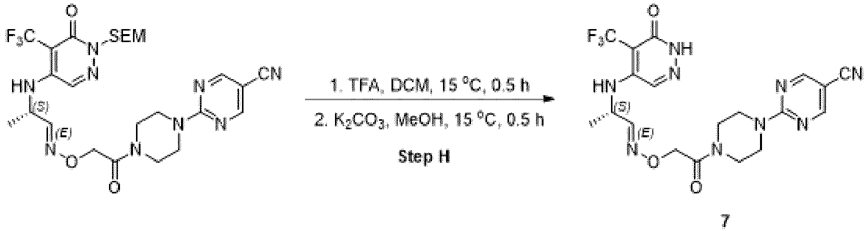

To a solution of (S,E)-2-(4-(2-(((2-((6-oxo-5-(trifluoromethyl)-1-((2-(trimethylsilyl) ethoxy)methyl)-1,6-dihydropyridazin-4-yl)amino)propylidene)amino)oxy)acetyl)pipera zin-1-yl)pyrimidine-5-carbonitrile (56 mg, 0.09 mmol, 1.0 eq.) in DCM (3 mL) was added TFA (1 mL). The resulting solution was stirred at 15 °C for 0.5 h. The mixture was concentrated under vacuum. The residue was redissolved in MeOH (1 mL) and K₂CO₃ (10 mg, 0.07 mmol) was added. The mixture was stirred at 15 °C for 0.5 h. The mixture was filtered and the filtrate was purified by prep-HPLC to afford (S,E)-2-(4-(2-(((2-((6-oxo-5-(trifluoromethyl)-1,6-dihydropyridazin-4-yl)amino)propyli dene)amino)oxy)acetyl)piperazin-1-yl)pyrimidine-5-carbonitrile (18.42 mg, 41.8%) as a white solid.
LCMS: *(ESI)* (M+H)⁺ = 480.2.
¹H NMR: (400 MHz, CDCl₃) *δ* 10.82 (s, 1H), 8.53 (s, 2H), 7.70 (s, 1H), 7.49 (d, *J* = 5.6 Hz, 1H), 5.89 - 5.76 (m, 1H), 4.87 - 4.71 (m, 2H), 4.50 - 4.37 (m, 1H), 4.05 - 3.88 (m, 4H), 3.79 - 3.67 (m, 2H), 3.59 - 3.48 (m, 2H), 1.52 (d, *J* = 6.8 Hz, 3H).

### Example 8:

### (S,E)-5-((3-((2-oxo-2-(4-(5-(trifluoromethyl)pyrimidin-2-yl)piperazin-1-yl)ethoxy)imin o)butan-2-yl)amino)-4-(trifluoromethyl)pyridazin-3(2H)-one

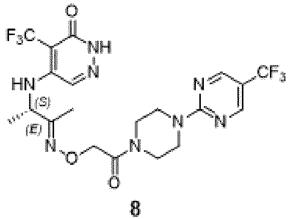

### Step A: synthesis of (S)-tert-butyl (3-oxobutan-2-yl)carbamate

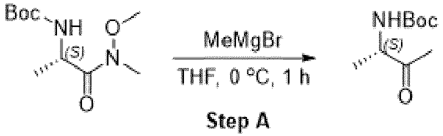

To a solution of (S)-tert-butyl (1-(methoxy(methyl)amino)-1-oxopropan-2-yl) carbamate (581 mg, 2.50 mmol, 1.0 eq.) in THF (12 mL) was added MeMgBr (1M, 7.5 mL, 7.50 mmol, 3.0 eq.) slowly at 0 °C in ice-water bath under N₂ atmosphere. The mixture was stirred at 0 °C for 1 h. The mixture was quenched slowly with saturated NH₄Cl aqueous solution (30 mL) and extracted with EtOAc (2 x 50 mL). The organic layers were combined and dried over Na₂SO₄, concentrated under vacuum. The residue was purified by silica gel chromatography (EtOAc/Petroleum= 1/4; V/V) to afford (S)-tert-butyl (3-oxobutan-2-yl)carbamate (400 mg, 85.4% yield) as a light yellow solid. LCMS: *(ESI)* (M+H-56)⁺ = 132.2.
¹H NMR: (400 MHz, CDCl₃) *δ* 5.25 (br s, 1H), 4.39 - 4.23 (m, 1H), 2.20 (s, 3H), 1.44 (s, 9H), 1.34 (d, *J* = 7.2 Hz, 3H).

### Step B: synthesis of (S-E)-tert-butyl (3-((2-oxo-2-(4-(5-(trifluoromethyl)pyrimidin-2-yl) piperazin-1-yl)ethoxy)imino)butan-2-yl)carbamate

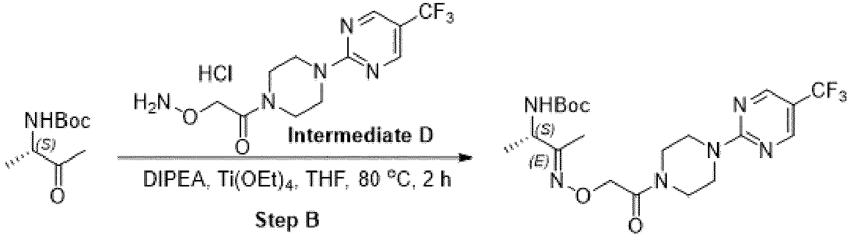

To a suspension of 2-(aminooxy)-1-(4-(5-(trifluoromethyl)pyrimidin-2-yl) hexahydropyrazin-1-yl)ethanone hydrochloride (205 mg, 0.60 mmol, 1.0 eq.) in THF (8 mL) were added DIPEA (233 mg, 1.80 mmol, 3.0 eq.), (S)-tert-butyl (3-oxobutan-2-yl)carbamate (135 mg, 0.72 mmol, 1.2 eq.) and tetraethoxy titanium (342 mg, 1.50 mmol, 2.5 eq.). The mixture was stirred at 80 °C for 2 h. The mixture was cooled to room temperature and quenched with saturated NaHCO₃ aqueous solution (50 mL), extracted with DCM (3 x 80 mL). The organic layers were combined and dried over Na₂SO₄, concentrated under vacuum. The residue was purified by silica gel chromatography (MeOH/DCM= 1/33; V/V) to afford (S,E)-tert-butyl (3-((2-oxo-2-(4-(5-(trifluoromethyl)pyrimidin-2-yl)piperazin-1-yl)ethoxy)imino)butan-2 -yl)carbamate (240 mg, 84.3% yield) as a light yellow oil.
LCMS: *(ESI)* (M+H-100)⁺ = 374.8.
¹H NMR: (400 MHz, CDCl₃) *δ* 8.51 (s, 2H), 5.03 (br s, 1H), 4.76 (s, 2H), 4.31 - 4.19 (m, 1H), 3.97 - 3.90 (m, 4H), 3.75 - 3.67 (m, 2H), 3.63 - 3.56 (m, 2H), 1.90 (s, 3H), 1.42 (s, 9H), 1.29 (d, *J* = 6.8 Hz, 3H).

### Step C: synthesis of (S,E)-2-(((3-aminobutan-2-ylidene)amino)oxy) -1-(4-(5-(trifluoromethyl)pyrimidin-2-yl)piperazin-1-yl)ethanone

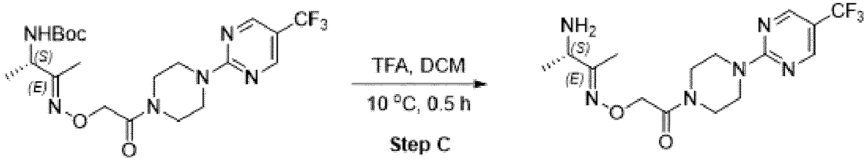

To a solution of (S,E)-tert-butyl (3-((2-oxo-2-(4-(5-(trifluoromethyl)pyrimidin-2-yl) piperazin-1-yl)ethoxy)imino)butan-2-yl)carbamate (250 mg, 0.53 mmol, 1.0 eq.) in DCM (4 mL) was added TFA (1 mL). The mixture was stirred at 10 °C for 0.5 h. The excess TFA was neutralized with saturated NaHCOs aqueous solution (40 mL) and the mixture was extracted with DCM (3 x 60 mL). The organic layers were combined and dried over Na₂SO₄, concentrated under vacuum to afford (S,E)-2-(((3-aminobutan-2-ylidene)amino)oxy)-1-(4-(5-(trifluoromethyl)pyrimidin-2-yl) piperazin-1-yl)ethanone (197 mg, 99.9% yield) as a light yellow oil.
LCMS: *(ESI)* (M+H)⁺ = 374.8.

### Step D: synthesis of (S,E)-5-((3-((2-oxo-2-(4-(5-(trifluoromethyl)pyrimidin-2-yl) piperazin-1-yl)ethoxy)imino)butan-2-yl)amino)-4-(trifluoromethyl)-2-((2-(trimethylsilyl )ethoxy)methyl)pyridazin-3(2H)-one

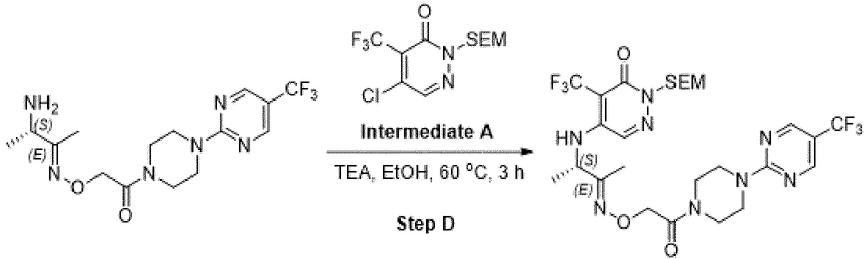

To a solution of (S,E)-2-(((3-aminobutan-2-ylidene)amino)oxy)-1-(4-(5-(trifluoromethyl)pyrimidin-2-yl)piperazin-1-yl)ethanone (197 mg, 0.53 mmol, 1.0 eq.) in EtOH (8 mL) were added TEA (213 mg, 2.11 mmol, 4.0 eq.) and followed by 5-chloro-4-(trifluoromethyl)-2-((2-(trimethylsilyl)ethoxy)methyl)pyridazin-3 (2H)-one (346 mg, 1.05 mmol, 2.0 eq.). The mixture was heated to 60 °C and stirred for 3 h. The mixture was cooled to room temperature and concentrated under vacuum. The residue was purified by silica gel chromatography (MeOH/DCM= 1/33; V/V) to afford (S,E)-5-((3-((2-oxo-2-(4-(5-(trifluoromethyl)pyrimidin-2-yl)piperazin-1-yl)ethoxy)imin o)butan-2-yl)amino)-4-(trifluoromethyl)-2-((2-(trimethylsilyl)ethoxy)methyl)pyridazin-3(2H)-one (190 mg, 30.9% yield) as a light yellow oil.
LCMS: *(ESI)* (M+H)⁺ = 666.6.

### Step E: Synthesis of (S,E)-5-((3-((2-oxo-2-(4-(5-(trifluoromethyl)pyrimidin-2-yl) piperazin-1-yl)ethoxy)imino)butan-2-yl)amino)-4-(trifluoromethyl)pyridazin-3(2H)-one

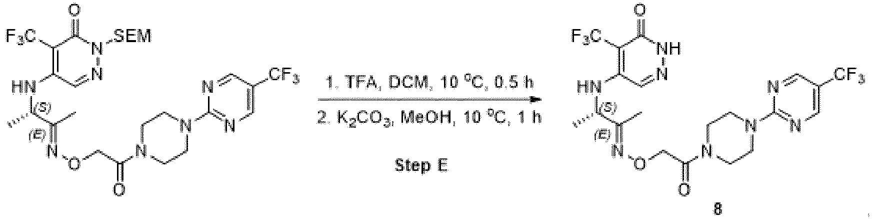

To a solution of (S,E)-5-((3-((2-oxo-2-(4-(5-(trifluoromethyl)pyrimidin-2-yl)piperazin-1-yl)ethoxy)imin o)butan-2-yl)amino)-4-(trifluoromethyl)-2-((2-(trimethylsilyl)ethoxy)methyl)pyridazin-3(2H)-one (187 mg, 0.16 mmol, 1.0 eq.) in DCM (3 mL) was added TFA (1 mL). The mixture was stirred at 10 °C for 0.5 h. The mixture was neutralized with saturated NaHCOs aqueous solution (30 mL) and extracted with DCM (3 x 50 mL). The organic layers were combined and dried over Na₂SO₄, concentrated under vacuum. The residue was redissolved in MeOH (3 mL) and K₂CO₃ (30 mg, 0.22 mmol) was added. The mixture was stirred at 10 °C for another 1 h. The mixture was filtered and the filtrate was purified by prep-HPLC (FA) to afford (S,E)-5-((3-((2-oxo-2-(4-(5-(trifluoromethyl) pyrimidin-2-yl)piperazin-1-yl)ethoxy)imino)butan-2-yl)amino)-4-(trifluoromethyl)pyrid azin-3(2H)-one (36.39 mg, 41.8% yield) as a yellow solid.
LCMS: *(ESI)* (M+H)⁺ = 537.2.
¹H NMR: (400 MHz, CDCl₃) *δ* 11.36 (br s, 1H), 8.51 (s, 2H), 7.70 (s, 1H), 6.11 - 6.02 (m, 1H), 4.85 - 4.75 (m, 2H), 4.39 - 4.29 (m, 1H), 3.98 - 3.88 (m, 4H), 3.78 - 3.65 (m, 2H), 3.60 - 3.44 (m, 2H), 1.95 (s, 3H), 1.49 (d, *J* = 6.8 Hz, 3H).

### Example 9:

### (S,E)-5-((2-((2-oxo-2-(4-(5-(trifluoromethyl)pyrimidin-2-yl)piperazin-1-yl)ethoxy)imin o)cyclopentyl)amino)-4-(trifluoromethyl)pyridazin-3(2H)-one

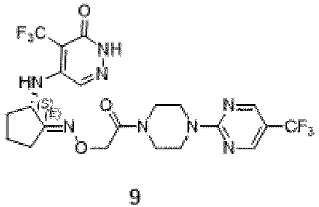

### Step A: synthesis of tert-butyl ((1S,2S)-2-hydroxycyclopenlyl)carbamate

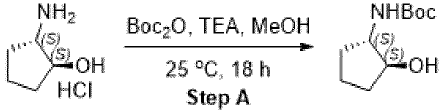

To a solution of (1S,2S)-2-aminocyclopentanol hydrochloride (2.00 g, 14.53 mmol, 1.0 eq.) in MeOH (40 mL) were added TEA (5.88 g, 58.12 mmol, 4.0 eq.) and Boc₂O (327 mg, 21.80 mmol, 1.5 eq.). The mixture was stirred at 25 °C for 18 h. The reaction was quenched with water (40 mL) and extracted with DCM (3 x 50 mL). The organic layers were combined and washed with brine (200 mL), dried over anhydrous Na₂SO₄ and concentrated under reduced pressure. The residue was purified by column chromatography (Petroleum ether/EtOAc= 1/1; V/V) to afford tert-butyl ((1S,2S)-2-hydroxycyclopentyl)carbamate (2.80 g, 95.7%) as a white solid.
LCMS: *(ESI)* (M+H-56)⁺ = 146.2.
¹H NMR: (400 MHz, CDCl₃) *δ* 4.72 (s, 1H), 4.01 - 3.95 (m, 1H), 3.67 - 3.58 (m, 1H), 2.14 - 2.05 (m, 1H), 2.04 - 1.95 (m, 1H), 1.84 - 1.72 (m, 1H), 1.71 - 1.58 (m, 2H), 1.45 (s, 9H), 1.41 - 1.28 (m, 1H).

### Step B: synthesis of (S)-tert-butyl (2-oxocyclopentyl)carbamate

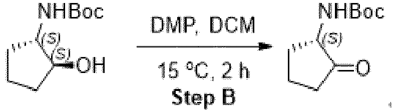

To a solution of tert-butyl ((1S,2S)-2-hydroxycyclopentyl)carbamate (1.00 g, 4.97 mmol, 1.0 eq.) in DCM (25 mL) was added Dess-Martin periodinane (4.22 g, 9.94 mmol, 2.0 eq.). The mixture was stirred at 15 °C for 2 h. The reaction was quenched with saturated NaHCO₃ aqueous solution (100 mL) and extracted with DCM (3 x 50 mL). The organic layers were combined and washed with brine (200 mL), dried over anhydrous Na₂SO₄ and concentrated under reduced pressure. The residue was purified by column chromatography (Petroleum ether/EtOAc= 1/1; V/V) to afford (S)-tert-butyl (2-oxocyclopentyl)carbamate (980 mg, 99.0%) as a colorless oil.
¹H NMR: (400 MHz, CDCl₃) *δ* 4.98 (s, 1H), 3.92 (s, 1H), 2.62 - 2.53 (m, 1H), 2.45 - 2.36 (m, 1H), 2.22 - 2.11 (m, 1H), 2.07 - 1.99 (m, 1H), 1.89 - 1.76 (m, 1H), 1.68 - 1.57 (m, 1H), 1.45 (s, 9H).

### Step C: synthesis of (S-E)-tert-butyl (2-((2-oxo-2-(4-(5-(trifluoromethyl)pyrimidin-2-yl) piperazin-1-yl)ethoxy)imino)cyclopentyl)carbamate

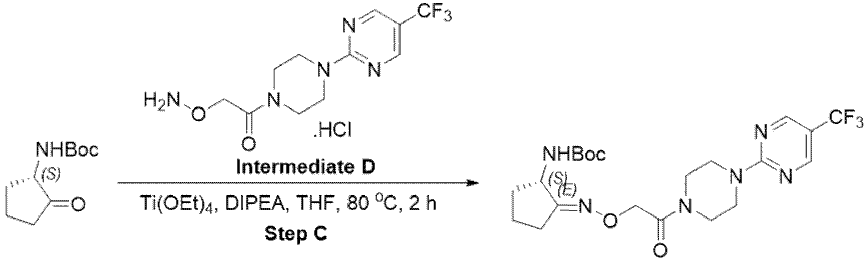

To mixture of 2-(aminooxy)-1-(4-(5-(trifluoromethyl)pyrimidin-2-yl)piperazin-1-yl)ethanone hydrochloride (229 mg, 0.67 mmol, 1.0 eq.) in THF (15 mL) were added DIPEA (260 mg, 2.01 mmol, 3.0 eq.), (S)-tert-butyl (2-oxocyclopentyl)carbamate (159 mg, 0.80 mmol, 1.2 eq.) and tetraethoxy titanium (383 mg, 1.68 mmol, 2.5 eq.). The mixture was stirred at 80 °C for 2 h. The mixture was cooled to room temperature and quenched with saturated NaHCOs aqueous solution (20 mL), extracted with DCM (3 x 50 mL). The organic layers were combined and dried over Na₂SO₄, concentrated under vacuum. The residue was purified by silica gel chromatography (DCM/MeOH= 20/1; V/V) to afford (S,E)-tert-butyl (2-((2-oxo-2-(4-(5-(trifluoromethyl)pyrimidin-2-yl)piperazin-1-yl) ethoxy)imino)cyclopentyl)carbamate (290 mg, 89.0%) as a light yellow oil.
LCMS: *(ESI)* (M+H-100)⁺ = 387.0.

### Step D: synthesis of (S,E)-2-(((2-aminocyclopenlylidene)amino)oxy)-1-(4-(5-(trifluoromethyl)pyrimidin-2-yl)piperazin-1-yl)ethanone

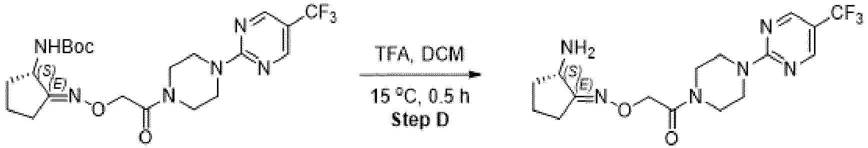

To a solution of (S,E)-tert-butyl (2-((2-oxo-2-(4-(5-(trifluoromethyl)pyrimidin-2-yl) piperazin-1-yl)ethoxy)imino)cyclopentyl)carbamate (290 mg, 0.60 mmol, 1.0 eq.) in DCM (9 mL) was added TFA (3 mL). The mixture was stirred at 15 °C for 0.5 h. The mixture was quenched with saturated NaHCOs aqueous solution (30 mL) and extracted with DCM (3 x 50 mL). The organic layers were combined and dried over Na₂SO₄, concentrated under reduced pressure to afford (S,E)-2-(((2-aminocyclopentylidene) amino)oxy)-1-(4-(5-(trifluoromethyl)pyrimidin-2-yl)piperazin-1-yl)ethanone (135 mg, 45.3%) as a white solid.
LCMS: *(ESI)* (M+H)⁺ = 387.2.

### Step E: Synthesis of (S,E)-5-((2-((2-oxo-2-(4-(5-(trifluoromethyl)pyrimidin-2-yl) piperazin-1-yl)ethoxy)imino)cyclopentyl)amino)-4-(trifluoromethyl)-2-((2-(trimethylsil yl)ethoxy)methyl)pyridazin-3(2H)-one

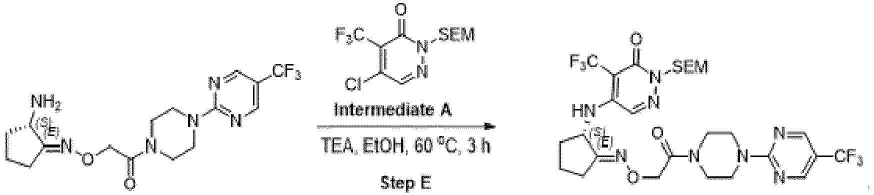

To a solution of (S,E)-2-(((2-aminocyclopentylidene)amino)oxy)-1-(4-(5-(trifluoromethyl)pyrimidin-2-y l)piperazin-1-yl)ethanone (151 mg, 0.39 mmol, 1.0 eq.) and 5-chloro-4-(trifluoromethyl)-2-((2-(trimethylsilyl)ethoxy)methyl)pyridazin-3(2H)-one (256 mg, 0.78 mmol, 2.0 eq.) in EtOH (5 mL) was added TEA (158 mg, 1.56 mmol, 4.0 eq.). The mixture was heated to 60 °C and stirred for 3 h. The reaction was cooled to room temperature and quenched with water (20 mL), extracted with EtOAc (3 x 20 mL). The organic layers were combined and washed with brine (40 mL), dried over Na₂SO₄ and concentrated under reduced pressure. The residue was purified by column chromatography (DCM/MeOH= 20/1; V/V) to afford (S,E)-5-((2-((2-oxo-2-(4-(5-(trifluoromethyl)pyrimidin-2-yl)piperazin-1-yl)ethoxy)imino)cyclopentyl)amino)-4-(trif luoromethyl)-2-((2-(trimethylsilyl)ethoxy)methyl)pyridazin-3(2H)-one (145 mg, 55.0%) as a yellow oil.
LCMS: *(ESI)* (M+H-28)⁺ = 651.2.

### Step F: synthesis of (S,E)-5-((2-((2-oxo-2-(4-(5-(trifluoromethyl)pyrimidin-2-yl) piperazin-1-yl)ethoxy)imino)cyclopentyl)amino)-4-(trifluoromethyl)pyridazin-3(2H) -one

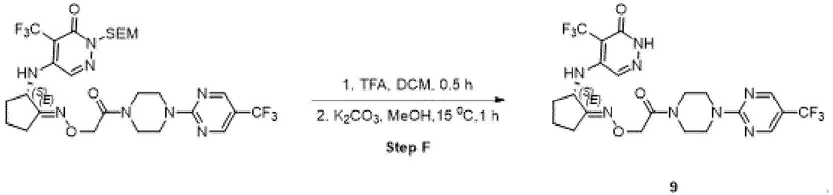

To a solution of (S,E)-5-((2-((2-oxo-2-(4-(5-(trifluoromethyl)pyrimidin-2-yl) piperazin-1-yl)ethoxy)imino)cyclopentyl)amino)-4-(trifluoromethyl)-2-((2-(trimethylsil yl)ethoxy)methyl)pyridazin-3(2H)-one (149 mg, 0.22 mmol, 1.0 eq.) in DCM (6 mL) was added TFA (2 mL). The mixture was stirred at 15 °C for 0.5 h. The mixture was concentrated under vacuum. The residue was redissolved in MeOH (2 mL) and K₂CO₃ (21 mg, 0.15 mmol) was added. The mixture was stirred at 15 °C for another 1 h. The mixture was filtered and the filtrate was purified by prep-HPLC to afford (S,E)-5-((2-((2-oxo-2-(4-(5-(trifluoromethyl)pyrimidin-2-yl)piperazin-1-yl)ethoxy)imin o)cyclopentyl)amino)-4-(trifluoromethyl)pyridazin-3(2H)-one (50.87 mg, 42.9%) as a white solid.
LCMS: *(ESI)* (M+H)⁺ = 549.2.
¹H NMR: (400 MHz, CDCl₃) *δ* 10.61 (s, 1H), 8.55 (s, 2H), 7.74 (s, 1H), 6.05 - 5.98 (m, 1H), 4.87 - 4.79 (m, 2H), 4.49 - 4.42 (m, 1H), 4.01 - 3.92 (m, 4H), 3.79 - 3.70 (m, 2H), 3.60 - 3.51 (m, 2H), 2.74 - 2.65 (m, 2H), 2.53 - 2.43 (m, 1H), 2.12 - 2.01 (m, 1H), 1.92 - 1.80 (m, 1H), 1.77 - 1.68 (m, 1H).

### Example 10:

### (S,E)-2-cyclopropyl-2-((6-oxo-5-(trifluoromethyl)-L6-dihydropyridazin-4-yl)amino)ace taldehyde O-(2-oxo-2-(4-(5-(trifluoromethyl)pyrimidin-2-yl)piperazin-1-yl)ethyl) oxime

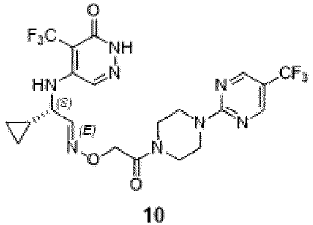

### Step A: synthesis of (S)-methyl 2-amino-2-cyclopropylacetate hydrochloride

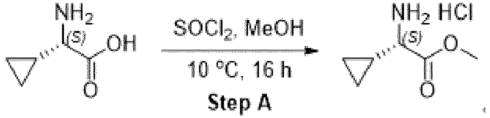

To a solution of (S)-2-amino-2-cyclopropylacetic acid (806 mg, 7.00 mmol, 1.0 eq.) in MeOH (9 mL) was added slowly SOCl₂ (3.1 mL, 42.01 mmol, 6.0 eq.) at 0 °C in ice-water bath. The mixture was stirred at 10 °C for 16 h. The mixture was concentrated under vacuum to afford crude (S)-methyl 2-amino-2-cyclopropylacetate hydrochloride (1.16 g, 99.1%) as a light yellow solid.
LCMS: *(ESI)* (M+H)⁺ = 130.0.
¹H NMR: (400 MHz, CD₃OD) *δ* 3.89 (s, 3H), 3.40 (d, *J =* 10.0 Hz, 1H), 1.25 - 1.14 (m, 1H), 0.89 - 0.77 (m, 2H), 0.76 - 0.68 (m, 1H), 0.65 - 0.57 (m, 1H).

### Step B: synthesis of (S)-methyl 2-((tert-butoxycarbonyl)amino)-2-cyclopropylacetate

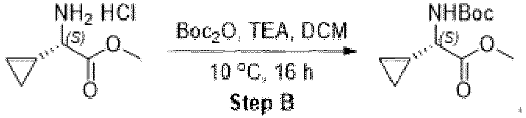

To a solution of (S)-methyl 2-amino-2-cyclopropylacetate hydrochloride (1.16 g, 7.00 mmol, 1.0 eq.) in DCM (20 mL) were added TEA (4.9 mL, 35.02 mmol, 5.0 eq.) and followed by BoczO (1.83 g, 8.40 mmol, 1.2 eq.) at 0 °C in ice-water bath. The mixture was stirred at 10 °C for 16 h. The mixture was diluted with saturated aqueous solution (80 mL) and extracted with EtOAc (2 x 80 mL). The organic layers were combined and washed with saturated NH₄Cl aqueous solution (80 mL), dried over Na₂SO₄ and concentrated under vacuum. The residue was purified by silica gel chromatography (EtOAc/Petroleum ether= 1/9; V/V) to afford (S)-methyl 2-((tert -butoxycarbonyl)amino)-2-cyclopropylacetate (1.56 g, 96.9%) as a light yellow oil.
LCMS: *(ESI)* (M+H-56)⁺ = 174.0.
¹H NMR: (400 MHz, CDCl₃) *δ* 5.11 (br s, 1H), 3.85 - 3.80 (m, 1H), 3.79 (s, 3H), 1.47 (s, 9H), 1.13 - 1.03 (m, 1H), 0.64 - 0.47 (m, 3H), 0.46 - 0.37 (m, 1H).

### Step C: synthesis of (S)-tert-butyl (1-cyclopropyl-2-oxoethyl)carbamate

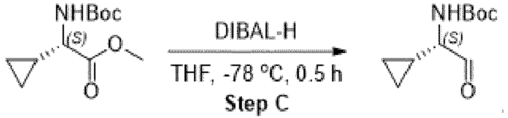

To a mixture of (S)-methyl 2-((tert-butoxycarbonyl)amino)-2-cyclopropylacetate (229 mg, 1.00 mmol, 1.0 eq.) in anhydrous THF (3 mL) under N₂ atmosphere was added DIBAL-H (1M, 1.40 mL, 1.40 mmol, 1.4 eq.) dropwise at -78 °C in dry ice-EtOH bath. The mixture was stirred at -78 °C for 0.5 h. The mixture was quenched with saturated NH₄Cl aqueous solution (50 mL) and extracted with a mixed solvent of Petroleum ether and EtOAc (1/1; V/V, 3 x 50 mL). The organic layers were combined and washed with saturated NH₄Cl aqueous solution (2 x 50 mL), dried over Na₂SO₄, concentrated under vacuum to afford crude (S)-tert-butyl (1-cyclopropyl-2-oxoethyl) carbamate (230 mg, 57.7% yield) as a yellow oil.
LCMS: *(ESI)* (M+H-56)⁺ = 144.2.

### Step D: synthesis of (S,E)-tert-butyl (1-cyclopropyl-2-((2-oxo-2-(4-(5-(trifluoromethyl) pyrimidin-2-yl)piperazin-1-yl)ethoxy)imino)ethyl)carbamate

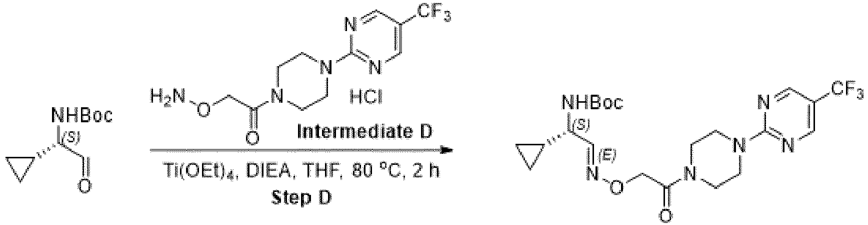

To mixture of 2-(aminooxy)-1-(4-(5-(trifluoromethyl)pyrimidin-2-yl)hexahydropyrazin-1-yl)ethanone hydrochloride (150 mg, 0.44 mmol, 1.0 eq.) in THF (7 mL) were added DIPEA (175 mg, 1.32 mmol, 3.0 eq.), (S)-tert-butyl (1-cyclopropyl-2-oxoethyl)carbamate (175 mg, 0.44 mmol, 1.0 eq.) and tetraethoxy titanium (250 mg, 1.10 mmol, 2.5 eq.). The mixture was stirred at 80 °C for 2 h. The mixture was cooled to room temperature and quenched with saturated NaHCO₃ aqueous solution (50 mL), extracted with EtOAc (2 x 80 mL). The organic layers were combined and dried over Na₂SO₄, concentrated under vacuum. The residue was purified by silica gel chromatography (EtOAc/Petroleum ether= 9/11; V/V) to afford (S,E)-tert-butyl (1-cyclopropyl-2-((2-oxo-2-(4-(5-(trifluoromethyl) pyrimidin-2-yl)piperazin-1-yl)ethoxy)imino)ethyl)carbamate (133 mg, 62.3%) as a white solid.
LCMS: *(ESI)* (M+H-100)⁺ = 387.2.
¹H NMR: (400 MHz, CDCl₃) *δ* 8.54 (s, 2H), 7.55 (d, *J* = 5.2 Hz, 1H), 5.06 - 4.89 (m, 1H), 4.84 - 4.74 (m, 2H), 4.05 - 3.89 (m, 4H), 3.83 - 3.67 (m, 3H), 3.66 - 3.55 (m, 2H), 1.45 (s, 9H), 1.07 - 0.94 (m, 1H), 0.66 - 0.55 (m, 2H), 0.53 - 0.44 (m, 1H), 0.39 - 0.29 (m, 1H).

### Step E: synthesis of (S,E)-2-amino-2-cyclopropylacetaldehyde O-(2-oxo-2-(4-(5-(trifluoromethyl)pyrimidin-2-yl)piperazin-1-yl)ethyl) oxime

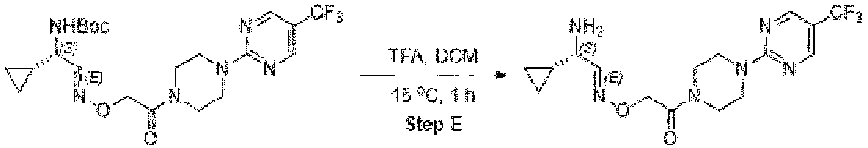

To a solution of (S,E)-tert-butyl (1-cyclopropyl-2-((2-oxo-2-(4-(5-(trifluoromethyl)pyrimidin-2-yl)piperazin-1-yl)ethoxy )imino)ethyl)carbamate (163 mg, 0.34 mmol, 1.0 eq.) in DCM (8 mL) was added TFA (2 mL). The mixture was stirred at 15 °C for 1 h. The mixture was quenched with saturated NaHCO₃ aqueous solution (40 mL) and extracted with DCM (3 x 35 mL). The organic layers were combined and dried over Na₂SO₄, concentrated under vacuum to afford (S,E)-2-amino-2-cyclopropylacetaldehyde O-(2-oxo-2-(4-(5-(trifluoromethyl) pyrimidin-2-yl)piperazin-1-yl)ethyl) oxime (106 mg, 81.9%) as a yellow oil.
LCMS: *(ESI)* (M+H)⁺ = 387.2.

### Step F: Synthesis of (S,E)-2-cyclopropyl-2-((6-oxo-5-(trifluoromethyl)-1-((2 -(trimethylsilyl)ethoxy)methyl)-1,6-dihydropyridazin-4-yl)amino)acetaldehyde O-(2-oxo-2-(4-(5-(trifluoromethyl)pyrimidin-2-yl)piperazin-1-yl)ethyl) oxime

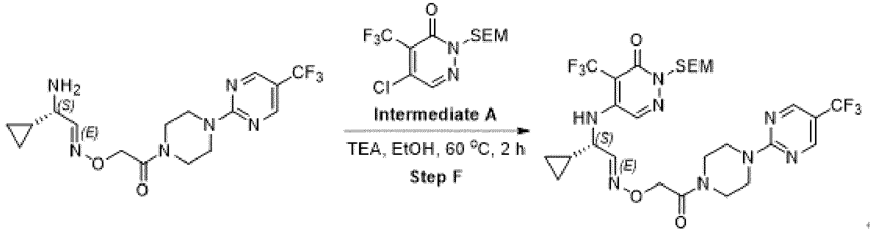

To a solution of (S,E)-2-amino-2-cyclopropylacetaldehyde O-(2-oxo-2-(4-(5-(trifluoromethyl)pyrimidin-2-yl)piperazin-1-yl)ethyl) oxime (106 mg, 0.27 mmol, 1.0 eq.) and 5-chloro-4-(trifluoromethyl)-2-((2-(trimethylsilyl)ethoxy)methyl)pyridazin-3 (2H)-one (178 mg, 0.54 mmol, 2.0 eq.) in EtOH (3 mL) was added TEA (55 mg, 0.54 mmol, 2.0 eq.). The mixture was heated to 60 °C and stirred for 2 h. The reaction was cooled to room temperature, quenched with water (15 mL) and extracted with EtOAc (3 x 20 mL). The organic layers were combined and washed with brine (40 mL), dried over Na₂SO₄ and concentrated under reduced pressure. The residue was purified by column chromatography (Petroleum ether/EtOAc= 1/3; V/V) to afford (S,E)-2-cyclopropyl -2-((6-oxo-5-(trifluoromethyl)-1-((2-(trimethylsilyl)ethoxy)methyl)-1,6-dihydropyridazi n-4-yl)amino)acetaldehyde O-(2-oxo-2-(4-(5-(trifluoromethyl)pyrimidin-2-yl)piperazin -1-yl)ethyl) oxime (74 mg, 39.7%) as a yellow oil.
LCMS: *(ESI)* (M+H)⁺ = 679.2.

### Step G: synthesis of (S,E)-2-cyclopropyl-2-((6-oxo-5-(trifluoromethyl)-L6 -dihydropyridazin-4-yl)amino)acetaldehyde O-(2-oxo-2-(4-(5-(trifluoromethyl) pyrimidin-2-yl)piperazin-1-yl)ethyl) oxime

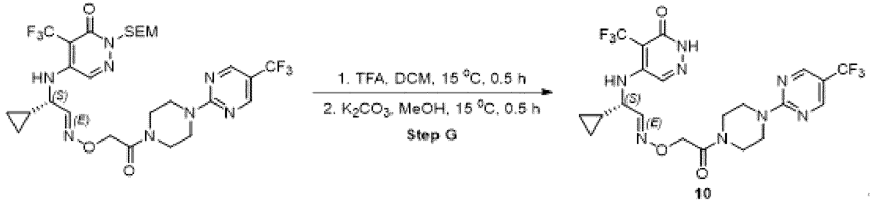

To a solution of (S,E)-2-cyclopropyl-2-((6-oxo-5-(trifluoromethyl) -1-((2-(trimethylsilyl)ethoxy)methyl)-1,6-dihydropyridazin-4-yl)amino)acetaldehyde O-(2-oxo-2-(4-(5-(trifluoromethyl)pyrimidin-2-yl)piperazin-1-yl)ethyl) oxime (74 mg, 0.11 mmol, 1.0 eq.) in DCM (3 mL) was added TFA (1 mL). The mixture was stirred at 15 °C for 0.5 h. The mixture was concentrated under vacuum. The residue was redissolved in MeOH (1 mL) and K₂CO₃ (10 mg, 0.07 mmol) was added. The mixture was stirred at 15 °C for another 0.5 h. The mixture was filtered. The filtrate was purified by prep-HPLC to afford (S,E)-2-cyclopropyl-2-((6-oxo-5-(trifluoromethyl)-1,6-dihydropyridazin-4-yl)amino)acetaldehyde O-(2-oxo-2-(4-(5-(trifluoromethyl)pyrimidin -2-yl)piperazin-1-yl)ethyl) oxime (15.28 mg, 25.6%) as a white solid.
LCMS: *(ESI)* (M+H)⁺ = 549.2.
¹H NMR: (400 MHz, CDCl₃) *δ* 10.82 (s, 1H), 8.56 (s, 2H), 7.71 (s, 1H), 7.54 (d, *J* = 6.4 Hz, 1H), 5.98 - 5.90 (m, 1H), 4.83 (s, 2H), 4.05 - 3.90 (m, 4H), 3.83 - 3.72 (m, 3H), 3.62 - 3.50 (m, 2H), 1.24 - 1.14 (m, 1H), 0.87 - 0.73 (m, 2H), 0.59 - 0.46 (m, 2H).

### Example 11:

### (S,E)-6-(4-(2-(((2-((6-oxo-5-(trifluoromethyl)-1,6-dihydropyridazin-4-yl)amino)propyli dene)amino)oxy)acetyl)piperazin-1-yl)nicotinonitrile

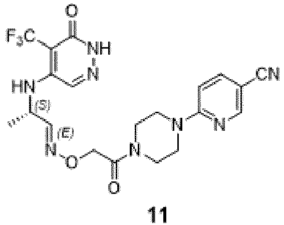

### 11.1) Synthesis of intermediate H

### Step A: synthesis of tert-butyl 4-(5-cyanopyridin-2-yl)piperazine-1-carboxylate

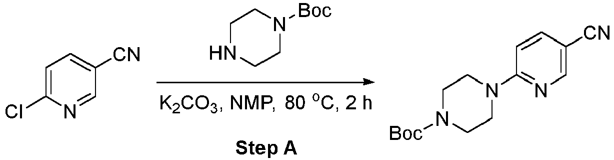

To a solution of 6-chloronicotinonitrile (5.00 g, 36.09 mmol, 1.0 eq.) in NMP (150 mL) were added tert-butyl piperazine-1-carboxylate (7.39 g, 39.70 mmol, 1.1eq.) and K₂CO₃ (14.96 g, 108.27 mmol, 3.0 eq.). The resulting solution was stirred at 80 °C for 2 h. The reaction was cooled to room temperature. The mixture was quenched with water (150 mL) and extracted with EtOAc (3 x 150 mL). The organic layers were combined and washed with brine (500 mL), dried over anhydrous Na₂SO₄ and concentrated under reduced pressure. The residue was purified by column chromatography (Petroleum ether/EtOAc= 2/1; V/V) to afford tert-butyl 4-(5-cyanopyridin-2-yl)piperazine -1-carboxylate (8.40 g, 80.7%) as a yellow solid.
LCMS: *(ESI)* (M+H-56)⁺ = 233.2.
¹H NMR: (400 MHz, CDCl₃) *δ* 8.41 (d, *J* = 2.4 Hz, 1H), 7.64 (dd, *J* = 8.8, 2.4 Hz, 1H), 6.61 (d, *J* = 9.2 Hz, 1H), 3.3.70 - 3.67 (m, 4H), 3.56 - 3.53 (m, 4H), 1.49 (s, 9H).

### Step B: synthesis of 6-(piperazin-1-yl)nicotinonitrile hydrochloride (Intermediate H)

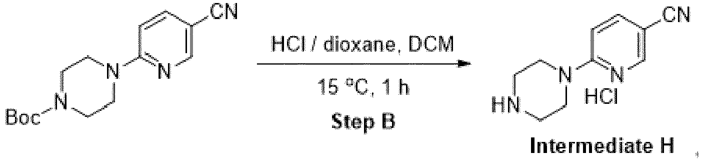

To a solution of tert-butyl 4-(5-cyanopyridin-2-yl)piperazine-1-carboxylate (1.30 g, 4.51 mmol, 1.0 eq.) in DCM (2 mL) was added HCl/dioxane (4M, 15 mL). The mixture was stirred at 15 °C for 1 h. The mixture was concentrated under reduced pressure to afford 6-(piperazin-1-yl)nicotinonitrile hydrochloride (0.99 g, 97.2%) as a white solid.
LCMS: *(ESI)* (M+H)⁺ = 189.2.

### 11.2) Synthesis of compound 11:

### Step A: Synthesis of (S,E)-tert-butyl (1-((2-(4-(5-cyanopyridin-2-yl)piperazin-1-yl) -2-oxoethoxy)imino)propan-2-yl)carbamate

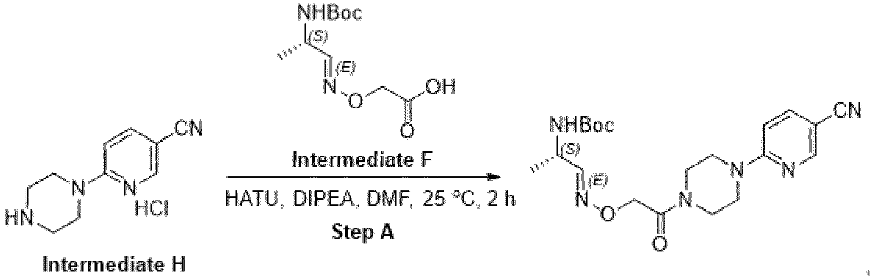

To a solution of ((((6S,7E)-2,2,6-trimethyl-4-oxo-5-aza-3-oxaheptan-7 -ylidene)amino)oxy)acetic acid (100 mg, 0.40 mmol, 1.0 eq.) and 6-(piperazin-1-yl)nicotinonitrile hydrochloride (99 mg, 0.44 mmol, 1.1 eq.) in DMF (6 mL) were added DIPEA (315 mg, 2.40 mmol, 6.0 eq.) and HATU (185 mg, 0.48 mmol, 1.2 eq.). The mixture was stirred at 25 °C for 2 h. The mixture was quenched with water (40 mL) and extracted with EtOAc (3 x 50 mL). The organic layers were combined and washed with brine (80 mL), dried over anhydrous Na₂SO₄ and concentrated under reduced pressure. The residue was purified by column chromatography (DCM/MeOH= 20/1; V/V) to afford (S,E)-tert-butyl (1-((2-(4-(5-cyanopyridin-2-yl)piperazin-1-yl) -2-oxoethoxy)imino)propan-2-yl)carbamate (120 mg, 71.0%) as yellow oil.
LCMS: *(ESI)* (M+H-100)⁺ = 317.2.
¹H NMR: (400 MHz, CDCl₃) *δ* 8.48 - 8.43 (m, 1H), 7.69 (dd, *J* = 8.8, 2.4 Hz, 1H), 7.53 (d, *J* = 4.4 Hz, 1H), 6.66 (d, *J* = 9.2 Hz, 1H), 4.98 - 4.86 (m, 1H), 4.78 (s, 2H), 4.48 - 4.28 (m, 1H), 3.88 - 3.76 (m, 4H), 3.75 - 3.62 (m, 4H), 1.46 (s, 9H), 1.34 (d, *J* = 6.8 Hz, 3H).

### Step B: synthesis of (S,E)-6-(4-(2-(((2-aminopropylidene)amino)oxy)acetyl)piperazin -1-yl)nicotinonitrile

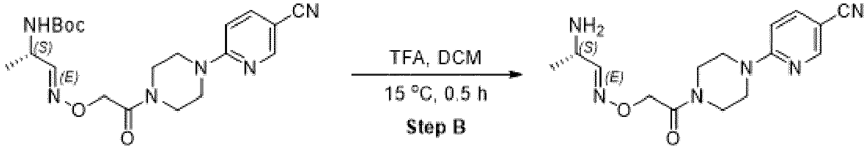

To a solution of (S,E)-tert-butyl (1-((2-(4-(5-cyanopyridin-2-yl)piperazin-1-yl)-2-oxoethoxy)imino)propan-2-yl)carbama te (120 mg, 0.29 mmol, 1.0 eq.) in DCM (4 mL) was added TFA (1 mL). The resulting solution was stirred at 15 °C for 0.5 h. The mixture was quenched with saturated NaHCOs aqueous solution (20 mL) and extracted with DCM (3 x 20 mL). The organic layers were combined and dried over Na₂SO₄, concentrated under vacuum to afford (S,E)-6-(4-(2-(((2-aminopropylidene)amino)oxy)acetyl)piperazin-1-yl)nicotinonitrile (70 mg, 76.8%) as a yellow oil.
LCMS: *(ESI)* (M+H)⁺ = 317.2.

### Step C: synthesis of (S,E)-6-(4-(2-(((2-((6-oxo-5-(trifluoromethyl)-1-((2-(trimethylsilyl) ethoxy)methyl)-1,6-dihydropyridazin-4-yl)amino)propylidene)amino)oxy)acetyl)pipera zin-1-yl)nicotinonitrile

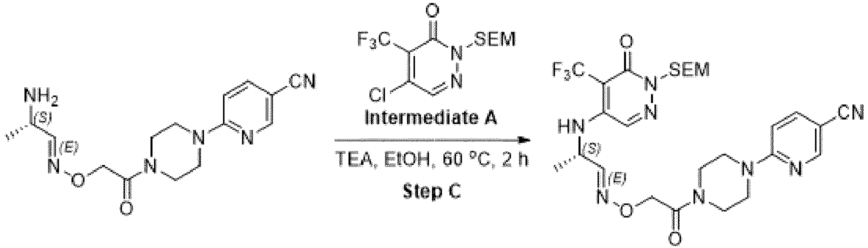

To a solution of (S,E)-6-(4-(2-(((2-aminopropylidene)amino)oxy)acetyl)piperazin-1-yl)nicotinonitrile (70 mg, 0.22 mmol, 1.0 eq.) and 5-chloro-4-(trifluoromethyl)-2-((2 -(trimethylsilyl)ethoxy)methyl)pyridazin-3(2H)-one (80 mg, 0.24 mmol, 1.1 eq.) in EtOH (3 mL) was added TEA (45 mg, 0.44 mmol, 2.0 eq.). The mixture was heated to 60 °C and stirred for 2 h. The reaction was quenched with water (20 mL) and extracted with EtOAc (3 x 20 mL). The organic layers were combined and washed with brine (80 mL), dried over Na₂SO₄ and concentrated under reduced pressure. The residue was purified by column chromatography (Petroleum ether/EtOAc= 1/3; V/V) to afford (S,E)-6-(4-(2-(((2-((6-oxo-5-(trifluoromethyl)-1-((2-(trimethylsilyl)ethoxy)methyl)-1,6-dihydropyridazin-4-yl)amino)propylidene)amino)oxy)acetyl)piperazin-1-yl)nicotinonitri le (75 mg, 56.0%) as a yellow oil.
LCMS: *(ESI)* (M+H)⁺ = 609.2.

### Step D: synthesis of (S,E)-6-(4-(2-(((2-((6-oxo-5-(trifluoromethyl) -1,6-dihydropyridazin-4-yl)amino)propylidene)amino)oxy)acetyl)piperazin-1-yl) nicotinonitrile

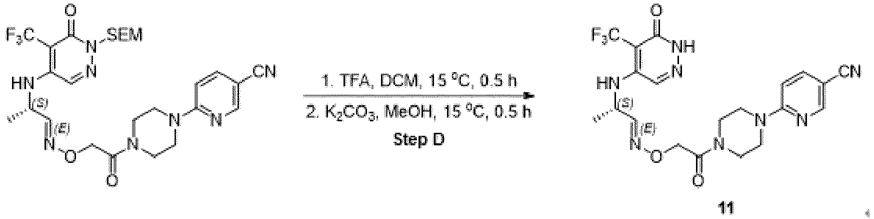

To a solution of (S,E)-6-(4-(2-(((2-((6-oxo-5-(trifluoromethyl)-1-((2-(trimethylsilyl)ethoxy)methyl)-1,6-dihydropyridazin-4-yl)amino)propylidene)amino)ox y)acetyl)piperazin-1-yl)nicotinonitrile (70 mg, 0.12 mmol, 1.0 eq.) in DCM (3 mL) was added TFA (1 mL). The resulting solution was stirred at 15 °C for 0.5 h. The mixture was concentrated under vacuum. The residue was redissolved in MeOH (1 mL) and K₂CO₃ (10 mg, 0.07 mmol) was added. The mixture was stirred at 15 °C for another 0.5 h. The mixture was filtered and the filtrate was purified by prep-HPLC to afford (S,E)-6-(4-(2-(((2-((6-oxo-5-(trifluoromethyl)-1,6-dihydropyridazin-4-yl)amino)propyli dene)amino)oxy)acetyl)piperazin-1-yl)nicotinonitrile (25.97 mg, 49.4%) as a white solid.
LCMS: *(ESI)* (M+H)⁺ = 479.2.
¹H NMR: (400 MHz, CDCl₃) *δ* 11.16 (s, 1H), 8.42 (d, *J* = 2.0 Hz, 1H), 7.70 (s, 1H), 7.66 (dd, *J* = 9.2, 2.4 Hz, 1H), 7.49 (d, *J* = 5.6 Hz, 1H), 6.62 (d, *J* = 8.8 Hz, 1H), 5.89 - 5.73 (m, 1H), 4.83 - 4.72 (m, 2H), 4.49 - 4.36 (m, 1H), 3.86 - 3.72 (m, 4H), 3.71 - 35.2 (m, 4H), 1.52 (d, *J* = 6.8 Hz, 3H).

### Example 12:

### (S,E)-2-((6-oxo-5-(trifluoromethyl)-1,6-dihydropyridazin-4-yl)amino)propanal O-(2-oxo-2-(4-(5-(trifluoromethyl)pyridin-2-yl)piperazin-1-yl)ethyl) oxime

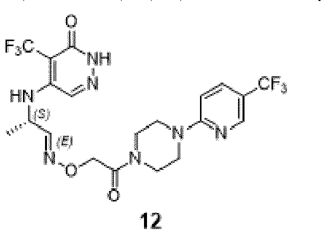

### 12.1) Synthesis of intermediate I

### Step A: synthesis of tert-butyl 4-(5-(trifluoromethyl)pyridin-2-yl)piperazine-1-carboxylate

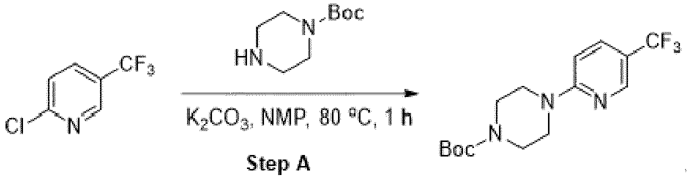

To a solution of 2-chloro-5-(trifluoromethyl)pyridine (4.13 g, 22.75 mmol, 1.0 eq.) and tert-butyl piperazine-1-carboxylate (3.26 g, 25.02 mmol, 1.1 eq.) in NMP (30 mL) was added K₂CO₃ (9.43 g, 68.25 mmol, 3.0 eq.) at room temperature. The mixture was heated to 80 °C and stirred for 1 h. The mixture was cooled to room temperature and diluted with water (500 mL). The formed precipitate was collected by filtration. The product was redissolved in EtOAc (500 mL) and dried over Na₂SO₄. The solvent was removed under vacuum to afford tert-butyl 4-(5-(trifluoromethyl)pyridin-2-yl) piperazine-1-carboxylate (3.50 g, 44.8% yield) as a white solid.
LCMS: *(ESI)* (M+H)⁺=332.2.
¹H NMR (400 MHz, CDCl₃) *δ* 8.42 - 8.39 (m, 1H), 7.65 (dd, *J* = 8.8, 2.4 Hz, 1H), 6.64 (d, *J* = 8.8 Hz, 1H), 3.68 - 3.61 (m, 4H), 3.58 - 3.52 (m, 4H), 1.49 (s, 9H).

### Step B: synthesis of 1-(5-(trifluoromethyl)pyridin-2-yl)piperazine hydrochloride (Intermediate I)

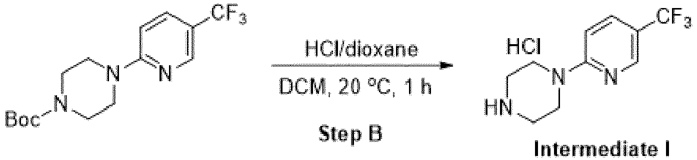

To a solution of tert-butyl 4-(5-(trifluoromethyl)pyridin-2-yl)piperazine -1-carboxylate (1.00 g, 3.02 mmol, 1.0 eq.) in DCM (10 mL) was added HCl/dioxane (4M, 10 mL). The resulting mixture was stirred at 20 °C for 1 h. The mixture was concentrated under vacuum to afford 1-(5-(trifluoromethyl)pyridin-2-yl)piperazine hydrochloride (808 mg, 99.9% yield) as a white solid.
LCMS: *(ESI)* (M+H)⁺ = 231.8.

### 12.2) Synthesis of compound 12

### Step A: Synthesis of (S,E)-tert-butyl (1-((2-oxo-2-(4-(5-(trifluoromethyl)pyridin-2-yl) piperazin-1-yl)ethoxy)imino)propan-2-yl)carbamate

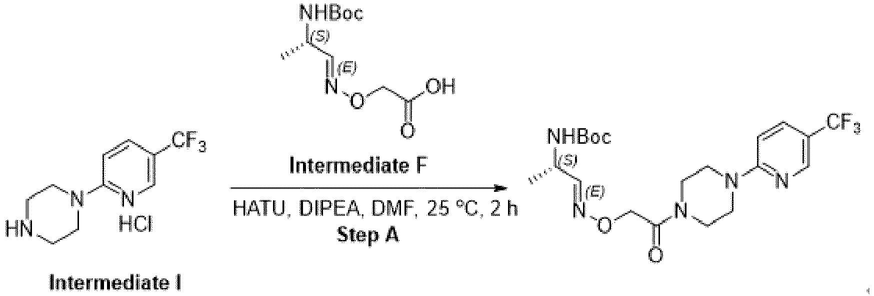

To a solution of ((((6S,7E)-2,2,6-trimethyl-4-oxo-5-aza-3-oxaheptan-7 -ylidene)amino)oxy)acetic acid (200 mg, 0.81 mmol, 1.0 eq.) and 1-(5-(trifluoromethyl)pyridin-2-yl)piperazine hydrochloride (239 mg, 0.89 mmol, 1.1 eq.) in DMF (10 mL) were added DIPEA (630 mg, 4.86 mmol, 6.0 eq.) and HATU (371 mg, 0.97 mmol, 1.2 eq.). The mixture was stirred at 25 °C for 2 h. The mixture was quenched with water (40 mL) and extracted with EtOAc (3 x 40 mL). The organic layers were combined and washed with brine (80 mL), dried over anhydrous Na₂SO₄ and concentrated under reduced pressure. The residue was purified by column chromatography (DCM/MeOH= 20/1; V/V) to afford (S,E)-tert-butyl (1-((2-oxo-2-(4-(5-(trifluoromethyl)pyridin-2-yl)piperazin-1-yl)ethoxy)imino)propan-2-yl)carbamate (275 mg, 73.7%) as yellow oil.
LCMS: *(ESI)* (M+H)⁺ = 460.2.

### Step B: synthesis of (S,E)-2-aminopropanal O-(2-oxo-2-(4-(5-(trifluoromethyl) pyridin-2-yl)piperazin-1-yl)ethyl) oxime

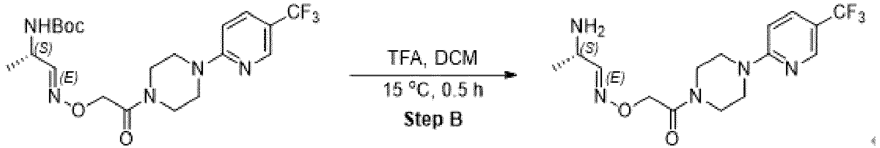

To a solution of (S,E)-tert-butyl (1-((2-oxo-2-(4-(5-(trifluoromethyl)pyridin-2-yl) piperazin-1-yl)ethoxy)imino)propan-2-yl)carbamate (275 mg, 0.60 mmol, 1.0 eq.) in DCM (8 mL) was added TFA (2 mL). The resulting solution was stirred at 15 °C for 0.5 h. The mixture was quenched with saturated NaHCO₃ aqueous solution (20 mL) and extracted with DCM (3 x 20 mL). The organic layers were combined and dried over Na₂SO₄, concentrated under vacuum to afford (S,E)-2-aminopropanal O-(2-oxo-2-(4-(5-(trifluoromethyl)pyridin-2-yl)piperazin-1-yl)ethyl) oxime (180 mg, 83.7%) as a yellow oil.
LCMS: *(ESI)* (M+H)⁺ = 360.2.
¹H NMR: (400 MHz, CDCl₃) *δ* 8.44 (s, 1H), 7.70 (dd, *J* = 8.8, 2.0 Hz, 1H), 7.50 (d, *J*= 5.6 Hz, 1H), 6.69 (d, *J* = 9.2 Hz, 1H), 4.76 (s, 2H), 3.83 - 3.76 (m, 4H), 3.75 - 3.71 (m, 3H), 3.69 - 3.58 (m, 4H), 1.26 (d, *J* = 6.8 Hz, 3H).

### Step C: synthesis of (S,E)-2-((6-oxo-5-(trifluoromethyl)-1-((2-(trimethylsilyl) ethoxy)methyl)-1,6-dihydropyridazin-4-yl)amino)propanal O-(2-oxo-2-(4-(5-(trifluoromethyl)pyridin-2-yl)piperazin-1-yl)ethyl) oxime

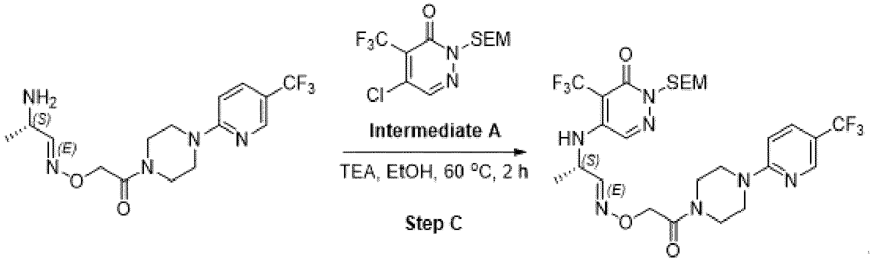

To a solution of (S,E)-2-aminopropanal O-(2-oxo-2-(4-(5-(trifluoromethyl) pyridin-2-yl)piperazin-1-yl)ethyl) oxime (79 mg, 0.22 mmol, 1.0 eq.) and 5-chloro-4-(trifluoromethyl)-2-((2-(trimethylsilyl)ethoxy)methyl)pyridazin-3 (2H)-one (80 mg, 0.24 mmol, 1.1 eq.) in EtOH (3 mL) was added TEA (44 mg, 0.44 mmol, 2.0 eq.). The mixture was heated to 60 °C and stirred for 2 h. The reaction was quenched with water (20 mL) and extracted with EtOAc (3 x 20 mL). The organic layers were combined and washed with brine (80 mL), dried over Na₂SO₄ and concentrated under reduced pressure. The residue was purified by column chromatography (Petroleum ether/EtOAc= 1/3; V/V) to afford (S,E)-2-((6-oxo-5-(trifluoromethyl)-1-((2-(trimethylsilyl)ethoxy)methyl)-1,6-dihydropyridazin-4-yl)amino)propanal O-(2-oxo-2-(4-(5-(trifluoromethyl)pyridin-2-yl)piperazin-1-yl)ethyl) oxime (72 mg, 50.3%) as a yellow oil.
LCMS: *(ESI)* (M+H)⁺ = 652.2.

### Step D: synthesis of (S,E)-2-((6-oxo-5-(trifluoromethyl) -1,6-dihydropyridazin-4-yl)amino)propanal O-(2-oxo-2-(4-(5-(trifluoromethyl)pyridin-2 -yl)piperazin-1-yl)ethyl) oxime

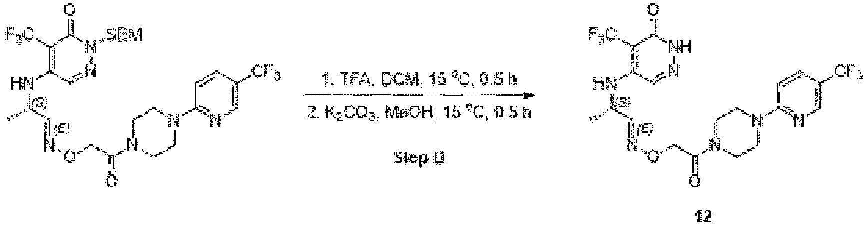

To a solution of (S,E)-2-((6-oxo-5-(trifluoromethyl)-1-((2-(trimethylsilyl)ethoxy) methyl)-1,6-dihydropyridazin-4-yl)amino)propanal O-(2-oxo-2-(4-(5-(trifluoromethyl) pyridin-2-yl)piperazin-1-yl)ethyl) oxime (72 mg, 0.11 mmol, 1.0 eq.) in DCM (3 mL) was added TFA (1 mL). The resulting solution was stirred at 15 °C for 0.5 h. The mixture was concentrated under vacuum. The residue was redissolved in MeOH (1 mL) and K₂CO₃ (10 mg, 0.07 mmol) was added. The mixture was stirred at 15 °C for another 0.5 h. The mixture was filtered and the filtrate was purified by prep-HPLC to afford (S,E)-2-((6-oxo-5-(trifluoromethyl)-1,6-dihydropyridazin-4-yl)amino)propanal O-(2-oxo-2-(4-(5-(trifluoromethyl)pyridin-2-yl)piperazin-1-yl)ethyl) oxime (31.76 mg, 55.3%) as a white solid.
LCMS: *(ESI)* (M+H)⁺ = 522.2.
¹H NMR: (400 MHz, CDCl₃) *δ* 10.77 (s, 1H), 8.41 (s, 1H), 7.70 (s, 1H), 7.68 (dd, *J=* 9.2, 2.4 Hz, 1H), 7.49 (d, *J* = 5.6 Hz, 1H), 6.66 (d, *J* = 8.8 Hz, 1H), 5.88 - 5.79 (m, 1H), 4.83 - 4.73 (m, 2H), 4.47 - 4.38 (m, 1H), 3.82 - 3.70 (m, 4H), 3.69 - 3.52 (m, 4H), 1.51 (d, *J* = 6.8 Hz, 3H).

### Example 13:

### 6-((R)-3-methyl-4-(2-(((E)-((S)-2-((6-oxo-5-(trifluoromethyl)-1,6-dihydropyridazin-4 -yl)amino)propylidene)amino)oxy)acetyl)piperazin-1-yl)nicotinonitrile

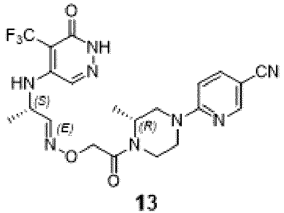

### Step A: Synthesis of (R)-tert-butyl 4-(5-cyanopyridin-2-yl)-2-methylpiperazine-1-carboxylate

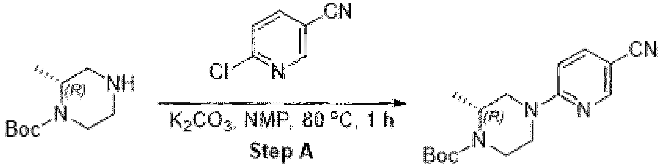

To a solution of 6-chloronicotinonitrile (2.51 g, 18.11 mmol, 1.0 eq.) in NMP (120 mL) were added (R)-tert-butyl 2-methylpiperazine-1-carboxylate (3.99 g, 19.92 mmol, 1.1 eq.) and K₂CO₃ (7.51 g, 54.33 mmol, 3.0 eq.). The mixture was stirred at 80 °C for 1 h. The mixture was cooled to room temperature and diluted with water (150 mL), extracted with EtOAc (3 × 150 mL). The organic layers were combined and washed with brine (500 mL), dried over anhydrous Na₂SO₄, and concentrated under reduced pressure. The residue was purified by column chromatography (Petroleum ether/EtOAc=2/1; V/V) to afford (R)-tert-butyl 4-(5-cyanopyridin-2-yl)-2-methylpiperazine-1-carboxylate (4.20 g, 76.7%) as yellow solid.
LCMS: *(ESI)* (M+H-56)⁺ = 247.0.
¹H NMR: (400 MHz, CDCl₃) *δ* 8.40 (d, *J* = 2.0 Hz, 1H), 7.62 (dd, *J* = 8.8, 2.0 Hz, 1H), 6.56 (d, *J* = 8.8 Hz, 1H), 4.37 - 4.27 (m, 1H), 4.20 - 4.12 (m, 1H), 4.12 - 4.04 (m, 1H), 4.00 - 3.90 (m, 1H), 3.42 (dd, *J* = 13.6, 4.0 Hz, 1H), 3.31 - 3.21 (m, 1H), 3.19 - 3.08 (m, 1H), 1.48 (s, 9H), 1.15 (d, *J* = 6.8 Hz, 3H).

### Step B: Synthesis of (R)-6-(3-methylpiperazin-1-yl)nicotinonitrile hydrochloride

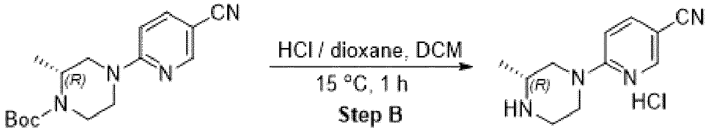

To a solution of (R)-tert-butyl 4-(5-cyanopyridin-2-yl)-2-methylpiperazine -1-carboxylate (500 mg, 1.65 mmol, 1.0 eq.) in DCM (2 mL) was added HCl/dioxane (4M, 10 mL). The mixture was stirred at 15 °C for 1 h. The mixture was concentrated under reduced pressure to afford (R)-6-(3-methylpiperazin-1-yl)nicotinonitrile hydrochloride (384 mg, 99.8%) as white solid.
LCMS: *(ESI)* (M+H)⁺ = 203.2.

### Step C: Synthesis of tert-butyl ((S,E)-1-((2-((R)-4-(5-cyanopyridin-2-yl) -2-methylpiperazin-1-yl)-2-oxoethoxy)imino)propan-2-yl)carbamate

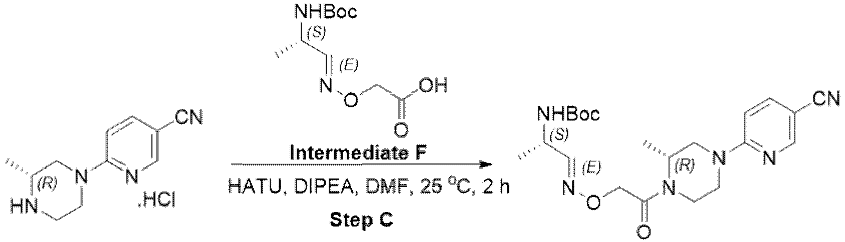

To a solution of ((((6S,7E)-2,2,6-trimethyl-4-oxo-5-aza-3-oxaheptan-7-ylidene) amino)oxy)acetic acid (200 mg, 0.84 mmol, 1.0 eq.) and (R)-6-(3-methylpiperazin-1-yl)nicotinonitrile hydrochloride (227 mg, 0.92 mmol, 1.1 eq.) in DMF (12 mL) were added DIPEA (651 mg, 5.04 mmol, 6.0 eq.) and HATU (384 mg, 1.01 mmol, 1.2 eq.). The mixture was stirred at 25 °C for 2 h. The reaction was quenched with water (50 mL) and extracted with EtOAc (3 x 50 mL). The organic layers were combined and washed with brine (80 mL), dried over anhydrous Na₂SO₄ and concentrated under reduced pressure. The residue was purified by column chromatography (DCM/MeOH=20/1; V/V) to afford tert-butyl ((S,E)-1-((2-((R)-4-(5-cyanopyridin-2-yl)-2-methylpiperazin-1-yl)-2-oxoethoxy)imino) propan-2-yl)carbamate (280 mg, 77.3%) as yellow oil.
LCMS: *(ESI)* (M+H)⁺ = 431.2.

### Step D: synthesis of 6-((R)-4-(2-(((E)-((S)-2-aminopropylidene)amino)oxy)acetyl) -3-methylpiperazin-1-yl)nicotinonitrile

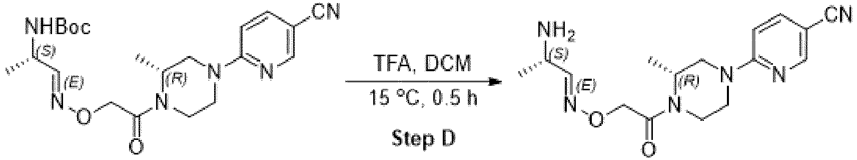

To a solution of tert-butyl ((S,E)-1-((2-((R)-4-(5-cyanopyridin-2-yl)-2-methylpiperazin-1-yl)-2-oxoethoxy)imino) propan-2-yl)carbamate (280 mg, 0.65 mmol, 1.0 eq.) in DCM (8 mL) was added TFA (2 mL). The resulting solution was stirred at 15 °C for 0.5 h. The mixture was quenched with saturated NaHCOs aqueous solution (20 mL) and extracted with DCM (3 x 20 mL). The organic layers were combined and dried over Na₂SO₄, concentrated under vacuum to afford 6-((R)-4-(2-(((E)-((S)-2-aminopropylidene)amino)oxy)acetyl)-3-methylpiperazin-1-yl)nicotinonitrile (209 mg, 97.3%) as a yellow oil.
LCMS: *(ESI)* (M+H)⁺ = 331.2.
¹H NMR: (400 MHz, CDCl₃) *δ* 8.43 (d, *J* = 2.0 Hz, 1H), 7.67 (dd, *J* = 8.8, 2.4 Hz, 1H), 7.49 (d, *J* = 5.6 Hz, 1H), 6.62 (d, *J* = 8.8 Hz, 1H), 4.85 - 4.65 (m, 2H), 4.60 - 4.05 (m, 3H), 3.78 - 3.63 (m, 4H), 3.56 - 3.04 (m, 3H), 1.28 - 1.25 (m, 6H).

### Step E: synthesis of 6-((R)-3-methyl-4-(2-(((E)-((S)-2-((6-oxo-5-(trifluoromethyl) -1-((2-(trimethylsilyl)ethoxy)methyl)-1,6-dihydropyridazin-4-yl)amino)propylidene)ami no)oxy)acetyl)piperazin-1-yl)nicotinonitrile

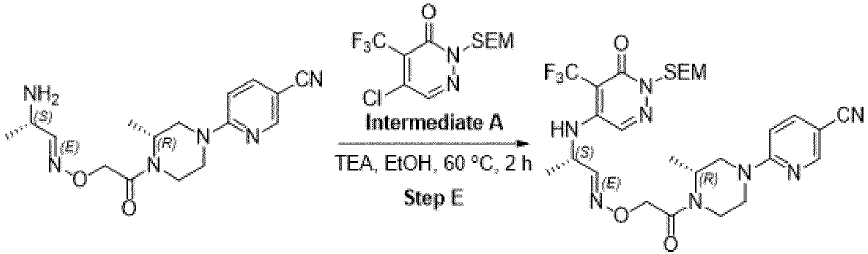

To a solution of 6-((R)-4-(2-(((E)-((S)-2-aminopropylidene)amino)oxy)acetyl) -3-methylpiperazin-1-yl)nicotinonitrile (73 mg, 0.22 mmol, 1.0 eq.) and 5-chloro-4-(trifluoromethyl)-2-((2-(trimethylsilyl)ethoxy)methyl)pyridazin-3 (2H)-one (80 mg, 0.24 mmol, 1.1 eq.) in EtOH (3 mL) was added TEA (45 mg, 0.44 mmol, 2.0 eq.). The mixture was heated to 60 °C and stirred for 2 h. The reaction was quenched with water (20 mL) and extracted with EtOAc (3 x 20 mL). The organic layers were combined and washed with brine (80 mL), dried over Na₂SO₄ and concentrated under reduced pressure. The residue was purified by column chromatography (Petroleum ether/EtOAc=1/3; V/V) to afford 6-((R)-3-methyl-4-(2-(((E)-((S)-2-((6-oxo-5-(trifluoromethyl)-1-((2-(trimethylsilyl)ethoxy)methyl)-1,6-dihydropyridazin-4-yl)amino )propylidene)amino)oxy)acetyl)piperazin-1-yl)nicotinonitrile (73 mg, 53.1%) as a yellow oil.
LCMS: *(ESI)* (M+H)⁺ = 623.4.

### Step F: synthesis of 6-((R)-3-methyl-4-(2-(((E)-((S)-2-((6-oxo-5-(trifluoromethyl) -1,6-dihydropyridazin-4-yl)amino)propylidene)amino)oxy)acetyl)piperazin-1-yl) nicotinonitrile

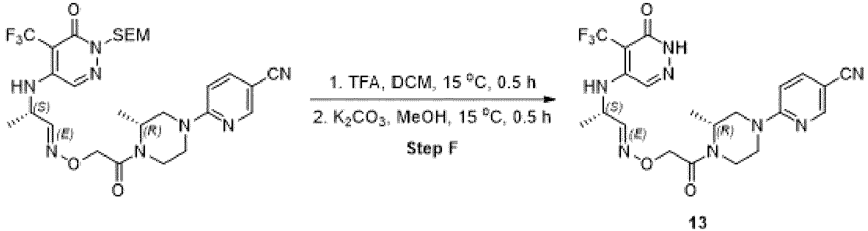

To a solution of 6-((R)-3-methyl-4-(2-(((E)-((S)-2-((6-oxo-5-(trifluoromethyl) -1-((2-(trimethylsilyl)ethoxy)methyl)-1,6-dihydropyridazin-4-yl)amino)propylidene)ami no)oxy)acetyl)piperazin-1-yl)nicotinonitrile (72 mg, 0.12 mmol, 1.0 eq.) in DCM (3 mL) was added TFA (1 mL). The resulting solution was stirred at 15 °C for 0.5 h. The mixture was concentrated under vacuum. The residue was redissolved in MeOH (1 mL) and K₂CO₃ (10 mg, 0.07 mmol) was added. The mixture was stirred at 15 °C for 0.5 h. The mixture was filtered and the filtrate was purified by prep-HPLC to afford 6-((R)-3-methyl-4-(2-(((E)-((S)-2-((6-oxo-5-(trifluoromethyl)-1,6-dihydropyridazin-4-y l)amino)propylidene)amino)oxy)acetyl)piperazin-1-yl)nicotinonitrile (30.73 mg, 52.0%) as a white solid.
LCMS: *(ESI)* (M+H)⁺ = 493.2.
¹H NMR: (400 MHz, CDCl₃) *δ* 11.10 (s, 1H), 8.41 (d, *J* = 2.0 Hz, 1H), 7.70 (s, 1H), 7.65 (dd, *J* = 9.2, 2.4 Hz, 1H), 7.50 (d, *J* = 5.6 Hz, 1H), 6.60 (d, *J* = 8.8 Hz, 1H), 5.90 - 5.81 (m, 1H), 4.84 - 4.71 (m, 2H), 4.50 - 4.00 (m, 4H), 3.74 - 3.05 (m, 4H), 1.51 (d, *J* = 6.8 Hz, 3H), 1.31 - 1.14 (m, 3H).

### Example 14:

### (S,E)-2-((6-oxo-5-(trifluoromethyl)-1,6-dihydropyridazin-4-yl)amino)propanal O-(2-(1-(5-(trifluoromethyl)pyrimidin-2-yl)piperidin-4-yl)ethyl) oxime

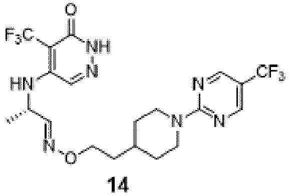

### Step A: synthesis of 2-(1-(5-(trifluoromethyl)pyrimidin-2-yl)piperidin-4-yl)ethanol

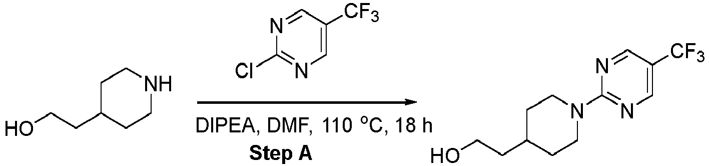

To a solution of 2-(piperidin-4-yl)ethanol (900 mg, 6.97 mmol, 1.0 eq.) in N,N-Dimethylformamide (10 mL) were added 2-chloro-5-(trifluoromethyl)pyrimidine (1.29 g, 7.04 mmol, 1.01 eq.) and DIPEA (2.70 g, 20.9 mmol, 3.0 eq.). The mixture was stirred at 110 °C for 18 h. The mixture was cooled to room temperature, diluted with water (100 mL) and extracted with ethyl acetate (2 x 100 mL). The organic layers were combined and washed with brine (100 mL), dried over Na₂SO₄ and concentrated under vacuum. The residue was purified by column chromatography (EtOAc/Petroleum ether= 1/2; V/V) to afford 2-(1-(5-(trifluoromethyl)pyrimidin-2-yl)piperidin-4-yl)ethanol (1.40 g, 70.8% yield) as a yellow solid.
LCMS: *(ESI)* (M+H)⁺ = 276.0.

### Step B: synthesis of 2-(2-(1-(5-(trifluoromethyl)pyrimidin-2-yl)piperidin-4-yl)ethoxy)isoindoline-1,3-dione

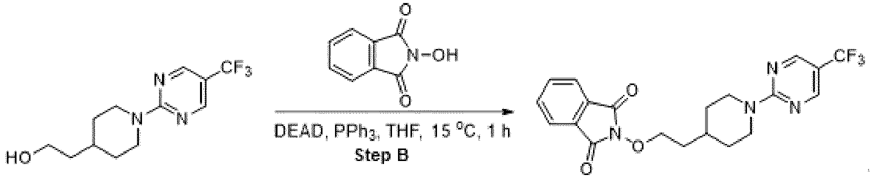

To a solution of 2-(1-(5-(trifluoromethyl)pyrimidin-2-yl)piperidin-4-yl)ethanol (150 mg, 0.55 mmol, 1.0 eq.) in THF (5 mL) were added 2-hydroxyisoindoline-1,3-dione (98 mg, 0.60 mmol, 1.1 eq.), Diethyl azodicarboxylate (116 mg, 0.67 mmol, 1.22 eq.) and triphenylphosphine (172 mg, 0.65 mmol, 1.2 eq.) at 0 °C. The resulting mixture was stirred at 15 °C for 1 h. The mixture was diluted with water (100 mL) and extracted with ethyl acetate (2 x 80 mL). The organic layers were combined and washed with brine (100 mL), dried over Na₂SO₄ and concentrated under vacuum. The residue was purified by column chromatography (EtOAc/Petroleum ether= 1/4; V/V) to afford 2-(2-(1-(5-(trifluoromethyl)pyrimidin-2-yl)piperidin-4-yl)ethoxy) isoindoline-1,3-dione (128 mg, 47.5% yield) as a yellow solid.
LCMS: *(ESI)* (M+H)⁺ = 420.8.
¹H NMR: (400 MHz, CDCl₃) *δ* 8.50 (s, 2H), 7.92 - 7.86 (m, 2H), 7.83 - 7.77 (m, 2H), 4.95 - 4.85 (m, 2H), 4.33 (t, *J* = 6.4 Hz, 2H), 3.08 - 2.96 (m, 2H), 2.01 - 1.93 (m, 2H), 1.81 (q, *J* = 6.4 Hz, 2H), 1.35 - 1.20 (m, 3H).

### Step C: synthesis of O-(2-(1-(5-(trifluoromethyl)pyrimidin-2-yl)piperidin-4-yl)ethyl) hydroxylamine

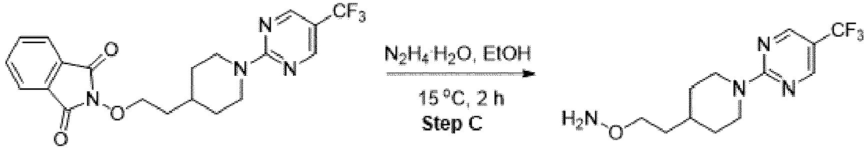

To a solution of 2-(2-(1-(5-(trifluoromethyl)pyrimidin-2-yl)piperidin-4-yl)ethoxy)isoindoline-1,3-dione (110 mg, 0.26 mmol, 1.0 eq.) in EtOH (5 mL) was added Hydrazine hydrate (27 mg, 0.52 mmol, 2.0 eq.). The resulting mixture was stirred at 15 °C for 2 h. The mixture was filtered and the filtrate was diluted with water (30 mL). The mixture was extracted with DCM (2 x 80 mL). The organic layers were combined and washed with brine (40 mL), dried over Na₂SO₄ and concentrated under vacuum to afford O-(2-(1-(5-(trifluoromethyl)pyrimidin-2-yl)piperidin-4-yl)ethyl)hydroxylamine (68 mg, 90.8% yield) as a yellow solid.
LCMS: *(ESI)* (M+H)⁺ = 291.2.

### Step D: synthesis of (S-E)-tert-butyl (1-((2-(1-(5-(trifluoromethyl)pyrimidin-2-yl) piperidin-4-yl)ethoxy)imino)propan-2-yl)carbamate

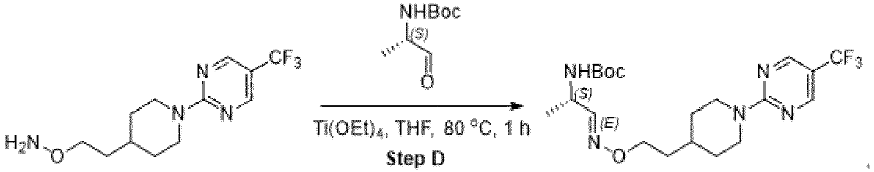

To a solution of O-(2-(1-(5-(trifluoromethyl)pyrimidin-2-yl)piperidin-4-yl) ethyl)hydroxylamine (100 mg, 0.34 mmol, 1.0 eq.) in THF (5 mL) were added (S)-tert-butyl (1-oxopropan-2-yl)carbamate (72 mg, 0.42 mmol, 1.2 eq.) and tetraethoxy titanium (79 mg, 0.34 mmol, 1.0 eq.). The resulting mixture was stirred at 80 °C for 1 h. The mixture was cooled to room temperature, diluted with water (100 mL) and extracted with ethyl acetate (2 × 80 mL). The organic layers were combined and washed with brine (100 mL), dried over Na₂SO₄ and concentrated under vacuum. The residue was purified by column chromatography (EtOAc/Petroleum ether= 20/1; V/V) to afford (S,E)-tert-butyl (1-((2-(1-(5-(trifluoromethyl)pyrimidin-2-yl)piperidin-4-yl)ethoxy)imino)propan-2-yl)c arbamate (120 mg, 75.9% yield) as a yellow solid.
LCMS: *(ESI)* (M+H)⁺ = 445.8.

### Step E: synthesis of (S,E)-2-aminopropanal O-(2-(1-(5-(trifluoromethyl)pyrimidin-2-yl) piperidin-4-yl)ethyl) oxime

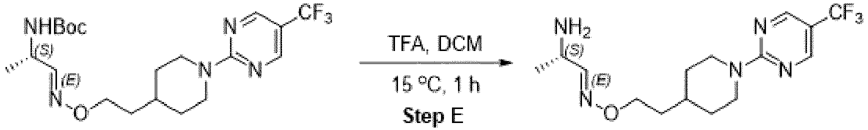

To a solution of (S,E)-tert-butyl (1-((2-(1-(5-(trifluoromethyl)pyrimidin-2-yl)piperidin-4-yl)ethoxy)imino)propan-2-yl)c arbamate (120 mg, 0.27 mmol, 1.0 eq.) in DCM (3 mL) was added TFA (1 mL). The resulting mixture was stirred at 15 °C for 1 h. The reaction mixture was quenched with NaHCOs aqueous solution (20 mL) and extracted with DCM (2 x 50 mL). The organic layers were combined and washed with brine (20 mL), dried over Na₂SO₄ and concentrated under vacuum to afford (S,E)-2-aminopropanal O-(2-(1-(5-(trifluoromethyl)pyrimidin-2-yl)piperidin-4-yl)ethyl) oxime (90 mg, 96.8% yield) as a yellow solid.
LCMS: *(ESI)* (M+H)⁺ = 346.2.

### Step F: synthesis of (S,E)-2-((6-oxo-5-(trifluoromethyl)-1-((2-(trimethylsilyl)ethoxy) methyl)-1,6-dihydropyridazin-4-yl)amino)propanal O-(2-(1-(5-(trifluoromethyl) pyrimidin-2-yl)piperidin-4-yl)ethyl) oxime

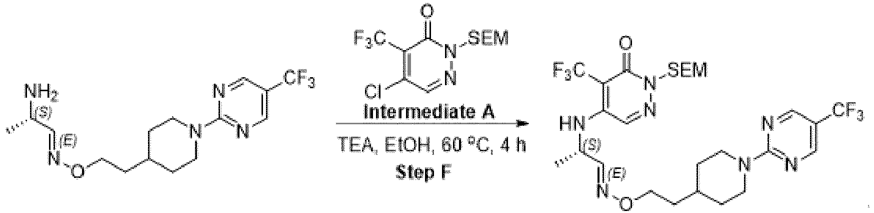

To a solution of (S,E)-2-aminopropanal O-(2-(1-(5-(trifluoromethyl)pyrimidin-2-yl) piperidin-4-yl)ethyl) oxime (100 mg, 0.29 mmol, 1.0 eq.) in EtOH (5 mL) were added 5-chloro-4-(trifluoromethyl)-2-((2-(trimethylsilyl)ethoxy)methyl)pyridazin-3 (2H)-one (143 mg, 0.43 mmol, 1.5 eq.) and TEA (147 mg, 1.45 mmol, 5.0 eq.). The mixture was stirred at 60 °C for 4 h. The mixture was cooled to room temperature, diluted with water (100 mL) and extracted with ethyl acetate (2 x 100 mL.). The organic layers were combined and washed with brine (100 mL), dried over Na₂SO₄, concentrated under vacuum. The residue was purified by column chromatography (EtOAc/Petroleum ether= 1/1; V/V) to afford (S,E)-2-((6-oxo-5-(trifluoromethyl)-1-((2-(trimethylsilyl)ethoxy) methyl)-1,6-dihydropyridazin-4-yl)amino)propanal O-(2-(1-(5-(trifluoromethyl) pyrimidin-2-yl)piperidin-4-yl)ethyl) oxime (40 mg, 19.5%) as a yellow oil.
LCMS: *(ESI)* (M+H)⁺ = 638.2.
¹H NMR: (400 MHz, CDCl₃) *δ* 8.49 (s, 2H), 7.77 (s, 1H), 7.36 (d, *J* = 5.6 Hz, 1H), 6.04 - 5.96 (m, 1H), 5.48 - 5.38 (m, 2H), 4.90 - 4.83 (m, 2H), 4.50 - 4.41 (m, 1H), 4.19 - 4.14 (m, 2H), 3.77 - 3.70 (m, 2H), 3.03 - 2.91 (m, 2H), 1.93 - 1.82 (m, 2H), 1.70 - 1.63 (m, 2H), 1.57 - 1.47 (m, 3H), 1.29 - 1.18 (m, 3H), 1.03 - 0.95 (m, 2H), 0.03 (s, 9H).

### Step G: Synthesis of (S,E)-2-((6-oxo-5-(trifluoromethyl)-L6-dihydropyridazin -4-yl)amino)propanal O-(2-(1-(5-(trifluoromethyl)pyrimidin-2-yl)piperidin-4-yl)ethyl) oxime

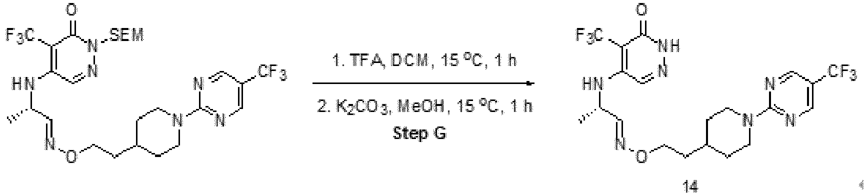

To a solution of (S,E)-2-((6-oxo-5-(trifluoromethyl)-1-((2-(trimethylsilyl)ethoxy) methyl)-1,6-dihydropyridazin-4-yl)amino)propanal O-(2-(1-(5-(trifluoromethyl) pyrimidin-2-yl)piperidin-4-yl) ethyl) oxime (30 mg, 0.05 mmol, 1.0 eq.) in DCM (1 mL) was added TFA (0.3 mL). The mixture was stirred at 15 °C for 1 h. The mixture was neutralized with saturated NaHCOs aqueous solution (20 mL) and extracted with DCM (3 x 40 mL). The organic layers were combined and dried over Na₂SO₄, concentrated under vacuum. The residue was redissolved in MeOH (2 mL) and potassium carbonate (24 mg, 0.17 mmol, 3.6 eq.) was added. The mixture was stirred at 15 °C for another 1 h. The mixture was filtered and the filtrate was purified by prep-HPLC to afford (S,E)-2-((6-oxo-5-(trifluoromethyl)-1,6-dihydropyridazin-4-yl)amino)propanal O-(2-(1-(5-(trifluoromethyl)pyrimidin-2-yl)piperidin-4-yl)ethyl) oxime (3.47 mg, 14.4% yield) as a white solid.
LCMS: *(ESI)* (M+H)⁺ = 507.8.
¹H NMR (400 MHz, CDCl₃) *δ* 10.96 (s, 1H), 8.45 (s, 2H), 7.72 (s, 1H), 7.32 (d, *J* = 5.2 Hz, 1H), 6.04 - 5.95 (m, 1H), 4.89 - 4.77 (m, 2H), 4.45 - 4.36 (m, 1H), 4.14 (t, *J* = 6.8 Hz, 2H), 3.01 - 2.85 (m, 2H), 1.86 - 1.77 (m, 2H), 1.75 - 1.67 (m, 1H), 1.66 - 1.58 (m, 4H), 1.50 (d, *J* = 6.4 Hz, 3H).

### Example 15:

### (S,E)-2-oxo-2-(4-(5-(trifluoromethyl)pyrimidin-2-yl)piperazin-1-yl)acetaldehyde O-(2-((6-oxo-5-(trifluoromethyl)-1,6-dihydropyridazin-4-yl)amino)propyl) oxime

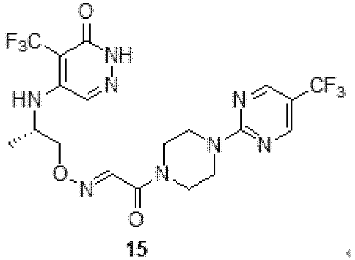

### Step A: Synthesis of (S)-2-(2-((6-oxo-5-(trifluoromethyl)-1-((2-(trimethylsilyl)ethoxy) methyl)-1,6-dihydropyridazin-4-yl)amino)propoxy)isoindoline-1,3-dione

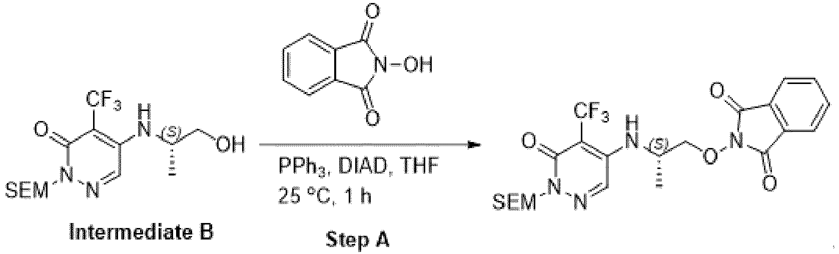

To a solution of (S)-5-((1-hydroxypropan-2-yl)amino)-4-(trifluoromethyl)-2-((2-(trimethylsilyl)ethoxy)methyl)pyridazin-3(2H)-one (360 mg, 0.981 mmol, 1.0 eq), 2-hydroxyisoindoline-1,3-dione (192 mg, 1.177 mmol, 1.2 eq) and PPh3 (308 mg, 1.177 mmol, 1.2 eq) in THF (5 mL) was added DEAD (238 mg, 1.177 mmol, 1.2 eq) at 0 °C under N₂ atmosphere. The mixture was stirred at 25 °C for 1 h under N₂ atmosphere. The mixture was concentrated under vacuum. The residue was dissolved in a mixed solvent of Petromleum ether and EtOAc (1/1; V/V, 50 mL). The mixture was filtered, the filtrate was concentrated under vacuum. The residue was purified by column chromatography (Petromleum ether/EtOAc= 2/3; V/V) to afford (S)-2-(2-((6-oxo-5-(trifluoromethyl)-1-((2-(trimethylsilyl)ethoxy)methyl)-1,6-dihydropy ridazin-4-yl)amino)propoxy)isoindoline-1,3-dione (350 mg, 70.0% yield) as a white solid.
LCMS: *(ESI)* (M+H)⁺ = 513.2.
¹H NMR: (400 MHz, DMSO) *δ* 8.10 (s, 1H), 7.96 - 7.82 (m, 4H), 6.83 (dd, J = 8.3, 3.8 Hz, 1H), 5.31 - 5.17 (m, 2H), 4.53 (s, 1H), 4.44 (dd, J = 10.8, 8.0 Hz, 1H), 4.26 (dd, J = 10.8, 3.8 Hz, 1H), 3.68 - 3.58 (m, 2H), 1.26 (d, J = 6.5 Hz, 3H), 0.95 - 0.83 (m, 2H), 0.03 - -0.02 (m, 9H).

### Step B: (S)-5-((1-(aminooxy)propan-2-yl)amino)-4-(trifluoromethyl)-2-((2-(trimethylsilyl)ethoxy)methyl)pyridazin-3(2H)-one (Intermediate J)

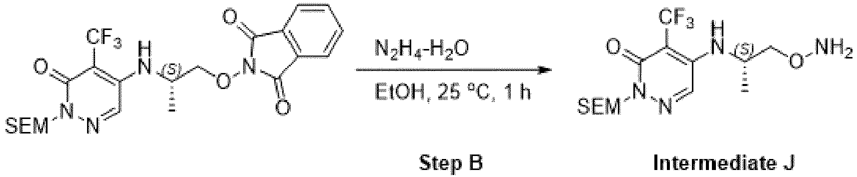

To a solution of (S)-2-(2-((6-oxo-5-(trifluoromethyl)-1-((2-(trimethylsilyl)ethoxy) methyl)-1,6-dihydropyridazin-4-yl)amino)propoxy)isoindoline-1,3-dione ( 350 mg, 0.683 mmol, 1.0 eq.) in EtOH (5 mL) was added hydrazine hydrate (100 mg, 1.025 mmol, 1.5 eq.) at 0 °C. The resulting mixture was stirred at 25 °C for 1h. The mixture was concentrated under vacuum. The formed precipitate was filtered and washed with MTBE (10 mL). The filtrate was concentrated in vacuum to afford (S)-5-((1-(aminooxy)propan-2-yl)amino)-4-(trifluoromethyl)-2-((2-(trimethylsilyl)ethox y)methyl)pyridazin-3(2H)-one (250 mg, 95.8% yield) as a white solid.
LCMS: *(ESI)* (M+H)⁺ = 383.2.

### Step C: Synthesis of (S,E)-2-((2-((6-oxo-5-(trifluoromethyl)-1-((2-(trimethylsilyl) ethoxy)methyl)-1,6-dihydropyridazin-4-yl)amino)propoxy)imino)acetic acid

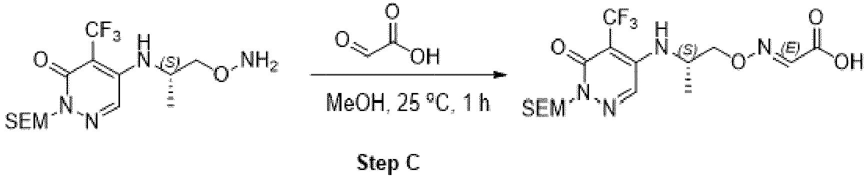

To a solution of (S)-5-((1-(aminooxy)propan-2-yl)amino)-4-(trifluoromethyl)-2-((2-(trimethylsilyl)ethox y)methyl)pyridazin-3(2H)-one (100 mg, 0.261 mmol, 1.0 eq.) in MeOH (3 mL) was added 2-oxoacetic acid (20 mg, 0.274 mmol, 1.05 eq.). The mixture was stirred at 25 °C for 1h. The mixture was diluted with water (5 mL) and extracted with EtOAc (2 × 5 mL). dried over Na₂SO₄ and concentrated under vacuum to afford (S,E)-2-((2-((6-oxo-5-(trifluoromethyl)-1-((2-(trimethylsilyl)ethoxy)methyl)-1,6-dihydr opyridazin-4-yl)amino)propoxy)imino)acetic acid (90 mg, 79% yield) as a white solid. LCMS: *(ESI)* (M+H)⁺ = 439.2.

### Step D: synthesis of (S,E)-2-oxo-2-(4-(5-(trifluoromethyl)pyrimidin-2-yl)piperazin-1-yl) acetaldehyde O-(2-((6-oxo-5-(trifluoromethyl)-1-((2-(trimethylsilyl)ethoxy)methyl)-1,6-dihydropyridazin-4-yl)amino)propyl) oxime

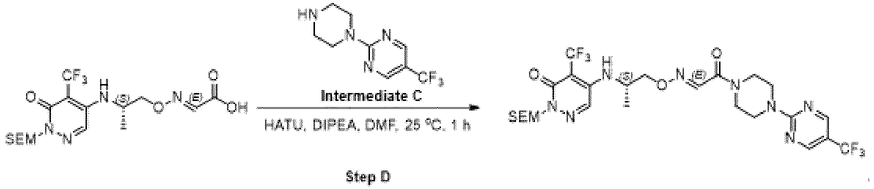

To a solution of (S,E)-2-((2-((6-oxo-5-(trifluoromethyl)-1-((2-(trimethylsilyl) ethoxy)methyl)-1,6-dihydropyridazin-4-yl)amino)propoxy)imino)acetic acid (90 mg, 0.205 mmol, 1.0 eq.) in DMF (2 mL) were added DIPEA (79 mg, 0.616 mmol, 3.0 eq.) and HATU (93 mg, 0.246 mmol, 1.2 eq.), followed by dropwise addition of 2-(piperazin-1-yl)-5-(trifluoromethyl)pyrimidine (57 mg, 0.246 mmol, 1.2 eq.). The resulting mixture was stirred at 25 °C for 1 h. The reaction mixture was quenched with water (10 mL) and extracted with EtOAc (3 × 10 mL). The organic lyaers were combined and washed with brine (10 mL), dried over Na₂SO₄ and concentrated under vacuum. The residue was purified by silica gel chromatography (Petroleum ether/EtOAc= 1/2; V/V) to afford (S,E)-2-oxo-2-(4-(5-(trifluoromethyl)pyrimidin -2-yl)piperazin-1-yl)acetaldehyde O-(2-((6-oxo-5-(trifluoromethyl)-1-((2-(trimethylsilyl) ethoxy)methyl)-1,6-dihydropyridazin-4-yl)amino)propyl) oxime (90 mg, 67% yield) as a white solid.
LCMS: *(ESI)* (M+H)⁺ = 653.2.

### Step E: synthesis of (S,E)-2-oxo-2-(4-(5-(trifluoromethyl)pyrimidin-2-yl)piperazin -1-yl)acetaldehyde O-(2-((6-oxo-5-(trifluoromethyl)-1,6-dihydropyridazin-4-yl)amino) propyl) oxime

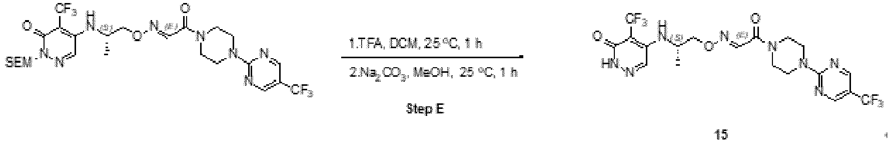

To a solution of (S,E)-2-oxo-2-(4-(5-(trifluoromethyl)pyrimidin-2-yl) piperazin-1-yl)acetaldehyde O-(2-((6-oxo-5-(trifluoromethyl)-1-((2-(trimethylsilyl) ethoxy)methyl)-1,6-dihydropyridazin-4-yl)amino)propyl) oxime (90 mg, 0.138 mmol, 1.0 eq.) in DCM (2 mL) was added TFA (0.3 mL). The resulting mixture was stirred at 25 °C for 1 h. The mixture was neutralized with saturated NaHCOs aqueous solution (5 mL) and extracted with DCM (3 x 10 mL). The organic layers were combined and dried over Na₂SO₄, concentrated under vacuum. The residue was redissolved in MeOH (5 mL) and Na2CO3 (36 mg, 0.345 mmol, 2.5 eq.) was added. The mixture was stirred at 25 °C for another 1 h. The reaction mixture was filtered and the filtrate was purified by reverse phase silica gel column chromatography (eluting with CH3CN/H₂O= 1/10; V/V) to afford (S,E)-2-oxo-2-(4-(5-(trifluoromethyl)pyrimidin-2-yl)piperazin-1-yl)acetaldehyde O-(2-((6-oxo-5-(trifluoromethyl)-1,6-dihydropyridazin-4-yl)amino)propyl) oxime (14 mg, 19% yield) as a white solid.
LCMS: *(ESI)* (M+H)⁺ = 522.8.
¹H NMR: (400 MHz, DMSO) *δ* 12.47 (s, 1H), 8.74 (s, 2H), 8.00 (s, 1H), 7.88 (s, 1H), 6.48 (dd, J = 8.3, 3.8 Hz, 1H), 4.32 - 4.11 (m, 3H), 3.91 - 3.84 (m, 4H), 3.67 - 3.60 (m, 4H), 1.21 (d, J = 6.3 Hz, 3H).

### Example 16:

### (S,E)-5-((1-(((1-oxo-1-(4-(5-(trifluoromethyl)pyrimidin-2-yl)piperazin-1-yl)propan-2-yl idene)amino)oxy)propan-2-yl)amino)-4-(trifluoromethyl)pyridazin-3(2H)-one

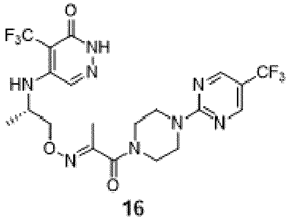

### Step A: (S,E)-2-((2-((6-oxo-5-(trifluoromethyl)-1-((2-(trimethylsilyl)ethoxy)methyl)-1,6 -dihydropyridazin-4-yl)amino)propoxy)imino)propanoic acid

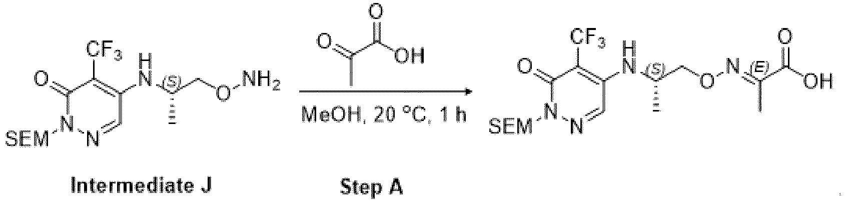

To a solution of (S)-5-((1-(aminooxy)propan-2-yl)amino)-4-(trifluoromethyl)-2-((2-(trimethylsilyl)ethoxy)methyl)pyridazin-3(2H)-one (90 mg, 0.235 mmol, 1.0 eq.) in MeOH (3 mL) was added 2-oxopropanoic acid (22 mg, 0.247 mmol, 1.05 eq.). The mixture was stirred at 20 °C for 1 h. The mixture was diluted with water (5 mL) and extracted with EtOAc (2 x 5 mL), dried over Na₂SO₄ and concentrated under vacuum to afford (S,E)-2-((2-((6-oxo-5-(trifluoromethyl)-1-((2-(trimethylsilyl)ethoxy)methyl)-1,6 -dihydropyridazin-4-yl)amino)propoxy)imino)propanoic acid (90 mg, 85% yield) as a white solid.
LCMS: *(ESI)* (M+H)⁺ = 453.2.

### Step B: synthesis of (S,E)-5-((1-(((1-oxo-1-(4-(5-(trifluoromethyl)pyrimidin-2-yl) piperazin-1-yl)propan-2-ylidene)amino)oxy)propan-2-yl)amino)-4-(trifluoromethyl)-2-( (2-(trimethylsilyl)ethoxy)methyl)pyridazin-3(2H)-one

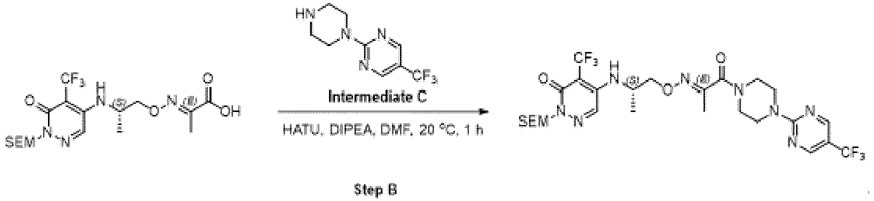

To a solution of (S,E)-2-((2-((6-oxo-5-(trifluoromethyl)-1-((2-(trimethylsilyl) ethoxy)methyl)-1,6-dihydropyridazin-4-yl)amino)propoxy)imino)propanoic acid (95 mg, 0.210 mmol, 1.0 eq.) in DMF (2 mL) were added DIPEA (81 mg, 0.630 mmol, 3.0 eq.) and HATU (96 mg, 0.252 mmol, 1.2 eq.), followed by dropwise addition of 2-(piperazin-1-yl)-5-(trifluoromethyl)pyrimidine (54 mg, 0.231 mmol, 1.2 eq.). The resulting mixture was stirred at 20 °C for 1 h. The reaction mixture was quenched with water (10 mL) and extracted with EtOAc (3 × 10 mL). The organic layers were combined and washed with brine (10 mL), dried over Na₂SO₄ and concentrated under vacuum. The residue was purified by silica gel column chromatography ( Petroleum ether/EtOAc=1/1; V/V) to afford (S,E)-5-((1-(((1-oxo-1-(4-(5-(trifluoromethyl) pyrimidin-2-yl)piperazin-1-yl)propan-2-ylidene)amino)oxy)propan-2-yl)amino)-4-(trifl uoromethyl)-2-((2-(trimethylsilyl)ethoxy)methyl)pyridazin-3(2H)-one (90 mg, 64% yield) as a yellow solid.
LCMS: *(ESI)* (M+H)⁺ = 667.2.
¹H NMR: (400 MHz, DMSO) *δ* 8.80 (s, 2H), 8.08 (s, 1H), 6.75 (dd, J = 8.3, 3.7 Hz, 1H), 5.27 (s, 2H), 4.49 - 4.33 (m, 1H), 4.31 - 4.18 (m, 2H), 4.02 - 3.83 (m, 4H), 3.61 - 3.68 (m, 6H), 1.95 (s, 3H), 1.28 (d, J = 6.5 Hz, 3H), 0.89 (dd, J = 10.4, 5.6 Hz, 2H), 0.05 (s, 9H).

### Step C: Synthesis of (S,E)-5-((1-(((1-oxo-1-(4-(5-(trifluoromethyl)pyrimidin-2-yl) piperazin-1-yl)propan-2-ylidene)amino)oxy)propan-2-yl)amino)-4-(trifluoromethyl)pyri dazin-3(2H)-one

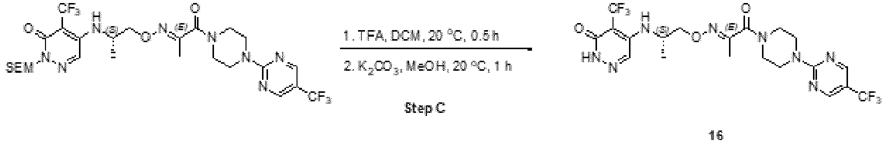

To a solution of (S,E)-5-((1-(((1-oxo-1-(4-(5-(trifluoromethyl)pyrimidin-2-yl) piperazin-1-yl)propan-2-ylidene)amino)oxy)propan-2-yl)amino)-4-(trifluoromethyl)-2-( (2-(trimethylsilyl)ethoxy)methyl)pyridazin-3(2H)-one (90 mg, 0.135 mmol, 1.0 eq.) in DCM (2 mL) was added TFA (0.3 mL). The resulting mixture was stirred at 20 °C for 0.5 h. The mixture was neutralized with saturated NaHCO₃ aqueous solution (5 mL) and extracted with DCM (3 x 10 mL). The organic layers were combined and dried over Na₂SO₄, concentrated under vacuum. The residue was redissolved in MeOH (5 mL) and K₂CO₃ (46 mg, 0.3338 mmol, 2.5 eq.) was added. The mixture was stirred at 20 °C for another 1 h. The reaction mixture was filtered and the filtrate was purified by reverse phase silica gel column chromatography (eluting with CH3CN/H₂O= 1/10 ~ 10/1; V/V) to afford (S,E)-5-((1-(((1-oxo-1-(4-(5-(trifluoromethyl)pyrimidin-2-yl)piperazin-1-yl) propan-2-ylidene)amino)oxy)propan-2-yl)amino)-4-(trifluoromethyl)pyridazin-3(2H)-o ne (52 mg, 72% yield) as a white solid.
LCMS: *(ESI)* (M+H)⁺ = 537.2.
¹H NMR: (400 MHz, DMSO) *δ* 12.49 (s, 1H), 8.77 (s, 2H), 7.93 (s, 1H), 6.54 (d, J = 4.2 Hz, 1H), 4.32 (s, 1H), 4.24 - 4.17 (m, 2H), 3.92 - 3.87 (m, 4H), 3.58 - 3.66 (m, 4H), 1.92 (s, 3H), 1.23 (d, J = 6.4 Hz, 3H).

### Example 17:

### (S)-N-(2-((6-oxo-5-(trifluoromethyl)-1,6-dihydropyridazin-4-yl)amino)propoxy)-2-(1-( 5-(trifluoromethyl)pyrimidin-2-yl)piperidin-4-yl)acetamide

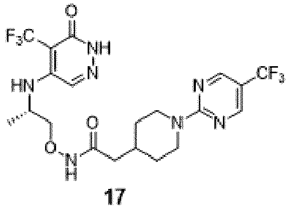

### 17.1) Synthesis of intermediate S

### Step A: synthesis of ethyl 2-(1-(5-(trifluoromethyl)pyrimidin-2-yl)piperidin-4-yl)acetate

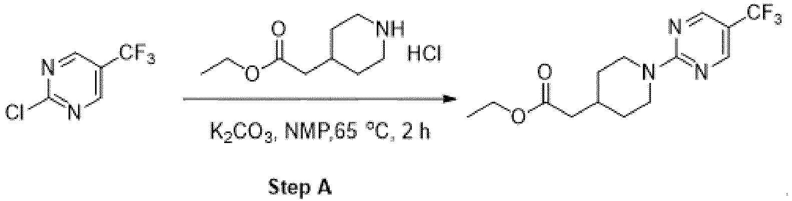

To a solution of 2-chloro-5-(trifluoromethyl)pyrimidine (200 mg, 1.09 mmol, 1.0 eq.) in NMP (3 mL) were added K₂CO₃ (455 mg, 3.29 mmol, 3.0 eq.) and ethyl 2-(piperidin-4-yl)acetate hydrochloride (251 mg, 1.19 mmol, 1.1 eq.), the resulting mixture was stirred at 65 °C for 2 h. The mixture was cooled to room temperature and diluted with water (5 mL). The mixture was extracted with EtOAc (3 x 10 mL). The organic layers were combined and washed with brine (10 mL), dried over Na₂SO₄ and concentrated under reduced pressure. The residue was purified by column chromatography (Petroleum ether/EtOAc= 4/1; V/V) to afford ethyl 2-(1-(5-(trifluoromethyl)pyrimidin-2-yl)piperidin-4-yl)acetate (300 mg, 86.8%) as a white solid.
LCMS: (*ESI)*(M+H)⁺ = 318.2.
¹H NMR: (400 MHz, DMSO) *δ* 8.66(s, 2H), 4.70(d, J = 13.3 Hz, 2H), 4.07(q, J = 7.1 Hz, 2H), 3.00(td, J = 13.0, 2.4 Hz, 2H), 2.27(d, J = 7.1 Hz, 2H), 2.10 - 1.93(m, 1H), 1.75(dd, J = 12.6, 2.1 Hz, 2H), 1.21 - 1.08(m, 5H).

### Step B: Synthesis of 2-(1-(5-(trifluoromethyl)pyrimidin-2-yl)piperidin-4-yl)acetic acid (Intermediate S)

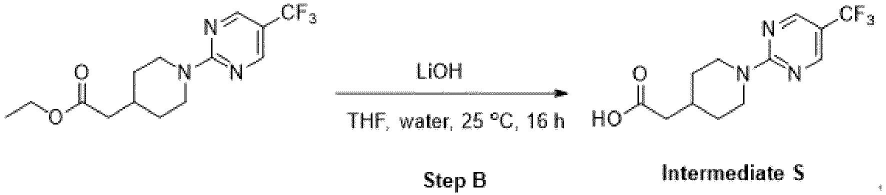

To a solution of ethyl 2-(1-(5-(trifluoromethyl)pyrimidin-2-yl)piperidin-4-yl) acetate (300 mg, 0.946 mmol, 1.0 eq.) in a mixed solvent of THF (3 mL) and water (3 mL) was added LiOH (68 mg, 2.839 mmol, 3.0 eq.). The mixture was stirred at 25 °C for 16 h. The mixture was diluted with water (5 mL) and extracted with EtOAc (3 x 10 mL). The water layer was adjusted to pH 6 by citric acid. The resulting mixture was extracted with EtOAc (3 x 15 mL), dried over Na₂SO₄ and concentrated under vacuum to afford 2-(1-(5-(trifluoromethyl)pyrimidin-2-yl)piperidin-4-yl)acetic acid (220 mg, 80% yield) as a white solid.
LCMS: (*ESI)*(M+H)⁺ = 290.2.

### 17.2) Synthesis of intermediate V

### Step A: synthesis of (S)-N-(2-((6-oxo-5-(trifluoromethyl)-1-((2-(trimethylsilyl)ethoxy) methyl)-1,6-dihydropyridazin-4-yl)amino)propoxy)-2-(1-(5-(trifluoromethyl)pyrimidin-2-yl)piperidin-4-yl)acetamide

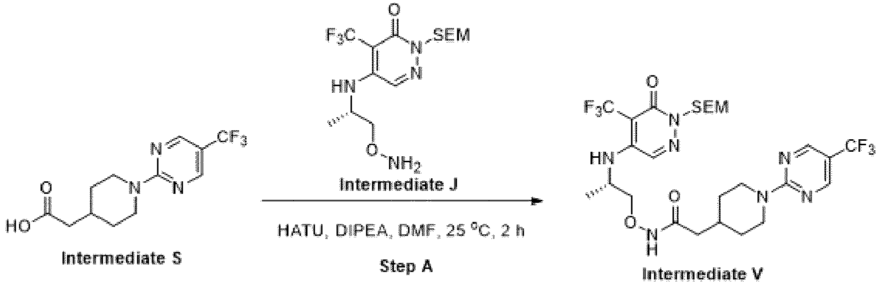

To a solution of 2-(1-(5-(trifluoromethyl)pyrimidin-2-yl)piperidin-4-yl)acetic acid (69 mg, 0.238 mmol, 1.3 eq.) in DMF (2 mL) were added DIPEA (118 mg, 0.914 mmol, 5.0 eq.) and HATU (83 mg, 0.219 mmol, 1.2 eq.), followed by dropwise addition of (S)-5-((1-(aminooxy)propan-2-yl)amino)-4-(trifluoromethyl)-2-((2-(trimethylsilyl)ethox y)methyl)pyridazin-3(2H)-one (70 mg, 0.183 mmol, 1.0 eq.). The resulting mixture was stirred at 25 °C for 2 h. The reaction mixture was quenched with water (10 mL) and extracted with EtOAc (3 x 10 mL). The organic lyaers were combined and washed with brine (10 mL), dried over Na₂SO₄ and concentrated under vacuum. The residue was purified by silica gel column chromatography (Petroleum ether/EtOAc= 1/2; V/V) to afford (S)-N-(2-((6-oxo-5-(trifluoromethyl)-1-((2-(trimethylsilyl)ethoxy)methyl)-1,6-dihydropyridazin-4-yl)amino)propoxy)-2-(1-(5-(trifluoromethyl)pyrimidin-2-yl)piperidi n-4-yl)acetamide (80 mg, 67% yield) as a white solid.
LCMS: (*ESI)*(M+H)⁺ = 654.2.

### 17.3) Synthesis of compound 17

### Step A: synthesis of (S)-N-(2-((6-oxo-5-(trifluoromethyl)-L6-dihydropyridazin-4-yl) amino)propoxy)-2-(1-(5-(trifluoromethyl)pyrimidin-2-yl)piperidin-4-yl)acetamide

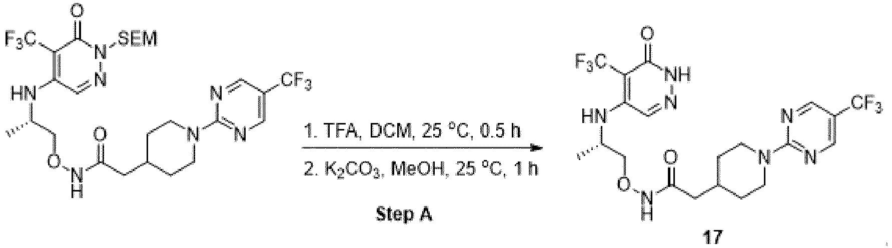

To a solution of (S)-N-(2-((6-oxo-5-(trifluoromethyl)-1-((2-(trimethylsilyl)ethoxy) methyl)-1,6-dihydropyridazin-4-yl)amino)propoxy)-2-(1-(5-(trifluoromethyl)pyrimidin-2-yl)piperidin-4-yl)acetamide (80 mg, 0.122 mmol, 1.0 eq.) in DCM (2 mL) was added TFA (0.3 mL). The resulting mixture was stirred at 25 °C for 0.5 h. The mixture was neutralized with saturated NaHCOs aqueous solution (5 mL) and extracted with DCM (3 × 10 mL). The organic layers were combined and dried over Na₂SO₄, concentrated under vacuum. The residue was redissolved in MeOH (5 mL) and K₂CO₃ (42 mg, 0.306 mmol, 2.5 eq.) was added. The mixture was stirred at 25 °C for another 1 h. The reaction mixture was filtered and the filtrate was purified by reverse phase silica gel column chromatography (eluting with CH3CN/H₂O= 1/10 ~ 10/1; V/V) to afford (S)-N-(2-((6-oxo-5-(trifluoromethyl)-1,6-dihydropyridazin-4-yl)amino)propoxy)-2-(1-( 5-(trifluoromethyl)pyrimidin-2-yl)piperidin-4-yl)acetamide (25.58 mg, 40% yield) as a white solid.
LCMS: (*ESI)*(M+H)⁺ = 524.2.
¹H NMR: (400 MHz, DMSO) *δ* 12.48 (s, 1H), 11.13 (s, 1H), 8.66 (s, 2H), 7.90 (s, 1H), 6.61 (s, 1H), 4.67 (d, J = 13.1 Hz, 2H), 4.20 (s, 1H), 3.94 - 3.78 (m, 2H), 2.98 (t, J = 12.2 Hz, 2H), 2.04 - 2.04 (m, 1H), 1.92 (d, J = 6.9 Hz, 2H), 1.72 (d, J = 12.7 Hz, 2H), 1.19 (d, J = 6.4 Hz, 3H), 1.15 - 1.01 (m, 2H).

### Example 18:

### (E)-2-(1-oxo-1,2-dihydrophthalazin-5-yl)propanal O-(2-oxo-2-(4-(5-(trifluoromethyl) pyrimidin-2-yl)piperazin-1-yl)ethyl) oxime

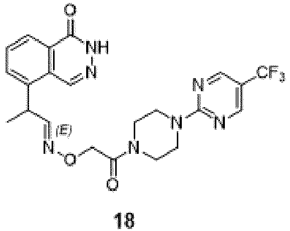

### Step A: synthesis of 4-bromo-3-hydroxyisobenzofuran-1(3H)-one

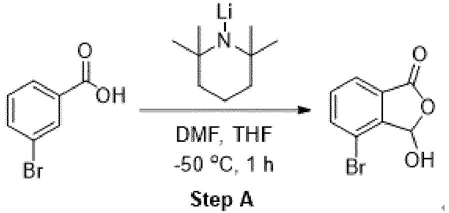

To a stirred solution of n-BuLi (2.5 M in hexanes, 45 mL, 112.50 mmol, 2.2 eq.) was added dropwise a solution of 2,2,6,6-tetramethylhexahydropyridine (15.71 g, 111.19 mmol, 2.2 eq.) in anhydrous THF (120 mL) at -20 °C under nitrogen. The mixture was stirred at -20 °C for 30 min. After cooling to -50 °C, a solution of 3-bromobenzoic acid (10.16 g, 50.54 mmol, 1.0 eq.) in anhydrous THF (40 mL) was added dropwise and the mixture was stirred at -50 °C for 1 h. The mixture was then treated with DMF (15 mL, 202.17 mmol, 4.0 eq.) at -50 °C. The resulting solution was allowed to warm up to room temperature and quenched with water (100 mL). The mixture was acidified to pH 4 with 2M HCl aqueous and extracted with EtOAc (3 x 100 mL). The organic layers were combined and washed with brine (2 x 100 mL), dried over Na₂SO₄ and concentrated under vacuum. The residue was purified by crystallization with a mixed solvent of EtOAc and Petroleum ether (1/1; V/V, 300 mL) to give 4-bromo-3-hydroxyisobenzofuran-1(3H)-one (10.65 g, 92.0% yield) as a yellow solid. LCMS: *(ESI)* (M+H)⁺ = 229.0.

### Step B: synthesis of 5-bromophthalazin-1(2H)-one

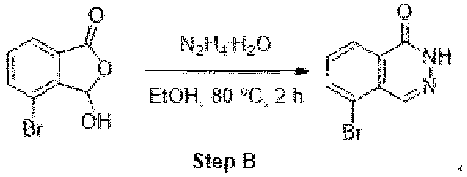

To a solution of 4-bromo-3-hydroxyisobenzofuran-1(3H)-one (10.65 g, 46.50 mmol, 1.0 eq.) in EtOH (170 mL) was added hydrazine hydrate (13.02 g, 260.40 mmol, 5.6 eq.). The resulting solution was stirred at 80 °C for 2 h. The mixture was cooled to room temperature and quenched by water (150 mL). The mxiture was extracted with EtOAc (3 x 150 mL). The organic layers were combined and washed with brine (300 mL), dried over Na₂SO₄ and concentrated under vacuum. The residue was purified by column chromatography (DCM/MeOH= 10/1) to afford 5-bromophthalazin-1(2H)-one (3.5 g, 33.5% yield) as white solid.
LCMS: *(ESI)* (M+H)⁺ = 225.0.
¹H NMR: (400 MHz, DMSO-*d₆*) *δ* 12.84(br s, 1H), 8.42(s, 1H), 8.24(d, J = 8.0 Hz, 1H), 8.21(dd, J = 8.0, 1.2 Hz, 1H), 7.76(t, J = 8.0 Hz, 1H).

### Step C: synthesis of 5-bromo-2-((2-(trimethylsilyl)ethoxy)methyl)phthalazin-1(2H)-one

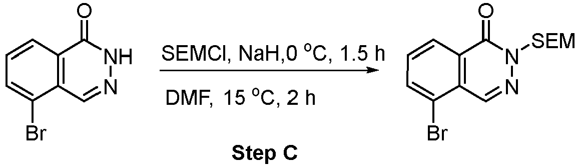

To a solution of 5-bromophthalazin-1(2H)-one (2.25 g, 4.00 mmol, 1.0 eq.) in DMF (50 mL) was added NaH (60%, 400 mg, 10.00 mmol, 2.5 eq.) at 0 °C. The mixture was stirred at 0 °C for 1.5 h. 2-(trimethylsilyl)ethoxymethyl chloride (1.00 g, 6.00 mmol, 1.5 eq.) was added to the mixture at 0 °C. The mixture was stirred at 15 °C for another 2 h. The mixture was quenched with water (100 mL) and extracted with EtOAc (2 x 100 mL). The organic layers were combined and washed with brine (50 mL), dried over Na₂SO₄ and concentrated under vacuum. The residue was purified by column chromatography (EtOAc/Petroleum ether= 1/10; V/V) to afford 5-bromo-2-((2-(trimethylsilyl)ethoxy)methyl)phthalazin-1(2H)-one (2.40 g, 67.9% yield) as a yellow oil.
LCMS: *(ESI)* (M+H-28)⁺ = 327.0.
¹H NMR: (400 MHz, CDCl₃) *δ* 8.44 (s, 1H), 8.36 (d, *J* = 7.6 Hz, 1H), 7.94 (d, *J* = 7.6 Hz, 1H), 7.55 (t, *J* = 8.0 Hz, 1H), 5.51 (s, 2H), 3.71 - 3.66 (m, 2H), 0.96 - 0.91 (m, 2H), -0.06 (s, 9H).

### Step D: synthesis of 5-(prop-1-en-2-yl)-2-((2-(trimethylsilyl)ethoxy)methyl)phthalazin -1(2H)-one

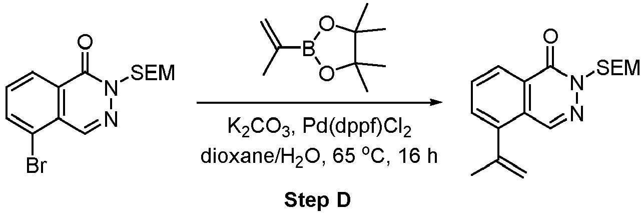

To a solution of 5-bromo-2-((2-(trimethylsilyl)ethoxy)methyl)phthalazin-1(2H) -one (4.50 g, 12.66 mmol, 1.0 eq.) in a mixed solvent of dioxane and H₂O (4/1; V/V, 45 mL) were added Isopropenylboronic acid pinacol ester (2.34 g, 13.93 mmol, 1.1 eq.), K₂CO₃ (3.50 g, 25.33 mmol, 2.0 eq.) and Pd(dppf)Cl2 (0.93 g, 1.27 mmol, 0.1 eq.) at r.t. under nitrogen. The mixture was stirred at 65 °C for 16 h under nitrogen. The mixture was cooled to room temperature, diluted with water (80 mL) and extracted with ethyl acetate (2 x 100 mL). The organic layers were combined and dried over Na₂SO₄, concentrated under vacuum to afford 5-(prop-1-en-2-yl)-2-((2-(trimethylsilyl)ethoxy) methyl)phthalazin-1(2H)-one (3.10 g, 62.7%) as a yellow solid.
LCMS: *(ESI)* (M+H-28)⁺ = 289.0.

### Step E: synthesis of 5-(1-hydroxypropan-2-yl)-2-((2-(trimethylsilyl)ethoxy)methyl) phthalazin-1(2H)-one

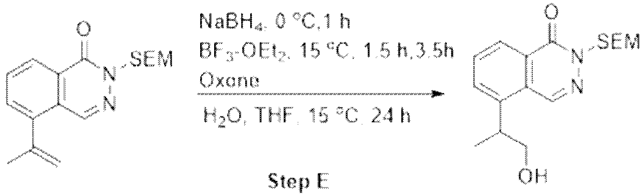

To a solution of 5-(prop-1-en-2-yl)-2-((2-(trimethylsilyl)ethoxy)methyl)phthalazin -1(2H)-one (3.10 g, 9.80 mmol, 1.0 eq.) in THF (85 mL) was added NaBH₄ (0.59 g, 15.67 mmol, 1.6 eq.) at 0 °C. The mixture was stirred at 0 °C for 1 h. A solution of BF₃-OEt₂ (1.53 g, 10.78 mmol, 1.1 eq.) in THF (5 mL) was added to the above mixture. The mixture was stirred at 15 °C for another 1.5 h. Water (5 mL) was added to the mixture at 0 °C and the mixture was stirred at 15 °C for 3.5 h. A solution of Oxone (9.98 g, 33.30 mmol, 3.4 eq.) in water (5 mL) was added to the mixture at 0 °C. The mixture was stirred at 15 °C for 24 h. The mixture was diluted with water (80 mL) and extracted with ethyl acetate (2 x 100 mL). The organic layers were combined and washed with brine (50 mL), dried over Na₂SO₄ and concentrated under vacuum. The residue was purified by column chromatography (EtOAc/Petroleum ether= 1/2; V/V) to afford 5-(1-hydroxypropan-2-yl)-2-((2-(trimethylsilyl)ethoxy)methyl)phthalazin-1(2H)-one (1.40 g, 39.3%) as a yellow solid.
LCMS: *(ESI)* (M+H-28)⁺ = 307.2.

### Step F: synthesis of 2-(1-oxo-2-((2-(trimethylsilyl)ethoxy)methyl)-1,2-dihydrophthalazin-5-yl)propanal

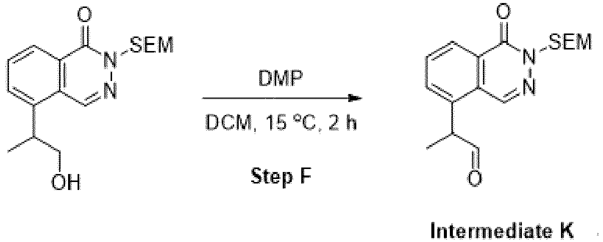

To a solution of 5-(1-hydroxypropan-2-yl)-2-((2-(trimethylsilyl)ethoxy) methyl)phthalazin-1(2H)-one (700 mg, 2.09 mmol, 1.0 eq.) in DCM (15 mL) was added Dess-Martin Periodinane (1.33 g, 3.14 mmol, 1.5 eq.) at room temperature. The mixture was stirred at 15 °C for 2 h. The mixture was quenched with saturated NaHCO₃ aqueous solution (50 mL) and extracted with ethyl acetate (2 x 100 mL). The organic layers were combined and washed with brine (100 mL), dried over Na₂SO₄ and concentrated under vacuum. The residue was purified by column chromatography (EtOAc/Petroleum ether= 2/1; V/V) to afford 2-(1-oxo-2-((2-(trimethylsilyl)ethoxy)methyl)-1,2-dihydrophthalazin -5-yl)propanal (350 mg, 50.3%) as a yellow oil.
LCMS: *(ESI)* (M+H-28)⁺ = 304.8.

### Step G: synthesis of (E)-2-(1-oxo-2-((2-(trimethylsilyl)ethoxy)methyl) -1,2-dihydrophthalazin-5-yl)propanal O-(2-oxo-2-(4-(5-(trifluoromethyl)pyrimidin-2-yl) piperazin-1-yl)ethyl) oxime

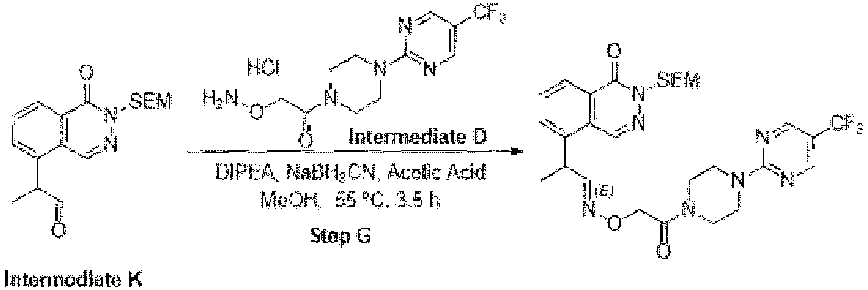

To a solution of 2-(aminooxy)-1-(4-(5-(trifluoromethyl)pyrimidin-2-yl) piperazin-1-yl)ethanone hydrochloride (100 mg, 0.33 mmol, 1.0 eq.) in MeOH (5 mL) were added 2-(1-oxo-2-((2-(trimethylsilyl)ethoxy)methyl)-1,2-dihydrophthalazin-5-yl) propanal (131 mg, 0.39 mmol, 1.2 eq.) and DIPEA (128 mg, 0.98 mmol, 3.0 eq.). The mixture was stirred at 55 °C for 3 h. Acetic acid (79 mg, 1.31 mmol, 4.0 eq.) and sodium cyanoborohydride (31 mg, 0.49 mmol, 1.5 eq.) were added to the mixture. The mixture was stirred at 55 °C for another 0.5 h. The mixture was quenched with saturated NaHCOs aqueous solution (50 mL) and extracted with ethyl acetate (2 x 100 mL). The organic layers were combined and washed with brine (50 mL), dried over Na₂SO₄ and concentrated under vacuum. The residue was purified by column chromatography (EtOAc/Petroleum ether= 2/1; V/V) to afford (E)-2-(1-oxo-2-((2-(trimethylsilyl)ethoxy) methyl)-1,2-dihydrophthalazin-5-yl)propanal O-(2-oxo-2-(4-(5-(trifluoromethyl) pyrimidin-2-yl)piperazin-1-yl)ethyl) oxime (40 mg, 22.1%) as a yellow oil.
LCMS: *(ESI)* (M+H-28)⁺ = 592.2.
¹H NMR: (400 MHz, CDCl₃) *δ* 8.59 - 8.52 (m, 3H), 8.43 (d, J = 8.0 Hz, 1H), 7.82 - 7.71 (m, 2H), 7.67 (d, J = 6.4 Hz, 1H), 5.66 - 5.54 (m, 2H), 4.89 - 4.79 (m, 2H), 4.45 - 4.33 (m, 1H), 4.00 - 3.93 (s, 4H), 3.81 - 3.73 (m, 4H), 3.64 - 3.52 (m, 2H), 1.63 (d, J = 7.2 Hz, 3H), 1.07 - 0.98 (m, 2H), 0.03 (s, 9H).

### Step H: Synthesis of (E)-2-(1-oxo-1,2-dihydrophthalazin-5-yl)propanal O-(2-oxo-2-(4-(5-(trifluoromethyl)pyrimidin-2-yl)piperazin-1-yl)ethyl) oxime

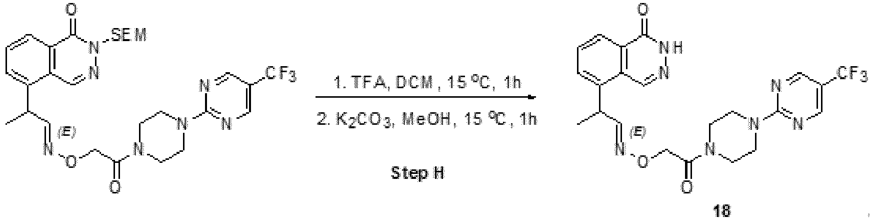

To a solution of (E)-2-(1-oxo-2-((2-(trimethylsilyl)ethoxy)methyl)-1,2-dihydrophthalazin-5-yl)propanal O-(2-oxo-2-(4-(5-(trifluoromethyl)pyrimidin-2-yl)piperazin-1-yl)ethyl) oxime (35 mg, 0.06 mmol, 1.0 eq.) in DCM (8 mL) was added TFA (1 mL). The mixture was stirred at 15 °C for 1 h. The mixture was neutralized with saturated NaHCO₃ aqueous solution (50 mL) and extracted with DCM (3 x 80 mL). The organic layers were combined and dried over Na₂SO₄, concentrated under vacuum. The residue was redissolved in MeOH (3 mL) and potassium carbonate (28 mg, 0.21 mmol, 3.6 eq.) was added. The mixture was stirred at 15 °C for another 1 h. The mixture was filtered and the filtrate was purified by prep-HPLC to afford (E)-2-(1-oxo-1,2-dihydrophthalazin-5-yl)propanal O-(2-oxo-2-(4-(5-(trifluoromethyl)pyrimidin-2-yl)piperazin-1-yl)ethyl) oxime (9.48 mg, 33.8% yield) as a white solid.
LCMS: *(ESI)* (M+H)⁺=490.2.
¹H NMR: (400 MHz, CDCl₃) *δ* 10.49 (s, 1H), 8.59 - 8.51 (m, 3H), 8.41 (d, J = 7.6 Hz, 1H), 7.83 - 7.72 (m, 2H), 7.67 (d, J = 6.4 Hz, 1H), 4.82 (s, 2H), 4.45 - 4.35 (m, 1H), 4.01 - 3.90 (m, 4H), 3.81 - 3.72 (m, 2H), 3.63 - 3.54 (m, 2H), 1.64 (d, J = 6.8 Hz, 3H).

### Example 19:

### (E)-2-(1-oxo-1,2-dihydrophthalazin-5-yl)propanal O-(2-oxo-2-(4-(5-(trifluoromethyl) pyridin -2-yl)piperazin-1-yl)ethyl) oxime

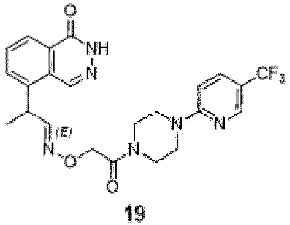

### Step A: synthesis of tert-butyl 2-oxo-2-(4-(5-(trifluoromethyl)pyridin-2-yl) piperazin-1-yl)ethoxycarbamate

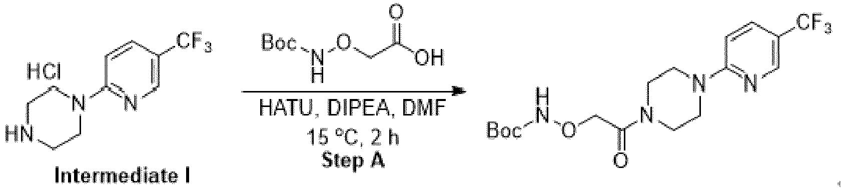

To a solution of 1-(5-(trifluoromethyl)pyridin-2-yl)piperazine hydrochloride (808 mg, 3.02 mmol, 1.0 eq.) in DMF (20 mL) were added DIPEA (1.95 g, 15.09 mmol, 5.0 eq.) and HATU (1.38 g, 3.62 mmol, 1.2 eq.), followed by the dropwise addition of 2-(((tert-butoxycarbonyl)amino)oxy)acetic acid (635 mg, 3.32 mmol, 1.1 eq.). The resulting mixture was stirred at 15 °C for 2 h. The reaction mixture was quenched with water (100 mL) and extracted with EtOAc (2 x 100 mL). The organic layers were combined and washed with brine (50 mL), dried over Na₂SO₄ and concentrated under vacuum. The residue was purified by silica gel chromatography (EtOAc/Petroleum ether= 1/3; V/V) to afford tert-butyl 2-oxo-2-(4-(5-(trifluoromethyl)pyridin-2-yl) piperazin-1-yl)ethoxycarbamate (1.03 g, 84.4% yield) as a yellow oil.
LCMS: *(ESI)* (M+H-56)⁺ = 348.8.

### Step B: synthesis of 2-(aminooxy)-1-(4-(5-(trifluoromethyl)pyridin-2-yl)piperazin -1-yl)ethanone hydrochloride

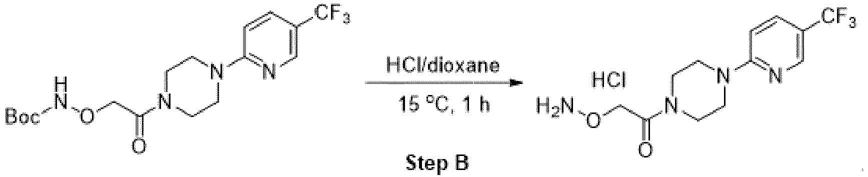

A solution of tert-butyl 2-oxo-2-(4-(5-(trifluoromethyl)pyridin-2-yl)piperazin-1-yl) ethoxycarbamate (700 mg, 1.73 mmol, 1.0 eq.) in HCl/dioxane (4M, 10 mL) was stirred at 15 °C for 1 h. The reaction mixture was concentrated under vacuum to afford 2-(aminooxy)-1-(4-(5-(trifluoromethyl)pyridin-2-yl)piperazin-1-yl)ethanone hydrochloride (590 mg, 99.9% yield) as a white solid.
LCMS: *(ESI)* (M+H)⁺ = 305.2.

### Step C: synthesis of (E)-2-(1-oxo-2-((2-(trimethylsilyl)ethoxy)methyl) -1,2-dihydrophthalazin-5-yl)propanal O-(2-oxo-2-(4-(5-(trifluoromethyl)pyridin-2-yl) piperazin-1-yl)ethyl) oxime

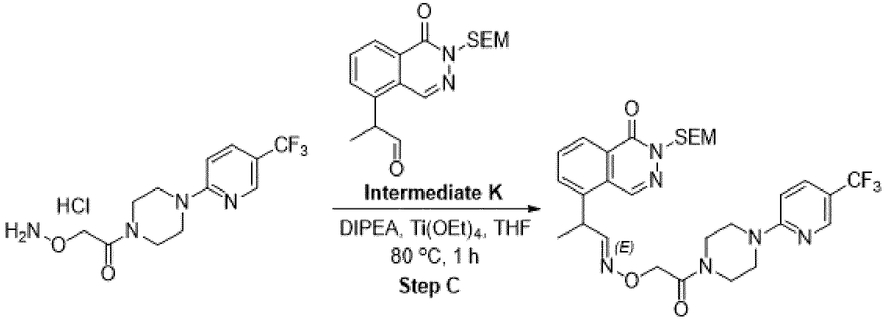

To a solution of 2-(aminooxy)-1-(4-(5-(trifluoromethyl)pyridin-2-yl)piperazin -1-yl)ethanone hydrochloride (100 mg, 0.29 mmol, 1.0 eq.) in THF (10 mL) were added 2-(1-oxo-2-((2-(trimethylsilyl)ethoxy)methyl)-1,2-dihydrophthalazin-5-yl)propanal (118 mg, 0.35 mmol, 1.2 eq.), DIPEA (114 mg, 0.88 mmol, 3.0 eq.) and tetraethoxy titanium (74 mg, 0.32 mmol, 1.1 eq.). The mixture was stirred at 80 °C for 1 h. The mixture was diluted with water (50 mL) and extracted with ethyl acetate (2 x 100 mL). The organic layers were washed with brine (50 mL), dried over Na₂SO₄ and concentrated under vacuum. The residue was purified by column chromatography (EtOAc/Petroleum ether= 2 /3; V/V) to afford (E)-2-(1-oxo-2-((2-(trimethylsilyl)ethoxy)methyl) -1,2-dihydrophthalazin-5-yl)propanal O-(2-oxo-2-(4-(5-(trifluoromethyl)pyridin-2-yl) piperazin-1-yl)ethyl) oxime (80 mg, 44.1%) as a yellow oil.
LCMS: *(ESI)* (M+H)⁺ = 620.2.

### Step D: Synthesis of (E)-2-(1-oxo-1,2-dihydrophthalazin-5-yl)propanal O-(2-oxo-2 -(4-(5-(trifluoromethyl)pyridin-2-yl)piperazin-1-yl)ethyl) oxime

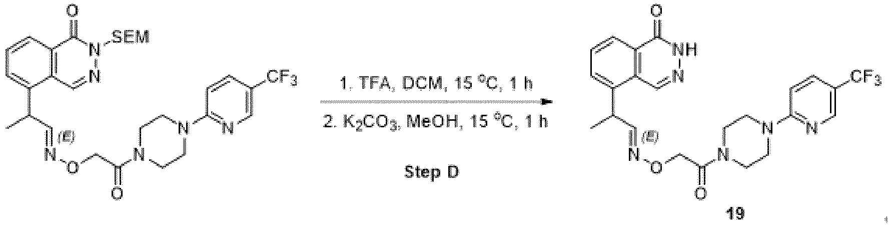

To a solution of (E)-2-(1-oxo-2-((2-(trimethylsilyl)ethoxy)methyl)-1,2-dihydrophthalazin-5-yl)propanal O-(2-oxo-2-(4-(5-(trifluoromethyl)pyridin-2-yl) piperazin-1-yl)ethyl) oxime (60 mg, 0.097 mmol, 1.0 eq.) in DCM (5 mL) was added TFA (1.5 mL). The mixture was stirred at 15 °C for 1 h. The mixture was neutralized with saturated NaHCO₃ aqueous solution (50 mL) and extracted with DCM (3 x 80 mL). The organic layers were combined and dried over Na₂SO₄, concentrated under vacuum. The residue was redissolved in MeOH (3 mL) and potassium carbonate (49 mg, 0.35 mmol, 3.6 eq.) was added. The mixture was stirred at 15 °C for another 1 h. The mixture was filtered and the filtrate was purified by prep-HPLC to afford (E)-2-(1-oxo-1,2-dihydrophthalazin-5-yl)propanal O-(2-oxo-2-(4-(5-(trifluoromethyl) pyridin-2-yl)piperazin-1-yl)ethyl) oxime (9.26 mg, 16.2% yield) as a white solid.
LCMS: *(ESI)* (M+H)⁺ = 488.8.
¹H NMR (400 MHz, CDCl₃) *δ* 10.20 (s, 1H), 8.50 (s, 1H), 8.43 - 8.39 (m, 1H), 8.36 (dd, *J* = 7.2, 1.6 Hz, 1H), 7.78 - 7.70 (m, 2H), 7.69 - 7.65 (m, 1H), 7.63 (d, *J* = 6.4 Hz, 1H), 6.68 - 6.62 (m, 1H), 4.77 (s, 2H), 4.40 - 4.32 (m, 1H), 3.78 - 3.69 (m, 4H), 3.64 - 3.58 (m, 4H), 1.59 (d, *J* = 7.2 Hz, 3H).

### Example 20:

### (S)-N-(2-((6-oxo-5-(trifluoromethyl)-1,6-dihydropyridazin-4-yl)amino)propoxy)-2-(1-( 5-(trifluoromethyl)pyrimidin-2-yl)piperidin-4-ylidene)acetamide

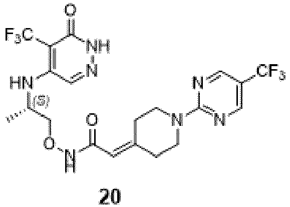

### 20.1) Synthesis of intermediate O and Synthesis of intermediate N

### Step A: synthesis of tert-butyl 4-(2-methoxy-2-oxoethylidene)piperidine-1-carboxylate

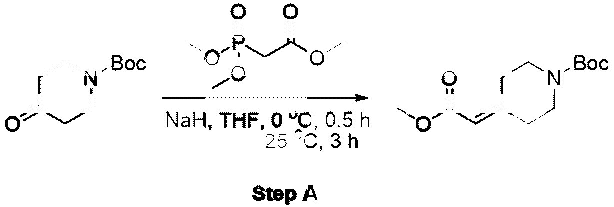

To a solution of trimethyl phosphonoacetate (27.32 g, 150.00 mmol, 1.0 eq.) in THF (350 mL) was added NaH (60%, 6.30 g, 157.50 mmol, 1.05 eq.) in portions at 0 °C in ice-water bath under N₂ atmosphere. The mixture was stirred at 0 °C for 0.5 h. N-(tert-Butoxycarbonyl)-4-piperidone (29.89 g, 150.00 mmol, 1.0 eq.) was added to the mixture. The mixture was stirred at 25 °C for 3 h. The mixture was quenched with cold saturated NH₄Cl aqueous solution (400 mL) and extracted with EtOAc (2 x 350 mL). The organic layers were combined and dried over Na₂SO₄, concentrated under vacuum. The residue was purified by silica gel chromatography (EtOAc/Petroleum ether= 1/10; V/V) to afford tert-butyl 4-(2-methoxy-2-oxoethylidene)piperidine-1-carboxylate (30.83 g, 80.5%) as a white solid.
LCMS: *(ESI)* (M+H-100)⁺ = 156.2.
¹H NMR: (400 MHz, CDCl₃) *δ* 5.65 (s, 1H), 3.63 (s, 3H), 3.48 - 3.35 (m, 4H), 2.91 - 2.82 (m, 2H), 2.26 - 2.15 (m, 2H), 1.40 (s, 9H).

### Step B: synthesis of methyl 2-(piperidin-4-ylidene)acetate hydrochloride (Intermediate O)

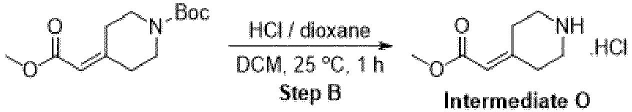

To mixture of tert-butyl 4-(2-methoxy-2-oxoethylidene)piperidine-1-carboxylate (36.00 g, 47.00 mmol, 1.0 eq.) in DCM (200 mL) was added HCl/dioxane (4M, 150 mL). The mixture was stirred at 25 °C for 1 h. The mixture was concentrated under vacuum to afford methyl 2-(piperidin-4-ylidene)acetate hydrochloride (34.84 g, crude) as a white solid .
LCMS: *(ESI)* (M+H)⁺ = 156.2.

### Step C: synthesis of methyl 2-(1-(5-(trifluoromethyl)pyrimidin-2-yl)piperidin-4-ylidene)acetate (Intermediate N)

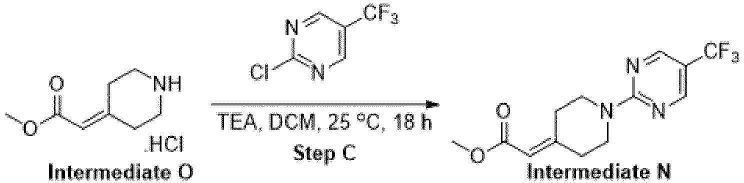

To a mixture of methyl 2-(piperidin-4-ylidene)acetate hydrochloride (21.96 g, 114.66 mmol, 1.2 eq.) and TEA (29.01 g, 286.65 mmol, 3.0 eq.) in DCM (300 mL) was added 2-chloro-5-(trifluoromethyl)pyrimidine (17.44 g, 95.55 mmol, 1.0 eq.). The reaction mixture was stirred at 25 °C for 18 h. The mixture was quenched with water (500 mL) and extracted with DCM (3 x 300 mL). The organic layers were combined and dried over Na₂SO₄, concentrated under vacuum. The residue was purified by silica gel chromatography (Petroleum ether/EtOAc= 10/1; V/V) to afford methyl 2-(1-(5-(trifluoromethyl)pyrimidin-2-yl)piperidin-4-ylidene)acetate (22.50 g, 78.2%) as a white solid.
LCMS: *(ESI)* (M+H)⁺ = 302.0.
¹H NMR: (400 MHz, DMSO-d₆) *δ* 8.72 (d, *J* = 0.4 Hz, 2H), 5.84 (s, 1H), 3.98 - 3.87 (m, 4H), 3.64 (s, 3H), 2.96 (t, *J* = 5.2 Hz, 2H), 2.45 - 2.38 (m, 2H).

### 20.2) Synthesis of compound 20

### Step A: synthesis of 2-(1-(5-(trifluoromethyl)pyrimidin-2-yl)piperidin-4-ylidene)acetic acid

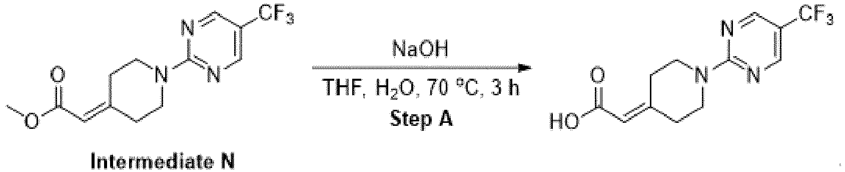

To a mixture of methyl 2-(1-(5-(trifluoromethyl)pyrimidin-2-yl)piperidin-4-ylidene) acetate (11.38 g, 37.77 mmol, 1.0 eq.) in a mixed solvent of THF (100 mL) and H₂O (100 mL) was added NaOH (4.53 g, 113.31 mmol, 3.0 eq.). The mixture was stirred at 70 °C for 3 h. The reaction mixture was adjusted to pH 6 with 1M HCl. The mixture was extracted with EtOAc (2 x 300 mL). The organic layers were combined and washed with brine (1000 mL), dried over Na₂SO₄ and concentrated under vacuum to afford 2-(1-(5-(trifluoromethyl)pyrimidin-2-yl)piperidin-4-ylidene)acetic acid (9.67 g, 89.1%) as a yellow solid.
LCMS: *(ESI)* (M+H)⁺ = 288.0.

### Step B: synthesis of 4-bromo-5-(2,2,2-trifluoroethoxy)-2-((2-(trimethylsilyl)ethoxy) methyl)pyridazin-3(2H)-one

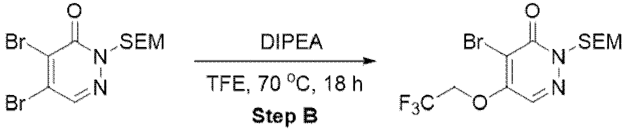

To a solution of 4,5-dibromo-2-((2-(trimethylsilyl)ethoxy)methyl) pyridazin-3(2H)-one (68.80 g, 179.10 mmol, 1.0 eq.) in 2,2,2-trifluoroethanol (280 mL) was added DIPEA (46.29 g, 358.20 mmol, 2.0 eq.). The mixture was stirred at 70 °C for 18 h. The mixture was cooled to room temperature and quenched with water (800 mL). The mxiture was extracted with EtOAc (3 x 500 mL). The organic layers were combined and washed with brine (3 × 1 L), dried over Na₂SO₄ and concentrated under vacuum. The crude product was purified by silica gel chromatography (Petroleum ether/EtOAc= 3/1, V/V) to afford 4-bromo-5-(2,2,2-trifluoroethoxy)-2-((2-(trimethylsilyl)ethoxy)methyl)pyridazin-3(2H)-one (82.80 g, 100%) as a yellow solid.
LCMS: *(ESI)* (M+H-28)⁺ = 375.0.
¹H NMR: (400 MHz, CDCl₃) *δ* 7.72 (s, 1H), 5.53 (s, 2H), 4.65 (q, *J* = 7.6 Hz, 2H), 3.76 - 3.68 (m, 2H), 1.01 - 0.93 (m, 2H), 0.02 (s, 9H).

### Step C: synthesis of 5-(2,2,2-trifluoroethoxy)-4-(trifluoromethyl)-2-((2-(trimethylsilyl) ethoxy)methyl)pyridazin-3(2H)-one

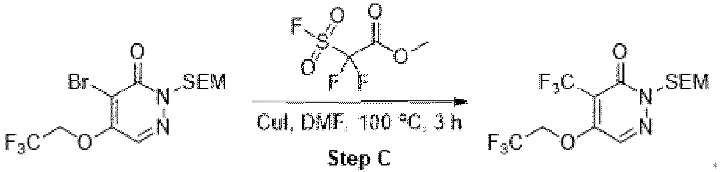

To a solution of 4-bromo-5-(2,2,2-trifluoroethoxy)-2-((2-(trimethylsilyl)ethoxy) methyl)pyridazin-3(2H)-one (82.80 g, 205.32 mmol, 1.0 eq.) in DMF (800 mL) were added CuI (19.55 g, 102.66 mmol, 0.5 eq.) and followed by dropwise addition of methyl 2,2-difluoro-2-(fluorosulfonyl)acetate (157.78 g, 821.28 mmol, 4.0 eq.). The mixture was stirred at 100 °C for 3 h. The mixture was cooled to room temperature and quenched with water (1.5 L). The mixture was extracted with EtOAc (3 × 1 L). The organic layers were combined and washed with brine (2 x 2 L), dried over anhydrous Na₂SO₄ and concentrated under vacuum. The residue was purified by column chromatography (Petroleum ether/EtOAc= 5/1, V/V) to afford 5-(2,2,2-trifluoroethoxy)-4-(trifluoromethyl)-2-((2-(trimethylsilyl)ethoxy)methyl)pyrida zin-3(2H)-one (78.00 g, 96.8%) as a white solid.
LCMS: *(ESI)* (M+H-28)⁺ = 365.0.
¹H NMR: (400 MHz, CDCl₃) *δ* 7.85 (s, 1H), 5.48 (s, 2H), 4.65 (q, *J* = 7.6 Hz, 2H), 3.79 - 3.68 (m, 2H), 1.04 - 0.91 (m, 2H), 0.03 (s, 9H).

### Step D: synthesis of (S)-5-((1-hydroxypropan-2-yl)amino)-4-(trifluoromethyl)-2-((2-(trimethylsilyl)ethoxy)methyl)pyridazin-3(2H)-one (Intermediate B)

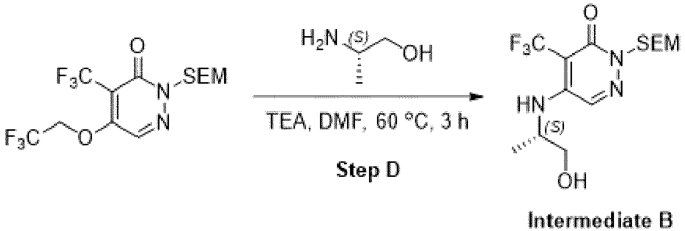

To a solution of 5-(2,2,2-trifluoroethoxy)-4-(trifluoromethyl)-2-((2-(trimethylsilyl)ethoxy)methyl)pyridazin-3(2H)-one (78.00 g, 198.79 mmol, 1.0 eq.) in DMF (780 mL) were added TEA (20.12 g, 198.79 mmol, 1.0 eq.) and L-Alaninol (29.86 g, 397.58 mmol, 2.0 eq.). The mixture was stirred at 60 °C for 3 h. The mixture was cooled to room temperature and quenched with water (1 L). The mixture was extracted with EtOAc (3 x 800 mL). The organic layers were combined and washed with brine (2 x 1.5 L), dried over anhydrous Na₂SO₄ and concentrated under vacuum. The residue was purified by column chromatography (Petroleum ether/EtOAc= 2/1, V/V) to afford (S)-5-((1-hydroxypropan-2-yl)amino)-4-(trifluoromethyl)-2-((2-(trimethylsilyl)ethoxy) methyl)pyridazin-3(2H)-one (72.08 g, 98.7%) as a white solid.
LCMS: *(ESI)* (M+H-28)⁺ = 340.1.
¹H NMR: (400 MHz, CDCl₃) *δ* 7.74 (s, 1H), 5.95 - 5.81 (m, 1H), 5.37 (dd, *J* = 22.8, 10.0 Hz, 2H), 4.0 - 3.85 (m, 1H), 3.84 - 3.75 (m, 1H), 3.73 - 3.63 (m, 3H), 1.32 (d, *J* = 6.4 Hz, 3H), 1.10 - 0.90 (m, 2H), 0.00 (s, 9H).

### Step E: synthesis of (S)-2-(2-((6-oxo-5-(trifluoromethyl)-1-((2-(trimethylsilyl)ethoxy) methyl)-1,6-dihydropyridazin-4-yl)amino)propoxy)isoindoline-1,3-dione

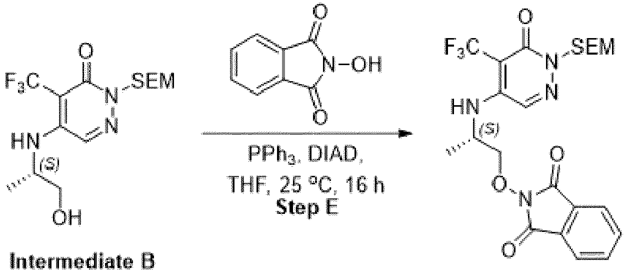

To a solution of (S)-5-((1-hydroxypropan-2-yl)amino)-4-(trifluoromethyl)-2-((2-(trimethylsilyl)ethoxy)methyl)pyridazin-3(2H)-one (57.00 g, 155.25 mmol, 1.0 eq.) in THF (400 mL) were added N-Hydroxyphthalimide (25.33 g, 155.25 mmol, 1.0 eq.), PPh3 (44.79 g, 170.78 mmol, 1.1 eq.) and followed by dropwise addition of DIAD (34.53 g, 170.78 mmol, 1.1 eq.). The mixture was stirred at 25 °C for 16 h. The mixture was quenched with water (800 mL) and extracted with EtOAc (3 x 600 mL). The organic layers were combined and washed with brine (1000 mL), dried over anhydrous Na₂SO₄ and concentrated under vacuum. The residue was purified by column chromatography (Petroleum ether/EtOAc= 3/1, V/V) to afford (S)-2-(2-((6-oxo-5-(trifluoromethyl)-1-((2-(trimethylsilyl)ethoxy)methyl)-1,6-dihydropy ridazin-4-yl)amino)propoxy)isoindoline-1,3-dione (79.02 g, 99.3%) as a colorless oil. LCMS: *(ESI)* (M+H)⁺ = 513.2.

### Step F: Synthesis of (S)-5-((1-(aminooxy)propan-2-yl)amino)-4-(trifluoromethyl)-2-((2-(trimethylsilyl)ethoxy)methyl)pyridazin-3(2H)-one (Intermediate J)

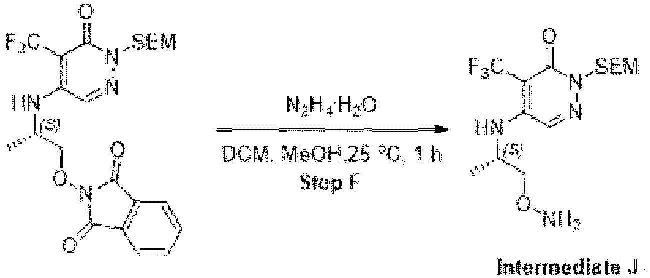

To a solution of (S)-2-(2-((6-oxo-5-(trifluoromethyl)-1-((2-(trimethylsilyl)ethoxy) methyl)-1, 6-dihydropyridazin-4-yl)amino)propoxy)isoindoline-1,3 -dione (40.00 g, 78.04 mmol, 1.0 eq.) in a mixed solvent of DCM (120 mL) and MeOH (60 mL) was added hydrazine hydrate (7.40 g, 116.96 mmol, 1.5 eq.). The mixture was stirred at 25 °C for 1 h. The mixture was concentrated under reduced pressure. The residue was suspended in EtOH (100 mL). The mixture was filtered and the filtrate was concentrated under vacuum, The residue was purified by column chromatography (Petroleum ether/EtOAc= 2/1, V/V) to afford (S)-5-((1-(aminooxy)propan-2-yl)amino) -4-(trifluoromethyl)-2-((2-(trimethylsilyl)ethoxy)methyl)pyridazin-3(2H)-one (27.8 g, 93.1%) as a yellow oil.
LCMS: *(ESI)* (M+H)⁺ = 383.1.
¹H NMR: (400 MHz, CDCl₃) *δ* 7.73 (s, 1H), 6.45 - 6.35 (m, 1H), 5.59 (s, 2H), 5.43 - 5.34 (m, 2H), 4.07 - 3.98 (m, 1H), 3.85 - 3.77 (m, 1H), 3.74 - 3.64 (m, 3H), 1.32 (d, *J* = 6.8 Hz, 3H), 0.99 - 0.93 (m, 2H), 0.00 (s, 9H).

### Step G: Synthesis of (S)-N-(2-((6-oxo-5-(trifluoromethyl)-1-((2-(trimethylsilyl)ethoxy) methyl)-1,6-dihydropyridazin-4-yl)amino)propoxy)-2-(1-(5-(trifluoromethyl)pyrimidin-2-yl)piperidin-4-ylidene)acetamide

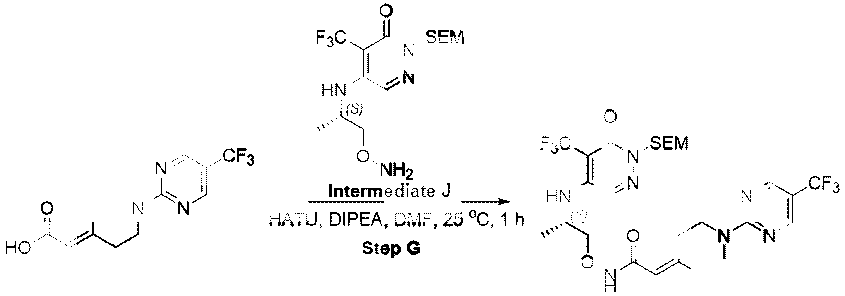

To a solution of 2-(1-(5-(trifluoromethyl)pyrimidin-2-yl)piperidin-4-ylidene)acetic acid (9.53 g, 33.18 mmol, 1.1 eq.) and (S)-5-((1-(aminooxy)propan-2-yl)amino) -4-(trifluoromethyl)-2-((2-(trimethylsilyl)ethoxy)methyl)pyridazin-3(2H)-one (11.53 g, 30.16 mmol, 1.0 eq.) in DMF (300 mL) were added DIPEA (11.69 g, 90.48 mmol, 3.0 eq.) and HATU (13.76 g, 36.19 mmol, 1.2 eq.). The mixture was stirred at 25 °C for 1 h. The mixture was quenched with water (800 mL) and extracted with EtOAc (3 x 500 mL). The organic layers were combined and washed with brine (2 x 1000 mL), dried over Na₂SO₄ and concentrated under reduced pressure. The residue was purified by column chromatography (EtOAc/Petroleum ether= 2/1; V/V) to afford (S)-N-(2-((6-oxo-5-(trifluoromethyl)-1-((2-(trimethylsilyl)ethoxy)methyl)-1,6-dihydrop yridazin-4-yl)amino)propoxy)-2-(1-(5-(trifluoromethyl)pyrimidin-2-yl)piperidin-4-ylide ne)acetamide (4.40 g, 22.4%) as a yellow oil.
LCMS: *(ESI)* (M+H)⁺ = 652.2.
¹H NMR: (400 MHz, CDCl₃) *δ* 8.97 (s, 1H), 8.52 (s, 2H), 7.76 (s, 1H), 6.09 (s, 1H), 5.74 - 5.56 (m, 1H), 5.48 - 5.40 (m, 1H), 5.39 - 5.28 (m, 1H), 4.18 - 4.10 (m, 2H), 4.08 - 3.91 (m, 5H), 3.75 - 3.68 (m, 2H), 3.18 - 3.05 (m, 2H), 2.39 (t, *J* = 5.6 Hz, 2H), 1.39 (d, *J* = 6.4 Hz, 3H), 1.01 - 0.95 (m, 2H), 0.02 (s, 9H).

### Step H: synthesis of (S)-N-(2-((6-oxo-5-(trifluoromethyl)-L6-dihydropyridazin-4-yl) amino)propoxy)-2-(1-(5-(trifluoromethyl)pyrimidin-2-yl)piperidin-4-ylidene)acetamide

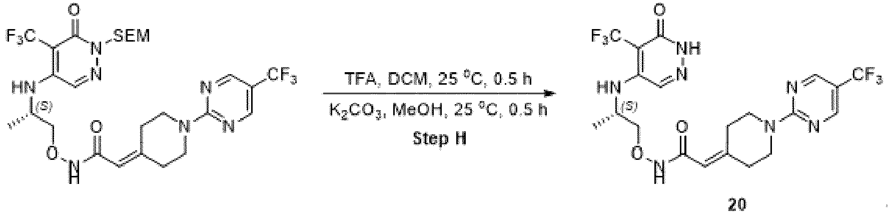

To a solution of (S)-N-(2-((6-oxo-5-(trifluoromethyl)-1-((2-(trimethylsilyl)ethoxy) methyl)-1,6-dihydropyridazin-4-yl)amino)propoxy)-2-(1-(5-(trifluoromethyl)pyrimidin-2-yl)piperidin-4-ylidene)acetamide (300 mg, 0.46 mmol, 1.0 eq.) in DCM (18 mL) was added TFA (3 mL). The resulting solution was stirred at 25 °C for 0.5 h. The mixture was concentrated under vacuum. MeOH (3 mL) and K₂CO₃ (100 mg, 0.72 mmol) were added to the resulting mixture. The mixture was stirred at 25 °C for another 0.5 h. The mixture was filtered and the filtrate was purified by prep-HPLC (FA) to afford (S)-N-(2-((6-oxo-5-(trifluoromethyl)-1,6-dihydropyridazin-4-yl)amino)propoxy)-2-(1-( 5-(trifluoromethyl)pyrimidin-2-yl)piperidin-4-ylidene)acetamide (41.89 mg, 17.5%) as a white solid.
LCMS: *(ESI)* (M+H)⁺ = 522.2.
¹H NMR: (400 MHz, DMSO-*d₆*) *δ* 12.49 (s, 1H), 11.21 (s, 1H), 8.72 (s, 2H), 7.93 (s, 1H), 6.68 - 6.60 (m, 1H), 5.57 (s, 1H), 4.39 - 4.15 (m, 1H), 3.97 - 3.82 (m, 6H), 3.02 - 2.90 (m, 2H), 2.37 - 2.25 (m, 2H), 1.20 (d, *J* = 6.8 Hz, 3H).

### Example 21:

### Step F: synthesis of (S)-2-fluoro-N-(2-((6-oxo-5-(trifluoromethyl)-1,6-dihydropyridazin -4-yl)amino)propoxy)-2-(1-(5-(trifluoromethyl)pyrimidin-2-yl)piperidin-4-ylidene) acetamide

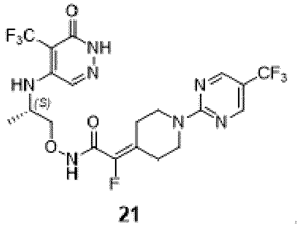

### 21.1) Synthesis of intermediate L

### Step A: synthesis of tert-butyl 4-(2-ethoxy-1-fluoro-2-oxoethylidene)piperidine-1-carboxylate

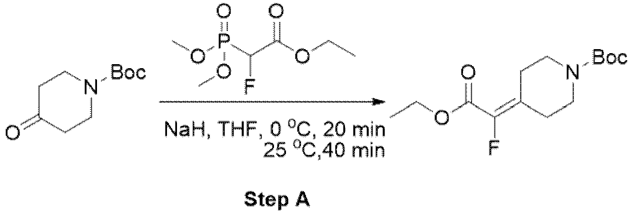

To a solution of ethyl 2-(dimethoxyphosphoryl)-2-fluoroacetate (1.34 g, 5.52 mmol, 1.1 eq.) in THF (20 mL) were added NaH (240 mg, 6.02 mmol, 1.2 eq.) at 0 °C. The resulting mixture was stirred at 0 °C for 20 mins. Tert-butyl 4-oxopiperidine-1-carboxylate (1 g, 5.02 mmol, 1.0 eq.) was added at 0 °C, the resulting mixture was stirred at 25 °C for another 40 mins. The mixture was quenched by water (30 mL), extracted with EtOAc (3 x 20 mL). The organic layers were combined, dried over Na₂SO₄ and concentrated under reduced pressure. The residue was purified by column chromatography (Petroleum ether /EtOAc =9/1, V/V) to afford tert-butyl 4-(2-ethoxy-1-fluoro-2-oxoethylidene)piperidine-1-carboxylate (900 mg, 64.2%) as a colorless oil.
LCMS: *(ESI)* (M+H-100)⁺ = 188.1.

### Step B: Synthesis of ethyl 2-fluoro-2-(piperidin-4-ylidene)acetate hydrogen chloride

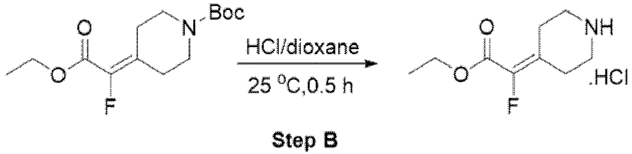

To a solution of tert-butyl 4-(2-ethoxy-1-fluoro-2-oxoethylidene)piperidine -1-carboxylate (900 mg) in MeOH (5 mL) was added HCl/dioxane (5 mL). The mixture was stirred at 25 °C for 30 mins. The mixture was concentrated under vacuum to afford ethyl 2-fluoro-2-(piperidin-4-ylidene)acetate hydrogen chloride (70 mg, 100% yield). LCMS: *(ESI)* (M+H)⁺ = 188.2.

### Step C: synthesis of ethyl 2-fluoro-2-(1-(5-(trifluoromethyl)pyrimidin-2-yl)piperidin -4-ylidene)acetate (Intermediate L)

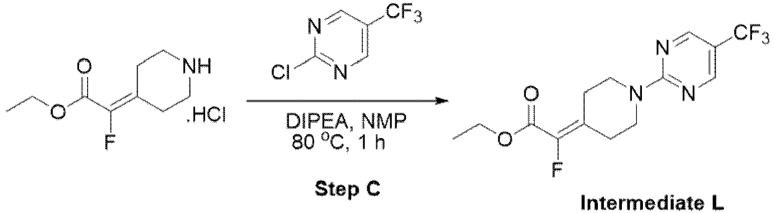

To a solution of ethyl 2-fluoro-2-(piperidin-4-ylidene)acetate hydrogen chloride (700 mg, 4.06 mmol, 1.1 eq.) in NMP (15 mL) was added DIPEA (1.4 g, 11.04 mmol, 3.0 eq.) and 2-chloro-5-(trifluoromethyl)pyrimidine (670 mg, 3.68 mmol, 1 eq.). The resulting mixture was stirred at 80 °C for 1 h. The mixture was cooled to room temperature and diluted with water (40 ml). The mixture was extracted with EtOAc (2 x 35 mL). The organic layers were combined, dried over Na₂SO₄ and concentrated under reduced pressure. The residue was purified by column chromatography (Petroleum ether /EtOAc= 9/1, V/V) to afford ethyl 2-fluoro-2-(1-(5-(trifluoromethyl)pyrimidin-2-yl) piperidin-4-ylidene)acetate (1 g, 86.9%) as a white solid.
LCMS: *(ESI)* (M+H)⁺ = 334.2.
¹H NMR (400 MHz, DMSO-*d₆*) *δ* 8.73 (s, 2H), 4.30 - 4.16 (m, 2H), 4.02 - 3.78 (m, 4H), 2.98 - 2.83 (m, 2H), 2.57 - 2.52 (m, 2H), 1.27 (t, J = 7.1 Hz, 3H).

### 21.2) Synthesis of compound 21

### Step A: Synthesis of 2-fluoro-2-(1-(5-(trifluoromethyl)pyrimidin-2-yl)piperidin-4-ylidene)acetic acid

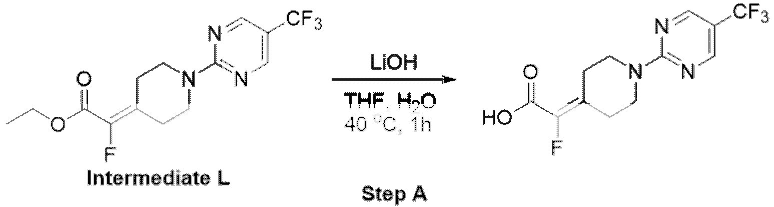

To a solution of ethyl 2-fluoro-2-(1-(5-(trifluoromethyl)pyrimidin-2-yl)piperidin -4-ylidene)acetate (1 g, 3.01 mmol, 1.0 eq.) in a mixed solvent of THF (8 mL) and water (8 mL) was added LiOH (289 mg, 12.04 mmol, 4.0 eq.). The mixture was stirred at 40 °C for 1 h. The mixture was diluted with water (30 mL) and extracted with EtOAc (3 x 25 mL). The water layer was adjusted to pH 6 with 1M HCl aqueous solution. The resulting mixture was extracted with EtOAc (3 x 25 mL), dried over Na₂SO₄ and concentrated under vacuum to afford 2-fluoro-2-(1-(5-(trifluoromethyl)pyrimidin-2-yl) piperidin-4-ylidene)acetic acid (720 mg, 91.3% yield) as a white solid.
LCMS: *(ESI)* (M+H)⁺ = 306.2.

### Step B: synthesis of (S)-2-fluoro-N-(2-((6-oxo-5-(trifluoromethyl)-1-((2-(trimethylsilyl) ethoxy)methyl)-1,6-dihydropyridazin-4-yl)amino)propoxy)-2-(1-(5-(trifluoromethyl)pyr imidin-2-yl)piperidin-4-ylidene)acetamide

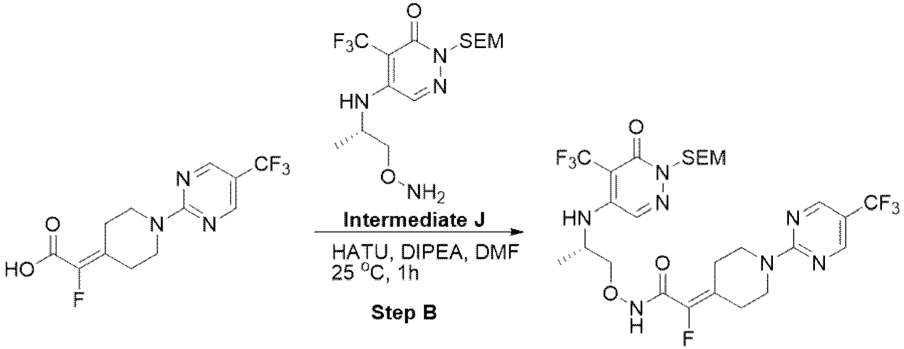

To a solution of 2-fluoro-2-(1-(5-(trifluoromethyl)pyrimidin-2-yl)piperidin-4-ylidene)acetic acid (200 mg, 0.66 mmol, 1.1 eq.) in THF (5 mL) were added DIPEA (153 mg, 1.18 mmol, 2.0 eq.) and HATU (337 mg, 0.89 mmol, 1.5 eq.) followed by dropwise addition of (S)-5-((1-(aminooxy)propan-2-yl)amino)-4-(trifluoromethyl)-2-((2-(trimethylsilyl)ethoxy)methyl)pyridazin-3(2H)-one (230 mg, 0.59 mmol, 1.0 eq.). The resulting mixture was stirred at 25 °C for 1 h. The reaction mixture was quenched with water (35 mL) and extracted with EtOAc (3 x 30mL). The organic layers were combined and washed with brine (10 mL), dried over Na₂SO₄ and concentrated under vacuum. The residue was purified by silica gel chromatography (Petroleum ether/EtOAc=7/3 V/V) to afford (S)-2-fluoro-N-(2-((6-oxo-5-(trifluoromethyl)-1-((2-(trimethylsilyl)ethoxy)methyl)-1,6-dihydropyridazin-4-yl)amino)propoxy)-2-(1-(5-(trifl uoromethyl)pyrimidin-2-yl)piperidin-4-ylidene)acetamide (290 mg, 66.9% yield) as a colorless oil.
LCMS: *(ESI)* (M+H)⁺ = 670.4.

### Step C: synthesis of (S)-2-fluoro-N-(2-((6-oxo-5-(tiifluoromethyl)-1,6-dihydropyridazin -4-yl)amino)propoxy)-2-(1-(5-(trifluoromethyl)pyrimidin-2-yl)piperidin-4-ylidene)aceta mide

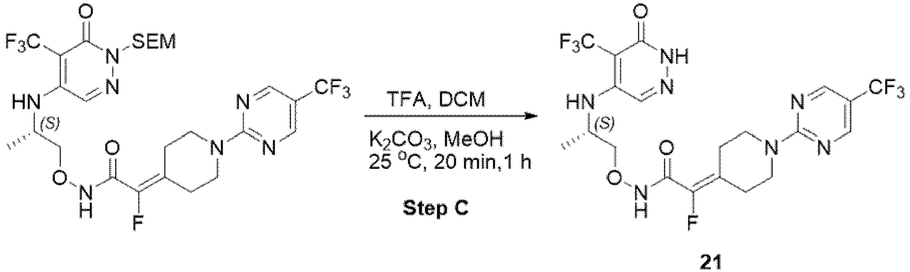

To a solution of (S)-2-fluoro-N-(2-((6-oxo-5-(trifluoromethyl)-1-((2-(trimethylsilyl) ethoxy)methyl)-1,6-dihydropyridazin-4-yl)amino)propoxy)-2-(1-(5-(trifluoromethyl)pyr imidin-2-yl)piperidin-4-ylidene)acetamide (290 mg, 0.434 mmol, 1.0 eq.) in DCM (3 mL) was added TFA (0.3 mL). The resulting mixture was stirred at 25 °C for 20 min. The mixture was neutralized with saturated NaHCOs aqueous solution (5 mL) and extracted with DCM (3 x 10 mL). The organic layers were combined and dried over Na₂SO₄, concentrated under vacuum. The residue was redissolved in MeOH (3 mL) and K₂CO₃ (230 mg, 1.67 mmol, 3.0 eq.) was added. The mixture was stirred at 25 °C for another 1 h. The reaction mixture was filtered and the filtrate was purified by silica gel chromatography (Petroleum ether/EtOAc= 1/100, V/V) to afford (S)-2-fluoro-N-(2-((6-oxo-5-(trifluoromethyl)-1,6-dihydropyridazin-4-yl)amino)propox y)-2-(1-(5-(trifluoromethyl)pyrimidin-2-yl)piperidin-4-ylidene)acetamide (5.7 mg, 4.1% yield) as a white solid.
LCMS: *(ESI)* (M+H)⁺ = 540.2.
¹H NMR (400 MHz, DMSO-*d₆*) *δ* 12.49 (s, 1H), 11.86 (s, 1H), 8.72 (s, 2H), 7.92 (s, 1H), 6.64 - 6.51 (m, 1H), 4.32 - 4.18 (m, 1H), 4.05 - 3.93 (m, 1H), 3.94 - 3.84 (m, 5H), 2.91 (s, 2H), 2.46 (s, 2H), 1.21 (d, J = 6.6 Hz, 3H).

### Example 22:

### 2-fluoro-N-((S)-2-((6-oxo-5-(trifluoromethyl)-1,6-dihydropyridazin-4-yl)amino) propoxy)-2-(1-(5-(trifluoromethyl)pyrimidin-2-yl)-1,2,3,6-tetrahydropyridin-4-yl) acetamide

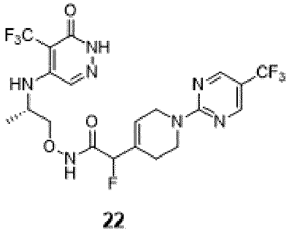

### Step A: synthesis of ethyl 2-fluoro-2-(1-(5-(trifluoromethyl)pyrimidin-2-yl)-1,2,3,6 -tetrahydropyridin-4-yl)acetate

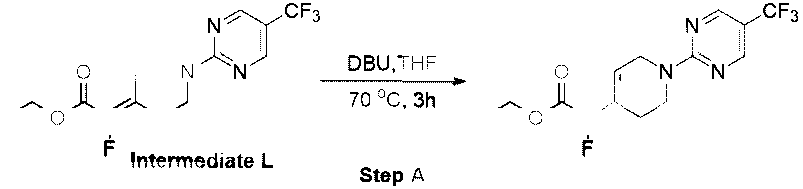

To a solution of ethyl 2-fluoro-2-(1-(5-(trifluoromethyl)pyrimidin-2-yl)piperidin-4 -ylidene)acetate (930 mg, 2.8 mmol, 1 eq.) in THF (15 mL) was added DBU (2.1 g, 13.9 mmol, 5.0 eq.) . The resulting mixture was stirred at 70 °C for 3 h. The mixture was concentrated under reduced pressure. The residue was purified by column chromatography (Petroleum ether/EtOAc=95/5, V/V) to afford ethyl 2-fluoro-2-(1-(5-(trifluoromethyl)pyrimidin-2-yl)-1,2,3,6-tetrahydropyridin-4-yl)acetate (210 mg, 22.8%) as a colorless oil.
LCMS: *(ESI)* (M+H)⁺ = 334.2.
¹H NMR (400 MHz, DMSO-*d₆*) *δ* 8.73 (s, 2H), 6.14 (s, 1H), 5.58 (d, J = 47.3 Hz, 1H), 4.45 - 4.33 (m, 1H), 4.33 - 4.23 (m, 1H), 4.20 (q, J = 7.1 Hz, 2H), 4.12 - 4.03 (m, 1H), 3.91 - 3.82 (m, 1H), 2.29 - 2.17 (m, 1H), 2.16 - 2.03 (m, 1H), 1.19 (t, J = 7.1 Hz, 3H).

### Step B: Synthesis of 2-fluoro-2-(1-(5-(trifluoromethyl)pyrimidin-2-yl)-1,2,3,6 -tetrahydropyridin-4-yl)acetic acid

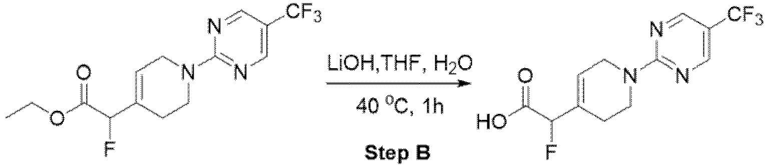

To a solution of ethyl 2-fluoro-2-(1-(5-(trifluoromethyl)pyrimidin-2-yl)-1,2,3,6 -tetrahydropyridin-4-yl)acetate (210 mg, 0.64 mmol, 1.0 eq.) in a mixed solvent of THF (3 mL) and water (3 mL) was added LiOH (46 mg, 1.9 mmol, 3.0 eq.). The mixture was stirred at 40 °C for 1 h. The mixture was diluted with water (30 mL) and extracted with EtOAc (3 x 25 mL). The water layer was adjusted to pH 6. The resulting mixture was extracted with EtOAc (3 x 25 mL), dried over Na₂SO₄ and concentrated under vacuum to afford 2-fluoro-2-(1-(5-(trifluoromethyl)pyrimidin-2-yl)-1,2,3,6-tetrahydropyridin -4-yl)acetic acid (210 mg, 100% yield) as a white solid.
LCMS: *(ESI)* (M+H)⁺ = 306.2.

### Step C: synthesis of 2-fluoro-N-((S)-2-((6-oxo-5-(trifluoromethyl)-1-((2-(trimethylsilyl) ethoxy)methyl)-1,6-dihydropyridazin-4-yl)amino)propoxy)-2-(1-(5-(trifluoromethyl)pyr imidin-2-yl)-1,2,3,6-tetrahydropyridin-4-yl)acetamide

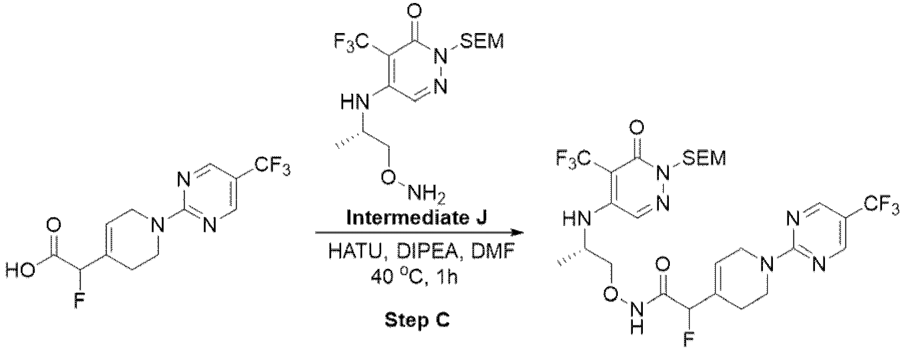

To a solution of 2-fluoro-2-(1-(5-(trifluoromethyl)pyrimidin-2-yl)-1,2,3,6-tetrahydropyridin-4-yl)acetic acid (170 mg, 0.56 mmol, 1 eq.) in THF (5 mL) were added DIPEA (145 mg, 1.12 mmol, 2.0 eq.) and HATU (320 mg, 0.84 mmol, 1.5 eq.) followed by dropwise addition of (S)-5-((1-(aminooxy)propan-2-yl)amino)-4-(trifluoromethyl)-2-((2-(trimethylsilyl)ethoxy)methyl)pyridazin-3(2H)-one (230 mg, 0.59 mmol, 1.05 eq.).The resulting mixture was stirred at 40 °C for 1 h. The reaction mixture was quenched with water (35 mL) and extracted with EtOAc (3 x 30mL). The organic layers were combined and washed with brine (10 mL), dried over Na₂SO₄ and concentrated under vacuum. The residue was purified by silica gel chromatography (DCM/MeOH= 98/2 V/V) to afford 2-fluoro-N-((S)-2-((6-oxo-5-(trifluoromethyl) -1-((2-(trimethylsilyl)ethoxy)methyl)-1,6-dihydropyridazin-4-yl)amino)propoxy)-2-(1-( 5-(trifluoromethyl)pyrimidin-2-yl)-1,2,3,6-tetrahydropyridin-4-yl)acetamide (450 mg, 100% yield) as a colorless oil.
LCMS: *(ESI)* (M+H)⁺ = 670.4.

### Step D: synthesis of 2-fluoro-N-((S)-2-((6-oxo-5-(trifluoromethyl)-L6-dihydropyridazin-4-yl)amino)propoxy)-2-(1-(5-(trifluoromethyl)pyrimidin-2-yl)-1,2,3,6 -tetrahvdropyridin-4-vl)acetamide

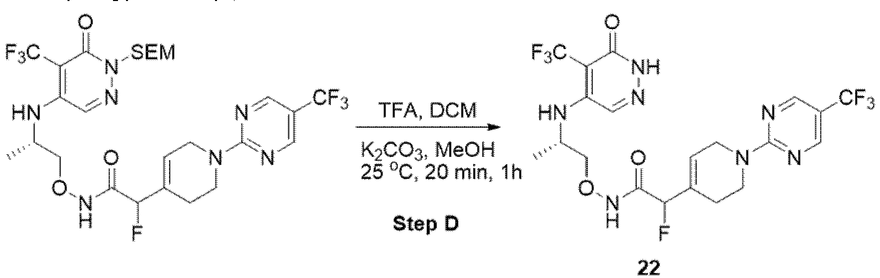

To a solution of 2-fluoro-N-((S)-2-((6-oxo-5-(trifluoromethyl)-1-((2-(trimethylsilyl) ethoxy)methyl)-1,6-dihydropyridazin-4-yl)amino)propoxy)-2-(1-(5-(trifluoromethyl) pyrimidin-2-yl)-1,2,3,6-tetrahydropyridin-4-yl)acetamide (450 mg, 0.672 mmol, 1.0 eq.) in DCM (5 mL) was added TFA (1 mL). The resulting mixture was stirred at 25 °C for 20 min. The mixture was neutralized with saturated NaHCOs aqueous solution (5 mL) and extracted with DCM (3 x 10 mL). The organic layers were combined and dried over Na₂SO₄, concentrated under vacuum. The residue was redissolved in MeOH (3 mL) and K₂CO₃ (230 mg, 1.67 mmol, 3.0 eq.) was added. The mixture was stirred at 25 °C for another 1 h. The reaction mixture was filtered and the filtrate was purified by pre-HPLC to afford 2-fluoro-N-((S)-2-((6-oxo-5-(trifluoromethyl)-1,6-dihydropyridazin-4-yl) amino)propoxy)-2-(1-(5-(trifluoromethyl)pyrimidin-2-yl)-1,2,3,6-tetrahydropyridin-4-yl )acetamide (1.5 mg, 0.4% yield) as a white solid.
LCMS: *(ESI)* (M+H)⁺ = 540.2.
¹H NMR (400 MHz, CDCl₃) *δ* 10.37 (s, 1H), 9.25 (s, 1H), 8.44 (s, 2H), 7.62 (s, 1H), 6.01 (s, 1H), 5.92 - 5.79 (m, 1H), 5.22 (d, J = 46.9 Hz, 1H), 4.46 - 4.19 (m, 2H), 4.14 - 3.81 (m, 5H), 2.35 - 2.21 (m, 1H), 2.21 - 2.10 (m, 1H), 1.31 (d, J = 6.4 Hz, 3H).

### Example 23:

### (E)-2-(3-fluoro-1-(5-(trifluoromethyl)pyrimidin-2-yl)piperidin-4-ylidene)-N-((S)-2-((6-oxo-5-(trifluoromethyl)-1,6-dihydropyridazin-4-yl)amino)propoxy)acetamide

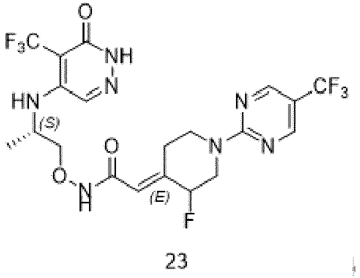

### 23.1) Synthesis of intermediate M

### Step A: synthesis of (E)-tert-butyl 3-fluoro-4-(2-methoxy-2-oxoethylidene)piperidine-1-carboxylate and (Z)-tert-butyl 3-fluoro-4-(2-methoxy-2-oxoethylidene)piperidine-1-carboxylate (Intermediate M)

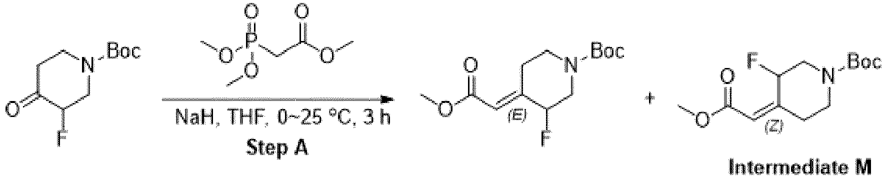

To a solution of trimethyl phosphonoacetate (3.35 g, 18.41 mmol, 1.0 eq.) in THF (45 mL) was added NaH (60%, 0.77 g, 19.33 mmol, 1.05 eq.) in portions at 0 °C in ice-water bath under N₂ atmosphere atmosphere. The mixture was stirred at 0 °C for 0.5 h. Tert-butyl 3-fluoro-4-oxopiperidine-1-carboxylate (4.00 g, 18.41 mmol, 1.0 eq.) in a solution of in THF (45 mL) was added dropwise to the mixture. The mixture was stirred at 25 °C for another 2.5 h. The mixture was quenched with cold saturated NH₄Cl aqueous solution (100 mL) and extracted with EtOAc (2 x 100 mL). The organic layers were combined and washed with brine (100 mL), dried over Na₂SO₄ and concentrated under vacuum. The residue was purified by silica gel chromatography (Petroleum ether/EtOAc= 10/1; V/V) to afford (E)-tert-butyl 3-fluoro-4-(2-methoxy-2-oxoethylidene)piperidine-1-carboxylate (940 mg, 18.7%) as a light yellow oil and (Z)-tert-butyl 3-fluoro-4-(2-methoxy-2-oxoethylidene)piperidine-1-carboxylate (1.30 g, 25.8%) as a light yellow oil.
(E)-tert-butyl 3-fluoro-4-(2-methoxy-2-oxoethylidene)piperidine-1-carboxylate: LCMS: *(ESI)* (M+H-100)⁺ = 174.2.
¹H NMR: (400 MHz, CDCl₃) *δ* 5.99 (s, 1H), 4.95 - 4.72 (m, 1H), 4.21 - 3.89 (m, 1H), 3.78 - 3.67 (m, 4H), 3.54 - 3.13 (m, 3H), 2.66 - 2.45 (m, 1H), 1.48 (s, 9H).
(Z)-tert-butyl 3-fluoro-4-(2-methoxy-2-oxoethylidene)piperidine-1-carboxylate: LCMS: *(ESI)* (M+H-100)⁺ = 174.2.
¹H NMR: (400 MHz, CDCl₃) *δ* 6.40 (d, *J* = 48.0 Hz, 1H), 5.83 (s, 1H), 4.72 - 4.16 (m, 2H), 3.73 (s, 3H), 3.07 - 2.86 (m, 1H), 2.85 - 2.68 (m, 2H), 2.17 - 2.07 (m, 1H), 1.48 (s, 9H).

### 23.2) Synthesis of compound 23

### Step A: synthesis of (E)-methyl 2-(3-fluoropiperidin-4-ylidene)acetate hydrochloride

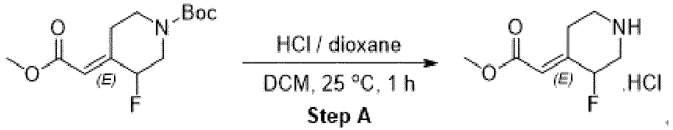

To a mixture of (E)-tert-butyl 3-fluoro-4-(2-methoxy-2-oxoethylidene) piperidine-1-carboxylate (250 mg, 0.92 mmol, 1.0 eq.) in DCM (5 mL) was added HCl/dioxane (4M, 3 mL). The mixture was stirred at 25 °C for 1 h. The mixture was concentrated under vacuum to afford (E)-methyl 2-(3-fluoropiperidin-4-ylidene)acetate hydrochloride (200 mg, crude) as a white solid .
LCMS: *(ESI)* (M+H)⁺ = 174.0.

### Step B: synthesis of (E)-methyl 2-(3-fluoro-1-(5-(trifluoromethyl)pyrimidin -2-yl)piperidin-4-ylidene)acetate

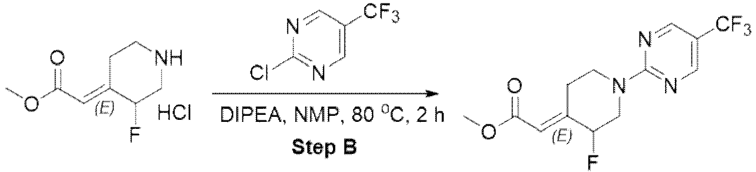

To a mixture of (E)-methyl 2-(3-fluoropiperidin-4-ylidene)acetate hydrochloride (170 mg, 0.76 mmol, 1.2 eq.) in NMP (2 mL) were added DIPEA (263.2 mg, 2.04 mmol, 3.0 eq.) and 2-chloro-5-(trifluoromethyl)pyrimidine (124 mg, 0.68 mmol, 1.0 eq.). The mixture was stirred at 80 °C for 2 h. The mixture was cooled to room temperature and diluted with saturated NH₄Cl aqueous solution (60 mL). The mixture was extracted with EtOAc (2 x 40 mL). The organic layers were combined and washed with saturated NH₄Cl aqueous solution (2 x 60 mL), dried over Na₂SO₄ and concentrated under vacuum. The residue was purified by silica gel chromatography (Petroleum ether/EtOAc= 6/1; V/V) to afford (E)-methyl 2-(3-fluoro-1-(5-(trifluoromethyl) pyrimidin-2-yl)piperidin-4-ylidene)acetate (207 mg, 95.6%) as a white solid.
LCMS: *(ESI)* (M+H)⁺ = 319.9.
¹H NMR : (400 MHz, CDCl₃) *δ* 8.53 (s, 2H), 6.06 (s, 1H), 4.99 - 4.87 (m, 1H), 4.54 - 4.47 (m, 1H), 4.28 - 4.19 (m, 1H), 3.95 - 3.88 (m, 1H), 3.76 (s, 3H), 3.75 - 3.71 (m, 1H), 3.52 - 3.45 (m, 1H), 2.77 - 2.68 (m, 1H).

### Step C: synthesis of (E)-2-(3-fluoro-1-(5-(trifluoromethyl)pyrimidin-2-yl)piperidin-4 -ylidene)acetic acid

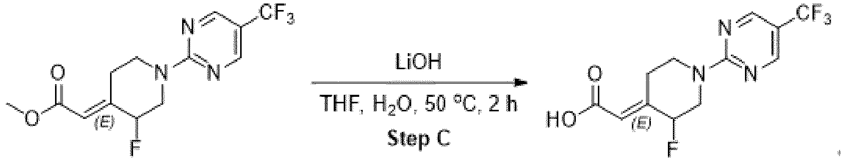

To a mixture of (E)-methyl 2-(3-fluoro-1-(5-(trifluoromethyl)pyrimidin-2-yl) piperidin-4-ylidene)acetate (100 mg, 0.31 mmol, 1.0 eq.) in THF (3 mL) and H₂O (1.5 mL) was added LiOH (53 mg, 1.25 mmol, 4.0 eq.). The mixture was stirred at 50 °C for 2 h. The mixture was cooled to room temperature and diluted with water (20 mL). The mixture was adjusted to pH 6 with 1M HCl and extracted with EtOAc (2 x 50 mL). The organic layers were combined and washed with brine (100 mL), dried over Na₂SO₄ and concentrated under vacuum to afford (E)-2-(3-fluoro-1-(5-(trifluoromethyl) pyrimidin-2-yl)piperidin-4-ylidene)acetic acid (93 mg, 97.3%) as a white solid.
LCMS: *(ESI)* (M+H)⁺ = 306.2.

### Step D: Synthesis of (E)-2-(3-fluoro-1-(5-(trifluoromethyl)pyrimidin-2-yl) piperidin-4-ylidene)-N-((S)-2-((6-oxo-5-(trifluoromethyl)-1-((2-(trimethylsilyl)ethoxy) methyl)-1,6-dihydropyridazin-4-yl)amino)propoxy)acetamide

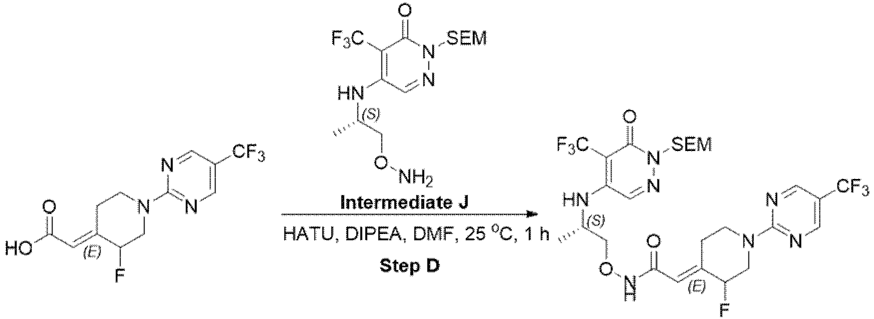

To a mixture of (E)-2-(3-fluoro-1-(5-(trifluoromethyl)pyrimidin-2-yl)piperidin-4 -ylidene)acetic acid (90 mg, 0.30 mmol, 1.0 eq.) in DMF (3 mL) were added (S)-5-((1-(aminooxy)propan-2-yl)amino)-4-(trifluoromethyl)-2-((2-(trimethylsilyl)ethox y)methyl)pyridazin-3(2H)-one (124 mg, 0.32 mmol, 1.1 eq.), DIPEA (0.15 mL, 0.89 mmol, 3.0 eq.) and HATU (135 mg, 0.35 mmol, 1.2 eq.). The mixture was stirred at 25 °C for 1 h. The reaction mixture was quenched with water (100 mL) and extracted with EtOAc (2 x 100 mL). The organic layers were combined and washed with brine (2 x 100 mL), dried over Na₂SO₄ and concentrated under vacuum. The residue was purified by chromatography (Petroleum ether/EtOAc= 3/2; V/V) to afford (E)-2-(3-fluoro-1-(5-(trifluoromethyl)pyrimidin-2-yl)piperidin-4-ylidene)-N-((S)-2-((6-oxo-5-(trifluoromethyl)-1-((2-(trimethylsilyl)ethoxy)methyl)-1,6-dihydropyridazin-4-yl) amino)propoxy)acetamide (90 mg, 46.6%) as a yellow solid.
LCMS: *(ESI)* (M+H)⁺ = 669.9.

### Step E: synthesis of (E)-2-(3-fluoro-1-(5-(trifluoromethyl)pyrimidin -2-yl)piperidin-4-ylidene)-N-((S)-2-((6-oxo-5-(trifluoromethyl)-1,6-dihydropyridazin-4-yl)amino)propoxy)acetamide

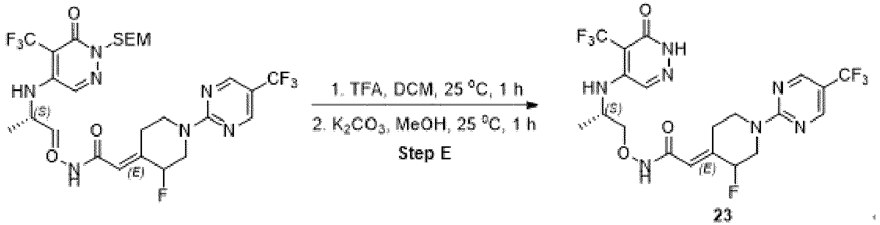

To a mixture of (E)-2-(3-fluoro-1-(5-(trifluoromethyl)pyrimidin-2-yl)piperidin -4-ylidene)-N-((S)-2-((6-oxo-5-(trifluoromethyl)-1-((2-(trimethylsilyl)ethoxy)methyl)-1 ,6-dihydropyridazin-4-yl)amino)propoxy)acetamide (80 mg, 0.12 mmol, 1.0 eq.) in DCM (3 mL) was added TFA (1 mL). The mixture was stirred at 25 °C for 1 h. The mixture was quenched with saturated aqueous NaHCOs solution (50 mL) and extracted with DCM (3 x 80 mL). The organic layers were combined and dried over Na₂SO₄, concentrated under vacuum. The residue was redissolved in MeOH (2 mL) and K₂CO₃ (76 mg, 0.55 mmol) was added. The mixture was stirred at 25 °C for another 1 h. The mixture was filtered and filtrate was purified by prep-HPLC (FA) to afford (E)-2-(3-fluoro-1-(5-(trifluoromethyl)pyrimidin-2-yl)piperidin-4-ylidene)-N-((S)-2-((6-oxo-5-(trifluoromethyl)-1,6-dihydropyridazin-4-yl)amino)propoxy)acetamide (8.66 mg, 13.4%) as a white solid.
LCMS: *(ESI)* (M+H)⁺ = 540.2.
¹H NMR: (400 MHz, DMSO-*d₆*) *δ* 12.49 (s, 1H), 11.45 (s, 1H), 8.76 (d, *J* = 0.4 Hz, 2H), 7.93 (s, 1H), 6.65 - 6.59 (m, 1H), 5.84 (s, 1H), 5.21 -5.05 (m, 1H), 4.33 - 4.17 (m, 2H), 4.05 - 4.16 (m, 1H), 3.93 - 4.03 (m, 2H), 3.80 - 3.92 (m, 2H), 3.09 - 3.12 (m, 1H), 2.94 - 3.97 (m, 1H), 1.20 (d, *J* = 6.8 Hz, 3H).

### Example 24:

### (Z)-2-(3-fluoro-1-(5-(trifluoromethyl)pyrimidin-2-yl)piperidin-4-ylidene)-N-((S)-2-((6-oxo-5-(trifluoromethyl)-1,6-dihydropyridazin-4-yl)amino)propoxy)acetamide

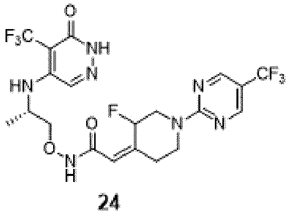

### Step A: synthesis of (Z)-methyl 2-(3-fluoropiperidin-4-ylidene)acetate hydrochloride

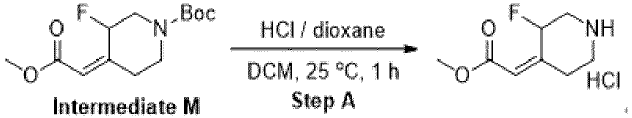

To a solution of (Z)-tert-butyl 3-fluoro-4-(2-methoxy-2-oxoethylidene)piperidine -1-carboxylate (400 mg, 1.46 mmol, 1.0 eq.) in DCM (4 mL) was added HCl/dioxane (4M, 2 mL). The mixture was stirred at 25 °C for 1 h. The mixture was concentrated under vacuum to afford (Z)-methyl 2-(3-fluoropiperidin-4-ylidene)acetate hydrochloride (310 mg, 100%) as a white solid.
LCMS: *(ESI)* (M+H)⁺ = 174.2.

### Step B: synthesis of (Z)-methyl 2-(3-fluoro-1-(5-(trifluoromethyl)pyrimidin-2-yl) piperidin-4-ylidene)acetate

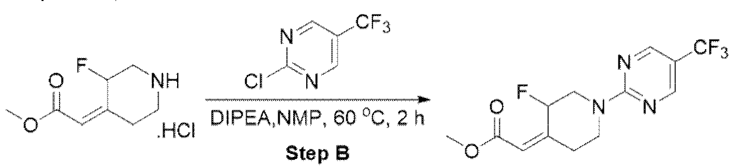

To a solution of (Z)-methyl 2-(3-fluoropiperidin-4-ylidene)acetate hydrochloride (310 mg, 1.48 mmol, 1.1 eq.) in NMP (5 mL) were added DIPEA (520 mg, 4.03 mmol, 3.0 eq.) and 2-chloro-5-(trifluoromethyl)pyrimidine (245 mg, 1.34 mmol, 1.0 eq.). The mixture was stirred at 60 °C for 2 h. The mixture was cooled to room temperature and diluted with water (50 mL), extracted with EtOAc (2 x 50 mL). The organic layers were combined and washed with brine (2 x 50 mL), dried over Na₂SO₄ and concentrated under vacuum. The residue was purified by silica gel chromatography (EtOAc/Petroleum ether= 1/5; V/V) to afford (Z)-methyl 2-(3-fluoro-1-(5-(trifluoromethyl)pyrimidin-2-yl)piperidin-4-ylidene)acetate (360 mg, 84.0%) as a light yellow solid.
LCMS: *(ESI)* (M+H)⁺ = 320.0.
¹H NMR: (400 MHz, CDCl₃) *δ* 8.51 (s, 2H), 6.57 (dd, *J* = 47.6, 2.8 Hz, 1H), 5.91 (s, 1H), 5.47 - 5.36 (m, 1H), 5.13 - 5.05 (m, 1H), 3.76 (s, 3H), 3.21 - 2.98 (m, 2H), 2.91 - 2.81 (m, 1H), 2.28 (dt, *J* = 13.6, 2.4 Hz, 1H).

### Step C: synthesis of (Z)-2-(3-fluoro-1-(5-(trifluoromethyl)pyrimidin-2-yl)piperidin-4 -ylidene)acetic acid

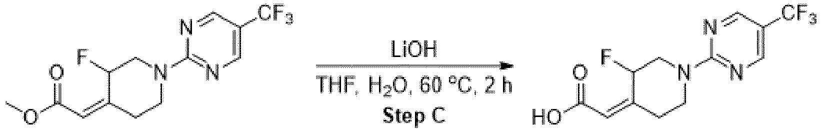

To a solution of (Z)-methyl 2-(3-fluoro-1-(5-(trifluoromethyl)pyrimidin-2-yl) piperidin-4-ylidene)acetate (200 mg, 0.63 mmol, 1.0 eq.) in THF (4 mL) and H₂O (2 mL) was added LiOH (79 mg, 1.88 mmol, 3.0 eq.). The mixture was heated to 60 °C and stirred for 2 h. The mixture was diluted with water (20 mL) and acidified to pH 5 with 1M HCl. The mixture was extracted with EtOAc (3 x 40 mL). The organic layers were combined and dried over Na₂SO₄, concentrated under vacuum to afford (Z)-2-(3-fluoro-1-(5-(trifluoromethyl)pyrimidin-2-yl)piperidin-4-ylidene)acetic acid (190 mg, 99.4%) as a light yellow solid.
LCMS: *(ESI)* (M+H)⁺ = 306.0.

### Step D: Synthesis of (Z)-2-(3-fluoro-1-(5-(trifluoromethyl)pyrimidin-2-yl)piperidin-4 -ylidene)-N-((S)-2-((6-oxo-5-(trifluoromethyl)-1-((2-(trimethylsilyl)ethoxy)methyl)-1,6 -dihydropyridazin-4-yl)amino)propoxy)acetamide

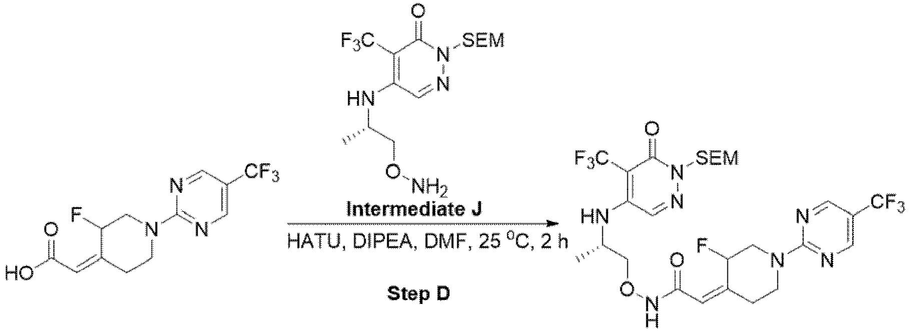

To a solution of (Z)-2-(3-fluoro-1-(5-(trifluoromethyl)pyrimidin-2-yl)piperidin -4-ylidene)acetic acid (182 mg, 0.60 mmol, 1.2 eq.) in DMF (5 mL) were added DIPEA (193 mg, 1.49 mmol, 3.0 eq.), HATU (283 mg, 0.75 mmol, 1.5 eq.) and followed by (S)-5-((1-(aminooxy)propan-2-yl)amino)-4-(trifluoromethyl)-2-((2-(trimethylsilyl)ethox y)methyl)pyridazin-3(2H)-one (190 mg, 0.50 mmol, 1.0 eq.). The mixture was stirred at 25 °C for 2 h. The mixture quenched with water (30 mL) and extracted with EtOAc (2 x 50 mL). The organic layers were combined and washed with brine (2 x 40 mL), dried over Na₂SO₄ and concentrated under vacuum. The residue was purified by silica gel chromatography (Petroleum ether/EtOAc= 4/6; V/V) to afford (Z)-2-(3-fluoro-1-(5-(trifluoromethyl)pyrimidin-2-yl)piperidin-4-ylidene)-N-((S)-2-((6-oxo-5-(trifluoromethyl)-1-((2-(trimethylsilyl)ethoxy)methyl)-1,6-dihydropyridazin-4-yl) amino)propoxy)acetamide (120 mg, 36.1%) as a light yellow oil.
LCMS: *(ESI)* (M+H)⁺ = 669.9.

### Step E: synthesis of (Z)-2-(3-fluoro-1-(5-(trifluoromethyl)pyrimidin-2-yl)piperidin-4 -ylidene)-N-((S)-2-((6-oxo-5-(trifluoromethyl)-1,6-dihydropyridazin-4-yl)amino)propox y)acetamide

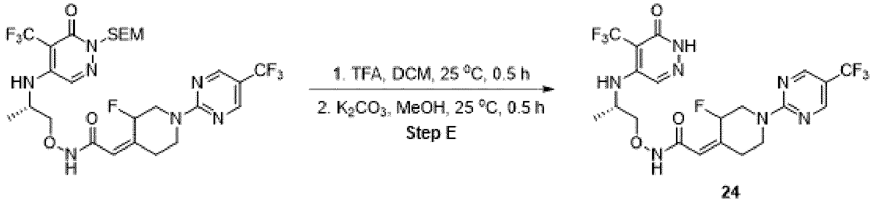

To a solution of (Z)-2-(3-fluoro-1-(5-(trifluoromethyl)pyrimidin-2-yl)piperidin-4 -ylidene)-N-((S)-2-((6-oxo-5-(trifluoromethyl)-1-((2-(trimethylsilyl)ethoxy)methyl)-1,6 -dihydropyridazin-4-yl)amino)propoxy)acetamide (100 mg, 0.15 mmol, 1.0 eq.) in DCM (3 mL) was added TFA (1 mL). The mixture was stirred at 25 °C for 0.5 h. The mixture was concentrated under vacuum. The residue was dissolved in MeOH (2 mL) and K₂CO₃ (40 mg, 0.29 mmol) was added. The mixture was stirred at 25 °C for another 0.5 h. The mixture was filtered and the filtrate was purified by prep-HPLC to afford (Z)-2-(3-fluoro-1-(5-(trifluoromethyl)pyrimidin-2-yl)piperidin-4-ylidene)-N-((S)-2-((6-oxo-5-(trifluoromethyl)-1,6-dihydropyridazin-4-yl)amino)propoxy)acetamide (3.47 mg, 4.31%) as a white solid.
LCMS: *(ESI)* (M+H)⁺ = 540.0.
¹H NMR: (400 MHz, DMSO-*d₆*) *δ* 12.49 (s, 1H), 11.52 (s, 1H), 8.76 - 8.70 (m, 2H), 7.93 (s, 1H), 6.72 - 6.52 (m, 2H), 5.78 (s, 1H), 5.27 - 5.15 (m, 1H), 4.95 - 4.84 (m, 1H), 4.31 - 4.21 (m, 1H), 4.01 - 3.95 (m, 1H), 3.93 - 3.86 (m, 1H), 3.32 - 3.17 (m, 1H), 3.13 - 3.02 (m, 1H), 2.64 - 2.57 (m, 1H), 2.35 - 2.30 (m, 1H), 1.20 (d, *J* = 6.4 Hz, 3H).

### Example 25:

### (S)-N-(2-((6-oxo-5-(trifluoromethyl)-1,6-dihydropyridazin-4-yl)amino)propoxy)-2-(1-( 5-(trifluoromethyl)pyrimidin-2-yl)-1,2,3,6-tetrahydropyridin-4-yl)acetamide

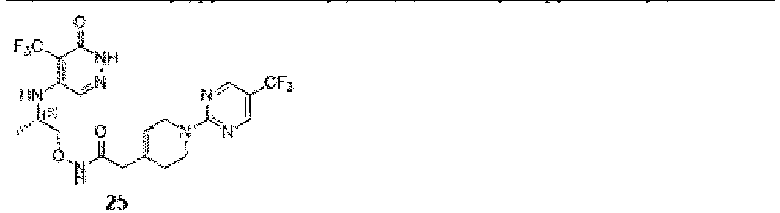

### 25.1) Synthesis of intermediate X

### Step A: synthesis of methyl 2-(1-(5-(trifluoromethyl)pyrimidin-2-yl)-1,2,3,6 -tetrahydropyridin-4-yl)acetate

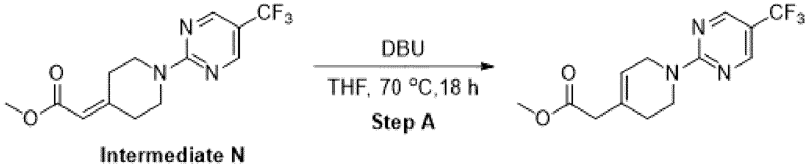

To a mixture of methyl 2-(1-(5-(trifluoromethyl)pyrimidin-2-yl)piperidin-4-ylidene) acetate (10.00 g, 33.19 mmol, 1.0 eq.) in THF (100 mL) was added DBU (40.43 g, 265.54 mmol, 8.0 eq.). The mixture was stirred at 70 °C for 18 h. The reaction mixture was cooled to room temperature and quenched with water (300 mL), extracted with EtOAc (3 x 200 mL). The organic layers were combined and dried over Na₂SO₄, concentrated under vacuum. The residue was purified by column chromatography (Petroleum ether/EtOAc= 10/1; V/V) to afford methyl 2-(1-(5-(trifluoromethyl)pyrimidin-2-yl)-1,2,3,6-tetrahydropyridin-4-yl)acetate (7.45 g, 74.5%) as a colorless oil.
LCMS: *(ESI)* (M+H)⁺ = 302.2.
¹H NMR: (400 MHz, DMSO-*d₆*) *δ* 8.73 - 8.68 (m, 2H), 5.70 - 5.65 (m, 1H), 4.26 - 4.21 (m, 2H), 3.95 (t, *J* = 5.6 Hz, 2H), 3.60 (s, 3H), 3.12 (s, 2H), 2.22 - 2.13 (m, 2H).

### Step B: synthesis of 2-(1-(5-(trifluoromethyl)pyrimidin-2-yl)-1,2,3,6-tetrahydropyridin -4-yl)acetic acid

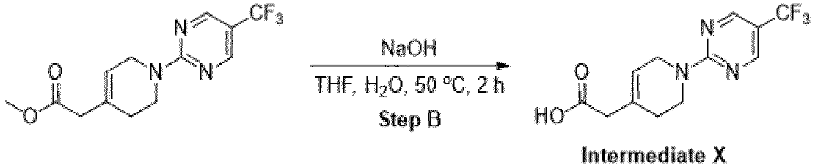

To a mixture of methyl 2-(1-(5-(trifluoromethyl)pyrimidin-2-yl)-1,2,3,6 -tetrahydropyridin-4-yl)acetate (6.93 g, 23.00 mmol, 1.0 eq.) in THF (34 mL) and H₂O (17 mL) was added NaOH (2.76 g, 69.00 mmol, 3.0 eq.). The mixture was stirred at 50 °C for 2 h. The mixture was cooled to room temperature and adjusted to pH 6 with 1M HCl. The mixture was extracted with EtOAc (2 x 100 mL). The organic layers were combined and washed with brine (300 mL), dried over Na₂SO₄ and concentrated under vacuum to afford 2-(1-(5-(trifluoromethyl)pyrimidin-2-yl)-1,2,3,6-tetrahydropyridin -4-yl)acetic acid (6.18 g, 93.5%) as a yellow solid.
LCMS: *(ESI)* (M+H)⁺ = 288.0.
¹H NMR: (400 MHz, CDCl₃) *δ* 8.43 (s, 2H), 5.67 - 5.60 (m, 1H), 4.26 - 4.18 (m, 2H), 3.96 (t, *J=* 5.6 Hz, 2H), 3.05 (s, 2H), 2.27 - 2.16 (m, 2H).

### 25.2) Synthesis of compound 25

### Step A: Synthesis of (S)-N-(2-((6-oxo-5-(trifluoromethyl)-1-((2-(trimethylsilyl)ethoxy) methyl)-1,6-dihydropyridazin-4-yl)amino)propoxy)-2-(1-(5-(trifluoromethyl)pyrimidin-2-yl)-1,2,3,6-tetrahydropyridin-4-yl)acetamide

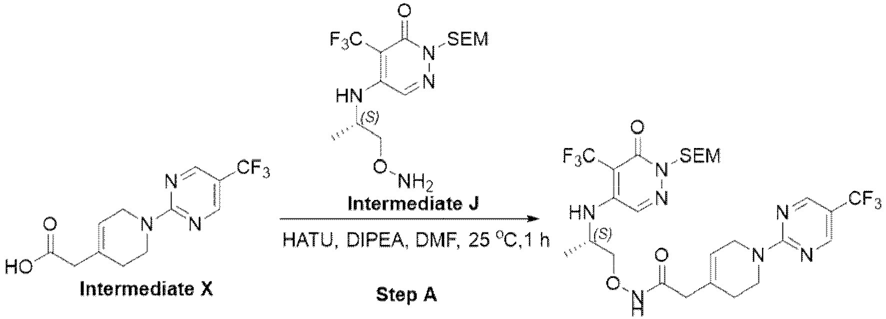

To a solution of 2-(1-(5-(trifluoromethyl)pyrimidin-2-yl)-1,2,3,6-tetrahydropyridin-4-yl)acetic acid (6.10 g, 21.23 mmol, 1.1 eq.) and (S)-5-((1-(aminooxy)propan-2-yl)amino)-4-(trifluoromethyl)-2-((2-(trimethylsilyl)ethox y)methyl)pyridazin-3(2H)-one (7.38 g, 19.30 mmol, 1.0 eq.) in DMF (200 mL) were added DIPEA (7.48 g, 57.90 mmol, 3.0 eq.) and HATU (8.81 g, 23.16 mmol, 1.2 eq.). The mixture was stirred at 25 °C for 1 h. The mixture was quenched with water (600 mL) and extracted with EtOAc (3 x 500 mL). The organic layers were combined and washed with brine (2 x 1000 mL), dried over Na₂SO₄ and concentrated under reduced pressure. The residue was purified by column chromatography (EtOAc/Petroleum ether= 2/1; V/V) to afford (S)-N-(2-((6-oxo-5-(trifluoromethyl)-1-((2-(trimethylsilyl)ethoxy)methyl)-1,6-dihydrop yridazin-4-yl)amino)propoxy)-2-(1-(5-(trifluoromethyl)pyrimidin-2-yl)-1,2,3,6-tetrahyd ropyridin-4-yl)acetamide (10.69 g, 84.9%) as a yellow oil.
LCMS: *(ESI)* (M+H)⁺ = 652.2.

### Step B: synthesis of (S)-N-(2-((6-oxo-5-(trifluoromethyl)-L6-dihydropyridazin-4-yl) amino)propoxy)-2-(1-(5-(trifluoromethyl)pyrimidin-2-yl)-1,2,3,6-tetrahydropyridin-4-yl acetamide

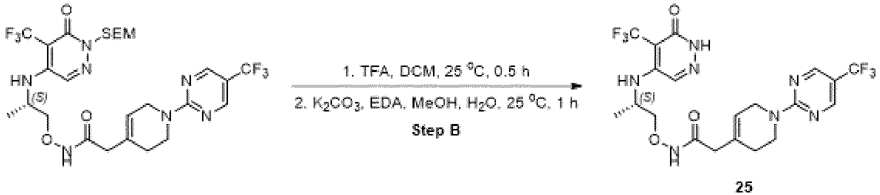

To a solution of (S)-N-(2-((6-oxo-5-(trifluoromethyl)-1-((2-(trimethylsilyl)ethoxy) methyl)-1,6-dihydropyridazin-4-yl)amino)propoxy)-2-(1-(5-(trifluoromethyl)pyrimidin-2-yl)-1,2,3,6-tetrahydropyridin-4-yl)acetamide (5.74 g, 8.81 mmol, 1.0 eq.) in DCM (60 mL) was added TFA (20 mL). The resulting solution was stirred at 25 °C for 0.5 h. The mixture was concentrated under vacuum. The residue was dissolved in a mixed solvent of MeOH (100 mL) and H₂O (100 mL). K₂CO₃ (4.87 g, 35.24 mmol, 4.0 eq.) and ethylenediamine (3.92 g, 65.19 mmol, 7.4 eq.) were added to the reaction mixture. The mixture was stirred at 25 °C for another 1 h. The mixture was quenched with water (300 mL) and extracted with DCM (3 x 200 mL). The organic layers were combined and washed with brine (300 mL), dried over Na₂SO₄ and concentrated under vacuum. The residue was purified by prep-HPLC to afford (S)-N-(2-((6-oxo-5-(trifluoromethyl)-1,6-dihydropyridazin-4-yl)amino)propoxy)-2-(1-( 5-(trifluoromethyl)pyrimidin-2-yl)-1,2,3,6-tetrahydropyridin-4-yl)acetamide (1.54 g, 33.6%) as a white solid.
LCMS: *(ESI)* (M+H)⁺ = 522.2.
¹H NMR: (400 MHz, DMSO-*d₆*) *δ* 12.49 (s, 1H), 11.23 (s, 1H), 8.70 (s, 2H), 7.90 (s, 1H), 6.65 - 6.48 (m, 1H), 5.63 (s, 1H), 4.28 - 4.15 (m, 3H), 3.97 - 3.88 (m, 3H), 3.86 - 3.79 (m, 1H), 2.76 (s, 2H), 2.19 - 2.10 (m, 2H), 1.18 (d, *J* = 6.8 Hz, 3H).

### Example 26:

### (S)-2-(1-(5-(2-fluorophenyl)pyrimidin-2-yl)-1,2,3,6-tetrahydropyridin-4-yl)-N-(2-((6 -oxo-5-(trifluoromethyl)-1,6-dihydropyridazin-4-yl)amino)propoxy)acetamide

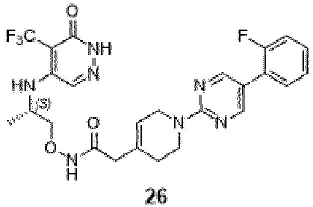

### Step A: synthesis of methyl 2-(1-(5-iodopyrimidin-2-yl)piperidin-4-ylidene)acetate

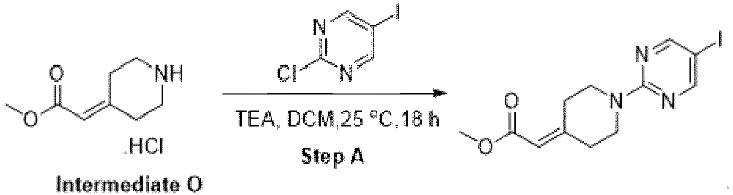

To a solution of methyl 2-(piperidin-4-ylidene)acetate hydrochloride (7.51 g, 39.18 mmol, 1.0 eq.) in DCM (80 mL) were added TEA (11.90 g, 117.55 mmol, 3.0 eq.) and 2-chloro-5-iodopyrimidine (9.61 g, 39.97 mmol, 1.02 eq.). The mixture was stirred at 25 °C for 16 h. The mixture was diluted with water (100 mL) and extracted with EtOAc (2 x 150 mL). The organic layers were combined and washed with saturated aqueous NH₄Cl (2 x 120 mL), dried over Na₂SO₄ and concentrated under vacuum. The residue was purified by silica gel column chromatography (EtOAc/Petroleum ether= 5/1; V/V) to afford methyl 2-(1-(5-iodopyrimidin-2-yl)piperidin-4-ylidene)acetate (8.20 g, 58.3%) as a white solid.
LCMS: *(ESI)* (M+H)⁺ = 360.0.
¹H NMR: (400 MHz, CDCl₃) *δ* 8.40 (s, 2H), 5.78 - 5.75 (m, 1H), 3.91 - 3.83 (m, 4H), 3.71 (s, 3H), 3.06 - 2.96 (m, 2H), 2.41 - 2.32 (m, 2H).

### Step B: synthesis of methyl 2-(1-(5-iodopyrimidin-2-yl)-1,2,3,6-tetrahydropyridin -4-yl)acetate

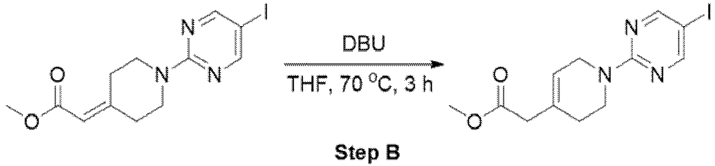

To a solution of methyl 2-(1-(5-iodopyrimidin-2-yl)piperidin-4-ylidene)acetate (2.5 g, 6.94 mmol, 1.0 eq.) in THF (30 mL) was added DBU (5.3 g, 34.72 mmol, 5.0 eq.). The resulting mixture was stirred at 70 °C. for 3 h. The mixture was concentrated under reduced pressure. The residue was purified by column chromatography (Petroleum ether/EtOAc=8/1, V/V) to afford methyl 2-(1-(5-iodopyrimidin-2-yl)-1,2,3,6 -tetrahydropyridin-4-yl)acetate (1.7 g, 68 %) as a colorless oil.
LCMS: (ESI) (M+H)⁺ = 360.0.
¹H NMR : N001-08173-1 (400 MHz, DMSO) *δ* 8.51 (s, 2H), 5.71 - 5.60 (m, 1H), 4.14 - 4.05 (m, 2H), 3.81 (t, J = 5.8 Hz, 2H), 3.60 (s, 3H), 3.10 (s, 2H), 2.10 - 2.14 (m, 2H).

### Step C: Synthesis of methyl 2-(1-(5-(2-fluorophenyl)pyrimidin-2-yl)-1,2,3,6 -tetrahydropyridin-4-yl)acetate

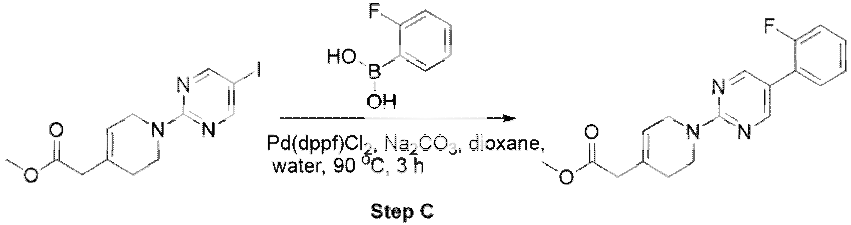

To a solution of methyl 2-(1-(5-iodopyrimidin-2-yl)-1,2,3,6 -tetrahydropyridin-4-yl)acetate (700 mg, 1.949 mmol, 1.0 eq), (2-fluorophenyl)boronic acid (542 mg, 3.899 mmol, 2.0 eq), water (1 ml) and Na₂CO₃, (515 mg, 4.861 mmol, 2.5 eq) in dioxane (6 mL) was added Pd(dppf)Cl₂ (114 mg, 0.155 mmol, 0.08 eq) at 0 °C under N₂ atmosphere. The mixture was stirred at 90 °C for 3 h under N₂ atmosphere. The mixture was concentrated under vacuum. The residue was purified by column chromatography (Petroleum ether/EtOAc=10/1, V/V) to afford methyl 2-(1-(5-(2-fluorophenyl)pyrimidin-2-yl)-1,2,3,6-tetrahydropyridin-4-yl)acetate (600 mg, 94% yield) as a white solid.
LCMS: *(ESI)* (M+H)⁺ = 327.9.
¹H NMR: (400 MHz, DMSO) *δ* 8.59 (d, J = 1.5 Hz, 2H), 7.57 (td, J = 7.8, 1.5 Hz, 1H), 7.45 - 7.36 (m, 1H), 7.30 - 7.28 (m, 2H), 5.69 (s, 1H), 4.21 (s, 2H), 3.93 (t, J = 5.8 Hz, 2H), 3.61 (s, 3H), 3.12 (s, 2H), 2.19 - 2.17 (m, 2H).

### Step D: Synthesis of 2-(1-(5-(2-fluorophenyl)pyrimidin-2-yl)-1,2,3,6-tetrahydropyridin -4-yl)acetic acid

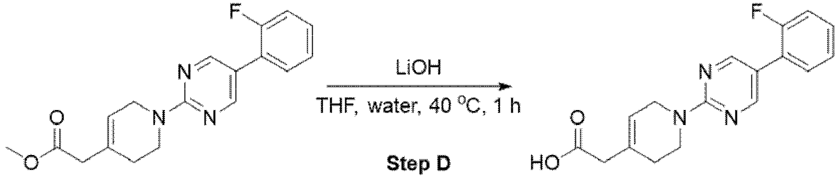

To a solution of methyl 2-(1-(5-(2-fluorophenyl)pyrimidin-2-yl)-1,2,3,6-tetrahydropyridin-4-yl)acetate (600 mg, 1.834 mmol, 1.0 eq.) in a mixed solvent of THF (6mL) and water (4 mL) was added LiOH (45 mg, 3.669 mmol, 2.0 eq.). The mixture was stirred at 40 °C for 1 h. The mixture was diluted with water (5 mL) and extracted with EtOAc (2 x 10 mL). The water layer was adjusted to pH 6 with 1M HCl. The mixture was extracted with EtOAc (3 x 15 mL), dried over Na₂SO₄ and concentrated under vacuum to afford 2-(1-(5-(2-fluorophenyl)pyrimidin-2-yl)-1,2,3,6 -tetrahydropyridin-4-yl)acetic acid (500 mg, 87% yield) as a white solid.
LCMS: *(ESI)* (M+H)⁺ = 314.2.
¹H NMR : (400 MHz, DMSO) *δ* 12.24 (s, 1H), 8.59 (d, J = 1.5 Hz, 2H), 7.57 (td, J = 7.8, 1.5 Hz, 1H), 7.44 - 7.37 (m, 1H), 7.35 - 7.26 (m, 2H), 5.67 (s, 1H), 4.21 (s, 2H), 3.93 (t, J = 5.7 Hz, 2H), 3.02 (s, 2H), 2.19 (s, 2H).

### Step E: Synthesis of (S)-2-(1-(5-(2-fluorophenyl)pyrimidin-2-yl)-1,2,3,6 -tetrahydropyridin-4-yl)-N-(2-((6-oxo-5-(trifluoromethyl)-1-((2-(trimethylsilyl)ethoxy) methyl)-1,6-dihydropyridazin-4-yl)amino)propoxy)acetamide

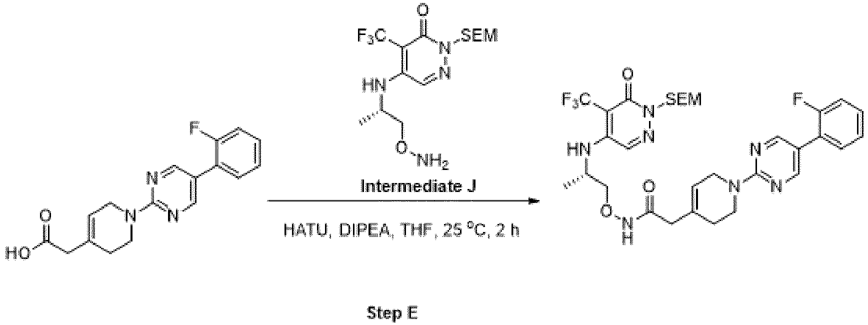

To a solution of 2-(1-(5-(2-fluorophenyl)pyrimidin-2-yl)-1,2,3,6-tetrahydropyridin -4-yl)acetic acid (300 mg, 0.958 mmol, 1.0 eq.) in THF (5 mL) were added DIPEA (371 mg, 2.875 mmol, 3.0 eq.) and HATU (437 mg, 1.150 mmol, 1.2 eq.) followed by dropwise addition of (S)-5-((1-(aminooxy)propan-2-yl)amino)-4-(trifluoromethyl)-2-((2-(trimethylsilyl)ethoxy)methyl)pyridazin-3(2H)-one (402 mg, 1.054 mmol, 1.1 eq.). The resulting mixture was stirred at 25 °C for 2 h. The reaction mixture was quenched with water (10 mL) and extracted with EtOAc (3 x 10 mL). The organic layers were combined and washed with brine (10 mL), dried over Na₂SO₄ and concentrated under vacuum. The residue was purified by column chromatography (Petroleum ether/EtOAc=1/3, V/V) to afford (S)-2-(1-(5-(2-fluorophenyl)pyrimidin-2-yl) -1,2,3,6-tetrahydropyridin-4-yl)-N-(2-((6-oxo-5-(trifluoromethyl)-1-((2-(trimethylsilyl)e thoxy)methyl)-1,6-dihydropyridazin-4-yl)amino)propoxy)acetamide (500 mg, 77% yield) as a yellow solid.
LCMS: *(ESI)* (M+H)⁺ = 678.2.

### Step F: synthesis of (S)-2-(1-(5-(2-fluorophenyl)pyrimidin-2-yl)-L2, 3,6-tetrahydropyridin-4-yl)-N-(2-((6-oxo-5-(trifluoromethyl)-1,6-dihydropyridazin-4-yl) amino)propoxy)acetamide

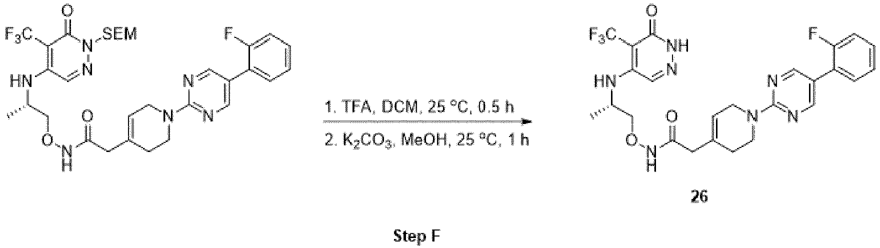

To a solution of (S)-2-(1-(5-(2-fluorophenyl)pyrimidin-2-yl)-1,2,3,6 -tetrahydropyridin-4-yl)-N-(2-((6-oxo-5-(trifluoromethyl)-1-((2-(trimethylsilyl)ethoxy) methyl)-1,6-dihydropyridazin-4-yl)amino)propoxy)acetamide (350 mg, 0.516 mmol, 1.0 eq.) in DCM (3 mL) was added TFA (0.5 mL). The resulting mixture was stirred at 25 °C for 0.5 h. The mixture was neutralized with saturated NaHCOs (5 mL) and extracted with DCM (3 x 10 mL). The organic layers were combined and dried over Na₂SO₄, concentrated under vacuum. The residue was redissolved in MeOH (5 mL) and K₂CO₃ (178 mg, 1.292 mmol, 2.5 eq.) was added. The mixture was stirred at 25 °C for another 1 h. The mixture was filtered and the filtrate was purified by prep-HPLC to afford (S)-2-(1-(5-(2-fluorophenyl)pyrimidin-2-yl)-1,2,3,6-tetrahydropyridin-4-yl)-N-(2-((6-oxo-5-(trifluoromethyl)-1,6-dihydropyridazin-4-yl)amino)propoxy)acetamide (122 mg, 43% yield) as a white solid.
LCMS: (ESI) (M+H)⁺ = 548.2.
¹H NMR: (400 MHz, DMSO-*d₆*) *δ* 12.51 (s, 1H), 11.26 (s, 1H), 8.61 (d, J = 1.4 Hz, 2H), 7.92 (s, 1H), 7.59 (dd, J = 8.8, 7.0 Hz, 1H), 7.42 (dt, J = 7.2, 3.7 Hz, 1H), 7.33 (dd, J = 15.8, 8.1 Hz, 2H), 6.60 (s, 1H), 5.67 (s, 1H), 4.23 (s, 3H), 3.94 - 3.91 (m, 3H), 3.88 - 3.84 (m, 1H), 2.78 (s, 2H), 2.16 (s, 2H), 1.21 (d, J = 6.5 Hz, 3H).

### Example 27:

### 2-(1-(5-(2-fluorophenyl)pyrimidin-2-yl)-3-methyl-1,2,3,6-tetrahydropyridin-4-yl)-N-((S )-2-((6-oxo-5-(trifluoromethyl)-1,6-dihydropyridazin-4-yl)amino)propoxy)acetamide

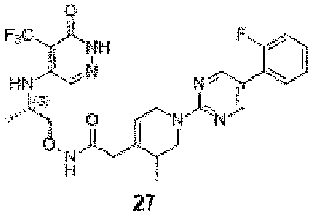

### 27.1) Synthesis of intermediate P

### Step A: synthesis of tert-butyl (E)-4-(2-methoxy-2-oxoethylidene)-3-methylpiperidine -1-carboxylate

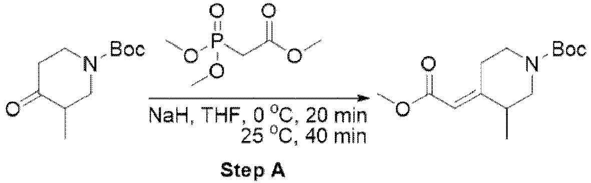

To a solution of methyl 2-(dimethoxyphosphoryl)acetate (95 mg, 0.517 mmol, 1.1 eq.) in THF (2 mL) was added NaH (21 mg, 0.517 mmol, 1.1 eq.) at 0 °C. The resulting mixture was stirred at 0 °C for 20 mins. Then tert-butyl 3-methyl-4-oxopiperidine-1-carboxylate (100 mg, 0.47 mmol, 1.0 eq.) was added to the mixture at 0 °C. The resulting mixture was stirred at 25 °C for 40 mins. The mixture was quenched by water (15 mL), extracted with EtOAc (3 x 20 mL). The organic layers were combined, dried over Na₂SO₄ and concentrated under reduced pressure. The residue was purified by column chromatography (Petroleum ether/EtOAc= 4/1, V/V) to afford tert-butyl (E)-4-(2-methoxy-2-oxoethylidene)-3-methylpiperidine-1-carboxylate (120 mg, 95.2%) as a colorless oil.
LCMS: *(ESI)* (M+H-100)⁺ = 170.2.
¹H NMR (400 MHz, DMSO-*d₆*) *δ* 5.70 (d, J = 1.6 Hz, 1H), 3.90 - 3.76 (m, 1H), 3.62 (s, 3H), 3.58 - 3.40 (m, 2H), 3.18 - 2.79 (m, 3H), 2.46 - 2.36 (m, 1H), 1.41 (s, 9H), 1.02 (d, J = 6.8 Hz, 3H).

### Step B: Synthesis of methyl (E)-2-(3-methylpiperidin-4-ylidene)acetate hydrochloride

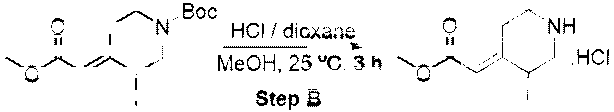

To a solution of tert-butyl (E)-4-(2-methoxy-2-oxoethylidene)-3-methylpiperidine -1-carboxylate (3.8 g, 14.13 mmol, 1.0 eq.) in MeOH (30 mL) was added HCl/dioxnae (4M, 15 mL). The mixture was stirred at 25 °C for 3 h. The mixture was concentrated under vacuum to afford methyl (E)-2-(3-methylpiperidin-4-ylidene)acetate (3.18 g, 100% yield) as a colorless oil.
LCMS: *(ESI)* (M+H)⁺ = 170.2.

### Step C: synthesis of methyl (E)-2-(1-(5-bromopyrimidin-2-yl)-3-methylpiperidin-4 -ylidene)acetate

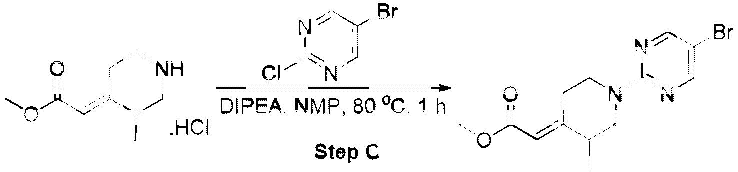

To a solution of methyl (E)-2-(3-methylpiperidin-4-ylidene)acetate hydrochloride (2.7 g, 15.98 mmol, 1 eq.) in NMP (30 mL) were added DIPEA (6.1 g, 47.94 mmol, 3.0 eq.) and 5-bromo-2-chloropyrimidine (3 g, 15.98 mmol, 1 eq.). The resulting mixture was stirred at 80 °C for 1 h. The mixture was cooled to room temperature and diluted with water (25 mL). The mixture was extracted with EtOAc (2 x 20 mL). The organic layers were combined, dried over Na₂SO₄ and concentrated under reduced pressure. The residue was purified by column chromatography (Petroleum ether/EtOAc= 96/4, V/V) to afford methyl (E)-2-(1-(5-bromopyrimidin-2-yl)-3-methylpiperidin-4-ylidene)acetate (3.5 g, 68.8%) as a pale yellow oil.
LCMS: *(ESI)* (M+H)⁺ = 326.0.
¹H NMR (400 MHz, DMSO-*d₆*) *δ* 8.49 (s, 2H), 5.74 (d, J = 1.4 Hz, 1H), 4.20 - 4.11 (m, 1H), 4.12 - 4.02 (m, 1H), 3.65 (s, 3H), 3.60 - 3.51 (m, 1H), 3.34 - 3.25 (m, 3H), 2.67 - 2.59 (m, 1H), 1.06 (d, J = 6.8 Hz, 3H).

### Step D: synthesis of methyl 2-(1-(5-bromopyrimidin-2-yl)-3-methyl-1,2,3,6-tetrahydropyridin-4-yl)acetate (Intermediate P)

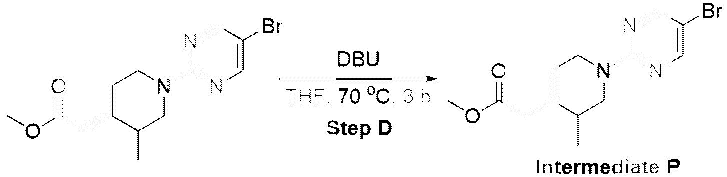

To a solution of methyl (E)-2-(1-(5-bromopyrimidin-2-yl)-3-methylpiperidin-4-ylidene)acetate (1.78 g, 5.48 mmol, 1 eq.) in THF (18 mL) was added DBU (4.2 g, 27.3 mmol, 5.0 eq.). The resulting mixture was stirred at 70 °C for 3 h. The mixture was concentrated under reduced pressure. The residue was purified by column chromatography (Petroleum ether/EtOAc= 96/4, V/V) to afford methyl 2-(1-(5-bromopyrimidin-2-yl)-3-methyl-1,2,3,6-tetrahydropyridin-4-yl)acetate (1 g, 56.8%) as a colorless oil.
LCMS: *(ESI)* (M+H)⁺ = 326.0.
¹H NMR (400 MHz, DMSO-*d₆*) *δ* 8.46 (d, J = 1.3 Hz, 2H), 5.69 - 5.51 (m, 1H), 4.35 - 4.12 (m, 1H), 4.04 - 3.96 (m, 1H), 3.96 - 3.87 (m, 1H), 3.62 (s, 3H), 3.61 - 3.56 (m, 1H), 3.20 - 3.09 (m, 2H), 2.45 - 2.31 (m, 1H), 0.96 (d, J = 7.0 Hz, 3H).

### 27.2) Synthesis of compound 27

### Step A: Synthesis of methyl 2-(1-(5-(2-fluorophenyl)pyrimidin-2-yl)-3-methyl-1,2,3,6 -tetrahydropyridin-4-yl)acetate

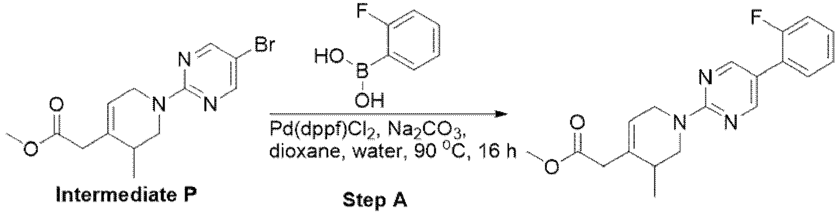

To a solution of methyl 2-(1-(5-bromopyrimidin-2-yl)-3-methyl-1,2,3,6 -tetrahydropyridin-4-yl)acetate (450 mg, 1.39 mmol, 1 eq.) in a mixed solvent of dioxane (7 mL) and H₂O (1 mL) were added (2-fluorophenyl)boronic acid (388 mg, 2.77 mmol, 2.0 eq.), Pd(dppf)Cl₂ (102 mg, 0.19 mmol, 0.14 eq.) and Na₂CO₃ (294 mg, 2.77 mmol, 2.0 eq.).The resulting mixture was stirred at 90 °C for 16 h under N₂ atmosphere. The reaction mixture was extracted with EtOAc (3 x 20 mL), washed with water (20 mL). The organic layers were combined and washed with brine (10 mL), dried over Na₂SO₄ and concentrated under vacuum. The residue was purified by silica gel chromatography (Petroleum ether/EtOAc= 97/3, V/V) to afford methyl 2-(1-(5-(2-fluorophenyl)pyrimidin-2-yl)-3-methyl-1,2,3,6-tetrahydropyridin-4-yl)acetat e (590 mg, 100% yield) as a yellow oil.
LCMS: *(ESI)* (M+H)⁺ = 342.2.
¹H NMR (400 MHz, DMSO-*d₆*) *δ* 8.60 (s, 2H), 7.62 - 7.54 (m, 1H), 7.47 - 7.38 (m, 1H), 7.37 - 7.27 (m, 2H), 5.66 (d, J = 3.4 Hz, 1H), 4.42 - 4.28 (m, 1H), 4.10 - 3.98 (m, 2H), 3.72 - 3.65 (m, 1H), 3.64 (s, 3H), 3.22 - 3.14 (m, 2H), 2.46 - 2.35 (m, 1H), 1.00 (d, J = 6.9 Hz, 3H).

### Step B: Synthesis of 2-(1-(5-(2-fluorophenyl)pyrimidin-2-yl)-3-methyl-1,2,3,6 -tetrahydropyridin-4-yl)acetic acid

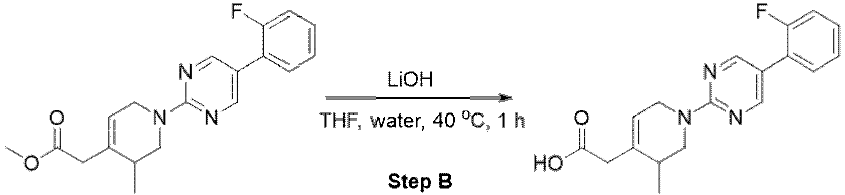

To a solution of methyl 2-(1-(5-(2-fluorophenyl)pyrimidin-2-yl)-3-methyl-1,2,3,6-tetrahydropyridin-4-yl)acetate (590 mg, 1.74 mmol, 1.0 eq.) in a mixed solvent of THF (4 mL) and water (2 mL) was added LiOH (125mg, 5.2 mmol, 3.0 eq.). The mixture was stirred at 40 °C for 1 h. The mixture was diluted with water (25 mL) and extracted with EtOAc (3 x 10 mL). The water layer was adjusted to pH 6 with 1M HCl. The mixture was extracted with EtOAc (3 x 15 mL), dried over Na₂SO₄ and concentrated under vacuum to afford 2-(1-(5-(2-fluorophenyl)pyrimidin-2-yl)-3-methyl-1,2,3,6 -tetrahydropyridin-4-yl)acetic acid (470 mg, 84.3% yield) as a yellow oil.
LCMS: *(ESI)* (M+H)⁺ = 328.2.

### Step C: synthesis of 2-(1-(5-(2-fluorophenyl)pyrimidin-2-yl)-3-methyl-1,2,3,6-tetrahydropyridin-4-yl)-N-((S)-2-((6-oxo-5-(trifluoromethyl)-1-((2-(trimethylsilyl)ethox y)methyl)-1,6-dihydropyridazin-4-yl)amino)propoxy)acetamide

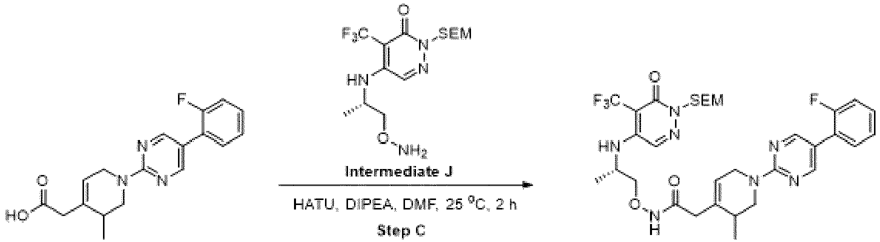

To a solution of 2-(1-(5-(2-fluorophenyl)pyrimidin-2-yl)-3-methyl-1,2,3,6-tetrahydropyridin-4-yl)acetic acid (470 mg, 1.44 mmol, 1 eq.) in DMF (8 mL) were added DIPEA (372 mg, 2.88 mmol, 2.0 eq.) and HATU (820 mg, 2.16 mmol, 1.5 eq.) followed by dropwise addition of (S)-5-((1-(aminooxy)propan-2-yl)amino)-4-(trifluoromethyl)-2-((2-(trimethylsilyl) ethoxy)methyl)pyridazin-3(2H)-one (570 mg, 1.51 mmol, 1.05 eq.). The resulting mixture was stirred at 25 °C for 2 h. The reaction mixture was quenched with water (30 mL) and extracted with EtOAc (3 x 20 mL). The organic layers were combined and washed with brine (10 mL), dried over Na₂SO₄ and concentrated under vacuum. The residue was purified by silica gel chromatography (Petroleum ether/EtOAc= 3/7, V/V) to afford 2-(1-(5-(2-fluorophenyl)pyrimidin-2-yl)-3-methyl-1,2,3,6-tetrahydropyridin-4 -yl)-N-((S)-2-((6-oxo-5-(trifluoromethyl)-1-((2-(trimethylsilyl)ethoxy)methyl)-1,6-dihy dropyridazin-4-yl)amino)propoxy)acetamide (540 mg, 54.3% yield) as a yellow solid. LCMS: *(ESI)* (M+H)⁺ = 692.4.

### Step D: synthesis of 2-(1-(5-(2-fluorophenyl)pyrimidin-2-yl)-3-methyl-1,2,3,6-tetrahydropyridin-4-yl)-N-((S)-2-((6-oxo-5-(trifluoromethyl)-1,6-dihydropyridazin-4-yl) amino)propoxy)acetamide

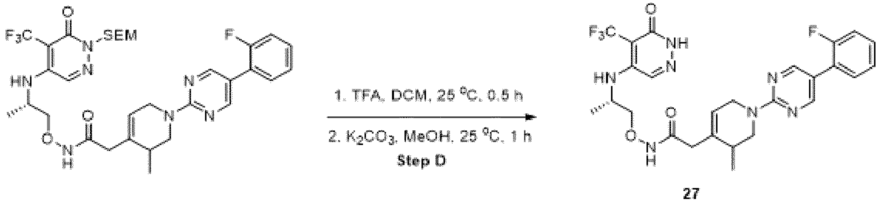

To a solution of 2-(1-(5-(2-fluorophenyl)pyrimidin-2-yl)-3-methyl-1,2,3,6-tetrahydropyridin-4-yl)-N-((S)-2-((6-oxo-5-(trifluoromethyl)-1-((2-(trimethylsilyl)ethox y)methyl)-1,6-dihydropyridazin-4-yl)amino)propoxy)acetamide (540 mg, 0.78 mmol, 1.0 eq.) in DCM (5 mL) was added TFA (0.5 mL). The resulting mixture was stirred at 25 °C for 0.5 h. The mixture was neutralized with saturated NaHCO₃ aqueous solution (5 mL) and extracted with DCM (3 x 10 mL). The organic layers were combined and dried over Na₂SO₄, concentrated under vacuum. The residue was redissolved in MeOH (5 mL) and K₂CO₃ (337 mg, 2.44 mmol, 3.0 eq.) was added. The mixture was stirred at 25 °C for another 1 h. The reaction mixture was filtered and the filtrate was purified by pre-HPLC to afford 2-(1-(5-(2-fluorophenyl)pyrimidin-2-yl)-3-methyl-1,2,3,6 -tetrahydropyridin-4-yl)-N-((S)-2-((6-oxo-5-(trifluoromethyl)-1,6-dihydropyridazin-4-yl )amino)propoxy)acetamide (46.9 mg, 10.7% yield) as a white solid.
LCMS: *(ESI)* (M+H)⁺ = 562.2.
¹H NMR (400 MHz, DMSO-*d₆*) *δ* 12.48 (s, 1H), 11.25 (s, 1H), 8.58 (d, J = 1.4 Hz, 2H), 7.90 (s, 1H), 7.60 - 7.53 (m, 1H), 7.46 - 7.35 (m, 1H), 7.35 - 7.25 (m, 2H), 6.68 - 6.54 (m, 1H), 5.60 (s, 1H), 4.36 (d, J = 18.2 Hz, 1H), 4.20 (s, 1H), 4.08 (dd, J = 12.9, 4.1 Hz, 1H), 4.03 - 3.78 (m, 3H), 3.57 (dd, J = 12.6, 3.9 Hz, 1H), 2.86 - 2.70 (m, 2H), 2.35 (d, J = 17.7 Hz, 1H), 1.19 (d, J = 6.5 Hz, 3H), 0.99 (d, 3H).

### Example 28:

### (S)-2-(1-(5-(2-fluorophenyl)pyrimidin-2-yl)-5-methyl-1,2,3,6-tetrahydropyridin-4-yl)-N -(2-((6-oxo-5-(trifluoromethyl)-1,6-dihydropyridazin-4-yl)amino)propoxy)acetamide

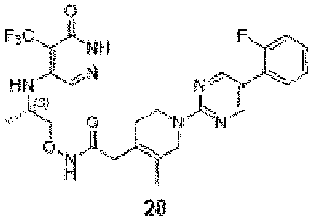

### Step A: synthesis of (S)-2-(1-(5-(2-fluorophenyl)pyrimidin-2-yl)-5-methyl-1,2,3,6-tetrahydropyridin-4-yl)-N-(2-((6-oxo-5-(trifluoromethyl)-1,6-dihydropyridazin-4-yl)ami no)propoxy)acetamide

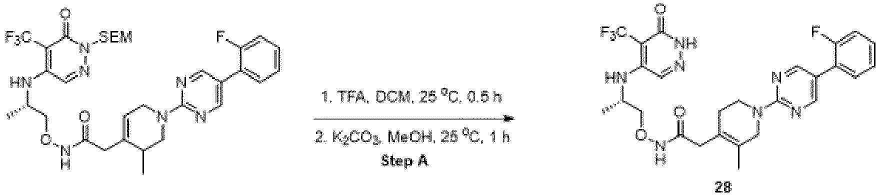

To a solution of 2-(1-(5-(2-fluorophenyl)pyrimidin-2-yl)-3-methyl-1,2,3,6-tetrahydropyridin-4-yl)-N-((S)-2-((6-oxo-5-(trifluoromethyl)-1-((2-(trimethylsilyl)ethox y)methyl)-1,6-dihydropyridazin-4-yl)amino)propoxy)acetamide (540 mg, 0.78 mmol, 1.0 eq.) in DCM (5 mL) was added TFA (0.5 mL). The resulting mixture was stirred at 25 °C for 0.5 h. The mixture was neutralized with saturated NaHCO₃ aqueous solution (5 mL) and extracted with DCM (3 x 10 mL). The organic layers were combined and dried over Na₂SO₄, concentrated under vacuum. The residue was redissolved in MeOH (5 mL) and K₂CO₃ (337 mg, 2.44 mmol, 3.0 eq.) was added. The mixture was stirred at 25 °C for another 1 h. The reaction mixture was filtered and the filtrate was purified by pre-HPLC to afford (S)-2-(1-(5-(2-fluorophenyl)pyrimidin-2-yl)-5-methyl-1,2,3,6-tetrahydropyridin-4-yl)-N-(2-((6-oxo-5-(trifluoromethyl)-1,6-dihydropyridazin-4-yl)ami no)propoxy)acetamide (33 mg, 15.1% yield) as a white solid.
LCMS: (ESI) (M+H)⁺ = 562.2.
¹H NMR (400 MHz, DMSO-*d₆*) *δ* 12.49 (s, 1H), 11.19 (s, 1H), 8.58 (d, J = 1.5 Hz, 2H), 7.89 (s, 1H), 7.61 - 7.52 (m, 1H), 7.44 - 7.36 (m, 1H), 7.35 - 7.26 (m, 2H), 6.66 - 6.54 (m, 1H), 4.20 (s, 1H), 4.10 (s, 2H), 3.94 - 3.78 (m, 4H), 2.82 (s, 2H), 2.15 (s, 2H), 1.71 (s, 3H), 1.19 (d, J = 6.5 Hz, 3H).

### Example 29:

### (S)-2-(1-(5-cyclopropylpyrimidin-2-yl)-1,2,3,6-tetrahydropyridin-4-yl)-N-(2-((6-oxo-5-(trifluoromethyl)-1,6-dihydropyridazin-4-yl)amino)propoxy)acetamide

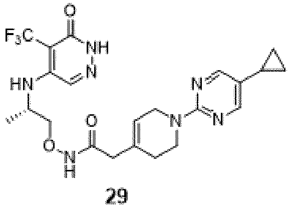

### Step A: synthesis of methyl 2-(1-(5-bromopyrimidin-2-yl)piperidin-4-ylidene)acetate

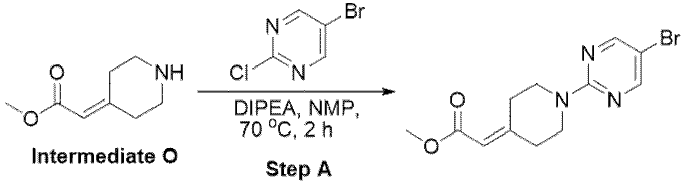

To a solution of methyl 2-(piperidin-4-ylidene)acetate (707 mg, 4.559 mmol, 1.1 eq.) in NMP (10 mL) were added DIPEA (1.6 g, 12.53 mmol, 3.0 eq.) and 5-bromo-2-chloropyrimidine (800 mg, 4.145 mmol, 1.0 eq.). The resulting mixture was stirred at 70 °C. for 2 h. The mixture was concentrated under reduced pressure. The residue was purified by column chromatography (Petroleum ether/EtOAc= 10/1, V/V) to afford methyl 2-(1-(5-bromopyrimidin-2-yl)piperidin-4-ylidene)acetate (870 mg, 67 %) as a colorless oil.
LCMS: *(ESI)* (M+H)⁺ = 312.0.
¹H NMR: (400 MHz, DMSO) *δ* 8.48 (s, 2H), 5.82 (s, 1H), 3.81 (dd, J = 11.6, 5.6 Hz, 4H), 3.62 (s, 3H), 2.91 (t, J = 5.5 Hz, 2H), 2.63 (t, J = 5.5 Hz, 2H).

### Step B: Synthesis of methyl 2-(1-(5-bromopyrimidin-2-yl)-1,2,3,6-tetrahydropyridin -4-yl)acetate

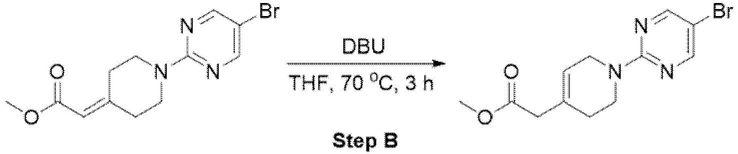

To a solution of methyl 2-(1-(5-bromopyrimidin-2-yl)piperidin-4-ylidene)acetate (870 mg, 2.797 mmol, 1.0 eq.) in THF (10 mL) was added DBU (2.12 g, 13.98 mmol, 5.0 eq.). The resulting mixture was stirred at 70 °C. for 3 h. The mixture was concentrated under reduced pressure. The residue was purified by column chromatography (Petroleum ether/EtOAc=10/1, V/V) to afford methyl 2-(1-(5-bromopyrimidin-2-yl)-1,2,3,6-tetrahydropyridin-4-yl)acetate (570 mg, 65 %) as a white solid.
LCMS: *(ESI)* (M+H)⁺ = 312.0.
¹H NMR: (400 MHz, DMSO) *δ* 8.46 (s, 2H), 5.68 - 5.64 (m, 1H), 4.14 - 4.07 (m, 2H), 3.83 (t, J = 5.8 Hz, 2H), 3.60 (s, 3H), 3.11 (s, 2H), 2.14 (d, J = 1.6 Hz, 2H).

### Step C: Synthesis of methyl 2-(1-(5-cyclopropylpyrimidin-2-yl)-1,2,3,6-tetrahydropyridin-4-yl)acetate

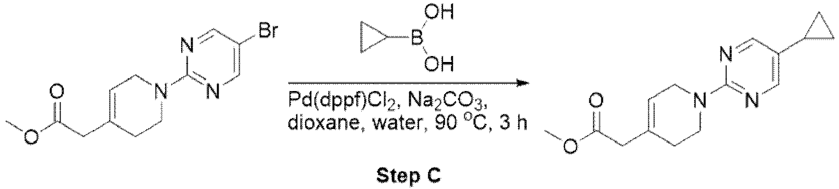

To a solution of methyl 2-(1-(5-bromopyrimidin-2-yl)-1,2,3,6-tetrahydropyridin-4-yl)acetate (530 mg, 1.704 mmol, 1.0 eq.) in dioxane (6 mL) was added cyclopropylboronic acid (293 mg, 3.408 mmol, 2.0 eq.) , Na2CO3 (542 mg, 5.112 mmol, 3.0 eq.), water (1ml) and Pd(dppf)Cl2 (124 mg, 0.170 mmol, 0.1 eq.) under N₂ atmosphere. The resulting mixture was stirred at 90 °C for 3 h under N₂ atmosphere. The mixture was concentrated under vacuum. The residue was purified by column chromatography (Petroleum ether/EtOAc=10/1, V/V) to afford methyl 2-(1-(5-cyclopropylpyrimidin-2-yl)-1,2,3,6-tetrahydropyridin-4-yl)acetate (260 mg, 55% yield)as a yellow oil.
LCMS: *(ESI)* (M+H)⁺ = 274.2.

### Step D: Synthesis of 2-(1-(5-cyclopropylpyrimidin-2-yl)-1,2,3,6-tetrahydropyridin-4-yl)acetic acid

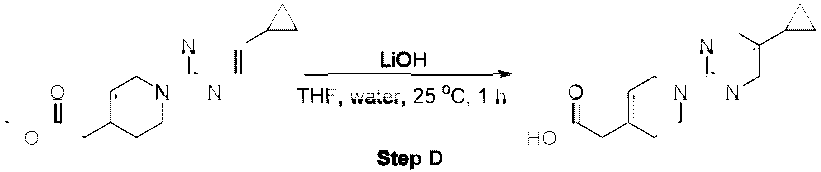

To a solution of methyl 2-(1-(5-cyclopropylpyrimidin-2-yl)-1,2,3,6-tetrahydropyridin-4-yl)acetate (260 mg, 0.952 mmol, 1.0 eq.) in a mixed solvent of THF (3mL) and water (2 mL) was added LiOH (45 mg, 1.904 mmol, 2.0 eq.). The mixture was stirred at 25 °C for 1 h. The mixture was diluted with water (5 mL) and extracted with EtOAc (2 x 10 mL). The water layer was adjusted to pH 6 with 1M HCl. The mixture was extracted with EtOAc (3 x 15 mL), dried over Na₂SO₄ and concentrated under vacuum to afford 2-(1-(5-cyclopropylpyrimidin-2-yl)-1,2,3,6-tetrahydropyridin -4-yl)acetic acid (230 mg, 93% yield) as a white solid.
LCMS: *(ESI)* (M+H)⁺ = 260.2.

### Step E: synthesis of (S)-2-(1-(5-cyclopropylpyrimidin-2-yl)-1,2,3,6 -tetrahydropyridin-4-yl)-N-(2-((6-oxo-5-(trifluoromethyl)-1-((2-(trimethylsilyl)ethoxy) methyl)-1,6-dihydropyridazin-4-yl)amino)propoxy)acetamide

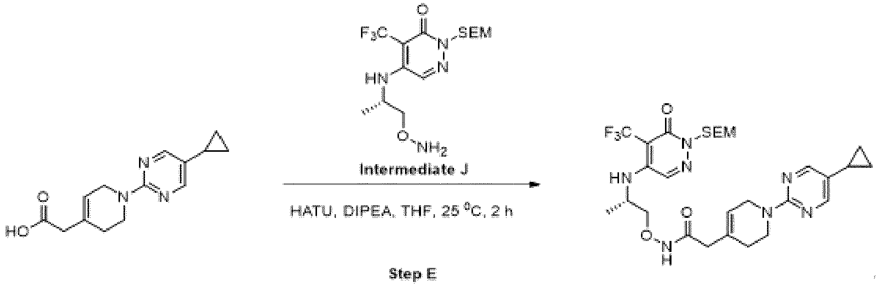

To a solution of 2-(1-(5-cyclopropylpyrimidin-2-yl)-1,2,3,6-tetrahydropyridin-4-yl) acetic acid (240 mg, 0.926 mmol, 1.0 eq.) in THF (4 mL) were added DIPEA (358 mg, 2.779 mmol, 3.0 eq.) and HATU (422 mg, 1.111 mmol, 1.2 eq.) followed by dropwise addition of (S)-5-((1-(aminooxy)propan-2-yl)amino)-4-(trifluoromethyl)-2-((2-(trimethylsilyl)ethoxy)methyl)pyridazin-3(2H)-one (389 mg, 1.019 mmol, 1.1 eq.). The resulting mixture was stirred at 25 °C for 2 h. The mixture was concentrated under vacuum. The residue was purified by silica gel chromatography (Petroleum ether/EtOAc=1/3, V/V) to afford (S)-2-(1-(5-cyclopropylpyrimidin-2-yl)-1,2,3,6-tetrahydropyridin-4-yl)-N-(2-((6-oxo-5-(trifluoromethyl)-1-((2-(trimethylsilyl)ethoxy)m ethyl)-1,6-dihydropyridazin-4-yl)amino)propoxy)acetamide (300 mg, 52% yield) as a white solid.
LCMS: *(ESI)* (M+H)⁺ = 623.9.

### Step F: synthesis of (S)-2-(1-(5-cyclopropylpyrimidin-2-yl)-1,2,3,6-tetrahydropyridin -4-yl)-N-(2-((6-oxo-5-(trifluoromethyl)-1,6-dihydropyridazin-4-yl)amino)propoxy)aceta mide

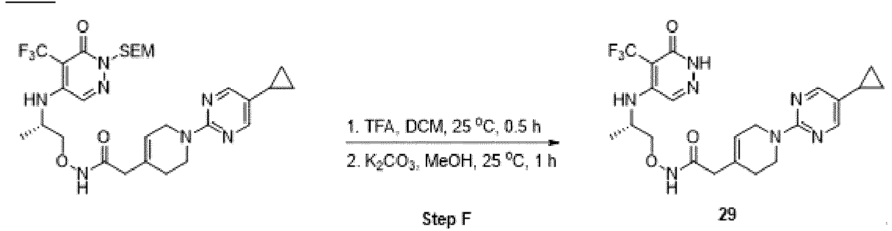

To a solution of (S)-2-(1-(5-cyclopropylpyrimidin-2-yl)-1,2,3,6-tetrahydropyridin -4-yl)-N-(2-((6-oxo-5-(trifluoromethyl)-1-((2-(trimethylsilyl)ethoxy)methyl)-1,6-dihydr opyridazin-4-yl)amino)propoxy)acetamide (300 mg, 0.481 mmol, 1.0 eq.) in DCM (3 mL) was added TFA (0.5 mL). The resulting mixture was stirred at 25 °C for 0.5 h. The mixture was neutralized with saturated NaHCOs aqueous solution (5 mL) and extracted with DCM (3 x 10 mL). The organic layers were combined and dried over Na₂SO₄, concentrated under vacuum. The residue was redissolved in MeOH (5 mL) and K₂CO₃ (166 mg, 1.203 mmol, 2.5 eq.) was added. The mixture was stirred at 25 °C for another 1 h. The mixture was filtered and the filtrate was purified by prep-HPLC to afford (S)-2-(1-(5-cyclopropylpyrimidin-2-yl)-1,2,3,6-tetrahydropyridin-4-yl)-N-(2-((6-oxo-5-(trifluoromethyl)-1,6-dihydropyridazin-4-yl)amino)propoxy)acetamide (100 mg, 42% yield) as a white solid.
LCMS: *(ESI)* (M+H)⁺ = 493.9.
¹H NMR: (400 MHz, DMSO-*d₆*) *δ* 12.46 (s, 1H), 11.17 (s, 1H), 8.16 (s, 2H), 7.89 (s, 1H), 6.60 (s, 1H), 5.60 (s, 1H), 4.20 - 4.18 (m, 1H), 4.08 (s, 2H), 3.93 - 3.82 (m, 2H), 3.79 (t, J = 5.7 Hz, 2H), 2.72 (s, 2H), 2.08 (s, 2H), 1.81 - 1.67 (m, 1H), 1.18 (d, J = 6.6 Hz, 3H), 0.89 - 0.82 (m, 2H), 0.64 - 0.58 (m, 2H).

### Example 30:

### 2-(1-(5-cyclopropylpyrimidin-2-yl)-3-methyl-1,2,3,6-tetrahydropyridin-4-yl)-N-((S)-2-( (6-oxo-5-(trifluoromethyl)-1,6-dihydropyridazin-4-yl)amino)propoxy)acetamide

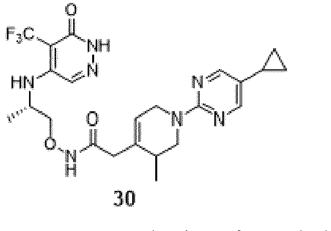

### Step A: Synthesis of methyl 2-(1-(5-cyclopropylpyrimidin-2-yl)-3-methyl-1,2,3,6-tetrahydropyridin-4-yl)acetate

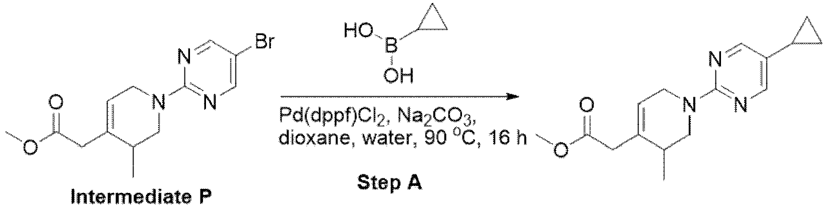

To a solution of methyl 2-(1-(5-bromopyrimidin-2-yl)-3-methyl-1,2,3,6-tetrahydropyridin-4-yl)acetate (450 mg, 1.39 mmol, 1 eq.) in a mixed solvent of dioxane (7 mL) and H₂O (1 mL) were added cyclopropylboronic acid (239 mg, 2.77 mmol, 2.0 eq.), Pd(dppf)Cl2 (102 mg, 0.19 mmol, 0.14 eq.) and Na2CO3 (294 mg, 2.77 mmol, 2.0 eq.). The resulting mixture was stirred at 90 °C for 16 h. The reaction mixture was extracted with EtOAc (3 x 20 mL), washed with water (20 mL). The organic lyaers were combined and washed with brine (10 mL), dried over Na₂SO₄ and concentrated under vacuum. The residue was purified by silica gel chromatography (Petroleum ether/EtOAc= 9/1, V/V) to afford methyl 2-(1-(5-cyclopropylpyrimidin-2-yl)-3-methyl -1,2,3,6-tetrahydropyridin-4-yl)acetate (170 mg, 42.8% yield) as a colorless oil.
LCMS: *(ESI)* (M+H)⁺ = 288.2.
¹H NMR (400 MHz, DMSO-*d₆*) *δ* 8.18 (s, 2H), 5.63 (s, 1H), 4.27 - 4.16 (m, 1H), 3.99 - 3.91 (m, 1H), 3.91 - 3.85 (m, 1H), 3.62 (s, 3H), 3.61 - 3.57 (m, 1H), 3.14 - 3.11 (m, 2H), 2.41 - 2.29 (m, 1H), 1.81 - 1.73 (m, 1H), 0.97 (d, J = 7.0 Hz, 3H), 0.91 - 0.84 (m, 2H), 0.67 - 0.61 (m, 2H).

### Step B: Synthesis of 2-(1-(5-cyclopropylpyrimidin-2-yl)-3-methyl-1,2,3,6-tetrahydropyridin-4-yl)acetic acid

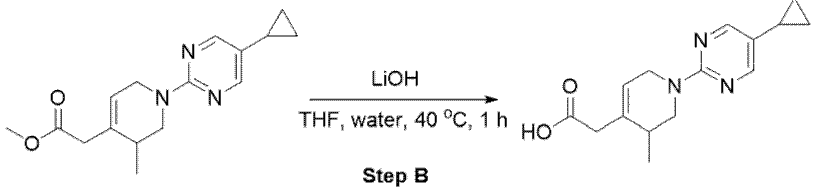

To a solution of methyl 2-(1-(5-cyclopropylpyrimidin-2-yl)-3-methyl-1,2,3,6-tetrahydropyridin-4-yl)acetate (170 mg, 0.593 mmol, 1.0 eq.) in a mixed solvent of THF (4 mL) and water (2 mL) was added LiOH (43 mg, 1.78 mmol, 3.0 eq.). The mixture was stirred at 40 °C for 1 h. The mixture was diluted with water (25 mL) and extracted with EtOAc (3 x 10 mL). The water layer was adjusted to pH 6 with 1M HCl. The mixture was extracted with EtOAc (3 x 15 mL), dried over Na₂SO₄ and concentrated under vacuum to afford 2-(1-(5-cyclopropylpyrimidin-2-yl)-3-methyl-1,2,3,6 -tetrahydropyridin-4-yl)acetic acid (140 mg, 86.9% yield) as a colorless oil.
LCMS: *(ESI)* (M+H)⁺ = 274.2.

### Step C: synthesis of 2-(1-(5-cyclopropylpyrimidin-2-yl)-3-methyl-1,2,3,6 -tetrahydropyridin-4-yl)-N-((S)-2-((6-oxo-5-(trifluoromethyl)-1-((2-(trimethylsilyl)etho xy)methyl)-1,6-dihydropyridazin-4-yl)amino)propoxy)acetamide

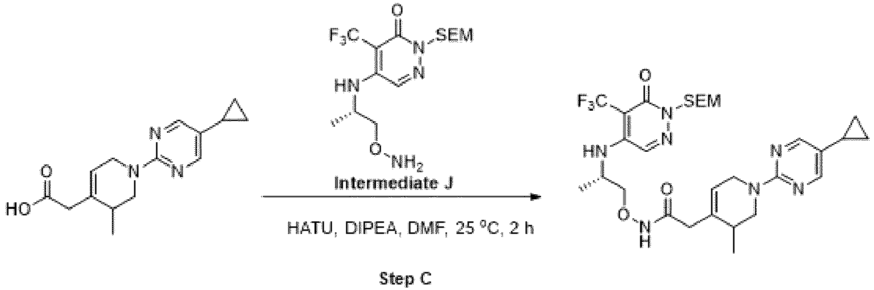

To a solution of 2-(1-(5-cyclopropylpyrimidin-2-yl)-3-methyl -1,2,3,6-tetrahydropyridin-4-yl)acetic acid (140 mg, 0.52 mmol, 1 eq.) in DMF (5 mL) were added DIPEA (135 mg, 1.04 mmol, 2.0 eq.) and HATU (296 mg, 0.78 mmol, 1.5 eq.) followed by dropwise addition of (S)-5-((1-(aminooxy)propan-2-yl)amino)-4-(trifluoromethyl)-2-((2-(trimethylsilyl)ethox y)methyl)pyridazin-3(2H)-one (140 mg, 0.52 mmol, 1 eq.).The resulting mixture was stirred at 25 °C for 2 h. The reaction mixture was quenched with water (30 mL) and extracted with EtOAc (3 x 20 mL). The organic layers were combined and washed with brine (10 mL), dried over Na₂SO₄ and concentrated under vacuum. The residue was purified by silica gel chromatography (Petroleum ether/EtOAc= 35/65, V/V) to afford 2-(1-(5-cyclopropylpyrimidin-2-yl)-3-methyl-1,2,3,6-tetrahydropyridin-4-yl)-N-((S)-2-( (6-oxo-5-(trifluoromethyl)-1-((2-(trimethylsilyl)ethoxy)methyl)-1,6-dihydropyridazin-4 -yl)amino)propoxy)acetamide (340 mg, 100% yield) as a white solid.
LCMS: *(ESI)* (M+H)⁺ = 638.4.

### Step D: synthesis of 2-(1-(5-cyclopropylpyrimidin-2-yl)-3-methyl-1,2,3,6 -tetrahydropyridin-4-yl)-N-((S)-2-((6-oxo-5-(trifluoromethyl)-1,6-dihydropyridazin-4-yl )amino)propoxy)acetamide

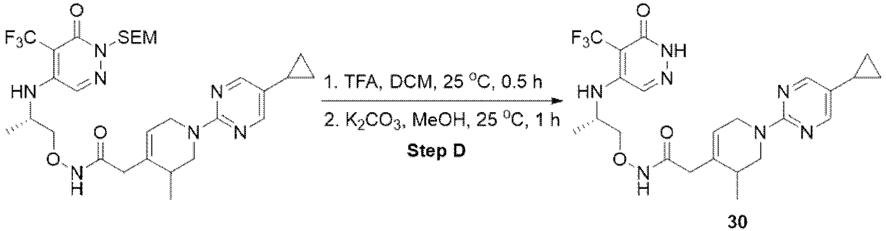

To a solution of 2-(1-(5-cyclopropylpyrimidin-2-yl)-3-methyl-1,2,3,6 -tetrahydropyridin-4-yl)-N-((S)-2-((6-oxo-5-(trifluoromethyl)-1-((2-(trimethylsilyl)etho xy)methyl)-1,6-dihydropyridazin-4-yl)amino)propoxy)acetamide (340 mg, 0.54 mmol, 1.0 eq.) in DCM (3 mL) was added TFA (0.5 mL). The resulting mixture was stirred at 25 °C for 0.5 h. The mixture was neutralized with saturated NaHCO₃ aqueous solution (5 mL) and extracted with DCM (3 x 10 mL). The organic layers were combined and dried over Na₂SO₄, concentrated under vacuum. The residue was redissolved in MeOH (3 mL) and K₂CO₃ (186 mg, 1.35 mmol, 3.0 eq.) was added. The mixture was stirred at 25 °C for another 1 h. The reaction mixture was filtered and the filtrate was purified by pre-HPLC to afford 2-(1-(5-cyclopropylpyrimidin-2-yl)-3-methyl-1,2,3,6 -tetrahydropyridin-4-yl)-N-((S)-2-((6-oxo-5-(trifluoromethyl)-1,6-dihydropyridazin-4-yl )amino)propoxy)acetamide (15.7 mg, 5.7% yield) as a white solid.
LCMS: *(ESI)* (M+H)⁺ = 508.2.
¹H NMR (400 MHz, DMSO-*d₆*) *δ* 12.46 (s, 1H), 11.22 (s, 1H), 8.16 (s, 2H), 7.89 (s, 1H), 6.65 - 6.53 (m, 1H), 5.57 (s, 1H), 4.20 - 4.25 (m, 2H), 3.99 - 3.76 (m, 4H), 3.48 (dd, J = 12.7, 4.0 Hz, 1H), 2.83 - 2.68 (m, 2H), 2.30 (s, 1H), 1.81 - 1.68 (m, 1H), 1.19 (d, J = 6.5 Hz, 3H), 0.95 (d, J = 6.9 Hz, 3H), 0.91 - 0.79 (m, 2H), 0.68 - 0.56 (m, 2H).

### Example 31:

### (S)-2-(1-(5-cyclopropylpyrimidin-2-yl)-5-methyl-1,2,3,6-tetrahydropyridin-4-yl)-N-(2-( (6-oxo-5-(tiifluoromethyl)-1,6-dihydropyridazin-4-yl)amino)propoxy)acetamide

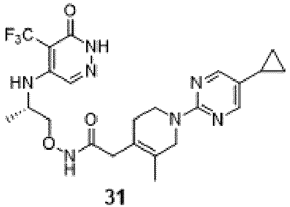

### Step A: synthesis of (S)-2-(1-(5-cyclopropylpyrimidin-2-yl)-5-methyl-1,2, 3,6-tetrahydropyridin-4-yl)-N-(2-((6-oxo-5-(trifluoromethyl)-1,6-dihydropyridazin-4-yl) amino)propoxy)acetamide

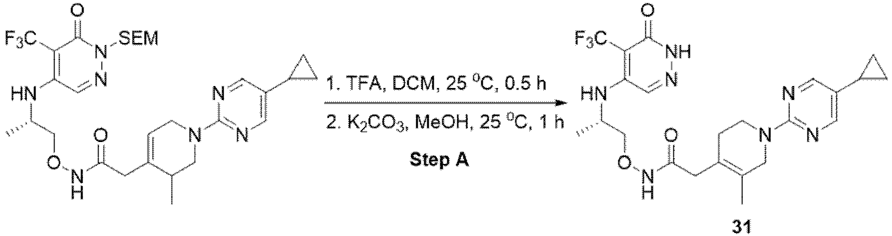

To a solution of 2-(1-(5-cyclopropylpyrimidin-2-yl)-3-methyl-1,2,3,6-tetrahydropyridin-4-yl)-N-((S)-2-((6-oxo-5-(trifluoromethyl)-1-((2-(trimethylsilyl) ethoxy)methyl)-1,6-dihydropyridazin-4-yl)amino)propoxy)acetamide (340 mg, 0.54 mmol, 1.0 eq.) in DCM (3 mL) was added TFA (0.5 mL). The resulting mixture was stirred at 25 °C for 0.5 h. The mixture was neutralized with saturated NaHCO₃ aqueous solution (5 mL) and extracted with DCM (3 x 10 mL). The organic layers were combined and dried over Na₂SO₄, concentrated under vacuum. The residue was redissolved in MeOH (3 mL) and K₂CO₃ (186 mg, 1.35 mmol, 3.0 eq.) was added. The mixture was stirred at 25 °C for another 1 h. The reaction mixture was filtered and the filtrate was purified by pre-HPLC to afford (S)-2-(1-(5-cyclopropylpyrimidin-2-yl)-5-methyl-1,2,3,6-tetrahydropyridin-4-yl)-N-(2-( (6-oxo-5-(trifluoromethyl)-1,6-dihydropyridazin-4-yl)amino)propoxy)acetamide (17 mg, 12.6% yield) as a white solid.
LCMS: *(ESI)* (M+H)⁺ = 508.2.
¹H NMR (400 MHz, DMSO-*d₆*) *δ* 12.45 (s, 1H), 11.16 (s, 1H), 8.16 (s, 2H), 7.89 (s, 1H), 6.67 - 6.48 (m, 1H), 4.20 - 4.16 (m, 1H), 3.97 (s, 2H), 3.92 - 3.86 (m, 1H), 3.85 - 3.80 (m, 1H), 3.76 (t, J = 5.7 Hz, 2H), 2.79 (s, 2H), 2.08 (d, J = 5.5 Hz, 2H), 1.81 - 1.70 (m, 1H), 1.67 (s, 3H), 1.18 (d, J = 6.6 Hz, 3H), 0.89 - 0.82 (m, 2H), 0.64 - 0.57 (m, 2H).

### Example 32:

### (S)-N-(2-((6-oxo-5-(trifluoromethyl)-1,6-dihydropyridazin-4-yl)amino)propoxy)-2-(1-( 5-(trifluoromethyl)pyridin-2-yl)piperidin-4-yl)acetamide

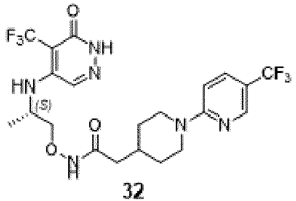

### 32.1) Synthesis of intermediate Q

### Step A: synthesis of 4,5-dibromo-2-(4-methoxybenzyl)pyridazin-3(2H)-one

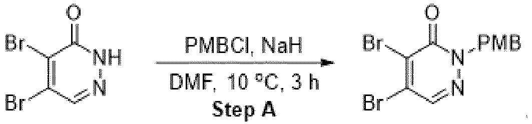

To a mixture of 4,5-dibromopyridazin-3(2H)-one (10.16 g, 40.00 mmol, 1.0 eq.) in DMF (100 mL) was added NaH (60%, 2.40 g, 60.00 mmol, 1.5 eq.) at 0 °C under N₂ atmosphere in ice-water bath. The mixture was stirred at 0 °C for 0.5 h. 4-methoxybenzylchloride (6.26 g, 40.00 mmol, 1.0 eq.) was added to the mixture. The mixture was stirred at 10 °C for another 3 h. The mixture was quenched with cold water (500 mL) and extracted with EtOAc (3 x 400 mL). The organic layers were combined and washed with brine (2 x 400 mL), dried over Na₂SO₄ and concentrated under vacuum. The crude solid was washed with MeOH (2 x 20 mL) to afford 4,5-dibromo-2-(4-methoxybenzyl)pyridazin-3(2H)-one (11.27 g, 75.3%) as a light brown solid.
LCMS: *(ESI)* (M+H)⁺ = 375.0.
¹H NMR: (400 MHz, DMSO-*d₆*) *δ* 8.18 (s, 1H), 7.28 (d, *J* = 8.4 Hz, 2H), 6.91 (d, *J* = 8.4 Hz, 2H), 5.20 (s, 2H), 3.74 (s, 3H).

### Step B: synthesis of 4-bromo-5-methoxy-2-(4-methoxybenzyl)pyridazin-3(2H)-one

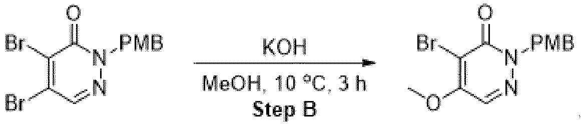

To a mixture of 4,5-dibromo-2-(4-methoxybenzyl)pyridazin-3(2H)-one (6.18 g, 16.52 mmol, 1.0 eq.) in MeOH (52 mL) was added KOH (2.78 g, 49.57 mmol, 3.0 eq.). The mixture was stirred at 10 °C for 3 h. The mixture was concentrated and the mixture was diluted with water (80 mL). The mixture was extracted with DCM (3 x 100 mL). The organic layers were combined and washed with brine (80 mL), dried over Na₂SO₄ and concentrated under vacuum. The crude solid was washed with MeOH (10 mL) to afford 4-bromo-5-methoxy-2-(4-methoxybenzyl)pyridazin-3(2H)-one (4.44 g, 82.6%) as a yellow solid.
LCMS: *(ESI)* (M+H)⁺ = 325.0.
¹H NMR: (400 MHz, DMSO-*d₆*) *δ* 8.15 (s, 1H), 7.25 (d, *J=* 8.4 Hz, 2H), 6.89 (d, *J=* 8.8 Hz, 2H), 5.21 (s, 2H), 4.06 (s, 3H), 3.72 (s, 3H).

### Step C: synthesis of 5-methoxy-2-(4-methoxybenzyl)-4-(trifluoromethyl)pyridazin -3(2H)-one

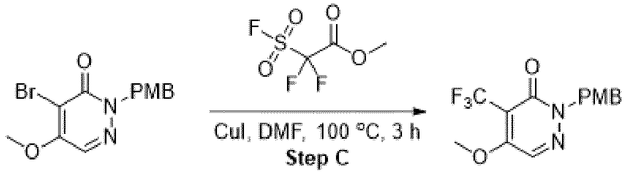

To a mixture of 4-bromo-5-methoxy-2-(4-methoxybenzyl)pyridazin-3(2H)-one (8.08 g, 24.85 mmol, 1.0 eq.) and CuI (2.37 g, 12.43 mmol, 0.5 eq.) in DMF (42 mL) was added dropwise methyl 2,2-difluoro-2-(fluorosulfonyl)acetate (19.10 g, 99.40 mmol, 4.0 eq.) under N₂ atmosphere. The mixture was stirred at 100 °C for 3 h. The mixture was cooled to room temperature and quenched with water (150 mL). The mixture was extracted with DCM (3 x 150 mL). The organic layers were combined and dried over Na₂SO₄, concentrated under vacuum. The residue was purified by silica gel chromatography (EtOAc/Petroleum ether= 2/3; V/V) to afford 5-methoxy-2-(4-methoxybenzyl)-4-(trifluoromethyl)pyridazin-3(2H)-one (5.30 g, 67.9%) as a light yellow solid.
LCMS: *(ESI)* (M+H)⁺ = 314.8.
¹H NMR: (400 MHz, DMSO-*d₆*) *δ* 8.34 (s, 1H), 7.28 (d, *J* = 8.4 Hz, 2H), 6.92 (d, *J* = 8.8 Hz, 2H), 5.19 (s, 2H), 4.11 (s, 3H), 3.75 (s, 3H).

### Step D: synthesis of 5-hydroxy-2-(4-methoxybenzyl)-4-(trifluoromethyl)pyridazin -3(2H)-one

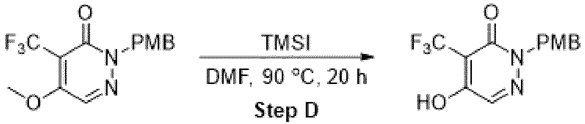

To a solution of 5-methoxy-2-(4-methoxybenzyl)-4-(trifluoromethyl)pyridazin -3(2H)-one (3.14 g, 10.00 mmol, 1.0 eq.) in DMF (16 mL) was added dropwise Iodotrimethylsilane (2.60 g, 13.00 mmol, 1.3 eq.). The mixture was stirred at 90 °C for 20 h. The mixture was cooled to room temperature and quenched with water (70 mL), extracted with DCM (2 x 80 mL). The organic layers were combined and dried over Na₂SO₄, concentrated under vacuum. The residue was purified by silica gel chromatography (EtOAc/Petroleum ether= 3/2; V/V) to afford 5-hydroxy-2-(4-methoxybenzyl)-4-(trifluoromethyl)pyridazin-3(2H)-one (2.82 g, 93.9%) as a light brown solid.
LCMS: *(ESI)* (M+H)⁺ = 300.8.
¹H NMR: (400 MHz, DMSO-*d₆*) *δ* 7.79 (s, 1H), 7.24 (d, *J* = 8.4 Hz, 2H), 6.90 (d, *J* = 8.4 Hz, 2H), 5.11 (s, 2H), 3.73 (s, 3H).

### Step E: synthesis of 5-chloro-2-(4-methoxybenzyl)-4-(trifluoromethyl)pyridazin -3(2H)-one

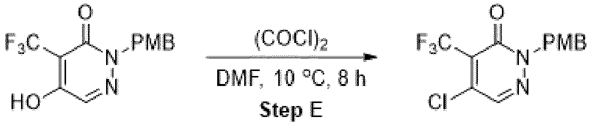

To a solution of 5-hydroxy-2-(4-methoxybenzyl)-4-(trifluoromethyl)pyridazin -3(2H)-one (2.49 g, 8.29 mmol, 1.0 eq.) in DMF (13 mL) was added dropwise oxalyl chloride (2.11 g, 16.59 mmol, 2.0 eq.) at 0 °C in ice-water bath. The mixture was stirred at 10 °C for 8 h. The mixture was quenched with water (80 mL) and extracted with EtOAc (2 x 80 mL). The organic layers were combined and washed with brine (2 x 80 mL), dried over Na₂SO₄ and concentrated under vacuum. The residue was purified by silica gel chromatography (EtOAc/Petroleum ether= 1/5; V/V) to afford 5-chloro-2-(4-methoxybenzyl)-4-(trifluoromethyl)pyridazin-3(2H)-one (2.51 g, 95.0%) as a light yellow solid.
LCMS: *(ESI)* (M+Na)⁺ = 341.0.
¹H NMR: (400 MHz, DMSO-*d₆*) *δ* 8.23 (s, 1H), 7.29 (d, *J* = 8.4 Hz, 2H), 6.91 (d, *J* = 8.4 Hz, 2H), 5.20 (s, 2H), 3.73 (s, 3H).

### Step F: Synthesis of 5-chloro-4-(trifluoromethyl)pyridazin-3(2H)-one (Intermediate Q)

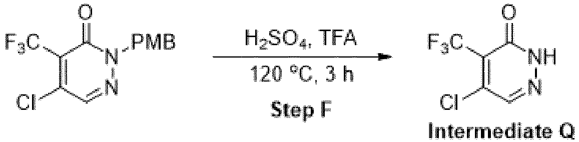

To a solution of 5-chloro-2-(4-methoxybenzyl)-4-(trifluoromethyl)pyridazin -3(2H)-one (2.00 g, 6.28 mmol, 1.0 eq.) in TFA (32 mL) was added H2SO4 (5.3 mL). The mixture was stirred at 120 °C for 3 h. The mixture was cooled to room temperature and quenched with water (100 mL). The mixture was neutralized to pH 7 with saturated NaHCOs aqueous solution and extracted with EtOAc (3 x 70 mL). The organic layers were combined and dried over Na₂SO₄, concentrated under vacuum to afford 5-chloro-4-(trifluoromethyl)pyridazin-3(2H)-one (1.05 g, 84.3%) as a white solid. LCMS: *(ESI)* (M+H)⁺ = 199.0.

### 32.2) Synthesis of intermediate R

### Step A: synthesis of (S)-tert-butyl (1-((1,3-dioxoisoindolin-2-yl)oxy)propan-2-yl) carbamate

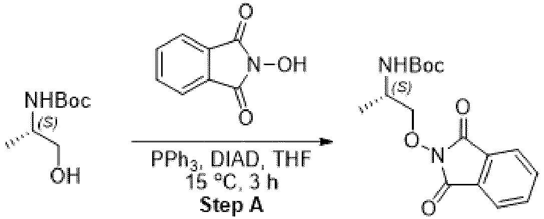

To a mixture of 2-hydroxyisoindole-1,3-dione (4.90 g, 30.04 mmol, 1.0 eq.), N-Boc-L-alaninol (5.32 g, 30.34 mmol, 1.01 eq.) and PPh3 (8.82 g, 33.64 mmol, 1.12 eq.) in THF (100 mL) was added DIAD (6.68 g, 33.04 mmol, 1.1 eq.) dropwise at 0 °C under N₂ atmosphere. The reaction mixture was stirred at 15 °C for 3 h. The reaction mixture was quenched with water (50 mL) and extracted with EtOAc (3 x 100 mL). The organic layers were combined and dried over Na₂SO₄, concentrated under vacuum. The residue was purified by reversed phase chromatography (CH₃CN/H₂O= 2/3; V/V) to afford (S)-tert-butyl (1-((1,3-dioxoisoindolin-2-yl)oxy)propan-2-yl)carbamate (7.80 g, 81.1%) as a white solid.
LCMS: *(ESI)* (M+H-100)⁺ = 221.2.
¹H NMR: (400 MHz, CDCl₃) *δ* 7.87 - 7.81 (m, 2H), 7.79 - 7.73 (m, 2H), 5.15 (br s, 1H), 4.25 (dd, *J* = 9.6, 4.4 Hz, 1H), 4.20 - 4.11 (m, 1H), 4.02 - 3.89 (m, 1H), 1.42 (s, 9H), 1.36 (d, *J* = 6.8 Hz, 3H).

### Step B: synthesis of (S)-tert-butyl (1-(aminooxy)propan-2-yl)carbamate (Intermediate R)

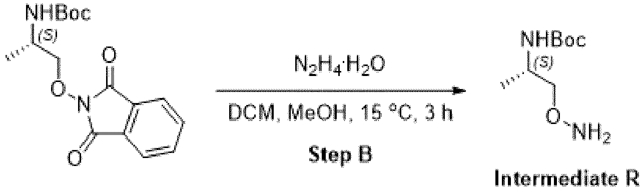

To mixture of (S)-tert-butyl (1-((1,3-dioxoisoindolin-2-yl)oxy)propan-2-yl) carbamate (1.00 g, 3.12 mmol) in DCM (20 mL) and MeOH (6 mL) was added hydrazine hydrate (0.16 g, 3.12 mmol). The mixture was stirred at 15 °C for 3 h. The mixture was concentrated under vacuum. The residue was treated with DCM (40 mL). The mixture was filtered and the filtrate was concentrated to afford (S)-tert-butyl (1-(aminooxy)propan-2-yl)carbamate (650 mg, 96.3%) as a white solid.

### 32.3) Synthesis of compound 32

### Step A: synthesis of methyl 2-(1-(5-(trifluoromethyl)pyridin-2-yl)piperidin-4-yl)acetate

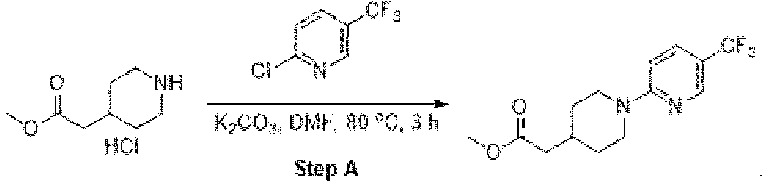

To a mixture of methyl 4-piperidineacetate hydrochloride (1.00 g, 5.16 mmol, 1.0 eq.) and K₂CO₃ (2.14 g, 15.49 mmol, 3.0 eq.) in DMF (20 mL) was added 2-chloro-5-(trifluoromethyl)pyridine (1.03 g, 5.68 mmol, 1.1 eq.). The mixture was stirred at 80 °C for 3 h. The mixture was cooled to room temperature and then poured into water (70 ml), extracted with EtOAc (3 x 50 mL). The organic layers were combined and washed with water (2 x 50 mL), dried over anhydrous Na₂SO₄ and concentrated under reduced pressure. The crude product was purified by flash column chromatography (Petroleum ether/EtOAc= 5/1; V/V) to afford methyl 2-(1-(5-(trifluoromethyl)pyridin-2-yl)piperidin-4-yl)acetate (0.55 g, 35.2%) as a yellow solid.
LCMS: *(ESI)* (M+H)⁺ = 303.2.
¹H NMR: (400 MHz, CDCl₃) *δ* 8.37 (d, *J* = 0.8 Hz, 1H), 7.59 (dd, *J* = 9.2, 2.4 Hz, 1H), 6.63 (d, *J* = 9.2 Hz, 1H), 4.44 - 4.35 (m, 2H), 3.69 (s, 3H), 2.93 (td, *J* = 13.2, 2.4 Hz, 2H), 2.28 (d, *J*= 7.2 Hz, 2H), 2.16 - 2.01 (m, 1H), 1.89 - 1.76 (m, 2H), 1.34 - 1.19 (m, 2H).

### Step B: synthesis of 2-(1-(5-(trifluoromethyl)pyridin-2-yl)piperidin-4-yl)acetic acid

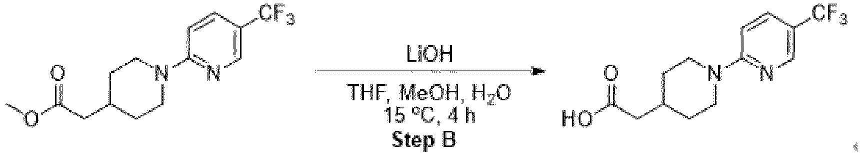

To a solution of methyl 2-(1-(5-(trifluoromethyl)pyridin-2-yl)piperidin-4-yl)acetate (300 mg, 0.99 mmol, 1.0 eq.) in a mixed solvent of THF (3 mL), MeOH (1 mL) and H₂O (1 mL) was added LiOH (48 mg, 1.99 mmol, 2.0 eq.). The mixture was stirred at 15 °C for 4 h. The mixture was neutralized to pH 7 with 2M HCl and concentrated under vacuum. The crude product was purified by flash column chromatography (DCM/MeOH= 9/1; V/V) to afford 2-(1-(5-(trifluoromethyl)pyridin-2-yl)piperidin-4-yl) acetic acid (200 mg, 69.9%) as a white solid.
LCMS: *(ESI)* (M+H)⁺ = 289.2.

### Step C: synthesis of (S)-tert-butyl (1-((2-(1-(5-(trifluoromethyl)pyridin-2-yl) piperidin-4-yl)acetamido)oxy)propan-2-yl)carbamate

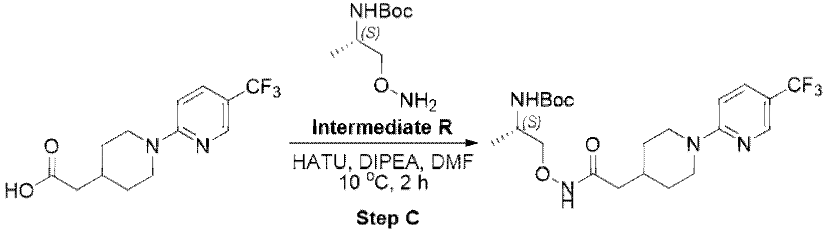

To a solution of 2-(1-(5-(trifluoromethyl)pyridin-2-yl)piperidin-4-yl)acetic acid (150 mg, 0.52 mmol, 1.0 eq.) and (S)-tert-butyl (1-(aminooxy)propan-2-yl)carbamate (109 mg, 0.57 mmol, 1.1 eq.) in DMF (10 mL) were added DIPEA (168 mg, 1.30 mmol, 2.5 eq.) and HATU (297 mg, 0.78 mmol, 1.5 eq.). The mixture was stirred at 10 °C for 2 h. The mixture was quenched with water (50 mL) and extracted with EtOAc (2 x 60 mL). The organic layers were combined and washed with brine (2 x 50 mL), dried over Na₂SO₄ and concentrated under vacuum. The residue was purified by silica gel chromatography (Petroleum ether/EtOAc= 3/7; V/V) to afford (S)-tert-butyl (1-((2-(1-(5-(trifluoromethyl)pyridin-2-yl)piperidin-4-yl)acetamido)oxy)propan-2-yl)car bamate (220 mg, 91.8%) as a white solid.
LCMS: *(ESI)* (M+H-100)⁺ = 461.2.

### Step D: synthesis of (S)-N-(2-aminopropoxy)-2-(1-(5-(trifluoromethyl)pyridin-2-yl) piperidin-4-yl)acetamide

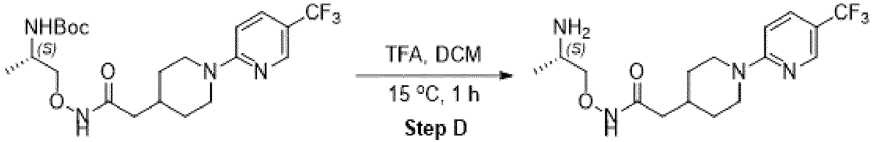

To a solution of (S)-tert-butyl (1-((2-(1-(5-(trifluoromethyl)pyridin-2-yl) piperidin-4-yl)acetamido)oxy)propan-2-yl)carbamate (100 mg, 0.22 mmol, 1.0 eq.) in DCM (3 mL) was added TFA (1 mL). The mixture was stirred at 15 °C for 1 h. The excess TFA was neutralized with saturated aqueous NaHCOs solution (20 mL) and the mixture was extracted with a mixed solution of DCM and MeOH (10/1; V/V, 6 x 40 mL). The organic layers were combined and dried over Na₂SO₄, concentrated under vacuum to afford (S)-N-(2-aminopropoxy)-2-(1-(5-(trifluoromethyl)pyridin-2-yl) piperidin-4-yl)acetamide (75 mg, 95.8%) as a yellow solid.
LCMS: *(ESI)* (M+H)⁺ = 361.2.

### Step E: synthesis of (S)-N-(2-((6-oxo-5-(trifluoromethyl)-1,6-dihydropyridazin-4-yl) amino)propoxy)-2-(1-(5-(trifluoromethyl)pyridin-2-yl)piperidin-4-yl)acetamide

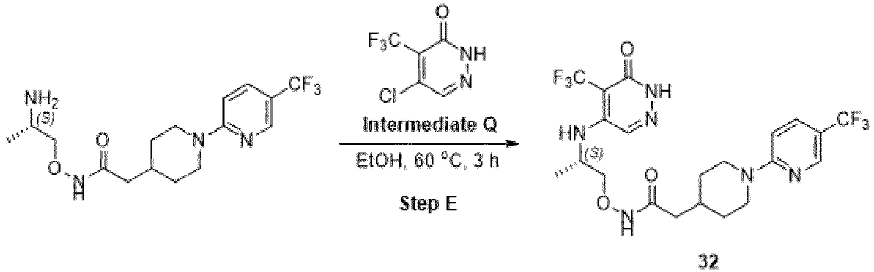

The mixture of (S)-N-(2-aminopropoxy)-2-(1-(5-(trifluoromethyl)pyridin-2-yl) piperidin-4-yl)acetamide (75 mg, 0.21 mmol, 1.0 eq.) in EtOH (2 mL) was added 5-chloro-4-(trifluoromethyl)pyridazin-3(2H)-one (41 mg, 0.21 mmol, 1.0 eq.). The mixture was stirred at 60 °C for 3 h. The mixture was cooled to room temperature and diluted with water (20 mL). The mixture was extracted with DCM (2 x 20 mL). The organic layers were combined and dried over Na₂SO₄, concentrated under vacuum. The residue was purified by prep-HPLC to afford (S)-N-(2-((6-oxo-5-(trifluoromethyl)-1,6-dihydropyridazin-4-yl)amino)propoxy)-2-(1-( 5-(trifluoromethyl)pyridin-2-yl)piperidin-4-yl)acetamide (10.57 mg, 9.7%) as a white solid.
LCMS: *(ESI)* (M+H)⁺ = 523.2.
¹H NMR: (400 MHz, DMSO-*d₆*) *δ* 12.51 (s, 1H), 11.15 (s, 1H), 8.39 (s, 1H), 7.92 (s, 1H), 7.76 (dd, *J* = 9.2, 2.4 Hz, 1H), 6.95 (d, *J* = 9.2 Hz, 1H), 6.63 (s, 1H), 4.39 (d, *J* = 13.6 Hz, 2H), 4.27 - 4.17 (m, 1H), 3.96 - 3.83 (m, 2H), 2.93 (t, *J* = 12.4 Hz, 2H), 2.06 - 1.97 (m, 1H), 1.93 (d, *J* = 6.8 Hz, 2H), 1.70 (d, *J* = 11.6 Hz, 2H), 1.21 (d, *J* = 6.4 Hz, 3H), 1.17 - 1.06 (m, 2H).

### Example 33:

### (S)-2-(1-(5-cyanopyrimidin-2-yl)piperidin-4-yl)-N-(2-((6-oxo-5-(trifluoromethyl)-1,6-di hydropyridazin-4-yl)amino)propoxy)acetamide

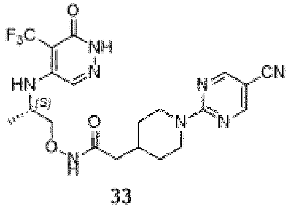

### Step A: synthesis of methyl 2-(1-(5-cyanopyrimidin-2-yl)piperidin-4-yl)acetate

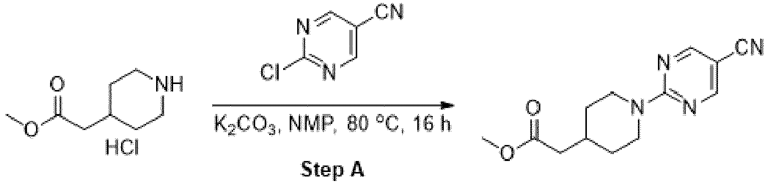

To a solution of 2-chloropyrimidine-5-carbonitrile (698 mg, 5.00 mmol, 1.0 eq.) and methyl 2-(piperidin-4-yl)acetate hydrochloride (1.02 g, 5.25 mmol, 1.05 eq.) in NMP (10 mL) was added K₂CO₃ (2.07 g, 15.00 mmol, 3.0 eq.). The mixture was stirred at 80 °C for 16 h. The mixture was cooled to room temperature and diluted with water (100 mL). The mixture was extracted with EtOAc (2 x 80 mL). The organic layers were combined and washed with brine (2 x 100 mL), dried over Na₂SO₄ and concentrated under vacuum. The residue was purified by silica gel chromatography (Petroleum ether/EtOAc= 3/1; V/V) to afford methyl 2-(1-(5-cyanopyrimidin-2-yl) piperidin-4-yl)acetate (1.04 g, 79.9%) as a light yellow solid.
LCMS: *(ESI)* (M+H)⁺ = 261.1.
¹H NMR: (400 MHz, CDCl₃) *δ* 8.46 (s, 2H), 4.88 - 4.79 (m, 2H), 3.69 (s, 3H), 2.97 (td, *J* = 13.2, 2.8 Hz, 2H), 2.29 (d, *J* = 6.8 Hz, 2H), 2.18 - 2.06 (m, 1H), 1.90 - 1.82 (m, 2H), 1.29 - 1.17 (m, 2H).

### Step B: synthesis of 2-(1-(5-cyanopyrimidin-2-yl)piperidin-4-yl)acetic acid

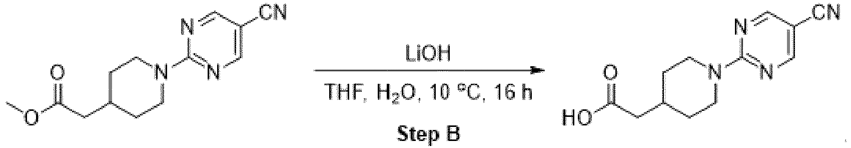

To a mixture of methyl 2-(1-(5-cyanopyrimidin-2-yl)piperidin-4-yl)acetate (390 mg, 1.50 mmol, 1.0 eq.) in THF (6 mL) and H₂O (3 mL) was added LiOH (126 mg, 3.00 mmol, 2.0 eq.). The mixture was stirred at 10 °C for 16 h. The mixture was diluted with water (10 mL) and neutralized to pH 7 with 1M HCl. The mixture was extracted with EtOAc (2 x 60 mL). The organic layers were combined and washed with brine (40 mL), dried over Na₂SO₄ and concentrated under vacuum to afford 2-(1-(5-cyanopyrimidin-2-yl)piperidin-4-yl)acetic acid (365 mg, 98.8%) as a white solid.
LCMS: *(ESI)* (M+H)⁺ = 247.2.

### Step C: synthesis of (S)-tert-butyl (1-((2-(1-(5-cyanopyrimidin-2-yl)piperidin-4-yl) acetamido)oxy)propan-2-yl)carbamate

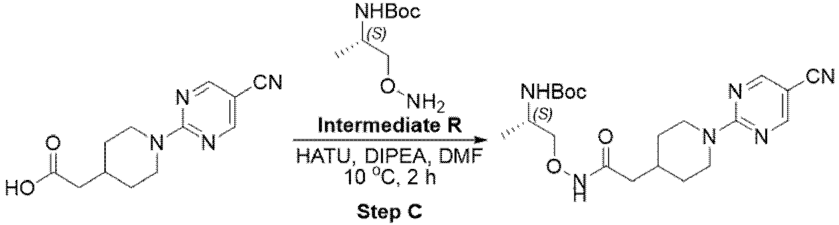

To a solution of 2-(1-(5-cyanopyrimidin-2-yl)piperidin-4-yl)acetic acid (246 mg, 1.00 mmol, 1.0 eq.) in DMF (5 mL) were added DIPEA (388 mg, 3.00 mmol, 3.0 eq.), HATU (456 mg, 1.20 mmol, 1.2 eq.) followed by (S)-tert-butyl (1-(aminooxy)propan-2-yl)carbamate (190 mg, 1.00 mmol, 1.0 eq.). The mixture was stirred at 10 °C for 2 h. The mixture was quenched with water (80 mL) and extracted with EtOAc (2 x 80 mL). The organic layers were combined and washed with brine (2 x 80 mL), dried over Na₂SO₄ and concentrated under vacuum. The residue was purified by silica gel chromatography (Petroleum ether/EtOAc= 3/7; V/V) to afford (S)-tert-butyl (1-((2-(1-(5-cyanopyrimidin-2-yl)piperidin-4-yl)acetamido)oxy)propan-2-yl)carbamate (295 mg, 70.5%) as a white solid.
LCMS: *(ESI)* (M+H-100)⁺ = 319.2.
¹H NMR: (400 MHz, CDCl₃) *δ* 9.68 (s, 1H), 8.50 (s, 2H), 4.93 - 4.83 (m, 2H), 4.62 (d, *J* = 8.8 Hz, 1H), 4.10 - 4.00 (m, 1H), 3.98 - 3.90 (m, 1H), 3.50 (t, *J* = 10.2 Hz, 1H), 3.05 - 2.95 (m, 2H), 2.35 - 2.17 (m, 1H), 2.14 - 2.00 (m, 2H), 1.97 - 1.84 (m, 2H), 1.49 (s, 9H), 1.34 - 1.23 (m, 2H), 1.17 (d, *J* = 6.8 Hz, 3H).

### Step D: synthesis of (S)-N-(2-aminopropoxy)-2-(1-(5-cyanopyrimidin-2-yl)piperidin -4-yl)acetamide

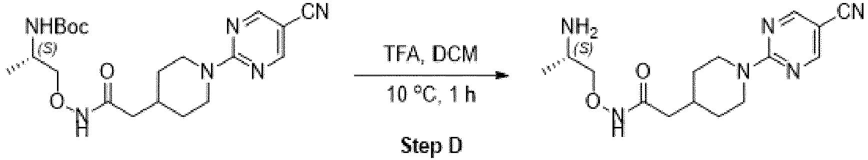

To a solution of (S)-tert-butyl (1-((2-(1-(5-cyanopyrimidin-2-yl)piperidin-4-yl) acetamido)oxy)propan-2-yl)carbamate (126 mg, 0.30 mmol, 1.0 eq.) in DCM (3 mL) was added TFA (1 mL). The mixture was stirred at 10 °C for 1 h. The excess TFA was neutralized with saturated aqueous NaHCOs aqueous solution (20 mL) and the mixture was extracted with a mixed solution of DCM and MeOH (10/1; V/V, 6 x 60 mL). The organic layers were combined and dried over Na₂SO₄, concentrated under vacuum to afford (S)-N-(2-aminopropoxy)-2-(1-(5-cyanopyrimidin-2-yl)piperidin-4-yl)acetamide (96 mg, 99.5%) as a light yellow oil.
LCMS: *(ESI)* (M+H)⁺ = 319.2.

### Step E: synthesis of (S)-2-(1-(5-cyanopyrimidin-2-yl)piperidin-4-yl)-N-(2-((6-oxo-5-(trifluoromethyl)-1,6-dihydropyridazin-4-yl)amino)propoxy)acetamide

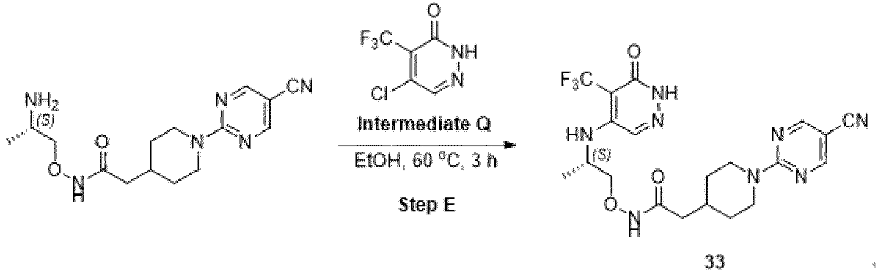

To a solution of (S)-N-(2-aminopropoxy)-2-(1-(5-cyanopyrimidin-2-yl) piperidin-4-yl)acetamide (96 mg, 0.30 mmol, 1.0 eq.) in EtOH (3 mL) was added 5-chloro-4-(trifluoromethyl)pyridazin-3(2H)-one (60 mg, 0.30 mmol, 1.0 eq.). The mixture was heated to 60 °C and stirred for 3 h. The mixture was cooled to room temperature and diluted with water (20 mL). The mixture was extracted with DCM (2 x 20 mL). The organic layers were combined and dried over Na₂SO₄, concentrated under vacuum. The residue was purified by prep-HPLC to afford (S)-2-(1-(5-cyanopyrimidin-2-yl)piperidin-4-yl)-N-(2-((6-oxo-5-(trifluoromethyl)-1,6-di hydropyridazin-4-yl)amino)propoxy)acetamide (12.22 mg, 8.46%) as a white solid.
LCMS: *(ESI)* (M+H)⁺ = 481.2.
¹H NMR: (400 MHz, DMSO-*d₆*) *δ* 12.49 (s, 1H), 11.14 (s, 1H), 8.72 (s, 2H), 7.91 (s, 1H), 6.66 - 6.57 (m, 1H), 4.67 (d, *J* = 13.2 Hz, 2H), 4.26 - 4.15 (m, 1H), 3.95 - 3.81 (m, 2H), 3.01 (t, *J* = 12.4 Hz, 2H), 2.07 - 1.97 (m, 1H), 1.93 (d, *J* = 7.2 Hz, 2H), 1.77 - 1.68 (m, 2H), 1.19 (d, *J* = 6.4 Hz, 3H), 1.15 - 1.04 (m, 2H).

### Example 34:

### (S)-2-(1-(5-cyanopyridin-2-yl)piperidin-4-yl)-N-(2-((6-oxo-5-(trifluoromethyl)-1,6-dihy dropyridazin-4-yl)amino)propoxy)acetamide

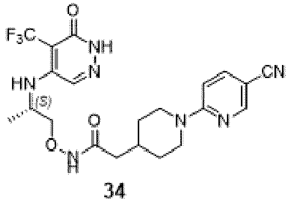

### Step A: synthesis of methyl 2-(1-(5-cyanopyridin-2-yl)piperidin-4-yl)acetate

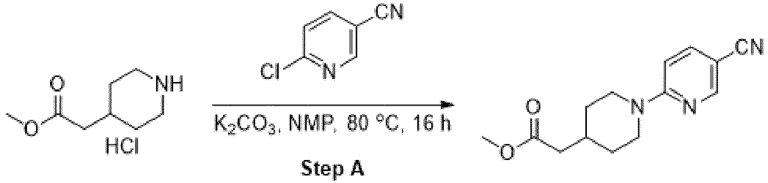

To a solution of 6-chloropyridine-3-carbonitrile (831 mg, 6.00 mmol, 1.0 eq.) and methyl 2-(piperidin-4-yl)acetate hydrochloride (1.16 g, 6.00 mmol, 1.0 eq.) in NMP (11 mL) was added K₂CO₃ (2.49 g, 18.00 mmol, 3.0 eq.). The mixture was stirred at 80 °C for 16 h. The mixture was cooled to room temperature and diluted with water (100 mL). The mixture was extracted with EtOAc (2 x 80 mL). The organic layers were combined and washed with brine (2 x 100 mL), dried over Na₂SO₄ and concentrated under vacuum. The residue was purified by silica gel chromatography (Petroleum ether/EtOAc= 3/1; V/V) to afford methyl 2-(1-(5-cyanopyridin-2-yl)piperidin-4-yl) acetate (1.35 g, 86.8%) as a yellow solid.
LCMS: *(ESI)* (M+H)⁺ = 260.2.
¹H NMR: (400 MHz, CDCl₃) *δ* 8.38 (dd, *J* = 2.4, 0.4 Hz, 1H), 7.57 (dd, *J* = 8.8, 2.4 Hz, 1H), 6.59 (d, *J* = 9.2 Hz, 1H), 4.46 - 4.38 (m, 2H), 3.69 (s, 3H), 2.96 (td, *J* = 13.2, 2.8 Hz, 2H), 2.28 (d, *J* = 6.8 Hz, 2H), 2.18 - 2.04 (m, 1H), 1.92 - 1.79 (m, 2H), 1.32 - 1.19 (m, 2H).

### Step B: synthesis of 2-(1-(5-cyanopyridin-2-yl)piperidin-4-yl)acetic acid

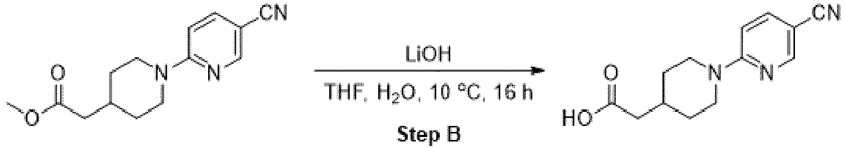

To a mixture of methyl 2-(1-(5-cyanopyridin-2-yl)piperidin-4-yl)acetate (389 mg, 1.50 mmol, 1.0 eq.) in THF (6 mL) and H₂O (3 mL) was added LiOH (126 mg, 3.00 mmol, 2.0 eq.). The mixture was stirred at 10 °C for 16 h. The mixture was diluted with water (10 mL) and neutralized to pH 7 with 1M HCl. The mixture was extracted with EtOAc (2 x 60 mL). The organic layers were combined and washed with brine (40 mL), dried over Na₂SO₄ and concentrated under vacuum to afford 2-(1-(5-cyanopyridin-2-yl)piperidin-4-yl)acetic acid (365 mg, 99.2%) as a light yellow solid.
LCMS: *(ESI)* (M+H)⁺ = 246.2.

### Step C: (S)-tert-butyl (1-((2-(1-(5-cyanopyridin-2-yl)piperidin-4-yl)acetamido)oxy) propan-2-yl)carbamate

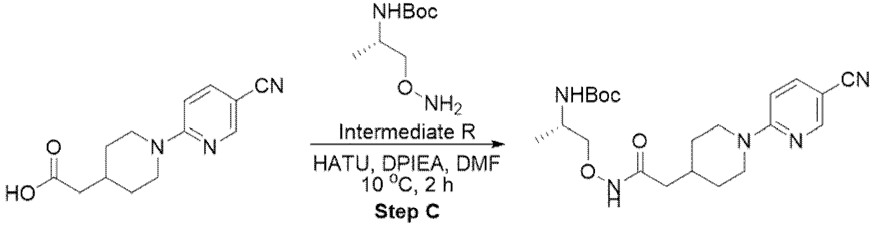

To a solution of 2-(1-(5-cyanopyridin-2-yl)piperidin-4-yl)acetic acid (245 mg, 1.00 mmol, 1.0 eq.) in DMF (5 mL) were added DIPEA (388 mg, 3.00 mmol, 3.0 eq.) and HATU (456 mg, 1.20 mmol, 1.2 eq.), followed by (S)-tert-butyl (1-(aminooxy)propan-2-yl)carbamate (190 mg, 1.00 mmol, 1.0 eq.). The mixture was stirred at 10 °C for 2 h. The mixture was quenched with water (80 mL) and extracted with EtOAc (2 x 80 mL). The organic layers were combined and washed with brine (2 x 80 mL), dried over Na₂SO₄ and concentrated under vacuum. The residue was purified by silica gel chromatography (Petroleum ether/EtOAc= 3/7; V/V) to afford (S)-tert-butyl (1-((2-(1-(5-cyanopyridin-2-yl)piperidin-4-yl)acetamido)oxy)propan-2-yl) carbamate (300 mg, 71.9%) as a white solid.
LCMS: *(ESI)* (M+H)⁺ = 418.2.
¹H NMR: (400 MHz, CDCl₃) *δ* 9.81 (s, 1H), 8.52 (s, 1H), 7.73 - 7.68 (m, 1H), 6.73 (d, *J* = 9.2 Hz, 1H), 4.77 (d, *J* = 7.6 Hz, 1H), 4.56 (t, *J* = 11.2 Hz, 2H), 4.20 - 4.09 (m, 1H), 4.03 (d, *J* = 12.0 Hz, 1H), 3.61 (t, *J* = 10.8 Hz, 1H), 3.15 - 3.01 (m, 2H), 2.41 - 2.27 (m, 1H), 2.20 (d, *J=* 7.2 Hz, 2H), 2.07 - 1.93 (m, 2H), 1.59 (s, 9H), 1.45 - 1.33 (m, 2H), 1.27 (d, *J* = 6.8 Hz, 3H).

### Step D: synthesis of (S)-N-(2-aminopropoxy)-2-(1-(5-cyanopyridin-2-yl)piperidin-4-yl) acetamide

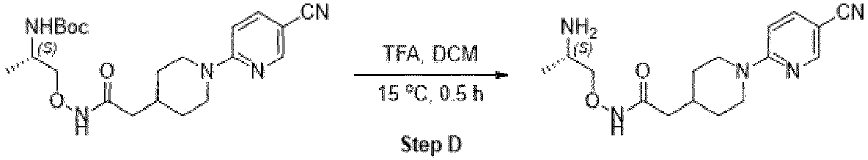

To a solution of (S)-tert-butyl (1-((2-(1-(5-cyanopyridin-2-yl)piperidin-4-yl) acetamido)oxy)propan-2-yl)carbamate (50 mg, 0.12 mmol, 1.0 eq.) in DCM (4 mL) was added TFA (1 mL). The mixture was stirred at 15 °C for 0.5 h. The excess TFA was neutralized with saturated aqueous NaHCO₃ aqueous solution (20 mL) and the mixture was extracted with DCM (4 x 60 mL). The organic layers were combined and dried over Na₂SO₄, concentrated under vacuum to afford (S)-N-(2-aminopropoxy)-2-(1-(5-cyanopyridin-2-yl)piperidin-4-yl)acetamide (32 mg, 84.2%) as a white solid.
LCMS: *(ESI)* (M+H)⁺ = 318.2.

### Step E: synthesis of (S)-2-(1-(5-cyanopyridin-2-yl)piperidin-4-yl)-N-(2-((6-oxo -5-(trifluoromethyl)-1,6-dihydropyridazin-4-yl)amino)propoxy)acetamide

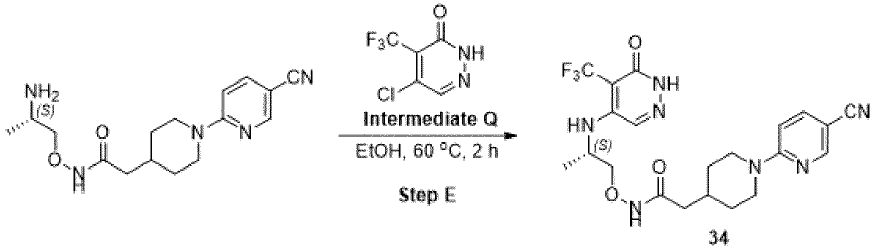

To a solution of (S)-N-(2-aminopropoxy)-2-(1-(5-cyanopyridin-2-yl)piperidin-4-yl) acetamide (16 mg, 0.05 mmol, 1.0 eq.) in EtOH (1 mL) was added 5-chloro-4-(trifluoromethyl)pyridazin-3(2H)-one (10 mg, 0.05 mmol, 1.0 eq.). The mixture was heated to 60 °C and stirred for 2 h. The mixture was cooled to room temperature and diluted with water (10 mL). The mixture was extracted with EtOAc (3 x 15 mL). The organic layers were combined and dried over Na₂SO₄, concentrated under vacuum. The residue was purified by prep-HPLC (FA) to afford (S)-2-(1-(5-cyanopyridin-2-yl)piperidin-4-yl)-N-(2-((6-oxo-5-(trifluoromethyl)-1,6-dihy dropyridazin-4-yl)amino)propoxy)acetamide (3.12 mg, 12.9%) as a white solid.
LCMS: *(ESI)* (M+H)⁺ = 480.2.
¹H NMR: (400 MHz, CDCl₃) *δ* 10.45 (s, 1H), 9.00 (s, 1H), 8.37 (d, *J* = 2.0 Hz, 1H), 7.71 (s, 1H), 7.57 (dd, *J* = 9.2, 2.4 Hz, 1H), 6.59 (d, *J* = 9.6 Hz, 1H), 5.96 (s, 1H), 4.48 - 4.35 (m, 2H), 4.18 - 4.02 (m, 2H), 3.93 - 3.82 (m, 1H), 3.02 - 2.90 (m, 2H), 2.25 - 2.12 (m, 1H), 2.12 - 2.00 (m, 2H), 1.90 - 1.78 (m, 2H), 1.35 (d, *J* = 6.4 Hz, 3H), 1.27 - 1.17 (m, 2H).

### Example 35:

### (S)-N-(2-((5-bromo-6-oxo-1,6-dihydropyridazin-4-yl)amino)propoxy)-2-(1-(5-(trifluoro methyl)pyrimidin-2-yl)piperidin-4-yl)acetamide

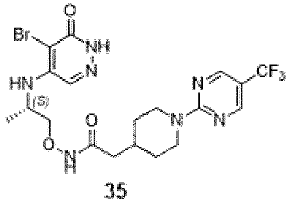

### 35.1) Synthesis of intermediate T

### Step A: (S)-4-bromo-5-((1-hydroxypropan-2-yl)amino)-2-((2-(trimethylsilyl)ethoxy) methyl)pyridazin-3(2H)-one

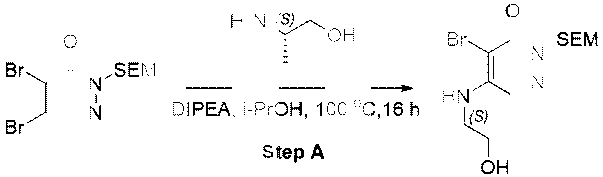

To a solution of 4,5-dibromo-2-((2-(trimethylsilyl)ethoxy)methyl)pyridazin -3(2H)-one (2.60 g, 6.79 mmol, 1.0 eq.) in i-PrOH (40 mL) were added DIPEA (2.63 g, 20.37 mmol, 3.0 eq.) and L-Alaninol (510 mg, 6.79 mmol, 1.0 eq.). The mixture was stirred at 100 °C for 16 h. The mixture was cooled to room temperature and quenched with water (200 mL), extracted with EtOAc (3 x 150 mL). The organic layers were combined and washed with brine (500 mL), dried over anhydrous Na₂SO₄ and concentrated under reduced pressure. The residue was purified by column chromatography (petroleum ether/EtOAc= 4/1; V/V) to afford (S)-4-bromo-5-((1-hydroxypropan-2-yl)amino)-2-((2-(trimethylsilyl)ethoxy)methyl)pyri dazin-3(2H)-one (1.32 g, 51.6%) as a white solid.
LCMS: *(ESI)* (M+H)⁺ = 378.0, 380.0.
¹H NMR: (400 MHz, CDCl₃) *δ* 7.63 (s, 1H), 5.52 - 5.40 (m, 2H), 5.01 (d, *J* = 8.0 Hz, 1H), 3.92 - 3.77 (m, 2H), 3.74 - 3.65 (m, 3H), 1.32 (d, *J* = 6.4 Hz, 3H), 0.99 - 0.93 (m, 2H), 0.02 (s, 9H).

### Step B: synthesis of (S)-2-(2-((5-bromo-6-oxo-1-((2-(trimethylsilyl)ethoxy)methyl) -1,6-dihydropyridazin-4-yl)amino)propoxy)isoindoline-1,3-dione (Intermediate T)

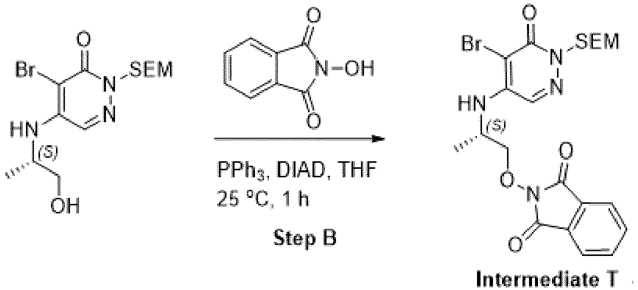

To a solution of (S)-4-bromo-5-((1-hydroxypropan-2-yl)amino)-2-((2-(trimethylsilyl)ethoxy)methyl)pyridazin-3(2H)-one (1.32 g, 3.49 mmol, 1.0 eq.) in THF (15 mL) were added N-Hydroxyphthalimide (569 mg, 3.49 mmol, 1.0 eq.), PPh₃ (1.01 g, 3.84 mmol, 1.1 eq.) and followed by dropwise addition of DIAD (776 mg, 3.84 mmol, 1.1 eq.). The mixture was stirred at 25 °C for 1 h. The mixture was quenched with water (100 mL) and extracted with EtOAc (3 x 80 mL). The organic layers were combined and washed with brine (300 mL), dried over anhydrous Na₂SO₄ and concentrated under vacuum. The residue was purified by column chromatography (petroleum ether/EtOAc= 3/1; V/V) to afford (S)-2-(2-((5-bromo-6-oxo-1-((2-(trimethylsilyl)ethoxy)methyl) -1,6-dihydropyridazin-4-yl)amino)propoxy)isoindoline-1,3-dione (1.44 g, 78.9%) as a colorless oil.
LCMS: *(ESI)* (M+H)⁺ = 523.0.
¹H NMR (400 MHz, DMSO-*d₆*) *δ* 7.97 (s, 1H), 7.89 (s, 4H), 6.32 (d, J = 8.3 Hz, 1H), 5.34 (s, 2H), 4.44 (d, J = 6.6 Hz, 2H), 4.26 (d, J = 6.4 Hz, 1H), 3.65 (t, J = 8.0 Hz, 2H), 1.28 (d, J = 5.5 Hz, 3H), 0.89 (t, J = 8.0 Hz, 2H), 0.00 (s, 9H).

### 35.2) Synthesis of compound 35

### Step A: (S)-5-((1-(aminooxy)propan-2-yl)amino)-4-bromo-2-((2-(trimethylsilyl)ethoxy) methyl)pyridazin-3(2H)-one

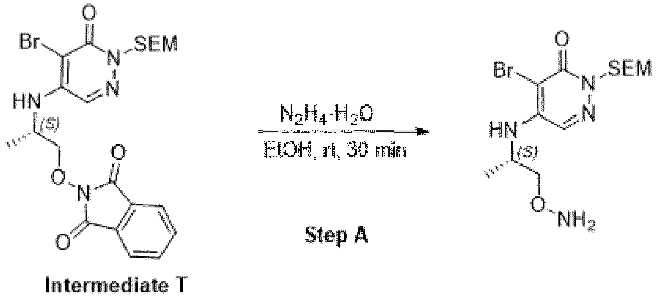

To a solution of (S)-2-(2-((5-bromo-6-oxo-1-((2-(trimethylsilyl)ethoxy)methyl)-1,6-dihydropyridazin-4-yl)amino)propoxy)isoindoline-1,3-dione (410 mg, 0.784 mmol, 1.0 eq.) in EtOH (5 mL) was added hydrazine hydrate (58 mg, 1.16 mmol, 1.5 eq.) at 0 °C. The resulting mixture was stirred at rt for 30 min. The mixture was concentrated under vacuum. The residue was triturated with MTBE (10mL). The insoluble precipitate was filtered off and washed with MTBE (10 mL). The filtrate was concentrated in vacuum to afford (S)-5-((1-(aminooxy)propan-2-yl)amino)-4-bromo-2-((2-(trimethylsilyl)ethoxy)methyl)pyridazin-3(2H)-one (170 mg, 55% yield) as a white solid.
LCMS: *(ESI)* (M+H)⁺ = 393.2, 395.2.

### Step B: Synthesis of (S)-N-(2-((5-bromo-6-oxo-1-((2-(trimethylsilyl)ethoxy)methyl) -1,6-dihydropyridazin-4-yl)amino)propoxy)-2-(1-(5-(trifluoromethyl)pyrimidin-2-yl)pip eridin-4-yl)acetamide

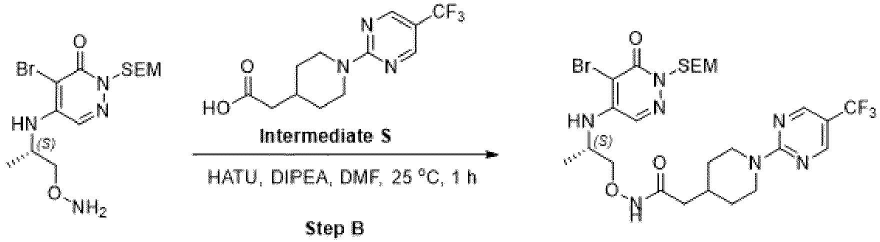

To a solution of 2-(1-(5-(trifluoromethyl)pyrimidin-2-yl)piperidin-4-yl)acetic acid (89 mg, 0.308 mmol, 1.1 eq.) in DMF (2 mL) were added DIPEA (108 mg, 0.842 mmol, 3.0 eq.) and HATU (128 mg, 0.336 mmol, 1.2 eq.) followed by dropwise addition of (S)-5-((1-(aminooxy)propan-2-yl)amino)-4-bromo-2-((2-(trimethylsilyl)ethoxy)methyl) pyridazin-3(2H)-one (110 mg, 0.280 mmol, 1.0 eq.). The resulting mixture was stirred at 25 °C for 1 h. The reaction mixture was quenched with water (10 mL) and extracted with EtOAc (3 x 10 mL). The organic layers were combined and washed with brine (10 mL), dried over Na₂SO₄ and concentrated under vacuum. The residue was purified by silica gel chromatography (Petroleum/EtOAc=1/2, V/V) to afford (S)-N-(2-((5-bromo-6-oxo-1-((2-(trimethylsilyl)ethoxy)methyl)-1,6-dihydropyridazin-4-yl)amino)propoxy)-2-(1-(5-(trifluoromethyl)pyrimidin-2-yl)piperidin-4-yl)acetamide (100 mg, 53% yield) as a colorless oil.
LCMS: *(ESI)* (M+H)⁺ = 664.2, 666.2.
¹H NMR: (400 MHz, CDCl₃) *δ* 8.98 (s, 1H), 8.45 (s, 2H), 7.58 (s, 1H), 5.49 (d, J = 9.8 Hz, 1H), 5.35 (d, J = 9.8 Hz, 1H), 5.28 (s, 1H), 4.84 (d, J = 12.0 Hz, 2H), 4.17 - 4.08 (m, 1H), 4.06 - 3.88 (m, 2H), 3.68 (t, J = 8.1 Hz, 2H), 2.95 (t, J = 12.7 Hz, 2H), 2.31 - 2.14 (m, 1H), 2.14 - 2.01 (m, 2H), 1.85 (d, J = 12.8 Hz, 2H), 1.36 (d, J = 6.5 Hz, 3H), 1.27 - 1.14 (m, 2H), 0.93 (t, J = 8.4 Hz, 2H), -0.02 (s, 9H).

### Step C: synthesis of (S)-N-(2-((5-bromo-6-oxo-1,6-dihydropyridazin-4-yl)amino) propoxy)-2-(1-(5-(trifluoromethyl)pyrimidin-2-yl)piperidin-4-yl)acetamide

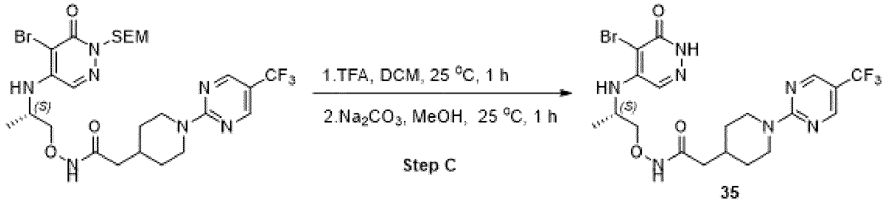

To a solution of (S)-N-(2-((5-bromo-6-oxo-1-((2-(trimethylsilyl)ethoxy)methyl) -1,6-dihydropyridazin-4-yl)amino)propoxy)-2-(1-(5-(trifluoromethyl)pyrimidin-2-yl)pip eridin-4-yl)acetamide (90 mg, 0.135 mmol, 1.0 eq.) in DCM (2 mL) was added TFA (0.3 mL). The resulting mixture was stirred at 25 °C for 1 h. The mixture was neutralized with saturated NaHCOs aqueous solution (5 mL) and extracted with DCM (3 x 10 mL). The organic layers were combined and dried over Na₂SO₄, concentrated under vacuum. The residue was redissolved in MeOH (5 mL) and K₂CO₃ (46 mg, 0.339 mmol, 2.5 eq.) was added. The mixture was stirred at 25 °C for another 1 h. The reaction mixture was filtered and the filtrate was purified by reverse phase silica gel chromatography (CH₃CN/H₂O= 10/1, V/V) to afford (S)-N-(2-((5-bromo-6-oxo-1,6 -dihydropyridazin-4-yl)amino)propoxy)-2-(1-(5-(trifluoromethyl)pyrimidin-2-yl)piperid in-4-yl)acetamide (49 mg, 68% yield) as a white solid.
LCMS: *(ESI)* (M+H)⁺ = 534.2.
¹H NMR: (400 MHz, CDCl₃) *δ* 10.62 (s, 1H), 9.50 (s, 1H), 8.45 (s, 2H), 7.59 (s, 1H), 5.30 (s, 1H), 4.82 (d, J = 13.5 Hz, 2H), 4.22 - 4.10 (m, 1H), 4.10 - 3.98 (m, 1H), 3.96 - 3.86 (m, 1H), 2.94 (t, J = 12.4 Hz, 2H), 2.29 - 2.14 (m, 1H), 2.13 - 2.00 (m, 2H), 1.83 (d, J = 12.7 Hz, 2H), 1.34 (d, J = 5.9 Hz, 3H), 1.26 - 1.11 (m, 2H).

### Example 36:

### (S)-N-(2-((5-acetyl-6-oxo-1,6-dihydropyridazin-4-yl)amino)propoxy)-2-(1-(5-(trifluoro methyl)pyrimidin-2-yl)piperidin-4-yl)acetamide

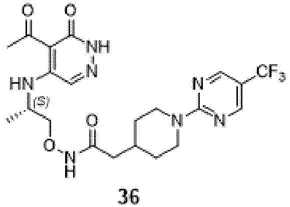

### 36.1) Synthesis of intermediate W

### Step A: Synthesis of (S)-2-(2-((5-acetyl-6-oxo-1-((2-(trimethylsilyl)ethoxy)methyl)-1,6-dihydropyridazin-4-yl)amino)propoxy)isoindoline-1,3-dione

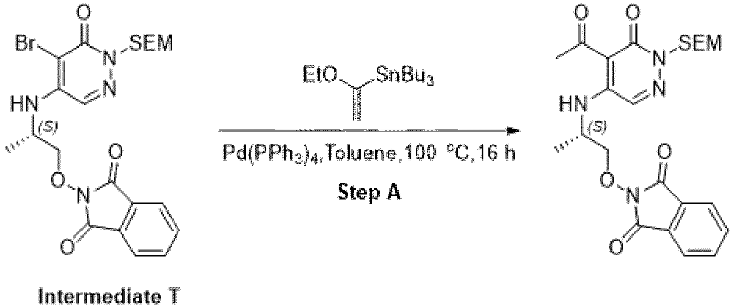

To a solution of (S)-2-(2-((5-bromo-6-oxo-1-((2-(trimethylsilyl)ethoxy)methyl) -1,6-dihydropyridazin-4-yl)amino)propoxy)isoindoline-1,3-dione (733 mg, 1.40 mmol, 1.0 eq.) and tributyl(1-ethoxyvinyl)stannane (607 mg, 1.68 mmol, 1.2 eq.) in toluene (28 mL) was added Pd(PPh₃)₄ (323 mg, 0.28 mmol, 0.2 eq.). The mixture was stirred at 100 °C for 16 h. The reaction was cooled to room temperature and quenched with water (50 mL), extracted with EtOAc (3 x 40 mL). The organic layers were combined and washed with brine (150 mL), dried over anhydrous Na₂SO₄ and concentrated under vacuum. The residue was purified by column chromatography (petroleum ether/EtOAc= 1/1; V/V) to afford (S)-2-(2-((5-acetyl-6-oxo-1-((2-(trimethylsilyl)ethoxy)methyl)-1,6-dihydropyridazin-4-yl)amino)propoxy)isoindoline-1,3-dione (446 mg, 61.9%) as a white solid.
LCMS: *(ESI)* (M+H)⁺ = 487.2.
¹H NMR (400 MHz, DMSO-*d₆*) *δ* 10.82 (d, *J* = 8.4 Hz, 1H), 8.15 (s, 1H), 7.92 - 7.84 (m, 4H), 5.31 (s, 2H), 4.65 - 4.50 (m, 1H), 4.39 - 4.27 (m, 2H), 3.70 - 3.61 (m, 2H), 2.52 (s, 3H), 1.32 (d, *J* = 6.8 Hz, 3H), 0.92 - 0.86 (m, 2H), 0.00 (s, 9H).

### Step B: Synthesis of (S)-4-acetyl-5-((1-(aminooxy)propan-2-yl)amino)-2-((2-(trimethylsilyl)ethoxy)methyl)pyridazin-3(2H)-one

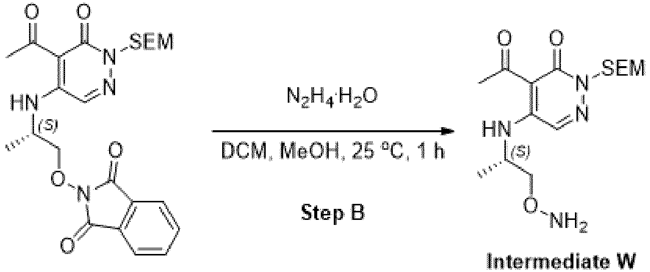

To a solution of (S)-2-(2-((5-acetyl-6-oxo-1-((2-(trimethylsilyl)ethoxy)methyl) -1,6-dihydropyridazin-4-yl)amino)propoxy)isoindoline-1,3-dione (390 mg, 0.80 mmol, 1.0 eq.) in DCM (10 mL) and MeOH (5 mL) was added hydrazine hydrate (76 mg, 1.52 mmol, 1.9 eq.). The mixture was stirred at 25 °C for 1 h. The mixture was concentrated under vacuum. The residue was triturated with EtOH (20 mL) and filtered, the filtrate was concentrated under vacuum. The residue was purified by column chromatography (petroleum ether/EtOAc= 2/1; V/V) to afford (S)-4-acetyl-5-((1-(aminooxy) propan-2-yl)amino)-2-((2-(trimethylsilyl)ethoxy)methyl)pyridazin-3(2H)-one (230 mg, 80.5%) as a yellow oil.
LCMS: *(ESI)* (M+H)⁺ = 357.2.

### 36.2) Synthesis of compound 36

### Step A: Synthesis of (S)-N-(2-((5-acetyl-6-oxo-1-((2-(trimethylsilyl)ethoxy) methyl)-1,6-dihydropyridazin-4-yl)amino)propoxy)-2-(1-(5-(trifluoromethyl)pyrimidin-2-yl)piperidin-4-yl)acetamide

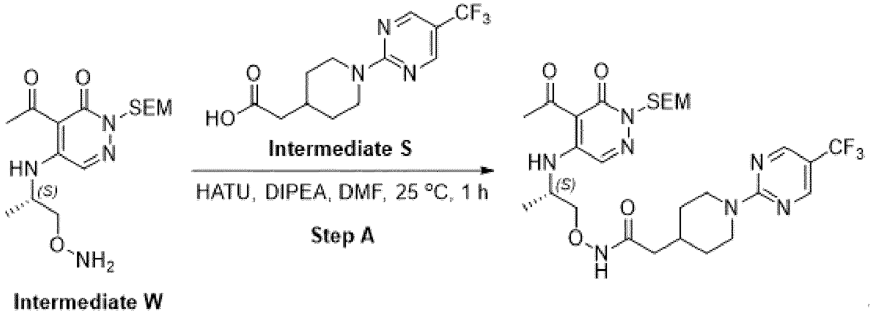

To a solution of ((S)-4-acetyl-5-((1-(aminooxy)propan-2-yl)amino) -2-((2-(trimethylsilyl)ethoxy)methyl)pyridazin-3(2H)-one (230 mg, 0.65 mmol, 1.0 eq.) and 2-(1-(5-(trifluoromethyl)pyrimidin-2-yl)piperidin-4-yl)acetic acid (208 mg, 0.72 mmol, 1.1 eq.) in DMF (15 mL) were added DIPEA (252 mg, 1.95 mmol, 3.0 eq.) and HATU (297 mg, 0.78 mmol, 1.2 eq.). The mixture was stirred at 25 °C for 1 h. The mixture was quenched with water (50 mL) and extracted with EtOAc (3 x 40 mL). The organic layers were combined and washed with brine (200 mL), dried over anhydrous Na₂SO₄ and concentrated under vacuum. The residue was purified by column chromatography (petroleum ether/EtOAc= 1/1; V/V) to afford (S)-N-(2-((5-acetyl-6-oxo-1-((2-(trimethylsilyl)ethoxy)methyl)-1,6-dihydropyridazin-4-yl)amino)propoxy)-2-(1-(5-(trifluoromethyl)pyrimidin-2-yl)piperidin-4-yl)acetamide (360 mg, 88.9%) as a white solid.
LCMS: *(ESI)* (M+H)⁺ = 628.2.

### Step B: synthesis of (S)-N-(2-((5-acetyl-6-oxo-1,6-dihydropyridazin-4-yl)amino) propoxy)-2-(1-(5-(trifluoromethyl)pyrimidin-2-yl)piperidin-4-yl)acetamide

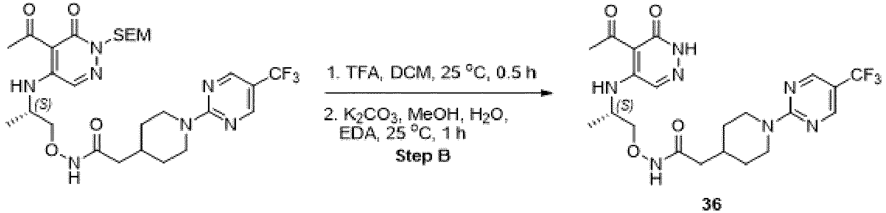

To a solution of (S)-N-(2-((5-acetyl-6-oxo-1-((2-(trimethylsilyl)ethoxy)methyl)-1,6 -dihydropyridazin-4-yl)amino)propoxy)-2-(1-(5-(trifluoromethyl)pyrimidin-2-yl)piperid in-4-yl)acetamide (180 mg, 0.28 mmol, 1.0 eq.) in DCM (6 mL) was added TFA (2 mL). The resulting solution was stirred at 25 °C for 0.5 h. The mixture was concentrated under vacuum. To the residue were added MeOH (6 mL), water (6 mL), K₂CO₃ (174 mg, 1.26 mmol, 4.5 eq.) and ethylenediamine (124 mg, 2.07 mmol, 7.4 eq.). The mixture was stirred at 25 °C for another 1 h. The mixture was quenched with water (50 mL) and extracted with EtOAc (3 x 40 mL). The organic layers were combined and washed with brine (200 mL), dried over anhydrous Na₂SO₄ and concentrated under reduced pressure. The residue was purified by prep-HPLC to afford (S)-N-(2-((5-acetyl-6-oxo-1,6-dihydropyridazin-4-yl)amino)propoxy)-2-(1-(5-(trifluoro methyl)pyrimidin-2-yl)piperidin-4-yl)acetamide (30.83 mg, 21.6%) as a white solid.
LCMS: *(ESI)* (M+H)⁺ = 498.2.
¹H NMR: 220817-072-P (400 MHz, DMSO-*d₆*) *δ* 12.35 (s, 1H), 11.11 (s, 1H), 10.72 (d, *J* = 8.4 Hz, 1H), 8.68 (s, 2H), 7.98 (s, 1H), 4.69 (d, *J* = 13.2 Hz, 2H), 4.37 - 4.25 (m, 1H), 3.98 - 3.89 (m, 1H), 3.87 - 3.78 (m, 1H), 3.00 (t, *J* = 12.0 Hz, 2H), 2.55 (s, 3H), 2.07 - 2.00 (m, 1H), 1.95 - 1.91 (m, 2H), 1.89 - 1.69 (m, 2H), 1.25 (d, *J* = 6.4 Hz, 3H), 1.18 - 1.02 (m, 2H).

### Example 37:

### N-(2-(5-oxo-3,4,5,6-tetrahydropyrido(2,3-d)pyridazin-1(2H)-yl)propoxy)-2-(1-(5-(triflu oromethyl)pyrimidin-2-yl)piperidin-4-yl)acetamide

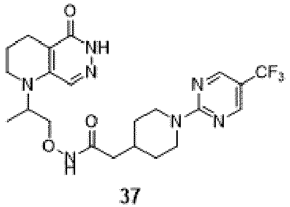

### Step A: synthesis of 6-((2-(trimethylsilyl)ethoxy)methyl)pyrido(2,3-d)pyridazin -5(6H)-one

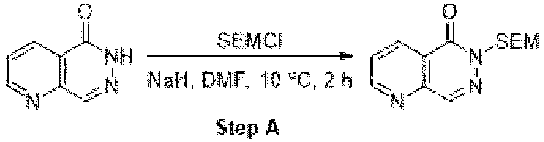

To a mixture of pyrido(2,3-d)pyridazin-5(6H)-one (1.03 g, 7.00 mmol, 1.0 eq.) in DMF (35 mL) was added NaH (60%, 0.70 g, 17.50 mmol, 2.5 eq.) in portions at 0 °C in ice-water bath under N₂ atmosphere . The mixture was stirred at 0 °C for 30 min. 2-(Trimethylsilyl)ethoxymethyl chloride (1.75 g, 10.50 mmol, 1.5 eq.) was added to the reaction mixture. The mixture was stirred for 2 h at 10 °C. The mixture was quenched with saturated NH₄Cl aqueous solution (120 mL) and extracted with EtOAc (2 x 100 mL). The organic layers were combined and washed with brine (2 x 100 mL), dried over Na₂SO₄ and concentrated under vacuum. The residue was purified by silica gel chromatography (EtOAc/Petroleum ether= 1/3; V/V) to afford 6-((2-(trimethylsilyl)ethoxy)methyl)pyrido(2,3-d)pyridazin-5(6H)-one (1.26 g, 64.9 %) as a yellow oil.
LCMS: *(ESI)* (M+H)⁺=278.1.
¹H NMR: (400 MHz, DMSO-*d₆*) *δ* 9.18 (dd, *J* = 4.4, 1.6 Hz, 1H), 8.66 (d, *J* = 8.0 Hz, 1H), 8.53 (s, 1H), 7.90 (dd, *J* = 8.0, 4.4 Hz, 1H), 5.47 (s, 2H), 3.70 (t, *J* = 8.0 Hz, 2H), 0.89 (t, *J* = 8.0 Hz, 2H), 0.00 (s, 9H).

### Step B: synthesis of 6-((2-(trimethylsilyl)ethoxy)methyl)-1,2,3,4-tetrahydropyrido(2,3-d) pyridazin-5(6H)-one

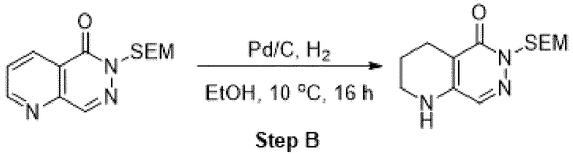

To a solution of 6-((2-(trimethylsilyl)ethoxy)methyl)pyrido(2,3-d)pyridazin -5(6H)-one (1.38 g, 4.98 mmol, 1.0 eq.) in EtOH (50 mL) was added Pd/C (10%, 0.20 g) under N₂ atmosphere. The mixture was degassed and purged with Hydrogen several times. The mixture was stirred at 10 °C for 16 h under Hydrogen atmoshpere. The mixture was filtered and the filtrate was concentrated under vacuum to afford 6-((2-(trimethylsilyl)ethoxy)methyl)-1,2,3,4-tetrahydropyrido(2,3-d)pyridazin-5(6H)-on e (1.38 g, 98.6% yield) as an off-white solid.
LCMS: *(ESI)* (M+H)⁺=282.0.
¹H NMR: (400 MHz, DMSO-*d₆*) *δ* 7.43 (s, 1H), 6.94 (br s, 1H), 5.23 (s, 2H), 3.61 (t, *J* = 8.0 Hz, 2H), 3.24 - 3.17 (m, 2H), 2.38 (t, *J* = 6.0 Hz, 2H), 1.80 - 1.71 (m, 2H), 0.87 (t, *J* = 8.0 Hz, 2H), 0.00 (s, 9H).

### Step C: synthesis of methyl 2-(5-oxo-6-((2-(tiimethylsilyl)ethoxy)methyl)-3,4,5,6 -tetrahydropyrido(2,3-d)pyridazin-1(2H)-yl)propanoate

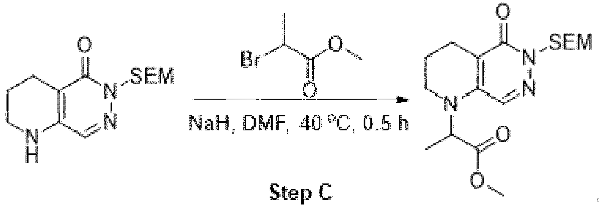

To a mixture of 6-((2-(trimethylsilyl)ethoxy)methyl)-1,2,3,4-tetrahydropyrido (2,3-d)pyridazin-5(6H)-one (790 mg, 2.81 mmol, 1.0 eq.) in DMF (12 mL) was added NaH (60%, 898 mg, 22.46 mmol, 8.0 eq.) at 0 °C in ice-water bath under N₂ atmosphere. The mixture was stirred at 0 °C for 30 min. To the mixture was added methyl 2-bromopropanoate (2.11 g, 12.63 mmol, 4.5 eq.). The mixture was stirred at 40 °C for 0.5 h. The mixture was quenched with cold water (60 mL) and extracted with EtOAc (2 x 120 mL). The organic layers were combined and washed with brine (60 mL), dried over Na₂SO₄ and concentrated under vacuum. The residue was purified by silica gel chromatography (EtOAc/Petroleum ether= 6/10; V/V) to afford methyl 2-(5-oxo-6-((2-(trimethylsilyl)ethoxy)methyl)-3,4,5,6-tetrahydropyrido(2,3-d)pyridazin-1(2H)-yl)propanoate (660 mg, 64.0% yield) as a yellow oil.
LCMS: *(ESI)* (M+H)⁺=367.8.
¹H NMR: (400 MHz, CDCl₃) *δ* 7.57 (s, 1H), 5.46 - 5.38 (m, 2H), 4.57 (q, *J* = 7.2 Hz, 1H), 3.75 (s, 3H), 3.73 - 3.67 (m, 2H), 3.32 - 3.15 (m, 2H), 2.70 - 2.50 (m, 2H), 1.95 - 1.86 (m, 2H), 1.54 (d, *J* = 7.2 Hz, 3H), 1.03 - 0.93 (m, 2H), 0.00 (s, 9H).

### Step D: synthesis of 1-(1-hydroxypropan-2-yl)-6-((2-(trimethylsilyl)ethoxy)methyl) -1,2,3,4-tetrahydropyrido(2,3-d)pyridazin-5(6H)-one

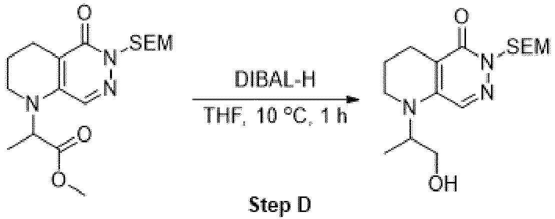

To a solution of methyl 2-(5-oxo-6-((2-(trimethylsilyl)ethoxy)methyl)-3,4,5,6 -tetrahydropyrido(2,3-d)pyridazin-1(2H)-yl)propanoate (890 mg, 2.42 mmol, 1.0 eq.) in THF (10 mL) was added DIBAL-H (1M, 12.1 mL, 12.10 mmol, 5.0 eq.) at 0 °C in ice-water bath under N₂ atmosphere. The mixture was stirred at 10 °C for 1 h. The mixture was quenched with saturated NH₄Cl aqueous solution (100 mL) and extracted with DCM (4 x 180 mL). The organic layers were combined and dried over Na₂SO₄, concentrated under vacuum. The residue was purified by silica gel chromatography (MeOH/DCM= 6/100, V/V) to afford 1-(1-hydroxypropan-2-yl)-6-((2-(trimethylsilyl)ethoxy)methyl)-1,2,3,4-tetrahydropyrido(2,3-d)pyridazin-5(6H)-one (426 mg, 51.8% yield) as a yellow oil.
LCMS: *(ESI)* (M+H)⁺=340.2.
¹H NMR: (400 MHz, DMSO-*d₆*) *δ* 7.93 (s, 1H), 5.24 (s, 2H), 4.79 (t, *J=* 5.6 Hz, 1H), 4.22 (q, *J* = 6.8 Hz, 1H), 3.66 - 3.58 (m, 2H), 3.55 - 3.38 (m, 2H), 3.18 (t, *J* = 5.6 Hz, 2H), 2.39 (t, *J* = 6.4 Hz, 2H), 1.86 - 1.66 (m, 2H), 1.10 (d, *J* = 6.8 Hz, 3H), 0.7 (t, *J* = 8.0 Hz, 2H), 0.00 (s, 9H).

### Step E: synthesis of 2-(5-oxo-6-((2-(trimethylsilyl)ethoxy)methyl)-3,4,5,6-tetrahydropyrido(2,3-d)pyridazin-1(2H)-yl)propyl 4-methylbenzenesulfonate

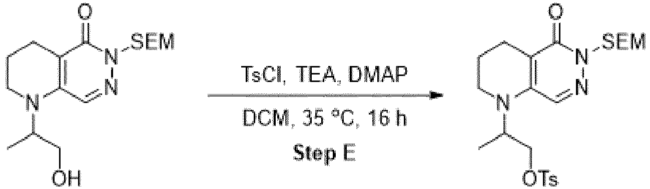

To a solution of 1-(1-hydroxypropan-2-yl)-6-((2-(trimethylsilyl)ethoxy)methyl) -1,2,3,4-tetrahydropyrido(2,3-d)pyridazin-5(6H)-one (100 mg, 0.29 mmol, 1.0 eq.) in DCM (10 mL) were added TEA (59 mg, 0.58 mmol, 2.0 eq.), DMAP (18 mg, 0.15 mmol, 0.5 eq.) and Tosyl chloride (55 mg, 0.29 mmol, 1.0 eq.). The mixture was stirred at 35 °C for 16 h. The mixture was quenched with water (30 mL) and extracted with EtOAc (3 x 40 mL). The organic layers were combined and washed with brine (100 mL), dried over anhydrous Na₂SO₄ and concentrated under vacuum. The residue was purified by column chromatography (Petroleum ether/EtOAc= 1/1, V/V) to afford 2-(5-oxo-6-((2-(trimethylsilyl)ethoxy)methyl)-3,4,5,6-tetrahydropyrido(2,3-d)pyridazin-1(2H)-yl)propyl 4-methylbenzenesulfonate (120 mg, 83.9%) as a yellow oil.
LCMS: *(ESI)* (M+H)⁺ = 493.9.

### Step F: synthesis of 2-(2-(5-oxo-6-((2-(trimethylsilyl)ethoxy)methyl)-3,4,5,6 -tetrahydropyrido(2,3-d)pyridazin-1(2H)-yl)propoxy)isoindoline-1,3-dione

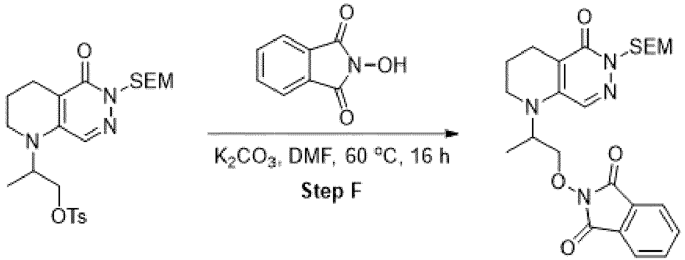

To a solution of 2-(5-oxo-6-((2-(trimethylsilyl)ethoxy)methyl)-3,4,5,6 -tetrahydropyrido(2,3-d)pyridazin-1(2H)-yl)propyl 4-methylbenzenesulfonate (150 mg, 0.30 mmol, 1.0 eq.) in DMF (10 mL) were added N-Hydroxyphthalimide (49 mg, 0.30 mmol, 1.0 eq.) and K₂CO₃ (124 mg, 0.90 mmol, 3.0 eq.). The mixture was stirred at 60 °C for 16 h. The mixture was cooled to room temperature and diluted with water (50 mL), extracted with EtOAc (3 x 50 mL). The organic layers were combined and washed with brine (2 x 60 mL), dried over anhydrous Na₂SO₄ and concentrated under vacuum. The residue was purified by column chromatography (Petroleum ether/EtOAc= 2/1, V/V) to afford 2-(2-(5-oxo-6-((2-(trimethylsilyl)ethoxy)methyl)-3,4,5,6-tetrahydropyrido(2,3-d)pyrida zin-1(2H)-yl)propoxy)isoindoline-1,3-dione (108 mg, 74.5%) as a colorless oil. LCMS: *(ESI)* (M+H)⁺ = 484.9.

### Step G: Synthesis of 1-(1-(aminooxy)propan-2-yl)-6-((2-(trimethylsilyl)ethoxy)methyl) -1,2,3,4-tetrahydropyrido(2,3-d)pyridazin-5(6H)-one

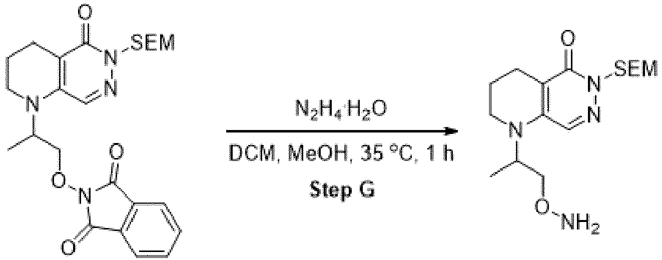

To a solution of 2-(2-(5-oxo-6-((2-(trimethylsilyl)ethoxy)methyl)-3,4,5,6 -tetrahydropyrido(2,3-d)pyridazin-1(2H)-yl)propoxy)isoindoline-1,3-dione (90 mg, 0.18 mmol, 1.0 eq.) in DCM (4 mL) and MeOH (2 mL) was added hydrazine hydrate (14 mg, 0.27 mmol, 1.5 eq.). The mixture was stirred at 35 °C for 1 h. The reaction mixture was concentrated under vacuum. The residue was triturated with EtOH (20 mL), filtered and the filtrate was concentrated under vacuum to afford 1-(1-(aminooxy)propan-2-yl)-6-((2-(trimethylsilyl)ethoxy)methyl)-1,2,3,4-tetrahydropy rido(2,3-d)pyridazin-5(6H)-one (70 mg, 100%) as a yellow oil.
LCMS: *(ESI)* (M+H)⁺ = 355.2.

### Step H: Synthesis of N-(2-(5-oxo-6-((2-(trimethylsilyl)ethoxy)methyl)-3,4,5,6 -tetrahydropyrido(2,3-d)pyridazin-1(2H)-yl)propoxy)-2-(1-(5-(trifluoromethyl)pyrimidi n-2-yl)piperidin-4-yl)acetamide

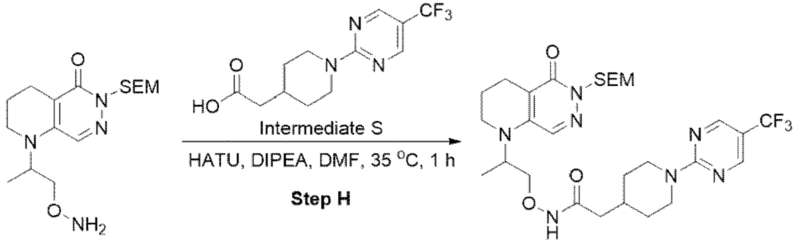

To a solution of 1-(1-(aminooxy)propan-2-yl)-6-((2-(trimethylsilyl)ethoxy)methyl) -1,2,3,4-tetrahydropyrido(2,3-d)pyridazin-5(6H)-one (64 mg, 0.18 mmol, 1.0 eq.) and 2-(1-(5-(trifluoromethyl)pyrimidin-2-yl)piperidin-4-yl)acetic acid (57 mg, 0.20 mmol, 1.1 eq.) in DMF (5 mL) were added DIPEA (70 mg, 0.54 mmol, 3.0 eq.) and HATU (82 mg, 0.21 mmol, 1.2 eq.). The mixture was stirred at 35 °C for 1 h. The reaction was quenched with water (50 mL) and extracted with EtOAc (3 x 40 mL). The organic layers were combined and washed with brine (2 x 60 mL), dried over anhydrous Na₂SO₄ and concentrated under vacuum. The residue was purified by column chromatography (Petroleum ether/EtOAc= 1/1, V/V) to afford N-(2-(5-oxo-6-((2-(trimethylsilyl)ethoxy) methyl)-3,4,5,6-tetrahydropyrido(2,3-d)pyridazin-1(2H)-yl)propoxy)-2-(1-(5-(trifluoro methyl)pyrimidin-2-yl)piperidin-4-yl)acetamide (60 mg, 53.5%) as a white solid.
LCMS: *(ESI)* (M+H)⁺ = 626.0.
¹H NMR: (400 MHz, CDCl₃) *δ* 9.82 (s, 1H), 8.47 (s, 2H), 7.73 (s, 1H), 5.47 (d, *J* = 9.6 Hz, 1H), 5.26 (d, *J* = 9.6 Hz, 1H), 4.84 (d, *J* = 13.6 Hz, 2H), 4.47 - 4.31 (m, 1H), 4.12 - 3.95 (m , 2H), 3.75 - 3.60 (m , 2H), 3.19 (t, *J=* 5.2 Hz, 2H), 3.04 - 2.95 (m, 2H), 2.60 - 2.40 (m, 2H), 2.27 - 2.05 (m, 3H), 1.95 - 1.75 (m, 4H), 1.30 - 1.17 (m, 5H), 0.99 - 0.91 (m, 2H), 0.04 (s, 9H).

### Step I: synthesis of N-(2-(5-oxo-3,4,5,6-tetrahydropyrido(2,3-d)pyridazin -1(2H)-yl)propoxy)-2-(1-(5-(trifluoromethyl)pyrimidin-2-yl)piperidin-4-yl)acetamide

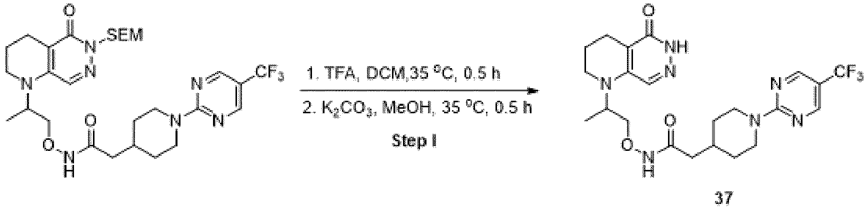

To a solution of N-(2-(5-oxo-6-((2-(trimethylsilyl)ethoxy)methyl)-3,4,5,6 -tetrahydropyrido(2,3-d)pyridazin-1(2H)-yl)propoxy)-2-(1-(5-(trifluoromethyl)pyrimidi n-2-yl)piperidin-4-yl)acetamide (60 mg, 0.09 mmol, 1.0 eq.) in DCM (3 mL) was added TFA (1 mL). The resulting solution was stirred at 35 °C for 0.5 h. The mixture was concentrated under vacuum and to the residue were added MeOH (2 mL) and K₂CO₃ (20 mg, 0.14 mmol, 1.5 eq.). The mixture was stirred at 35 °C for another 0.5 h. The mixture was filtered and the filtrate was purified by prep-HPLC to afford N-(2-(5-oxo-3,4,5,6-tetrahydropyrido(2,3-d)pyridazin-1(2H)-yl)propoxy)-2-(1-(5-(triflu oromethyl)pyrimidin-2-yl)piperidin-4-yl)acetamide (15.16 mg, 33.6%) as a white solid. LCMS: *(ESI)* (M+H)⁺ = 495.9.
¹H NMR: (400 MHz, CDCl₃) *δ* 11.12 (s, 1H), 10.41 (s, 1H), 8.44 (s, 2H), 7.72 (s, 1H), 4.80 (d, *J* = 12.4 Hz, 2H), 4.47 - 4.36 (m, 1H), 4.15 - 3.93 (m, 2H), 3.18 (t, *J* = 5.6 Hz, 2H), 2.93 (t, *J=* 12.4 Hz, 2H), 2.52 - 2.39 (m, 1H), 2.37 - 2.15 (m, 2H), 2.14 - 2.05 (m, 2H), 1.93 - 1.73 (m, 4H), 1.27 - 1.12 (m, 5H).

### Example 38:

### (S)-2-(1-(7H-pyrrolo(2,3-d)pyrimidin-2-yl)piperidin-4-yl)-N-(2-((6-oxo-5-(trifluoromet hyl)-1,6-dihydropyridazin-4-yl)amino)propoxy)acetamide

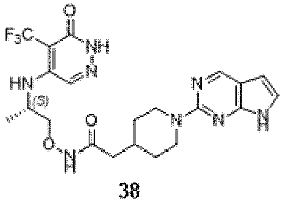

### Step A: synthesis of 2-chloro-7-((2-(trimethylsilyl)ethoxy)methyl)-7H-pyrrolo(2,3-d) pyrimidine

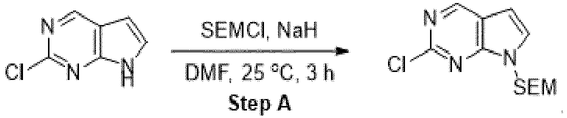

To a mixture of 2-chloro-7H-pyrrolo(2,3-d)pyrimidine (150 mg, 0.98 mmol, 1.0 eq.) in DMF (5 mL) was added NaH (60%, 47 mg, 1.18 mmol, 1.2 eq.) at 0 °C under N₂ atmosphere. The mixture was stirred at 25 °C for 1 h. 2-(Trimethylsilyl)ethoxymethyl chloride (179 mg, 1.08 mmol, 1.1 eq.) was added dropwise to the mixture at 0 °C. The mixture was stirred at 25 °C for another 2 h. The mixture was quenched with water (150 mL) and extracted with EtOAc (2 x 100 mL). The organic layers were combined and washed with brine (2 x 80 mL), dried over Na₂SO₄ and concentrated under vacuum. The residue was purified by silica gel chromatograhpy (Petroleum ether/EtOAc= 9/1; V/V) to afford 2-chloro-7-((2-(trimethylsilyl)ethoxy)methyl)-7H-pyrrolo(2,3-d)pyrimidine (160 mg, 57.7%) as a yellow solid.
LCMS: *(ESI)* (M+H)⁺ = 284.2.

### Step B: synthesis of methyl 2-(1-(7-((2-(trimethylsilyl)ethoxy)methyl)-7H-pyrrolo(2,3-d) pyrimidin-2-yl)piperidin-4-yl)acetate

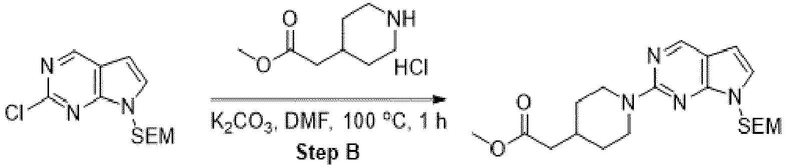

To a mixture of 2-chloro-7-((2-(trimethylsilyl)ethoxy)methyl)-7H-pyrrolo(2,3-d) pyrimidine (160 mg, 0.59 mmol, 1.0 eq.) in DMF (5 mL) were added K₂CO₃ (246 mg, 1.78 mmol, 3.0 eq.) and methyl 2-(piperidin-4-yl)acetate hydrochloride (114 mg, 0.59 mmol, 1.0 eq.). The mixture was stirred at 100 °C for 1 h. The reaction mixture was cooled to room temperature and diluted with water (100 mL), extracted with EtOAc (2 x 100 mL). The organic layers were combined and washed with brine (2 x 80 mL), dried over Na₂SO₄ and concentrated under vacuum. The residue was purified by prep-TLC (Petroleum ether/EtOAc= 10/1; V/V) to afford methyl 2-(1-(7-((2-(trimethylsilyl)ethoxy) methyl)-7H-pyrrolo(2,3-d)pyrimidin-2-yl)piperidin-4-yl)acetate (140 mg, 60.5%) as a colorless oil.
LCMS: *(ESI)* (M+H)⁺ = 404.9.
¹H NMR : (400 MHz, CDCl₃) *δ* 8.60 (s, 1H), 6.99 (d, *J* = 3.6 Hz, 1H), 6.40 (d, *J* = 3.6 Hz, 1H), 5.52 (s, 2H), 4.89 - 4.81 (m, 2H), 3.74 (s, 3H), 3.60 - 3.56 (m, 2H), 3.02 - 2.89 (m, 2H), 2.32 (d, *J* = 6.8 Hz, 2H), 2.19 - 2.05 (m, 1H), 1.85 (d, *J* = 11.6 Hz, 2H), 1.36 - 1.27 (m, 2H), 0.98 - 0.94 (m, 2H), -0.02 (s, 9H).

### Step C: synthesis of 2-(1-(7-((2-(trimethylsilyl)ethoxy)methyl)-7H-pyrrolo(2,3-d) pyrimidin-2-yl)piperidin-4-yl)acetic acid

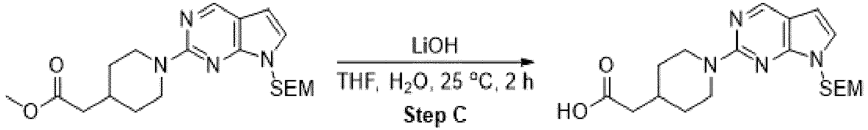

To a mixture of methyl 2-(1-(7-((2-(trimethylsilyl)ethoxy)methyl)-7H-pyrrolo (2,3-d)pyrimidin-2-yl)piperidin-4-yl)acetate (130 mg, 0.32 mmol, 1.0 eq.) in THF (4 mL) and H₂O (4 mL) was added LiOH (54 mg, 1.29 mmol, 4.0 eq.). The mixture was stirred at 25 °C for 2 h. The mixture was diluted with water (20 mL) and adjusted to pH 6 with 1M HCl. The mixture was extracted with EtOAc (2 x 100 mL). The organic layers were combined and washed with brine (100 mL), dried over Na₂SO₄ and concentrated under vacuum to afford 2-(1-(7-((2-(trimethylsilyl)ethoxy)methyl)-7H-pyrrolo(2,3-d)pyrimidin-2-yl)piperidin-4-yl)acetic acid (110 mg, 87.7%) as a yellow solid.
LCMS: *(ESI)* (M+H)⁺ = 390.9.

### Step D: Synthesis of (S)-N-(2-((6-oxo-5-(trifluoromethyl)-1-((2-(trimethylsilyl)ethoxy) methyl)-1,6-dihydropyridazin-4-yl)amino)propoxy)-2-(1-(7-((2-(trimethylsilyl)ethoxy) methyl)-7H-pyrrolo(2,3-d)pyrimidin-2-yl)piperidin-4-yl)acetamide

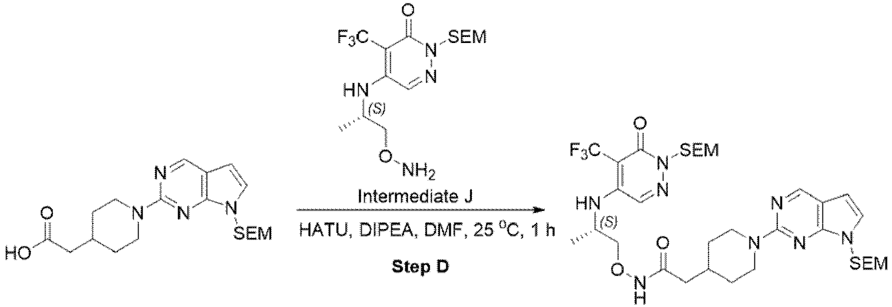

To a mixture of 2-(1-(7-((2-(trimethylsilyl)ethoxy)methyl)-7H-pyrrolo(2,3-d) pyrimidin-2-yl)piperidin-4-yl)acetic acid (110 mg, 0.28 mmol, 1.0 eq.) in DMF (5 mL) were added (S)-5-((1-(aminooxy)propan-2-yl)amino)-4-(trifluoromethyl)-2-((2-(trimethylsilyl)ethoxy)methyl)pyridazin-3(2H)-one (119 mg, 0.31 mmol, 1.1 eq.), DIPEA (0.14 mL, 0.85 mmol, 3.0 eq.) and HATU (129 mg, 0.34 mmol, 1.2 eq.). The mixture was stirred at 25 °C for 1 h. The reaction mixture was quenched with water (100 mL) and extracted with EtOAc (2 x 150 mL). The organic layers were combined and washed with brine (2 x 80 ml), dried over Na₂SO₄ and concentrated under vacuum. The residue was purified by silica gel chromatography (Petroleum ether/EtOAc= 1/1; V/V) to afford (S)-N-(2-((6-oxo-5-(trifluoromethyl)-1-((2-(trimethylsilyl)ethoxy) methyl)-1,6-dihydropyridazin-4-yl)amino)propoxy)-2-(1-(7-((2-(trimethylsilyl)ethoxy) methyl)-7H-pyrrolo(2,3-d)pyrimidin-2-yl)piperidin-4-yl)acetamide (110 mg, 51.7%) as a light yellow oil.
LCMS: *(ESI)* (M+H)⁺ = 755.4.

### Step E: synthesis of (S)-2-(1-(7H-pyrrolo(2,3-d)pyrimidin-2-yl)piperidin-4-yl)-N-(2-((6-oxo-5-(trifluoromethyl)-1,6-dihydropyridazin-4-yl)amino)propoxy)acetamide

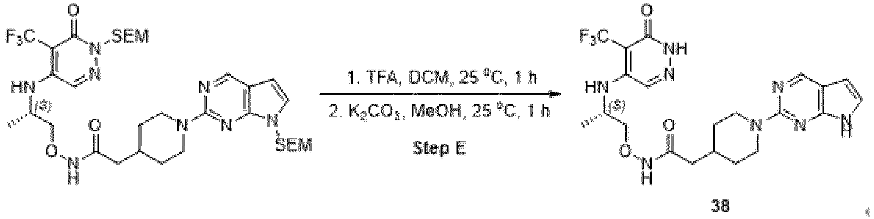

To a mixture of (S)-N-(2-((6-oxo-5-(trifluoromethyl)-1-((2-(trimethylsilyl)ethoxy) methyl)-1,6-dihydropyridazin-4-yl)amino)propoxy)-2-(1-(7-((2-(trimethylsilyl)ethoxy) methyl)-7H-pyrrolo(2,3-d)pyrimidin-2-yl)piperidin-4-yl)acetamide (95 mg, 0.13 mmol, 1.0 eq.) in DCM (6 mL) was added TFA (2 mL). The mixture was stirred at 25 °C for 1 h. The mixture was quenched with saturated aqueous NaHCO₃ aqueous solution (50 mL) and extracted with DCM (3 x 100 mL). The organic layers were combined and dried over Na₂SO₄, concentrated under vacuum. The residue was redissolved in MeOH (3 mL) and K₂CO₃ (81 mg, 0.45 mmol) was added. The mixture was stirred at 25 °C for another 1 h. The mixture was filtered and filtrate was purified by prep-HPLC to afford (S)-2-(1-(7H-pyrrolo(2,3-d)pyrimidin-2-yl)piperidin-4-yl)-N-(2-((6-oxo-5-(trifluoromet hyl)-1,6-dihydropyridazin-4-yl)amino)propoxy)acetamide (8.03 mg, 12.9%) as a white solid.
LCMS: *(ESI)* (M+H)⁺ = 495.2.
¹H NMR: (400 MHz, DMSO-*d₆*) *δ* 12.49 (s, 1H), 11.26 (s, 1H), 11.11 (s, 1H), 8.57 (s, 1H), 7.91 (s, 1H), 7.05 (dd, *J* = 3.6, 2.0 Hz, 1H), 6.68 - 6.55 (m, 1H), 6.29 (dd, *J* = 3.6, 2.0 Hz, 1H), 4.63 (d, *J* = 12.8 Hz, 2H), 4.27 - 4.13 (m, 1H), 3.95 - 3.80 (m, 2H), 2.91 - 2.76 (m, 2H), 2.03 - 1.85 (m, 3H), 1.66 (d, *J=* 12.4 Hz, 2H), 1.19 (d, *J=* 6.8 Hz, 3H), 1.16 - 1.04 (m, 2H).

### Example 39:

### (S)-2-(1-(6-fluoroquinazolin-2-yl)piperidin-4-yl)-N-(2-((6-oxo-5-(trifluoromethyl)-1,6-dihydropyridazin-4-yl)amino)propoxy)acetamide

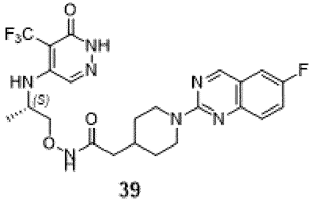

### Step A: synthesis of 2-chloro-6-fluoroquinazolin

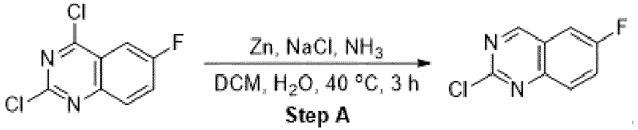

To a solution of 2,4-dichloro-6-fluoroquinazoline (350 mg, 1.61 mmol, 1.0 eq.) in DCM (7 mL) were added NH3·H₂O (1 mL) and saturated NaCl aqueous solution (7 mL). Then Zn powder (316 mg, 4.84 mmol, 3.0 eq.) was added to the mixture. The mixture was stirred at 40 °C for 3 h under N₂ atmosphere. The mixture was cooled to room temperature and diluted with DCM (15 mL). The organic was washed with saturated NH₄Cl aqueous solution (2 x 10 mL), dried over Na₂SO₄ and concentrated under vacuum. The residue was purified by column chromatography (Petroleum ether/EtOAc= 2/1; V/V) to afford 2-chloro-6-fluoroquinazoline (150 mg, 50.9%) as a yellow solid.
¹H NMR: (400 MHz, CDCl₃) *δ* 9.26 (s, 1H), 8.04 - 7.97 (m, 1H), 7.77 - 7.69 (m, 1H), 7.56 (dd, *J* = 7.6, 2.4 Hz, 1H).

### Step B: synthesis of methyl 2-(1-(6-fluoroquinazolin-2-yl)piperidin-4-yl)acetate

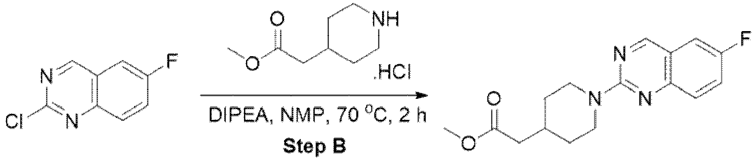

To a mixture of methyl 2-(piperidin-4-yl)acetate hydrochloride (145 mg, 0.75 mmol, 1.05 eq.) and DIPEA (276 mg, 2.13 mmol, 3.0 eq.) in NMP (8 mL) was added 2-chloro-6-fluoroquinazoline (130 mg, 0.71 mmol, 1.0 eq.). The mixture was stirred at 70 °C for 2 h. The mixture was cooled to room temperature and quenched with water (30 mL), extracted with EtOAc (3 x 30 mL). The organic layers were combined and washed with brine (2 x 30 mL), dried over Na₂SO₄ and concentrated under vacuum. The residue was purified by column chromatography (Petroleum ether/EtOAc= 2/1; V/V) to afford methyl 2-(1-(6-fluoroquinazolin-2-yl)piperidin-4-yl)acetate (150 mg, 69.4%) as a white solid.
LCMS: *(ESI)* (M+H)⁺ = 304.2.
¹H NMR: (400 MHz, DMSO-*d₆*) *δ* 9.17 (s, 1H), 7.69 - 7.58 (m, 2H), 7.57 - 7.49 (m, 1H), 4.84 - 4.73 (m, 2H), 3.61 (s, 3H), 2.96 (td, *J* = 12.8, 2.4 Hz, 2H), 2.29 (d, *J* = 7.2 Hz, 2H), 1.95 - 2.06 (m, 1H), 1.80 - 1.70 (m, 2H),1.18 - 1.13 (m, 2H).

### Step C: synthesis of 2-(1-(6-fluoroquinazolin-2-yl)piperidin-4-yl)acetic acid

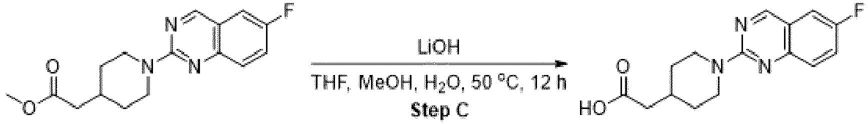

To a mixture of methyl 2-(1-(6-fluoroquinazolin-2-yl)piperidin-4-yl)acetate (145 mg, 0.48 mmol, 1.0 eq.) in a mixed solution of THF (2 mL), MeOH (2 mL) and H₂O (1 mL) was added lithium hydroxide (80 mg, 1.92 mmol, 4.0 eq.). The mixture was stirred at 50 °C for 12 h. The mixture was cooled to room temperature diluted with water (6 mL). The mixture was adjusted to pH 6 with 1M HCl. The mixture was extracted with EtOAc (3 x 10 mL). The organic layers were combined and washed with brine (10 mL), dried over Na₂SO₄ and concentrated under vacuum to afford 2-(1-(6-fluoroquinazolin-2-yl)piperidin-4-yl)acetic acid (110 mg, 79.5%) as a white solid.
LCMS: *(ESI)* (M+H)⁺ = 290.2.

### Step D: synthesis of (S)-2-(1-(6-fluoroquinazolin-2-yl)piperidin-4-yl)-N-(2-((6-oxo-5-(trifluoromethyl)-1-((2-(trimethylsilyl)ethoxy)methyl)-1,6-dihydropyridazin-4-yl)amino )propoxy)acetamide

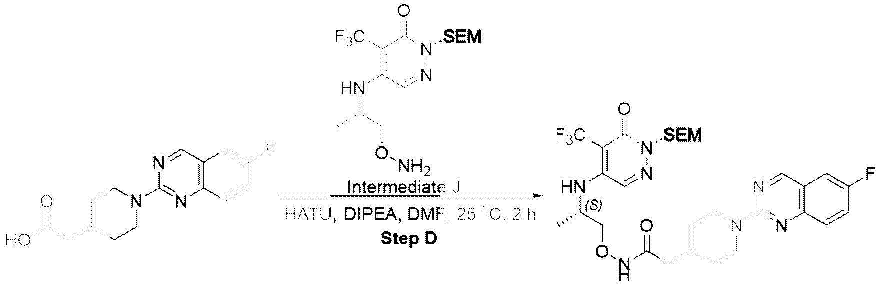

To a mixture of 2-(1-(6-fluoroquinazolin-2-yl)piperidin-4-yl)acetic acid (100 mg, 0.35 mmol, 1.0 eq.) and (S)-5-((1-(aminooxy)propan-2-yl)amino)-4-(trifluoromethyl) -2-((2-(trimethylsilyl)ethoxy)methyl)pyridazin-3(2H)-one (159 mg, 0.42 mmol, 1.2 eq.) in DMF (5 mL) were added DIPEA (134 mg, 1.05 mmol, 3.0 eq.) and HATU (158 mg, 0.42 mmol, 1.2 eq.). The mixture was stirred at 25 °C for 2 h. The mixture was diluted with water (10 mL) and extracted with EtOAc (3 x 15 mL). The organic layers were combined washed with saturated NH₄Cl aqueous solution (2 x 20 mL), dried over Na₂SO₄ and concentrated under vacuum. The residue was purified by silica gel chromatography (EtOAc/Petroleum ether= 1/1; V/V) to afford (S)-2-(1-(6-fluoroquinazolin-2-yl)piperidin-4-yl)-N-(2-((6-oxo-5-(trifluoromethyl)-1-((2 -(trimethylsilyl)ethoxy)methyl)-1,6-dihydropyridazin-4-yl)amino)propoxy)acetamide (195 mg, 86.3 %) as a yellow solid.
LCMS: *(ESI)* (M+H)⁺ = 654.2.

### Step E: synthesis of (S)-2-(1-(6-fluoroquinazolin-2-yl)piperidin-4-yl)-N-(2-((6-oxo-5-(trifluoromethyl)-1,6-dihydropyridazin-4-yl)amino)propoxy)acetamide

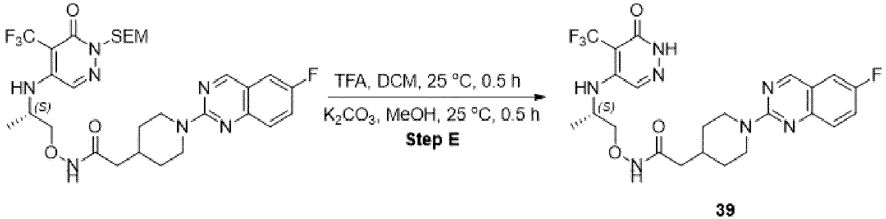

To a mixture of (S)-2-(1-(6-fluoroquinazolin-2-yl)piperidin-4-yl)-N-(2-((6-oxo-5-(trifluoromethyl)-1-((2-(trimethylsilyl)ethoxy)methyl)-1,6-dihydropyridazin-4-yl)amino )propoxy)acetamide (140 mg, 0.21 mmol, 1.0 eq.) in DCM (4.5 mL) was added TFA (1.5 mL). The mixture was stirred at 25 °C for 0.5 h. The mixture was concentrated vacuum. MeOH (4 mL) and K₂CO₃ (176 mg, 1.26 mmol, 6.0 eq.) were added to the resulting mixture. The mixture was stirred at 25 °C for another 0.5 h. The mixture was filtered and the filtrate was purified by prep-HPLC to afford (S)-2-(1-(6-fluoroquinazolin-2-yl)piperidin-4-yl)-N-(2-((6-oxo-5-(trifluoromethyl)-1,6-dihydropyridazin-4-yl)amino)propoxy)acetamide (14.73 mg, 13.1%) as a white solid. LCMS: *(ESI)* (M+H)⁺ = 524.2.
¹H NMR: (400 MHz, DMSO-*d₆*) *δ* 12.52 (s, 1H), 11.15 (s, 1H), 9.20 (s, 1H), 7.93 (s, 1H), 7.70 - 7.61 (m, 2H), 7.58 - 7.35 (m, 1H), 6.69 - 6.58 (m, 1H), 4.78 (d, *J* = 13.2 Hz, 2H), 4.30 - 4.16 (m, 1H), 4.00 - 3.81 (m, 2H), 2.97 (t, *J=* 12.0 Hz, 2H), 2.07 - 1.99 (m, 1H), 1.95 (d, *J* = 6.8 Hz, 2H), 1.74 (d, *J* = 11.2 Hz, 2H), 1.22 (d, *J* = 6.4 Hz, 3H), 1.18 - 1.07 (m, 2H).

### Example 40:

### (S)-2-(1-(4-(hydroxymethyl)-5-(trifluoromethyl)pyridin-2-yl)piperidin-4-yl)-N-(2-((6-oxo-5-(trifluoromethyl)-1,6-dihydropyridazin-4-yl)amino)propoxy)acetamide

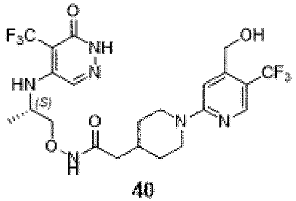

### Step A: synthesis of 2-(4-(2-methoxy-2-oxoethyl)piperidin-1-yl)-5-(trifluoromethyl) isonicotinic acid

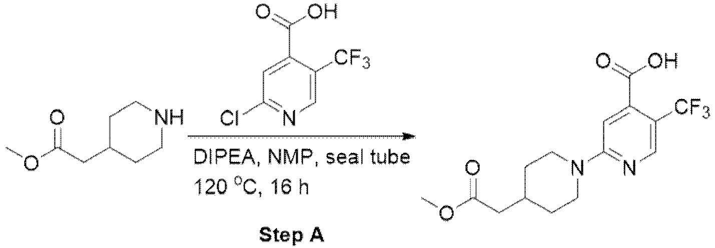

To a solution of methyl 2-(piperidin-4-yl)acetate (380 mg, 1.97 mmol, 1.5 eq.) in NMP (8 mL) were added DIPEA (1 g, 7.92 mmol, 6.0 eq.) and 2-chloro-5-(trifluoromethyl)isonicotinic acid (300 mg, 1.32 mmol, 1 eq.), the resulting mixture was stirred at 120 °C for 16 h in seal tube. The mixture was cooled to room temperature and diluted with water (80 mL). The mixture was extracted with EtOAc (3 x 50 mL). The water phase was adjusted pH 6 by 2M HCl, extracted with EtOAc (3 x 50 mL).

The organic layers were combined, dried over Na₂SO₄ and concentrated under reduced pressure. The residue was purified by column chromatography (DCM/MeOH= 95/5, V/V) to afford 2-(4-(2-methoxy-2-oxoethyl)piperidin-1-yl)-5-(trifluoromethyl) isonicotinic acid (340 mg, 51.8%) as a colorless oil.
LCMS: *(ESI)* (M+H)⁺ = 347.1.

### Step B: Synthesis of methyl 2-(1-(4-(hydroxymethyl)-5-(trifluoromethyl)pyridin-2-yl) piperidin-4-yl)acetate

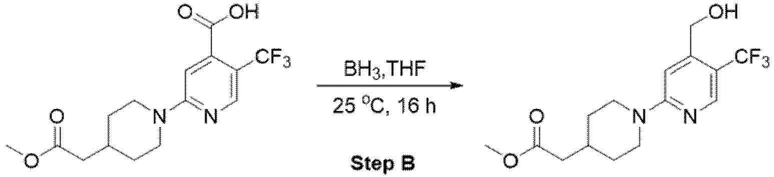

To a solution of 2-(4-(2-methoxy-2-oxoethyl)piperidin-1-yl)-5-(trifluoromethyl) isonicotinic acid (280 mg, 0.81 mmol, 1.0 eq.) in THF (3mL) was added BH₃ in THF (1.62 mL, 1.62 mmol, 2.0 eq.). The mixture was stirred for 16 h at 25 °C. The mixture was quenched by MeOH. The mixture was concentrated under vacuum. The residue was purified by silica gel chromatography (Petroleum ether/EtOAc= 2/1, V/V) to afford methyl 2-(1-(4-(hydroxymethyl)-5-(trifluoromethyl)pyridin-2-yl)piperidin-4-yl)acetate (130 mg, 48.6% yield) as a colorless oil.
LCMS: *(ESI)* (M+H)⁺ = 333.1.
¹H NMR: (400 MHz, DMSO-*d₆*) *δ* 8.26 (s, 1H), 7.04 (s, 1H), 5.56 (t, J = 5.7 Hz, 1H), 4.55 (d, J = 5.6 Hz, 2H), 4.45 - 4.31 (m, 2H), 3.60 (s, 3H), 2.94 (td, J = 12.8, 2.6 Hz, 2H), 2.28 (d, J = 7.0 Hz, 2H), 2.08 - 1.96 (m, 1H), 1.73 (dd, J = 13.7, 3.6 Hz, 2H), 1.11 - 1.18 (m, 2H).

### Step C: Synthesis of 2-(1-(4-(hydroxymethyl)-5-(trifluoromethyl)pyridin-2-yl)piperidin-4-yl)acetic acid

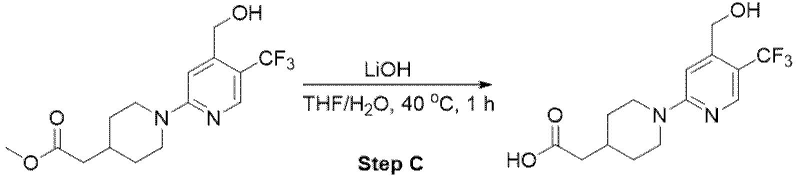

To a solution of methyl 2-(1-(4-(hydroxymethyl)-5-(trifluoromethyl)pyridin-2-yl) piperidin-4-yl)acetate (130 mg, 0.392 mmol, 1.0 eq.) in THF (3 mL) and water (3 mL) was added LiOH (38 mg, 1.568 mmol, 4.0 eq.). The mixture was stirred at 40 °C for 1 h. The mixture was diluted with water (15 mL) and extracted with EtOAc (3 x 10 mL). The water layer was adjusted pH 6 with 2M HCl. The mixture was extracted with EtOAc (3 x 15 mL), dried over Na₂SO₄ and concentrated under vacuum to afford 2-(1-(4-(hydroxymethyl)-5-(trifluoromethyl)pyridin-2-yl)piperidin-4-yl)acetic acid (90 mg, 72.5% yield) as a colorless oil.
LCMS: *(ESI)* (M+H)⁺ = 319.2.

### Step D: synthesis of (S)-2-(1-(4-(hydroxymethyl)-5-(trifluoromethyl)pyridin-2-yl) piperidin-4-yl)-N-(2-((6-oxo-5-(trifluoromethyl)-1-((2-(trimethylsilyl)ethoxy)methyl)-1, 6-dihydropyridazin-4-yl)amino)propoxy)acetamide

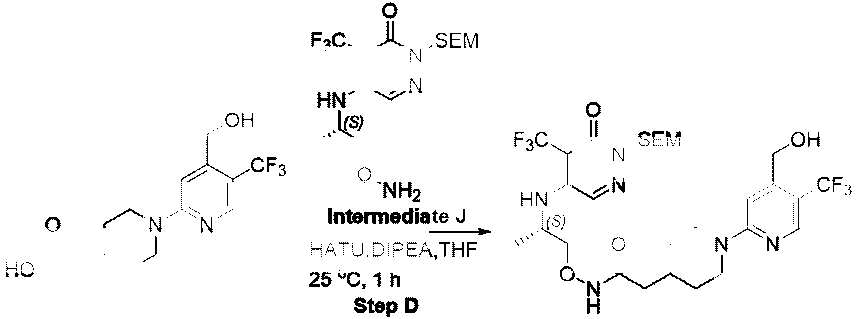

To a solution of 2-(1-(4-(hydroxymethyl)-5-(trifluoromethyl)pyridin-2-yl) piperidin-4-yl)acetic acid (92 mg, 0.24 mmol, 1 eq.) in THF (3.6 mL) were added DIPEA (62 mg, 0.48 mmol, 2.0 eq.) and HATU (137 mg, 0.36 mmol, 1.5 eq.) followed by dropwise addition of (S)-5-((1-(aminooxy)propan-2-yl)amino)-4-(trifluoromethyl)-2-((2-(trimethylsilyl)ethoxy)methyl)pyridazin-3(2H)-one (80 mg, 0.252 mmol, 1.05 eq.). The resulting mixture was stirred at 25 °C for 1 h. The reaction mixture was quenched with water (10 mL) and extracted with EtOAc (3 x 10 mL). The organic layers were combined and washed with brine (10 mL), dried over Na₂SO₄ and concentrated under vacuum. The residue was purified by silica gel chromatography (Petroleum ether/EtOAc= 3/7, V/V) to afford (S)-2-(1-(4-(hydroxymethyl)-5-(trifluoromethyl) pyridin-2-yl)piperidin-4-yl)-N-(2-((6-oxo-5-(trifluoromethyl)-1-((2-(trimethylsilyl)etho xy)methyl)-1,6-dihydropyridazin-4-yl)amino)propoxy)acetamide (120 mg, 62.1% yield) as a colorless oil.
LCMS: *(ESI)* (M+H)⁺ = 683.4.

### Step E: synthesis of (S)-2-(1-(4-(hydroxymethyl)-5-(trifluoromethyl)pyridin-2-yl) piperidin-4-yl)-N-(2-((6-oxo-5-(trifluoromethyl)-1,6-dihydropyridazin-4-yl)amino)prop oxy)acetamide

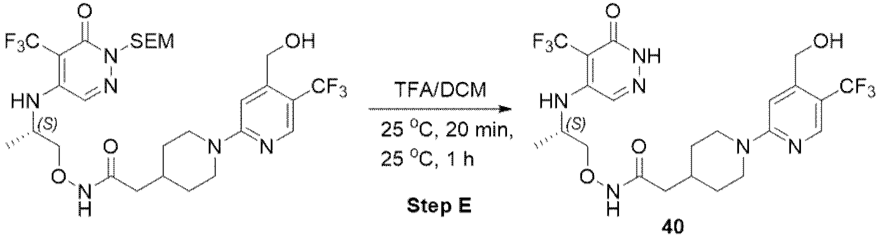

To a solution of (S)-2-(1-(4-(hydroxymethyl)-5-(trifluoromethyl)pyridin-2-yl) piperidin-4-yl)-N-(2-((6-oxo-5-(trifluoromethyl)-1-((2-(trimethylsilyl)ethoxy)methyl)-1, 6-dihydropyridazin-4-yl)amino)propoxy)acetamide (120 mg, 0.176 mmol, 1.0 eq.) in DCM (2 mL) was added TFA (0.2 mL). The resulting mixture was stirred at 25 °C for 20 min. The mixture was neutralized with saturated NaHCO₃ aqueous solution (5 mL) and extracted with DCM (3 x 10 mL). The organic layers were combined and dried over Na₂SO₄, concentrated under vacuum. The residue was redissolved in MeOH (2 mL) and K₂CO₃ (83 mg, 0.6 mmol, 3.0 eq.) was added. The mixture was stirred at 25 °C for another 1 h. The reaction mixture was filtered and the filtrate was purified by silica gel chromatography (Petorleum ether/EtOAc= 1/100, V/V) to afford (S)-2-(1-(4-(hydroxymethyl)-5-(trifluoromethyl)pyridin-2-yl)piperidin-4-yl)-N-(2-((6-o xo-5-(trifluoromethyl)-1,6-dihydropyridazin-4-yl)amino)propoxy)acetamide (25.5 mg, 28.1% yield) as a white solid.
LCMS: *(ESI)* (M+H)⁺ = 553.4.
¹H NMR: (400 MHz, DMSO) *δ* 8.28 (s, 1H), 7.93 (s, 1H), 7.06 (s, 1H), 6.64 (s, 1H), 4.57 (s, 2H), 4.39 (d, J = 13.1 Hz, 2H), 4.30 - 4.17 (m, 1H), 3.99 - 3.79 (m, 2H), 2.95 (t, J = 13.2 Hz, 2H), 2.13 - 1.85 (m, 3H), 1.73 (d, J = 15.1 Hz, 2H), 1.21 (d, J = 6.4 Hz, 3H), 1.18 - 1.06 (m, 2H).

### Example 41:

### (S)-N-(2-((6-oxo-5-(trifluoromethyl)-1,6-dihydropyridazin-4-yl)amino)propoxy)-2-(1-(t hiazol-2-yl)piperidin-4-yl)acetamide

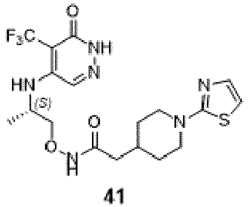

### Step A: synthesis of methyl 2-(1-(thiazol-2-yl)piperidin-4-yl)acetate

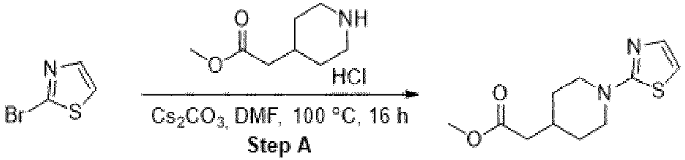

To a solution of 2-bromothiazole (800 mg, 4.88 mmol, 1.0 eq.) in DMF (16 mL) were added Cs2CO3 (4.77 g, 14.63 mmol, 3.0 eq.) and methyl 2-(piperidin-4-yl)acetate hydrochloride (1.13 g, 5.85 mmol, 1.2 eq.). The mixture was stirred at 100 °C for 16 h. The mixture was cooled to room temperature and diluted with water (50 mL). The mixture was extracted with EtOAc (2 x 60 mL). The organic layers were combined and washed with brine (2 x 60 mL), dried over Na₂SO₄ and concentrated under vacuum. The residue was purified by silica gel chromatography (Petroleum ether/EtOAc= 4/1; V/V) to afford methyl 2-(1-(thiazol-2-yl)piperidin-4-yl)acetate (250 mg, 21.3%) as a light yellow solid.
LCMS: *(ESI)* (M+H)⁺ = 241.2.

### Step B: synthesis of 2-(1-(thiazol-2-yl)piperidin-4-yl)acetic acid

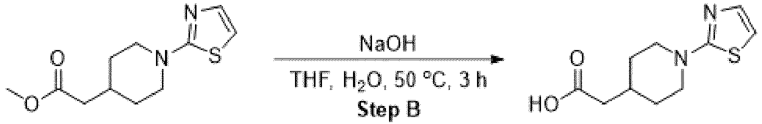

To a mixture of methyl 2-(1-(thiazol-2-yl)piperidin-4-yl)acetate (150 mg, 0.62 mmol, 1.0 eq.) in THF (3 mL) and H₂O (1 mL) was added NaOH (75 mg, 1.87 mmol, 3.0 eq.). The mixture was stirred at 50 °C for 3 h. The mixture was cooled to room temperature and diluted with water (30 mL), adjusted pH 6 with 1M HCl. The mixture was extracted with EtOAc (2 x 50 mL). The organic layers were combined and dried over Na₂SO₄, concentrated under vacuum to afford 2-(1-(thiazol-2-yl)piperidin-4-yl) acetic acid (140 mg, 99.1%) as a white solid.
LCMS: *(ESI)* (M+H)⁺ = 227.2.
¹H NMR: (400 MHz, CDCl₃) *δ* 7.10 (d, J = 3.7 Hz, 1H), 6.46 (d, J = 3.6 Hz, 1H), 3.99 - 3.86 (m, 2H), 3.62 (s, 3H), 2.96 (td, J = 12.7, 2.9 Hz, 2H), 2.22 (d, J = 7.0 Hz, 2H), 2.04 - 1.89 (m, 1H), 1.80 - 1.70 (m, 2H), 1.31 (qd, J = 12.4, 4.4 Hz, 2H).

### Step C: Synthesis of (S)-N-(2-((6-oxo-5-(trifluoromethyl)-1-((2-(trimethylsilyl)ethoxy) methyl)-1,6-dihydropyridazin-4-yl)amino)propoxy)-2-(1-(thiazol-2-yl)piperidin-4-yl)ac etamide

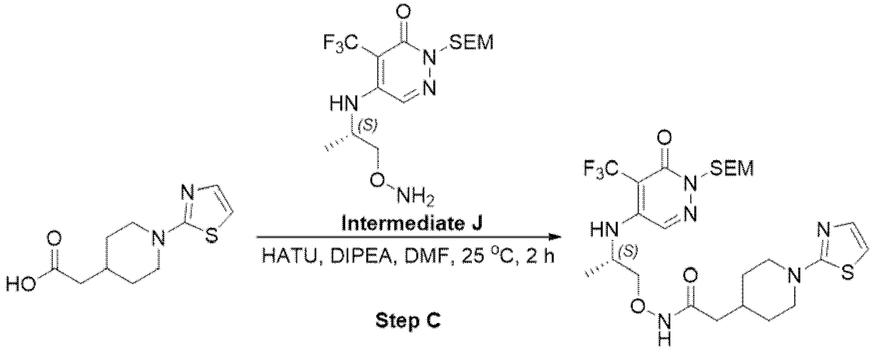

To a mixture of 2-(1-(thiazol-2-yl)piperidin-4-yl)acetic acid (142 mg, 0.63 mmol, 1.2 eq.) in DMF (3 mL) were added (S)-5-((1-(aminooxy)propan-2-yl)amino)-4-(trifluoromethyl)-2-((2-(trimethylsilyl)ethoxy)methyl)pyridazin-3(2H)-one (200 mg, 0.52 mmol, 1.0 eq.), DIPEA (203 mg, 1.57 mmol, 3.0 eq.) and HATU (239 mg, 0.63 mmol, 1.2 eq.). The mixture was stirred at 25 °C for 2 h. The mixture was quenched with water (30 mL) and extracted with EtOAc (2 x 50 mL). The organic layers were combined and washed with brine (2 x 50 mL), dried over Na₂SO₄ and concentrated under vacuum. The residue was purified by silica gel chromatography (Petroleum ether/EtOAc= 3/7; V/V) to afford (S)-N-(2-((6-oxo-5-(trifluoromethyl)-1-((2-(trimethylsilyl)ethoxy)methyl)-1,6-dihydropyridazin-4-yl)amino)propoxy)-2-(1-(thiazol -2-yl)piperidin-4-yl)acetamide (150 mg, 48.6%) as a light yellow solid.
LCMS: *(ESI)* (M+H)⁺ = 591.2.

### Step D: synthesis of (S)-N-(2-((6-oxo-5-(trifluoromethyl)-L6-dihydropyridazin-4-yl) amino)propoxy)-2-(1-(thiazol-2-yl)piperidin-4-yl)acetamide

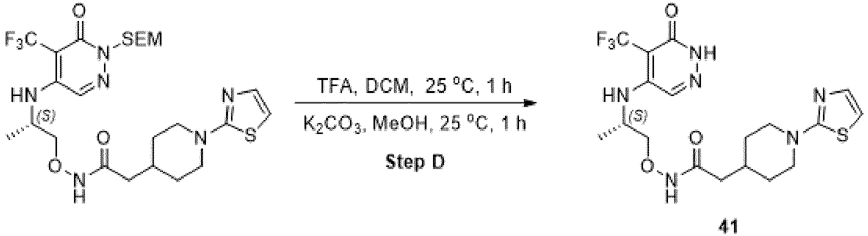

To a mixture of (S)-N-(2-((6-oxo-5-(trifluoromethyl)-1-((2-(trimethylsilyl)ethoxy) methyl)-1,6-dihydropyridazin-4-yl)amino)propoxy)-2-(1-(thiazol-2-yl)piperidin-4-yl)ac etamide (150 mg, 0.25 mmol, 1.0 eq.) in DCM (6 mL) was added TFA (1.5 mL). The mixture was stirred at 25 °C for 1 h. The mixture was concentrated under vacuum. The residue was redissolved in MeOH (3 mL) and K₂CO₃ (70 mg, 0.51 mmol) was added. The mixture was stirred at 25 °C for another 1 h. The mixture was filtered and filtrate was purified by prep-HPLC to afford (S)-N-(2-((6-oxo-5-(trifluoromethyl)-1,6-dihydropyridazin-4-yl)amino)propoxy)-2-(1-(thiazol-2-yl)piperidin-4-yl)acetamide (17.56 mg, 15.0%) as a white solid.
LCMS: *(ESI)* (M+H)⁺ = 461.2.
¹H NMR: (400 MHz, DMSO-*d₆*) *δ* 12.49 (s, 1H), 11.14 (s, 1H), 7.91 (s, 1H), 7.12 (d, *J* = 3.6 Hz, 1H), 6.79 (d, *J* = 3.6 Hz, 1H), 6.65 - 6.56 (m, 1H), 4.25 - 4.15 (m, 1H), 3.94 - 3.79 (m, 4H), 2.97 (t, *J* = 12.0 Hz, 2H), 1.98 - 1.83 (m, 3H), 1.69 (d, *J* = 12.8 Hz, 2H), 1.26 - 1.14 (m, 5H).

### Example 42:

### (S)-N-(2-((6-oxo-5-(trifluoromethyl)-1,6-dihydropyridazin-4-yl)amino)propoxy)-2-(1-( 5-oxo-6,7-dihydro-5H-cyclopenta(d)pyrimidin-2-yl)piperidin-4-yl)acetamide

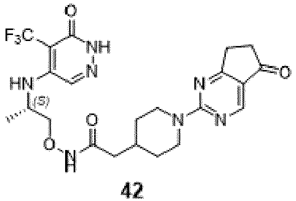

### Step A: synthesis of 2-((dimethylamino)methylene)cyclopentane-1,3-dione

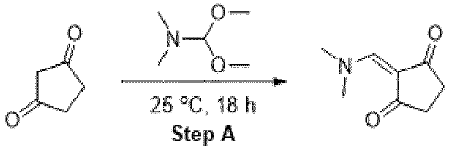

A mixture of cyclopentane-1,3-dione (5.00 g, 50.97 mmol, 1.0 eq.) in N,N-Dimethylformamide dimethyl acetal (10 mL) was stirred at 25 °C for 18 h. The mixture was concentrated under vacuum. The residue was purified by silica gel chromatography (DCM/MeOH= 9/1; V/V) to afford 2-((dimethylamino)methylene) cyclopentane-1,3-dione (4.00 g, 49.1%) as a yellow solid.
LCMS: *(ESI)* (M+H)⁺ = 154.1.
¹H NMR: (400 MHz, CDCl₃) *δ* 7.47 (s, 1H), 3.76 (s, 3H), 3.39 (s, 3H), 2.54 (s, 4H).

### Step B: synthesis of 2-(methylthio)-6,7-dihydro-5H-cvclopenta(d)pyrimidin-5-one

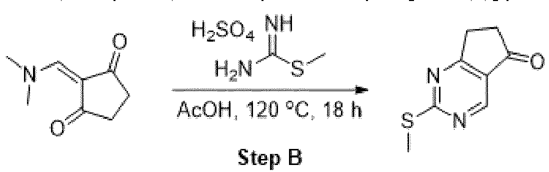

To a mixture of 2-((dimethylamino) methylene)cyclopentane-1,3-dione (3.00 g, 19.59 mmol, 1.0 eq.) in AcOH (15 mL) was added 2-Methyl-2-thiopseudourea sulfate (4.06 g, 21.55 mmol, 1.1 eq.). The mixture was stirred at 120 °C for 18 h. The mixture was cooled to room temperature and the mixture was neutralized with saturated NaHCOs aqueous solution to pH 7. The mixture was extracted with EtOAc (2 x 250 mL). The organic layers were combined and washed with brine (100 mL), dried over Na₂SO₄ and concentrated under vacuum. The residue was purified by column chromatography (Petroleum ether/EtOAc= 7/3; V/V) to afford 2-(methylthio)-6,7-dihydro-5H-cyclopenta(d)pyrimidin-5-one (370 mg, 10.5%) as a white solid.
LCMS: *(ESI)* (M+H)⁺ = 181.4.
¹H NMR: (400 MHz, CDCl₃) *δ* 8.81 (s, 1H), 3.21 - 3.15 (m, 2H), 2.77 - 2.71 (m, 2H), 2.65 (s, 3H).

### Step C: synthesis of 2-(methylsulfinyl)-6,7-dihydro-5H-cyclopenta(d)pyrimidin-5-one

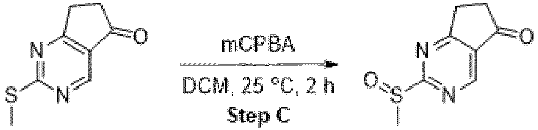

To a mixture of 2-(methylthio)-6,7-dihydro-5H-cyclopenta(d)pyrimidin-5-one (200 mg, 1.11 mmol, 1.0 eq.) in DCM (10 mL) was added mCPBA (226 mg, 1.11 mmol, 1.0 eq.). The mixture was stirred at 25 °C for 2 h. The mixture was quenched with water (100 mL) and extracted with DCM (3 x 100 mL). The organic layers were combined and washed with brine (100 mL), dried over Na₂SO₄ and concentrated under vacuum to afford 2-(methylsulfinyl)-6,7-dihydro-5H-cyclopenta(d)pyrimidin-5-one (260 mg, crude) as a white solid .
LCMS: *(ESI)* (M+H)⁺ = 197.0.

### Step D: synthesis of methyl 2-(1-(5-oxo-6,7-dihydro-5H-cyclopenta(d)pyrimidin-2-yl) piperidin-4-yl)acetate

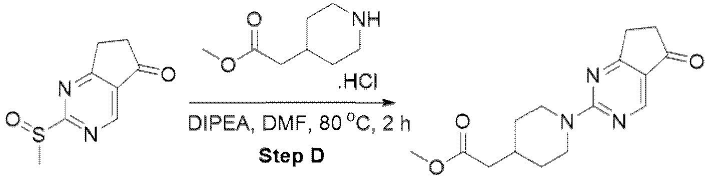

To a mixture of 2-(methylsulfinyl)-6,7-dihydro-5H-cyclopenta(d)pyrimidin-5-one (500 mg, 2.36 mmol, 1.0 eq.) in DMF (8 mL) were added methyl 2-(piperidin-4-yl)acetate hydrochloride (500 mg, 2.59 mmol, 1.1 eq.) and DIPEA (1.17 mL, 7.07 mmol, 3.0 eq.). The mixture was stirred at 80 °C for 2 h. The mixture was cooled to room temperature and diluted with water (200 mL). The mixture was extracted with EtOAc (2 x 100 mL). The organic layers were combined and washed with brine (100 mL), dried over Na₂SO₄ and concentrated under vacuum. The residue was purified by column chromatography (Petroleum ether/EtOAc= 7/3; V/V) to afford methyl 2-(1-(5-oxo-6,7-dihydro-5H-cyclopenta(d)pyrimidin-2-yl)piperidin-4-yl)acetate (270 mg, 39.6%) as a yellow solid.
LCMS: *(ESI)* (M+H)⁺ = 289.9.
¹H NMR: (400 MHz, DMSO-*d₆*) *δ* 8.62 (s, 1H), 4.82 (d, *J* = 13.2 Hz, 2H), 3.62 (s, 3H), 3.05 (t, *J* = 12.4 Hz, 2H), 2.96 - 2.91 (m, 2H), 2.56 - 2.54 (m, 2H), 2.31 (d, *J* = 7.2 Hz, 2H), 2.12 - 2.00 (m, 1H), 1.78 (d, *J=* 12.0 Hz, 2H), 1.24 - 1.08 (m, 2H).

### Step E: synthesis of 2-(1-(5-oxo-6,7-dihydro-5H-cyclopenta(d)pyrimidin-2-yl) piperidin-4-yl)acetic acid

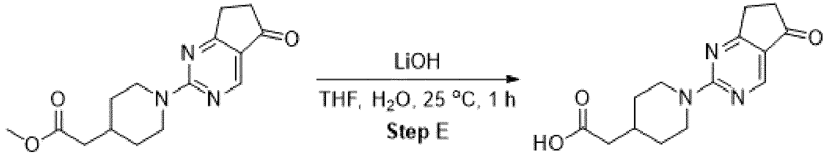

To mixture of methyl 2-(1-(5-oxo-6,7-dihydro-5H-cyclopenta(d)pyrimidin-2-yl)piperidin-4-yl)acetate (50 mg, 0.17 mmol, 1.0 eq.) in THF (2 mL) and H₂O (1 mL) was added LiOH (29 mg, 0.69 mmol, 4.0 eq.). The mixture was stirred at 25 °C for 1 h. The mixture was diluted with water (30 mL) and adjusted to pH 6 with 1M HCl, extracted with EtOAc (2 x 50 mL). The organic layers were combined and washed with brine (100 mL), dried over Na₂SO₄ and concentrated under vacuum to afford 2-(1-(5-oxo-6,7-dihydro-5H-cyclopenta(d) pyrimidin-2-yl)piperidin-4-yl)acetic acid (45 mg, crude) as a yellow solid.
LCMS: *(ESI)* (M+H)⁺ = 276.2.

### Step F: Synthesis of (S)-N-(2-((6-oxo-5-(trifluoromethyl)-1-((2-(trimethylsilyl) ethoxy)methyl)-1,6-dihydropyridazin-4-yl)amino)propoxy)-2-(1-(5-oxo-6,7-dihydro-5H -cyclopenta(d)pyrimidin-2-yl)piperidin-4-yl)acetamide

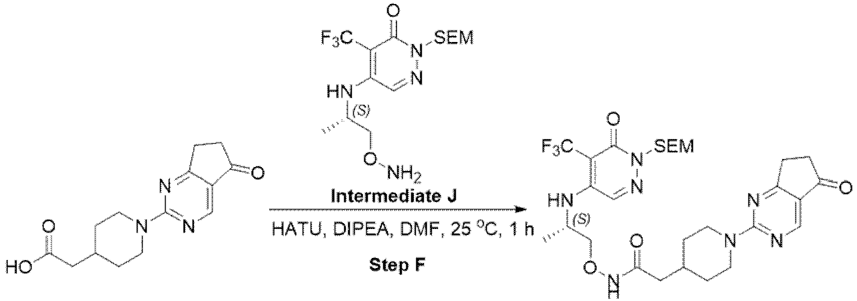

To a mixture of 2-(1-(5-oxo-6,7-dihydro-5H-cyclopenta(d)pyrimidin-2-yl) piperidin-4-yl)acetic acid (45 mg, 0.16 mmol, 1.0 eq.) in DMF (2 mL) were added (S)-5-((1-(aminooxy)propan-2-yl)amino)-4-(trifluoromethyl)-2-((2-(trimethylsilyl)ethox y)methyl)pyridazin-3(2H)-one (69 mg, 0.18 mmol, 1.1 eq.), DIPEA (63 mg, 0.49 mmol, 3.0 eq.) and HATU (75 mg, 0.20 mmol, 1.2 eq.). The mixture was stirred at 25 °C for 1 h. The reaction mixture was quenched with water (100 mL) and extracted with EtOAc (2 x 100 mL). The organic layers were combined and washed with brine (2 x 80 mL), dried over Na₂SO₄ and concentrated under vacuum. The residue was purified by column chromatography (Petroleum ether/EtOAc= 7/3; V/V) to afford (S)-N-(2-((6-oxo-5-(trifluoromethyl)-1-((2-(trimethylsilyl)ethoxy)methyl)-1,6-dihydrop yridazin-4-yl)amino)propoxy)-2-(1-(5-oxo-6,7-dihydro-5H-cyclopenta(d)pyrimidin-2-yl )piperidin-4-yl)acetamide (70 mg, 64.5%) as a yellow solid.
LCMS: *(ESI)* (M+H)⁺ = 640.2.

### Step G: Synthesis of (S)-N-(2-((6-oxo-5-(trifluoromethyl)-L6-dihydropyridazin-4-yl) amino)propoxy)-2-(1-(5-oxo-6,7-dihydro-5H-cyclopenta(d)pyrimidin-2-yl)piperidin-4-yl)acetamide

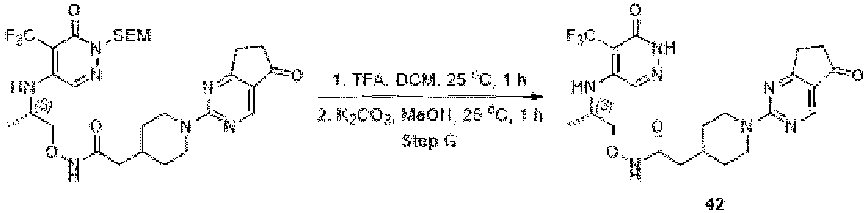

To a mixture of (S)-N-(2-((6-oxo-5-(trifluoromethyl)-1-((2-(trimethylsilyl)ethoxy) methyl)-1,6-dihydropyridazin-4-yl)amino)propoxy)-2-(1-(5-oxo-6,7-dihydro-5H-cyclop enta(d)pyrimidin-2-yl)piperidin-4-yl)acetamide (70 mg, 0.11 mmol, 1.0 eq.) in DCM (3 mL) was added TFA (1 mL). The mixture was stirred at 25 °C for 1 h. The mixture was quenched with saturated NaHCOs aqueous solution (50 mL) and extracted with DCM (3 x 100 mL). The organic layers were combined and dried over Na₂SO₄, concentrated under vacuum. The residue was redissolved in MeOH (3 mL) and K₂CO₃ (70 mg, 0.50 mmol, 4.6 eq.) was added. The mixture was stirred at 25 °C for another 1 h. The mixture was filtered and filtrate was purified by prep-HPLC to afford (S)-N-(2-((6-oxo-5-(trifluoromethyl)-1,6-dihydropyridazin-4-yl)amino)propoxy)-2-(1-( 5-oxo-6,7-dihydro-5H-cyclopenta(d)pyrimidin-2-yl)piperidin-4-yl)acetamide (18.48 mg, 32.5%) as a white solid.
LCMS: *(ESI)* (M+H)⁺ = 510.2.
¹H NMR: (400 MHz, DMSO-*d₆*) *δ* 12.49 (s, 1H), 11.13 (s, 1H), 8.61 (s, 1H), 7.91 (s, 1H), 6.64 - 6.58 (m, 1H), 4.78 (d, *J=* 12.8 Hz, 2H), 4.24 - 4.16 (m, 1H), 3.93 - 3.82 (m, 2H), 3.02 (t, *J* = 12.4 Hz, 2H), 2.94 - 2.89 (m, 2H), 2.54 - 2.52 (m, 2H), 2.07 - 1.98 (m, 1H), 1.93 (d, *J* = 6.8 Hz, 2H), 1.74 (d, *J* = 12.8 Hz, 2H), 1.19 (d, *J=* 6.4 Hz, 3H), 1.16 - 1.08 (m, 2H).

### Example 43:

### 2-(1-(5-hydroxy-6,7-dihydro-5H-cyclopenta(d)pyrimidin-2-yl)piperidin-4-yl)-N-((S)-2-((6-oxo-5-(trifluoromethyl)-1,6-dihydropyridazin-4-yl)amino)propoxy)acetamide

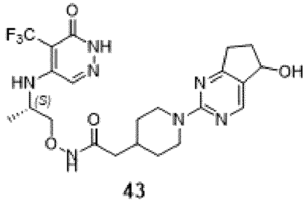

### Step A: synthesis of 2-(1-(5-hydroxy-6,7-dihydro-5H-cyclopenta(d)pyrimidin-2-yl) piperidin-4-yl)-N-((S)-2-((6-oxo-5-(trifluoromethyl)-1,6-dihydropyridazin-4-yl)amino) propoxy)acetamide

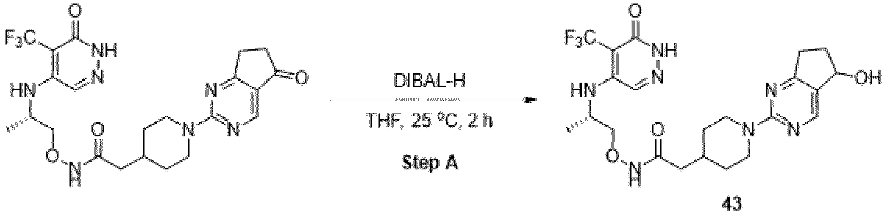

To a mixture of (S)-N-(2-((6-oxo-5-(trifluoromethyl)-1,6-dihydropyridazin-4-yl) amino)propoxy)-2-(1-(5-oxo-6,7-dihydro-5H-cyclopenta(d)pyrimidin-2-yl)piperidin-4-y l)acetamide (40 mg, 0.08 mmol, 1.0 eq.) in THF (1 mL) was added DIBAL-H (1M, 0.24 mL, 0.24 mmol, 3.0 eq.) at 0 °C. The mixture was stirred at 25 °C for 2 h. The mixture was quenched with water (20 mL). The mixture was extracted with EtOAc (3 x 30 mL). The organic layers were combined and dried over Na₂SO₄, concentrated under vacuum. The residue was purified by prep-HPLC to afford 2-(1-(5-hydroxy-6,7-dihydro-5H-cyclopenta(d)pyrimidin-2-yl)piperidin-4-yl)-N-((S)-2-((6-oxo-5-(trifluoromethyl)-1,6-di hydropyridazin-4-yl)amino)propoxy)acetamide (0.94 mg, 2.3%) as a white solid.
LCMS: *(ESI)* (M+H)⁺ = 494.0.
¹H NMR: (400 MHz, DMSO-*d₆*) *δ* 12.49 (s, 1H), 11.13 (s, 1H), 8.23 (s, 1H), 7.90 (s, 1H), 6.62 (s, 1H), 5.19 - 5.13 (m, 1H), 5.03 - 4.95 (m, 1H), 4.62 (d, *J* = 12.8 Hz, 2H), 4.26 - 4.15 (m, 1H), 3.93 - 3.80 (m, 2H), 2.88 - 2.77 (m, 3H), 2.64 - 2.58 (m, 1H), 2.02 - 1.92 (m, 2H), 1.90 (d, J = 6.0 Hz, 2H), 1.78 - 1.73 (m, 1H), 1.67 - 1.62 (m, 2H), 1.19 (d, J = 6.6 Hz, 3H), 1.07 - 1.03 (m, 2H).

### Example 44:

### (S)-N-(2-((6-oxo-5-(trifluoromethyl)-1,6-dihydropyridazin-4-yl)amino)propoxy)-N-(2-( 4-(5-(trifluoromethyl)pyrimidin-2-yl)piperazin-1-yl)ethyl)acetamide

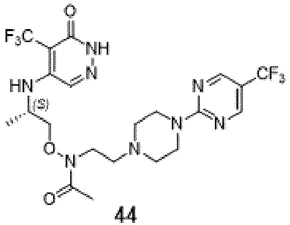

### Step A: synthesis of tert-butyl 4-(5-(trifluoromethyl)pyrimidin-2-yl)piperazine-1-carboxylate

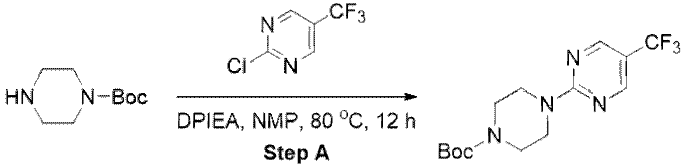

To a mixture of tert-butyl piperazine-1-carboxylate (1.00 g, 5.37 mmol, 1.0 eq.) and DIPEA (2.08 g, 16.11 mmol, 3.0 eq.) in NMP (20 mL) was added 2-chloro-5-(trifluoromethyl)pyrimidine (0.98 g, 5.37 mmol, 1.0 eq.). The mixture was stirred at 80 °C for 12 h. The mixture was cooled to room temperature and then poured into water (40 ml), extracted with EtOAc (3 x 40 mL). The organic layers were combined and washed with brine (2 x 50 mL), dried over Na₂SO₄ and concentrated under vacuum. The residue was purified by flash chromatography (Petroleum ether/EtOAc= 4/1; V/V) to afford tert-butyl 4-(5-(trifluoromethyl)pyrimidin-2-yl) piperazine-1-carboxylate (1.78 g, 99.5%) as a white solid.
LCMS: *(ESI)* (M+H-56)⁺ = 277.2.

### Step B: synthesis of 2-(piperazin-1-yl)-5-(trifluoromethyl)pyrimidine hydrochloride

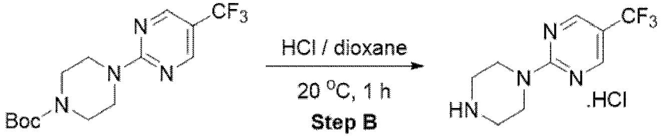

To a mixture of tert-butyl 4-(5-(trifluoromethyl)pyrimidin-2-yl)piperazine-1-carboxylate (1.78 g, 5.36 mmol) in dioxane (5 mL) was added HCl/dioxane (4M, 5 mL). The mixture was stirred at 20 °C for 1 h. The mixture was concentrated under vacuum to afford 2-(piperazin-1-yl)-5-(trifluoromethyl)pyrimidine hydrochloride (1.43 g, 99.4%) as a white solid.
LCMS: *(ESI)* (M+H)⁺ = 233.2.

### Step C: synthesis of 2-(4-(2,2-diethoxyethyl)piperazin-1-yl)-5-(trifluoromethyl) pyrimidine

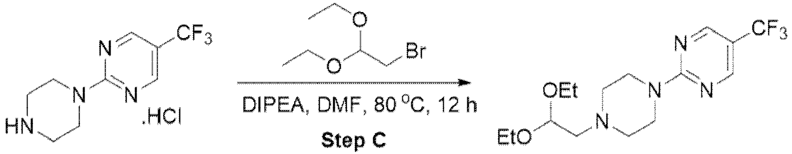

To a mixture of 2-(piperazin-1-yl)-5-(trifluoromethyl)pyrimidine hydrochloride (300 mg, 1.12 mmol, 1.0 eq.) and DIPEA (577 mg, 4.48 mmol, 4.0 eq.) in DMF (10 mL) was added 2-bromo-1,1-diethoxyethane (264 mg, 1.34 mmol, 1.2 eq.). The reaction mixture was stirred at 80 °C for 12 h. The reaction mixture was cooled to room temperature and poured into water (50 mL), extracted with EtOAc (3 x 50 mL). The combined organic extracts were washed with brine (2 x 50 mL) dried over Na₂SO₄, filtered and concentrated under vacuum. The mixture was purified by flash chromatography (Petroleum ether/EtOAc= 3/2; V/V) to afford 2-(4-(2,2-diethoxyethyl) piperazin-1-yl)-5-(trifluoromethyl)pyrimidine (140 mg, 36.0 %) as yellow oil.
LCMS: *(ESI)* (M+H)⁺ = 349.2.
¹H NMR: (400 MHz, CDCl₃) *δ* 8.47 (s, 2H), 4.68 (t, *J* = 5.2 Hz, 1H), 3.91 (t, *J* = 4.8, 4H), 3.74 - 3.66 (m, 2H), 3.60 - 3.52 (m, 2H), 2.61 (t, *J=* 4.8, 4H), 2.59 (d, *J=* 5.2 Hz, 2H), 1.23 (t, *J* = 7.2 Hz, 6H).

### Step D: synthesis of 2-(4-(5-(trifluoromethyl)pyrimidin-2-yl)piperazin-1-yl) acetaldehyde

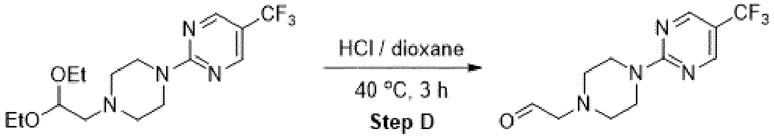

A mixture of 2-(4-(2,2-diethoxyethyl)piperazin-1-yl)-5-(trifluoromethyl)pyrimidine (70 mg, 0.19 mmol, 1.0 eq.) in HCl/dioxane (4M, 2 mL) was stirred at 40 °C for 3 h. The reaction mixture was concentrated to afford 2-(4-(5-(trifluoromethyl)pyrimidin-2-yl) piperazin-1-yl)acetaldehyde (50 mg, 94.5%) as yellow oil.
LCMS: (*ESI)* (M+H+18)⁺ = 293.1.

### Step E: synthesis of (S)-4-(trifluoromethyl)-5-((1-(((2-(4-(5-(trifluoromethyl)pyrimidin-2-yl)piperazin-1-yl)ethyl)amino)oxy)propan-2-yl)amino)-2-((2-(trimethylsilyl)ethoxy)m ethyl)pyridazin-3(2H)-one

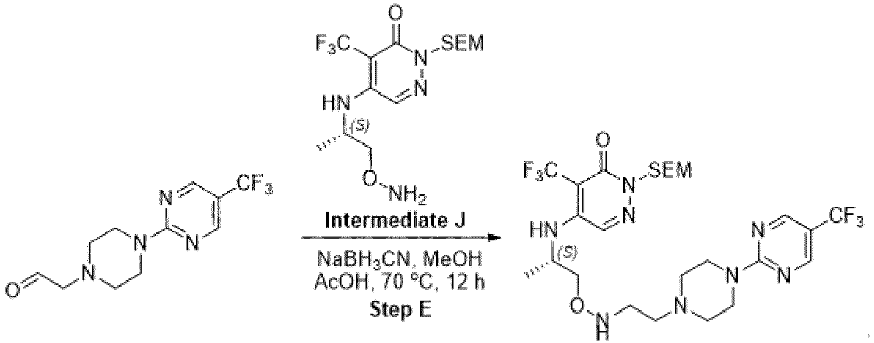

To a mixture of (S)-5-((1-(aminooxy)propan-2-yl)amino)-4-(trifluoromethyl)-2-((2-(trimethylsilyl)ethoxy)methyl)pyridazin-3(2H)-one (58 mg, 0.15 mmol, 1.0 eq.) and 2-(4-(5-(trifluoromethyl)pyrimidin-2-yl)piperazin-1-yl)acetaldehyde (50 mg, 0.18 mmol, 1.2eq.) in MeOH (4 mL) were added AcOH (0.02 mL) and sodium cyanoboranuide (57 mg, 0.91 mmol, 6.0 eq.). The mixture was stirred at 70 °C for 12 h. The reaction mixture was cooled to room temperature and poured into water (10 mL), extracted with DCM (3 x 20 mL). The organic extracts were combined and dried over Na₂SO₄, concentrated under vacuum. The residue was purified by prep-TLC (Petroleum ether/EtOAc= 1/1; V/V) to afford (S)-4-(trifluoromethyl)-5-((1-(((2-(4-(5-(trifluoromethyl)pyrimidin-2-yl)piperazin-1-yl)ethyl)amino)oxy)propan-2-yl)amino)-2-( (2-(trimethylsilyl)ethoxy)methyl)pyridazin-3(2H)-one (50 mg, 51.3%) as yellow solid. LCMS: *(ESI)* (M+H)⁺ = 641.3.
¹H NMR: (400 MHz, CDCl₃) *δ* 8.50 (s, 2H), 7.72 (s, 1H), 6.95 (s, 1H), 5.46 - 5.36 (m, 2H), 4.04 - 3.97 (m, 1H), 3.96 - 3.91 (m, 4H), 3.90 - 3.87 (m, 1H), 3.76 - 3.70 (m, 3H), 3.06 (t, *J* = 5.6 Hz, 2H), 2.61 - 2.49 (m, 6H), 1.37 (d, *J* = 6.8 Hz, 3H), 1.02 - 0.95 (m, 2H), 0.03 (s, 9H).

### Step F: synthesis of (S)-N-(2-((6-oxo-5-(trifluoromethyl)-1-((2-(trimethylsilyl)ethoxy) methyl)-1,6-dihydropyridazin-4-yl)amino)propoxy)-N-(2-(4-(5-(trifluoromethyl)pyrimi din-2-yl)piperazin-1-yl)ethyl)acetamide

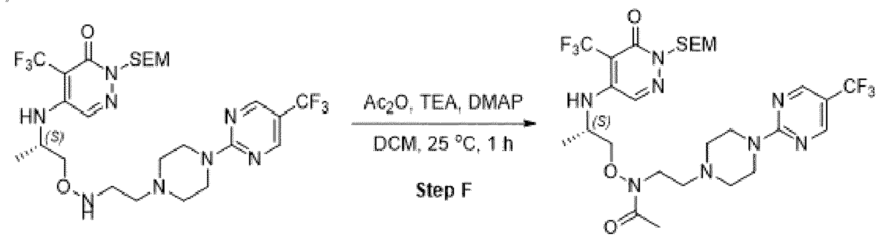

To a mixture of (S)-4-(trifluoromethyl)-5-((1-(((2-(4-(5-(trifluoromethyl)pyrimidin-2-yl)piperazin-1-yl)e thyl)amino)oxy)propan-2-yl)amino)-2-((2-(trimethylsilyl)ethoxy)methyl)pyridazin-3 (2 H)-one (50 mg, 0.07 mmol, 1.0 eq.) in DCM (4 mL) were added TEA (36 mg, 0.35 mmol, 5.0 eq.), DMAP (35 mg, 0.28 mmol, 4.0 eq.) and AczO (15 mg, 0.14 mmol, 2.0 eq.). The mixture was stirred at 25 °C for 1 hr. The reaction mixture was quenched with water (10 mL) and extracted with DCM (3 x 20 mL). The organic extracts were combined and dried over Na₂SO₄, concentrated under vacuum. The residue was purified by prep-TLC (Petroleum ether/EtOAc= 1/1; V/V) to afford (S)-N-(2-((6-oxo-5-(trifluoromethyl)-1-((2-(trimethylsilyl)ethoxy)methyl)-1,6-dihydropyridazin-4-yl)amino )propoxy)-N-(2-(4-(5-(trifluoromethyl)pyrimidin-2-yl)piperazin-1-yl)ethyl)acetamide (40.0 mg, 83.4%) as light yellow solid.
LCMS: *(ESI)* (M+H)⁺ = 683.3.

### Step G: synthesis of (S)-N-(2-((6-oxo-5-(trifluoromethyl)-1,6-dihydropyridazin-4-yl) amino)propoxy)-N-(2-(4-(5-(trifluoromethyl)pyrimidin-2-yl)piperazin-1-yl)ethyl) acetamide

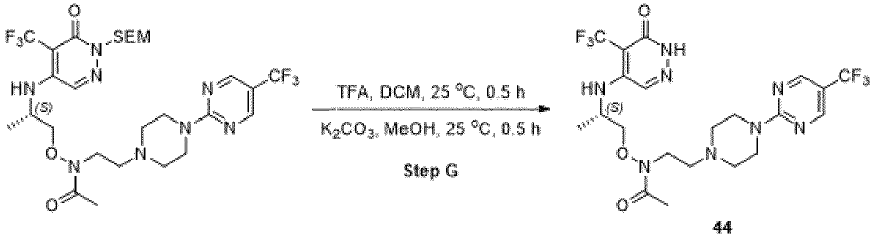

To a mixture of (S)-N-(2-((6-oxo-5-(trifluoromethyl)-1-((2-(trimethylsilyl)ethoxy) methyl)-1,6-dihydropyridazin-4-yl)amino)propoxy)-N-(2-(4-(5-(trifluoromethyl)pyrimi din-2-yl)piperazin-1-yl)ethyl)acetamide (35 mg, 0.05 mmol, 1.0 eq. ) in DCM (1.5 mL) was added TFA (0.5 mL). The mixture was stirred at 25 °C for 0.5 h. The reaction mixture was concentrated under vacuum. To the residue were added MeOH (2 mL) and K₂CO₃ (20 mg, 0.15 mmol). The mixture was stirred at 25 °C for another 0.5 h. The mixture was filtered and the filtrate was purified by prep-HPLC to afford (S)-N-(2-((6-oxo-5-(trifluoromethyl)-1,6-dihydropyridazin-4-yl)amino)propoxy)-N-(2-( 4-(5-(trifluoromethyl)pyrimidin-2-yl)piperazin-1-yl)ethyl)acetamide (11.97 mg, 41.6%) as white solid.
LCMS: *(ESI)* (M+H)⁺ = 553.4.
¹H NMR: (400 MHz, CDCl₃) *δ* 11.24 (s, 1H), 8.40 (s, 2H), 7.62 (s, 1H), 5.66 (br, 1H), 4.06 - 3.96 (m, 2H), 3.88 - 3.83 (m, 1H), 3.82 - 3.75 (m, 4H), 3.73 - 3.64 (m, 2H), 2.54 - 2.47 (m, 2H), 2.47 - 2.42 (m, 4H), 2.06 (s, 3H), 1.34 (d, *J=* 6.0 Hz, 3H).

### Example 45:

### (S)-N-methyl-N-(2-((6-oxo-5-(trifluoromethyl)-1,6-dihydropyridazin-4-yl)amino)propo xy)-2-(1-(5-(trifluoromethyl)pyrimidin-2-yl)piperidin-4-yl)acetamide

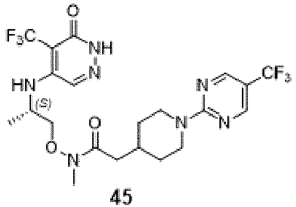

### Step A: Synthesis of (S)-N-methyl-N-(2-((6-oxo-5-(trifluoromethyl)-1-((2-(trimethylsilyl)ethoxy)methyl)-1,6-dihydropyridazin-4-yl)amino)propoxy)-2-(1-(5-(trifluoromethyl)pyrimidin-2-yl)piperidin-4-yl)acetamide

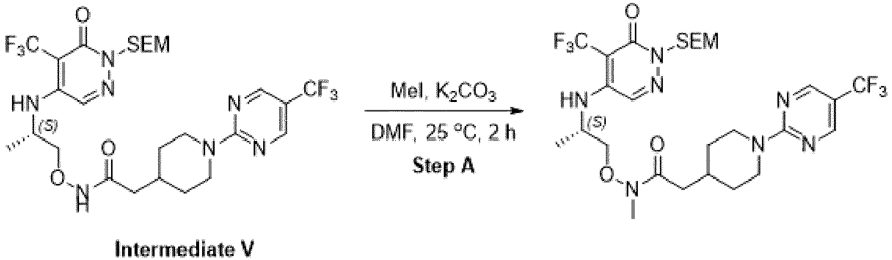

To a solution of (S)-N-(2-((6-oxo-5-(trifluoromethyl)-1-((2-(trimethylsilyl)ethoxy) methyl)-1,6-dihydropyridazin-4-yl)amino)propoxy)-2-(1-(5-(trifluoromethyl)pyrimidin-2-yl)piperidin-4-yl)acetamide (100 mg, 0.15 mmol, 1.0 eq.) in DMF (5 mL) were added K₂CO₃ (62 mg, 0.45 mmol, 3.0 eq.) and iodomethane (21 mg, 0.15 mmol, 1.0 eq.). The mixture was stirred at 25 °C for 2 h. The mixture was then quenched with water (20 mL) and extracted with EtOAc (3 x 30 mL). The organic layers were combined and washed with brine (2 x 30 mL), dried over Na₂SO₄ and concentrated under reduced pressure. The residue was purified by column chromatography (EtOAc/Petroleum ether= 1/1; V/V) to afford (S)-N-methyl-N-(2-((6-oxo-5-(trifluoromethyl)-1-((2-(trimethylsilyl) ethoxy)methyl)-1,6-dihydropyridazin-4-yl)amino)propoxy)-2-(1-(5-(trifluoromethyl) pyrimidin-2-yl)piperidin-4-yl)acetamide (60 mg, 60.0%) as a yellow solid.
LCMS: *(ESI)* (M+H)⁺ = 668.3.

### Step B: synthesis of (S)-N-methyl-N-(2-((6-oxo-5-(trifluoromethyl)-L6-dihydropyridazin-4-yl)amino)propoxy)-2-(1-(5-(trifluoromethyl)pyrimidin-2-yl) piperidin-4-yl)acetamide

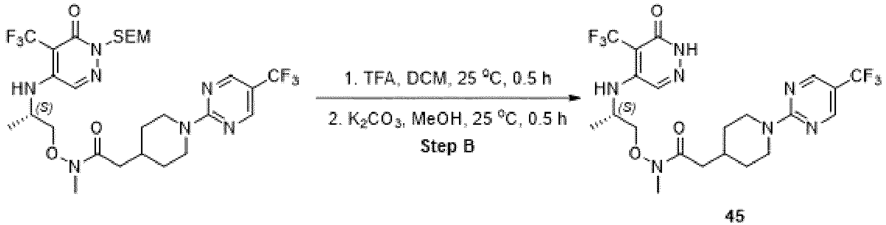

To a solution of (S)-N-methyl-N-(2-((6-oxo-5-(trifluoromethyl)-1-((2-(trimethylsilyl)ethoxy)methyl)-1,6-dihydropyridazin-4-yl)amino)propoxy)-2-(1-(5-(trifl uoromethyl)pyrimidin-2-yl)piperidin-4-yl)acetamide (80 mg, 0.12 mmol) in DCM (3 mL) was added TFA (1 mL). The resulting solution was stirred at 25 °C for 0.5 h. The mixture was concentrated under vacuum. MeOH (2 mL) and K₂CO₃ (20 mg, 0.15 mmol) were added to the mixture. The mixture was stirred at 25 °C for another 0.5 h. The mixture was filtered and the filtrate was purified by prep-HPLC to afford (S)-N-methyl-N-(2-((6-oxo-5-(trifluoromethyl)-1,6-dihydropyridazin-4-yl)amino)propo xy)-2-(1-(5-(trifluoromethyl)pyrimidin-2-yl)piperidin-4-yl)acetamide (15.31 mg, 23.7%) as a white solid.
LCMS: *(ESI)* (M+H)⁺ = 538.2.
¹H NMR: (400 MHz, CDCl₃) *δ* 11.48 (s, 1H), 8.46 (s, 2H), 7.68 (s, 1H), 5.64 (br s, 1H), 4.86 - 4.81 (m, 2H), 4.13 - 3.98 (m, 2H), 3.91 - 3.81 (m, 1H), 3.21 (s, 3H), 3.00 - 2.87 (m, 2H), 2.35 - 2.25 (m, 2H), 2.24 - 2.10 (m, 1H), 1.85 (t, *J=* 11.2 Hz, 2H), 1.40 (d, *J* = 6.8 Hz, 3H), 1.25 - 1.10 (m, 2H).

### Example 46:

### (S)-1-(2-((6-oxo-5-(trifluoromethyl)-1,6-dihydropyridazin-4-yl)amino)propoxy)-3-(1-(5 -(trifluoromethyl)pyrimidin-2-yl)piperidin-4-yl)urea.

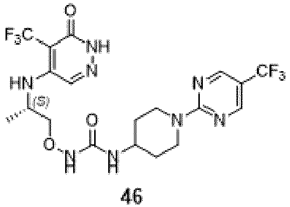

### Step A: synthesis of 4-nitrophenyl (S)-(2-((tert-butoxycarbonyl)amino)propoxy) carbamate

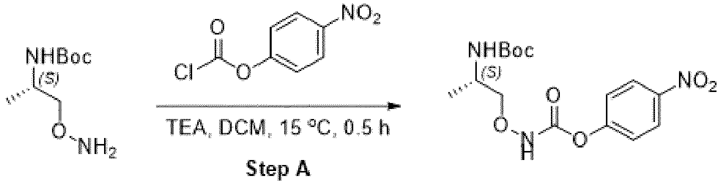

To mixture of 4-nitrophenyl chloromethanoate (200 mg, 0.99 mmol, 1.2 eq.) in DCM (10 mL) were added a solution of (S)-tert-butyl (1-(aminooxy)propan-2-yl)carbamate (157 mg, 0.82 mmol, 1.0 eq.) in DCM (10 mL) and TEA (100 mg, 0.99 mmol, 1.2 eq.) at 0 °C. The mixture was stirred at 15 °C for 0.5 h. The reaction mixture was quenched with water (10 mL). The organic layer was washed with brine (10 mL) and dried over Na₂SO₄, concentrated under vacuum to give 4-nitrophenyl (S)-(2-((tert-butoxycarbonyl)amino)propoxy)carbamate (350 mg, crude) as a yellow oil, which was used in the next step without further purification.

### Step B: synthesis of tert-butyl (1-(5-(trifluoromethyl)pyrimidin-2-yl)piperidin-4-yl) carbamate

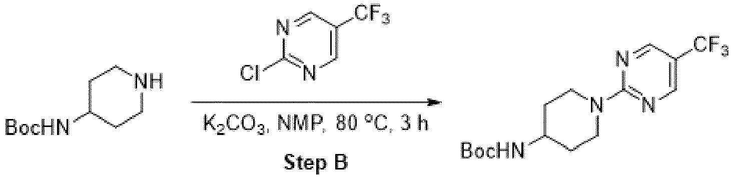

To mixture of 4-N-BOC-Aminopiperidine (1.09 g, 5.42 mmol, 1.1 eq.) in NMP (10 mL) were added 2-chloro-5-(trifluoromethyl)pyrimidine (900 mg, 4.93 mmol, 1.0 eq.) and K₂CO₃ (2.04 g, 14.79 mmol, 3.0 eq.). The mixture was stirred at 80 °C for 3 h. The mixture was cooled to room temperature and diluted with diluted with water (80 mL), extracted with EtOAc (2 x 80 mL). The organic layers were combined and washed with brine (100 mL), dried over Na₂SO₄ and concentrated under vacuum. The residue was purified by column chromatography (EtOAc/Petroleum ether= 1/3; V/V) to afford tert-butyl (1-(5-(trifluoromethyl)pyrimidin-2-yl)piperidin-4-yl)carbamate (1.30 g, 48.0%) as a yellow solid.
LCMS: *(ESI)* (M+H-56)⁺ = 291.0.
¹H NMR: (400 MHz, CDCl₃) *δ* 8.46 (s, 2H), 4.76 - 4.69 (m, 2H), 4.45 (s, 1H), 3.75 (s, 1H), 3.16 - 3.08 (m, 2H), 2.09 - 2.00 (m, 2H), 1.46 (s, 9H), 1.40 - 1.30 (m, 2H).

### Step C: synthesis of 1-(5-(trifluoromethyl)pyrimidin-2-yl)piperidin-4-amine hydrochloride

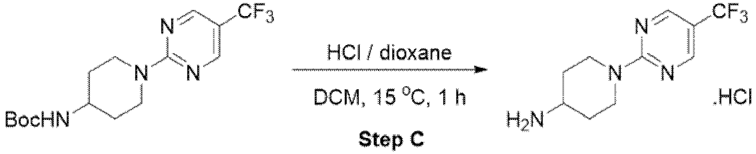

To mixture of tert-butyl (1-(5-(trifluoromethyl)pyrimidin-2-yl)piperidin-4-yl) carbamate (200 mg, 0.58 mmol) in DCM (4 mL) was added HCl/dioxane (4 M, 4 mL). The mixture was stirred at 15 °C for 1 h. The mixture was concentrated under vacuum to afford 1-(5-(trifluoromethyl)pyrimidin-2-yl)piperidin-4-amine hydrochloride (130 mg, 91.43%) as a white solid.
LCMS: *(ESI)* (M+H)⁺ = 247.2.

### Step D: Synthesis of (S)-tert-butyl (1-((3-(1-(5-(trifluoromethyl)pyrimidin-2-yl) piperidin-4-yl)ureido)oxy)propan-2-yl)carbamate

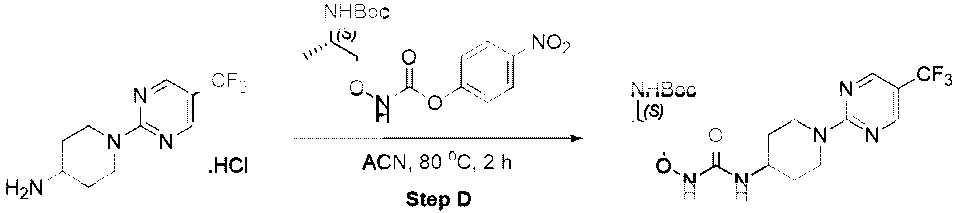

To a solution of 4-nitrophenyl (S)-(2-((tert-butoxycarbonyl)amino)propoxy) carbamate (350 mg, 0.82 mmol, 1.0 eq.) in acetonitrile (10 mL) was added 1-(5-(trifluoromethyl)pyrimidin-2-yl)piperidin-4-amine hydrochloride (228 mg, 0.99 mmol, 1.2 eq.). The mixture was stirred at 80 °C for 2 h. The mixture was cooled to room temperature and diluted with water (40 mL) and extracted with DCM (2 x 80 mL). The organic layers were combined and washed with brine (60 mL), dried over Na₂SO₄ and concertrated under vacuum. The residue was purified by column chromatography (EtOAc/Petroleum ether= 8/1; V/V) to afford (S)-tert-butyl (1-((3-(1-(5-(trifluoromethyl)pyrimidin-2-yl)piperidin-4-yl)ureido)oxy)propan-2-yl)carbamate (120 mg, 27.0%) as a white solid.
LCMS: *(ESI)* (M+H-100)⁺ = 363.2.
¹H NMR: (400 MHz, CDCl₃) *δ* 8.51 (s, 2H), 7.62 - 7.53 (m, 1H), 6.06 (s, 1H), 4.83 (d, *J* = 13.6 Hz, 2H), 4.47 - 4.41 (m, 1H), 4.13 - 4.00 (m, 2H), 3.82 (dd, *J* = 11.2, 3.6 Hz, 1H), 3.62 (dd, *J* = 11.2, 8.8 Hz, 1H), 3.22 - 3.12 (m, 2H), 2.09 - 2.16 (m, 2H), 1.54 - 1.48 (m, 2H), 1.44 (s, 9H), 1.16 (d, *J=* 6.8 Hz, 3H).

### Step E: Synthesis of (S)-1-(2-aminopropoxy)-3-(1-(5-(trifluoromethyl)pyrimidin-2-yl) piperidin-4-yl)urea

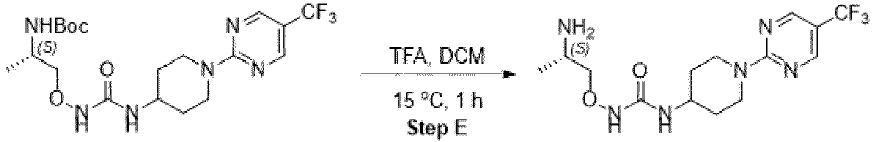

To a suspension of (S)-tert-butyl (1-((3-(1-(5-(trifluoromethyl)pyrimidin-2-yl) piperidin-4-yl)ureido)oxy)propan-2-yl)carbamate (50 mg, 0.11 mmol, 1.0 eq.) in DCM (3 mL) was added TFA (1 mL). The mixture was stirred at 15 °C for 1 h. The mixture was quenched with saturated aqueous NaHCOs solution (20 mL) and extracted with DCM (2 x 40 mL). The organic layers were combined and washed with brine (20 mL), dried over Na₂SO₄ and concentrated under vacuum to afford (S)-1-(2-aminopropoxy)-3-(1-(5-(trifluoromethyl)pyrimidin-2-yl)piperidin-4-yl)urea (30 mg, 76.6%) as a yellow oil.
LCMS: *(ESI)* (M+H)⁺ = 363.2.

### Step F: Synthesis of (S)-1-(2-((6-oxo-5-(trifluoromethyl)-1-((2-(trimethylsilyl)ethoxy) methyl)-1,6-dihydropyridazin-4-yl)amino)propoxy)-3-(1-(5-(trifluoromethyl)pyrimidin-2-yl)piperidin-4-yl)urea

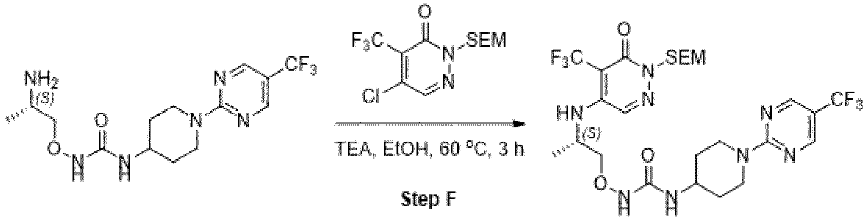

To mixture of (S)-1-(2-aminopropoxy)-3-(1-(5-(trifluoromethyl)pyrimidin-2-yl) piperidin-4-yl)urea (30 mg, 0.08 mmol, 1.0 eq.) in EtOH (5 mL) were added 5-chloro-4-(trifluoromethyl)-2-((2-(trimethylsilyl)ethoxy)methyl)pyridazin-3 (2H)-one (27 mg, 0.08 mmol, 1.0 eq.) and TEA (8 mg, 0.08 mmol, 1.0 eq.). The mixture was stirred at 60 °C for 3 h. The mixture was cooled to room temperature and concentrated under vacuum. The residue was purified by column chromatography (Petroleum ether/EtOAc= 1/2; V/V) to afford (S)-1-(2-((6-oxo-5-(trifluoromethyl)-1 -((2-(trimethylsilyl)ethoxy)methyl)-1,6-dihydropyridazin-4-yl)amino)propoxy)-3-(1-(5-(trifluoromethyl)pyrimidin-2-yl)piperidin-4-yl)urea (30 mg, 55.4%) as a yellow oil. LCMS: *(ESI)* (M+H)⁺ = 655.2.

### Step G: synthesis of (S)-1-(2-((6-oxo-5-(trifluoromethyl)-1,6-dihydropyridazin-4-yl) amino)propoxy)-3-(1-(5-(trifluoromethyl)pyrimidin-2-yl)piperidin-4-yl)urea

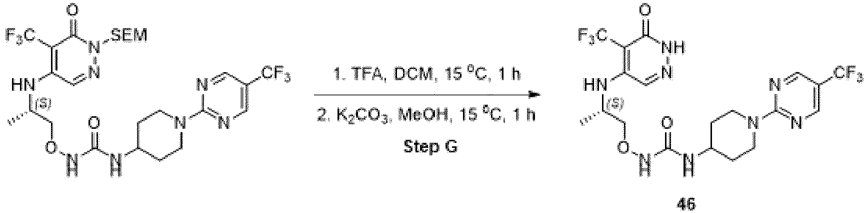

To mixture of (S)-1-(2-((6-oxo-5-(trifluoromethyl)-1-((2-(trimethylsilyl)ethoxy) methyl)-1,6-dihydropyridazin-4-yl)amino)propoxy)-3-(1-(5-(trifluoromethyl)pyrimidin-2-yl)piperidin-4-yl)urea (30 mg, 0.05 mmol, 1.0 eq.) in DCM (3 mL) was added TFA (1 mL). The mixture was stirred at 15 °C for 1 h. The mixture was quenched with saturated aqueous NaHCOs solution (20 mL) and extracted with DCM (3 x 40 mL). The organic layers were combined and dried over Na₂SO₄, concentrated under vacuum. The residue was redissolved in MeOH (2 mL) and K₂CO₃ (29 mg, 0.21 mmol) was added. The mixture was stirred at 15 °C for another 1 h. The mixture was filtered and filtrate was purified by prep-HPLC to afford (S)-1-(2-((6-oxo-5-(trifluoromethyl)-1,6-dihydropyridazin-4-yl)amino)propoxy)-3-(1-(5-(trifluoromethyl)pyrimidin-2-yl)piperidi n-4-yl)urea (13.15 mg, 54.38%) as a white solid.
LCMS: *(ESI)* (M+H)⁺ = 525.2.
¹H NMR: (400 MHz, CDCl₃) *δ* 10.81 (s, 1H), 8.45 (s, 2H), 7.76 (s, 1H), 7.65 (s, 1H), 5.56 - 5.50 (m, 1H), 5.48 (d, *J* = 7.6 Hz, 1H), 4.74 (d, *J* = 13.2 Hz, 2H), 4.09 (s, 1H), 3.96 - 3.87 (m, 2H), 3.82 - 3.76 (m, 1H), 3.11 - 3.02 (m, 2H), 2.06 - 1.97 (m, 2H), 1.40 - 1.29 (m, 5H).

### Example 47:

### (S)-N-(2-((6-oxo-5-(trifluoromethyl)-1,6-dihydropyridazin-4-yl)amino)propoxy)-1-(1-( 5-(trifluoromethyl)pyrimidin-2-yl)piperidin-4-yl)methanesulfonamide

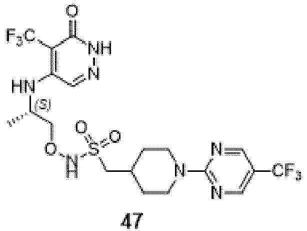

### Step A: Synthesis of (1-(5-(trifluoromethyl)pyrimidin-2-yl)piperidin-4-yl)methanol

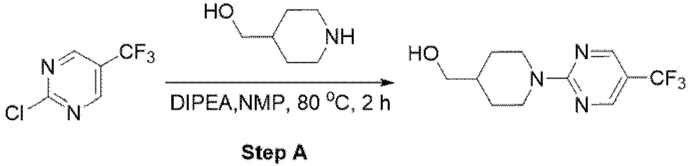

To a solution of 4-Piperidinemethanol (530 mg, 4.60 mmol, 1.2 eq.) in NMP (10 mL) were added 2-chloro-5-(trifluoromethyl)pyrimidine (700 mg, 3.84 mmol, 1.0 eq.) and DIPEA (1.98 g, 15.34 mmol, 4.0 eq.). The mixture was stirred at 80 °C for 2 h. The mixture was cooled to room temperature and diluted with water (60 mL), extracted with EtOAc (2 x 50 mL). The organic layers were combined and washed with brine (2 x 50 mL), dried over Na₂SO₄ and concentrated under vacuum. The residue was purified by column chromatography (Petroleum ether/EtOAc= 2/1; V/V) to afford (1-(5-(trifluoromethyl)pyrimidin-2-yl)piperidin-4-yl)methanol (900 mg, 89.8%) as a white solid.
LCMS: *(ESI)* (M+H)⁺ = 262.1.
¹H NMR: (400 MHz, CDCl₃) *δ* 8.50 (s, 2H), 4.98 - 4.85 (m, 2H), 3.59 (d, *J* = 6.0 Hz, 2H), 3.07 - 2.92 (m, 2H), 1.97 - 1.79 (m, 3H), 1.42 (br s, 1H), 1.33 - 1.23 (m, 2H).

### Step B: Synthesis of (1-(5-(trifluoromethyl)pyrimidin-2-yl)piperidin-4-yl)methyl methanesulfonate

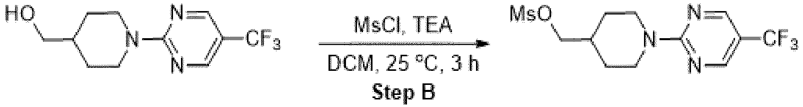

To a solution of (1-(5-(trifluoromethyl)pyrimidin-2-yl)piperidin-4-yl)methanol (1.58 mg, 6.05 mmol, 1.0 eq.) in DCM (10 mL) were added TEA (1.84 g, 18.14 mmol, 3.0 eq.) and MsCl (1.39 g, 12.10 mmol, 2.0 eq.) at 0 °C in ice-water bath. The mixture was stirred at 25 °C for 3 h. The mixture was quenched with saturated NH₄Cl aqueous solution (40 mL) and extracted with DCM (2 x 50 mL). The organic layers were combined and washed with brine (50 mL), dried over anhydrous Na₂SO₄ and concentrated under vacuum to afford (1-(5-(trifluoromethyl)pyrimidin-2-yl)piperidin-4-yl)methyl methanesulfonate (2.00 g, crude) as a white solid.
LCMS: *(ESI)* (M+H)⁺ = 340.2.

### Step C: Synthesis of S-((1-(5-(trifluoromethyl)pyrimidin-2-yl)piperidin-4-yl)methyl) ethanethioate

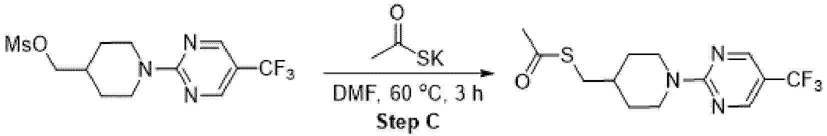

To a solution of (1-(5-(trifluoromethyl)pyrimidin-2-yl)piperidin-4-yl)methyl methanesulfonate (2.00 g, 5.89 mmol, 1.0 eq.) in DMF (25 mL) was added Potassium thioacetate (2.72 g, 23.58 mmol, 4.0 eq.). The mixture was stirred at 60 °C for 3 h. The mixture was cooled to room temperature and diluted with water (70 mL), extracted with EtOAc (2 x 50 mL). The organic layers were combined and washed with brine (2 x 60 mL), dried over Na₂SO₄ and concentrated under vacuum. The residue was purified by silica gel chromatography (EtOAc/Petroleum ether= 1/2; V/V) to afford S-((1-(5-(trifluoromethyl)pyrimidin-2-yl)piperidin-4-yl)methyl) ethanethioate (1.65 g, 87.7%) as a light yellow solid.
LCMS: *(ESI)* (M+H)⁺ = 320.0.
¹H NMR: (400 MHz, CDCl₃) *δ* 8.45 (s, 2H), 4.89 - 4.80 (m, 2H), 2.94 - 2.84 (m, 4H), 2.35 (s, 3H), 1.92 - 1.84 (m, 2H), 1.83 - 1.73 (m, 1H), 1.28 - 1.15 (m, 2H).

### Step D: Synthesis of (1-(5-(trifluoromethyl)pyrimidin-2-yl)piperidin-4-yl) methanesulfonyl chloride

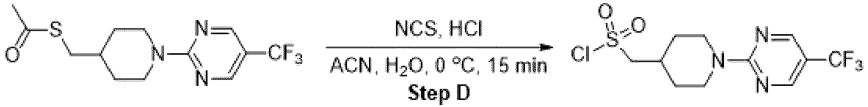

To a solution of HCl (2M, 3.13 mL, 6.26 mmol, 4.0 eq.) in ACN (8 mL) was added NCS (836 mg, 6.26 mmol, 4.0 eq.) at 0 °C in ice-water bath. A solution of S-((1-(5-(trifluoromethyl)pyrimidin-2-yl)piperidin-4-yl)methyl) ethanethioate (500 mg, 1.57 mmol, 1.0 eq.) in ACN (2 mL) was added dropwise to the mixture. The mixture was stirred at 0 °C for 15 min. The mixture was diluted with DCM (80 mL) and washed with brine (2 x 50 mL). The organic layer was dried over Na₂SO₄ and concentrated under vacuum to afford (1-(5-(trifluoromethyl)pyrimidin-2-yl)piperidin-4-yl) methanesulfonyl chloride (500 mg, 92.9%) as a light yellow oil.
LCMS: *(ESI)* (M+H)⁺ = 344.0.
¹H NMR: (400 MHz, CDCl₃) *δ* 8.48 (d, *J=* 0.4 Hz, 2H), 4.96 - 4.86 (m, 2H), 3.69 (d, *J* = 6.4 Hz, 2H), 3.07 - 2.96 (m, 2H), 2.60 - 2.46 (m, 1H), 2.14 - 2.06 (m, 2H), 1.47 - 1.36 (m, 2H).

### Step E: Synthesis of (S)-tert-butyl (1-(((1-(5-(trifluoromethyl)pyrimidin-2-yl)piperidin -4-yl)methylsulfonamido)oxy)propan-2-yl)carbamate

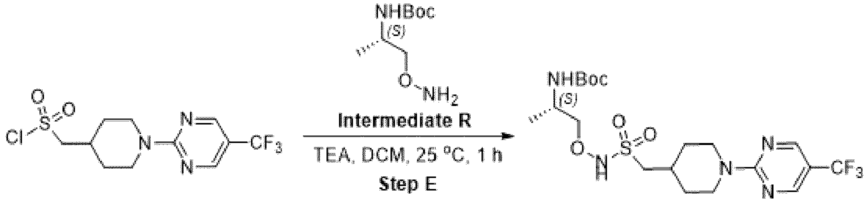

To a solution of (1-(5-(trifluoromethyl)pyrimidin-2-yl)piperidin-4-yl) methanesulfonyl chloride (536 mg, 1.56 mmol, 1.0 eq.) and (S)-tert-butyl (1-(aminooxy)propan-2-yl)carbamate (297 mg, 1.56 mmol, 1.0 eq.) in DCM (10 mL) were added dropwise TEA (316 mg, 3.12 mmol, 2.0 eq.) at 0 °C. The mixture was stirred at 25 °C for 1 h. The mixture was quenched with water (40 mL) and extracted with EtOAc (3 x 40 mL). The organic layers were combined and washed with brine (80 mL), dried over anhydrous Na₂SO₄ and concentrated under vacuum. The residue was purified by column chromatography (EtOAc/Petroleum ether= 1/1; V/V) to afford (S)-tert-butyl (1-(((1-(5-(trifluoromethyl)pyrimidin-2-yl)piperidin-4-yl) methylsulfonamido)oxy)propan-2-yl)carbamate (420 mg, 54.1%) as a yellow oil.
LCMS: *(ESI)* (M+H)⁺ = 498.2.

### Step F: synthesis of (S)-N-(2-aminopropoxy)-1-(1-(5-(trifluoromethyl)pyrimidin-2-yl) piperidin-4-yl)methanesulfonamide

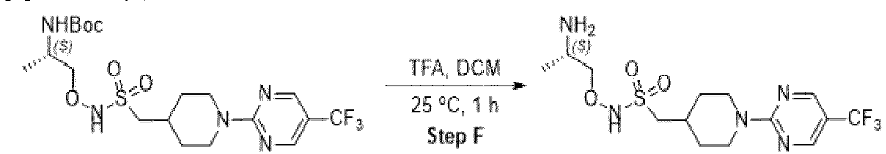

To a solution of (S)-tert-butyl (1-(((1-(5-(trifluoromethyl)pyrimidin-2-yl)piperidin -4-yl)methylsulfonamido)oxy)propan-2-yl)carbamate (148 mg, 0.30 mmol, 1.0 eq.) in DCM (8 mL) was added TFA (2 mL). The mixture was stirred at 25 °C for 1 h. The mixture was quenched with saturated NaHCOs aqueous solution (20 mL) and extracted with DCM (3 x 20 mL). The organic layers were combined and dried over Na₂SO₄, concentrated under vacuum to afford (S)-N-(2-aminopropoxy)-1-(1-(5-(trifluoromethyl) pyrimidin-2-yl)piperidin-4-yl)methanesulfonamide (112 mg, 94.1%) as a yellow oil.
LCMS: *(ESI)* (M+H)⁺ = 398.1.

### Step G: synthesis of (S)-N-(2-((6-oxo-5-(trifluoromethyl)-1,6-dihydropyridazin-4-yl) amino)propoxy)-1-(1-(5-(trifluoromethyl)pyrimidin-2-yl)piperidin-4-yl) methanesulfonamide

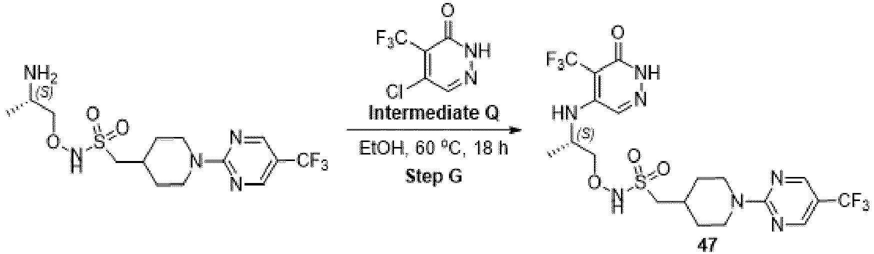

To a solution of (S)-N-(2-aminopropoxy)-1-(1-(5-(trifluoromethyl)pyrimidin-2-yl) piperidin-4-yl)methanesulfonamide (50 mg, 0.13 mmol, 1.0 eq.) in EtOH (5 mL) was added 5-chloro-4-(trifluoromethyl)pyridazin-3(2H)-one (26 mg, 0.13 mmol, 1.0 eq.). The mixture was heated to 60 °C and stirred for 18 h. The mixture was filtered and the filtrate was purified by prep-HPLC to afford (S)-N-(2-((6-oxo-5-(trifluoromethyl)-1,6-dihydropyridazin-4-yl)amino)propoxy)-1-(1-(5-(trifluoromethyl)pyrimidin-2-yl)piperidi n-4-yl)methanesulfonamide (6.78 mg, 9.3%) as a white solid.
LCMS: *(ESI)* (M+H)⁺ = 560.2.
¹H NMR: (400 MHz, CDCl₃) *δ* 10.60 (s, 1H), 8.45 (s, 2H), 8.36 (s, 1H), 7.70 (s, 1H), 5.74 - 5.48 (m, 1H), 4.81 (d, *J=* 13.6 Hz, 2H), 4.15 - 4.12 (m, 2H), 3.94 - 3.83 (m, 1H), 3.21 - 3.08 (m, 2H), 3.02 - 2.92 (m, 2H), 2.41 - 2.25 (m, 1H), 2.10 - 1.95 (m, 2H), 1.43 - 1.32 (m, 2H), 1.28 (d, *J* = 6.4 Hz, 3H).

### Example 48:

### 2-fluoro-N-((S)-2-((6-oxo-5-(trifluoromethyl)-1,6-dihydropyridazin-4-yl)amino)propox y)-2-(4-(5-(trifluoromethyl)pyrimidin-2-yl)piperazin-1-yl)acetamide

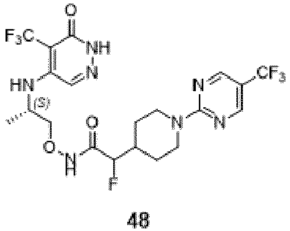

### Step A: synthesis of benzyl 4-(2-ethoxy-1-fluoro-2-oxoethylidene)piperidine-1-carboxylate

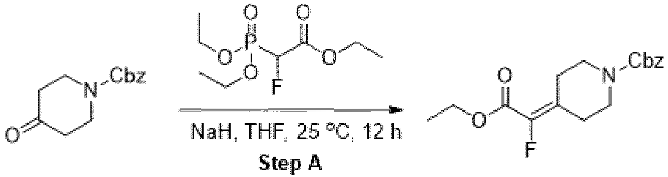

To a solution of triethyl 2-fluoro-2-phosphonoacetate (799 mg, 3.30 mmol, 1.1 eq.) in THF (12 mL) was added NaH (60%, 144 mg, 3.60 mmol, 1.2 eq.) at 0 °C in ice-water bath under N₂ atmosphere. The mixture was stirred at 0 °C for 10 min. Benzyl 4-oxopiperidine-1-carboxylate (700 mg, 3.00 mmol, 1.0 eq.) was added to the reaction mixture. The mixture was stirred at 25 °C for 12 h. The mixture was quenched with ice-water (30 mL) and extracted with EtOAc (3 x 30 mL). The organic extracts were combined and dried over Na₂SO₄, concentrated under vacuum. The residue was purified by flash chromatography (Petroleum ether/EtOAc= 3/1; V/V) to afford benzyl 4-(2-ethoxy-1-fluoro-2-oxoethylidene)piperidine-1-carboxylate (300 mg, 31.1%) as a colorless oil.
LCMS: *(ESI)* (M+Na)⁺ =344.1.

### Step B: synthesis of ethyl 2-fluoro-2-(piperidin-4-yl)acetate

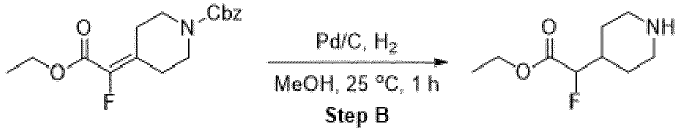

To a mixture of benzyl 4-(2-ethoxy-1-fluoro-2-oxoethylidene)piperidine-1-carboxylate (300 mg, 1.11 mmol, 1.0 eq) in MeOH (5 mL) was added Pd/C (10%, 100 mg) under N₂ atmosphere. The reaction mixture was degassed and purged with H₂ several times. The reaction mixture was stirred at 25 °C for 1 h under H₂ atmosphere. The mixture was filtered and the filtrate was concentrated to afford ethyl 2-fluoro-2-(piperidin-4-yl)acetate (150 mg, 87.7%) as a colorless oil.
LCMS: *(ESI)* (M+H)⁺ = 190.2.
¹H NMR: (400 MHz, DMSO-*d₆*) *δ* 5.01 (dd, *J* = 48.0, 4.0 Hz, 1H), 4.20 (q, *J* = 7.2 Hz, 2H), 3.12 (d, *J* = 12.0 Hz, 2H), 2.71 - 2.61 (m, 2H), 2.13 - 2.00 (m, 1H), 1.71 (d, *J* = 12.8 Hz, 1H), 1.57 - 1.40 (m, 4H), 1.24 (t, *J* = 7.2 Hz, 3H).

### Step C: synthesis of ethyl 2-fluoro-2-(1-(5-(trifluoromethyl)pyrimidin-2-yl)piperidin-4-yl)acetate

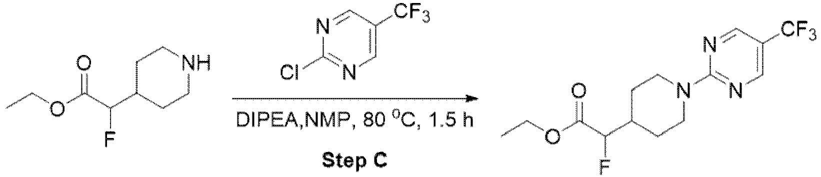

To a mixture of ethyl 2-fluoro-2-(piperidin-4-yl)acetate (150 mg, 0.79 mmol, 1.1 eq.) and DIPEA (373 mg, 2.88 mmol, 4.0 eq.) in NMP (12 mL) was added 2-chloro-5-(trifluoromethyl)pyrimidine (131 mg, 0.72 mmol, 1.0 eq.). The mixture was stirred at 80 °C for 1.5 h. The mixture was cooled to room temperature and poured into water (50 mL), extracted with EtOAc (3 x 50 mL). The organic extracts were combined and washed with saturated aqueous NH₄Cl solution (2 x 50 mL), dried over Na₂SO₄ and concentrated under vacuum. The residue was purified by flash chromatography (Petroleum ether/EtOAc= 3/1; V/V) to afford ethyl 2-fluoro-2-(1-(5-(trifluoromethyl) pyrimidin-2-yl)piperidin-4-yl)acetate (140 mg, 57.9 %) as a yellow solid.
LCMS: *(ESI)* (M+H)⁺ = 336.1.

### Step D: synthesis of

### 2-fluoro-2-(1-(5-(trifluoromethyl)pyrimidin-2-yl)piperidin-4-yl)acetic acid

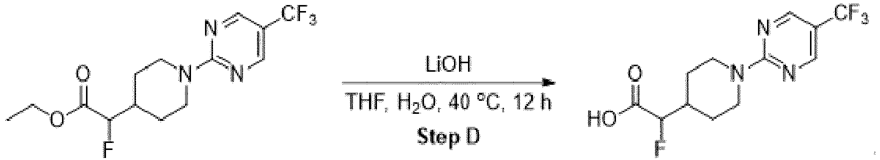

To a mixture of ethyl 2-fluoro-2-(1-(5-(trifluoromethyl)pyrimidin-2-yl)piperidin-4-yl)acetate (95 mg, 0.28 mmol, 1.0 eq.) in THF (1.8 mL) and H₂O (0.6 mL) was added LiOH (36 mg, 0.84 mmol, 3.0 eq.). The mixture was stirred at 40 °C for 12 h. The mixture was diluted with water (15 mL) and neutralized to pH 7 with 1M HCl. The mixture was extracted with EtOAc (2 x 30 mL). The organic layers were combined and washed with brine (20 mL), dried over Na₂SO₄ and concentrated under vacuum to afford 2-fluoro-2-(1-(5-(trifluoromethyl)pyrimidin-2-yl)piperidin-4-yl)acetic acid (80 mg, 91.9%) as a white solid.
LCMS: *(ESI)* (M+H)⁺ = 308.1.

### Step E: synthesis of 2-fluoro-N-((S)-2-((6-oxo-5-(trifluoromethyl)-1-((2-(trimethylsilyl) ethoxy)methyl)-1,6-dihydropyridazin-4-yl)amino)propoxy)-2-(1-(5-(trifluoromethyl) pyrimidin-2-yl)piperidin-4-yl)acetamide

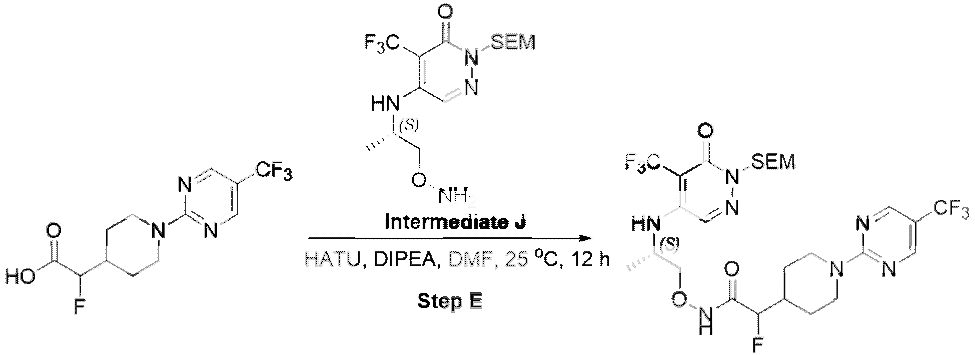

To a mixture of (S)-5-((1-(aminooxy)propan-2-yl)amino)-4-(trifluoromethyl)-2-((2-(trimethylsilyl)ethox y)methyl)pyridazin-3(2H)-one (68 mg, 0.18 mmol, 1.1 eq.) and 2-fluoro-2-(1-(5-(trifluoromethyl)pyrimidin-2-yl)piperidin-4-yl)acetic acid (50 mg, 0.16 mmol, 1.0 eq.) in DMF (5 mL) were added DIPEA (63 mg, 0.48 mmol, 3.0 eq.) and HATU (74 mg, 0.19 mmol, 1.2 eq.). The mixture was stirred at 25 °C for 12 h. The mixture was diluted with water (30 mL) and extracted with EtOAc (3 x 40 mL). The organic extracts were combined and washed with brine (20 mL), dried over Na₂SO₄ and concentrated under vacuum. The residue was purified by prep-TLC (Petroleum ether/EtOAc= 1/1; V/V) to afford 2-fluoro-N-((S)-2-((6-oxo-5-(trifluoromethyl)-1 -((2-(trimethylsilyl)ethoxy)methyl)-1,6-dihydropyridazin-4-yl)amino)propoxy)-2-(1-(5-(trifluoromethyl)pyrimidin-2-yl)piperidin-4-yl)acetamide (80 mg , 73.1%) as a yellow solid.
LCMS: *(ESI)* (M+H)⁺ = 672.2.

### Step F: synthesis of 2-fluoro-N-((S)-2-((6-oxo-5-(trifluoromethyl)-1,6-dihydropyridazin -4-yl)amino)propoxy)-2-(4-(5-(trifluoromethyl)pyrimidin-2-yl)piperazin-1-yl)acetamide

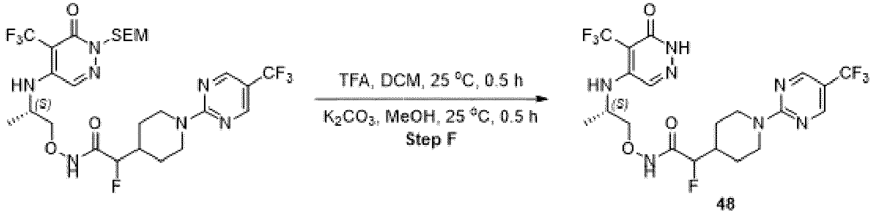

To a mixture of 2-fluoro-N-((S)-2-((6-oxo-5-(trifluoromethyl)-1-((2-(trimethylsilyl) ethoxy)methyl)-1,6-dihydropyridazin-4-yl)amino)propoxy)-2-(4-(5-(trifluoromethyl)pyr imidin-2-yl)piperazin-1-yl)acetamide (40 mg, 0.06 mmol, 1.0 eq. ) in DCM (1.5 mL) was added TFA (0.5 mL). The reaction mixture was stirred at 25 °C for 0.5 h. The mixture was concentrated under vacuum. The residue was dissolved in MeOH (2 mL) and K₂CO₃ (25 mg, 0.18 mmol, 3.0 eq.) was added to the mixture. The reaction mixture was stirred at 25 °C for another 0.5 h. The mixture was filtered and the filtrate was purified by prep-HPLC to afford 2-fluoro-N-((S)-2-((6-oxo-5-(trifluoromethyl)-1,6-dihydropyridazin-4-yl)amino)propoxy)-2-(4-(5-(trifluoromethyl)pyrimidin-2-yl) piperazin-1-yl)acetamide (19.08 mg, 59.1%) as a white solid.
LCMS: *(ESI)* (M+H)⁺ = 542.2.
¹H NMR: (400 MHz, DMSO-*d₆*) *δ* 12.53 (s, 1H), 11.77 (s, 1H), 8.70 (s, 2H), 7.93 (s, 1H), 6.64 - 6.55 (m, 1H), 4.91 - 4.71 (m, 3H), 4.30 - 4.18 (m, 1H), 4.02 - 3.84 (m, 2H), 3.00 (q, *J* = 13.2 Hz, 2H), 2.32 - 2.16 (m, 1H), 1.72 (t, *J* = 14.4 Hz, 2H), 1.37 - 1.25 (m, 2H), 1.22 (d, *J* = 6.4 Hz, 3H).

### Example 49:

### (S)-2-methyl-N-(2-((6-oxo-5-(trifluoromethyl)-1,6-dihydropyridazin-4-yl)amino) propoxy)-2-(1-(5-(trifluoromethyl)pyrimidin-2-yl)piperidin-4-yl)propanamide

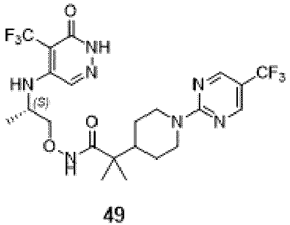

### 49.1) Synthesis of intermediate U

### Step A: synthesis of tert-butyl 4-(1-methoxy-2-methyl-1-oxopropan-2-yl)piperidine-1-carboxylate and tert-butyl 4-(1-methoxy-1-oxopropan-2-yl)piperidine-1-carboxylate (Intermediate U)

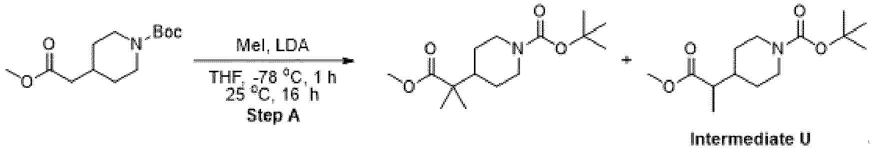

To a mixture of tert-butyl 4-(2-methoxy-2-oxoethyl)piperidine-1-carboxylate (2.00 g, 7.77 mmol, 1.0 eq.) in THF (30 mL) under N₂ atmosphere was added dropwise LDA (2M, 15.6 mL, 31.2 mmol, 4.0 eq.) at -78 °C in dry ice-EtOH bath. The mixture was stirred at -78 °C for 1 h. Iodomethane (3.31 g, 23.31 mmol, 3.0 eq.) was added dropwise to the mixture at -78 °C. The mixture was stirred at 25 °C for 16 h. The mixture was quenched with water (50 mL) and extracted with EtOAc (2 x 50 mL). The organic layers were combined and dried over Na₂SO₄, concentrated under vacuum. The residue was purified by prep-HPLC to afford tert-butyl 4-(1-methoxy-2-methyl-1-oxopropan-2-yl)piperidine-1-carboxylate (330 mg, 14.9%) as a colorless oil and tert-butyl 4-(1-methoxy-1-oxopropan-2-yl)piperidine-1-carboxylate (1.26 g, 59.7%) as a colorless oil.
tert-butyl 4-(1-methoxy-2-methyl-1-oxopropan-2-yl)piperidine-1-carboxylate:
   LCMS: *(ESI)* (M+H-100)⁺ = 186.2
tert-butyl 4-(1-methoxy-1-oxopropan-2-yl)piperidine-1-carboxylate:
   LCMS: *(ESI)* (M+H-100)⁺ = 172.2

### 49.2) Synthesis of compound 49

### Step A: synthesis of methyl 2-methyl-2-(piperidin-4-yl)propanoate hydrochloride

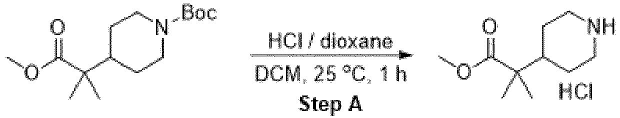

To a mixture of tert-butyl 4-(1-methoxy-2-methyl-1-oxopropan-2-yl)piperidine-1-carboxylate (320 mg, 1.12 mmol, 1.0 eq.) in DCM (5 mL) was added HCl/dioxane (4M, 5 mL). The mixture was stirred at 25 °C for 1 h. The mixture was concentrated under vacuum to afford methyl 2-methyl-2-(piperidin-4-yl)propanoate hydrochloride (330 mg, crude) as a white solid.
LCMS: *(ESI)* (M+H)⁺ = 186.2.

### Step B: synthesis of methyl

### 2-methyl-2-(1-(5-(trifluoromethyl)pyrimidin-2-yl)piperidin-4-yl)propanoate

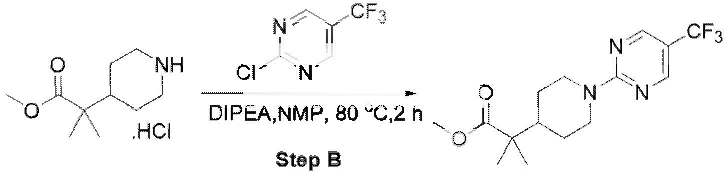

To a mixture of 2-methyl-2-(piperidin-4-yl)propanoate hydrochloride (243 mg, 1.10 mmol, 1.0 eq.), DIPEA (426 mg, 3.30 mmol, 3.0 eq.) in NMP (20 mL) was added 2-chloro-5-(trifluoromethyl)pyrimidine (200 mg, 1.10 mmol, 1.0 eq.). The reaction mixture was stirred at 80 °C for 2 h. The reaction mixture was cooled to room temperature and quenched with water (50 mL), extracted with EtOAc (3 x 50 mL). The organic layers were combined and washed with brine (2 x 60 mL), dried over Na₂SO₄, concentrated under vacuum. The residue was purified by column chromatography (Petroleum ether/EtOAc= 2/1; V/V) to afford methyl 2-methyl-2-(1-(5-(trifluoromethyl) pyrimidin-2-yl)piperidin-4-yl)propanoate (253 mg, 70.0%) as a white solid.
LCMS: *(ESI)* (M+H)⁺ = 332.2.
¹H NMR: (400 MHz, CDCl₃) *δ* 8.45 (s, 2H), 4.99 - 4.83 (m, 2H), 3.69 (s, 3H), 2.85 (td, *J* = 13.2, 2.0 Hz, 2H), 1.97 - 1.85 (m, 1H), 1.70 - 1.62 (m, 2H), 1.35 - 1.22 (m, 2H), 1.14 (s, 6H).

### Step C: synthesis of 2-methyl-2-(1-(5-(trifluoromethyl)pyrimidin-2-yl)piperidin-4-yl propanoic acid

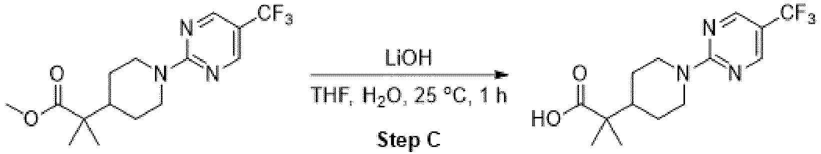

To a mixture of methyl 2-methyl-2-(1-(5-(trifluoromethyl)pyrimidin-2-yl) piperidin-4-yl)propanoate (200 mg, 0.60 mmol, 1.0 eq.) in THF (5 mL) and H₂O (5 mL) was added lithium hydroxide (36 mg, 1.50 mmol, 2.5 eq.). The mixture was stirred at 25 °C for 1 h. The mixture was diluted with water (20 mL) and adjusted to pH 6 with 1M HCl. The mixture was extracted with EtOAc (3 x 40 mL). The organic layers were combined and washed with brine (80 mL), dried over Na₂SO₄ and concentrated under vacuum to afford 2-methyl-2-(1-(5-(trifluoromethyl)pyrimidin-2-yl)piperidin-4-yl) propanoic acid (170 mg, 89.4%) as a yellow solid.
LCMS: *(ESI)* (M+H)⁺ = 318.1.

### Step D: Synthesis of (S)-2-methyl-N-(2-((6-oxo-5-(trifluoromethyl)-1-((2-(trimethylsilyl)ethoxy)methyl)-1,6-dihydropyridazin-4-yl)amino)propoxy)-2-(1-(5-(trifluoromethyl)pyrimidin-2-yl)piperidin-4-yl)propanamide

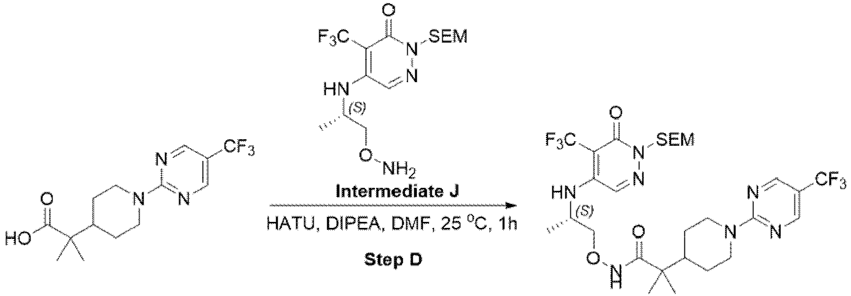

To a mixture of 2-methyl-2-(1-(5-(trifluoromethyl)pyrimidin-2-yl)piperidin-4-yl) propanoic acid (57 mg, 0.18 mmol, 1.1 eq.) in DMF (2 mL) were added (S)-5-((1-(aminooxy)propan-2-yl)amino)-4-(trifluoromethyl)-2-((2-(trimethylsilyl)ethox y)methyl)pyridazin-3(2H)-one (60 mg, 0.16 mmol, 1.0 eq.), DIPEA (62 mg, 0.48 mmol, 3.0 eq.) and HATU (72 mg, 0.19 mmol, 1.2 eq.). The mixture was stirred at 25 °C for 1 h. The reaction mixture was quenched with water (50 mL) and extracted with EtOAc (2 x 50 mL). The organic layers were combined and washed with brine (2 x 50 mL), dried over Na₂SO₄ and concentrated under vacuum. The residue was purified by column chromatography (Petroleum ether/EtOAc= 2/3; V/V) to afford (S)-2-methyl-N-(2-((6-oxo-5-(trifluoromethyl)-1-((2-(trimethylsilyl)ethoxy)methyl)-1,6-dihydropyridazin-4-yl) amino)propoxy)-2-(1-(5-(trifluoromethyl)pyrimidin-2-yl)piperidin-4-yl)propenamide (70 mg, 64.2%) as a yellow solid.
LCMS: *(ESI)* (M+H)⁺ = 682.3.

### Step E: synthesis of (S)-2-methyl-N-(2-((6-oxo-5-(trifluoromethyl)-1,6-dihydropyridazin-4-yl)amino)propoxy)-2-(1-(5-(trifluoromethyl)pyrimidin-2-yl) piperidin-4-yl)propanamide

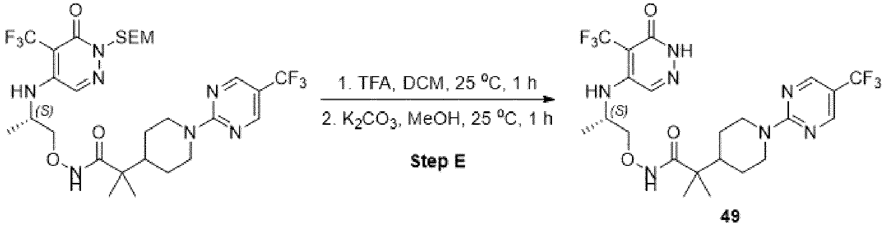

To a mixture of (S)-2-methyl-N-(2-((6-oxo-5-(trifluoromethyl)-1-((2-(trimethylsilyl)ethoxy)methyl)-1,6-dihydropyridazin-4-yl)amino)propoxy)-2-(1-(5-(trifluoromethyl)pyrimidin-2-yl)piperidin-4-yl)propanamide (70 mg, 0.10 mmol, 1.0 eq.) in DCM (3 mL) was added TFA (1 mL). The mixture was stirred at 25 °C for 1 h. The mixture was concentrated under vacuum. The residue was redissolved in MeOH (2 mL) and K₂CO₃ (62 mg, 0.45 mmol) was added. The mixture was stirred at 25 °C for another 1 h. The mixture was filtered and filtrate was purified by prep-HPLC to afford (S)-2-methyl-N-(2-((6-oxo-5-(trifluoromethyl)-1,6-dihydropyridazin-4-yl)amino)propox y)-2-(1-(5-(trifluoromethyl)pyrimidin-2-yl)piperidin-4-yl)propanamide (33.10 mg, 60.0%) as a white soild.
LCMS: *(ESI)* (M+H)⁺ = 552.2.
¹H NMR: (400 MHz, CDCl₃) *δ* 10.12 (s, 1H), 8.66 (s, 1H), 8.45 (s, 2H), 7.70 (s, 1H), 6.08 - 5.98 (m, 1H), 4.93 (d, *J=* 13.2 Hz, 2H), 4.15 - 4.03 (m, 2H), 3.98 - 3.87 (m, 1H), 2.86 (t, *J* = 12.4 Hz, 2H), 2.15 - 1.95 (m, 1H), 1.75 - 1.60 (m, 2H), 1.37 (d, *J* = 6.4 Hz, 3H), 1.32 - 1.23 (m, 2H), 1.14 (s, 6H).

### Example 50:

### (S)-N-(2-((6-oxo-5-(trifluoromethyl)-1,6-dihydropyridazin-4-yl)amino)propoxy)-1-(1-( 5-(trifluoromethyl)pyrimidin-2-yl)piperidin-4-yl)cyclopropanecarboxamide

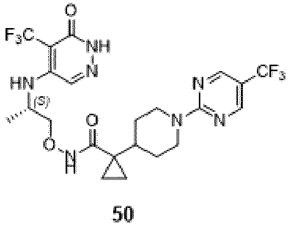

### Step A: synthesis of 1-(piperidin-4-yl)cyclopropanecarboxylic acid hydrochloride

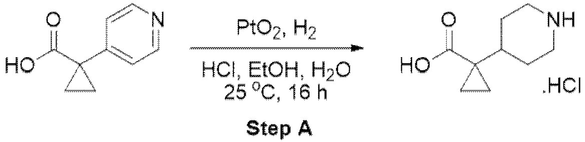

To a solution of 1-(pyridin-4-yl)cyclopropanecarboxylic acid (100 mg, 0.61 mmol, 1.0 eq.) in EtOH (5 mL) were added concentrated HCl (0.1 mL) and followed by PtO₂ (20 mg) under N₂ atmosphere. The mixture was degassed and purged with H₂ for 3 times. The mixture was stirred at 25 °C under H₂ Balloon atmosphere for 16 h. The mixture was filtered and the filtrate was concentrated under vacuum to afford 1-(piperidin-4-yl)cyclopropanecarboxylic acid hydrochloride (126 mg, 99.9%) as a light yellow oil.
LCMS: *(ESI)* (M+H)⁺ = 170.2.

### Step B: synthesis of 1-(1-(5-(trifluoromethyl)pyrimidin-2-yl)piperidin-4-yl) cyclopropanecarboxylic acid

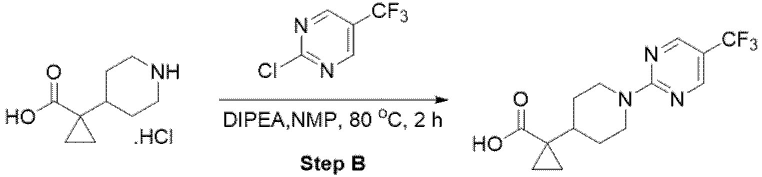

To a solution of 2-chloro-5-(trifluoromethyl)pyrimidine (91 mg, 0.50 mmol, 1.0 eq.) and 1-(piperidin-4-yl)cyclopropanecarboxylic acid hydrochloride (123 mg, 0.60 mmol, 1.2 eq.) in NMP (3 mL) was added DIPEA (323 mg, 2.50 mmol, 5.0 eq.). The mixture was stirred at 80 °C for 2 h. The mixture was cooled to room temperature and diluted with water (40 mL). The mixture was extracted with EtOAc (2 x 40 mL). The organic layers were combined and washed with saturated aqueous NH₄Cl (2 x 30 mL), dried over Na₂SO₄ and concentrated under vacuum. The residue was purified by prep-TLC (Petroleum ether/EtOAc= 2/1; V/V) to afford 1-(1-(5-(trifluoromethyl)pyrimidin-2-yl) piperidin-4-yl)cyclopropanecarboxylic acid (120 mg, 76.1%) as a light yellow solid.
LCMS: *(ESI)* (M+H)⁺ = 316.2.

### Step C: synthesis of (S)-N-(2-((6-oxo-5-(trifluoromethyl)-1-((2-(trimethylsilyl)ethoxy) methyl)-1,6-dihydropyridazin-4-yl)amino)propoxy)-1-(1-(5-(trifluoromethyl)pyrimidin-2-yl)piperidin-4-yl)cyclopropanecarboxamide

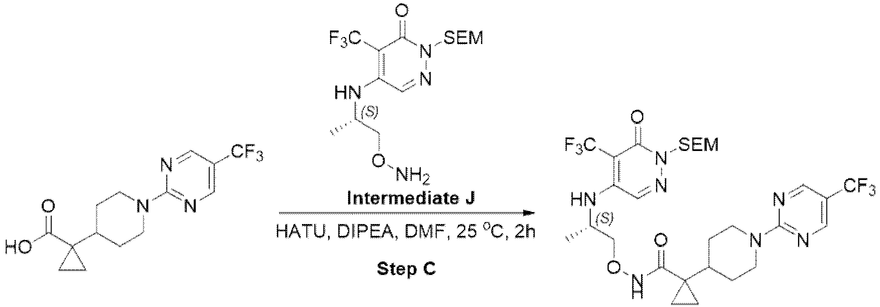

To a solution of 1-(1-(5-(trifluoromethyl)pyrimidin-2-yl)piperidin-4-yl) cyclopropanecarboxylic acid (50 mg, 0.16 mmol, 1.5 eq.) in DMF (2 mL) were added DIPEA (68 mg, 0.52 mmol, 5.0 eq.), HATU (80 mg, 0.21 mmol, 2.0 eq.) and followed by (S)-5-((1-(aminooxy)propan-2-yl)amino)-4-(trifluoromethyl)-2-((2-(trimethylsilyl) ethoxy)methyl)pyridazin-3(2H)-one (40 mg, 0.11 mmol, 1.0 eq.). The mixture was stirred at 25 °C for 2 h. The mixture quenched with water (30 mL) and extracted with EtOAc (2 x 40 mL). The organic layers were combined and washed with brine (2 x 40 mL), dried over Na₂SO₄ and concentrated under vacuum. The residue was purified by silica gel chromatography (Petroleum ether/EtOAc= 2/3; V/V) to afford (S)-N-(2-((6-oxo-5-(trifluoromethyl)-1-((2-(trimethylsilyl)ethoxy)methyl)-1,6-dihydropyridazin-4-yl)amino)propoxy)-1-(1-(5-(trifluoromethyl)pyrimidin-2-yl)piperidi n-4-yl)cyclopropanecarboxamide (55 mg, 77.4%) as a light yellow oil.
LCMS: *(ESI)* (M+H)⁺ = 680.2.

### Step D: synthesis of (S)-N-(2-((6-oxo-5-(trifluoromethyl)-1,6-dihydropyridazin-4-yl) amino)propoxy)-1-(1-(5-(trifluoromethyl)pyrimidin-2-yl)piperidin-4-yl) cyclopropanecarboxamide

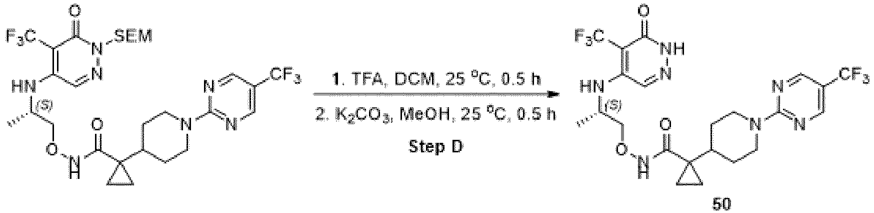

To a solution of (S)-N-(2-((6-oxo-5-(trifluoromethyl)-1-((2-(trimethylsilyl)ethoxy) methyl)-1,6-dihydropyridazin-4-yl)amino)propoxy)-1-(1-(5-(trifluoromethyl)pyrimidin-2-yl)piperidin-4-yl)cyclopropanecarboxamide (45 mg, 0.066 mmol, 1.0 eq.) in DCM (3 mL) was added TFA (1 mL). The mixture was stirred at 25 °C for 0.5 h. The mixture was concentrated under vacuum. The residue was dissolved MeOH (2 mL) and K₂CO₃ (40 mg, 0.29 mmol) was added to the mixture. The mixture was stirred at 25 °C for another 0.5 h. The mixture was filtered and the filtrate was purified by prep-HPLC to afford (S)-N-(2-((6-oxo-5-(trifluoromethyl)-1,6-dihydropyridazin-4-yl)amino)propoxy) -1-(1-(5-(trifluoromethyl)pyrimidin-2-yl)piperidin-4-yl)cyclopropanecarboxamide (6.57 mg, 18.1%) as a white solid.
LCMS: *(ESI)* (M+H)⁺ = 550.2.
¹H NMR: (400 MHz, DMSO-*d₆*) *δ* 12.49 (s, 1H), 11.07 (s, 1H), 8.70 - 8.60 (m, 2H), 7.87 (s, 1H), 6.66 - 6.57 (m, 1H), 4.78 (d, *J* = 12.0 Hz, 2H), 4.22 - 4.09 (m, 1H), 3.88 - 3.74 (m, 2H), 2.92 - 2.80 (m, 2H), 1.81 - 1.61 (m, 3H), 1.28 - 1.19 (m, 2H), 1.17 (d, *J* = 6.8 Hz, 3H), 0.80 - 0.72 (m, 2H), 0.68 - 0.60 (m, 2H).

### Example 51:

### N-((S)-2-((6-oxo-5-(trifluoromethyl)-1,6-dihydropyridazin-4-yl)amino)propoxy)-2-(1-( 5-(trifluoromethyl)pyrimidin-2-yl)piperidin-4-yl)propanamide

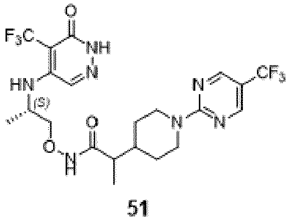

### Step A: synthesis of methyl 2-(piperidin-4-yl)propanoate hydrochloride

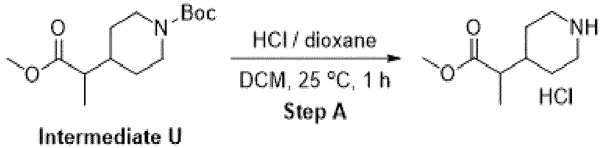

To a solution of tert-butyl 4-(1-methoxy-1-oxopropan-2-yl)piperidine-1-carboxylate (300 mg, 1.11 mmol, 1.0 eq.) in DCM (2 mL) was added HCl/dioxane (4M, 0.5 mL). The mixture was stirred at 25 °C for 1 h. The mixture was concentrated under vacuum to afford methyl 2-(piperidin-4-yl)propanoate hydrochloride (230 mg, 100%) as a white solid.
LCMS: *(ESI)* (M+H)⁺ = 172.1.

### Step B: synthesis of methyl 2-(1-(5-(trifluoromethyl)pyrimidin-2-yl)piperidin-4-yl) propanoate

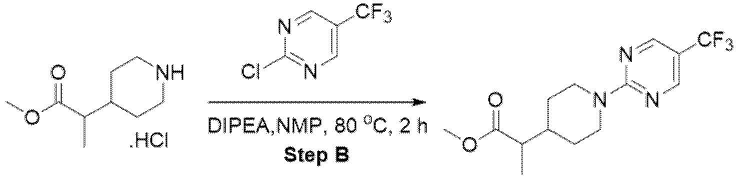

To a solution of 2-chloro-5-(trifluoromethyl)pyrimidine (235 mg, 1.29 mmol, 1.0 eq.) in NMP (20 mL) were added methyl 2-(piperidin-4-yl)propanoate hydrochloride (267 mg, 1.29 mmol, 1.0 eq.) and DIPEA (498 mg, 3.85 mmol, 3.0 eq.). The mixture was stirred at 80 °C for 2 h. The reaction was cooled to room temperature and diluted with water (80 mL). The mixture was extracted with EtOAc (3 x 50 mL). The organic layers were combined and washed with brine (2 x 50 mL), dried over Na₂SO₄ and concentrated under vacuum. The residue was purified by silica gel chromatography (Petroleum ether/EtOAc= 1/1; V/V) to afford methyl 2-(1-(5-(trifluoromethyl) pyrimidin-2-yl)piperidin-4-yl)propanoate (320 mg , 78.5%) as a yellow solid.
LCMS: *(ESI)* (M+H)⁺ = 318.1.
¹H NMR: (400 MHz, CDCl₃) *δ* 8.46 (s, 2H), 4.93 - 4.82 (m, 2H), 3.69 (s, 3H), 2.89 (td, *J* = 12.8, 2.4 Hz, 2H), 2.37 - 2.28 (m, 1H), 1.93 - 1.78 (m, 2H), 1.73 - 1.66 (m, 1H), 1.34 - 1.20 (m, 2H), 1.16 (d, *J* = 7.2 Hz, 3H).

### Step C: synthesis of 2-(1-(5-(trifluoromethyl)pyrimidin-2-yl)piperidin-4-yl)propanoic acid

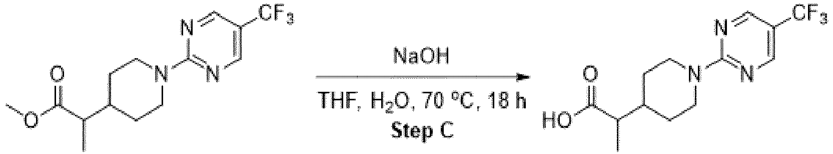

To a solution of methyl 2-(1-(5-(trifluoromethyl)pyrimidin-2-yl)piperidin-4-yl) propanoate (110 mg, 0.35 mmol, 1.0 eq.) in THF (2 mL) and H₂O (1 mL) was added NaOH (55 mg, 1.39 mmol, 4.0 eq.). The mixture was heated to 70 °C and stirred for 18 h. The mixture was cooled to room temperature and diluted with water (15 mL), acidified to pH 5 with 1M HCl. The mixture was extracted with EtOAc (3 x 40 mL). The organic layers were combined and dried over Na₂SO₄, concentrated under vacuum to afford 2-(1-(5-(trifluoromethyl)pyrimidin-2-yl)piperidin-4-yl)propanoic acid (105 mg, 99.9%) as a light yellow solid.
LCMS: *(ESI)* (M+H)⁺ = 304.0.

### Step D: synthesis of N-((S)-2-((6-oxo-5-(trifluoromethyl)-1-((2-(trimethylsilyl)ethoxy) methyl)-1,6-dihydropyridazin-4-yl)amino)propoxy)-2-(1-(5-(trifluoromethyl)pyrimidin-2-yl)piperidin-4-yl)propanamide

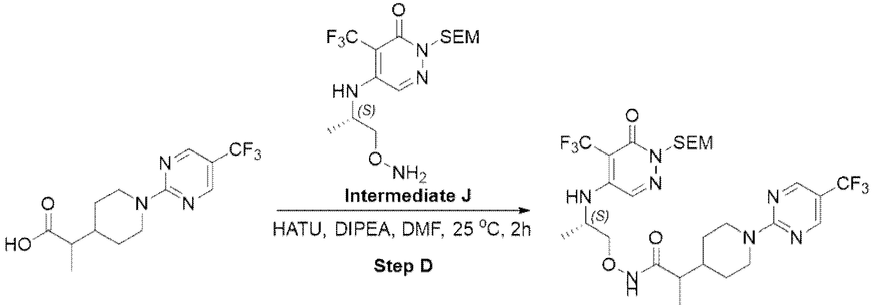

To a solution of 2-(1-(5-(trifluoromethyl)pyrimidin-2-yl)piperidin-4-yl)propanoic acid (70 mg, 0.23 mmol, 1.1 eq.) in DMF (3 mL) were added DIPEA (135 mg, 1.05 mmol, 5.0 eq.), HATU (95 mg, 0.25 mmol, 1.2 eq.) and followed by (S)-5-((1-(aminooxy)propan-2-yl)amino)-4-(trifluoromethyl)-2-((2-(trimethylsilyl)ethox y)methyl)pyridazin-3(2H)-one (80 mg, 0.21 mmol, 1.0 eq.). The mixture was stirred at 25 °C for 2 h. The mixture was diluted with saturated NH₄Cl aqueous solution (30 mL) and extracted with EtOAc (2 x 50 mL). The organic layers were combined and washed with brine (2 x 40 mL), dried over Na₂SO₄ and concentrated under vacuum. The residue was purified by silica gel chromatography (Petroleum ether/EtOAc= 3/7; V/V) to afford N-((S)-2-((6-oxo-5-(trifluoromethyl)-1-((2-(trimethylsilyl)ethoxy)methyl)-1,6-dihydrop yridazin-4-yl)amino)propoxy)-2-(1-(5-(trifluoromethyl)pyrimidin-2-yl)piperidin-4-yl)pr opanamide (70 mg, 50.2%) as a light yellow solid.
LCMS: *(ESI)* (M+H)⁺ = 668.4.

### Step E: synthesis of N-((S)-2-((6-oxo-5-(trifluoromethyl)-L6-dihydropyridazin-4-yl) amino)propoxy)-2-(1-(5-(trifluoromethyl)pyrimidin-2-yl)piperidin-4-yl)propanamide

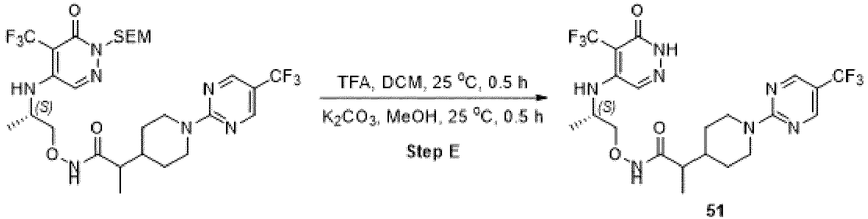

To a solution of N-((S)-2-((6-oxo-5-(trifluoromethyl)-1-((2-(trimethylsilyl)ethoxy) methyl)-1,6-dihydropyridazin-4-yl)amino)propoxy)-2-(1-(5-(trifluoromethyl)pyrimidin-2-yl)piperidin-4-yl)propanamide (90 mg, 0.14 mmol, 1.0 eq.) in DCM (4 mL) was added TFA (1 mL). The mixture was stirred at 25 °C for 0.5 h. The mixture was concentrated under vacuum. The residue was dissolved MeOH (2 mL) and K₂CO₃ (40 mg, 0.29 mmol) was added to the mixture. The mixture was stirred at 25 °C for another 0.5 h. The mixture was filtered and the filtrate was purified by prep-HPLC to afford N-((S)-2-((6-oxo-5-(trifluoromethyl)-1,6-dihydropyridazin-4-yl)amino)propoxy)-2-(1-( 5-(trifluoromethyl)pyrimidin-2-yl)piperidin-4-yl)propanamide (17.08 mg, 23.5%) as a white solid.
LCMS: *(ESI)* (M+H)⁺ = 538.2.
¹H NMR: (400 MHz, DMSO-*d₆*) *δ* 12.49 (s, 1H), 11.14 (s, 1H), 8.66 (s, 2H), 7.90 (s, 1H), 6.64 - 6.53 (m, 1H), 4.72 (t, *J* = 13.6 Hz, 2H), 4.25 - 4.12 (m, 1H), 3.96 - 3.78 (m, 2H), 3.01 - 2.84 (m, 2H), 1.89 - 1.78 (m, 2H), 1.77 - 1.67 (m, 1H), 1.62 (d, *J* = 12.4 Hz, 1H), 1.19 (d, *J* = 6.8 Hz, 3H), 1.16 - 0.96 (m, 5H).

### Example 52:

### 2-(3-methyl-1-(5-(trifluoromethyl)pyrimidin-2-yl)piperidin-4-yl)-N-((S)-2-((6-oxo-5-(trifluoromethyl)-1,6-dihydropyridazin-4-yl)amino)propoxy)acetamide

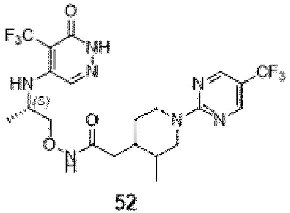

### Step A: synthesis of tert-butyl (E)-4-(2-methoxy-2-oxoethylidene)-3-methylpiperidine -1-carboxylate

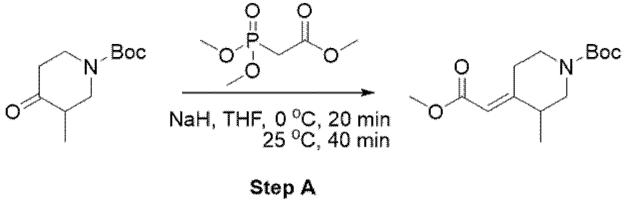

To a solution of methyl 2-(dimethoxyphosphoryl)acetate (95 mg, 0.517 mmol, 1.1 eq.) in THF (8 mL) was added NaH (21 mg, 0.517 mmol, 1.1 eq.) at 0 °C. The resulting mixture was stirred at 0 °C for 20 mins. Then tert-butyl 3-methyl-4-oxopiperidine-1-carboxylate (100 mg, 0.47 mmol, 1.0 eq.) was added at 0 °C to the mixture. The resulting mixture was stirred at 25 °C for 40 mins. The mixture was quenched by water (15 mL), extracted with EtOAc (3 x 20 mL). The organic layers were combined, dried over Na₂SO₄ and concentrated under reduced pressure. The residue was purified by column chromatography (Petroleum ether/EtOAc =4/1, V/V) to afford tert-butyl (E)-4-(2-methoxy-2-oxoethylidene)-3-methylpiperidine -1-carboxylate (120 mg, 95.2%) as a colorless oil.
LCMS: *(ESI)* (M+H-100)⁺ = 170.2.
¹H NMR (400 MHz, DMSO-*d₆) δ* 5.70 (d, J = 1.6 Hz, 1H), 3.90 - 3.76 (m, 1H), 3.62 (s, 3H), 3.58 - 3.40 (m, 2H), 3.18 - 2.79 (m, 3H), 2.46 - 2.36 (m, 1H), 1.41 (s, 9H), 1.02 (d, J = 6.8 Hz, 3H).

### Step B: Synthesis of tert-butyl 4-(2-methoxy-2-oxoethyl)-3-methylpiperidine-1-carboxylate

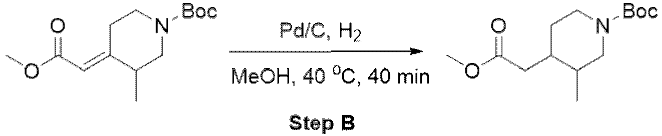

To a solution of tert-butyl (E)-4-(2-methoxy-2-oxoethylidene)-3-methylpiperidine -1-carboxylate (120 mg, 0.44 mmol, 1.0 eq.) in MeOH (2 mL) was added Pd/C (12 mg). The reaction mixture was degassed and purged with H₂ several times. The mixture was stirred at 40 °C for 40 mins under H₂ atmosphere. The mixture was filtered through a pad of celite, washed with MeOH. The filtrate was concentrated under vacuum to afford tert-butyl 4-(2-methoxy-2-oxoethyl)-3-methylpiperidine-1-carboxylate (110 mg, 91.6% yield) as a colorless oil.
LCMS: *(ESI)* (M+H-56)⁺ = 216.2.
¹H NMR: (400 MHz, DMSO-*d₆*) *δ* 3.91 - 3.77 (m, 1H), 3.62 (ddd, J = 14.1, 4.0, 2.0 Hz, 1H), 3.59 (s, 3H), 3.03 - 2.88 (m, 1H), 2.85 - 2.64 (m, 1H), 2.23 (dd, J = 7.5, 3.1 Hz, 2H), 2.08 - 1.98 (m, 1H), 1.75 (qt, J = 7.2, 3.4 Hz, 1H), 1.38 (s, 9H), 1.36 - 1.31 (m, 1H), 1.30 - 1.19 (m, 1H), 0.76 (d, J = 7.0 Hz, 3H).

### Step C: Synthesis of methyl 2-(3-methylpiperidin-4-yl)acetate hydrochloride

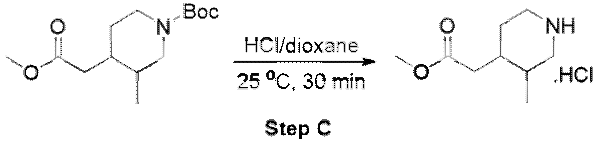

To a solution of tert-butyl 4-(2-methoxy-2-oxoethyl)-3-methylpiperidine-1-carboxylate (110 mg, 0.406 mmol, 1.0 eq.) in MeOH (1 mL) was added HCl-dioxnae (4 M, 1 mL). The mixture was stirred at 25 °C for 30 mins. The mixture was concentrated under vacuum to afford methyl 2-(3-methylpiperidin-4-yl)acetate hydrochloride (70 mg, 100% yield). The obtained product was used in the next reaction step without further purification.

### Step D: synthesis of methyl 2-(3-methyl-1-(5-(trifluoromethyl)pyrimidin-2-yl)piperidin-4-yl)acetate

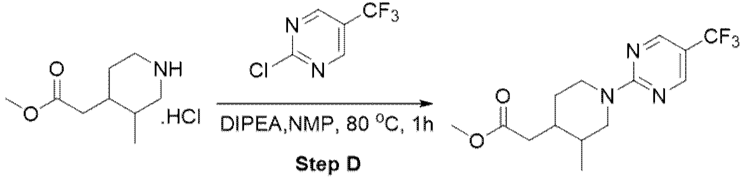

To a solution of methyl 2-(3-methylpiperidin-4-yl)acetate hydrochloride (180 mg, 1.06 mmol, 1.1 eq.) in NMP (3 mL) was added DIPEA (372 mg, 2.88 mmol, 3.0 eq.) and 2-chloro-5-(trifluoromethyl)pyrimidine (300 mg, 1.32 mmol, 1 eq.). The resulting mixture was stirred at 80 °C for 1 h. The mixture was cooled to room temperature and diluted with water (25 mL). The mixture was extracted with EtOAc (2 x 20 mL). The organic layers were combined, dried over Na₂SO₄ and concentrated under reduced pressure. The residue was purified by column chromatography (Petroleum ether/EtOAc= 9/1, V/V) to afford methyl 2-(3-methyl-1-(5-(trifluoromethyl)pyrimidin -2-yl)piperidin-4-yl)acetate (200 mg, 60.8%) as a pale yellow oil.
LCMS: *(ESI)* (M+H)⁺ = 318.2.
¹H NMR: (400 MHz, DMSO-*d₆*) *δ* 8.64 (s, 2H), 4.49 (dtd, J = 13.2, 4.3, 1.5 Hz, 1H), 4.36 (ddd, J = 13.2, 4.1, 1.7 Hz, 1H), 3.61 (s, 3H), 3.36 - 3.29 (m, 1H), 3.19 (ddd, J = 13.6, 11.0, 3.6 Hz, 1H), 2.32 - 2.28 (m, 2H), 2.26 - 2.16 (m, 1H), 1.94 (tq, J = 7.1, 3.6 Hz, 1H), 1.58 - 1.50 (m, 1H), 1.47 - 1.39 (m, 1H), 0.75 (d, J = 7.0 Hz, 3H).

### Step E: Synthesis of 2-(3-methyl-1-(5-(trifluoromethyl)pyrimidin-2-yl)piperidin-4-yl) acetic acid

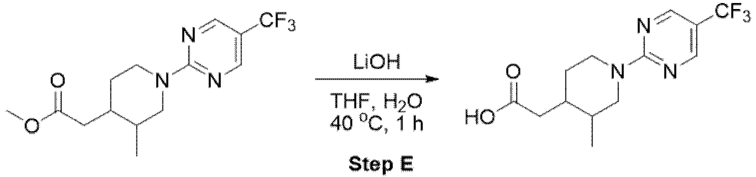

To a solution of methyl 2-(3-methyl-1-(5-(trifluoromethyl)pyrimidin-2-yl)piperidin -4-yl)acetate (240 mg, 0.723 mmol, 1.0 eq.) in THF (3 mL) and water (3 mL) was added LiOH (70 mg, 2.9 mmol, 4.0 eq.). The mixture was stirred at 40 °C for 1 h. The mixture was diluted with water (25 mL) and extracted with EtOAc (3 x 10 mL). The water layer was adjusted to pH 6 with 2M HCl. The mixture was extracted with EtOAc (3 x 15 mL), dried over Na₂SO₄ and concentrated under vacuum to afford 2-(3-methyl-1-(5-(trifluoromethyl)pyrimidin-2-yl)piperidin-4-yl)acetic acid (140 mg, 61.3% yield) as a colorless oil.
LCMS: *(ESI)* (M+H)⁺ = 304.1.

### Step F: synthesis of 2-(3-methyl-1-(5-(trifluoromethyl)pyrimidin-2-yl)piperidin-4-yl) -N-((S)-2-((6-oxo-5-(trifluoromethyl)-1-((2-(trimethylsilyl)ethoxy)methyl)-1,6-dihydro pyridazin-4-yl)amino)propoxy)acetamide

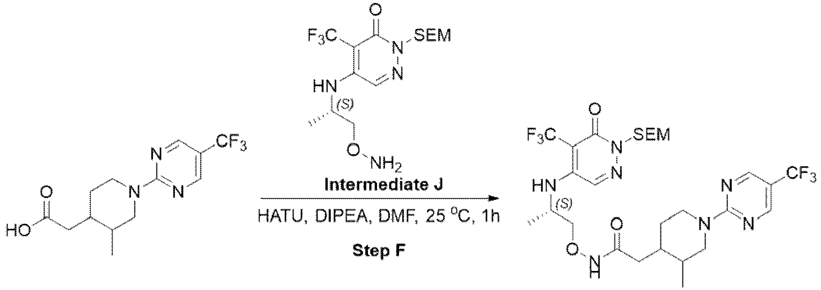

To a solution of 2-(3-methyl-1-(5-(trifluoromethyl)pyrimidin-2-yl)piperidin-4-yl) acetic acid (160 mg, 0.42 mmol, 1 eq.) in THF (5 mL) were added DIPEA (109 mg, 0.84 mmol, 2.0 eq.) and HATU (239 mg, 0.63 mmol, 1.5 eq.) followed by dropwise addition of (S)-5-((1-(aminooxy)propan-2-yl)amino)-4-(trifluoromethyl)-2-((2-(trimethylsilyl)ethoxy)methyl)pyridazin-3(2H)-one (140 mg, 0.46 mmol, 1.1 eq.).The resulting mixture was stirred at 25 °C for 1 h. The reaction mixture was quenched with water (10 mL) and extracted with EtOAc (3 x 10 mL). The organic lyaers were combined and washed with brine (10 mL), dried over Na₂SO₄ and concentrated under vacuum. The residue was purified by silica gel chromatography (Petroleum ether/EtOAc= 4/6, V/V) to afford 2-(3-methyl-1-(5-(trifluoromethyl)pyrimidin-2-yl) piperidin-4-yl)-N-((S)-2-((6-oxo-5-(trifluoromethyl)-1-((2-(trimethylsilyl)ethoxy)methy l)-1,6-dihydropyridazin-4-yl)amino)propoxy)acetamide (170 mg, 48.9% yield) as a colorless oil.
LCMS: *(ESI)* (M+H)⁺ = 668.3.

### Step G: synthesis of 2-(3-methyl-1-(5-(trifluoromethyl)pyrimidin-2-yl) piperidin-4-yl)-N-((S)-2-((6-oxo-5-(trifluoromethyl)-1,6-dihydropyridazin-4-yl)amino) propoxy)acetamide

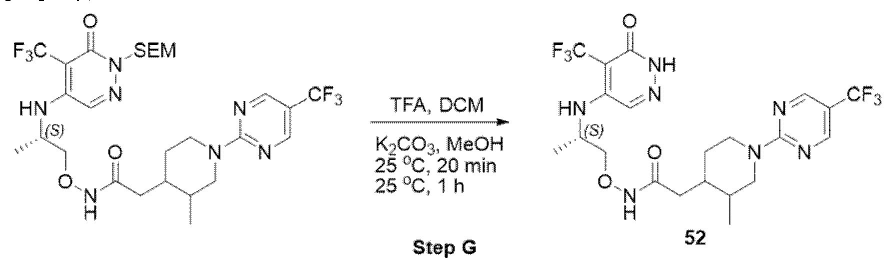

To a solution of 2-(3-methyl-1-(5-(trifluoromethyl)pyrimidin-2-yl)piperidin-4-yl) -N-((S)-2-((6-oxo-5-(trifluoromethyl)-1-((2-(trimethylsilyl)ethoxy)methyl)-1,6-dihydro pyridazin-4-yl)amino)propoxy)acetamide (170 mg, 0.256 mmol, 1.0 eq.) in DCM (2 mL) was added TFA (0.2 mL). The resulting mixture was stirred at 25 °C for 20 min. The mixture was neutralized with saturated NaHCOs aqueous solution (5 mL) and extracted with DCM (3 x 10 mL). The organic layers were combined and dried over Na₂SO₄, concentrated under vacuum. The residue was redissolved in MeOH (3 mL) and K₂CO₃ (93 mg, 0.67 mmol, 3.0 eq.) was added. The mixture was stirred at 25 °C for another 1 h. The reaction mixture was filtered and the filtrate was purified by silica gel chromatography (Petroleum ether/EtOAc= 1/100, V/V) to afford 2-(3-methyl-1-(5-(trifluoromethyl)pyrimidin-2-yl)piperidin-4-yl)-N-((S)-2-((6-oxo-5-(tr ifluoromethyl)-1,6-dihydropyridazin-4-yl)amino)propoxy)acetamide (5.7 mg, 4.1% yield) as a white solid.
LCMS: LCMS *(ESI)* (M+H)⁺ = 538.4.
¹H NMR: (400 MHz, DMSO-*d₆*) *δ* 12.52 (s, 1H), 11.20 (s, 1H), 8.66 (s, 2H), 7.92 (s, 1H), 6.63 (s, 1H), 4.52 (d, J = 13.3 Hz, 1H), 4.37 (d, J = 13.2 Hz, 1H), 4.27 - 4.13 (m, 1H), 4.00 - 3.80 (m, 2H), 3.33 - 3.28 (m, 1H), 3.20 - 3.11 (m, 1H), 2.05 - 1.88 (m, 3H), 1.54 - 1.45 (m, 1H), 1.45 - 1.33 (m, 1H), 1.23 - 1.16 (m, 4H), 0.77 (d, J = 6.9 Hz, 3H).

### Example 53:

### 2-(3-fluoro-1-(5-(trifluoromethyl)pyrimidin-2-yl)piperidin-4-yl)-N-((S)-2-((6-oxo-5-(trifluoromethyl)-1,6-dihydropyridazin-4-yl)amino)propoxy)acetamide

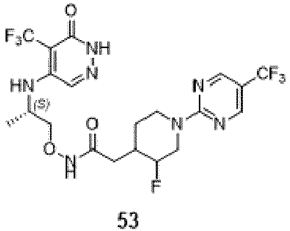

### Step A: synthesis of (E)-tert-butyl 3-fluoro-4-(2-methoxy-2-oxoethylidene)piperidine-1-carboxylate

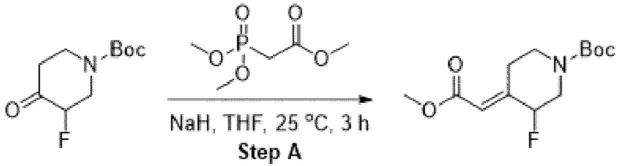

To a solution of trimethyl phosphonoacetate (755 mg, 4.14 mmol, 1.0 eq.) in THF (12 mL) was added NaH (60%, 174 mg, 4.35 mmol, 1.05 eq.) at 0°C in ice-water bath under N₂ atmosphere. The mixture was stirred at 0 °C for 0.5 h. Then a solution of tert-butyl 3-fluoro-4-oxopiperidine-1-carboxylate (900 mg, 4.14 mmol, 1.0 eq.) in THF (4 mL) was added dropwise to the above reaction mixture. The mixtue and stirred at 25 °C for 3 h. The mixture was quenched with saturated aqueous NH₄Cl (30 mL) and extracted with EtOAc (2 x 40 mL). The organic layers were combined and dried over Na₂SO₄, concentrated under vacuum. The residue was purified by silica gel chromatography (Petroleum ether/EtOAc= 10/1; V/V) to afford (E)-tert-butyl 3-fluoro-4-(2-methoxy-2-oxoethylidene)piperidine-1-carboxylate (280 mg, 24.7%) as a white solid.
LCMS: *(ESI)* (M+H-100)⁺ = 174.1.
¹H NMR: (400 MHz, CDCl₃) *δ* 6.01 (s, 1H), 4.86 (d, *J=* 47.6 Hz, 1H), 4.19 - 3.82 (m, 1H), 3.75 (s, 3H), 3.74 - 3.69 (m, 1H), 3.53 - 3.13 (m, 3H), 2.58 (s, 1H), 1.49 (s, 9H).

### Step B: synthesis of tert-butyl 3-fluoro-4-(2-methoxy-2-oxoethyl)piperidine-1-carboxylate

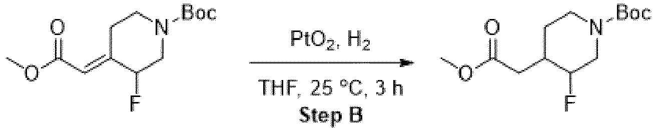

To a mixture of (E)-tert-butyl 3-fluoro-4-(2-methoxy-2-oxoethylidene)piperidine -1-carboxylate (250 mg, 0.92 mmol, 1.0 eq.) in THF (6 mL) was added PtO₂ (46 mg). The mixture was stirred at 25 °C under H₂ atmosphere for 3 h. The mixture was filtered and the filtrate was concentrated under vacuum to afford tert-butyl 3-fluoro-4-(2-methoxy-2-oxoethyl)piperidine-1-carboxylate (250 mg, 99.3%) as a white solid.
LCMS: *(ESI)* (M+H-56)⁺ = 220.2.

### Step C: synthesis of methyl 2-(3-fluoropiperidin-4-yl)acetate hydrochloride

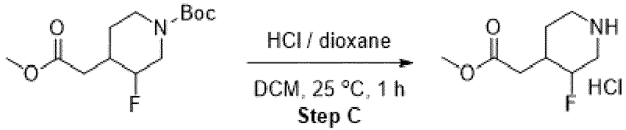

To a mixture of tert-butyl 3-fluoro-4-(2-methoxy-2-oxoethyl)piperidine-1-carboxylate (250 mg, 0.91 mmol, 1.0 eq.) in DCM (6 mL) was added HCl/dioxane (4 M, 3 mL). The mixture was stirred at 25 °C for 1 h. The mixture was concentrated under vacuum to afford methyl 2-(3-fluoropiperidin-4-yl)acetate hydrochloride (170 mg, 88.5%) as a white solid.
LCMS: *(ESI)* (M+H)⁺ = 176.1.

### Step D: synthesis of methyl 2-(3-fluoro-1-(5-(trifluoromethyl)pyrimidin-2-yl)piperidin -4-yl)acetate

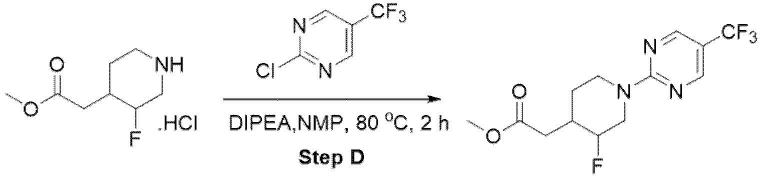

A mixture of methyl 2-(3-fluoropiperidin-4-yl)acetate hydrochloride (165 mg, 0.78 mmol, 1.0 eq.) in NMP (2 mL) were added DIPEA (302 mg, 2.34 mmol, 3.0 eq.) and 2-chloro-5-(trifluoromethyl)pyrimidine (157 mg, 0.86 mmol, 1.1 eq.). The mixture was stirred at 80 °C for 2 h. The mixture was cooled to room temperature and diluted with water (30 mL), extracted with EtOAc (2 x 50 mL). The organic layers were combined and washed with brine (2 x 50 mL), dried over Na₂SO₄ and concentrated under vacuum. The residue was purified by column chromatography (Petroleum ether/EtOAc= 7/1; V/V) to afford methyl 2-(3-fluoro-1-(5-(trifluoromethyl)pyrimidin-2-yl)piperidin-4-yl) acetate (100 mg, 39.9%) as a white solid.
LCMS: *(ESI)* (M+H)⁺ = 322.1.
¹H NMR: (400 MHz, CDCl₃) *δ* 8.48 (s, 2H), 5.31 - 5.22 (m, 1H), 4.97 - 4.92 (m, 1H), 4.81 (d, *J* = 47.6 Hz, 1H), 3.71 (s, 3H), 3.18 - 3.02 (m, 1H), 3.01 - 2.92 (m, 1H), 2.63 -2.53 (m, 1H), 2.40 - 2.32 (m, 1H), 2.31 - 2.18 (m, 1H), 1.71 - 1.65 (m, 2H).

### Step E: synthesis of 2-(3-fluoro-1-(5-(trifluoromethyl)pyrimidin-2-yl)piperidin-4-yl) acetic acid

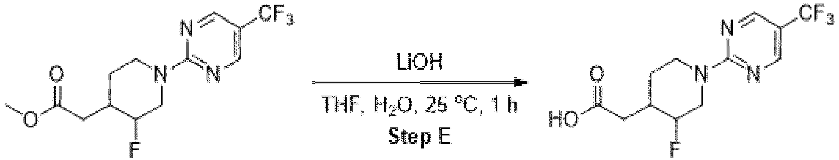

To a mixture of methyl 2-(3-fluoro-1-(5-(trifluoromethyl)pyrimidin-2-yl)piperidin -4-yl)acetate (95 mg, 0.30 mmol, 1.0 eq.) in THF (3 mL) and H₂O (1.5 mL) was added lithium hydroxide (29 mg, 1.18 mmol, 4.0 eq.). The mixture was stirred at 25 °C for 1 h. The mixture was diluted with water (20 mL) adjusted pH 6 with 1M HCl. The mixture was extracted with EtOAc (2 x 50 mL). The organic layers were combined and washed with brine (50 mL), dried over Na₂SO₄ and concentrated under vacuum to afford 2-(3-fluoro-1-(5-(trifluoromethyl)pyrimidin-2-yl)piperidin-4-yl)acetic acid (90 mg, 99.1%) as a white solid.
LCMS: *(ESI)* (M+H)⁺ = 308.1.

### Step F: Synthesis of 2-(3-fluoro-1-(5-(trifluoromethyl)pyrimidin-2-yl)piperidin-4-yl) -N-((S)-2-((6-oxo-5-(trifluoromethyl)-1-((2-(trimethylsilyl)ethoxy)methyl)-1,6-dihydro pyridazin-4-yl)amino)propoxy)acetamide

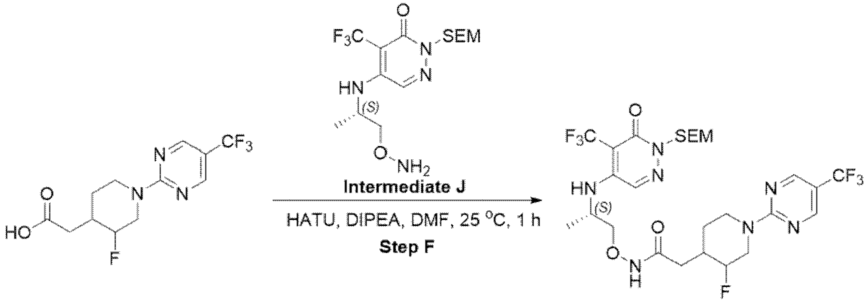

To a mixture of 2-(3-fluoro-1-(5-(trifluoromethyl)pyrimidin-2-yl)piperidin-4-yl) acetic acid (95 mg, 0.31 mmol, 1.0 eq.) in DMF (5 mL) were added (S)-5-((1-(aminooxy)propan-2-yl)amino)-4-(trifluoromethyl)-2-((2-(trimethylsilyl)ethox y)methyl)pyridazin-3(2H)-one (126 mg, 0.34 mmol, 1.1 eq.), DIPEA (120 mg, 0.93 mmol, 3.0 eq.) and HATU (142 mg, 0.37 mmol, 1.2 eq.). The mixture was stirred at 25 °C for 1 h. The mixture was quenched with water (30 mL) and extracted with EtOAc (2 x 50 mL). The organic layers were combined and washed with brine (2 x 50 mL), dried over Na₂SO₄ and concentrated under vacuum. The residue was purified by silica gel chromatography (Petroleum ether/EtOAc= 3/7; V/V) to afford 2-(3-fluoro-1-(5-(trifluoromethyl)pyrimidin-2-yl)piperidin-4-yl)-N-((S)-2-((6-oxo-5-(tri fluoromethyl)-1-((2-(trimethylsilyl)ethoxy)methyl)-1,6-dihydropyridazin-4-yl)amino)pr opoxy)acetamide (140 mg, 68.9%) as a white solid.
LCMS: *(ESI)* (M+H)⁺ = 672.2.

### Step G: synthesis of 2-(3-fluoro-1-(5-(trifluoromethyl)pyrimidin-2-yl)piperidin-4-yl)-N-((S)-2-((6-oxo-5-(trifluoromethyl)-1,6-dihydropyridazin-4-yl)amino)propoxy)acetamide

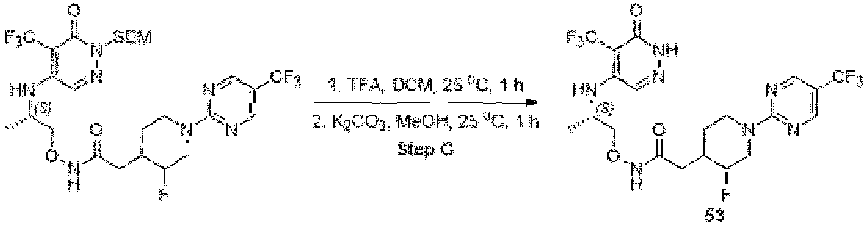

To a mixture of 2-(3-fluoro-1-(5-(trifluoromethyl)pyrimidin-2-yl)piperidin-4-yl) -N-((S)-2-((6-oxo-5-(trifluoromethyl)-1-((2-(trimethylsilyl)ethoxy)methyl)-1,6-dihydropyridazin-4-yl)amino)propoxy)acetamide (130 mg, 0.2 mmol, 1.0 eq.) in DCM (6 mL) was added TFA (2 mL). The mixture was stirred at 25 °C for 1 h. The mixture was concentrated under vacuum. The residue was redissolved in MeOH (4 mL) and K₂CO₃ (126 mg, 0.91 mmol) was added. The mixture was stirred at 25 °C for another 1 h. The mixture was filtered and filtrate was purified by prep-HPLC to afford 2-(3-fluoro-1-(5-(trifluoromethyl)pyrimidin-2-yl)piperidin-4-yl)-N-((S)-2-((6-oxo-5-(trifluoromethyl)-1,6-dihydropyridazin-4-yl)amino)propoxy)acetamide (24.34 mg, 22.7%) as a white solid.
LCMS: *(ESI)* (M+H)⁺ = 542.2.
¹H NMR: (400 MHz, DMSO-*d₆*) *δ* 12.49 (s, 1H), 11.22 (s, 1H), 8.68 (s, 2H), 7.91 (s, 1H), 6.60 (s, 1H), 5.10 - 4.98 (m, 1H), 4.86 - 4.71 (m, 2H), 4.28 - 4.15 (m, 1H), 3.97 - 3.80 (m, 2H), 3.27 - 3.15 (m, 1H), 3.02 (t, *J=* 12.8 Hz, 1H), 2.34 - 2.27 (m, 1H), 2.19 - 2.12 (m, 1H), 2.03 - 1.93 (m, 1H), 1.63 - 1.54 (m, 1H), 1.47 - 1.33 (m, 1H), 1.19 (d, *J* = 6.4 Hz, 3H).

### Example 54:

### 2-((3S,4R)-3-hydroxy-1-(5-(trifluoromethyl)pyrimidin-2-yl)piperidin-4-yl)-N-((S)-2-((6 -oxo-5-(trifluoromethyl)-1,6-dihydropyridazin-4-yl)amino)propoxy)acetamide

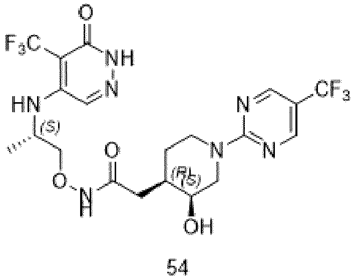

### Step A: synthesis of tert-butyl 3-hydroxy-4-(2-methoxy-2-oxoethylidene)piperidine-1-carboxylate

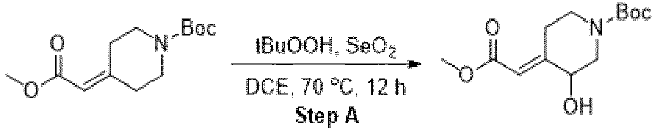

To a solution of tert-Butyl hydroperoxide (1.59 g, 17.60 mmol, 1.0 eq.) in DCE (60 mL) were added tert-butyl 4-(2-methoxy-2-oxoethylidene)piperidine-1-carboxylate (4.50 g, 17.60 mmol, 1.0 eq.) and SeO₂ (1.96 g, 17.60 mmol, 1.0 eq.). The mixture was stirred at 70 °C for 12 h. The reaction mixture was cooled to room temperature and diluted with water (40 mL), extracted with DCM (3 x 50 mL). The organic extracts were combined and washed with brine (50 mL), dried over Na₂SO₄ and concentrated under vacuum. The residue was purified by flash chromatography (EtOAc/Petroleum ether= 1/1; V/V) to afford tert-butyl 3-hydroxy-4-(2-methoxy-2-oxoethylidene)piperidine-1-carboxylate (740 mg, 15.5%) as a yellow solid.
LCMS: *(ESI)* (M+H-100)⁺ = 172.2.
¹H NMR: (400 MHz, CDCl₃) *δ* 6.05 (s, 1H), 4.21 - 4.12 (m, 1H), 4.02 (d, *J* = 11.2 Hz, 1H), 3.84 - 3.74 (m, 1H), 3.72 (s, 3H), 3.54 (dt, *J* = 14.4, 4.4 Hz, 1H), 3.19 - 2.99 (m, 2H), 2.48 - 2.37 (m, 1H), 1.47 (s, 9H).

### Step B: synthesis of tert-butyl 3-hydroxy-4-(2-methoxy-2-oxoethyl)piperidine-1-carboxylate

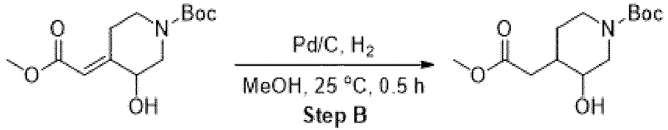

To a mixture of tert-butyl 3-hydroxy-4-(2-methoxy-2-oxoethylidene)piperidine-1-carboxylate (740 mg, 2.72 mmol, 1.0 eq.) in MeOH (10 mL) was added Pd/C (10%, 483 mg) under N₂ atmosphere. The reaction mixture was degassed and purged with H₂ for 3 times. The mixture was stirred at 25 °C under H₂ atmosphere for 0.5 h. The mixture was filtered and the filtrate was concentrated under vacuum to afford tert-butyl 3-hydroxy-4-(2-methoxy-2-oxoethyl)piperidine-1-carboxylate (670 mg, 89.9%) as a colorless oil.
LCMS: *(ESI)* (M+Na)⁺ = 295.9.

### Step C: synthesis of methyl 2-(3-hydroxypiperidin-4-yl)acetate hydrochloride

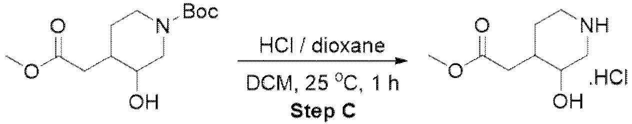

To a mixture of tert-butyl 3-hydroxy-4-(2-methoxy-2-oxoethyl)piperidine-1-carboxylate (640 mg, 2.34 mmol, 1.0 eq.) in DCM (3 mL) was added HCl/dioxane (4 M, 2 mL). The mixture was stirred at 25 °C for 1 h. The mixture was concentrated under vacuum to afford methyl 2-(3-hydroxypiperidin-4-yl)acetate hydrochloride (490 mg, 99.0%) as a colorless oil.
LCMS: *(ESI)* (M+H)⁺ = 174.2.

### Step D: synthesis of methyl 2-(3-hydroxy-1-(5-(trifluoromethyl)pyrimidin-2-yl) piperidin-4-yl)acetate

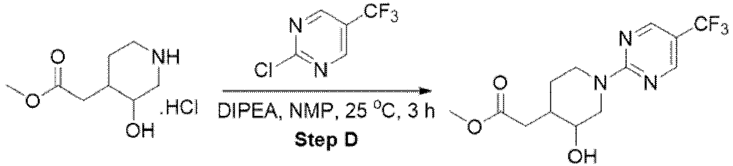

To a mixture of methyl 2-(3-hydroxypiperidin-4-yl)acetate hydrochloride (490 mg, 2.34 mmol, 1.1 eq.) and DIPEA (1.09 g, 8.44 mmol, 4.0 eq.) in NMP (12 mL) was added 2-chloro-5-(trifluoromethyl)pyrimidine (385 mg, 2.11 mmol, 1.0 eq.). The mixture was stirred at 25 °C for 3 h. The mixture was diluted with water (60 mL) and extracted with EtOAc (3 x 50 mL). The organic extracts were combined and washed with saturated aqueous NH₄Cl (2 x 50 mL), dried over Na₂SO₄ and concentrated under vacuum. The residue was purified by flash chromatography (EtOAc/Petroleum ether= 1/3; V/V) to afford methyl 2-(3-hydroxy-1-(5-(trifluoromethyl)pyrimidin-2-yl)piperidin -4-yl)acetate (170 mg, 25.3%) as a yellow solid.
LCMS: *(ESI)* (M+H)⁺ = 320.1.
¹H NMR: (400 MHz, CDCl₃) *δ* 8.49 (s, 2H), 4.99 (ddd, *J* = 12.8, 4.8, 2.0 Hz, 1H), 4.84 - 4.76 (m, 1H), 3.73 (s, 3H), 3.45 - 3.36 (m, 1H), 2.96 (td, *J* = 12.8, 2.8 Hz, 1H), 2.82 (dd, *J* = 12.8, 10.4 Hz, 1H), 2.76 - 2.71 (m, 1H), 2.45 (d, *J* = 5.2 Hz, 1H), 2.33 (dd, *J*= 16.0, 6.4 Hz, 1H), 2.14 -2.01 (m, 1H), 1.96 - 1.87 (m, 1H) , 1.55 - 1.45 (m, 1H).

### Step E: synthesis of methyl 2-(3-((tetrahydro-2H-pyran-2-yl)oxy)-1-(5-(trifluoromethyl) pyrimidin-2-yl)piperidin-4-yl)acetate

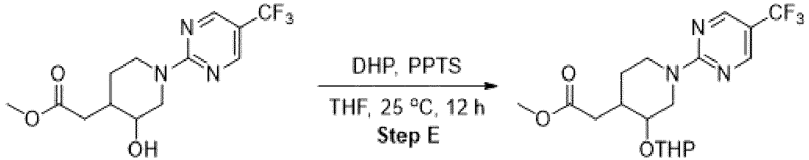

To a mixture of methyl 2-(3-hydroxy-1-(5-(trifluoromethyl)pyrimidin-2-yl) piperidin-4-yl)acetate (160 mg, 0.50 mmol, 1.0 eq.) and 3,4-dihydro-2H-pyran (129 mg, 1.50 mmol, 3.0 eq.) in THF (6 mL) was added Pyridinium p-Toluenesulfonate (13 mg, 0.05 mmol, 0.1 eq.). The reaction mixture was stirred at 25 °C for 12 h. The mixture was diluted with water (10 mL) and extracted with DCM (3 x 15 mL). The organic extracts were combined and washed with brine (2 x 10 mL), dried over Na₂SO₄ and concentrated under vacuum. The residue was purified by flash chromatography (EtOAc/Petroleum ether= 1/3; V/V) to afford methyl 2-(3-((tetrahydro-2H-pyran -2-yl)oxy)-1-(5-(trifluoromethyl)pyrimidin-2-yl)piperidin-4-yl)acetate (160 mg, 79.0%) as a yellow solid.
LCMS: *(ESI)* (M+H)⁺ = 404.2.
¹H NMR: (400 MHz, CDCl₃) *δ* 8.46 (s, 2H), 5.27 - 4.96 (m, 1H), 4.81 - 4.54 (m, 2H), 4.02 - 3.82 (m, 1H), 3.69 (d, J = 4.0 Hz, 3H), 3.63 - 3.48 (m, 1H), 3.44 - 3.21 (m, 1H), 3.01 - 2.75 (m, 2H), 2.68 - 2.45 (m, 1H), 2.24 - 2.09 (m, 2H), 2.02 - 1.90 (m, 1H), 1.86 - 1.70 (m, 3H), 1.68 - 1.60 (m, 1H), 1.56 - 1.50 (m, 2H), 1.35 - 1.24 (m, 1H).

### Step F: synthesis of 2-(3-((tetrahydro-2H-pyran-2-yl)oxy)-1-(5-(trifluoromethyl) pyrimidin-2-yl)piperidin-4-yl)acetic acid

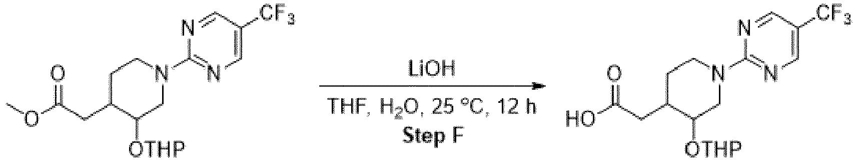

To a mixture of methyl 2-(3-((tetrahydro-2H-pyran-2-yl)oxy)-1-(5-(trifluoromethyl) pyrimidin-2-yl)piperidin-4-yl)acetate (160 mg, 0.40 mmol, 1.0 eq.) in THF (3 mL) and H₂O (1 mL) was added LiOH (50 mg, 1.20 mmol, 3.0 eq.). The mixture was stirred at 25 °C for 12 h. The mixture was diluted with water (6 mL) and neutralized to pH 7 with 1M HCl. The mixture was extracted with EtOAc (3 x 10 mL). The organic layers were combined and washed with brine (10 mL), dried over Na₂SO₄ and concentrated under vacuum to afford 2-(3-((tetrahydro-2H-pyran-2-yl)oxy)-1-(5-(trifluoromethyl) pyrimidin-2-yl)piperidin-4-yl)acetic acid (153 mg, 98.0% yield) as a white solid.
LCMS: *(ESI)* (M+H)⁺ = 390.2.

### Step G: synthesis of N-((S)-2-((6-oxo-5-(trifluoromethyl)-1-((2-(trimethylsilyl)ethoxy) methyl)-1,6-dihydropyridazin-4-yl)amino)propoxy)-2-(3-((tetrahydro-2H-pyran-2-yl)ox y)-1-(5-(trifluoromethyl)pyrimidin-2-yl)piperidin-4-yl)acetamide

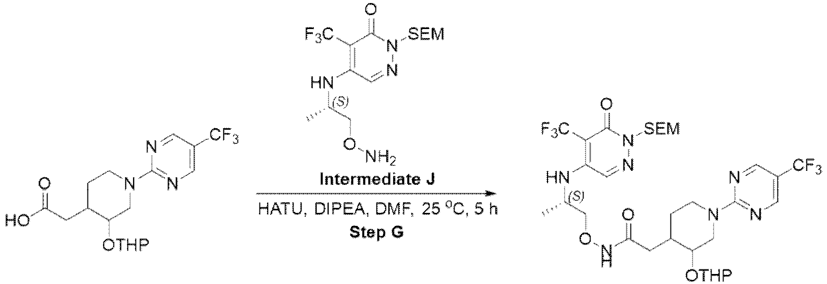

To a mixture of 2-(3-((tetrahydro-2H-pyran-2-yl)oxy)-1-(5-(trifluoromethyl) pyrimidin-2-yl)piperidin-4-yl)acetic acid (153 mg, 0.39 mmol, 1.0 eq.) and (S)-5-((1-(aminooxy)propan-2-yl)amino)-4-(trifluoromethyl)-2-((2-(trimethylsilyl)ethox y)methyl)pyridazin-3(2H)-one (164 mg, 0.43 mmol, 1.1 eq.) in DMF (5 mL) were added DIPEA (152 mg, 1.18 mmol, 3.0 eq.) and HATU (178 mg, 0.47 mmol, 1.2 eq.). The mixture was stirred at 25 °C for 5 h. The mixture was diluted with water (30 mL) and extracted with EtOAc (3 x 30 mL). The organic extracts were combined washed with saturated NH₄Cl aqueous solution (2 x 30 mL), dried over Na₂SO₄ and concentrated under vacuum. The residue was purified by prep-TLC (MeOH/DCM= 1/20; V/V) to afford N-((S)-2-((6-oxo-5-(trifluoromethyl)-1-((2-(trimethylsilyl)ethoxy) methyl)-1,6-dihydropyridazin-4-yl)amino)propoxy)-2-(3-((tetrahydro-2H-pyran-2-yl)ox y)-1-(5-(trifluoromethyl)pyrimidin-2-yl)piperidin-4-yl)acetamide (135 mg, 45.5%) as a yellow solid.
LCMS: *(ESI)* (M+Na)⁺ = 776.2.

### Step H: synthesis of 2-((3S,4R)-3-hydroxy-1-(5-(trifluoromethyl)pyrimidin-2-yl) piperidin-4-yl)-N-((S)-2-((6-oxo-5-(trifluoromethyl)-1,6-dihydropyridazin-4-yl)amino) propoxy)acetamide

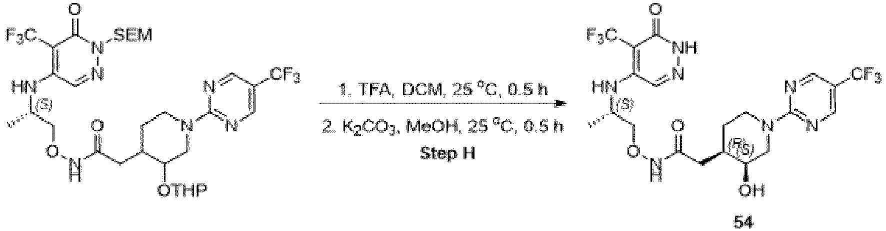

To a mixture of N-((S)-2-((6-oxo-5-(trifluoromethyl)-1-((2-(trimethylsilyl)ethoxy) methyl)-1,6-dihydropyridazin-4-yl)amino)propoxy)-2-(3-((tetrahydro-2H-pyran-2-yl) oxy)-1-(5-(trifluoromethyl)pyrimidin-2-yl)piperidin-4-yl)acetamide (65 mg, 0.086 mmol, 1.0 eq.) in DCM (2.0 mL) was added TFA (0.6 mL). The mixture was stirred at 25 °C for 0.5 h. The reaction mixture was concentrated vacuum. The residue was dissolved in MeOH (2 mL) and K₂CO₃ (36 mg, 0.26 mmol, 3.0 eq.) was added to the mixture. The mixture was stirred at 25 °C for another 0.5 h. The mixture was filtered and the filtrate was purified by prep-HPLC to afford 2-((3 S,4R)-3-hydroxy-1-(5-(trifluoromethyl)pyrimidin-2-yl)piperidin-4-yl)-N-((S)-2-((6 -oxo-5-(trifluoromethyl)-1,6-dihydropyridazin-4-yl)amino)propoxy)acetamide (9.17 mg, 21.0%) as a white solid.
LCMS: *(ESI)* (M+H)⁺ = 540.2.
¹H NMR: (400 MHz, DMSO-*d₆*) *δ* 12.51 (s, 1H), 11.14 (s, 1H), 8.69 (s, 2H), 7.93 (s, 1H), 6.64 (s, 1H), 5.22 (d, *J* = 3.2 Hz, 1H), 4.77 (dd, *J* = 12.4, 3.6 Hz, 1H), 4.63 (d, *J* = 13.2 Hz, 1H), 4.29 - 4.16 (m, 1H), 3.95 - 3.80 (m, 2H), 3.16 - 3.06 (m, 1H), 2.93 (t, *J* = 12.4 Hz, 1H), 2.80 - 2.71 (m, 1H), 2.51 - 2.45 (m, 1H), 1.95 - 1.83 (m, 1H), 1.82 - 1.66 (m, 2H), 1.21 (d, *J* = 6.4 Hz, 3H), 1.16 - 1.05 (m, 1H).

### Example 55:

### 2-((3R,4R)-3-hydroxy-1-(5-(trifluoromethyl)pyrimidin-2-yl)piperidin-4-yl)-N-((S)-2-((6 -oxo-5-(trifluoromethyl)-1,6-dihydropyridazin-4-yl)amino)proxy)acetamide

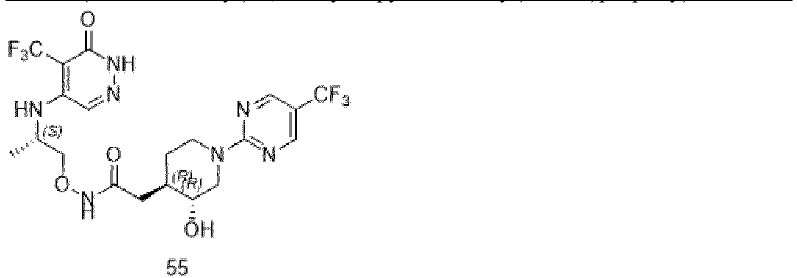

### Step A: synthesis of 2-((3R,4R)-3-hydroxy-1-(5-(trifluoromethyl)pyrimidin-2-yl) piperidin-4-yl)-N-((S)-2-((6-oxo-5-(trifluoromethyl)-1,6-dihydropyridazin-4-yl)amino) propoxy)acetamide

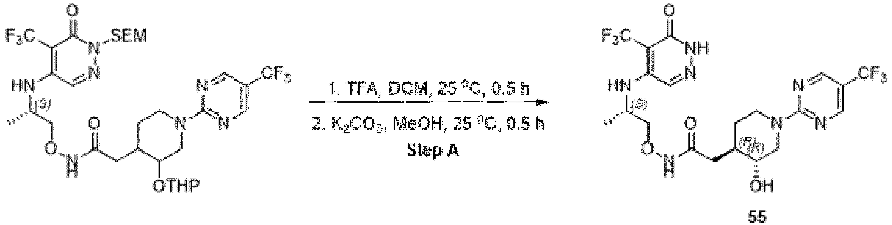

To a mixture of N-((S)-2-((6-oxo-5-(trifluoromethyl)-1-((2-(trimethylsilyl)ethoxy) methyl)-1,6-dihydropyridazin-4-yl)amino)propoxy)-2-(3-((tetrahydro-2H-pyran-2-yl) oxy)-1-(5-(trifluoromethyl)pyrimidin-2-yl)piperidin-4-yl)acetamide (65 mg, 0.086 mmol, 1.0 eq.) in DCM (2.0 mL) was added TFA (0.6 mL). The mixture was stirred at 25 °C for 0.5 h. The reaction mixture was concentrated vacuum. The residue was dissolved in MeOH (2 mL) and K₂CO₃ (36 mg, 0.26 mmol, 3.0 eq.) was added to the mixture. The mixture was stirred at 25 °C for another 0.5 h. The mixture was filtered and the filtrate was purified by prep-HPLC to afford 2-((3R,4R)-3-hydroxy-1-(5-(trifluoromethyl)pyrimidin-2-yl)piperidin-4-yl)-N-((S)-2-((6-oxo-5-(trifluoromethyl)-1, 6-dihydropyridazin-4-yl)amino)propoxy)acetamide (11.25 mg, 22.5%) as a white solid. LCMS: *(ESI)* (M+H)⁺ = 540.2.
¹H NMR: (400 MHz, DMSO-*d₆*) *δ* 12.50 (s, 1H), 11.13 (s, 1H), 8.67 (s, 2H), 7.91 (s, 1H), 6.67 - 6.56 (m, 1H), 5.25 - 5.16 (m, 1H), 4.80 - 4.71 (m, 1H), 4.62 (d, J = 13.2 Hz, 1H), 4.27 - 4.16 (m, 1H), 3.95 - 3.80 (m, 2H), 3.14 - 3.05 (m, 1H), 2.95 - 2.86 (m, 1H), 2.78 - 2.70 (m, 1H), 2.50 - 2.44 (m, 1H), 1.90 - 1.82 (m, 1H), 1.80 - 1.66 (m, 2H), 1.19 (d, J = 6.4 Hz, 3H), 1.15 - 1.05 (m, 1H).

### Example 56:

### (S)-N-(2-((6-oxo-5-(trifluoromethyl)-1,6-dihydropyridazin-4-yl)amino)propoxy)-2-(8-( 5-(trifluoromethyl)pyrimidin-2-yl)-2,8-diazaspiro(4.5)decan-2-yl)acetamide

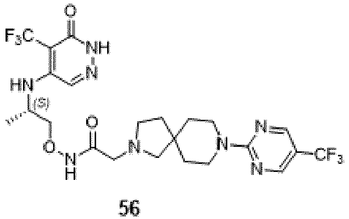

### Step A: tert-butyl 8-(5-(trifluoromethyl)pyrimidin-2-yl)-2,8-diazaspiro(4.5)decane-2-carboxylate

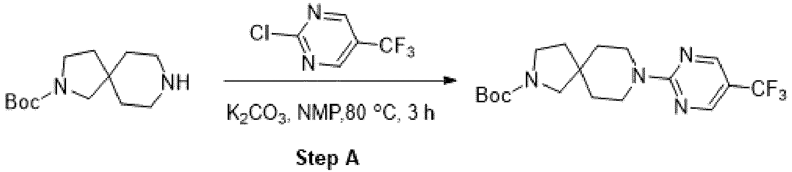

To a solution of tert-butyl 2,8-diazaspiro(4.5)decane-2-carboxylate (200 mg, 0.833 mmol, 1.0 eq.) in NMP (4 mL) were added K₂CO₃ (345 mg, 2.500 mmol, 3.0 eq.) and 2-chloro-5-(trifluoromethyl)pyrimidine (167 mg, 0.916 mmol, 1.1 eq.), the resulting mixture was stirred at 80 °C for 3 h. The mixture was cooled to room temperature and diluted with water (10 mL). The mixture was extracted with EtOAc (3 × 10 mL). The organic layers were combined and washed with brine (10 mL), dried over Na₂SO₄ and concentrated under reduced pressure. The residue was purified by column chromatography (Petroleum ether/EtOAc=1/1, V/V) to afford tert-butyl 8-(5-(trifluoromethyl)pyrimidin-2-yl)-2,8-diazaspiro(4.5)decane-2-carboxylate (290 mg, 90%) as a white solid.
LCMS: (*ESI)* (M+H-56)⁺ = 331.2.
¹H NMR: (400 MHz, CDCl₃) *δ* 8.47 (s, 2H), 4.15 - 4.03 (m, 1H), 4.01 - 3.90 (m, 1H), 3.87 - 3.77 (m, 1H), 3.74 - 3.64 (m, 1H), 3.44 (dt, J = 22.3, 7.0 Hz, 2H), 3.31 (s, 1H), 3.22 (s, 1H), 1.79 (t, J = 7.2 Hz, 2H), 1.66 - 1.56 (m, 4H), 1.47 (s, 9H).

### Step B: 8-(5-(trifluoromethyl)pyrimidin-2-yl)-2,8-diazaspiro(4.5)decane

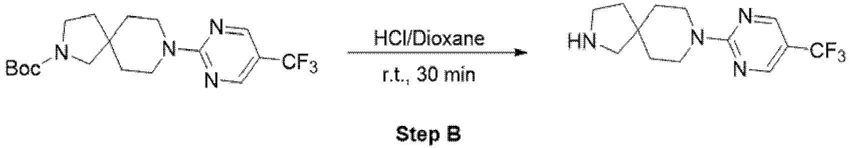

To a solution of tert-butyl 8-(5-(trifluoromethyl)pyrimidin-2-yl)-2,8-diazaspiro(4.5) decane-2-carboxylate (290 mg, 0.732 mmol, 1.0 eq) in Dioxane (2 mL) was added HCl/Dioxane (4 M, 2 ml). The mixture was stirred at r.t. for 30 min. The mixture was concentrated under vacuum to afford 8-(5-(trifluoromethyl)pyrimidin-2-yl)-2,8-diazaspiro(4.5)decane (209 mg, 100% yield) as a white solid.
LCMS: *(ESI)* (M+H)⁺ = 287.2.

### Step C: ethyl 2-(8-(5-(trifluoromethyl)pyrimidin-2-yl)-2,8-diazaspiro(4.5)decan-2-yl) acetate

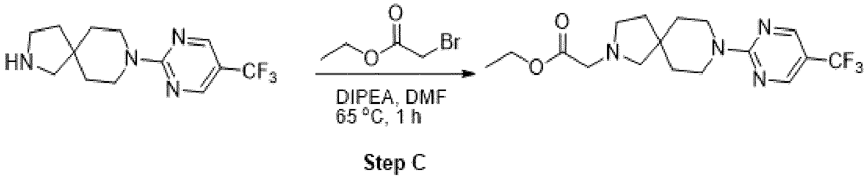

To a solution of 8-(5-(trifluoromethyl)pyrimidin-2-yl)-2,8-diazaspiro(4.5)decane (130 mg, 0.454 mmol, 1.0 eq.) in DMF (3 mL) were added ethyl 2-bromoacetate (91 mg, 0.545 mmol, 1.2 eq.) and DIPEA (70.3 mg, 0.545 mmol, 1.2 eq.).at rt. The resulting mixture was stirred at 65 °C for 1h. The mixture was cooled to room temperature and diluted with water (10 mL). The mixture was extracted with EtOAc (3 × 10 mL). The organic layers were combined and washed with brine (10 mL), dried over Na₂SO₄ and concentrated under reduced pressure. The residue was purified by column chromatography (Petroleum ether/EtOAc=3/1, V/V) to afford ethyl 2-(8-(5-(trifluoromethyl)pyrimidin-2-yl)-2,8-diazaspiro(4.5)decan-2-yl)acetate (150 mg, 88%) as a colorless oil.
LCMS: *(ESI)* (M+H)⁺ = 373.2.
¹H NMR: (400 MHz, CDCl₃) *δ* 8.45 (d, 2H), 4.19 (q, J = 7.1 Hz, 2H), 3.85 (dt, J = 8.0, 4.6 Hz, 4H), 3.33 (s, 2H), 2.80 (t, J = 6.9 Hz, 2H), 2.63 (s, 2H), 1.76 (t, J = 6.9 Hz, 2H), 1.71 - 1.59 (m, 4H), 1.28 (t, J = 7.1 Hz, 3H).

### Step D: Synthesis of 2-(8-(5-(trifluoromethyl)pyrimidin-2-yl)-2,8-diazaspiro(4.5)decan -2-yl)acetic acid

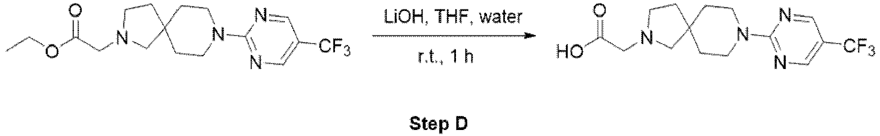

To a solution of ethyl 2-(8-(5-(trifluoromethyl)pyrimidin-2-yl)-2,8-diazaspiro(4.5) decan-2-yl)acetate (150 mg, 0.403 mmol, 1.0 eq.) in THF (3 mL) and water (3 mL) was added LiOH (29 mg, 1.209 mmol, 3.0 eq.). The mixture was stirred at r.t. for 3 h. The mixture was diluted with water (5 mL) and extracted with EtOAc (3 × 10 mL). The water layer was adjusted to pH 6 with citric acid. The resulting water layer was extracted with EtOAc (3 × 15 mL), dried over Na₂SO₄ and concentrated under vacuum to afford 2-(8-(5-(trifluoromethyl)pyrimidin-2-yl)-2,8-diazaspiro(4.5)decan-2-yl)acetic acid (120 mg, 86% yield) as a white solid.
LCMS: *(ESI)* (M+H)⁺ = 345.2.

### Step E: synthesis of (S)-N-(2-((6-oxo-5-(trifluoromethyl)-1-((2-(trimethylsilyl)ethoxy) methyl)-1,6-dihydropyridazin-4-yl)amino)propoxy)-2-(8-(5-(trifluoromethyl)pyrimidin-2-yl)-2,8-diazaspiro(4,5)decan-2-yl)acetamide

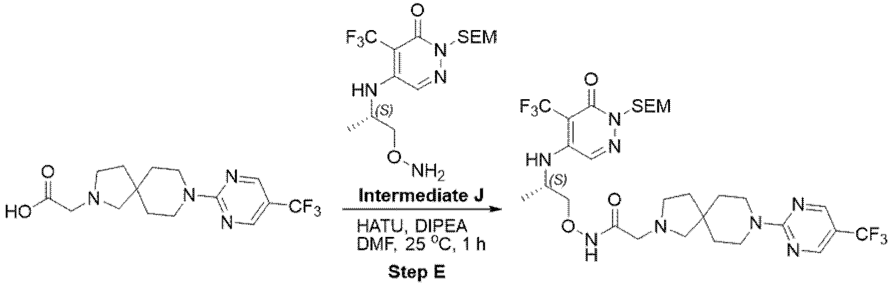

To a solution of 2-(8-(5-(trifluoromethyl)pyrimidin-2-yl)-2,8-diazaspiro(4.5)decan -2-yl)acetic acid (120 mg, 0.348 mmol, 1.0 eq.) in DMF (2 mL) were added DIPEA (135 mg, 1.046 mmol, 3.0 eq.) and HATU (158 mg, 0.417 mmol, 1.2 eq.) followed by dropwise addition of (S)-5-((1-(aminooxy)propan-2-yl)amino)-4-(trifluoromethyl)-2-((2-(trimethylsilyl)ethoxy)methyl)pyridazin-3(2H)-one (159 mg, 0.417 mmol, 1.2 eq.). The resulting mixture was stirred at 25 °C for 1 h. The reaction mixture was quenched with water (10 mL) and extracted with EtOAc (3 × 10 mL). The organic lyaers were combined and washed with brine (10 mL), dried over Na₂SO₄ and concentrated under vacuum. The residue was purified by silica gel chromatography (Petroleum ether/EtOAc= 1/3, V/V) to afford (S)-N-(2-((6-oxo-5-(trifluoromethyl)-1-((2-(trimethylsilyl)ethoxy)methyl)-1,6-dihydropyridazin-4-yl)amino)propoxy)-2-(8-(5-(trifluoromethyl)pyrimidin-2-yl)-2,8-diazaspiro(4.5)decan-2-yl)acetamide (120 mg, 40% yield) as a white solid.
LCMS: *(ESI)* (M+H)⁺ = 709.4.

### Step F: synthesis of (S)-N-(2-((6-oxo-5-(trifluoromethyl)-L6-dihydropyridazin-4-yl) amino)propoxy)-2-(8-(5-(trifluoromethyl)pyrimidin-2-yl)-2,8-diazaspiro(4.5)decan-2-yl acetamide

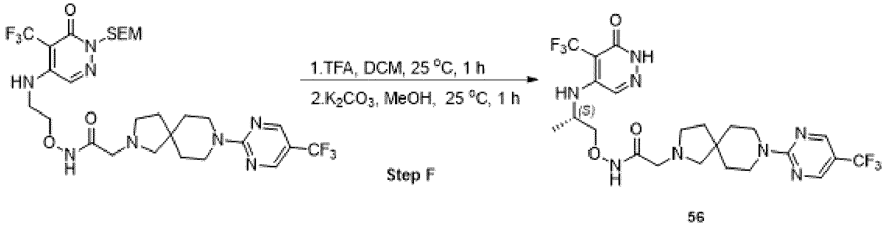

To a solution of (S)-N-(2-((6-oxo-5-(trifluoromethyl)-1-((2-(trimethylsilyl)ethoxy) methyl)-1,6-dihydropyridazin-4-yl)amino)propoxy)-2-(8-(5-(trifluoromethyl)pyrimidin-2-yl)-2,8-diazaspiro(4.5)decan-2-yl)acetamide (120 mg, 0.169 mmol, 1.0 eq.) in DCM (2 mL) was added TFA (0.3 mL). The resulting mixture was stirred at 25 °C for 1 h. The mixture was neutralized with saturated NaHCOs aqueous solution (5 mL) and extracted with DCM (3 × 10 mL). The organic layers were combined and dried over Na₂SO₄, concentrated under vacuum. The residue was redissolved in MeOH (5 mL) and K₂CO₃ (58 mg, 0.423 mmol, 2.5 eq.) was addedto the mixture. The mixture was stirred at 25 °C for another 1 h. The reaction mixture was filtered and the filtrate was purified by silica gel chromatography (Petroleum ether/EtOAc=1/5, V/V) to afford (S)-N-(2-((6-oxo-5-(trifluoromethyl)-1,6-dihydropyridazin-4-yl)amino)propoxy)-2-(8-( 5-(trifluoromethyl)pyrimidin-2-yl)-2,8-diazaspiro(4.5)decan-2-yl)acetamide (60 mg, 61% yield) as a white solid.
LCMS: *(ESI)* (M+H)⁺ = 579.4.
¹H NMR (400 MHz, DMSO-*d₆*) *δ* 12.49 (s, 1H), 8.66 (s, 2H), 7.90 (s, 1H), 6.61 (s, 1H), 4.28 - 4.14 (m, 1H), 3.99 - 3.69 (m, 6H), 3.01 (s, 2H), 2.65 - 2.58 (m, 2H), 2.48 - 2.43 (m, 2H), 1.64 (t, J = 6.7 Hz, 2H), 1.58 - 1.47 (m, 4H), 1.20 (d, J = 6.5 Hz, 3H).

### Example 57:

### (S)-N-(2-((6-oxo-5-(trifluoromethyl)-1,6-dihydropyridazin-4-yl)amino)propoxy)-2-(1-( 5-(trifluoromethyl)pyrimidin-2-yl)azetidin-3-yl)acetamide

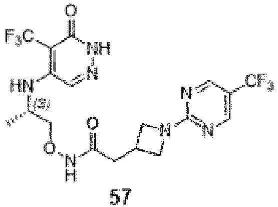

### Step A: methyl 2-(azetidin-3-yl)acetate

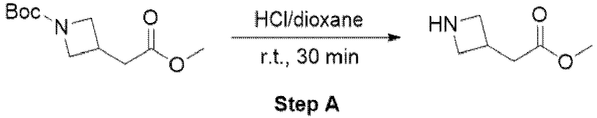

To a solution of tert-butyl 3-(2-methoxy-2-oxoethyl)azetidine-1-carboxylate (500 mg, 2.183 mmol, 1.0 eq) in Dioxane (2 mL) was added HCl/Dioxane (4 M, 4 mL). The mixture was stirred at rt for 30 min. The mixture was concentrated under vacuum to afford methyl 2-(azetidin-3-yl)acetate (281 mg, 100% yield) as a white solid.
LCMS: (ESI) (M+H)⁺ = 130.2.

### Step B: methyl 2-(1-(5-(trifluoromethyl)pyrimidin-2-yl)azetidin-3-yl)acetate

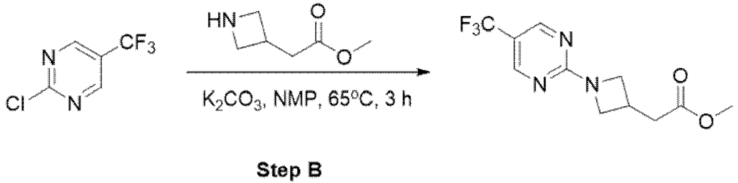

To a solution of 2-chloro-5-(trifluoromethyl)pyrimidine (200 mg, 1.098 mmol, 1.0 eq.) in NMP (4 mL) were added K₂CO₃ (455 mg, 3.296 mmol, 3.0 eq.) and methyl 2-(azetidin-3-yl)acetate (184 mg, 1.4427 mmol, 1.3 eq.), the resulting mixture was stirred at 65 °C for 3 h. The mixture was cooled to room temperature and diluted with water (10 mL). The mixture was extracted with EtOAc (3 × 100 mL). The organic layers were combined and washed with brine (50 mL), dried over Na₂SO₄ and concentrated under reduced pressure. The residue was purified by column chromatography (Petroleum ether/EtOAc= 1/3, V/V) to afford methyl 2-(1-(5-(trifluoromethyl)pyrimidin -2-yl)azetidin-3-yl)acetate (160 mg, 53%) as a white solid.
LCMS: (ESI) (M+H)⁺ = 276.2.
¹H NMR: (400 MHz, CDCl₃) *δ* 8.47 (s, 2H), 4.38 (t, J = 9.0 Hz, 2H), 3.89 (dd, J = 9.7, 5.5 Hz, 2H), 3.70 (s, 3H), 3.21 - 3.03 (m, 1H), 2.74 (d, J = 7.8 Hz, 2H).

### Step C: 2-(1-(5-(trifluoromethyl)pyrimidin-2-yl)azetidin-3-yl)acetic acid

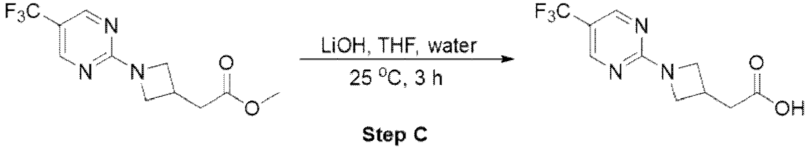

To a solution of methyl 2-(1-(5-(trifluoromethyl)pyrimidin-2-yl)azetidin-3-yl) acetate (140 mg, 0.509 mmol, 1.0 eq.) in THF (3 mL) and water (3 mL) was added LiOH (36 mg, 1.527 mmol, 3.0 eq.). The mixture was stirred at 25 °C for 3 h. The mixture was diluted with water (5 mL) and extracted with EtOAc (3 × 10 mL). The water layer was adjusted to pH 6 with citric acid. The resulting mixture was extracted with EtOAc (3 × 15 mL), dried over Na₂SO₄ and concentrated under vacuum to afford 2-(1-(5-(trifluoromethyl)pyrimidin-2-yl)azetidin-3-yl)acetic acid (120 mg, 90% yield) as a white solid.
LCMS: *(ESI)* (M+H)⁺ = 262.2.

### Step D: Synthesis of (S)-N-(2-((6-oxo-5-(trifluoromethyl)-1-((2-(trimethylsilyl)ethoxy) methyl)-1,6-dihydropyridazin-4-yl)amino)propoxy)-2-(1-(5-(trifluoromethyl)pyrimidin-2-yl)azetidin-3 -yl)acetamide

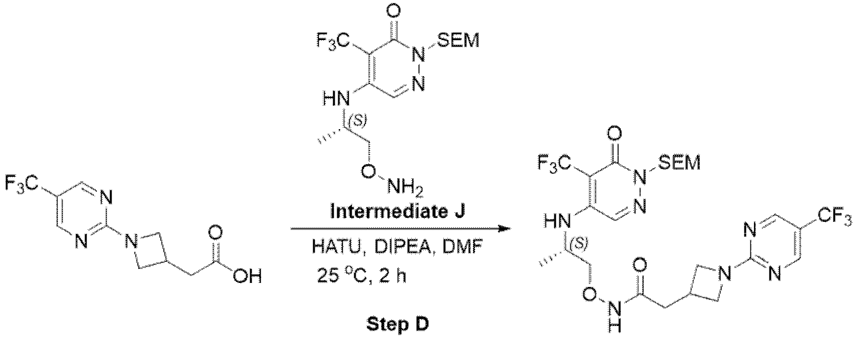

To a solution of 2-(1-(5-(trifluoromethyl)pyrimidin-2-yl)azetidin-3-yl)acetic acid (90 mg, 0.343 mmol, 1.2 eq.) in DMF (2 mL) were added DIPEA (110 mg, 0.858 mmol, 3.0 eq.) and HATU (130 mg, 0.343 mmol, 1.2 eq.) followed by dropwise addition of (S)-5-((1-(aminooxy)propan-2-yl)amino)-4-(trifluoromethyl)-2-((2-(trimethylsilyl)ethox y)methyl)pyridazin-3(2H)-one (109 mg, 0.286 mmol, 1.0 eq.).The resulting mixture was stirred at 25 °C for 2 h. The reaction mixture was quenched with water (10 mL) and extracted with EtOAc (3 × 10 mL). The organic layers were combined and washed with brine (10 mL), dried over Na₂SO₄ and concentrated under vacuum. The residue was purified by silica gel chromatography (Petroleum ether/EtOAc=1/3, V/V) to afford (S)-N-(2-((6-oxo-5-(trifluoromethyl)-1-((2-(trimethylsilyl)ethoxy)methyl)-1,6-dihydrop yridazin-4-yl)amino)propoxy)-2-(1-(5-(trifluoromethyl)pyrimidin-2-yl)azetidin-3-yl) acetamide (120 mg, 67% yield) as a white solid.
LCMS: *(ESI)* (M+H)⁺ = 626.4.
¹H NMR: (400 MHz, CDCl₃) *δ* 9.26 (s, 1H), 8.48 (s, 2H), 7.72 (s, 1H), 5.93 (s, 1H), 5.42 (d, J = 9.9 Hz, 1H), 5.29 (d, J = 9.9 Hz, 1H), 4.39 (t, J = 8.9 Hz, 2H), 4.16 - 4.04 (m, 2H), 3.99 - 3.83 (m, 3H), 3.68 (t, J = 8.4 Hz, 2H), 3.24 - 3.12 (m, 1H), 2.65 - 2.50 (m, 2H), 1.36 (d, J = 6.5 Hz, 3H), 0.95 (t, J = 8.3 Hz, 2H), 0.00 (s, 9H).

### Step E: synthesis of (S)-N-(2-((6-oxo-5-(trifluoromethyl)-L6-dihydropyridazin-4-yl) amino)propoxy)-2-(1-(5-(trifluoromethyl)pyrimidin-2-yl)azetidin-3-yl)acetamide

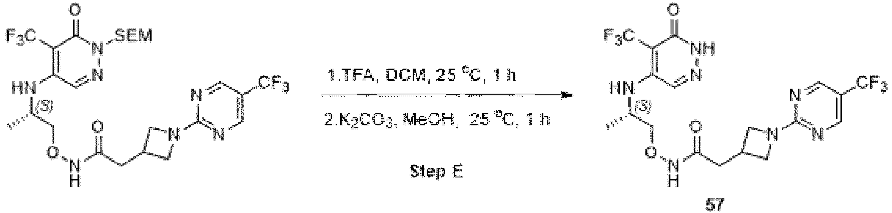

To a solution of (S)-N-(2-((6-oxo-5-(trifluoromethyl)-1-((2-(trimethylsilyl)ethoxy) methyl)-1,6-dihydropyridazin-4-yl)amino)propoxy)-2-(1-(5-(trifluoromethyl)pyrimidin-2-yl)azetidin-3-yl)acetamide (110 mg, 0.176 mmol, 1.0 eq.) in DCM (2 mL) was added TFA (0.3 mL). The resulting mixture was stirred at 25 °C for 1 h. The mixture was neutralized with saturated NaHCOs aqueous solution (5 mL) and extracted with DCM (3 × 10 mL). The organic layers were combined and dried over Na₂SO₄, concentrated under vacuum. The residue was redissolved in MeOH (5 mL) and K₂CO₃ (61 mg, 0.44 mmol, 2.5 eq.) was added to the mixture. The mixture was stirred at 25 °C for another 1 h. The reaction mixture was filtered and the filtrate was purified by silica gel chromatography (Petroleum ether/EtOAc= 1/5, V/V) to afford (S)-N-(2-((6-oxo-5-(trifluoromethyl)-1,6-dihydropyridazin-4-yl)amino)propoxy)-2-(1-( 5-(trifluoromethyl)pyrimidin-2-yl)azetidin-3-yl)acetamide (40 mg, 46% yield) as a white solid.
LCMS: *(ESI)* (M+H)⁺ = 496.2.
¹H NMR (400 MHz, CDCl₃) *δ* 10.94 (s, 1H), 9.90 (s, 1H), 8.48 (s, 2H), 7.75 (s, 1H), 5.77 (s, 1H), 4.37 (q, J = 9.0 Hz, 2H), 4.22 - 4.08 (m, 2H), 4.07 - 3.98 (m, 1H), 3.90 (dd, J = 9.5, 5.1 Hz, 1H), 3.85 - 3.75 (m, 1H), 3.22 - 3.08 (m, 1H), 2.65 - 2.45 (m, 2H), 1.32 (d, J = 6.3 Hz, 3H).

### Example 58:

### N-((S)-2-((6-oxo-5-(trifluoromethyl)-1,6-dihydropyridazin-4-yl)amino)propoxy)-2-(3-( 5-(trifluoromethyl)pyrimidin-2-yl)-3,6-diazabicyclo(3.1.1)heptan-6-yl)acetamide

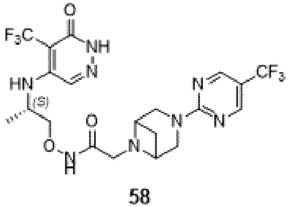

### Step A: synthesis of tert-butyl 3-(5-(trifluoromethyl)pyrimidin-2-yl)-3,6-diazabicyclo (3.1.1)heptane-6-carboxylate

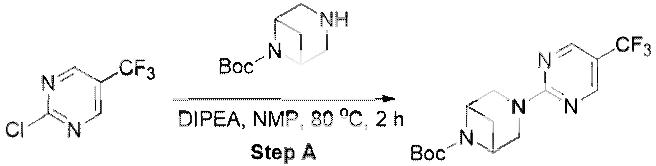

To a mixture of tert-butyl 3,6-diazabicyclo(3.1.1)heptane-6-carboxylate (522 mg, 2.63 mmol, 1.2 eq.) and DIPEA (1.13 g, 8.77 mmol, 4.0 eq.) in NMP (8 mL) was added 2-chloro-5-(trifluoromethyl)pyrimidine (400 mg, 2.19 mmol, 1.0 eq.). The reaction mixture was stirred at 80 °C for 2 h. The reaction mixture was cooled to room temperature and quenched with water (50 mL). The mixture was extracted with EtOAc (3 × 50 mL). The organic layers were combined and washed with brine (2 × 50 mL), dried over Na₂SO₄ and concentrated under vacuum. The residue was purified by silica gel chromatography (Petroleum ether/EtOAc= 7/3; V/V) to afford tert-butyl 3-(5-(trifluoromethyl)pyrimidin-2-yl)-3,6-diazabicyclo(3.1.1)heptane-6-carboxylate (680 mg, 90.0%) as a light yellow solid.
LCMS: *(ESI)* (M+H-100)⁺ = 245.1.
¹H NMR: (400 MHz, CDCl₃) *δ* 8.60 (s, 2H), 4.40 - 4.17 (m, 4H), 3.71 (d, *J* = 12.8 Hz, 2H), 2.76 - 2.68 (m, 1H), 1.52 (d, *J* = 8.8 Hz, 1H), 1.42 (s, 9H).

### Step B: synthesis of 3-(5-(trifluoromethyl)pyrimidin-2-yl)-3,6-diazabicyclo(3.1.1) heptane hydrochloride

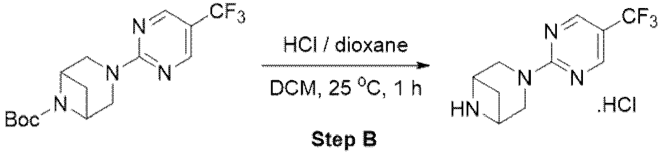

To a solution of tert-butyl 3-(5-(trifluoromethyl)pyrimidin-2-yl)-3,6-diazabicyclo (3.1.1)heptane-6-carboxylate (612 mg, 1.78 mmol) in DCM (5 mL) was added HCl/dioxane (4 M, 5 mL). The mixture was stirred at 25 °C for 1 h. The mixture was concentrated under vacuum to afford 3-(5-(trifluoromethyl)pyrimidin-2-yl)-3,6-diazabicyclo(3.1.1)heptane hydrochloride (499 mg, 99%) as a white solid.
LCMS: *(ESI)* (M+H)⁺ = 245.1.

### Step C: synthesis of ethyl 2-(3-(5-(trifluoromethyl)pyrimidin-2-yl)-3,6-diazabicyclo (3.1.1)heptan-6-yl)acetate

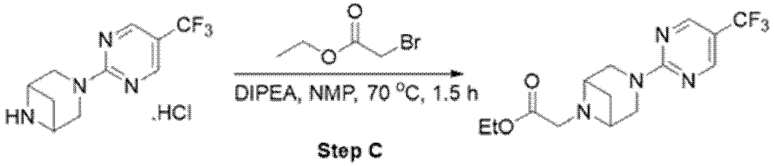

To a mixture of 3-(5-(trifluoromethyl)pyrimidin-2-yl)-3,6-diazabicyclo(3.1.1) heptane hydrochloride (499 mg, 1.78 mmol, 1.0 eq.) in NMP (5 mL) were added DIPEA (1.15 g, 8.89 mmol, 5.0 eq.) and ethyl 2-bromoacetate (594 mg, 3.56 mmol, 2.0 eq.). The mixture was stirred at 70 °C for 1.5 h. The mixture was cooled to room temperature and diluted with EtOAc (60 mL). The mixture was washed with saturated NH₄Cl aqueous solution (3 × 40 mL). The organic layer was dried over Na₂SO₄ and concentrated under vacuum. The residue was purified by silica gel column chromatography (Petroleum ether/EtOAc= 1/1; V/V) to afford ethyl 2-(3-(5-(trifluoromethyl)pyrimidin-2-yl)-3,6-diazabicyclo(3.1. 1)heptan-6-yl)acetate (394 mg, 67.1%) as a white solid.
LCMS: *(ESI)* (M+H)⁺ = 331.1.

### Step D: synthesis of 2-(3-(5-(trifluoromethyl)pyrimidin-2-yl)-3,6-diazabicyc10(3.1.1) heptan-6-yl)acetic acid

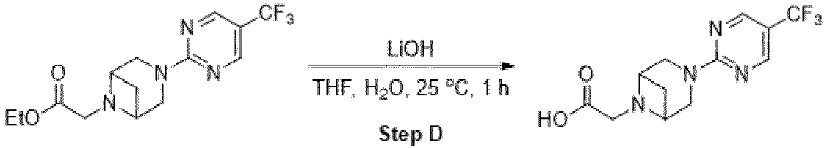

To mixture of ethyl 2-(3-(5-(trifluoromethyl)pyrimidin-2-yl)-3,6-diazabicyclo (3.1.1)heptan-6-yl)acetate (150 mg, 0.46 mmol, 1.0 eq.) in THF (3 mL) and H₂O (1.5 mL) was added lithium hydroxide (44 mg, 1.82 mmol, 4.0 eq.). The mixture was stirred at 25 °C for 1 h. To the mixture was adjusted to pH 6 with 1M HCl. The mixture was purified by reversed phase chromatography (ACN/H₂O= 20/80; V/V) to afford 2-(3-(5-(trifluoromethyl)pyrimidin-2-yl)-3,6-diazabicyclo(3.1.1)heptan-6-yl)acetic acid (80 mg, 58.3%) as a yellow solid.
LCMS: *(ESI)* (M+H)⁺ = 303.1.

### Step E: Synthesis of N-((S)-2-((6-oxo-5-(trifluoromethyl)-1-((2-(trimethylsilyl)ethoxy) methyl)-1,6-dihydropyridazin-4-yl)amino)propoxy)-2-(3-(5-(trifluoromethyl)pyrimidin-2-yl)-3,6-diazabicyclo(3.1.1)heptan-6-yl)acetamide

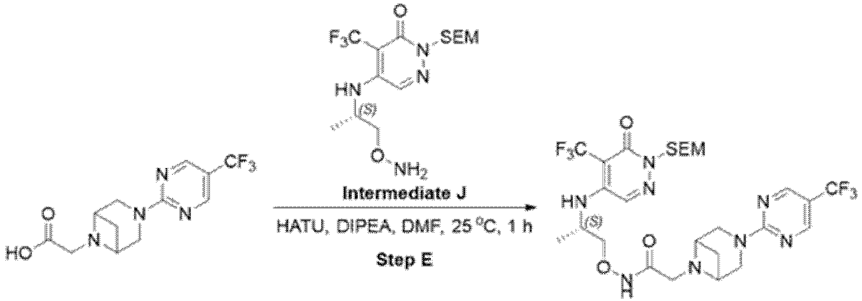

To mixture of 2-(3-(5-(trifluoromethyl)pyrimidin-2-yl)-3,6-diazabicyclo(3.1.1) heptan-6-yl)acetic acid (40 mg, 0.13 mmol, 1.1 eq.) in DMF (5 mL) were added (S)-5-((1-(aminooxy)propan-2-yl)amino)-4-(trifluoromethyl)-2-((2-(trimethylsilyl)ethox y)methyl)pyridazin-3(2H)-one (44 mg, 0.12 mmol, 1.0 eq.), DIPEA (47 mg, 0.36 mmol, 3.0 eq.) and HATU (53 mg, 0.14 mmol, 1.2 eq.). The mixture was stirred at 25 °C for 1 h. The reaction mixture was quenched with water (50 mL) and extracted with EtOAc (2 × 40 mL). The organic layers were combined and washed with brine (2 × 40 mL), dried over Na₂SO₄ and concentrated under vacuum. The residue was purified by silica gel column chromatography (Petroleum ether/EtOAc= 1/1; V/V) to afford N-((S)-2-((6-oxo-5-(trifluoromethyl)-1-((2-(trimethylsilyl)ethoxy)methyl)-1,6-dihydrop yridazin-4-yl)amino)propoxy)-2-(3-(5-(trifluoromethyl)pyrimidin-2-yl)-3,6-diazabicycl o(3.1.1)heptan-6-yl)acetamide (50 mg, 62.5%) as a yellow solid.
LCMS: *(ESI)* (M+H)⁺ = 667.3.

### Step F: synthesis of N-((S)-2-((6-oxo-5-(trifluoromethyl)-L6-dihydropyridazin-4-yl) amino)propoxy)-2-(3-(5-(trifluoromethyl)pyrimidin-2-yl)-3,6-diazabicyclo(3.1.1)heptan -6-yl)acetamide

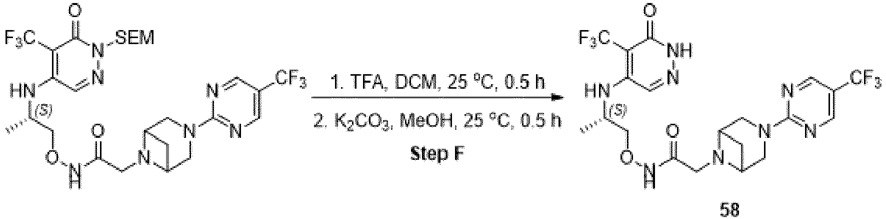

To mixture of N-((S)-2-((6-oxo-5-(trifluoromethyl)-1-((2-(trimethylsilyl)ethoxy) methyl)-1,6-dihydropyridazin-4-yl)amino)propoxy)-2-(3-(5-(trifluoromethyl)pyrimidin-2-yl)-3,6-diazabicyclo(3.1.1)heptan-6-yl)acetamide (50 mg, 0.07 mmol, 1.0 eq.) in DCM (3 mL) was added TFA (1 mL). The mixture was stirred at 25 °C for 0.5 h. The mixture was concentrated under vacuum. The residue was redissolved in MeOH (2 mL) and K₂CO₃ (62 mg, 0.45 mmol) was added. The mixture was stirred at 25 °C for another 0.5 h. The mixture was filtered and filtrate was purified by prep-HPLC to afford N-((S)-2-((6-oxo-5-(trifluoromethyl)-1,6-dihydropyridazin-4-yl)amino)propoxy)-2-(3-( 5-(trifluoromethyl)pyrimidin-2-yl)-3,6-diazabicyclo(3.1.1)heptan-6-yl)acetamide (10.18 mg, 25.3%) as a white soild.
LCMS: *(ESI)* (M+H)⁺ = 537.2.
¹H NMR: (400 MHz, CDCl₃) *δ* 11.26 (br s, 1H), 8.63 (s, 2H), 7.77 (s, 1H), 5.99 (s, 1H), 4.25 - 4.08 (m, 4H), 4.06 - 3.85 (m, 5H), 3.60 - 3.30 (m, 2H), 3.03 - 2.90 (m, 1H), 1.78 (d, *J* = 9.2 Hz, 1H), 1.36 (d, *J* = 6.0 Hz, 3H).

### Example 59:

### N-((S)-2-((6-oxo-5-(trifluoromethyl)-1,6-dihydropyridazin-4-yl)amino)propoxy)-2-(3-( 5-(trifluoromethyl)pyrimidin-2-yl)-3,8-diazabicyclo(3.2.1)octan-8-yl)acetamide

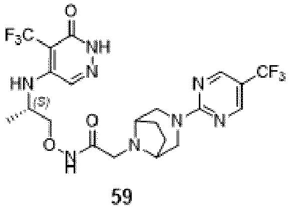

### Step A: synthesis of tert-butyl 3-(5-(trifluoromethyl)pyrimidin-2-yl)-3,8-diazabicyclo (3.2.1)octane-8-carboxylate

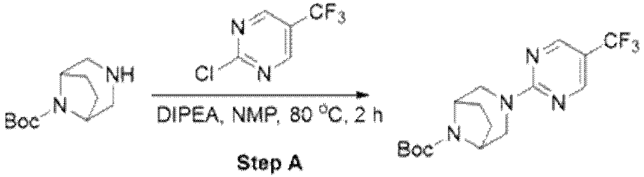

To mixture of 2-chloro-5-(trifluoromethyl)pyrimidine (250 mg, 1.37 mmol, 1.0 eq.) in NMP (5 mL) were added tert-butyl 3,8-diazabicyclo(3.2.1)octane-8-carboxylate (291 mg, 1.37 mmol, 1.0 eq.) and DIPEA (531 mg, 4.11 mmol, 3.0 eq.). The mixture was stirred at 80 °C for 2 h. The reaction mixture was cooled to room temperature and diluted with water (50 mL), extracted with EtOAc (2 × 50 mL). The organic layers were combined and washed with brine (2 × 50 mL), dried over Na₂SO₄ and concentrated under vacuum. The residue was purified by silica gel chromatography (Petroleum ether/EtOAc= 4/1; V/V) to afford tert-butyl 3-(5-(trifluoromethyl)pyrimidin-2-yl)-3,8-diazabicyclo(3.2.1)octane-8-carboxylate (453 mg, 92.2%) as a white solid.
LCMS: *(ESI)* (M+H-56)⁺ = 303.1.
¹H NMR: (400 MHz, CDCl₃) *δ* 8.53 (s, 2H), 4.59 - 4.49 (m, 2H), 4.49 - 4.27 (m, 2H), 3.34 - 3.12 (m, 2H), 2.06 - 1.88 (m, 2H), 1.73 - 1.67 (m, 2H), 1.54 (s, 9H).

### Step B: synthesis of 3-(5-(trifluoromethyl)pyrimidin-2-yl)-3,8-diazabicyclo(3.2.1) octane hydrochloride

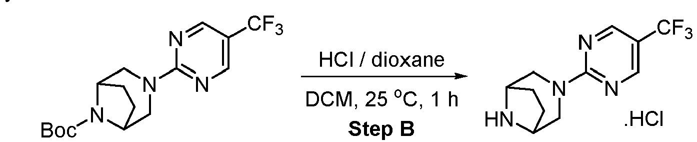

To a mixture of tert-butyl 3-(5-(trifluoromethyl)pyrimidin-2-yl)-3,8-diazabicyclo (3.2.1)octane-8-carboxylate (275 mg, 0.77 mmol, 1.0 eq.) in DCM (3 mL) was added HCl/dioxane (4 M, 3 mL). The mixture was stirred at 25 °C for 1 h. The mixture was concentrated under vacuum to afford 3-(5-(trifluoromethyl)pyrimidin-2-yl)-3,8-diazabicyclo(3.2.1)octane hydrochloride (200 mg, 88.5%) as a white solid.
LCMS: *(ESI)* (M+H)⁺ = 259.1.

### Step C: synthesis of ethyl 2-(3-(5-(trifluoromethyl)pyrimidin-2-yl)-3,8-diazabicyclo (3.2.1)octan-8-yl)acetate

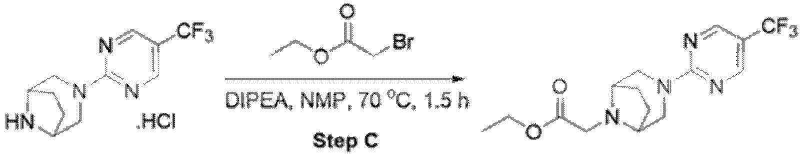

To mixture of 3-(5-(trifluoromethyl)pyrimidin-2-yl)-3,8-diazabicyclo(3.2.1)octane hydrochloride (270 mg, 0.92 mmol, 1.0 eq.) in NMP (5 mL) were added DIPEA (592 mg, 4.58 mmol, 5.0 eq.) and ethyl 2-bromoacetate (306 mg, 1.83 mmol, 2.0 eq.). The mixture was stirred at 70 °C for 1.5 h. The reaction mixture was cooled to room temperature and diluted with water (50 mL), extracted with EtOAc (2 × 80 mL). The organic layers were combined and washed with brine (2 × 60 mL), dried over Na₂SO₄ and concentrated under vacuum. The residue was purified by silica gel chromatography (Petroleum ether/EtOAc= 4/1; V/V) to afford ethyl 2-(3-(5-(trifluoromethyl)pyrimidin -2-yl)-3,8-diazabicyclo(3.2.1)octan-8-yl)acetate (300 mg, 95.1%) as a yellow solid.
LCMS: *(ESI)* (M+H)⁺ = 345.1.
¹H NMR: (400 MHz, CDCl₃) *δ* 8.50 (s, 2H), 4.41 (dd, *J* = 13.2, 2.0 Hz, 2H), 4.26 (q, *J* = 7.2 Hz, 2H), 3.49 - 3.43 (m, 2H), 3.34 (d, *J* = 12.4 Hz, 2H), 3.28 (s, 2H), 2.03 - 1.94 (m, 2H), 1.72 - 1.61 (m, 2H), 1.33 (t, *J* = 7.2 Hz, 3H).

### Step D: synthesis of 2-(3-(5-(trifluoromethyl)pyrimidin-2-yl)-3,8-diazabicyc10(3 .2.1) octan-8-yl)acetic acid

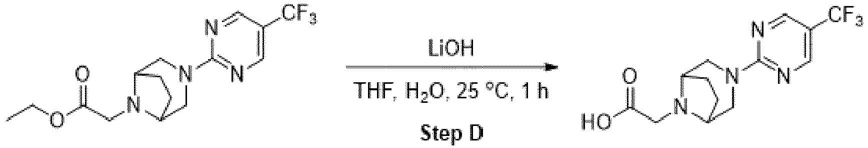

To a mixture of ethyl 2-(3-(5-(trifluoromethyl)pyrimidin-2-yl)-3,8-diazabicyclo (3.2.1)octan-8-yl)acetate (150 mg, 0.44 mmol, 1.0 eq.) in THF (4 mL) and H₂O (2 mL) was added lithium hydroxide (42 mg, 1.74 mmol, 4.0 eq.). The mixture was stirred at 25 °C for 1 h. To the mixture was carefully adjusted to pH 6 with 1M HCl. The mixture was purified by reversed phase chromatography (ACN/H₂O= 20/80; V/V) to afford 2-(3-(5-(trifluoromethyl)pyrimidin-2-yl)-3,8-diazabicyclo(3.2.1)octan-8-yl)acetic acid (100 mg, 72.6%) as a yellow solid.
LCMS: *(ESI)* (M+H)⁺ = 317.2.

### Step E: Synthesis of N-((S)-2-((6-oxo-5-(trifluoromethyl)-1-((2-(trimethylsilyl)ethoxy) methyl)-L6-dihydropyridazin-4-yl)amino)propoxy)-2-(3-(5-(trifluoromethyl)pyrimidin-2-yl)-3,8-diazabicyclo(3.2.1)octan-8-yl)acetamide

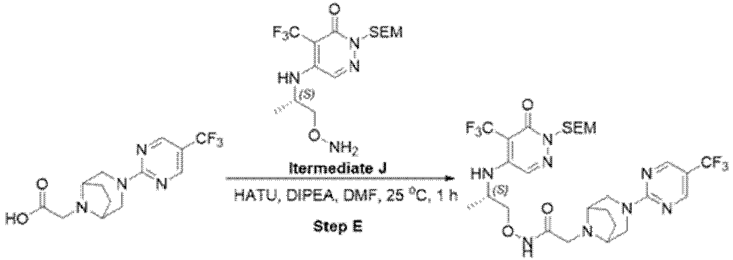

To a mixture of 2-(3-(5-(trifluoromethyl)pyrimidin-2-yl)-3,8-diazabicyclo(3.2.1) octan-8-yl)acetic acid (57 mg, 0.18 mmol, 1.1 eq.) in DMF (1 mL) were added (S)-5-((1-(aminooxy)propan-2-yl)amino)-4-(trifluoromethyl)-2-((2-(trimethylsilyl)ethox y)methyl)pyridazin-3(2H)-one (60 mg, 0.16 mmol, 1.0 eq.), DIPEA (63 mg, 0.49 mmol, 3.0 eq.) and HATU (112 mg, 0.29 mmol, 1.8 eq.). The mixture was stirred at 25 °C for 1 h. The reaction mixture was quenched with water (40 mL) and extracted with EtOAc (2 × 50 mL). The organic layers were combined and washed with brine (2 × 50 mL), dried over Na₂SO₄ and concentrated under vacuum. The residue was purified by silica gel chromatography (Petroleum ether/EtOAc= 3/7; V/V) to afford N-((S) -2-((6-oxo-5-(trifluoromethyl)-1-((2-(trimethylsilyl)ethoxy)methyl)-1,6-dihydropyridazi n-4-yl)amino)propoxy)-2-(3-(5-(trifluoromethyl)pyrimidin-2-yl)-3, 8-diazabicyclo(3 .2.1) octan-8-yl)acetamide (70 mg, 64.5%) as a yellow solid.
LCMS: *(ESI)* (M+H)⁺ = 681.2.

### Step F: synthesis of N-((S)-2-((6-oxo-5-(trifluoromethyl)-L6-dihydropyridazin-4-yl) amino)propoxy)-2-(3-(5-(trifluoromethyl)pyrimidin-2-yl)-3,8-diazabicyclo(3.2.1)octan-8-yl)acetamide

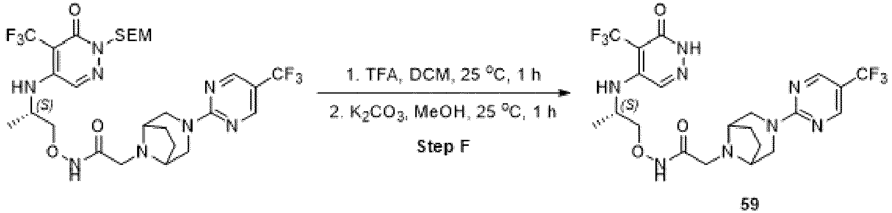

To a mixture of N-((S)-2-((6-oxo-5-(trifluoromethyl)-1-((2-(trimethylsilyl)ethoxy) methyl)-1,6-dihydropyridazin-4-yl)amino)propoxy)-2-(3-(5-(trifluoromethyl)pyrimidin-2-yl)-3,8-diazabicyclo(3.2.1)octan-8-yl)acetamide (65 mg, 0.1 mmol, 1.0 eq.) in DCM (3 mL) was added TFA (1 mL). The mixture was stirred at 25 °C for 1 h. The mixture was concentrated under vacuum. The residue was redissolved in MeOH (2 mL) and K₂CO₃ (62 mg, 0.45 mmol) was added. The mixture was stirred at 25 °C for another 1 h. The mixture was filtered and filtrate was purified by prep-HPLC to afford N-((S)-2-((6-oxo-5-(trifluoromethyl)-1,6-dihydropyridazin-4-yl)amino)propoxy)-2-(3-( 5-(trifluoromethyl)pyrimidin-2-yl)-3,8-diazabicyclo(3.2.1)octan-8-yl)acetamide (12.30 mg, 22.9%) as a white solid.
LCMS: *(ESI)* (M+H)⁺ = 551.2.
¹H NMR (400 MHz, CDCl₃) *δ* 11.64 (s, 1H), 8.54 (s, 2H), 7.72 (s, 1H), 6.00 (s, 1H), 4.82 - 4.68 (m, 2H), 4.4 - 4.36 (m, 1H), 4.22 - 4.07 (m, 3H), 3.98 - 3.82 (m, 2H), 3.71 (d, J = 14.4 Hz, 3H), 2.34 - 2.25 (m, 2H), 1.99 (d, J = 8.7 Hz, 2H), 1.30 (d, J = 6.3 Hz, 3H).

### Example 60:

### (S)-N-(2-((5-acetyl-6-oxo-1,6-dihydropyridazin-4-yl)amino)propoxy)-2-(1-(5-(trifluoromethyl)pyrimidin-2-yl)-1,2,3,6-tetrahydropyridin-4-yl)acetamide

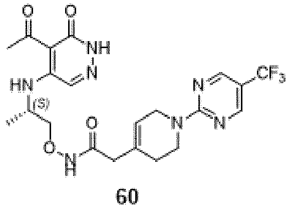

### Step A: Synthesis of (S)-N-(2-((5-acetyl-6-oxo-1-((2-(trimethylsilyl)ethoxy)methyl) -1,6-dihydropyridazin-4-yl)amino)propoxy)-2-(1-(5-(trifluoromethyl)pyrimidin-2-yl)-1, 2,3,6-tetrahydropyridin-4-yl(acetamide

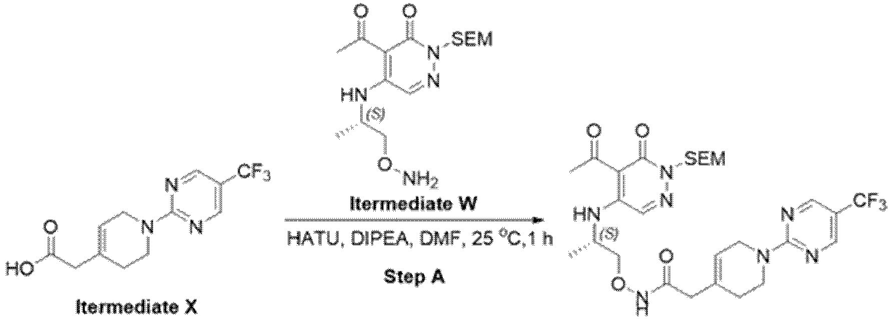

To a solution of (S)-4-acetyl-5-((1-(aminooxy)propan-2-yl)amino)-2-((2-(trimethylsilyl)ethoxy)methyl)pyridazin-3(2H)-one (260 mg, 0.73 mmol, 1.0 eq.) and 2-(1-(5-(trifluoromethyl)pyrimidin-2-yl)-1,2,3,6-tetrahydropyridin-4-yl)acetic acid (229 mg, 0.80 mmol, 1.1 eq.) in DMF (10 mL) were added DIPEA (283 mg, 2.19 mmol, 3.0 eq.) and HATU (335 mg, 0.88 mmol, 1.2 eq.). The mixture was stirred at 25 °C for 1 h. The mixture was quenched with water (50 mL) and extracted with EtOAc (3 × 40 mL). The organic layers were combined and washed with brine (200 mL), dried over anhydrous Na₂SO₄ and concentrated under vacuum. The residue was purified by column chromatography (Petroleum ether/EtOAc= 1/2; V/V) to afford (S)-N-(2-((5-acetyl-6-oxo-1-((2-(trimethylsilyl)ethoxy)methyl)-1,6-dihydropyridazin-4-yl)amino)propoxy)-2-(1-(5-(trifluoromethyl)pyrimidin-2-yl)-1,2,3,6-tetrahydropyridin-4 -yl)acetamide (200 mg, 43.7%) as a white solid.
LCMS: *(ESI)* (M+H)⁺ = 626.4.

### Step B: Synthesis of (S)-N-(2-((5-acetyl-6-oxo-1,6-dihydropyridazin-4-yl)amino) propoxy)-2-(1-(5-(trifluoromethyl)pyrimidin-2-yl)-1,2,3,6-tetrahydropyridin-4-yl) acetamide

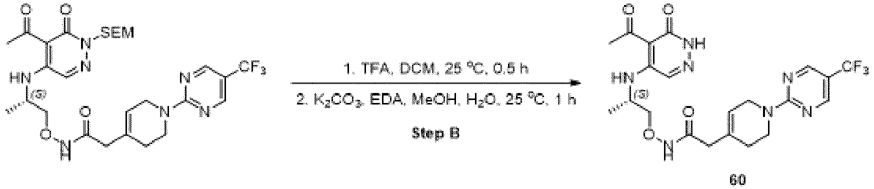

To a mixture of (S)-N-(2-((5-acetyl-6-oxo-1-((2-(trimethylsilyl)ethoxy)methyl) -1,6-dihydropyridazin-4-yl)amino)propoxy)-2-(1-(5-(trifluoromethyl)pyrimidin-2-yl)-1, 2,3,6-tetrahydropyridin-4-yl)acetamide (200 mg, 0.32 mmol, 1.0 eq.) in DCM (6 mL) was added TFA (2 mL). The mixture was stirred at 25 °C for 0.5 h. The mixture was quenched with saturated aqueous NaHCO₃ solution (30 mL) and extracted with DCM (3 × 50 mL). The organic layers were combined and dried over Na₂SO₄, concentrated under vacuum. The residue was treated with MeOH (2 mL), H₂O (2 mL), K₂CO₃ (200 mg, 1.45 mmol, 4.5 eq.) and ethylenediamine (132 mg, 2.20 mmol, 6.8 eq.). The mixture was stirred at 25 °C for another 1 h. The mixture was diluted with water (30 mL) and extracted with DCM (3 × 30 mL). The organic layers were combined and dried over Na₂SO₄, concentrated under vacuum. The residue was purified by prep-HPLC to afford (S)-N-(2-((5-acetyl-6-oxo-1,6-dihydropyridazin-4-yl)amino)propoxy)-2-(1-(5-(trifluoro methyl)pyrimidin-2-yl)-1,2,3,6-tetrahydropyridin-4-yl)acetamide (38.14 mg, 24.1%) as a white solid.
LCMS: *(ESI)* (M+H)⁺ = 496.2.
¹H NMR: (400 MHz, DMSO-*d₆*) *δ* 12.32 (s, 1H), 11.20 (s, 1H), 10.70 (d, *J* = 8.4 Hz, 1H), 8.70 (s, 2H), 7.95 (s, 1H), 5.62 (s, 1H), 4.33 - 4.25 (m, 1H), 4.25- 4.18 (s, 2H), 3.97 - 3.86 (m, 3H), 3.85 - 3.78 (m, 1H), 2.75 (s, 2H), 2.53 (s, 3H), 2.17 - 2.08 (m, 2H), 1.23 (d, *J* = 6.8 Hz, 3H).

### Example 61~87:

### Compounds 61 to 87 listed in the following table were synthesized according to methods similar to Examples 1 to 60.

| | | |
|---|---|---|
| 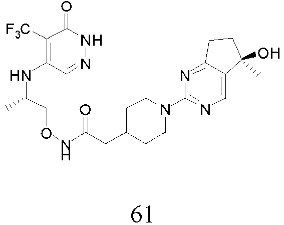 | 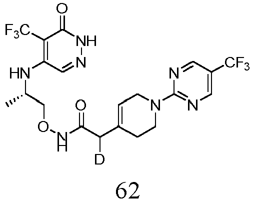 | 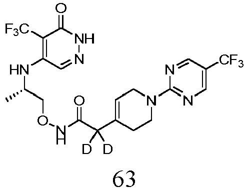 |
| LCMS: [M+H]⁺ = 525.2 | LCMS: [M+H]⁺ = 522.2 | LCMS: [M+H]⁺ = 523.2 |
| 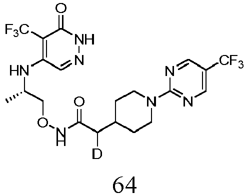 | 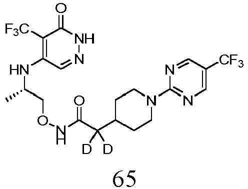 | 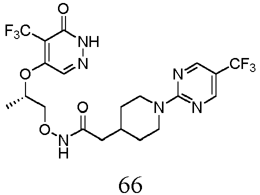 |
| LCMS: [M+H]⁺ = 524.2 | LCMS: [M+H]⁺ = 525.2 | LCMS: [M+H]⁺ = 524.2 |
| 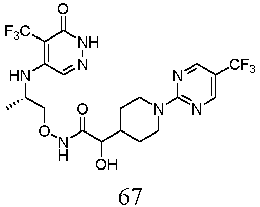 | 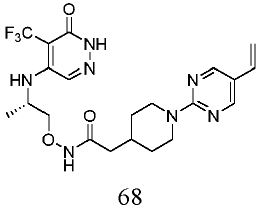 | 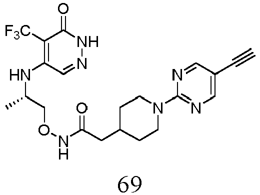 |
| LCMS: [M+H]⁺ = 539.2 | LCMS: [M+H]⁺ = 481.2 | LCMS: [M+H]⁺ = 479.2 |
| 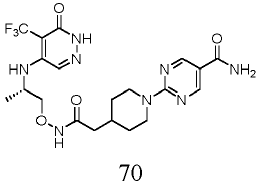 | 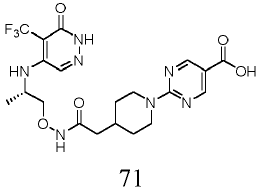 | 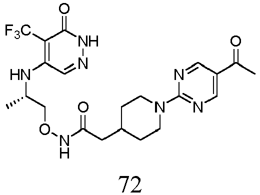 |
| LCMS: [M+H]⁺ = 498.2 | LCMS: [M+H]⁺ = 499.2 | LCMS: [M+H]⁺ = 497.2 |
| 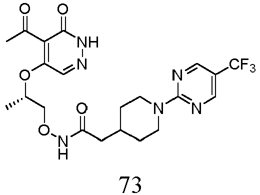 | 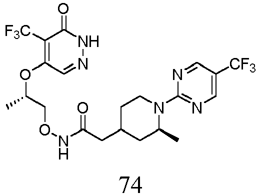 | 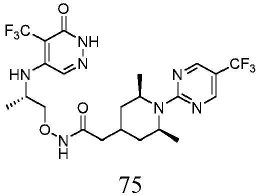 |
| LCMS: [M+H]⁺ = 498.2 | LCMS: [M+H]⁺ = 538.2 | LCMS: [M+H]⁺ = 551.2 |
| 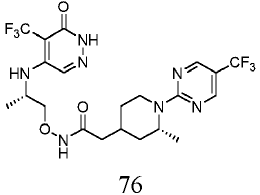 | 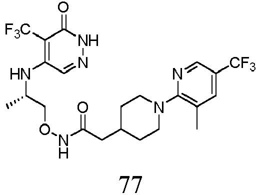 | 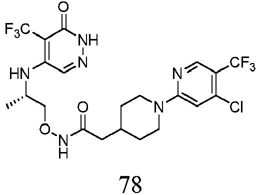 |
| LCMS: [M+H]⁺ = 537.2 | LCMS: [M+H]⁺ = 536.2 | LCMS: [M+H]⁺ = 556.1 |
| 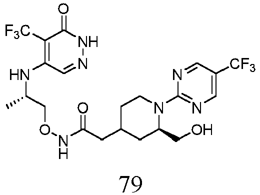 | 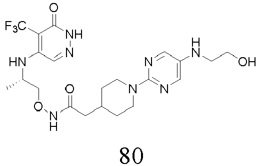 | 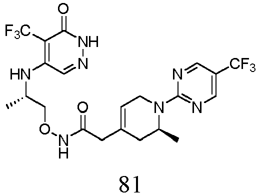 |
| LCMS: [M+H]⁺ = 553.2 | LCMS: [M+H]⁺ = 514.2 | LCMS: [M+H]⁺ = 535.2 |
| 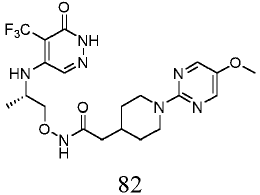 | 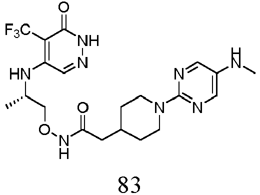 | 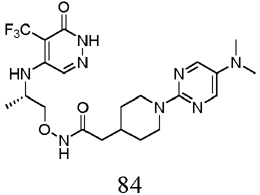 |
| LCMS: [M+H]⁺ = 485.2 | LCMS: [M+H]⁺ = 484.2 | LCMS: [M+H]⁺ = 498.2 |
| 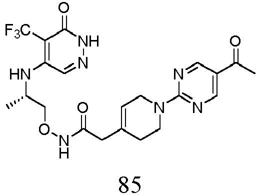 | 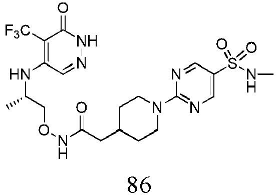 | 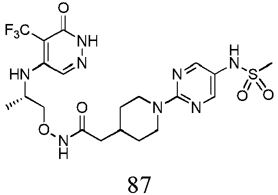 |
| LCMS: [M+H]⁺ = 495.2 | LCMS: [M+H]⁺ = 548.2 | LCMS: [M+H]⁺ = 548.2 |

### Example 88

### Biological Detection

### Determination of inhibition of PARP7 biological enzyme activity by compound.

The recombinant human PARP7 protein kit (Abcam, catalog number ab271651) was used for the assay. According to the kit instructions, 30 µL of histone coating solution (1.5 µg/mL) was first applied to the 384 well holes of the reaction plate and coated at 4°C for 2 hours. Then, the histone coated solution was washed off, and 50 µL sealing buffer was added to each well, and the sealing buffer was washed off after incubation at 25°C for 60 minutes. 1000 times the concentration of the compound was configured and diluted from 1 mM to 0.05 µM, and the configured compound was transferred to 384-well plates with 25 nL per well using Echo. The final concentration of the compound in the reaction system was 1000 nM, 333.33 nM, 111.11 nM, 37.03 nM, 12.34 nM, 4.11 nM, 1.37 nM, 0.45 nM, 0.15 nM, 0.05 nM, which was used as the test hole. At the same time, 25 nL DMSO was added to the negative control well and the positive control well. The PARP7 enzyme solution with a concentration of 1.67 times was prepared, and the PARP7 enzyme solution was added to the test hole and the positive control hole with 15 µE each, while the negative control hole was added with 15 µE base Buffer, and incubated at 25°C for 15 minutes. Then 10 µL 2.5 times Biotin-NAD+ substrate solution was added and incubated at 25°C for 60 min. After the reaction solution was washed off, the 384-well reaction plates were treated with closed buffer solution diluted with streptavidin-HRP (1:500), each well was 30 µL, and incubated at 25°C for 30 min. After the HRP buffer was washed off, 30µL HRP chemiluminescent substrate mixture was added to each well, and the luminescence signal (RLU) of each well was detected by Envision.

Calculation formula: Inhibition rate %= (average positive control -RLU comp ound)/(average positive control - average negative control))*100%. The dose-respo nse curves of each compound were fitted by GraphPad Prism 8 to get IC₅₀ valu es.

The results of the inhibition of PARP7 biological enzyme activity by each compound are listed in Table 1 below. IC₅₀ values less than 0.01 µM are labeled as "+++", IC₅₀ values from 0.01 to 0.1 µM are labeled as "++", and IC₅₀ values greater than 0.1 to 10.0 µM are labeled as "+".

**Table 1. Inhibition of PARP7 biological enzyme activity by each compound (IC₅₀)**

| Compound Number | PARP7 IC₅₀ | Compound Number | PARP7 IC₅₀ |
|---|---|---|---|
| 1 | +++ | 17 | +++ |
| 2 | +++ | 18 | +++ |
| 3 | ++ | 19 | +++ |
| 4 | ++ | 22 | +++ |
| 5 | +++ | 23 | +++ |
| 6 | +++ | 24 | +++ |
| 7 | +++ | 26 | +++ |
| 8 | ++ | 27 | +++ |
| 9 | +++ | 28 | +++ |
| 10 | +++ | 29 | +++ |
| 11 | +++ | 30 | +++ |
| 12 | +++ | 36 | +++ |
| 13 | +++ | 38 | +++ |
| 14 | +++ | 39 | +++ |
| 15 | +++ | 48 | +++ |
| 16 | ++ | 53 | +++ |

Inhibition of NCI-H1373 cancer cell growth by treatment with PARP7 inhibitor

Promega CellTiter-Glo cell activity assay kit (Item No.: Promege-G7573) was used to determine the cell activity. 90 µE NCI-H1373 cell suspension (ATCC, catalog No. CRL-5866, batch No. 58078716) was spread into the pores of the 96-well plate with 3500 cells per well. After 24 hours, the 10µL prepared 10× compound working solution was added into the pores of the cell culture plate with a final concentration of 10µM. 3.3333µM, 1.1111µM, 0.3704µM, 0.1235µM, 0.0412µM, 0.0137µM, 0.0046pM, 0.0015µM, 0.0005µM, 10µL DMSO was added to the solvent control, and the final concentration of DMSO was 0.2%. The 0-day plates were collected for analysis, and after 144 hours of culture, the growth condition of cells was detected according to the instructions of CellTiter-Glo cell activity assay kit. Add 50µL of CellTiter-Glo working liquid into each hole of the cell culture plate, shake the plates in the dark for 2 minutes until cell lysis, and place at room temperature for 10 minutes to detect the luminous signal (RUL) of each cell hole on the 2104En Vision board reader.

Calculation formula: Growth inhibition rate %= (1-(RLU compound -RLU Day0)/(RLU solvent control -RLU Day0))*100%. The dose-response curves of each compound were fitted by GraphPad Prism 8 to obtain IC50 values.

The results of each compound on NCI-H1373 cancer cell growth inhibition are listed in Table 2 below. For the matter, IC₅₀ values less than 0.1 µM are labeled "+++", values from 0.1 to 1 µM are labeled "++", and values greater than 1 to 10 µM are labeled "+".

**Table 2: Growth inhibition assay (IC₅₀) of NCI-H1373 cancer cells by each compound**

| Compound Number | NCI-H1373 IC₅₀ | Compound Number | NCI-H1373 IC₅₀ |
|---|---|---|---|
| 5 | +++ | 31 | ++ |
| 6 | ++ | 32 | ++ |
| 7 | + | 34 | ++ |
| 9 | ++ | 36 | +++ |
| 10 | ++ | 38 | ++ |
| 11 | + | 39 | ++ |
| 12 | ++ | 42 | + |
| 13 | + | 45 | + |
| 14 | + | 47 | + |
| 15 | ++ | 48 | +++ |
| 17 | +++ | 50 | + |
| 18 | + | 52 | + |
| 19 | + | 53 | ++ |
| 20 | + | 54 | + |
| 22 | +++ | 55 | + |
| 23 | ++ | 56 | + |
| 24 | ++ | 61 | + |
| 25 | +++ | 62 | + |
| 26 | +++ | | |
| 27 | ++ | | |
| 28 | ++ | | |
| 29 | +++ | | |
| 30 | +++ | | |

### Pharmacokinetic testing of compound

In the embodiment, compound RBN-2397 (Cat.No: HY-136174, MedChemEcpress, Shanghai, China), compound 17, and compound 25 were tested in mice to demonstrate their pharmacokinetic properties in mice.

Preparation method of the compound to be tested:
Formulation of intravenous and oral gavage administration solutions: The compounds to be tested were dissolved in solutions formulated with 5% DMSO, 10% solutol HS15, and 85% saline.

Dosage (measured as the amount of compound in the administration solution (mg)/mouse body weight (kg)): 3 mg/kg intravenously (IV), 10 mg/kg oral gavage (PO).

Experimental methods of pharmacokinetic study in mice:
A total of 18 CD-1 mice (Shanghai Jihui Experimental Animal Feeding Co., LTD.), male, weight 28-31 g, were fasted for 12 hours and randomly divided into 2 groups (Group A and Group B) with 9 mice in each group. Animals in Group A were given the compound solution by oral gavage at the dose of 10 mg/kg. Animals in Group B were given the compound solution by tail vein injection at the dose of 3 mg/kg, and blank blood was taken before administration. About 110 µL of venous blood was taken from animals in Group A at 0.25h, 0.5 h, 1 h, 2 h, 4 h, 8 h, and 24 h after administration. About 110 µL of venous blood was taken from animals in Group B at 0.083h, 0.25h, 0.5 h, 1 h, 2 h, 4 h, 8 h, and 24 h after administration, and all were placed in test tubes with heparin, centrifuged, and plasma was collected and stored at -70°C for testing.

The experimental results are shown in Table 3.

**Table 3: Comparison of pharmacokinetic data of compound RBN-2397, compound 17, and compound 25**

| **Compound** | **IV 3mg/kg** | | | **PO 10mg/kg** | | |
|---|---|---|---|---|---|---|
| | AUC ₗₐₛₜ | CL(iv) | T_{1/2} | AUC ₗₐₛₜ | Cₘₐₓ | F% |
| | (ng/mL*hr) | (L/hr/kg) | (hr) | (ng/mL*hr) | (ng/mL) | |
| RBN-2397 | 732 | 4.10 | 0.2 | 582 | 682 | 23.9 |
| Compound17 | 3206 | 0.93 | 1.62 | 6548 | 5323 | 61.3 |
| Compound25 | 2782 | 1.07 | 5.55 | 7061 | 7227 | 75.9 |

AUCₗₐₛₜ represents the area under the drug time curve from the beginning of the administration time to the last point, that is, the area surrounded by the blood drug concentration curve to the time axis. CL(iv) represents the drug clearance of intravenous administration; T_{1/2} represents the plasma half-life of the drug, which refers to the time required for half of the drug to be eliminated from the body. Cₘₐₓ represents the maximum concentration of the drug in the plasma after administration. F% indicates oral bioavailability of the drug.

As shown in Table 3, after 3 mg/kg intravenously injection, the AUCₗₐₛₜ of compound 17, that is, the plasma exposure was about 4.4 times that of RBN-2397, and that of compound 25 was about 3.8 times that of RBN-2397.

The drug clearance (CL(iv)) of compounds 17 and 25 after intravenous administration was only about 1/5 of that of RBN-2397, and the drug plasma half-life (T_{1/2}) of compounds 17 and 25 was 8 and 27.8 times that of RBN-2397, respectively. After oral gavage administration of 10mg/kg, the maximum blood concentration (Cmax) of compound 17 and 25 was 7.8 and 10.6 times that of RBN-2397, respectively. The area under the drug time curve (AUCₗₐₛₜ), that is, the plasma exposure was about 11.3 times and 12.1 times of RBN-2397, respectively. Oral bioavailability (F%) was 2.6 and 3.2 times that of RBN-2397, respectively. The experimental results showed that compounds 17 and 25 have better pharmacokinetic properties than RBN-2397, overcoming the shortcomings of compound RBN-2397, such as short half-life and poor oral bioavailability, and are expected to be translated into better clinical efficacy. Its excellent target-inhibiting activity and pharmacokinetic properties provide additional possibilities for its expansion into a wider range of clinical indications.

### Industrial applicability

The invention provides a PARP7 inhibitor that can be used to inhibit PARP7 activity, or to treat or prevent diseases, disorders or conditions regulated by or affected by PARP7 activity or which involve PARP7 activity or overexpression. Thus, the invention is suitable for industrial application.

Although the invention is described in detail herein, the invention is not limited thereto, and a person skilled in the art may make modifications in accordance with the principles of the invention, and therefore, all kinds of modifications made in accordance with the principles of the invention should be understood to fall within the scope of protection of the invention.

## Claims

1. A poly(adenosine diphosphate-ribose) polymerase-7 inhibitor comprising a compound of formula (I) or a pharmaceutically acceptable salt, deuterated compound, solvate, ester, acid, metabolite or prodrug thereof, 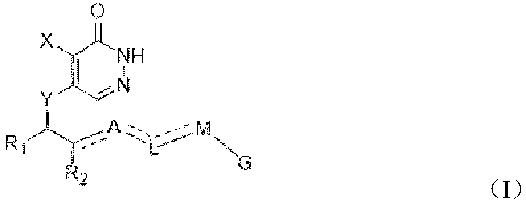 Wherein:
- represents a double or single bond, provided that at most one double bond is attached to either atom;
X is selected from the group consisting of halogen, C₁₋₆ haloalkyl, and C₂₋₆ alkanoyl, or forms a fused benzene ring or heterocycle together with Y and the carbon atoms to which they are attached;
Y is selected from the group consisting of NH and O, or forms a fused benzene ring or heterocycle together with X and the carbon atoms to which they are attached;
A is a nitrogen-oxo group selected from the group consisting of -N-O-, =N-O-, -O-N-, and -O-N=, and the N atom is optionally substituted with hydrogen, C₁₋₆ alkyl, C₂₋₆ alkanoyl, or C₁₋₆ sulfonyl when the valence thereof is not saturated;
L is selected from the group consisting of -(CH₂)ₙ-, 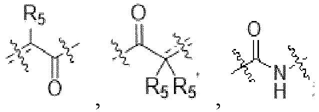 and 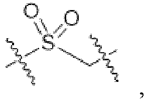 wherein n is 1, 2 or 3, R₅ and R₅' are each independently selected from the group consisting of hydrogen, C₁₋₃ alkyl, halogen, deuterium, and hydroxyl, or R₅ and R₅' together with the carbon atom to which they are attached form C₃₋₆ cycloalkyl;
R₁ and R₂ are each independently selected from the group consisting of hydrogen, C₁₋₆ alkyl, C₁₋₆ hydroxyalkyl, C₁₋₆ haloalkyl, C₃₋₆ cycloalkyl, C₃₋₆ hydroxycycloalkyl, or C₃₋₆ halocycloalkyl, or R₁ and R₂ together with the carbon atoms to which they are attached form C₅₋₈ cycloalkyl;
M is a moiety selected from the group consisting of formulae (M-1), (M-2), (M-3), (M-4) and (M-5): 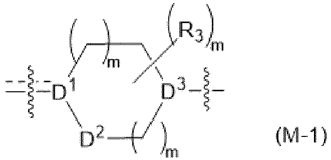 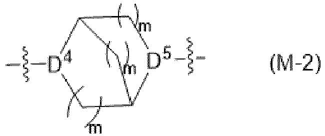 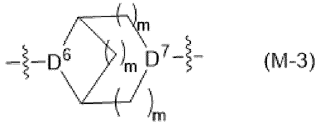 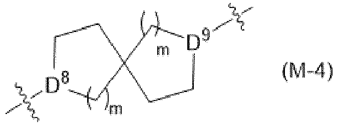 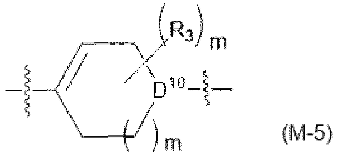
wherein D¹, D², D³, D⁴, D⁵, D⁶, D⁷, D⁸, D⁹, and D¹⁰ are each independently selected from the group consisting of N and CH, provided that at least one N is contained as the ring atom of the M moiety,
R₃ is each independently selected from the group consisting of hydrogen, halogen, hydroxyl, C₁₋₄ alkyl, C₁₋₆ haloalkyl, and C₁₋₆ hydroxyalkyl,
m is each independently selected from the group consisting of 0, 1 or 2;
G is a 5- to 6- membered aromatic heterocyclic group optionally substituted with one or two R₄ groups, wherein R₄ are each independently selected from the group consisting of hydroxyl, cyano, C₁₋₆ alkyl, C₁₋₆ hydroxyalkyl, C₁₋₆ haloalkyl, halogen, C₃₋₆ cycloalkyl, C₂₋₆ alkenyl, C₂₋₆ alkynyl, -NR₆R₆', -C(O)NR₆R₆', -NR₆C(O)R₇, -C(O)OR₆, -OR₆, -S(O)₂NR₆R₆', -NR₆S(O)₂R₇, -S(O)₂R₇, -C(O)R₆, and phenyl optionally substituted with halogen, or two R₄ groups together with the carbon atoms to which they are attached form C₅₋₈ cycloalkyl, phenyl, or heteroaryl optionally substituted with hydroxyl, methyl, halogen or oxo, wherein R₆, R₆' and R₇ are each independently selected from the group consisting of hydrogen, C₁₋₆ alkyl, C₁₋₆ haloalkyl, C₃₋₆ cycloalkyl, and C₁₋₆ hydroxyalkyl.

2. The inhibitor as claimed in claim 1, wherein M is a moiety selected from the group consisting of formulae (M-1a), (M-2a), (M-3a), (M-4a) and (M-5a): 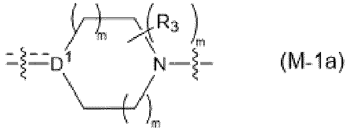 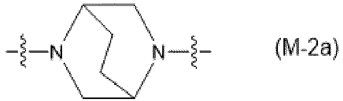 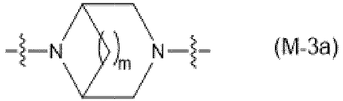 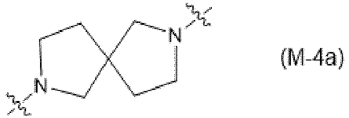 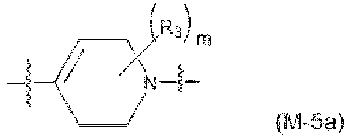 wherein D¹ is selected from the group consisting of N or CH, m is each independently selected from the group consisting of 0, 1 or 2, and R₃ is each independently selected from the group consisting of hydrogen, hydroxyl, halogen or C₁₋₄ alkyl.

3. The inhibitor as claimed in claim 1 or 2, wherein G is a moiety selected from the group consisting of the formula (G-1), (G-2), (G-3), (G-4), (G-5), (G-6) and (G-7): 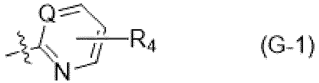 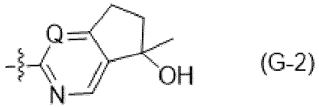 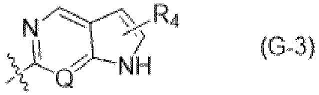 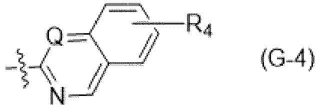 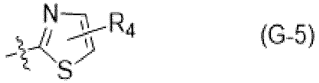 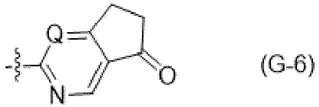 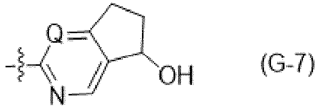
wherein Q is each independently selected from the group consisting of N or CH, and more preferably Q is each independently N;
R₄ groups are optional substituents and are each independently selected from the group consisting of cyano, C₁₋₄ alkyl, C₁₋₄ hydroxyalkyl, C₁₋₄ haloalkyl, halogen, C₃₋₆ cycloalkyl, C₂₋₄ alkenyl, C₂₋₄ alkynyl, -NR₆R₆', -C(O)NR₆R₆', -C(O)OR₆, -OR₆, -C(O)R₆, and phenyl optionally substituted with halogen, wherein R₆ and R₆' are each independently selected from the group consisting of hydrogen and C₁₋₄ alkyl.

4. The inhibitor as claimed in any one of claims 1 to 3, wherein X is selected from the group consisting of bromine, trifluoromethyl, and acetyl, or X forms a fused benzene ring or a fused piperidine ring together with Y and the carbon atoms to which they are attached;
Y is selected from NH or forms a fused benzene ring or a fused piperidine ring together with X and the carbon atoms to which they are attached.

5. The inhibitor as claimed in any one of claims 1 to 4, wherein L is selected from the group consisting of -(CH₂)ₙ-, 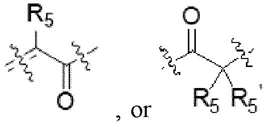 when D¹ in formula (M-1) or formula (M-1a) is N; and L is selected from the group consisting of -(CH₂)ₙ-, 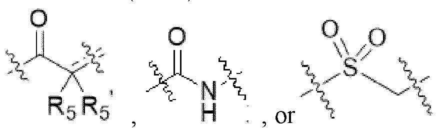 when D¹ is CH.

6. The inhibitor as claimed in any one of claims 1 to 5, wherein L is 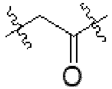 when A is -N-O-; L is 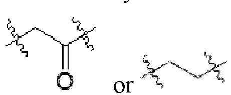 when A is =N-O-; L is 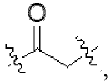 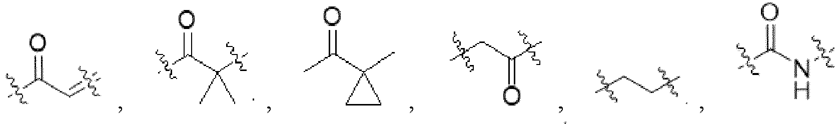 or 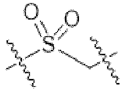 when A is -O-N-; and L is 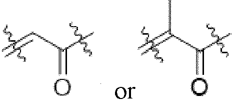 when A is -O-N=.

7. The inhibitor as claimed in any one of claims 1 to 6, wherein:
X is selected from the group consisting of C₁₋₆ haloalkyl and C₂₋₆ alkanoyl, and X is more preferably C₁₋₆ haloalkyl, in particular trifluoromethyl;
Y is NH;
A is a nitrogen-oxo group selected from the group consisting of =N-O-, -O-N- and -O-N=, more preferably -O-N-;
L is selected from the group consisting of 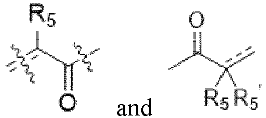 more preferably 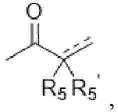 wherein R₅ and R₅' are each independently selected from the group consisting of hydrogen or halogen, more preferably hydrogen;
M is a moiety selected from the group consisting of formulae (M-1a) and (M-5a), wherein D¹ is selected from the group consisting of N or CH, more preferably CH, and m is independently 1, and R₃ is each independently selected from the group consisting of hydrogen, halogen or C₁₋₃ alkyl, more preferably hydrogen;
G is a moiety selected from the group consisting of the formulae (G-1), (G-3) and (G-4), more preferably formula (G-1), wherein Q is selected from the group consisting of N or CH, more preferably N, and R₄ is selected from the group consisting of C₁₋₆ haloalkyl, halogen, C₃₋₆ cycloalkyl, and phenyl optionally substituted with halogen, more preferably C₁₋₆ haloalkyl, in particular trifluoromethyl;
R₁ and R₂ are each independently selected from the group consisting of hydrogen, C₁₋₆ alkyl, and C₃₋₆ cycloalkyl, or R₁ and R₂ together with the carbon atoms to which they are attached form C₅₋₈ cycloalkyl, and more preferably, R₁ and R₂ are each independently selected from the group consisting of hydrogen and C₁₋₆ alkyl.

8. The inhibitor as claimed in any one of claims 1 to 7, wherein,
--- represents a double or single bond, provided that at most one double bond is attached to either atom;
X is selected from the group consisting of halogen and C₁₋₆ haloalkyl, or forms a fused benzene ring or heterocycle together with Y and the carbon atoms to which they are attached;
Y is NH or forms a fused benzene ring or heterocycle together with X and the carbon atoms to which they are attached;
A is a nitrogen-oxo group selected from the group consisting of -N-O-, =N-O-, -O-N-, and -O-N=, and the N atom is optionally substituted with hydrogen, C₁₋₆ alkyl, C₂₋₆ alkanoyl, or C₁₋₆ sulfonyl when the valence thereof is not saturated;
L is selected from the group consisting of -(CH₂)ₙ-, 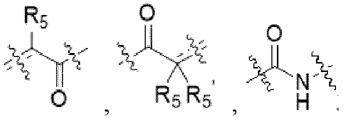 and 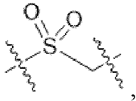 wherein n is 1, 2 or 3, and R₅ and R₅' are each independently selected from the group consisting of hydrogen or C₁₋₃ alkyl, or R₅ and R₅' together with the carbon atom to which they are attached form C₃₋₆ cycloalkyl;
R₁ and R₂ are each independently selected from the group consisting of hydrogen, C₁₋₆ alkyl, C₁₋₆ hydroxyalkyl, C₁₋₆ haloalkyl, C₃₋₆ cycloalkyl, C₃₋₆ hydroxycycloalkyl, or C₃₋₆ halocycloalkyl, or R₁ and R₂ together with the carbon atoms to which they are attached form C₅₋₈ cycloalkyl:
M is a moiety selected from the group consisting of formulae (M-1), (M-2), (M-3) and (M-4): 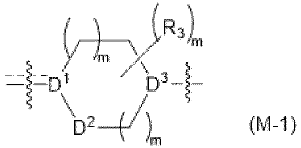 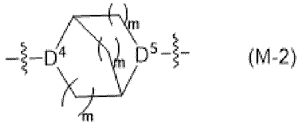 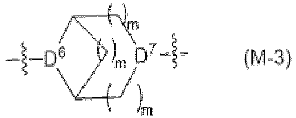 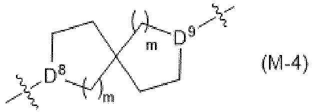
wherein D¹, D², D³, D⁴, D⁵, D⁶, D⁷, D⁸ and D⁹ are each independently selected from the group consisting of N and CH, provided that at least one N is contained as the ring atom of the M moiety,
R₃ is each independently selected from the group consisting of halogen, hydroxyl, C₁₋₄ alkyl and C₁₋₆ haloalkyl,
m is each independently selected from the group consisting of 0, 1 or 2;
G is a 5- to 6- membered aromatic heterocyclic group optionally substituted with one or two R₄ groups, wherein R₄ are each independently selected from the group consisting of hydroxyl, cyano, C₁₋₆ alkyl, C₁₋₆ hydroxyalkyl, and C₁₋₆ haloalkyl, or two R₄ groups together with the carbon atoms to which they are attached form C₅₋₈ cycloalkyl optionally substituted with hydroxyl, or methyl.

9. The inhibitor as claimed in claim 8, wherein M is a moiety selected from the group consisting of formulae (M-1a), (M-2a), (M-3a) and (M-4a): 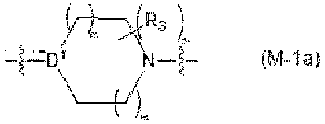 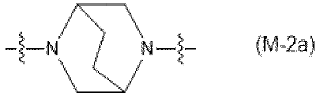 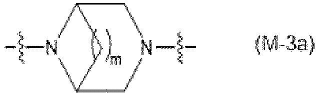 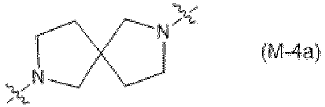 wherein D¹ is selected from the group consisting of N or CH, m is each independently selected from the group consisting of 0, 1 or 2, and R₃ is each independently selected from the group consisting of hydroxyl, halogen or C₁₋₄ alkyl.

10. The inhibitor as claimed in claim 8 or 9, wherein G is a moiety selected from the group consisting of the formulae (G-1) and (G-2): 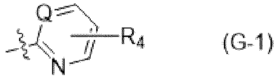 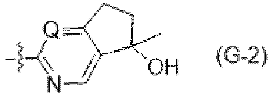 where Q is selected from the group consisting of N or CH.

11. The inhibitor as claimed in any one of claims 8 to 10, wherein L is selected from the group consisting of -(CH₂)ₙ-, 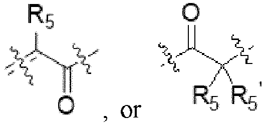 when D¹ in formula (M-1) or formula (M-1a) is N; and L is selected from the group consisting of -(CH₂)ₙ-, 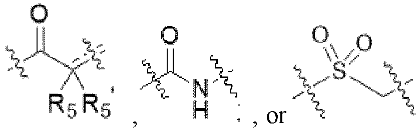 when D¹ is CH.

12. The inhibitor as claimed in any one of claims 8 to 11, wherein L is 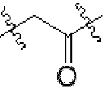 when A is -N-O-; L is 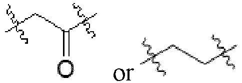 when A is =N-O-; L is 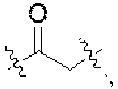 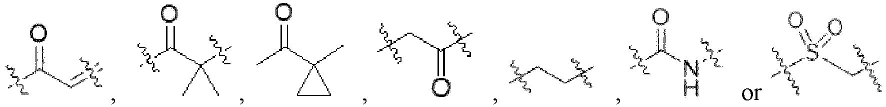 when A is -O-N-; and L is 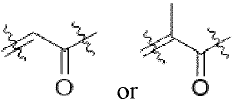 when A is -O-N=.

13. The inhibitor as claimed in any one of claims 8 to 12, comprising a compound of formula (II), or a pharmaceutically acceptable salt, deuterated compound, solvate, ester, acid, metabolite or prodrug thereof. 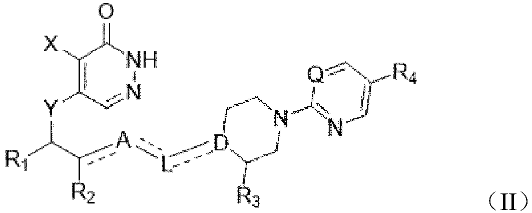
wherein, 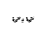 represents a double or single bond, provided that at most one double bond is attached to either atom,
X is selected from the group consisting of halogen and C₁₋₆ haloalkyl, or forms a fused benzene ring or piperidine ring together with Y and the carbon atoms to which they are attached;
Y is selected from NH or forms a fused benzene ring or piperidine ring together with X and the carbon atoms to which they are attached;
A is a nitrogen-oxo group selected from the group consisting of -N-O-, =N-O-, -O-N-, and -O-N=, and the N atom is optionally substituted with hydrogen, C₁₋₆ alkyl, C₂₋₆ alkanoyl, or C₁₋₆ sulfonyl when the valence thereof is not saturated;
L is selected from the group consisting of -(CH₂)ₙ-, 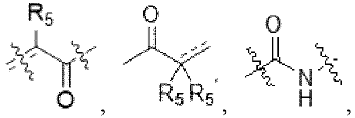 and 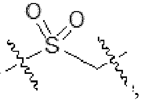 wherein n is 1, 2, or 3, and R₅ and R₅' are each independently selected from the group consisting of hydrogen or C₁₋₃ alkyl, or R₅ and R₅' together with the carbon atom to which they are attached form C₃₋₆ cycloalkyl;
D is selected from the group consisting of N or CH;
Q is selected from the group consisting of N or CH;
R₁ and R₂ are each independently selected from the group consisting of hydrogen, C₁₋₆ alkyl, C₃₋₆ cycloalkyl, or R₁ and R₂ together with the carbon atoms to which they are attached form C₅₋₈ cycloalkyl;
R₃ is selected from the group consisting of hydrogen or C₁₋₃ alkyl;
R₄ is selected from the group consisting of hydroxyl, cyano, C₁₋₆ alkyl, C₁₋₆ hydroxyalkyl and C₁₋₆ haloalkyl.

14. The inhibitor as claimed in any one of claims 1 to 13, wherein the compound of formula (I) is selected from the group consisting of:

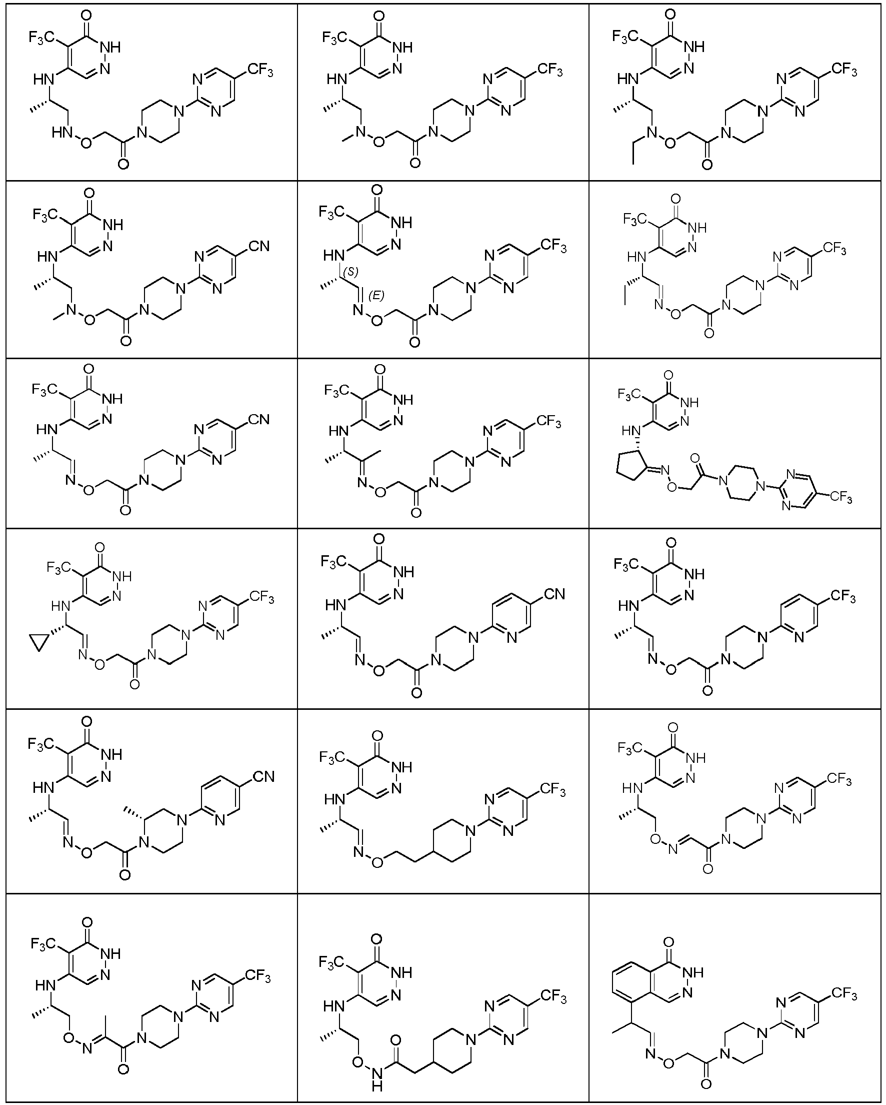

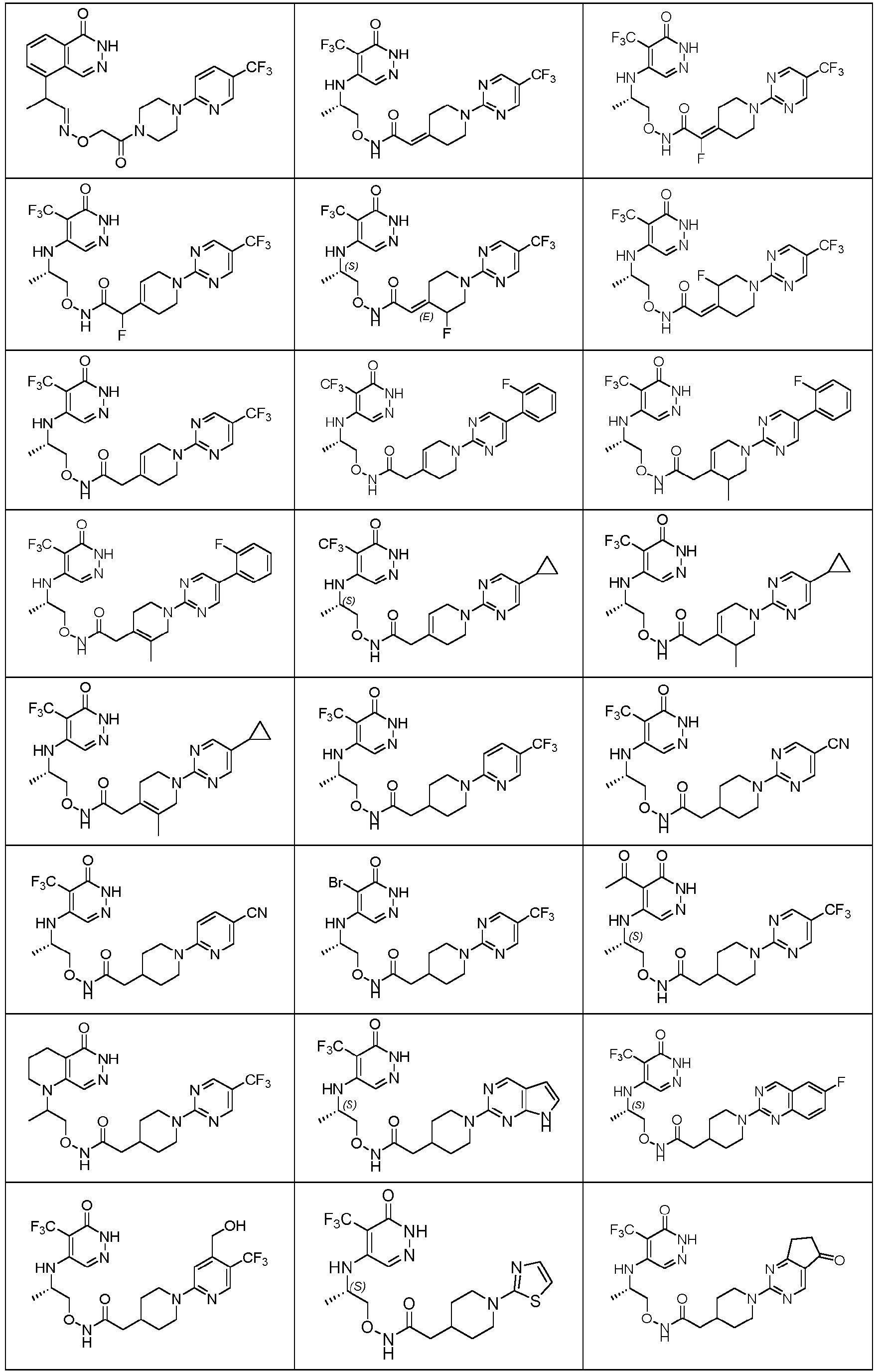

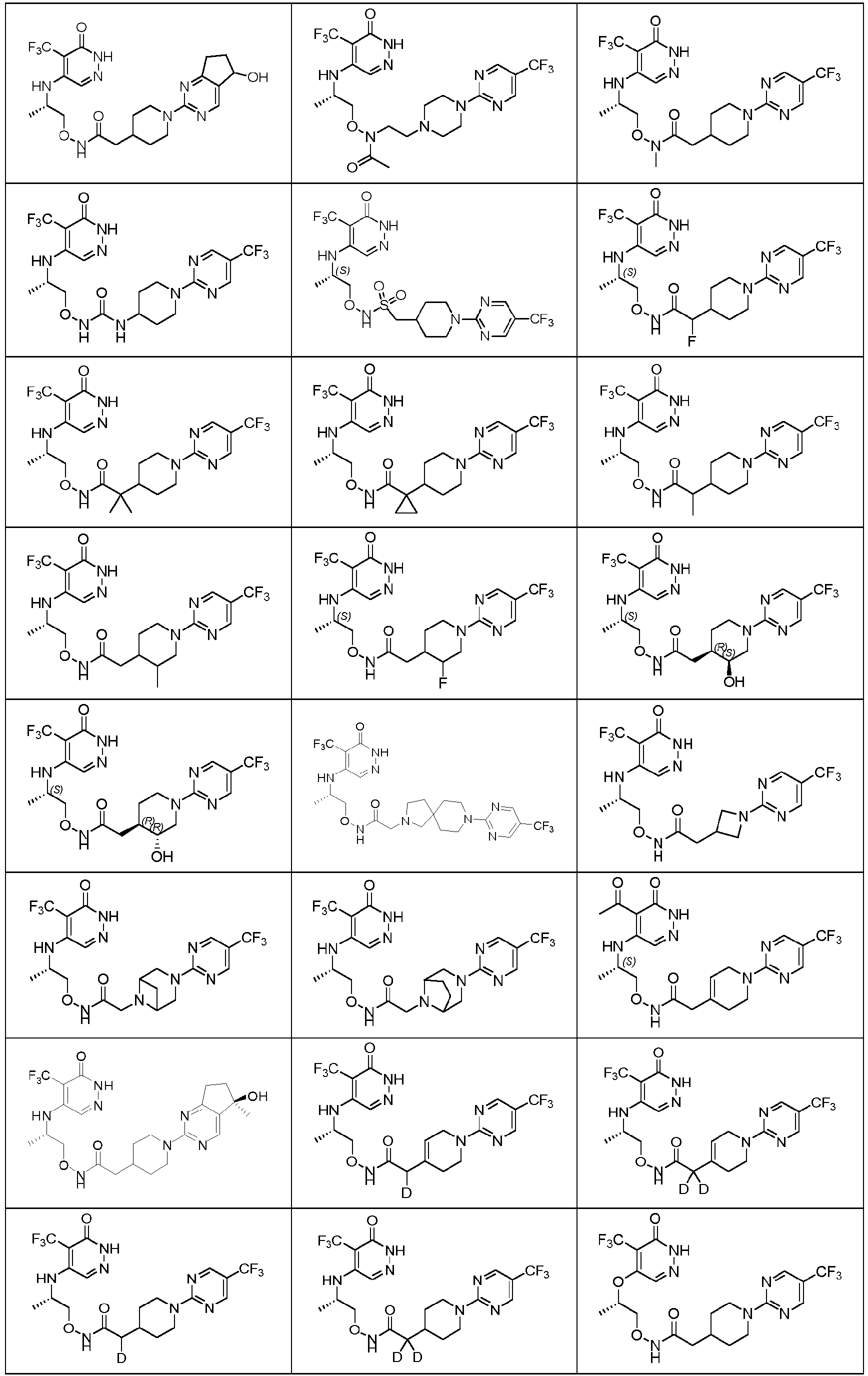

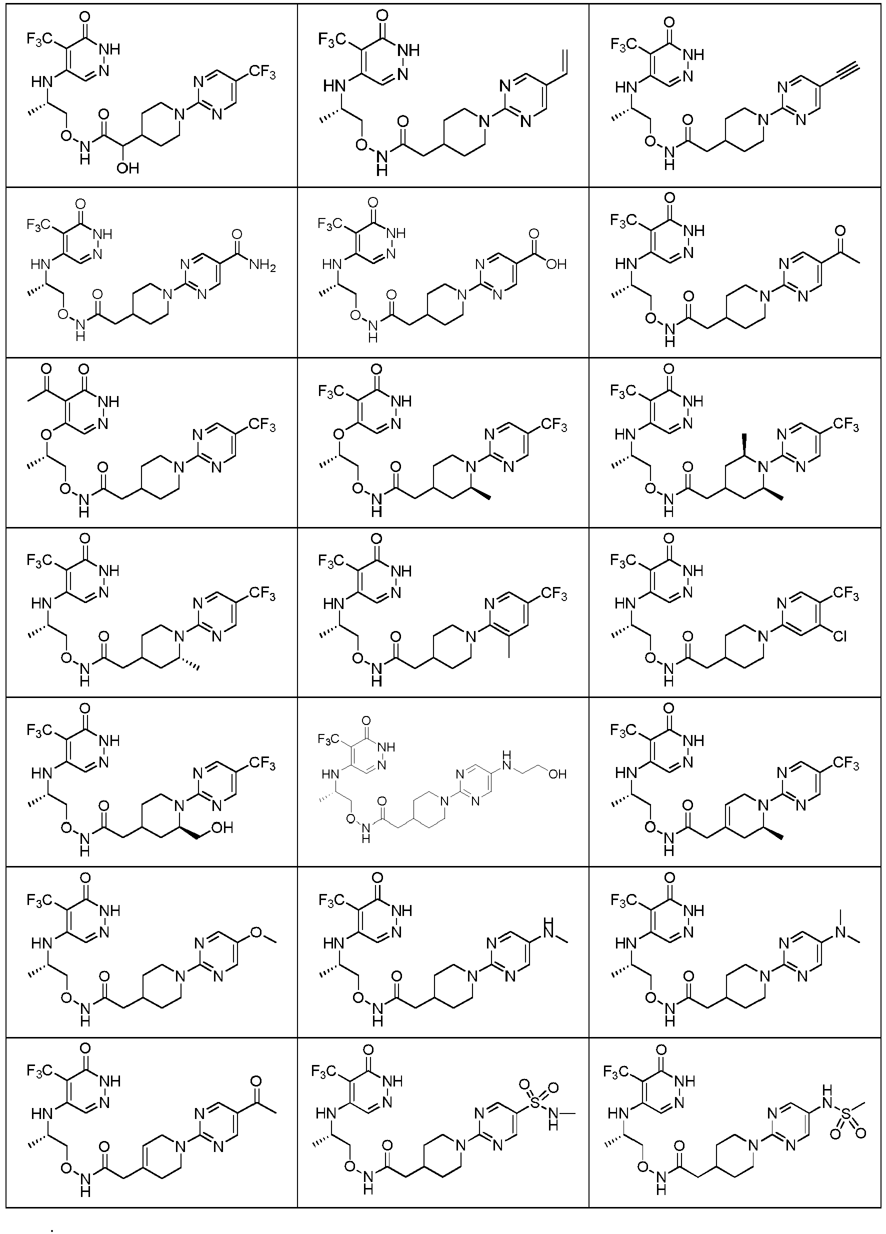

15. A pharmaceutical composition comprising the inhibitor according to any one of claims 1 to 14, and a pharmaceutically acceptable carrier or excipient, and optionally other therapeutic agents.

16. Use of the inhibitor according to any one of claims 1 to 14 in the preparation of a drug for treating or preventing a disease, disorder or condition regulated by or affected by PARP7 activity or which involves PARP7 activity or overexpression.

17. The use as claimed in claim 16, wherein said disease, disorder or condition is a hyperproliferative disease, autoimmune or inflammatory disease.

18. The use as claimed in claim 16 or 17, wherein said disease, disorder, or condition is a cancer selected from the group consisting of squamous lung cancer, lung adenocarcinoma, non-small cell lung cancer, small cell lung cancer, head and neck squamous cell carcinoma, breast cancer, pancreatic cancer, pancreatic ductal adenocarcinoma, colorectal cancer, melanoma, ovarian cancer, esophageal squamous cell carcinoma, gastric cancer, liver cancer, oral cancer, urothelial cancer, prostate cancer, bladder cancer, renal cell carcinoma, gastrointestinal stromal tumor, cervical cancer, endometrial cancer, rhabdomyosarcoma, fibrosarcoma, neuroendocrine tumor, mesothelioma, brain cancer, or malignant glioma, or
said disease, disorder, or condition is an autoimmune or inflammatory disease selected from the group consisting of ulcerative colitis, Crohn's disease, multiple sclerosis, autoimmune liver disease, type I diabetes mellitus, bronchial asthma, systemic lupus erythematosus, rheumatoid arthritis, ankylosing spondylitis, juvenile idiopathic arthritis, psoriasis, polymyositis, or dermatomyositis.

19. An inhibitor according to any one of claims 1 to 14 for use in the treatment or prevention of a disease, disorder or condition regulated by or affected by PARP7 activity or which involves PARP7 activity or overexpression.

20. The inhibitor for use as claimed in claim 19, wherein said disease, disorder or condition is a hyperproliferative disease, autoimmune or inflammatory disease.

21. The inhibitor for use as claimed in claim 19 or 20, wherein said disease, disorder, or condition is a cancer selected from the group consisting of squamous lung cancer, lung adenocarcinoma, non-small cell lung cancer, small cell lung cancer, head and neck squamous cell carcinoma, breast cancer, pancreatic cancer, pancreatic ductal adenocarcinoma, colorectal cancer, melanoma, ovarian cancer, esophageal squamous cell carcinoma, gastric cancer, liver cancer, oral cancer, urothelial cancer, prostate cancer, bladder cancer, renal cell carcinoma, gastrointestinal stromal tumor, cervical cancer, endometrial cancer, rhabdomyosarcoma, fibrosarcoma, neuroendocrine tumor, mesothelioma, brain cancer, or malignant glioma, or
said disease, disorder, or condition is an autoimmune or inflammatory disease selected from the group consisting of ulcerative colitis, Crohn's disease, multiple sclerosis, autoimmune liver disease, type I diabetes mellitus, bronchial asthma, systemic lupus erythematosus, rheumatoid arthritis, ankylosing spondylitis, juvenile idiopathic arthritis, psoriasis, polymyositis, or dermatomyositis.
